# EUROPÄISCHE PATENTSCHRIFT

(11) **EP 2 356 101 B1**
(45) Veröffentlichungstag und Bekanntmachung des Hinweises auf die Patenterteilung: **27.02.2013**
(21) Anmeldenummer: 09740860.3
(22) Anmeldetag: 22.10.2009
(51) Int. Cl.: C07D 401/04, C07D 401/12, C07D 401/14, C07D 471/10, C07D 487/10, A61K 31/506, A61P 25/04

(54) **PYRIMIDIN- UND TRIAZIN-SULFONAMIDDERIVATE ALS BRADYKININ B1 REZEPTOR (B1R) INHIBITOREN ZUR BEHANDLUNG VON SCHMERZ**
PYRIMIDINE- AND TRIAZINE-SULFONAMIDE DERIVATIVES AS BRADYKININ B1 RECEPTOR (B1R) INHIBITORS FOR THE TREATMENT OF PAIN
DÉRIVÉS DE PYRIMIDINE- ET DE TRIAZINE-SULFONAMIDE EN TANT QUE INHIBITEURS DU RÉCEPTEUR BRADYKININ (B1R) POUR LE TRAITEMENT DE LA DOULEUR

(30) Priorität: 23.10.2008 EP 08018514
(43) Veröffentlichungstag der Anmeldung: 17.08.2011
(73) Patentinhaber: Grünenthal GmbH, 52078 Aachen (DE)
(72) Erfinder: SCHUNK, Stefan, 52070 Aachen (DE); REICH, Melanie, 52072 Aachen (DE); HENNIG, Kamila, 52249 Eschweiler (DE); ENGELS, Michael, B-2300 Turnhout (BE); GERMANN, Tieno, 52080 Aachen (DE); JOSTOCK, Ruth, 52223 Stolberg (DE); HEES, Sabine, 52076 Aachen (DE)
(86) Internationale Anmeldenummer: PCT/EP2009/007568
(87) Internationale Veröffentlichungsnummer: WO 2010/046109

(56) Entgegenhaltungen:
- WO-A-01/05783
- WO-A-2008/046573

## Beschreibung

Die vorliegende Erfindung betrifft substituierte Pyrimidin- und Triazinderivate, Verfahren zu deren Herstellung, Arzneimittel enthaltend diese Verbindungen und die Verwendung von substituierten Pyrimidin- und Triazinderivaten zur Herstellung von Arzneimitteln.

Im Gegensatz zur konstitutiven Expression des Bradykinin 2 Rezeptors (B2R) wird der Bradykinin 1 Rezeptor (B1 R) in den meisten Geweben nicht oder nur schwach exprimiert. Allerdings ist die Expression des B1 R auf verschiedenen Zellen induzierbar. Beispielsweise erfolgt im Verlauf von Entzündungsreaktionen eine rasche und ausgeprägte Induktion des B1 R auf neuronalen Zellen aber auch verschiedenen peripheren Zellen wie Fibroblasten, Endothelzellen, Granulozyten, Makrophagen und Lymphozyten. Somit kommt es im Verlauf von Entzündungsreaktionen zu einem Switch von einer B2R zu einer B1 R Dominanz auf den beteiligten Zellen. Wesentlich an dieser B1R Heraufregulation beteiligt sind die Zytokine Interleukin-1 (IL-1) und Tumor Nekrose Faktor alpha (TNFα) (Passos et al. J. Immunol. 2004, 172, 1839-1847). Nach Aktivierung mit spezifischen Liganden können B1R-exprimierende Zellen anschließend selbst entzündungsfördernde Zytokine wie IL-6 und IL-8 sezernieren (Hayashi et al., Eur. Respir. J. 2000, 16, 452-458). Dies führt zur Einwanderung weiterer Entzündungszellen, z.B. neutrophiler Granulozyten (Pesquero et al., PNAS 2000, 97, 8140-8145). Über diese Mechanismen kann das Bradykinin-B1R-System zur Chronifizierung von Erkrankungen beitragen. Dies belegt eine Vielzahl tierexperimenteller Untersuchungen (Übersichten in Leeb-Lundberg et al., Pharmacol Rev. 2005, 57, 27-77 und Pesquero et al., Biol. Chem. 2006, 387, 119-126). Auch am Menschen zeigt sich eine verstärkte Expression des B1R, z.B. auf Enterozyten und Makrophagen im betroffenen Gewebe von Patienten mit entzündlichen Darmerkrankungen (Stadnicki et al., Am. J. Physiol. Gastrointest. Liver Physiol. 2005, 289, G361-366) bzw. auf T-Lymphozyten von Patienten mit Multipler Sklerose (Pratet al., Neurology. 1999;53,2087-2092) oder eine Aktivierung des Bradykinin-B2R-B1R Systems im Verlauf von Infektionen mit Staphyloccocus aureus (Bengtson et al., Blood 2006, 108, 2055-2063). Infektionen mit Staphyloccocus aureus sind verantwortlich für Krankheitsbilder wie oberflächliche Infektionen der Haut bis hin zu septischem Schock.

Aufgrund der dargestellten pathophysiologischen Zusammenhänge ergibt sich für die Anwendung von B1 R-Antagonisten ein großes therapeutisches Potential bei akuten und insbesondere chronisch-entzündlichen Erkrankungen. Hierzu gehören Erkrankungen der Atemwege (Asthma bronchiale, Allergien, COPD/chronic-obstruktive pulmonary disease, zystische Fibrose usw.), entzündliche Darmerkrankungen (Ulcerative Colitis, CD/Crohn's disease usw.), neurologische Erkrankungen (Multiple Sklerose, Neurodegeneration usw.), Entzündungen der Haut (atopische Dermatitis, Psoriasis, bakterielle Infektionen usw.) und Schleimhäute (M. Behcet, Pelvitis, Prostatitis usw.), rheumatische Erkrankungen (rheumatoide Arthritis, Osteoarthritis usw.), septischen Schock und Reperfusionssyndrom (nach Herzinfarkt, Schlaganfall).

Darüber hinaus ist das Bradykinin(Rezeptor)-System auch an der Regulation der Angiogenese beteiligt (Potential als Angiogenese-Inhibitor bei Krebs sowie Makula-Degeneration am Auge) und B1 R-knockout Mäuse sind geschützt vor der Induktion von Übergewicht durch eine besonders fettreiche Ernährung (Pesquero et al., Biol. Chem. 2006, 387, 119-126). B1 R-Antagonisten eignen sich daher auch zur Behandlung von Fettleibigkeit.

B1 R-Antagonisten sind insbesondere geeignet zur Behandlung von Schmerz, insbesondere Entzündungsschmerz und neuropathischem Schmerz (Calixto et al., Br. J. Pharmacol 2004, 1-16), hier insbesondere von diabetischer Neuropathie (Gabra et al., Biol. Chem. 2006, 387, 127-143). Weiterhin eignen sie sich zur Behandlung von Migräne.

Bei der Entwicklung von B1R-Modulatoren besteht jedoch das Problem, dass der humane und der Ratten-B1-Rezeptor sich so stark unterscheiden, dass viele Verbindungen, die gute B1R-Modulatoren am humanen Rezeptor sind, nur eine schlechte oder keine Affinität zum Ratten-Rezeptor aufweisen. Dies erschwert die tierpharmakologischen Untersuchungen beträchtlich, da viele Untersuchungen normalerweise an der Ratte durchgeführt werden. Wenn jedoch keine Wirksamkeit am Ratten-Rezeptor vorhanden ist, können weder Wirkung noch Nebenwirkung an der Ratte untersucht werden. Dies hat bereits dazu geführt, dass transgene Tiere mit humanen B1-Rezeptoren für tierpharmakologische Untersuchungen erzeugt wurden (Hess et al., Biol. Chem 2006; 387(2):195-201). Die Arbeit mit transgenen Tieren ist jedoch teurer als die Arbeit mit den unveränderten Tieren.

In den Patentanmeldungen WO 2008/040492 und WO 2008/046573 werden Verbindungen beschrieben, die in in-vitro-Assays sowohl am humanen B1-Rezeptor als auch am B1-Rezeptor der Ratte antagonistische Wirkung zeigen.

In den Patentanmeldungen WO 2007/140383 und WO 2007/101007 werden Verbindungen beschrieben, die in in-vitro-Assays eine antagonistische Wirkung am Makak-B1-Rezeptor aufweisen. Experimentelle Daten zur Aktivität am humanen B1-Rezeptor oder dem B1-Rezeptor der Ratte sind nicht offenbart.

Es besteht weiterhin der Bedarf an neuen B1 R-Modulatoren, wobei B1 R-Modulatoren, die sowohl an den Ratten-Rezeptor als auch an den humanen Rezeptor binden, besondere Vorteile bieten.

Eine Aufgabe der vorliegenden Erfindung bestand daher darin, neue Verbindungen zur Verfügung zu stellen, die sich insbesondere als pharmakologische Wirkstoffe in Arzneimitteln eignen, vorzugsweise in Arzneimitteln zur Behandlung von Störungen oder Krankheiten, die zumindest teilweise durch B1 R-Rezeptoren vermittelt werden.

Diese Aufgabe wird durch die erfindungsgemäßen substituierten Pyrimidin- und Triazin-Derivate gelöst.

Ein Gegenstand der Erfindung sind daher Verbindungen der allgemeinen Formel I 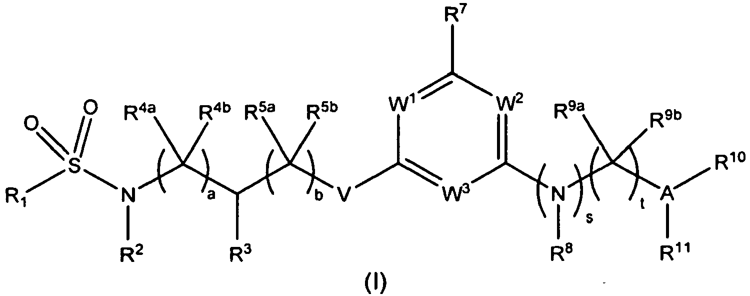 worin
a für 0, 1 oder 2 steht;
b für 0, 1, oder 2 steht;
R¹ für Aryl, Heteroaryl oder ein über eine C₁₋₃-Alkylengruppe gebundenes Aryl oder Heteroaryl steht;
R² und R³ wie unter (i) oder (ii) beschrieben definiert sind:
   (i) R² für H, C₁₋₆-Alkyl, C₃₋₈-Cycloalkyl, Aryl oder Heteroaryl steht; oder R² ein über eine C₁₋₆-Alkylengruppe, C₂₋₆-Alkenylengruppe oder C₂₋₆-Alkinylengruppe gebundenes C₃₋₈-Cycloalkyl, Aryl oder Heteroaryl bedeutet;
      R³ für H, F, Cl, Br, I, -CF₃, -OCF₃, OH, O-C₁₋₆-Alkyl, C₁₋₆-Alkyl, C₃₋₈Cycloalkyl, Aryl, Heteroaryl steht; oder R³ ein über eine C₁₋₆-Alkylengruppe, C₂₋₆-Alkenylengruppe oder C₂₋₆-Alkinylengruppe gebundenes C₃₋₈Cycloalkyl, Aryl oder Heteroaryl bedeutet;
      oder
   (ii) R² und R³ zusammen mit der sie verbindenden Gruppe -N-(CR^{4a}R^{4b})ₐ-CH-einen Heterocyclus bilden, der an einem oder mehreren seiner Kohlenstoff-Ringgliedern mit einem oder mehreren Resten unabhängig voneinander ausgewählt aus der Gruppe bestehend aus F, Cl, Br, I, -CF₃, =O, -O-CF₃, -OH, -SH, -O-C₁₋₆-Alkyl, C₁₋₆-Alkyl, C₃₋₈Cyclo-alkyl, Aryl und Heteroaryl substituiert und/oder mit einem Aryl- oder Heteroaryl anelliert sein kann und/oder zwei seiner Kohlenstoffringglieder über eine C₁₋₃-Alkylenbrücke miteinander verbunden sind,
      worin der Heterocyclus gesättigt oder wenigstens einfach ungesättigt, nicht aber aromatisch ist, 4-, 5-, 6- oder 7-gliedrig ist, neben dem N-Heteratom, an welches der Rest R² gebunden ist, ein oder mehrere Heteroatome oder Heteroatomgruppen unabhänig voneinander ausgewählt aus der Gruppe bestehend aus N, NR⁵⁰, O, S, S=O oder S(=O)₂ enthalten kann; wobei der Rest R⁵⁰ H, C₁₋₆-Alkyl, -C(=O)-R⁵¹, C₃₋₈-Cycloalkyl, Aryl, Heteroaryl oder ein über eine C₁₋₃-Alkylengruppe gebundenes C₃₋₈-Cycloalkyl, Aryl oder Heteroaryl bedeutet, und R⁵¹ C₁₋₆-Alkyl, C₃₋₈-Cycloalkyl, Aryl, Heteroaryl oder ein über eine C₁₋₃-Alkylengruppe gebundenes C₃₋₈-Cycloalkyl, Aryl oder Heteroaryl bedeutet;
      V für C(R^{6a})(R^{6b}), NR^{6c}, O oder eine Einfachbindung steht,
      wobei R^{6c} für einen Rest aus der Gruppe H, C₁₋₆-Alkyl, C₃₋₈-Cycloalkyl, Aryl, Heteroaryl oder für ein über eine C₁₋₆-Alkylengruppe, C₂₋₆-Alkenylengruppe oder C₂₋₆-Alkinylengruppe gebundenes C₃₋₈Cycloalkyl, Aryl oder Heteroaryl steht,
      R^{4a}, R^{4b}, R^{5a}, R^{5b}, R^{6a}, R^{6b}, unabhängig voneinander, für H, F, Cl, Br, I, -CF₃, O-CF₃, OH, SH, O-C₁₋₆-Alkyl, C₁₋₆-Alkyl, C₃₋₈Cycloalkyl, Aryl, oder Heteroaryl stehen; oder für ein über eine C₁₋₆-Alkylengruppe oder C₂₋₆-Alkenylengruppe gebundenes C₃₋₈Cycloalkyl, Aryl oder Heteroaryl stehen; und R^{6a} und R^{6b} zusätzlich zusammen =O bedeuten können;
      und/oder
      R^{4a} und R^{4b} gemeinsam mit dem sie verbindenden C-Atom einen gesättigten Cyclus bilden, der unsubstituiert oder an einem oder mehreren, beispielsweise 1, 2, 3 oder 4, seiner Kohlenstoff-Ringglieder mit einem oder mehreren, beispielsweise 1, 2, 3 oder 4, Substituenten unabhängig voneinander ausgewählt aus der Gruppe bestehend aus F, CF₃, C₁₋₆-Alkyl, O-C₁₋₆-Alkyl, OH, OCF₃, Aryl und Heteroaryl substituiert ist, wobei der Cyclus 3,- 4-, 5- oder 6-gliedrig ist, und ggf. ein oder mehr, beispielsweise 1 oder 2, Sauerstoffatome enthalten kann;
      R⁷ für einen Substituenten aus der Gruppe H, C₁₋₆-Alkyl, -CN, -CF₃, OH, C₁₋₆-Alkoxy, -O-CF₃ steht;
      W¹, W² und W³ unabhängig voneinander für N oder CR⁶⁰ stehen, mit der Maßgabe, dass von W¹, W² und W³ mindestens zwei N darstellen, und R⁶⁰ für H, C₁₋₆-Alkyl, Halogen, -CN, CF₃, OH, C₁₋₆-Alkoxy oder -O-CF₃ steht;
      s = 0 oder 1 ist,
      t = 0, 1, 2 oder 3 ist,
      R⁸ für H; C₁₋₆-Alkyl; C₃₋₈-Cycloalkyl; Aryl oder Heteroaryl; über eine C₁₋₆-Alkylengruppe gebundenes C₃₋₈-Cycloalkyl, Aryl oder Heteroaryl, steht;
      R^{9a} und R^{9b} jeweils unabhängig voneinander H; F; Cl; OH; C₁₋₆-Alkyl; O-C₁₋₆-Alkyl; C₃₋₈-Cycloalkyl; Aryl oder Heteroaryl; über eine C₁₋₆-Alkylengruppe gebundenes C₃₋₈-Cycloalkyl, Aryl oder Heteroaryl, bedeuten;
      - A: für N oder CH steht,
      mit der Maßgabe, dass wenn s für 1 und t für 0 steht, A für CH steht; und
      mit der Maßgabe, dass wenn s und t jeweils für 0 stehen, A für N steht;
      die Reste R¹⁰ und R¹¹ unter Einschluss von A eine spirocyclische oder cyclische Gruppe gemäß einer der allgemeinen Formeln II oder III darstellen, 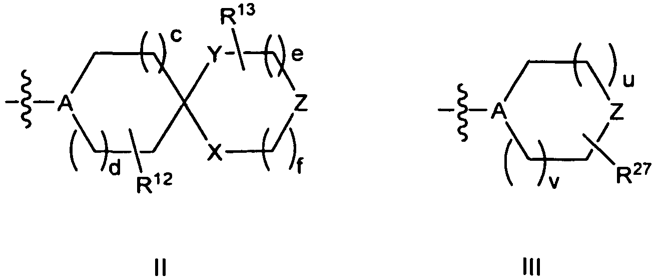 wobei
      - c, d, e, f, u und v: jeweils unabhängig voneinander 0, 1 oder 2 bedeuten;
      R¹², R¹³ und R²⁷ jeweils unabhängig voneinander für 0 bis 4 Substituenten stehen, die jeweils unabhängig voneinander ausgewählt sind aus F; Cl; OH; =O; C₁₋₆-Alkyl; O-C₁₋₆-Alkyl; C₃₋₈-Cycloalkyl; Aryl oder Heteroaryl; über eine C₁₋₆-Alkylengruppe gebundenes C₃₋₈-Cycloalkyl, Aryl oder Heteroaryl;
      und/oder jeweils zwei Substituenten R²⁷ zusammen eine C₁₋₃-Alkylenbrücke darstellen, so dass der in der allgemeinen Formel III dargestellte Cyclus eine bicyclisch verbrückte Form annimmt;
      und/oder zwei benachbarte Substituenten R¹³ ein anelliertes Aryl oder Heteroaryl bilden;
      und/oder zwei benachbarte Substituenten R²⁷ ein anelliertes Aryl oder Heteroaryl bilden
      - X: für CR^{14a}R^{14b}, NR¹⁵ oder O steht;
      - Y: für CR^{16a}R^{16b}, NR¹⁷ oder O steht;
      mit der Maßgabe, dass X nicht NR¹⁵ bedeutet, wenn Y NR¹⁷ bedeutet; und
      mit der Maßgabe, dass X und Y nicht gleichzeitig O bedeuten;
      wobei
      R^{14a}, R^{14b}, R^{16a} und R^{16b} jeweils unabhängig voneinander H; F; Cl; OH; C₁₋₆-Alkyl; O-C₁₋₆-Alkyl; C₃₋₈-Cycloalkyl; Aryl oder Heteroaryl; über eine C₁₋₆-Alkylengruppe gebundenes C₃₋₈-Cycloalkyl, Aryl oder Heteroaryl; bedeuten
      und/oder jeweils R^{14a} und R^{14b} gemeinsam für =O und/oder jeweils R^{16a} und R^{16b} gemeinsam für =O stehen können;
      R¹⁵ und R¹⁷ jeweils unabhängig voneinander für H; C₁₋₆-Alkyl; C₃₋₈-Cycloalkyl, Aryl oder Heteroaryl; über eine C₁₋₆-Alkylengruppe gebundenes C₃₋₈-Cycloalkyl, Aryl oder Heteroaryl stehen;
      - Z: in der allgemeinen Formel II für CR^{11a}R^{18b}, NR¹⁹ oder O steht;
      - Z: in der allgemeinen Formel II, in dem Fall, dass X für O und f für 0 steht, - (C(R¹²⁴)-C(R¹²⁵))-bedeutet, wobei
      R¹²⁴ und R¹²⁵ gemeinsam mit den sie verbindenden Kohlenstoffatomen ein ankondensiertes Aryl oder Heteroaryl bilden;
      oder
      - Z: in der allgemeinen Formel II, in dem Fall dass X für O und f für 0 steht, =N-(CR¹²⁶)- bedeutet, wobei das N-Atom einfach an das O-Atom gebunden ist, und
      - R¹²⁶: für H; C₁₋₆Alkyl; C₃₋₈Cycloalkyl; Aryl oder Heteroaryl; über eine C₁₋₆-Alkylengruppe gebundenes C₃₋₈Cycloalkyl, Aryl oder Heteroaryl steht;
      - Z: in der allgemeinen Formel III für CR^{18a}R^{18b}, NR¹⁹, O, S, S(=O) oder S(=O)₂ steht;
      wobei
      R^{18a} für H; C₁₋₆-Alkyl; C₃₋₈-Cycloalkyl; Aryl oder Heteroaryl; über eine C₁₋₆-Alkylengruppe gebundenes C₃₋₈-Cycloalkyl, Aryl oder Heteroaryl, steht;
      oder R^{18a} für eine Gruppe gemäß der allgemeinen Formel IV steht 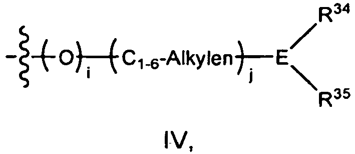 worin
      i und j jeweils unabhängig voneinander für 0 oder 1 stehen;
      E für N oder CH steht,
      mit der Maßgabe, dass wenn i für 1 und j für 0 steht, E für CH steht;
      R³⁴und R³⁵ jeweils unabhängig voneinander H; C₁₋₆-Alkyl;
      C₃₋₈Cycloalkyl; Aryl oder Heteroaryl; über eine C₁₋₃-Alkylengruppe gebundenes Aryl, Heteroaryl oder C₃₋₈Cycloalkyl bedeuten; oder
      R³⁴ und R³⁵ unter Einschluss von E einen 5- oder 6-gliedriges Aryl oder Heteroaryl bilden;
      oder R³⁴ und R³⁵ unter Einschluss von E einen gesättigten Heterocyclus gemäß der allgemeinen Formel V bilden, 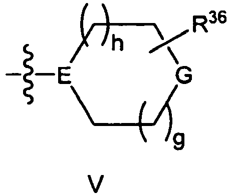 wobei
      - h und g: unabhängig voneinander 0, 1 oder 2 bedeuten;
      - G: für CR^{37a}R^{37b}, NR³⁸, O, S, S=O oder S(=O)₂ steht, mit der Maßgabe, dass wenn E für CH steht, G nicht für CR^{37a} R^{37b} steht;
      R³⁶ für 0 bis 4 Substituenten steht, die jeweils unabhängig voneinander ausgewählt sind aus F; Cl; Br; I; OH; SH; =O; O-C₁₋₆-Alkyl; C₁₋₆-Alkyl; C₃₋₈-Cycloalkyl; Aryl oder Heteroaryl; über eine C₁₋₆-Alkylengruppe gebundenes C₃₋₈-Cycloalkyl, Aryl oder Heteroaryl;
      und/oder zwei benachbarte Substituenten R³⁶ zusammen ein anelliertes Aryl oder Heteroaryl darstellen;
      R^{37a} und R^{37b} jeweils unabhängig voneinander H; F; Cl; Br; I; OH; SH; =O; O-C₁₋₆-Alkyl; C₁₋₆-Alkyl; C₃₋₈-Cycloalkyl; Aryl oder Heteroaryl; über eine C₁₋₆-Alkylengruppe gebundenes C₃₋₈-Cycloalkyl, Aryl und Heteroaryl bedeuten;
      R³⁸ für H; C₁₋₆Alkyl; C₃₋₈Cycloalkyl; Aryl oder Heteroaryl; über eine C₁₋₃-Alkylengruppe gebundenes Aryl, Heteroaryl oder C₃₋₈-Cycloalkyl steht;
      wobei
      R^{18b} für H; OH; C₁₋₆-Alkyl; C₃₋₈-Cycloalkyl; O-C₁₋₆Alkyl; O-(C₃₋₈-Cycloalkyl); (C₁₋₆-Alkylen)-O-C₁₋₆Alkyl; (C₁₋₆Alkylen)-O-(C₃₋₈-Cycloalkyl); Aryl oder Heteroaryl; O-Aryl oder O-Heteroaryl; über C₁₋₆-Alkylen gebundenes Aryl, O-Aryl, Heteroaryl oder O-Heteroaryl steht;
      oder R^{18b} für eine Gruppe gemäß der allgemeinen Formel VI steht, 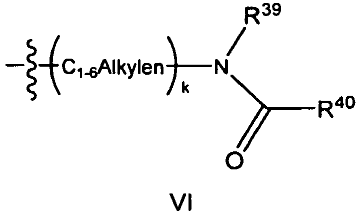 worin
      - k: für 0 oder 1 steht;
      R³⁹ für H; C₁₋₆-Alkyl; C₃₋₈-Cycloalkyl; Aryl oder Heteroaryl; über eine C₁₋₃-Alkylengruppe gebundenes C₃₋₈-Cycloalkyl, Aryl oder Heteroaryl; steht;
      R⁴⁰ für C₁₋₆-Alkyl; C₃₋₈-Cycloalkyl; Aryl oder Heteroaryl; über eine C₁₋₆-Alkylengruppe gebundenes C₃₋₈-Cycloalkyl, Aryl oder Heteroaryl; steht;
      oder
      R³⁹ und R⁴⁰ gemeinsam mit der sie verbindenden N-C(=O)-Gruppe einen Ring gemäß der allgemeinen Formel VII bilden, 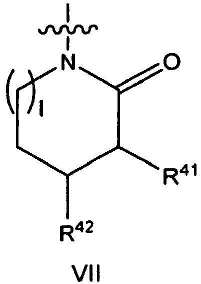 worin
      I für 0, 1 oder 2 steht
      und R⁴¹ und R⁴² gemeinsam mit den sie verbindenden Kohlenstoffatomen ein anelliertes Aryl oder Heteroaryl bilden;
      wobei R¹⁹ für H; oder (P)_{z}-R²² steht,
      wobei
      - Z: für 0 oder 1 steht;
      - P: für (C=O), S(=O)₂, oder C(=O)-N(R²⁴) steht; wobei das Stickstoffatom der C(=O)-N(R²⁴)-Gruppe mit R²² verknüpft ist;
      R²⁴ für H; C₁₋₆-Alkyl; C₃₋₈Cycloalkyl; über eine C₁₋₃-Alkylengruppe gebundenes Aryl, Heteroaryl oder C₃₋₈Cycloalkyl steht;
      R²² für C₁₋₆-Alkyl; Aryl oder Heteroaryl; über eine C₁₋₆-Alkylengruppe gebundenes Aryl oder Heteroaryl steht; oder
      R²² für eine Gruppe gemäß der allgemeinen Formel VIII steht, worin 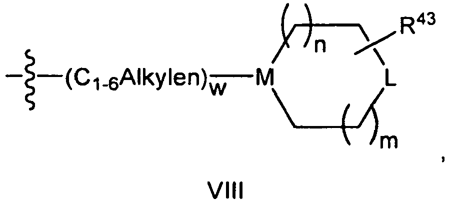
      - n: für 0, 1 oder 2 steht;
      - m: für 0, 1 oder 2 steht;
      - w: für 0 oder 1 steht,
      - M: für CH oder N steht;
      mit der Maßgabe, dass wenn P für C(=O)-NR²⁴ und w für 0 steht, M für CH steht; und
      mit der Maßgabe, dass wenn z und w gleichzeitig für 0 stehen, M für CH steht;
      - L: für CR^{44a}R^{44b}, NR⁴⁵, O, S, S=O oder S(=O)₂ steht;
      R⁴³ für 0 bis 4 Substituenten steht, die jeweils unabhängig voneinander ausgewählt sind aus F; Cl; OH; =O; C₁₋₆-Alkyl; O-C₁₋₆-Alkyl; C₃₋₈-Cycloalkyl; Aryl oder Heteroaryl; über eine C₁₋₆-Alkylengruppe gebundenes C₃₋₈-Cycloalkyl, Aryl oder Heteroaryl;
      und/oder zwei benachbarte Reste R⁴³ zusammen ein anelliertes Aryl- oder Heteroaryl darstellen;
      R^{44a} und R^{44b} jeweils unabhängig voneinander für H; F; Cl; Br; I; OH; C₁₋₆-Alkyl; O-C₁₋₆-Alkyl; C₃₋₈-Cycloalkyl; Aryl oder Heteroaryl; über eine C₁₋₆-alkylengruppe gebundenes C₃₋₈-Cycloalkyl, Aryl oder Heteroaryl stehen;
      oder R^{44a} und R^{44b} gemeinsam für =O stehen können;
      R⁴⁵ für H; C₁₋₆Alkyl; C₃₋₈Cycloalkyl; Aryl oder Heteroaryl; über eine C₁₋₃-Alkylengruppe gebundenes Aryl, Heteroaryl oder C₃₋₈-Cycloalkyl steht;
wobei die oben genannten Reste C₁₋₆-Alkyl, C₁₋₃-Alkylen, C₁₋₆-Alkylen, C₂₋₆-Alkenylen, C₂₋₆-Alkinylen, C₃₋₆-Cycloalkyl, C₃₋₈-Cycloalkyl, Aryl und Heteroaryl jeweils unsubstituiert oder einfach oder mehrfach mit gleichen oder verschiedenen Resten substituiert sein können; die oben angegebenen Reste C₁₋₆-Alkyl, C₁₋₃-Alkylen, C₁₋₆-Alkylen, C₂₋₆-Alkenylen und C₂₋₆-Alkinylen, jeweils verzweigt oder unverzweigt sein können;
in Form eines einzelnen Enantiomers oder eines einzelnen Diastereomers, des Razemats, der Enantiomere, der Diastereomere, Mischungen der Enantiomere und/oder Diastereomere, sowie jeweils in Form ihrer Basen und/ oder physiologisch verträglichen Salze.

In der oben verwendeten, allgemeinen Formel IV sollen die zwischen E und den Resten R³⁴ und R³⁵ gezeigten Bindungen nicht ausschließlich als Einfachbindungen verstanden werden, sondern können auch Teil eines aromatischen Systems sein. Im Sinne der vorliegenden Erfindung steht der Begriff "Halogen" vorzugsweise für die Reste F, Cl, Br und I, insbesondere für die Reste F und Cl.

Der Ausdruck "C₁₋₆-Alkyl" umfasst im Sinne dieser Erfindung acyclische gesättigte Kohlenwasserstoffreste mit 1, 2, 3, 4, 5 oder 6 C-Atomen, die verzweigt- oder geradkettig (unverzweigt) sowie unsubstituiert oder ein- oder mehrfach, beispielsweise 2-, 3-, 4- oder 5-fach, mit gleichen oder verschiedenen Resten substituiert sein können. Vorzugsweise können die Alkylreste ausgewählt sein aus der Gruppe bestehend aus Methyl, Ethyl, n-Propyl, iso-Propyl, n-Butyl, iso-Butyl, sec-Butyl, tert-Butyl, n-Pentyl, iso-Pentyl, neo-Pentyl und Hexyl. Besonders bevorzugte Alkylreste können ausgewählt werden aus der Gruppe bestehend aus Methyl, Ethyl, n-Propyl, iso-Propyl, n-Butyl, sec-Butyl, iso-Butyl und tert-Butyl.

Der Ausdruck "C₂₋₆-Alkenyl" umfasst im Sinne dieser Erfindung acyclische ungesättigte Kohlenwasserstoffreste mit 2, 3, 4, 5 oder 6 C-Atomen, die verzweigt oder geradkettig (unverzweigt) sowie unsubstituiert oder ein- oder mehrfach, beispielsweise 2-, 3-, 4- oder 5-fach, mit gleichen oder verschiedenen Resten substituiert sein können. Dabei weisen die Alkenylreste wenigstens eine C=C-Doppelbindung auf. Vorzugsweise können Alkenylreste ausgewählt sein aus der Gruppe bestehend aus Vinyl, Prop-1-enyl, Allyl, 2-Methylprop-1-enyl, But-1-enyl, But-2-enyl, But-3-enyl, But-1,3-dienyl, 2-Methylprop-1-enyl, But-2-en-2-yl, But-1-en-2-yl, Pentenyl und Hexenyl. Besonders bevorzugte Alkenylreste können ausgewählt werden aus der Gruppe bestehend aus Vinyl, Prop-1-enyl, Allyl, 2-Methylprop-1-enyl, But-1-enyl, But-2-enyl, But-3-enyl, But-1,3-dienyl, 2-Methylprop-1-enyl, But-2-en-2-yl und But-1-en-2-yl.

Im Sinne dieser Erfindung bedeutet der Ausdruck "C₃₋₈-Cycloalkyl" cyclische gesättigte Kohlenwasserstoffe mit 3, 4, 5, 6, 7 oder 8 Kohlenstoffatomen, die unsubstituiert oder an einem oder mehreren Ringgliedern einfach oder mehrfach, beispielsweise mit 2, 3, 4 oder 5, gleichen oder verschiedenen Resten substituiert sein können. Vorzugsweise kann C₃₋₈-Cycloalkyl ausgewählt sein aus der Gruppe bestehend aus Cyclopropyl, Cyclobutyl, Cyclopentyl, Cyclohexyl, Cycloheptyl und Cyclooctyl, insbesondere Cyclopropyl, Cyclobutyl, Cyclopentyl oder Cyclohexyl.

Der Ausdruck "Aryl" bedeutet im Sinne dieser Erfindung aromatische Kohlenwasserstoffe, insbesondere Phenyle und Naphthyle. Die Aryl-Reste können auch mit weiteren gesättigten, (partiell) ungesättigten oder aromatischen Ringsystemen kondensiert sein. Jeder Aryl-Rest kann unsubstituiert oder einfach oder mehrfach, beispielsweise 2-, 3-, 4- oder 5-fach, substituiert vorliegen, wobei die Aryl-Substituenten gleich oder verschieden und in jeder beliebigen und möglichen Position des Aryls sein können. Vorteilhafterweise kann Aryl ausgewählt sein aus der Gruppe bestehend aus Phenyl, 1-Naphthyl und 2-Naphthyl, welche jeweils unsubstituiert oder ein- oder mehrfach, beispielsweise mit 2, 3, 4 oder 5 Resten, substituiert sein können.

Der Ausdruck "Heteroaryl" steht im Sinne der vorliegenden Erfindung für einen 5-, 6-oder 7-gliedrigen zyklischen aromatischen Rest, der mindestens 1, ggf. auch 2, 3, 4 oder 5 Heteroatome, enthält, wobei die Heteroatome gleich oder verschieden sein können und das Heteroaryl unsubstituiert oder ein- oder mehrfach, beispielsweise 2-, 3-, 4- oder 5-fach, mit gleichen oder verschiedenen Resten substituiert sein kann. Die Substituenten können in jeder beliebigen und möglichen Position des Heteroaryls gebunden sein. Der Heterocyclus kann auch Teil eines bi- oder polyzyklischen, insbesondere eines mono-, bi- oder trizyklischen Systems sein, das dann insgesamt mehr als 7-gliedrig sein kann, vorzugsweise bis zu 14-gliedrig. Bevorzugte Heteroatome sind ausgewählt aus der Gruppe bestehend aus N, O und S. Vorzugsweise kann der Heteroarylrest ausgewählt werden aus der Gruppe bestehend aus Pyrrolyl, Indolyl, Furyl (Furanyl), Benzofuranyl, Thienyl (Thiophenyl), Benzothienyl, Benzothiadiazolyl, Benzothiazolyl, Benzotriazolyl, Benzodioxolanyl, Benzodioxanyl, Benzooxazolyl, Benzooxadiazolyl, Imidazothiazolyl, Dibenzofuranyl, Dibenzothienyl, Phtalazinyl, Pyrazolyl, Imidazolyl, Thiazolyl, Oxazolyl, Oxadiazolyl, Triazolyl, Tetrazolyl, Isoxazoyl, Pyridinyl (Pyridyl), Pyridazinyl, Pyrimidinyl (Pyrimidyl), Pyrazinyl, Pyranyl, Indazolyl, Purinyl, Indolizinyl, Chinolinyl, Isochinolinyl, Chinazolinyl, Chinoxalinyl, Carbazolyl, Phenazinyl, Phenothiazinyl und Oxadiazolyl, insbesondere aus der Gruppe bestehend aus Thienyl (Thiophenyl), Pyridinyl (Pyridyl), Pyrimidinyl, Thiazolyl, Imidazolyl, Oxazolyl, Chinazolinyl, Chinolinyl, Isochinolinyl und Oxadiazolyl, wobei die Bindung an die allgemeinen Struktur I über jedes beliebige und mögliche Ringglied des Heteroaryl-Restes erfolgen kann.

Besonders bevorzugt kann der Heteroarylrest ausgewählt werden aus der Gruppe bestehend aus Pyridinyl, Pyrimidinyl, Imidazoyl, Thienyl, Thiazolyl und Triazolyl.

Der Ausdruck "C₁₋₃-Alkylengruppe" bzw. "C₁₋₆-Alkylengruppe" umfasst im Sinne der vorliegenden Erfindung acyclische gesättigte Kohlenwasserstoffreste mit 1, 2 oder 3, bzw. mit 1, 2, 3, 4, 5 oder 6 C-Atomen, die verzweigt- oder geradkettig (unverzweigt) sowie unsubstituiert oder ein- oder mehrfach, beispielsweise 2-, 3-, 4- oder 5-fach, mit gleichen oder verschiedenen Resten substituiert sein können und die einen entsprechenden Rest mit der übergeordneten allgemeinen Struktur verknüpfen. Vorzugsweise können die Alkylen-Gruppen ausgewählt sein aus der Gruppe bestehend aus -CH₂-, -CH₂-CH₂-, -CH(CH₃)-, -CH₂-CH₂-CH₂-, -CH(CH₃)-CH₂-, -CH(CH₂CH₃)-, -CH₂-(CH₂)₂-CH₂-, -CH(CH₃)-CH₂-CH₂-, -CH₂-CH(CH₃)-CH₂-, -CH(CH₃)-CH(CH₃)-, -CH(CH₂CH₃)-CH₂-, -C(CH₃)₂-CH₂-, -CH(CH₂CH₂CH₃)-, -C(CH₃)(CH₂CH₃)-, -CH₂-(CH₂)₃-CH₂-, -CH(CH₃)-CH₂-CH₂-CH₂-, -CH₂-CH(CH₃)-CH₂-CH₂-, -CH(CH₃)-CH₂-CH(CH₃)-, -CH(CH₃)-CH(CH₃)-CH₂-, -C(CH₃)₂-CH₂-CH₂-, -CH₂-C(CH₃)₂-CH₂-, -CH(CH₂CH₃)-CH₂-CH₂-, -CH₂-CH(CH₂CH₃)-CH₂-, -C(CH₃)₂-CH(CH₃)-, -CH(CH₂CH₃)-CH(CH₃)-, -C(CH₃)(CH₂CH₃)-CH₂-, -CH(CH₂CH₂CH₃)-CH₂-, -C(CH₂CH₂CH₃)-CH₂-, -CH(CH₂CH₂CH₂CH₃)-, -C(CH₃)(CH₂CH₂CH₃)-, -C(CH₂CH₃)₂-und -CH₂-(CH₂)₄-CH₂-. Besonders bevorzugt können die Alkylen-Gruppen ausgewählt sein aus der Gruppe bestehend aus -CH₂-, -CH₂-CH₂- und -CH₂-CH₂-CH₂-.

Der Ausdruck "-(O)_{0/1}-C₁₋₆Alkylengruppe" umfasst im Sinne der vorliegenden Erfindung neben den oben beschriebenen C₁₋₆-Alkylengruppen zusätzlich solche Gruppen in denen diese Gruppen über ein Sauerstoffatom mit der übergeordneten Struktur verknüpft sind.

Der Ausdruck "C₂₋₆-Alkenylengruppe" umfasst im Sinne der vorliegenden Erfindung acyclische, einfach oder mehrfach, beispielsweise 2-, 3- oder 4-fach, ungesättigte Kohlenwasserstoffreste mit 2, 3, 4, 5 oder 6 C-Atomen, die verzweigt- oder geradkettig (unverzweigt) sowie unsubstituiert oder ein- oder mehrfach, beispielsweise 2-, 3-, 4- oder 5-fach, mit gleichen oder verschiedenen Resten substituiert sein können und die einen entsprechenden Rest mit der übergeordneten allgemeinen Struktur verknüpfen. Dabei weisen die Alkenylengruppen wenigstens eine C=C-Doppelbindung auf. Vorzugsweise können die Alkenylengruppen ausgewählt sein aus der Gruppe bestehend aus -CH=CH-, -CH=CH-CH₂-, -C(CH₃)=CH₂-, -CH=CH-CH₂-CH₂-, -CH₂-CH=CH-CH₂-, -CH=CH-CH=CH-, -C(CH₃)=CH-CH₂-, -CH=C(CH₃)-CH₂-, -C(CH₃)=C(CH₃)-, -C(CH₂CH₃)=CH-, -CH=CH-CH₂-CH₂-CH₂-, -CH₂-CH=CH₂-CH₂-CH₂-, -CH=CH=CH-CH₂-CH₂- und -CH=CH₂-CH-CH=CH₂-.

Der Ausdruck "C₂₋₆-Alkinylengruppe" umfasst im Sinne der Erfindung acyclische, einfach oder mehrfach, beispielsweise 2-, 3- oder 4-fach, ungesättigte Kohlenwasserstoffreste mit 2, 3, 4, 5 oder 6 C-Atomen, die verzweigt- oder geradkettig (unverzweigt) sowie unsubstituiert oder ein- oder mehrfach, beispielsweise 2-, 3-, 4- oder 5-fach, mit gleichen oder verschiedenen Resten substituiert sein können und die einen entsprechenden Rest mit der übergeordneten allgemeinen Struktur verknüpfen. Dabei weisen die Alkinylengruppen wenigstens eine C≡C-Dreifachbindung auf. Vorzugsweise können die Alkinylengruppen ausgewählt sein aus der Gruppe bestehend aus -C≡C-, -C≡C-CH₂-, -C≡C-CH₂-CH₂-, -C≡C-CH(CH₃)-, -CH₂-C≡C-CH₂-, -C≡C-C≡C-, -C=C-C (CH₃)₂-, -C≡C-CH₂-CH₂-CH₂-, -CH₂-C≡C-CH₂-CH₂-, -C≡C-C≡C-CH₂- und -C≡C-CH₂-C≡C-.

Der Ausdruck "über eine C₁₋₃-Alkylengruppe, eine C₁₋₆-Alkylengruppe, C₂₋₆-Alkenylengruppe oder C₂₋₆-Alkinylengruppe gebundenes Aryl oder Heteroaryl" bedeutet im Sinne der vorliegenden Erfindung, dass die C₁₋₆-Alkylengruppen, C₁₋₃-Alkylengruppen, C₂₋₆-Alkenylengruppen, C₂₋₆-Alkinylengruppen sowie Aryl bzw. Heteroaryl die oben definierten Bedeutungen haben und das Aryl bzw. Heteroaryl über eine C₁₋₃-Alkylengruppe, C₁₋₆-Alkylengruppe, C₂₋₆-Alkenylengruppe bzw. C₂₋₆-Alkinylengruppe an die übergeordnete allgemeine Struktur gebunden ist. Beispielhaft seien genannt Benzyl, Phenethyl und Phenylpropyl.

Der Ausdruck "über eine C₁₋₃-Alkylengruppe, C₁₋₆-Alkylengruppe, C₂₋₆-Alkenylengruppe oder C₂₋₆-Alkinylengruppe gebundenes C₃₋₈-Cycloalkyl und Heterocycloalkyl" bedeutet im Sinne der vorliegenden Erfindung, dass die C₁₋₃-Alkylengruppe, C₁₋₆-Alkylengruppe, C₂₋₆-Alkenylengruppe, C₂₋₆-Alkinylengruppe, C₃₋₈-Cycloalkyl und Heterocycloalkyl die oben definierten Bedeutungen haben und C₃₋₈-Cycloalkyl und Heterocycloalkyl über eine C₁₋₃-Alkylengruppe, C₁₋₆-Alkylengruppe, C₂-₆-Alkenylengruppe oder C₂₋₆-Alkinylengruppe an die übergeordnete allgemeine Struktur gebunden ist.

Im Zusammenhang mit "Alkyl", "Alkenyl", "Alkylen", "Alkenylen", "Alkinylen" und "Cycloalkyl" versteht man unter dem Begriff "substituiert" im Sinne dieser Erfindung die Substitution eines Wasserstoffrestes durch F, Cl, Br, I, CN, NH₂, NH-C₁₋₆-Alkyl, NH-C₁₋₆-Alkylen-OH, C₁₋₆-Alkyl, N(C₁₋₆-Alkyl)₂, N(C₁₋₆-Alkylen-OH)₂, NO₂, SH, S-C₁₋₆-Alkyl, S-Benzyl, O-C₁₋₆-Alkyl, OH, O-C₁₋₆-Alkylen-OH, =O, O-Benzyl, C(=O)C₁₋₆-Alkyl, CO₂H, CO₂-C₁₋₆-Alkyl, oder Benzyl, wobei unter mehrfach substituierten Resten solche Reste zu verstehen sind, die entweder an verschiedenen oder an gleichen Atomen mehrfach, beispielsweise zweifach oder dreifach, substituiert sind, beispielsweise dreifach am gleichen C-Atom wie im Falle von CF₃ oder CH₂CF₃ oder an verschiedenen Stellen wie im Falle von CH(Cl)-CH=CH-CHCl₂. Die Mehrfachsubstitution kann mit gleichen oder verschiedenen Substituenten erfolgen, wie beispielsweise im Falle von CH(OH)-CH=CH-CHCl₂. Insbesondere soll hier darunter die Substituion eines oder mehrerer Wasserstoffreste durch F, Cl, NH₂, OH, O-CF₃ oder O-C₁₋₆Alkyl, insbesondere Methoxy, verstanden werden.

In Bezug auf "Aryl" und "Heteroaryl" versteht man im Sinne dieser Erfindung unter "substituiert" die ein- oder mehrfache, beispielsweise 2-, 3-, 4- oder 5-fache, Substitution eines oder mehrerer Wasserstoffatome des entsprechenden Ringsystems durch F, Cl, Br, I, CN, NH₂, NH-C₁₋₆-Alkyl, NH-C₁₋₆-Alkylen-OH, N(C₁₋₆-Alkyl)₂, N(C₁₋₆-Alkylen-OH)₂, NH-Aryl¹, N(Aryl¹)₂, N(C₁₋₆-Alkyl)Aryl¹, Pyrrolinyl, Piperazinyl, Morpholinyl; (C₁₋₃-Alkylen)-Azetidinyl, -Pyrrolinyl oder -Piperidinyl, Azepanyl, Diazepanly; NO₂, SH, S-C₁₋₆-Alkyl, OH, O-C₁₋₆-Alkyl, O-C₁₋₆-Alkyl-OH, C(=O)C₁₋₆-Alkyl, NHSO₂C₁₋₆-Alkyl, NHCOC₁₋₆-Alkyl, CO₂H, CH₂SO₂-Phenyl, CO₂-C₁₋₆-Alkyl, OCF₃, CF₃, -O-CH₂-O-, -O-CH₂-CH₂-O-, -O-C(CH₃)₂-CH₂-, unsubstituiertes C₁₋₆-Alkyl; Pyrrolidinyl, Imdazolyl, Piperidinyl, Benzyloxy, Phenoxy, Phenyl, Naphthyl, Pyridinyl, Thiazolinyl, -C₁₋₃-Alkylen-Aryl¹, Benzyl, Thienyl, Furyl, wobei Aryl¹ für Phenyl, Thiazolyl, Thienyl oder Pyridinyl steht, an einem oder verschiedenen Atomen, wobei die vorstehend genannten Substituenten - sofern nicht anders angegeben - ggf. ihrerseits mit den genannten Substituenten substituiert sein können. Die Mehrfachsubstitution von Aryl und Heteroaryl kann mit gleichen oder verschiedenen Substituenten erfolgen. Bevorzugte Substituenten für Aryl und Heteroaryl können ausgewählt werden aus der Gruppe bestehend aus -O-C₁₋₃-Alkyl, unsubstituiertem C₁₋₆-Alkyl, F, Cl, Br, I, CN, CF₃, OCF₃, OH, SH, -CH₂-Azetidinyl, - CH₂-Pyrrolidinyl, -CH₂-Piperidinyl, -CH₂-Piperazinyl, -CH₂-Morpholinyl, Phenyl, Naphthyl, Thiazolyl, Thienyl und Pyridinyl, insbesondere aus der Gruppe bestehend aus F, Cl, CN, CF₃, CH₃; OCH₃, OCF₃, und -CH₂-Azetidinyl.

In den chemischen Strukturformeln, die zur Beschreibung der erfindungsgemäßen Verbindungen hier verwendet werden, wird zur Beschreibung eines oder mehrerer Substitutionsmuster auch das Symbol "R^{a}" verwendet, wobei diese Gruppe im Gegensatz zur Darstellung einer Bindung an ein bestimmtes Atom nicht an ein bestimmtes Atom innerhalb der chemischen Strukturformel gebunden ist (R^{a} steht hier beispielhaft für einen Substituenten R mit einer durch die Variable "a" dargestellten Nummerierung).

Dies sei beispielhaft anhand der Gruppe 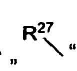 aus der oben gezeigten allgemeinen Formel III erläutert: Die Definition für R²⁷ besagt, dass R²⁷ für 0 bis 4 Substituenten stehen kann. R²⁷ kann also abwesend sein oder es können 1, 2, 3 oder 4 der C-gebundenen Wasserstoffatome innerhalb der durch die allgemeine Formel III dargestellten Teilstruktur durch einen in der Definition für den Rest R²⁷ vorgesehenen Substituenten ersetzt sein, wobei die jeweiligen Substituenten unabhängig voneinander ausgewählt sein können, also auch unterschiedliche Bedeutungen haben können, und C-gebundene Wasserstoffatome an einem oder mehreren C-Atomen ersetzen können. Wie in der Definition von R²⁷ erläutert, können auch jeweils zwei der Substituenten R²⁷ zusammen eine C₁₋₃Alkylenbrücke oder ein anelliertes Aryl oder Heteroaryl (auch ankondensiertes Aryl oder Heteroaryl oder anellierte/ankondesierte Aryl- oder Heteroarylgruppe genannt) darstellen, so dass R²⁷ in der allgemeinen Formel III auch die nachfolgend beispielhaft gezeigten Bedeutungen hat, in denen R²⁷ für zwei Substituenten an jeweils unterschiedlichen C-Atomen steht, die in diesen lediglich beispielhaften Fällen zusammen entweder eine C₂-Alkylenbrücke oder eine anellierte Benzogruppe bilden, und im zweiten Beispiel die Variable u für 1 steht: 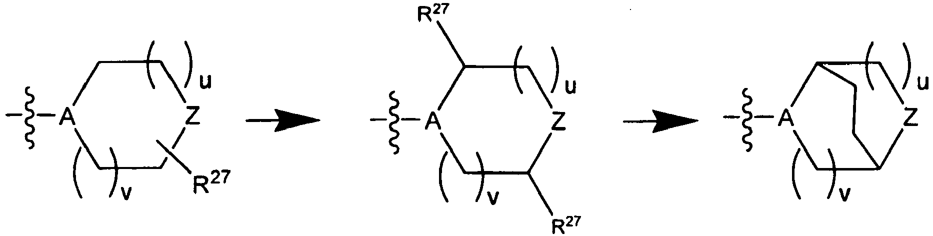 oder

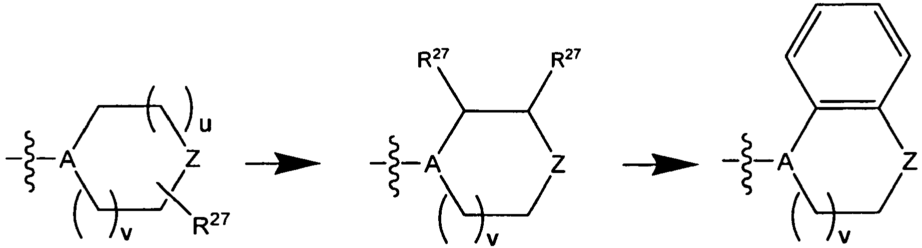

Im Rahmen der vorliegenden Erfindung bezeichnet das in Formeln verwendete Symbol 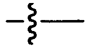 eine Verknüpfung eines entsprechenden Restes an die jeweilige übergeordnete allgemeine Struktur.

Der Fachmann versteht, dass gleiche Reste, die zur Definition unterschiedlicher Substituenten verwendet werden, jeweils unabhänging voneinander sind.

Unter dem Begriff "physiologisch verträgliches Salz" versteht man im Sinne dieser Erfindung vorzugsweise Salze der erfindungsgemäßen Verbindungen mit anorganischen oder organischen Säuren, die physiologisch - insbesondere bei Anwendung am Menschen und/ oder Säugetier - verträglich sind. Beispiele für geeignete Säuren sind Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Maleinsäure, Milchsäure, Zitronensäure, Glutaminsäure, 1,1-Dioxo-1,2-dihydro1λ⁶-benzo[d]isothiazol-3-on (Saccharinsäure), Monomethylsebacinsäure, 5-Oxo-prolin, Hexan-1-sulfonsäure, Nicotinsäure, 2-, 3-oder 4-Aminobenzoesäure, 2,4,6-Trimethyl-benzoesäure, α-Liponsäure, Acetylglycin, Hippursäure, Phosphorsäure und/oder Asparaginsäure. Besonders bevorzugt sind die Salze der Salzsäure (Hydrochloride) sowie der Zitronensäure (Citrate).

In bevorzugten Ausführungsformen der durch die allgemeine Formel I dargestellten erfindungsgemäßen Verbindungen stehen W¹ und W³ für N und W² steht für CR⁶⁰. Alternativ stehen W¹ und W² für N und W³ steht für CR⁶⁰. In einer alternativen Variante der erfindungsgemäßen Verbindungen stehen W¹, W² und W³ alle für N. R⁶⁰ kann insbesondere für H oder C₁₋₆Alkyl, insbesondere Methyl, stehen.

In ebenfalls bevorzugten Ausführungsformen der erfindungsgemäßen Verbindungen steht V für O. In alternativen, bevorzugten Ausführungsformen steht V für NR^{6c}. R^{6c} kann insbesondere für H, Methyl oder Cyclopropyl stehen.

In weiteren bevorzugten Ausführungsformen der erfindungsgemäßen Verbindungen steht V für CR^{6a}R^{6b}. R^{6a} und R^{6b} kann insbesondere für H stehen.

R¹ steht in den erfindungsgemäßen Verbindungen vorzugsweise für Phenyl, Naphthyl, Indolyl, Benzofuranyl, Benzothiophenyl (Benzothienyl); Benzooxazolyl, Benzooxadiazolyl, Pyrrolyl, Furanyl, Thienyl, Pyridinyl, Pyridazinyl, Pyrimidinyl, Pyrazinyl, Imidazothiazolyl, Carbazolyl, Dibenzofuranyl, Dibenzothiophenyl (Dibenzothienyl), Chinolinyl, Isochinolinyl, für eine über eine C₁₋₃-Alkylengruppe gebundenes Phenyl oder Naphthyl, oder CH(Phenyl)₂, bevorzugt für Phenyl, Naphthyl, Benzothiophenyl, Chinolinyl, Isochinolinyl oder Thienyl, besonders bevorzugt für Phenyl, Naphthyl oder Benzothiophenyl (Benzothienyl), jeweils unsubstituiert oder einfach oder mehrfach, gleich oder verschieden, substituiert, wobei die Substituenten insbesondere ausgewählt sind aus -O-C₁₋₃-Alkyl, C₁₋₆-Alkyl, F, Cl, Br, CF₃, OCF₃, OH, Phenyl, Naphthyl, Thienyl, Thiazolyl und Pyridinyl, und wobei die vorstehend genannten Alkylengruppen jeweils unsubstituiert oder einfach oder mehrfach, gleich oder verschieden, substituiert sind, wobei die Substituenten unabhängig voneinander ausgewählt sind aus der Gruppe bestehend aus -O-C₁₋₃-Alkyl, F, Cl, Br, CF₃, -OCF₃, OH, Phenyl, Phenoxy, Naphthyl, Furyl, Thienyl, und Pyridinyl.

Der Rest R¹ kann insbesondere für Phenyl oder Naphthyl stehen, wobei das Phenyl oder Naphthyl unsubstituiert oder einfach oder mehrfach, beispielsweise 2-, 3-, 4-oder 5-fach, mit gleichen oder verschiedenen Resten ausgewählt aus Methyl, Methoxy, CF₃, OCF₃, F und Cl substituiert ist.

In ebenfalls bevorzugten Ausführungsformen der erfindungsgemäßen Verbindungen ist der Rest R¹ ausgewählt aus 4-Methoxy-2,3,6-trimethylphenyl, 4-Methoxy-2,6-dimethylphenyl, 4-Methoxy-2,3,5-trimethylphenyl, 2,4,6-Trimethylphenyl, 1,3-Dichloro-5-(trifluormethyl)phenyl, 2-Chlor-6-methylphenyl, 2,4,6-Trichlorphenyl, 2-Chlor-6-(trifluormethyl)phenyl, 2,6-Dichlor-4-methoxyphenyl, 2,6-Dichlor-4-(trifluormethyl)phenyl, 2-Methyl-1-naphthyl, 2-Chlor-1-naphthyl, 2-Fluor-1-naphthyl, 6-Methoxy-2-naphthyl, 2-Chlor-4-(trifluormethoxy)phenyl, 4-Chlor-2,5-dimethylphenyl, 2,6-Dichlor-3-methylphenyl, 2,6-Dichlorphenyl, 2,3-Dichlorphenyl, 3,4-Dichlorphenyl, 2,4-Dichlorphenyl, 2-(Trifluormethyl)phenyl, 3-(Trifluormethyl)phenyl, 4-(Trifluormethyl)phenyl, 1-Naphthyl und 2-Naphthyl; insbesondere 4-Methoxy-2,6-dimethylphenyl, 2-Chlor-6-methylphenyl und 2-(Trifluormethyl)phenyl.

In ebenfalls bevorzugten Ausführungsformen der erfindungsgemäßen Verbindungen gemäß der allgemeinen Formel I stellt die die nachfolgend dargestellte Teilstruktur Ac I 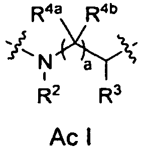 eine Gruppe gemäß allgemeinen Formel Ac I.a dar 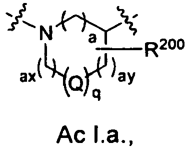 wobei
a für 0 oder 1 steht;
ax für 0, 1, 2 oder 3 steht;
ay für 0, 1 oder 2;
q für 0 oder 1 steht;
mit der Maßgabe, dass a + ax + ay + q ≥ 2;
Q für CH₂, NR⁵⁰, O, S, S=O oder S(=O)₂ steht, und
R²⁰⁰ für 0-4 Substituenten steht, die unabhängig voneinander ausgesucht sind aus aus F, Cl, -CF₃, =O, -O-CF₃, -OH, -O-C₁₋₆-Alkyl oder C₁₋₆-Alkyl, insbesondere für F oder CF₃ steht oder zwei der Reste R²⁰⁰ zusammen ein anelliertes Aryl- oder Heteroaryl, insbesondere eine Benzogruppe, darstellen. Falls die Struktur des N-haltigen Heterocyclus dies zulässt, kann R²⁰⁰ somit auch für zwei an den Heterocyclus anellierte Aryle, insbesondere Benzogruppen, stehen. In bestimmten Ausführungsformen steht R²⁰⁰ für 0 Substituenten, ist also abwesend.

Insbesondere kann die Teilstruktur Ac I für eine der nachfolgend aufgeführten Gruppen stehen: 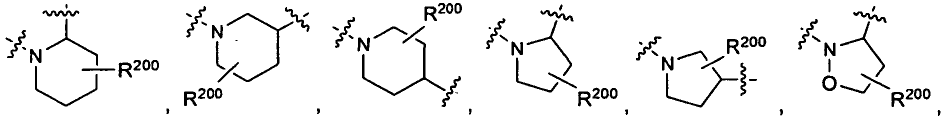 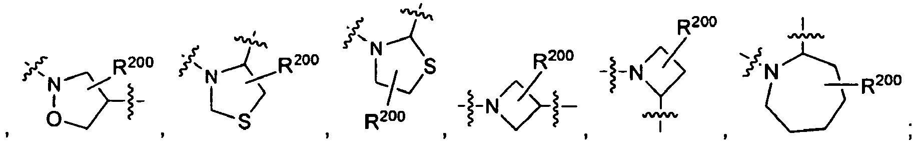 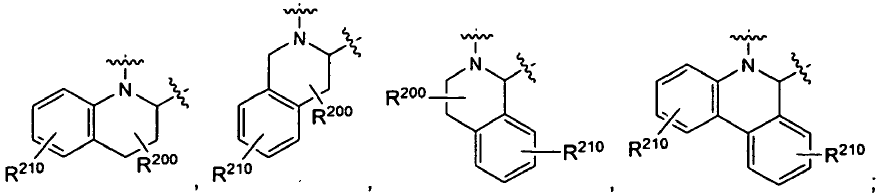 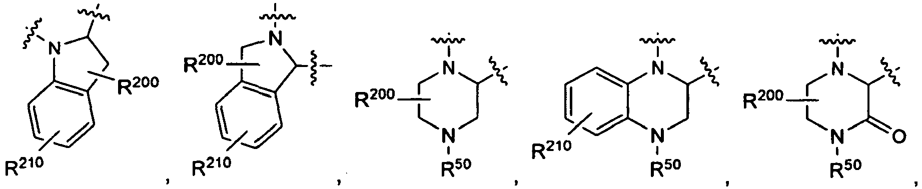 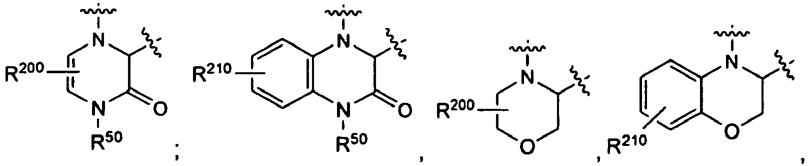 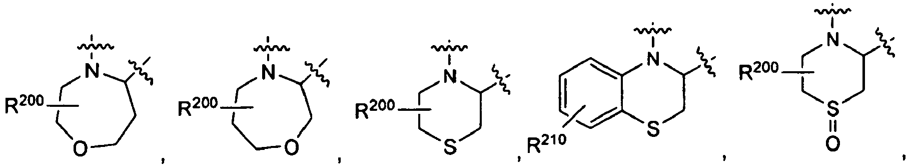 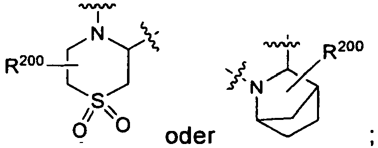 worin
R²¹⁰ für 0-4 Substituenten steht, die unabhängig voneinander ausgesucht sind aus F, Cl, -CF₃, =O, -O-CF₃, -OH, -O-C₁₋₆-Alkyl oder C₁₋₆-Alkyl, insbesondere für F oder CF₃ steht, und/oder zwei benachbarte Reste R²⁰⁰ bilden zusammen ein anelliertes Aryl oder Heteroaryl, insbesondere eine Benzogruppe;
R²¹⁰ für 0-4 Substituenten steht, die unabhängig voneinander ausgesucht sind aus -O-C₁₋₃-Alkyl, C₁₋₆-Alkyl, F, Cl, Br, I, CF₃, -OCF₃, OH, SH, Phenyl, Naphthyl, Furyl, Thienyl und Pyridinyl, insbesondere für Methyl, Methoxy, CF₃, F, Cl, Br und -OCF₃.

In bestimmten Ausführungsformen der erfindungsgemäßen Verbindungen stehen R²⁰⁰ und/oder R²¹⁰ für 0 Substituenten; sind also jeweils abwesend.

Insbesondere kann die Teilstruktur Ac I für eine der nachfolgend aufgeführten Gruppen stehen: 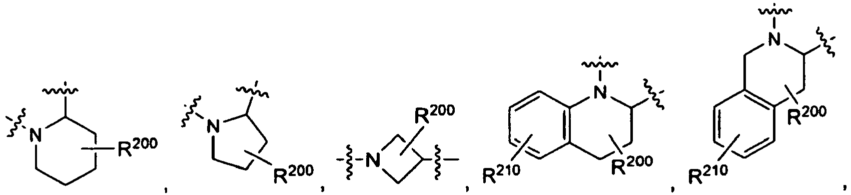 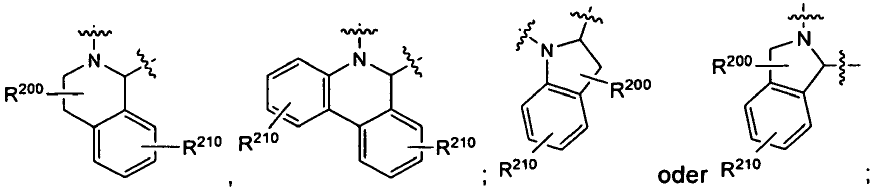 wobei die Reste R²⁰⁰ und R²¹⁰ vorzugsweise die oben beschriebenen Bedeutungen annehmen.

In einer ebenfalls bevorzugten Ausführungsform der erfindungsgemäßen Verbindungen steht R² vorzugsweise für H, C₁₋₆-Alkyl, C₃₋₆-Cycloalkyl, Aryl oder ein über eine C₁₋₃-Alkylengruppe gebundenes C₃₋₆-Cycloalkyl oder Aryl; jeweils unsubstituiert oder einfach oder mehrfach substituiert ist mit gleichen oder verschiedenen Resten. Insbesondere kann R² für H, Methyl, Ethyl, n-Propyl, i-Propyl, n-Butyl, i-Butyl, t-Butyl, Cyclopropyl stehen, jeweils unsubstituiert oder einfach oder mehrfach substituiert mit gleichen oder verschiedenen Resten ausgesucht aus F, Cl, OH, OCH₃ oder OCF₃. Oder R² steht für Phenyl oder Pyridinyl, dass unsubstituiert oder einfach oder mehrfach substituiert ist mit gleichen oder verschiedenen Resten ausgesucht aus C₁₋₆-Alkyl, C₁₋₆-Alkyl-O-, F, Cl, Br, I, CF₃, OCF₃, OH und SH, insbesondere aus Methyl, Methoxy, F, Cl, CF₃, oder OCF₃, wobei Phenyl und Pyridinly über eine C₁₋₃-Alkylengruppe gebunden sein können.

In einer ebenfalls bevorzugten Ausführungsform der erfindungsgemäßen Verbindungen steht R³ vorzugsweise für H, F, Cl, -CF₃, -OH, -O-C₁₋₆-Alkyl, C₁₋₆-Alkyl, Aryl; oder ein über eine C₁₋₃-Alkylengruppe gebundenes Aryl, jeweils unsubstituiert oder einfach oder mehrfach mit gleichen oder verschiedenen Resten substituiert. Insbesondere kann R³ für H, F, n-Propyl, i-Propyl, n-Butyl, i-Butyl, t-Butyl, Cyclopropyl, Methoxy oder Ethoxy stehen, jeweils unsubstituiert oder einfach oder mehrfach substituiert mit gleichen oder verschiedenen Resten ausgesucht aus F, Cl, OH, OCH₃ oder OCF₃. Oder R³ steht für Phenyl oder Benzyl, jeweils unsubstituiert oder einfach oder mehrfach substitutiert mit gleichen oder verschiedenen Resten ausgesucht aus C₁₋₆-Alkyl, C₁₋₆-Alkyl-O-, F, Cl, Br, I, CF₃, OCF₃, OH und SH, insbesondere aus Methyl, Methoxy, F, Cl, CF₃, oder OCF₃.

Ebenfalls bevorzugte Ausführungsformen der erfindungsgemäßen Verbindungen sind solche, in denen R^{4a}, R^{4b}, R^{5a}, R^{5b}, R^{6a}, R^{6b}, R³⁶, R^{37a}, R^{37b}, R^{44a} und R^{44b}, jeweils unabhängig voneinander, ausgewählt sind aus H; F; Cl; OH; =O; O-C₁₋₄-Alkyl; -OCF₃, C₁₋₄-Alkyl; -CF₃ C₃₋₆-Cycloalkyl; Aryl oder Heteroaryl; über eine C₁₋₆-Alkylengruppe gebundenes C₃₋₆-Cycloalkyl, Aryl oder Heteroary.

In ebenfalls bevorzugten Ausführungsformen der erfindungsgemäßen Verbindungen stehen R^{4a}, R^{4b}, R^{5a}, R^{5b}, R^{6a} und/oder R^{6b}, jeweils unabhängig voneinander, für H, F, Cl, -CF₃, OH, OCF₃, oder 0-C₁₋₆-Alkyl, insbesondere für H oder F, insbesonder H.

In bevorzugten Ausführungsformen der erfindungsgemäßen Verbindungen stehen R^{4a} und R^{4b}, unabhängig voneinander, für H, F, Cl, Br, I, -CF₃, O-CF₃, OH, SH, O-C₁₋₆-Alkyl, C₁₋₆-Alkyl, C₃₋₈Cycloalkyl, Aryl, oder Heteroaryl; oder für ein über eine C₁₋₆-Alkylengruppe oder C₂₋₆-Alkenylengruppe gebundenes C₃₋₈Cycloalkyl, Aryl oder Heteroaryl.

In ebenfalls bevorzugten Ausführungsformen der erfindungsgemäßen Verbindungen bilden R^{4a} und R^{4b} gemeinsam mit dem sie verbindenden C-Atom einen gesättigtes C₃₋₆-Cycloalkyl, insbesondere Cyclopropyl oder Cyclobutyl. Diese können unsubstituiert oder an einem oder mehreren, beispielsweise 1, 2, 3 oder 4, ihrer Kohlenstoff-Ringglieder mit einem oder mehreren, beispielsweise 1, 2, 3 oder 4, Substituenten unabhängig voneinander ausgewählt aus der Gruppe bestehend aus F, CF₃, C₁₋₆-Alkyl, O-C₁₋₆-Alkyl, OH, OCF₃, Aryl und Heteroaryl substituiert sein.

In weiteren bevorzugten Ausführungsformen stellen R^{4a} und R^{4b} jeweils unabhängig voneinander H oder CH₃ dar.

Ebenfalls bevorzugte Ausführungsformen der erfindungsgemäßen Verbindungen sind solche, in denen die Bedingung a + b = 1, insbesondere die Bedingung a = 0, b = 1 oder a = 1, b = 0 erfüllt ist. In ebenfalls bevorzugten Varianten dieser Verbindungen stehen R^{4a}, R^{4b}, R^{5a}, R^{5b}, R^{6a} und/oder R^{6b}, insofern vorhanden, unabhängig voneinander für H oder F, insbesondere für H.

In weiteren bevorzugten Ausführungsformen der erfindungsgemäßen Verbindungen steht R⁷ vorzugsweise für H oder C₁₋₆-Alkyl, jeweils unsubsitituiert oder einfach oder mehrfach mit gleichen oder verschiedenen Substituenten substituiert, insbesondere für H, CF₃ oder Methyl.

In ebenfalls bevorzugten Ausführungsformen der erfindungsgemäßen Verbindungen gemäß der allgemeinen Formel I stellt die die nachfolgend dargestellte Teilstruktur Ac II

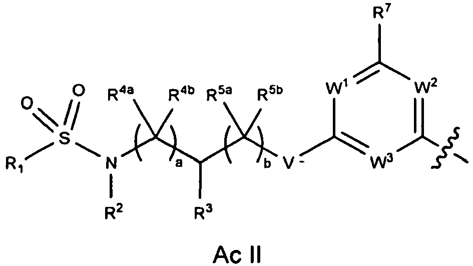

Eine der folgenden Teilstrukturen Ac II.a bis Ac.II.h dar, worin die jeweiligen Reste, Variabeln und Indizes die oben beschriebenen Bedeutungen haben:

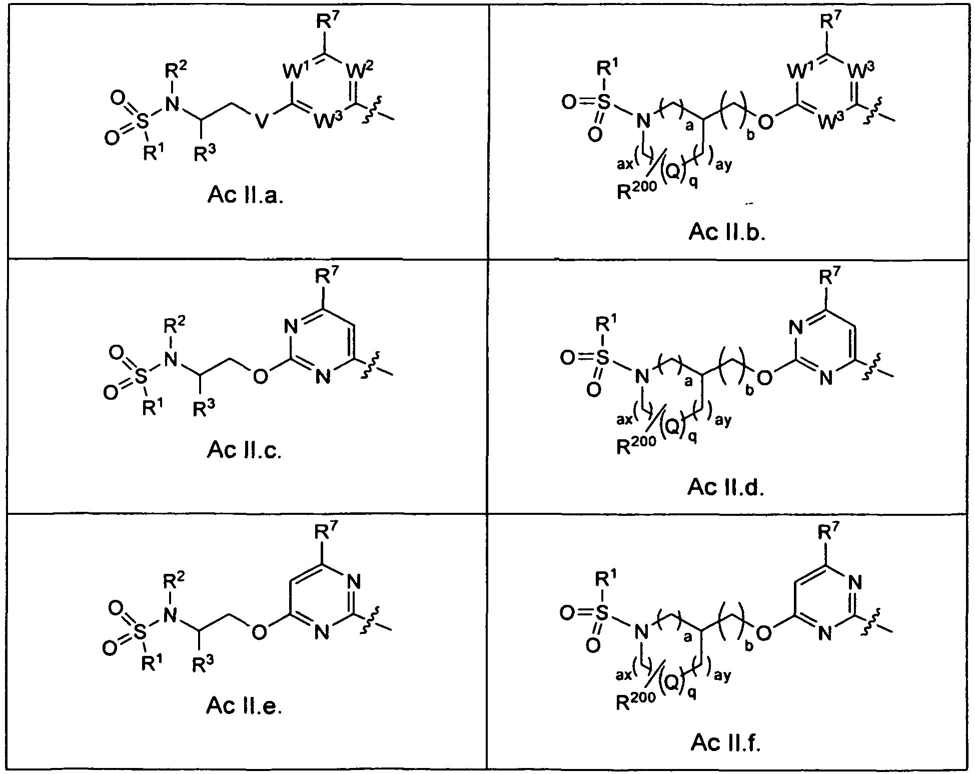

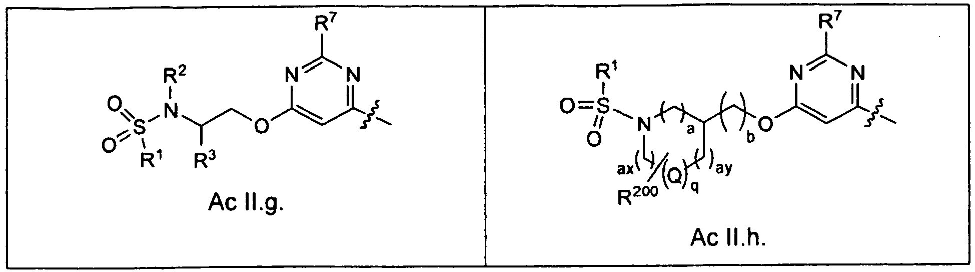

Ebenfalls bevorzugt sind Ausführungsformen der erfindungsgemäßen Verbindungen, in denen die die oben gezeigte Teilstruktur Ac II eine Struktur gemäß den nachfolgend gezeigten Formeln Ac II,i. und Ac II.j. annimmt.

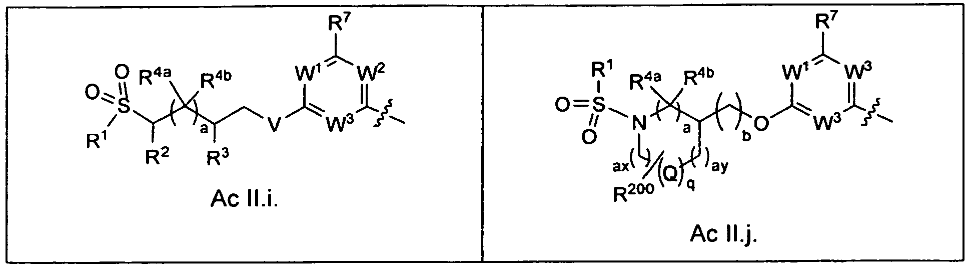

Auch in diesen Formeln haben die jeweiligen Reste, Variabel und Indizes die oben beschriebenen Bedeutungen.

Ebenfalls bevorzugte Ausführungsformen der erfindungsgemäßen Verbindungen sind solche, in denen R⁸ vorzugsweise für H; C₁₋₆-Alkyl; insbesondere Methyl, Ethyl, n-Propyl, iso-Propyl, n-Butyl, iso-Butyl, sec-Butyl, tert-Butyl; Cyclopropyl, Cyclobutyl, Cyclopentyl, Cyclohexyl, -CH₂CF₃, Phenyl, Benzyl, Phenylethyl, Phenylpropyl, oder über eine C₁₋₃-Alkylengruppe gebundenes Cyclopropyl, Cyclobutyl, Cyclopentyl, Cyclohexyl, jeweils unsubstituiert unsubsitituiert oder einfach oder mehrfach mit gleichen oder verschiedenen Substituenten substituiert, steht. Insbesondere kann R₈ für H, Methyl, Ethyl, iso-Propyl oder Cyclopropyl stehen.

Ebenfalls bevorzugte Ausführungsformen der erfindungsgemäßen Verbindungen sind solche, in denen R^{9a} und R^{9b} jeweils unabhängig voneinander für H; F; Methyl; Ethyl, iso-Propyl, CF₃, Methoxy; Cyclopropyl; Phenyl; Benzyl, Phenylethyl oder ein über eine C₁₋₃-Alkylengruppe gebundenes Cycloalkyl oder -CF₃, jeweils unsubstituiert unsubsitituiert oder einfach oder mehrfach mit gleichen oder verschiedenen Substituenten substituiert, steht. Insbesondere stehen R^{9a} und R^{9b} für H.

Ebenfalls bevorzugte Ausführungsformen der erfindungsgemäßen Verbindungen sind solche, in denen die oben beschreibene allgemeine Formel II die folgende Teilstruktur Ila annimmt:

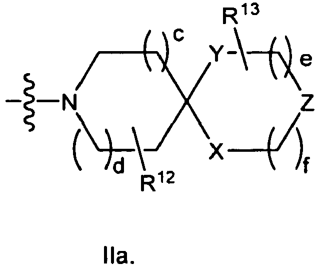

Ebenfalls bevorzugte Ausführungsformen der erfindungsgemäßen Verbindungen sind solche, in denen die oben beschriebende allgemeine Formel III eine der folgenden Teilstrukturen IIIa oder IIIb annimmt:

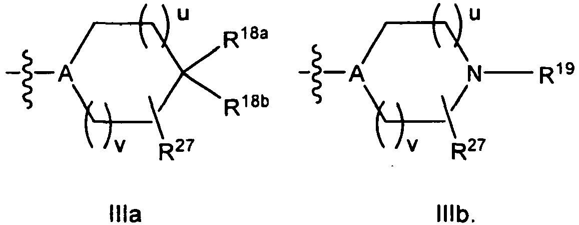

Ebenfalls bevorzugte Ausführungsformen der erfindungsgemäßen Verbindungen sind solche, in denen die Teilstruktur gemäß der oben gezeigten Formel IIa die folgende Teilstruktur IIb annimmt: 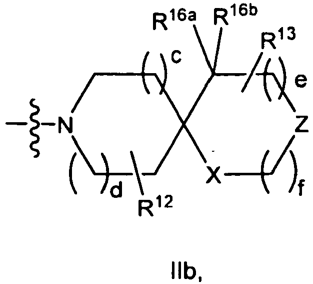 wobei in bestimmten Ausführungsformen dieser erfindungsgemäßen Verbindungen R⁸ für H, C₁₋₆-Alkyl oder C₃₋₆-Cycloalkyl steht, jeweils unsubstituiert oder einfach oder mehrfach substituiert mit gleichen oder verschiedenen Resten, und R^{9a} und R^{9b} jeweils für H stehen.

Ebenfalls bevorzugte Ausführungsformen der erfindungsgemäßen Verbindungen sind solche Verbindungen, in denen die Teilstrukturen gemäß den oben gezeigten Formeln IIIa und IIIb eine der folgenden Teilstrukturen IIIc, IIId oder IIIe annehmen:

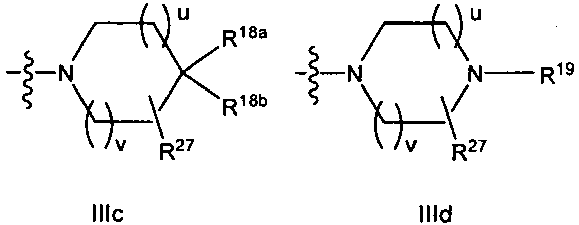

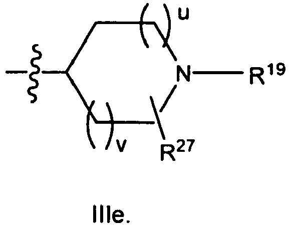

In bestimmten Ausführungsformen dieser erfindungsgemäßen Verbindungen stehen s und t jeweils für 0.

Ebenfalls bevorzugte Ausführungsformen der erfindungsgemäßen Verbindungen sind solche, in denen die Teilstrukturen gemäß den oben gezeigten Formeln IIIa und IIIb eine der oben gezeigten Teilstrukturen IIIc oder IIId annehmen und zwei der Substituenten R²⁷ zusammen eine C₁₋₃-Alkylenbrücke darstellen, so dass der in der Teilstruktur IIIc oder IIId dargestellte Cyclus eine bicyclisch verbrückte Form annimmt. In bestimmten Ausführungsformen dieser Verbindungen sind s und t jeweils = 0.

Ebenfalls bevorzugte Ausführungsformen der erfindungsgemäßen Verbindungen sind solche, in denen die Teilstrukturen gemäß den oben gezeigten Formeln IIIa und IIIb eine der ebenfalls oben gezeigten Teilstrukturen IIIc oder IIIe annehmen, s für 1 und t für 1, 2 oder 3 stehen. In bestimmten Ausführungsformen dieser erfindungsgemäßen Verbindungen steht R⁸ für H, C₁₋₆-Alkyl, C₃₋₆-Cycloalkyl, jeweils unsubstituiert oder einfach oder mehrfach substituiert. In weiteren bestimmten Ausführungsformen dieser erfindungsgemäßen Verbindungen stehen R^{9a} und R^{9b} für H.

Weitere bevorzugte Ausführungsformen der erfindungsgemäßen Verbindungen sind solche, in denen die Teilstruktur gemäß der oben gezeigten Formel IIb die folgende Teilstruktur IIc annimmt: 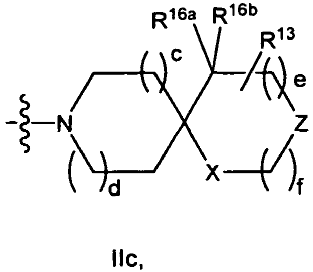 wobei in bestimmten Ausführungsformen dieser Verbindungen s und t jeweils 0 bedeuten.

In weiteren bevorzugten Ausführungsformen der erfindungsgemäßen Verbindungen nehmen die oben gezeigten Teilstrukturen gemäß den Formeln IIIc oder IIId eine der folgenden Teilstrukturen IIIf oder IIIg an, 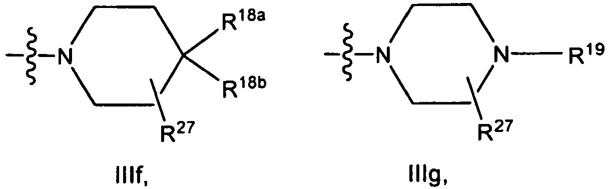 worin in bestimmten Ausführungsformen dieser Verbindungen R²⁷ für H oder Methyl steht und/oder zwei der Substituenten R²⁷ ein anelliertes Aryl oder Heteroaryl, insbesondere eine Benzogruppe, bilden.

Weiterhin sind bevorzugte Ausführungsformen der erfindungsgemäßen Verbindungen solche Verbindungen, in den die oben gezeigten Teilstrukturen IIIc oder IIId einen der folgenden Reste A bis H, insbesondere G und H, darstellen,

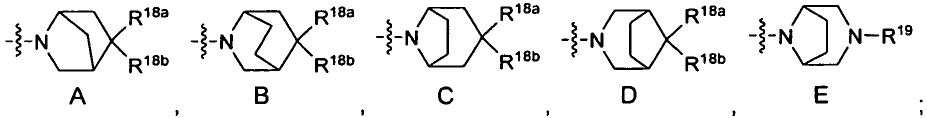

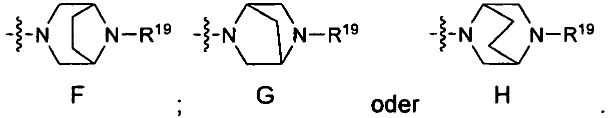

Der Fachmann versteht, dass die für die Reste A bis H gewählte Darstellung alle jeweilig möglichen Stereoisomere dieser Reste umfasst.

Weitere bevorzugte Ausführungsformen der erfindungsgemäßen Verbindungen sind solche Verbindungen, in denen die oben gezeigten Teilstrukturen IIIc oder IIIe eine Gruppe gemäß einer der Formeln IIIh oder IIIi darstellen, 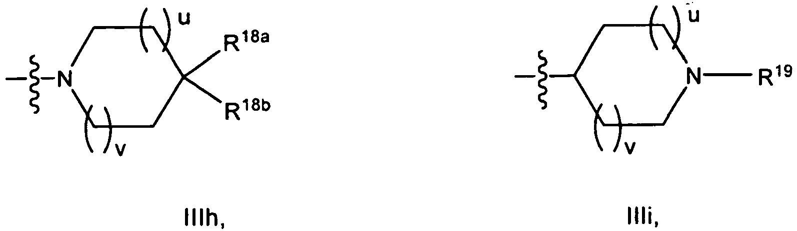 und R^{9a} und R^{9b} jeweils für H stehen. In bestimmten Ausführungsformen dieser Verbindungen stehen u und v jeweils unabhängig voneinander für 0 oder 1. Insbesondere stehen u und v beide für 1.

Weitere bevorzugte Ausführungsformen der erfindungsgemäßen Verbindungen sind solche Verbindungen, in denen in der oben gezeigten Teilstruktur IIc die Reste R^{16a} und R^{16b} jeweils für H stehen oder zusammen =O bilden; R¹³ für H, Aryl oder Heteroaryl steht und/oder zwei der Substituenten R¹³ zusammen =O bilden und/oder zwei benachbarte Substituenten R¹³ zusammen ein anelliertes Aryl oder Heteroaryl, insbesondere eine Benzogruppe, bilden, jeweils unsubstituiert oder einfach oder mehrfach substituiert mit gleichen oder verschiedenen Substituenten.

Weitere bevorzugte Ausführungsformen der erfindungsgemäßen Verbindungen sind solche Verbindungen, in denen in den oben gezeigten Teilstrukturen gemäß den Formeln IIIf oder IIIg:
R^{18a} für H; C₁₋₆-Alkyl; C₃₋₈-Cycloalkyl, -NH(C₁₋₆Alkyl), -N(C₁₋₆Alkyl)₂, Phenyl, Pyridinyl, Pyrimidinyl, Imidazolyl, Thiazolyl, Triazolyl oder Thienyl, jeweils unsubstituiert oder einfach oder mehrfach substituiert; über eine -(O)₀₋₁-C₁₋₆-Alkylengruppe gebundenes Phenyl, Pyridinyl, Pyrimidinyl, Imidazolyl, Thiazolyl, Triazolyl oder Thienyl, jeweils unsubstituiert oder einfach oder mehrfach substituiert, steht; oder
R^{18a} für einen Rest gemäß der allgemeinen Formel VIIa 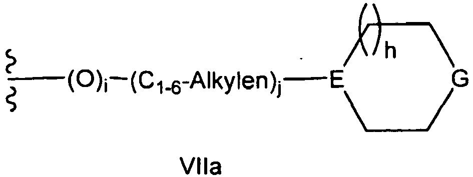
steht, worin
- i: für 0 oder 1 steht; -
- j: für 0 oder 1 steht;
- h: für 0 oder 1 steht;
- E: für N oder CH steht; mit der Maßgabe, dass wenn i für 1 und j für 0 steht, E für CH steht;
- G: für CR^{37a}R^{37b} oder NR³⁸ steht;
wobei R^{37a} und R^{37b} unabhängig voneinander für H; F oder C₁₋₆-Alkyl stehen;
R³⁸ für H; C₁₋₆-Alkyl, C₃₋₆-Cycloalkyl oder Pyridyl, insbesondere Pyridin-3-yl oder Pyridin-4-yl steht; und
R^{18b} für H; OH; C₁₋₆-Alkyl; Phenyl, Pyridinyl, Pyrimidinyl, Imidazolyl, Thiazolyl, Triazolyl oder Thienyl, jeweils unsubstituiert oder einfach oder mehrfach substituiert; über eine C₁₋₆-Alkylengruppe gebundenes Phenyl, Pyridinyl, Pyrimidinyl, Imidazolyl, Thiazolyl, Triazolyl oder Thienyl, O-Phenyl, oder O-Pyridyl, jeweils unsubstituiert oder einfach oder mehrfach substituiert mit gleichen oder verschiedenen Substituenten; über C₁₋₆Alkylen-NH(C=O) verbrücktes Phenyl, Pyridinyl, Pyrimidinyl, Imidazolyl, Thiazolyl, Triazolyl oder Thienyl, jeweils unsubstituiert oder einfach oder mehrfach substituiert mit gleichen oder verschiedenen Substituenten, steht;
R¹⁹ für H; C₁₋₆-Alkyl; C₃₋₈-Cycloalkyl, oder für über (C=O)₀₋₁ verbundenes C₁₋₆-Alkyl; Phenyl, Pyridinyl, Pyrimidinyl, Imidazolyl, Thiazolyl, Triazolyl oder Thienyl; jeweils unsubstituiert oder einfach oder mehrfach substituiert mit gleichen oder verschiedenen Substituenten; über eine C₁₋₆-Alkylengruppe gebundenes Phenyl, Pyridinyl, Pyrimidinyl, Imidazolyl, Thiazolyl, Triazolyl oder Thienyl, jeweils unsubstituiert oder einfach oder mehrfach substituiert mit gleichen oder verschiedenen Substituenten, steht;
oder R¹⁹ für den Rest gemäß der allgemeinen Formel VIIIa 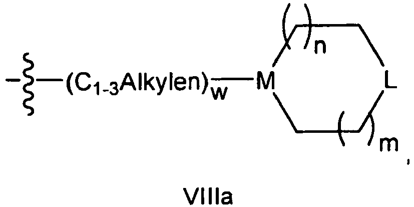 steht, worin
- w: für 0 oder 1 steht;
- n: für 0 oder 1 steht;
- m: für 0 oder 1 steht;
- M: für CH oder N steht, mit der Maßgabe, dass wenn w für 0 steht, M für CH steht;
- L: für CR^{44a}R^{44b} oder NR⁴⁵ steht;
wobei R^{44a} und R^{44b} unabhängig voneinander für H; F oder C₁₋₆-Alkyl, jeweils unsubstituiert oder einfach oder mehrfach substituiert mit gleichen oder verschiedenen Substituenten, stehen;
R⁴⁵ für H; C₁₋₆-Alkyl, C₃₋₆-Cycloalkyl oder Pyridyl, jeweils unsubstituiert oder einfach oder mehrfach substituiert mit gleichen oder verschiedenen Substituenten, steht.

Weitere bevorzugte Ausführungsformen der erfindungsgemäßen Verbindungen sind solche Verbindungen, in denen in den oben gezeigten Teilstrukturen gemäß den Formeln IIIc oder IIId eine der folgenden Gruppen A bis H, insbesondere G oder H darstellen: 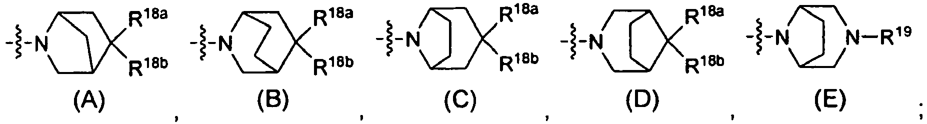 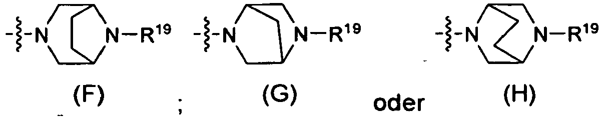 und worin
R^{18a} für H; C₁₋₆-Alkyl; C₃₋₈-Cycloalkyl, N(C₁₋₆-Alkyl)₂; NH(C₁₋₆-Alkyl); Azetidinyl; Pyrrolidinyl, Piperidinyl, 4-(C₁₋₆Alkyl)-Piperazinyl; Phenyl, Pyridinyl, Pyrimidinyl, Imidazolyl, Thiazolyl, Triazolyl oder Thienyl, jeweils unsubstituiert oder einfach oder mehrfach substituiert mit gleichen oder verschiedenen Substituenten; über eine -(O)₀₋₁-C₁₋₆-Alkylengruppe gebundenes N(C₁₋₆-Alkyl)₂; NH(C₁₋₆-Alkyl); Azetidinyl; Pyrrolidinyl, Piperidinyl, 4-(C₁₋₆Alkyl)-Piperazinyl; Phenyl, Pyridinyl, Pyrimidinyl, Imidazolyl, Thiazolyl, Triazolyl oder Thienyl, jeweils unsubstituiert oder einfach oder mehrfach substituiert mit gleichen oder verschiedenen Substituenten, steht;
R^{18b} für H; OH; C₁₋₆-Alkyl; Phenyl, Pyridinyl, Pyrimidinyl, Imidazolyl, Thiazolyl, Triazolyl oder Thienyl, jeweils unsubstituiert oder einfach oder mehrfach substituiert mit gleichen oder verschiedenen Substituenten; über eine C₁₋₆-Alkylengruppe gebundenes Phenyl, Pyridinyl, Pyrimidinyl, Imidazolyl, Thiazolyl, Triazolyl oder Thienyl, jeweils unsubstituiert oder einfach oder mehrfach substituiert mit gleichen oder verschiedenen Substituenten, steht;
R¹⁹ für H; C₁₋₆-Alkyl; C₃₋₈-Cycloalkyl, Phenyl, Pyridinyl, Pyrimidinyl, Imidazolyl, Thiazolyl, Triazolyl oder Thienyl, jeweils unsubstituiert oder einfach oder mehrfach substituiert mit gleichen oder verschiedenen Substituenten; über eine C₁₋₆-Alkylengruppe oder eine (C=O)-Gruppe gebundenes Phenyl, Pyridinyl, Pyrimidinyl, Imidazolyl, Thiazolyl, Triazolyl oder Thienyl, jeweils unsubstituiert oder einfach oder mehrfach substituiert mit gleichen oder verschiedenen Substituenten; steht.

Erneut versteht der Fachmann, dass die für die Reste A bis H gewählte Darstellung alle möglichen Stereoisomere dieser Reste mit umfasst.

Weitere bevorzugte Ausführungsformen der erfindungsgemäßen Verbindungen sind solche Verbindungen, in denen in den oben gezeigten Teilstrukturen gemäß den Formeln IIIh oder IIIi:
R^{18a} für H; C₁₋₆-Alkyl; C₃₋₈-Cycloalkyl, N(C₁₋₆-Alkyl)₂; NH(C₁₋₆-Alkyl), Azetidinyl; Pyrrolidinyl, Piperidinyl, 4-(C₁₋₆Alkyl)-Piperazinyl; Phenyl, Pyridinyl, Pyrimidinyl, Imidazolyl, Thiazolyl, Triazolyl oder Thienyl, jeweils unsubstituiert oder einfach oder mehrfach substituiert mit gleichen oder verschiedenen Substituenten; über eine - (O)_{0/1}-C₁₋₆-Alkylengruppe gebundenes N(C₁₋₆-Alkyl)₂; NH(C₁₋₆-Alkyl), Azetidinyl; Pyrrolidinyl, Piperidinyl, 4-(C₁₋₆Alkyl)-Piperazinyl; Phenyl, Pyridinyl, Pyrimidinyl, Imidazolyl, Thiazolyl, Triazolyl oder Thienyl, jeweils unsubstituiert oder einfach oder mehrfach substituiert mit gleichen oder verschiedenen Substituenten, steht;
R^{18b} für H; OH;; C₁₋₆-Alkyl; Phenyl, Pyridinyl, Pyrimidinyl, Imidazolyl, Thiazolyl, Triazolyl oder Thienyl, jeweils unsubstituiert oder einfach oder mehrfach substituiert mit gleichen oder verschiedenen Substituenten; über eine C₁₋₆-Alkylengruppe gebundenes Phenyl, Pyridinyl, Pyrimidinyl, Imidazolyl, Thiazolyl, Triazolyl oder Thienyl, jeweils unsubstituiert oder einfach oder mehrfach substituiert mit gleichen oder verschiedenen Substituenten, steht;
R¹⁹ für H; C₁₋₆-Alkyl; C₃₋₈-Cycloalkyl, Phenyl, Pyridinyl, Pyrimidinyl, Imidazolyl, Thiazolyl, Triazolyl oder Thienyl, jeweils unsubstituiert oder einfach oder mehrfach substituiert mit gleichen oder verschiedenen Substituenten;über eine C₁₋₆-Alkylengruppe oder (C=O)-Gruppe gebundenes Phenyl, Pyridinyl, Pyrimidinyl, Imidazolyl, Thiazolyl, Triazolyl oder Thienyl, jeweils unsubstituiert oder einfach oder mehrfach substituiert mit gleichen oder verschiedenen Substituenten, steht.

Weitere bevorzugte Ausführungsformen der erfindungsgemäßen Verbindungen sind solche Verbindungen, in denen die oben gezeigte Teilstruktur gemäß der Formel IIc eine der folgenden Teilstrukturen SP 1 bis SP 34 annehmen kann: 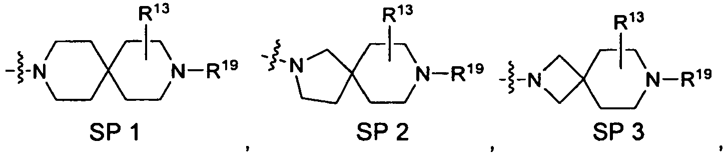 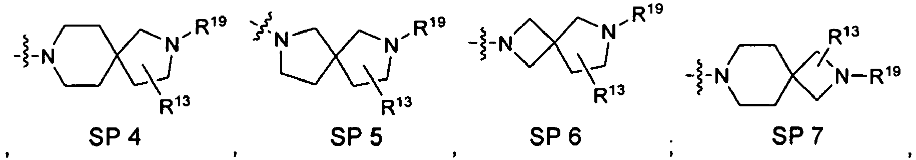 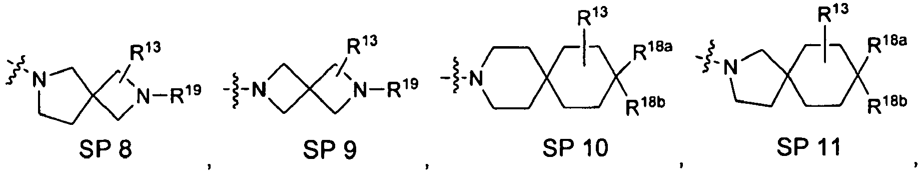 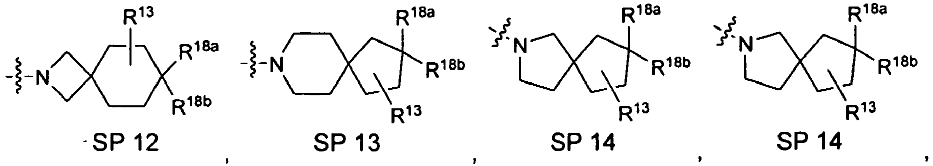 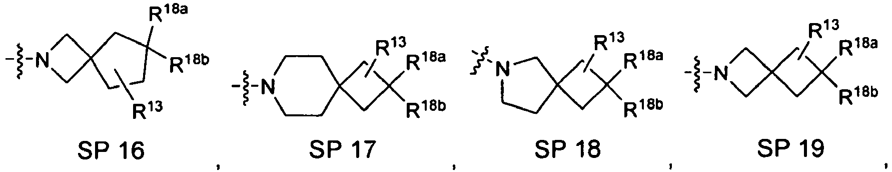 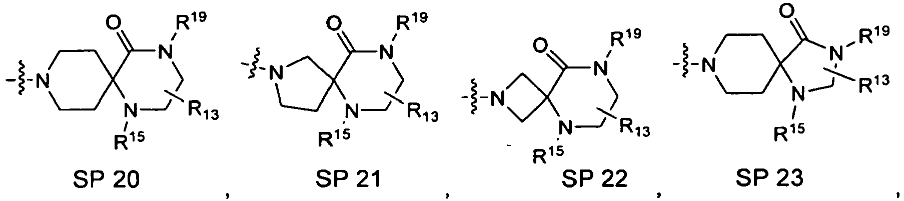 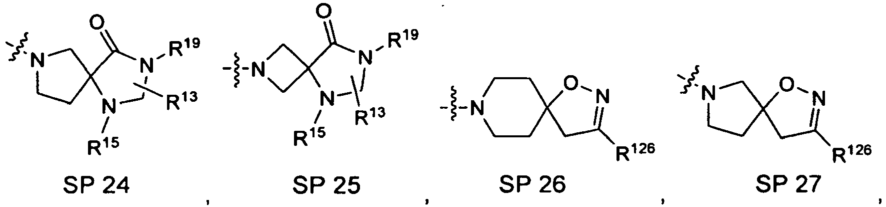 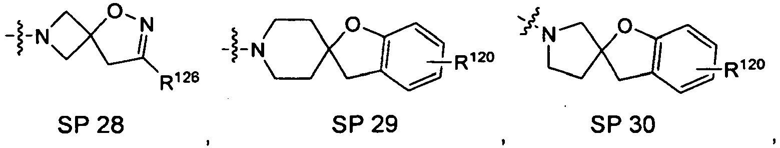 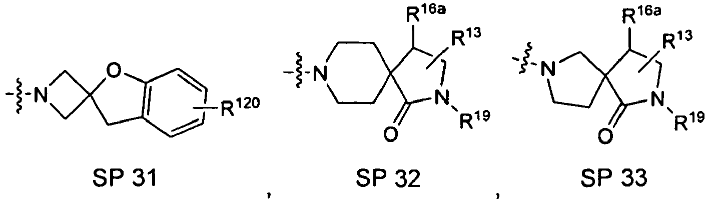 oder 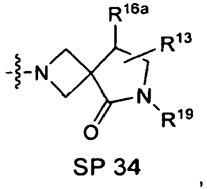 wobei
R¹³ für H oder Phenyl, unsubstituiert oder einfach oder mehrfach substituiert mit gleichen oder verschiedenen Substituenten, steht; und/oder zwei der Substituenten R¹³ zusammen =O bilden
und/oder zwei benachbarte Substituenten R¹³ zusammen ein anelliertes Aryl oder Heteroaryl, insbesondere eine Benzogruppe, jeweils unsubstituiert oder einfach oder mehrfach substituiert mit gleichen oder verschiedenen Substituenten, bilden,
R¹⁵ H; C₁₋₆-Alkyl; C₃₋₈-Cycloalkyl, Phenyl, Pyridinyl, Pyrimidinyl, Imidazolyl, Thiazolyl, Triazolyl oder Thienyl, jeweils unsubstituiert oder einfach oder mehrfach substituiert mit gleichen oder verschiedenen Substituenten; über eine C₁₋₆-Alkylengruppe gebundenes Phenyl, Pyridinyl, Pyrimidinyl, Imidazolyl, Thiazolyl, Triazolyl oder Thienyl, jeweils unsubstituiert oder einfach oder mehrfach substituiert mit gleichen oder verschiedenen Substituenten, steht;
R^{16a} für H, C₁₋₆-Alkyl, Phenyl, Pyridinyl, Pyrimidinyl, Imidazolyl, Thiazolyl, Triazolyl oder Thienyl, jeweils unsubstituiert oder einfach oder mehrfach substituiert mit gleichen oder verschiedenen Substituenten, steht;
R^{18a} für H; C₁₋₆-Alkyl; C₃₋₈-Cycloalkyl, N(C₁₋₆-Alkyl)₂; NH(C₁₋₆-Alkyl), Azetidinyl; Pyrrolidinyl, Piperidinyl, 4-(C₁₋₆Alkyl)-Piperazinyl; Phenyl, Pyridinyl, Pyrimidinyl, Imidazolyl, Thiazolyl, Triazolyl oder Thienyl, jeweils unsubstituiert oder einfach oder mehrfach substituiert mit gleichen oder verschiedenen Substituenten; über eine - (O)_{0/1}-C₁₋₆-Alkylengruppe gebundenes N(C₁₋₆-Alkyl)₂; NH(C₁₋₆-Alkyl), Azetidinyl; Pyrrolidinyl, Piperidinyl, 4-(C₁₋₆Alkyl)-Piperazinyl; Phenyl, Pyridinyl, Pyrimidinyl, Imidazolyl, Thiazolyl, Triazolyl oder Thienyl, jeweils unsubstituiert oder einfach oder mehrfach substituiert mit gleichen oder verschiedenen Substituenten, steht;
R^{18b} für H; OH; C₁₋₆-Alkyl; Phenyl, Pyridinyl, Pyrimidinyl, Imidazolyl, Thiazolyl, Triazolyl oder Thienyl, jeweils unsubstituiert oder einfach oder mehrfach substituiert mit gleichen oder verschiedenen Substituenten; über eine C₁₋₆-Alkylengruppe gebundenes Phenyl, Pyridinyl, Pyrimidinyl, Imidazolyl, Thiazolyl, Triazolyl oder Thienyl, jeweils unsubstituiert oder einfach oder mehrfach substituiert mit gleichen oder verschiedenen Substituenten; steht;
R¹⁹ für H; C₁₋₆-Alkyl; C₃₋₈-Cycloalkyl, Phenyl, Pyridinyl, Pyrimidinyl, Imidazolyl, Thiazolyl, Triazolyl oder Thienyl, jeweils unsubstituiert oder einfach oder mehrfach substituiert mit gleichen oder verschiedenen Substituenten; über eine C₁₋₆-Alkylengruppe oder (C=O)-Gruppe gebundenes Phenyl, Pyridinyl, Pyrimidinyl, Imidazolyl, Thiazolyl, Triazolyl oder Thienyl, jeweils unsubstituiert oder einfach oder mehrfach substituiert mit gleichen oder verschiedenen Substituenten, steht;
R¹²⁰ für H; F; Cl; OH; OCH₃, O-CF₃, C₁₋₆-Alkyl; CF₃, Phenyl, unsubstituiert oder einfach oder mehrfach substituiert, steht;
R¹²⁶ für H; C₁₋₆Alkyl; C₃₋₆Cycloalkyl; Phenyl, Pyridinyl, Pyrimidinyl, Imidazolyl, Thiazolyl, Triazolyl oder Thienyl; über eine C₁₋₃-Alkylengruppe gebundenes C₃₋₆Cycloalkyl, Phenyl, Pyridinyl, Pyrimidinyl, Imidazolyl, Thiazolyl, Triazolyl oder Thienyl, jeweils unsubstituiert oder einfach oder mehrfach substituiert mit gleichen oder verschiedenen Substituenten, steht.

Weitere bevorzugte Ausführungsformen der erfindungsgemäßen Verbindungen sind solche Verbindungen, in denen in der der oben gezeigten allgemeinen Formel I die nachfolgend gezeigte Teilstruktur (B) 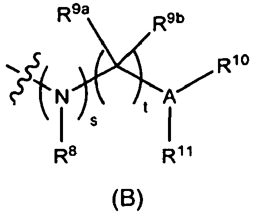 ausgesucht ist aus einer der Teilstrukturen folgenden B.1. bis B.45. 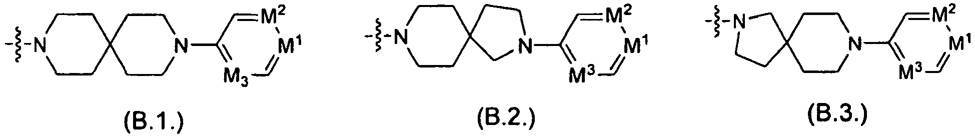 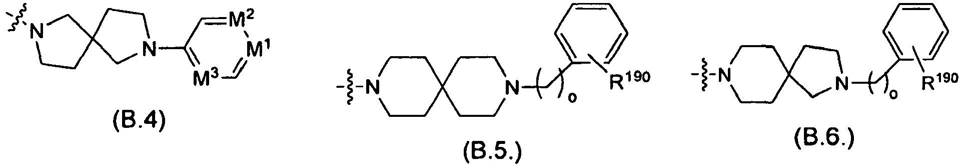 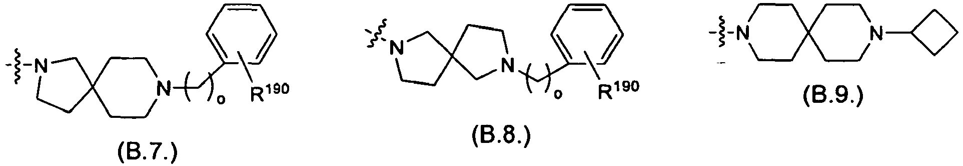 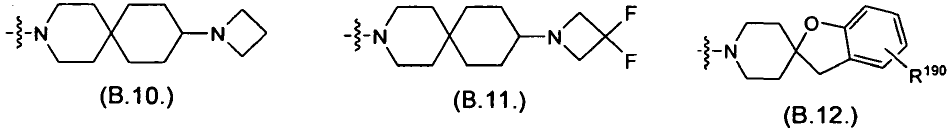 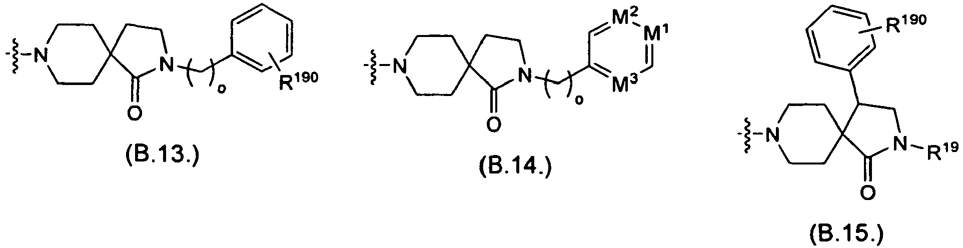 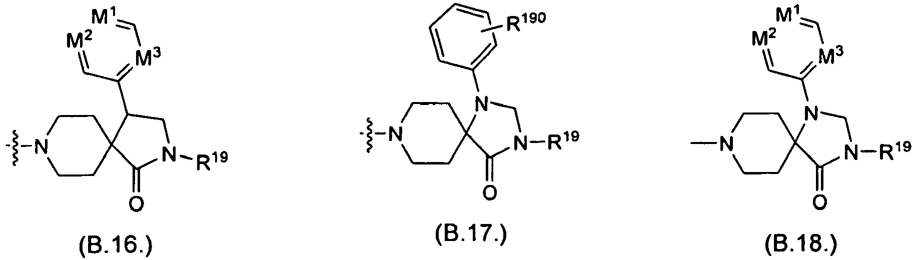 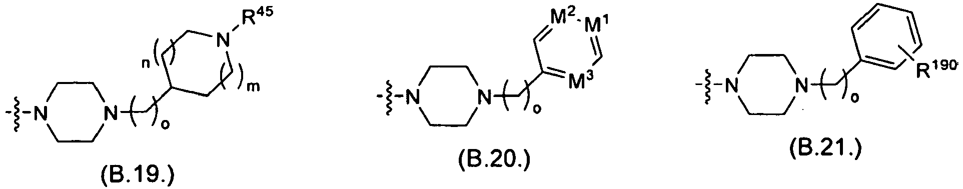 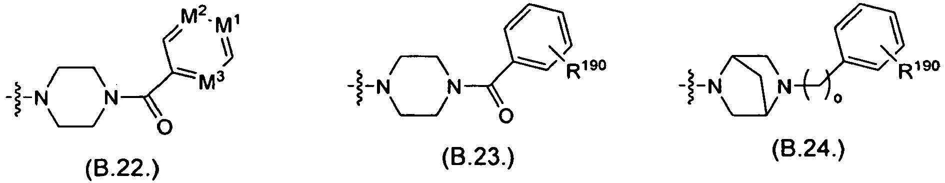 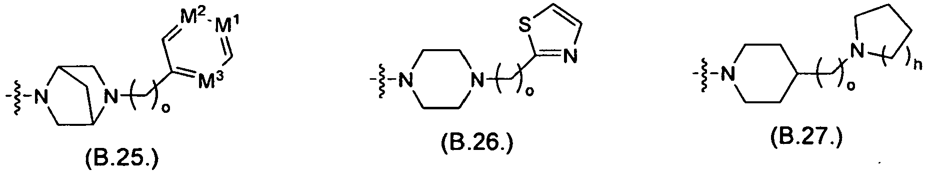 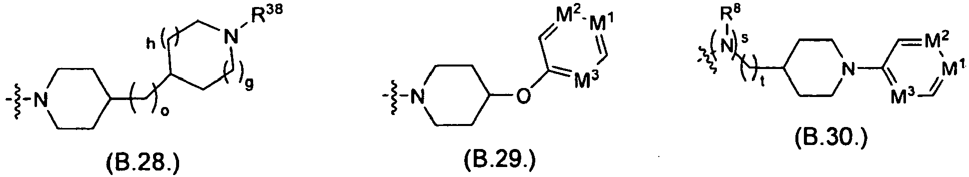 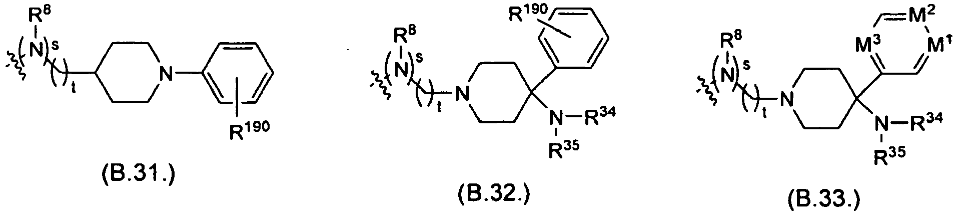 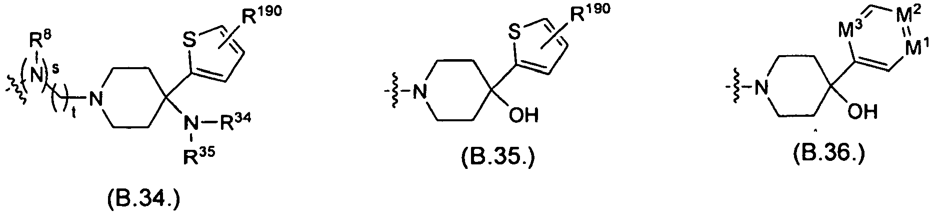 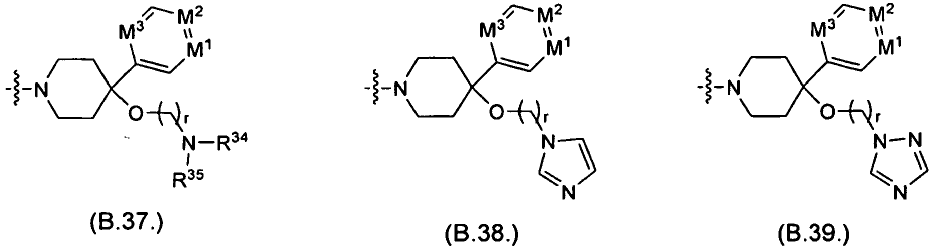 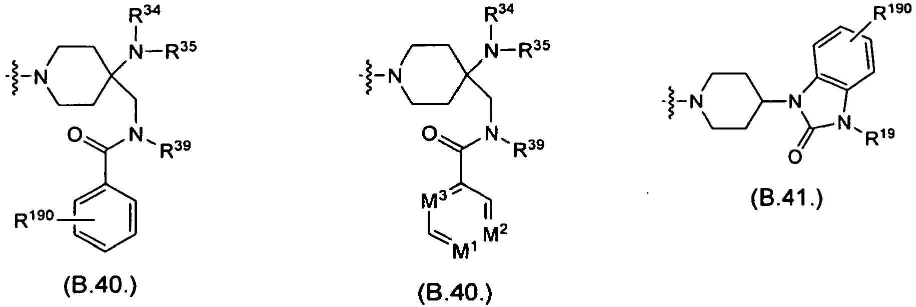 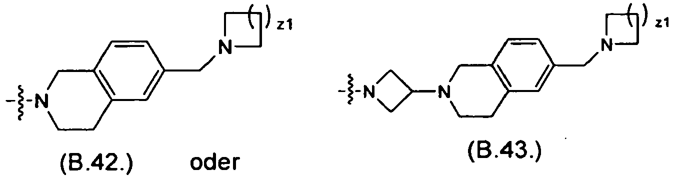 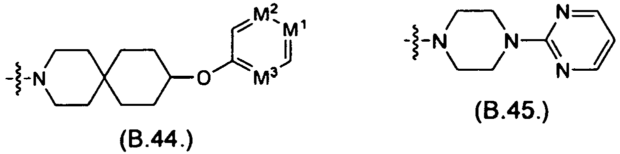 worin
h = 0 oder 1;
g = 0 oder 1;
n = 0 oder 1;
m =0 oder 1;
o=0, 1, 2 oder 3;
r = 1, 2 oder 3, insbesondere 1 oder 2;
s = 0 oder 1;
t = 0, 1, 2 oder 3, insbesondere 0, 1, oder 2, mit der Maßgabe, dass wenn s für 0 steht, t ebenfalls für 0 steht;
z1 = 0, 1, 2, oder 3, insbesondere 1;
M¹, M² und M³, unabhängig voneinander, jeweils für N oder CH stehen, unter der Bedinung, dass immer nur eine der Variablen M¹, M² und M³ für N steht und die jeweils anderen beiden für CH; (so dass der mittels der Variablen M¹, M² und M³ beschriebene Heterocyclus für 2-Pyridinyl, 3-Pyridinyl und 4-Pyridinyl stehen kann.)

In den oben gezeigten Gruppen B 42 und B43 kann z1 insbesondere für 1 stehen.

R⁸ für H; C₁₋₆Alkyl, insbesondere Methyl, Ethyl, n-Propyl, iso-Propyl, n-Butyl, sec-Butyl, iso-Butyl und tert-Butyl; C₃₋₆Cycloalkyl, insbesondere Cyclopropyl; jeweils unsubstituiert oder einfach oder mehrfach substituiert mit gleichen oder verschiedenen Substituenten,
R¹⁹ ausgesucht ist aus H; C₁₋₆Alkyl, insbesondere Methyl, Ethyl, n-Propyl, iso-Propyl, n-Butyl, sec-Butyl, iso-Butyl und tert-Butyl; C₃₋₆Cycloalkyl, insbesondere Cyclopropyl; jeweils unsubstituiert oder einfach oder mehrfach substituiert mit gleichen oder verschiedenen Substituenten.

R³⁴ und R³⁵ vorzugsweise unabhängig voneinander Methyl oder Ethyl sind oder zusammen mit dem sie verbindenden N-Atom eine Azetidinyl-; Pyrrolidinyl-, Piperidinyl-, 4-(C₁₋₆Alkyl)-Piperazinyl-Gruppe bilden; jeweils unsubstituiert oder einfach oder mehrfach substituiert mit gleichen oder verschiedenen Substituenten;
R³⁸ für H, C1-6-Alkyl, C3-6-Cycloalkyl oder Pyridinyl (Pyridyl) steht;
R³⁹ ausgesucht ist aus H; C₁₋₆Alkyl, insbesondere Methyl, Ethyl, n-Propyl, iso-Propyl, n-Butyl, sec-Butyl, iso-Butyl und tert-Butyl; C₃₋₆Cycloalkyl, insbesondere Cyclopropyl, jeweils unsubstituiert oder einfach oder mehrfach substituiert mit gleichen oder verschiedenen Substituenten; und
R⁴⁵ für H, C₁₋₆-Alkyl, C₃₋₆-Cycloalkyl oder Pyridyl steht;

R¹⁹⁰ 0-4 Substituenten darstellt, die unabhängig voneinander ausgesucht sind aus F, Cl, O-CF₃, CF₃ oder CN.

In bestimmten Ausführungsformen der erfindungsgemäßen Verbindungen, die eine der oben beschriebenen Teilstrukturen B.1. bis B.45. aufweisen, sind R⁸, R^{9a}, R^{9b}, R¹⁹ und R³⁹ jeweils, unabhängig voneinander, H oder Methyl.

In den oben gezeigten Teilstrukturen B.5., B.6., B.7., B.8., B.13., B.14., B. 20., B. 21., B.24., B. 25. und B.26. steht o vorzugsweise für 0 oder 1, in den Teilstrukturen

B.5., B.6., B.7., B.8. und B.13. steht o vorzugsweise für 1, in der Teilstruktur B.26. vorzugsweise für 0. In den Teilstrukturen B.27. und B.28. steht o vorzugsweise für 1 oder 2.

In den oben gezeigten Teilstrukturen B.1. bis B.45. steht R¹⁹⁰, wenn er an eine Phenylgruppe gebunden ist, vorzugsweise für einen Substituenten, der ausgesucht ist aus F oder CF₃, und der vorzugsweise in der 3- oder 4-Position an den Phenylring gebunden ist.

Weitere Ausführungsformen der erfindungsgemäßen Verbindungen sind solche, die durch die im Folgenden gezeigten allgemeinen Formel C1 bis C21 dargestellt werden: 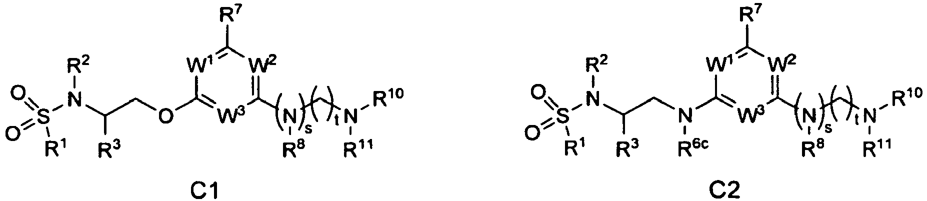 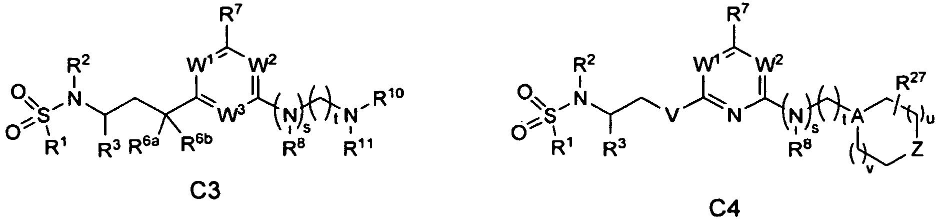 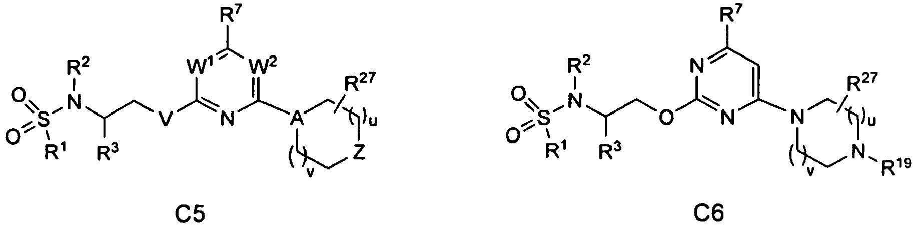 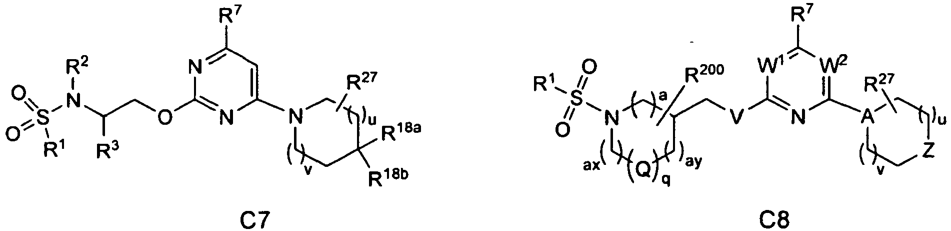 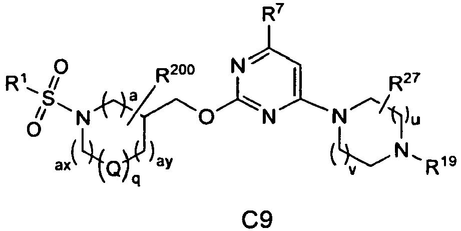 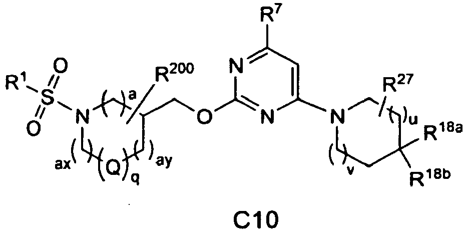 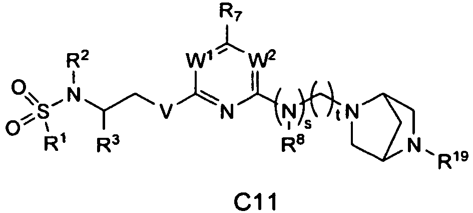 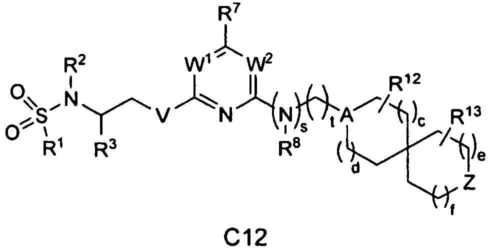 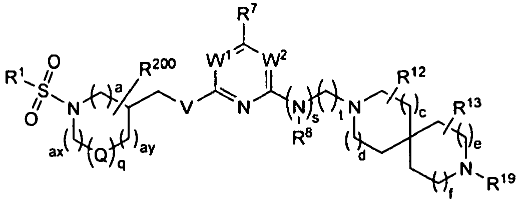 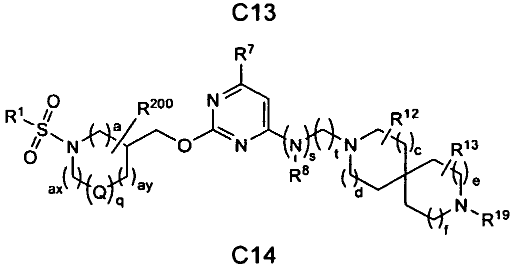 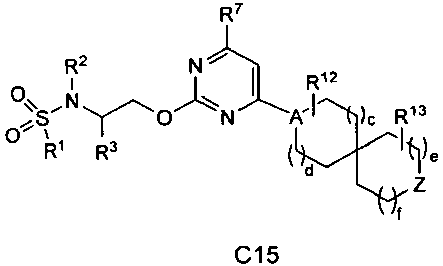 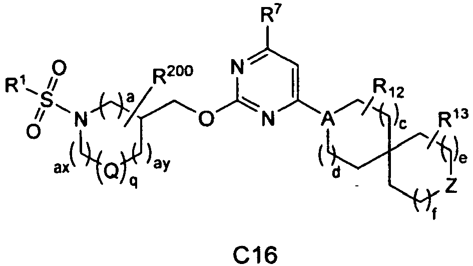 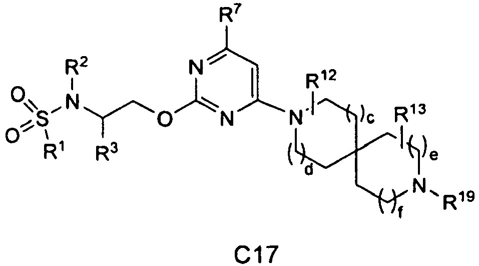 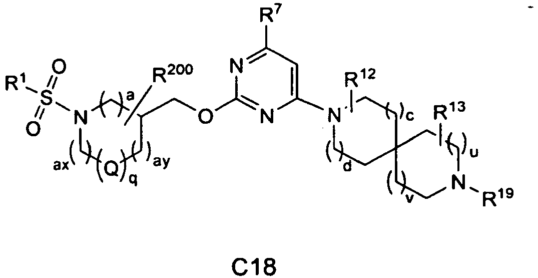 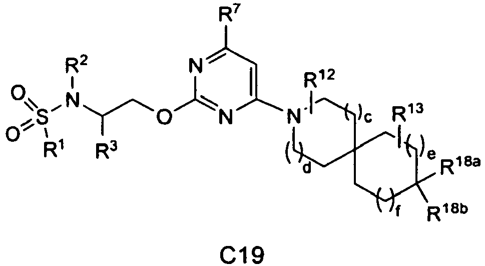 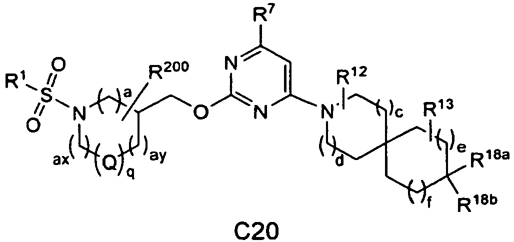 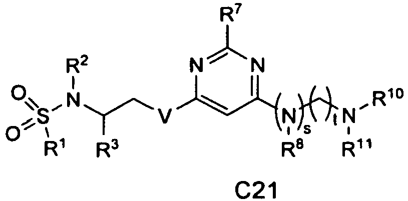 und worin
q für 0 oder 1 steht,
a für 0, 1 oder 2 steht;
ax für 0, 1, 2 oder 3 steht;
ay für 0, 1 oder 2;
q für 0 oder 1 steht;
mit der Maßgabe, dass a + ax + ay + q ≥ 2;
Q für CH₂, NR⁵⁰, O, S, S=O oder S(=O)₂ steht,
und alle weiteren Reste, Variabeln und Indices die oben im Zusammenhang mit den erfindungsgemäßen Verbindungen und deren bevorzugten Ausführungsformen beschriebenen Bedeutungen haben.

In bevorzugten Ausführungsformen der erfindungsgemäßen Verbindungen sind diese Verbindungen gemäß den oben gezeigten allgemeinen Formeln C1 bis C21,
worin, insoweit in der jeweiligen allgemeinen Formel vorhanden,
a für 0, 1 oder 2 steht;
ax für 0,1 oder 2 steht;
ay für 0, 1 oder 2 steht
q für 0 oder 1 steht;
mit der Maßgabe, dass a + ax + ay + q ≥ 2;
Q für CH₂, NR⁵⁰ oder O steht,
V für O steht;
c, d, e und f, jeweils unabhängig voneinander, für 0 oder 1 stehen
s für 1 steht,
t für 1, 2 oder 3 steht,
u und v, unabhängig voneinander, für 0 oder 1 stehen;
W¹ und W³ für N stehen und W² für CH steht, oder
W² und W³ für N stehen und W¹ für CH steht, oder
W¹ und W² für N stehen und W³ für CH steht;
R¹ für Phenyl oder Naphthyl steht, wobei das Phenyl oder Naphthyl unsubstituiert oder einfach oder mehrfach, beispielsweise 2-, 3-, 4- oder 5-fach, mit gleichen oder verschiedenen Resten ausgewählt aus Methyl, Methoxy, CF₃, OCF₃, F, Cl und Br substituiert ist;
R² für H, C₁₋₆-Alkyl, C₃₋₆-Cycloalkyl, Phenyl oder ein über eine C₁₋₃-Alkylengruppe gebundenes C₃₋₆-Cycloalkyl oder Phenyl; jeweils unsubstituiert oder einfach oder mehrfach mit gleichen oder verschiedenen Resten substituiert, steht;
R³ für H, F, Cl, -CF₃, OH, Methoxy, Methyl oder Phenyl oder Benzyl, jeweils unsubstituiert oder einfach oder mehrfach mit gleichen oder verschiedenen Resten substituiert, steht;
R⁷ für H oder Methyl, Ethyl, n-Propyl, iso-Propyl, n-Butyl, iso-Butyl, sec-Butyl, tert-Butyl, insbesondere für H oder Methyl, jeweils unsubsitituiert oder einfach oder mehrfach mit gleichen oder verschiedenen Substituenten substituiert, steht;
R⁸ für H, Methyl, Ethyl, n-Propyl, iso-Propyl, n-Butyl, iso-Butyl, sec-Butyl, tert-Butyl; Cyclopropyl, Cyclobutyl, Cyclopentyl, Cyclohexyl, Phenyl, Benzyl, Phenylethyl, Phenylpropyl, oder für über eine C₁₋₃Alkylengruppe gebundenes Cyclopropyl, Cyclobutyl, Cyclopentyl oder Cyclohexyl, jeweils unsubstituiert oder einfach oder mehrfach mit gleichen oder verschiedenen Substituenten substituiert, steht;
R^{9a} und R^{9b} jeweils unabhängig voneinander für H; F Cyclobutyl, Cyclopentyl, Cyclohexyl; Methyl; Methoxy; Cyclopropyl; Phenyl; Benzyl, Phenylethyl oder ein über eine C₁₋₃-Alkylengruppe gebundenes C₃₋₆-Cycloalkyl, jeweils unsubsitituiert oder einfach oder mehrfach mit gleichen oder verschiedenen Substituenten substituiert, steht;
R¹² abwesend ist oder für 0-4 -F oder Methyl steht;
R¹³ und R²⁷ abwesend sind oder für 1-4 -F oder Methyl stehen oder für eine anellierte, unsubstituierte oder einfach oder mehrfach mit gleichen oder verschiedenen Substituenten substituierte Benzogruppe stehen;
R^{18a} für H; C₁₋₆-Alkyl; C₃₋₈-Cycloalkyl, N(C₁₋₆-Alkyl)₂; NH(C₁₋₆-Alkyl), Azetidinyl; Pyrrolidinyl, Piperidinyl, 4-(C₁₋₆Alkyl)-Piperazinyl; Phenyl, Pyridyl, Pyrimidyl, Imidazolyl, Triazolyl, Thiazolyl oder Thienyl, jeweils unsubstituiert oder einfach oder mehrfach substituiert; über eine -(O)_{0/1}-C₁₋₆-Alkylengruppe gebundenes N(C₁₋₆-Alkyl)₂; NH(C₁₋₆-Alkyl), Azetidinyl; Pyrrolidinyl, Piperidinyl, 4-(C₁₋₆Alkyl)-Piperazinyl; Phenyl, Pyridyl, Pyrimidyl, Imidazolyl, Triazolyl, Thiazolyl oder Thienyl, jeweils unsubstituiert oder einfach oder mehrfach substituiert, steht;
R^{18b} für H; OH; C₁₋₆-Alkyl; Phenyl, Pyridyl, Pyrimidyl, Imidazolyl, Triazolyl, Thiazolyl oder Thienyl jeweils unsubstituiert oder einfach oder mehrfach substituiert; über eine C₁₋₆-Alkylengruppe gebundenes Phenyl, Pyridyl, Pyrimidyl, Imidazolyl, Triazolyl, Thiazolyl oder Thienyl, O-Phenyl oder O-Pyridyl, jeweils unsubstituiert oder einfach oder mehrfach substituiert; über C₁₋₆Alkylen-NH(C=O) verbrücktes Phenyl, Pyridyl, Pyrimidyl, Imidazolyl, Triazolyl, Thiazolyl oder Thienyl, jeweils unsubstituiert oder einfach oder mehrfach substituiert, steht
R¹⁹ für H; C₁₋₆-Alkyl; C₃₋₈-Cycloalkyl, oder für über (C=O)₀₋₁ verbundenes C₁₋₆-Alkyl; Phenyl, Pyridyl, Pyrimidyl, Imidazolyl, Triazolyl, Thiazolyl oder Thienyl; jeweils unsubstituiert oder einfach oder mehrfach substituiert; über eine C₁₋₆-Alkylengruppe gebundenes Phenyl, Pyridyl, Pyrimidyl, Imidazolyl, Triazolyl, Thiazolyl oder Thienyl; jeweils unsubstituiert oder einfach oder mehrfach substituiert, steht.

In ebenfalls bevorzugten Ausführungsformen der erfindungsgemäßen Verbindungen sind in den Verbindungen gemäß den Formeln C1 bis C21, die folgenden Kombinationen erfüllt, nämlich
a steht für 0, ax für 2, q für 1, ay für 1 und Q für CH₂;
a steht für 0, ax für 2, q für 1, ay für 1 und Q für O;
a steht für 0, ax für 2, q für 1, ay für 1 und Q für NR⁵⁰;
a steht für 1, ax für 1, q für 1, ay für 1 und Q für CH₂;
a steht für 2, ax für 1, q für 1, ay für 0 und Q für CH₂;
a steht für 0, ax für 1, q für 1, ay für 1 und Q für CH₂;
a steht für 1, ax für 1, q für 0; und ay für 1;
a steht für 0, ax für 0, q für 1, ay für 2 und Q für O;
a steht für 1, ax für 0, q für 1, ay für 1 und Q für O;
a steht für 1; ax für 0, q für 1, ay für 0 und Q für CH₂; oder
a steht für 0, ax für 0, q für 1, ay für 1 und Q für CH₂.

Ebenfalls bevorzugte Ausführungsformen der erfindungsgemäßen Verbindungen sind Verbindungen ausgewählt aus:
[G-001] 4-Methoxy-N,2,6-trimethyl-N-[2-[[4-[4-(2-1-pyrrolidinylethyl)-1-piperidinyl]-2-pyrimidinyl]oxy]ethyl]benzolsulfonamid
[G-002] 4-Methoxy-N,2,6-trimethyl-N-[2-[[4-[4-(4-pyridyl)-1-piperazinyl]-2-pyrimidinyl]oxy]ethyl]benzolsulfonamid
[G-003] N-[2-[[4-[2-(4-Dimethylamino-4-phenyl-1-piperidinyl)ethyl-methylamino]-2-pyrimidinyl]oxy]ethyl]-4-methoxy-N,2,6-trimethylbenzolsulfonamid
[G-004] 2-[[1-(4-Methoxy-2,6-dimethylphenyl)sulfonyl-2-piperidinyl]methoxy]-4-[4-(3-pyridyl)-4-(2-1-pyrrolidinylethoxy)-1-piperidinyl]pyrimidin
[G-005] N-Cyclopropyl-4-methoxy-2,6-dimethyl-N-[2-[[4-[9-(4-pyridyl)-3,9-diazaspiro[5.5]undecan-3-yl]-2-pyrimidinyl]oxy]ethyl]benzolsulfonamid hydroclorid
[G-006] N-Cyclopropyl-4-methoxy-2,6-dimethyl-N-[2-[[4-[2-[1-(4-pyridyl)-4-piperidinyl]ethylamino]-2-pyrimidinyl]oxy]ethyl]benzolsulfonamid
[G-007] N-Cyclopropyl-4-methoxy-2,6-dimethyl-N-[2-[[4-[4-(3-pyridyl)-4-(2-1-pyrrolidinylethoxy)-1-piperidinyl]-2-pyrimidinyl]oxy]ethyl]benzolsulfonamid
[G-008] N-Cyclopropyl-N-[2-[[4-[4-hydroxy-4-(3-pyridyl)-1-piperidinyl]-2-pyrimidinyl]oxy]ethyl]-4-methoxy-2,6-dimethylbenzolsulfonamid
[G-009] 2-Chlor-N-cyclopropyl-6-methyl-N-[2-[[4-[9-(4-pyridyl)-3,9-diazaspiro[5.5]undecan-3-yl]-2-pyrimidinyl]oxy]ethyl]benzolsulfonamid
[G-010] N-Cyclopropyl-N-[2-[[4-[9-(4-pyridyl)-3,9-diazaspiro[5.5]undecan-3-yl]-2-pyrimidinyl]oxy]ethyl]-2-(trifluormethyl)benzolsulfonamid
[G-011] 3-[2-([(2S,4R)-4-Fluor-1-(4-methoxy-2,6-dimethylphenyl)sulfonyl-2-pyrrolidinyl]methoxy]-4-pyrimidinyl]-9-(4-pyridyl)-3,9-diazaspiro[5.5]undecan
[G-012] N-Cyclopropyl-4-methoxy-2,6-dimethyl-N-[2-[[4-[4-(4-pyridyloxy)-1-piperidinyl]-2-pyrimidinyl]oxy]ethyl]benzolsulfonamid
[G-013] N-[2-[[4-[6-(1-Azetidinylmethyl)-3,4-dihydro-1H-isoquinolin-2-yl]-2-pyrimidinyl]oxy]ethyl]-N-cyclopropyl-4-methoxy-2,6-dimethylbenzolsulfonamid
[G-014] N-Cyclopropyl-4-methoxy-2,6-dimethyl-N-[2-[[4-[8-(4-pyridyl)-3,8-diazaspiro[4.4]nonan-3-yl]-2-pyrimidinyl]oxy]ethyl]benzolsulfonamid
[G-015] N-[2-[[4-[9-(1-Azetidinyl)-3-azaspiro[5.5]undecan-3-yl]-2-pyrimidinyl]oxy]ethyl]-N-cyclopropyl-4-methoxy-2,6-dimethylbenzolsulfonamid hydroclorid
[G-016] N-Cyclopropyl-4-methoxy-2,6-dimethyl-N-[2-[[4-[9-(4-pyridyloxy)-3-azaspiro[5.5]undecan-3-yl]-2-pyrimidinyl]oxy]ethyl]benzolsulfonamid
[G-017] N-Cyclopropyl-N-[2-[[4-[9-(3,3-difluor-1-azetidinyl)-3-azaspiro[5.5]undecan-3-yl]-2-pyrimidinyl]oxy]ethyl]-4-methoxy-2,6-dimethylbenzolsulfonamid
[G-018] 3-[2-[[1-(4-Methoxy-2,6-dimethylphenyl)sulfonyl-3-azetidinyl]oxy]-4-pyrimidinyl]-9-(4-pyridyl)-3,9-diazaspiro[5.5]undecan
[G-019] N-Cyclopropyl-4-methoxy-2,6-dimethyl-N-[2-[[4-[8-(4-pyridyl)-3,8-diazaspiro[4.5]decan-3-yl]-2-pyrimidinyl]oxy]ethyl]benzolsulfonamid
[G-020] N-[2-[[4-[3-[6-(1-Azetidinylmethyl)-3,4-dihydro-1H-isoquinolin-2-yl]-1-azetidinyl]-2-pyrimidinyl]oxy]ethyl]-N-cydopropyl-4-methoxy-2,6-dimethylbenzolsulfonamid
G-021 2,6-Dichlor-N-cyclopropyl-3-methyl-N-[2-[4-(9-pyridin-4-yl-3,9-diazaspiro[5.5]undecan-3-yl)-pyrimidin-2-yl]oxy-ethyl]-benzolsulfonsäure amid
G-022 4-Methoxy-2,6-dimethyl-N-[1-[[4-(9-pyridin-4-yl-3,9-diazaspiro[5.5]undecan-3-yl)-pyrimidin-2-yl]oxy-methyl]-cyclobutyl]-benzolsulfonsäure amid
G-023 N-Cyclopropyl-4-methoxy-2,6-dimethyl-N-[2-[4-[9-pyridin-3-yl-9-(2-pyrrolidin-1-yl-ethoxy)-3-azaspiro[5.5]undecan-3-yl]-pyrimidin-2-yl]oxy-ethyl]-benzolsulfonsäure amid
G-024 N-[1,1-Dimethyl-2-[4-(9-pyridin-4-yl-3,9-diazaspiro[5.5]undecan-3-yl)-pyrimidin-2-yl]oxy-ethyl]-4-methoxy-2,6-dimethyl-benzolsulfonsäure amid
G-025 N-Cyclopropyl-4-methoxy-2,6-dimethyl-N-[3-[4-(9-pyridin-4-yl-3,9-diazaspiro[5.5]undecan-3-yl)-pyrimidin-2-yl]oxy-propyl]-benzolsulfonsäure amid
G-026 3-[2-[((2S)-1-[(4-Methoxy-2,6-dimethyl-phenyl)sulfonyl]-piperidin-2-yl]-methoxy]-pyrimidin-4-yl]-9-pyridin-4-yl-3,9-diazaspiro[5.5]undecan

[H-001] 4-Methoxy-N,2,6-trimethyl-N-[2-[[2-[4-(2-1-pyrrolidinylethyl)-1-piperidinyl]-4-pyrimidinyl]oxy]ethyl]benzolsulfonamid
[H-002] 4-Methoxy-N,2,6-trimethyl-N-[2-[[2-[4-(4-pyridyl)-1-piperazinyl]-4-pyrimidinyl]oxy]ethyl]benzolsulfonamid
[H-003] N-[2-[[2-[2-(4-Dimethylamino-4-phenyl-1-piperidinyl)ethyl-methylamino]-4-pyrimidinyl]oxy]ethyl]-4-methoxy-N,2,6-trimethylbenzolsulfonamid
[H-004] N-[2-(4-Dimethylamino-4-phenyl-1-piperidinyl)ethyl]-4-[[1-(4-methoxy-2,6-dimethylphenyl)sulfonyl-2-piperidinyl]methoxy]-N-methyl-2-pyrimidinamin
[H-005] N-Cyclopropyl-4-methoxy-2,6-dimethyl-N-[2-[[2-[4-(4-pyridyloxy)-1-piperidinyl]-4-pyrimidinyl]oxy]ethyl]benzolsulfonamid
[H-006] 2-Chlor-N-cyclopropyl-6-methyl-N-[2-[[2-[9-(4-pyridyl)-3,9-diazaspiro[5.5]undecan-3-yl]-4-pyrimidinyl]oxy]ethyl]benzolsulfonamid
[H-007] 3-[4-[[1-(4-Methoxy-2,6-dimethylphenyl)sulfonyl-3-azetidinyl]oxy]-2-pyrimidinyl]-9-(4-pyridyl)-3,9-diazaspiro[5.5]undecan
[I-001] N-Cyclopropyl-4-methoxy-2,6-dimethyl-N-[2-[[6-[9-(4-pyridyl)-3,9-diazaspiro[5.5]undecan-3-yl]-4-pyrimidinyl]oxy]ethyl]benzolsulfonamid
I-002 N-Cyclopropyl-4-methoxy-2,6-dimethyl-N-[2-[methyl-[6-(9-pyridin-4-yl-3,9-diazaspiro[5.5]undecan-3-yl)-pyrazin-2-yl]-amino]-ethyl]-benzolsulfonsäure amid
I-003 N-Cyclopropyl-4-methoxy-2,6-dimethyl-N-[2-[methyl-[6-(9-pyridin-4-yl-3,9-diazaspiro[5.5]undecan-3-yl)-pyrimidin-4-yl]-amino]-ethyl]-benzolsulfonsäure amid
I-004 N-Cyclopropyl-4-methoxy-2,6-dimethyl-N-[2-[methyl-[2-(9-pyridin-4-yl-3,9-diazaspiro[5.5]undecan-3-yl)-pyrimidin-4-yl]-amino]-ethyl]-benzolsulfonsäure amid
I-005 N-Cyclopropyl-4-methoxy-2,6-dimethyl-N-[2-[methyl-[4-(9-pyridin-4-yl-3,9-diazaspiro[5.5]undecan-3-yl)-pyrimidin-2-yl]-amino]-ethyl]-benzolsulfonsäure amid
I-006 N-Cyclopropyl-4-methoxy-2,6-dimethyl-N-[3-[6-(9-pyridin-4-yl-3,9-diazaspiro[5.5]undecan-3-yl)-pyrazin-2-yl]-propyl]-benzolsulfonsäure amid
I-007 N-Cyclopropyl-4-methoxy-2,6-dimethyl-N-[3-[2-(9-pyridin-4-yl-3,9-diazaspiro[5.5]undecan-3-yl)-pyrimidin-4-yl]-propyl]-benzolsulfonsäure amid
I-008 N-Cyclopropyl-N-[3-[2-(9-pyridin-4-yl-3,9-diazaspiro[5.5]undecan-3-yl)-pyrimidin-4-yl]-propyl]-3-(trifluormethyl)-benzolsulfonsäure amid

[G_CC- 4-Methoxy-N,2,6-trimethyl-N-[2-[[4-[4-(1-methyl-4-piperidinyl)-1-piperazinyl]-2-001] pyrimidinyl]oxy]ethyl]benzolsulfonamid
[G_CC- 2-[[1-(4-Methoxy-2,6-dimethylphenyl)sulfonyl-2-piperidinyl]methoxy]-4-[4-(1-methyl-4-002] piperidinyl)-1-piperazinyl]pyrimidin
[G_CC- 2-[[1-(4-Methoxy-2,6-dimethylphenyl)sulfonyl-2-piperidinyl]methoxy]-4-[4-(2-1-003] pyrrolidinylethyl)-1-piperidinyl]pyrimidin
[G_CC- N-[2-(4-Dimethylamino-4-phenyl-1-piperidinyl)ethyl]-2-[[1-(4-methoxy-2,6-004] dimethylphenyl)sulfonyl-2-piperidinyl]methoxy]-N-methyl-4-pyrimidinamin
[G_CC- N-[2-[[4-[4-(4-Fluorphenyl)-1-piperazinyl]-2-pyrimidinyl]oxy]ethyl]-4-methoxy-N,2,6-005] trimethylbenzolsulfonamid
[G_CC- 4-Methoxy-N,2,6-trimethyl-N-[2-[[4-[4-(4-methyl-1-piperazinyl)-1-piperidinyl]-2-006] pyrimidinyl]oxy]ethyl]benzolsulfonamid
[G_CC- 4-Methoxy-N,2,6-trimethyl-N-[2-[[4-[4-[(1-methyl-4-piperidinyl)methyl]-1-piperazinyl]-2-007] pyrimidinyl]oxy]ethyl]benzolsulfonamid
[G_CC- N-[2-[[4-[4-Hydroxy-4-(3-pyridyl)-1-piperidinyl]-2-pyrimidinyl]oxy]ethyl]-4-methoxy-N,2,6-008] trimethylbenzolsulfonamid
[G_CC- 2-[[1-(4-Methoxy-2,6-dimethylphenyl)sulfonyl-2-piperidinyl]methoxy]-4-(4-methyl-1-009] piperazinyl)pyrimidin
[G_CC- 4-[4-(4-Fluorphenyl)-1-piperazinyl]-2-[[1-(4-methoxy-2,6-dimethylphenyl)sulfonyl-2-010] piperidinyl]methoxy]pyrimidin
[G_CC- 2-[[1-(4-Methoxy-2,6-dimethylphenyl)sulfonyl-2-piperidinyl]methoxy]-4-[4-(4-methyl-1-011] piperazinyl)-1-piperidinyl]pyrimidin
[G_CC- 2-[[1-(4-Methoxy-2,6-dimethylphenyl)sulfonyl-2-piperidinyl]methoxy]-4-[4-(2-pyrimidinyl)-1-012] piperazinyl]pyrimidin
[G_CC- 2-[[1-(4-Methoxy-2,6-dimethylphenyl)sulfonyl-2-piperidinyl]methoxy]-4-[4-(4-pyridyl)-1-013] piperazinyl]pyrimidin
[G_CC- 2-[[1-(4-Methoxy-2,6-dimethylphenyl)sulfonyl-2-piperidinyl]methoxy]-4-[4-[(1-methyl-4-014] piperidinyl)methyl]-1-piperazinyl]pyrimidin
[G_CC- 2-[[(2S)-1-(4-Methoxy-2,6-dimethylphenyl)sulfonyl-2-pyrrolidinyl]methoxy]-4-(4-methyl-1-015] piperazinyl)pyrimidin
[G_CC- 2-[[(2S)-1-(4-Methoxy-2,6-dimethylphenyl)sulfonyl-2-pyrrolidinyl]methoxy]-4-[4-(2-1-016] pyrrolidinylethyl)-1-piperidinyl]pyrimidin
[G_CC- 2-[[(2S)-1-(4-Methoxy-2,6-dimethylphenyl)sulfonyl-2-pyrrolidinyl]methoxy]-4-[4-(4-methyl-1-017] piperazinyl)-1-piperidinyl]pyrimidin
[G_CC- 2-[[(2S)-1-(4-Methoxy-2,6-dimethylphenyl)sulfonyl-2-pyrrolidinyl]methoxy]-4-[4-(2-018] pyrimidinyl)-1-piperazinyl]pyrimidin
[G_CC- 2-[[(2S)-1-(4-Methoxy-2,6-dimethylphenyl)sulfonyl-2-pyrrolidinyl]methoxy]-4-[4-(4-pyridyl)-1-019] piperazinyl]pyrimidin
[G_CC- 1-[2-[[(2S)-1-(4-Methoxy-2,6-dimethylphenyl)sulfonyl-2-pyrrolidinyl]methoxy]-4-pyrimidinyl]-4-020] (3-pyridyl)-4-piperidinol
[G_CC- 1-[2-[[(2S)-1-(4-Methoxy-2,6-dimethylphenyl)sulfonyl-2-pyrrolidinyl]methoxy]-4-pyrimidinyl]-4-021] (2-thienyl)-4-piperidinol
[G_CC- 3-benzyl-7-[2-[[1-(4-Methoxy-2,6-dimethylphenyl)sulfonyl-2-piperidinyl]methoxy]-4-022] pyrimidinyl)-3,7-diazaspiro[4.4]nonan
[G_CC- 1'-[2-[[1-(4-Methoxy-2,6-dimethylphenyl)sulfonyl-2-piperidinyl]methoxy]-4-023] pyrimidinyl]spiro[1H-isobenzofuran-3,4'-piperidin]
[G_CC- 6-chlor-3-[1-[2-[[1-(4-Methoxy-2,6-dimethylphenyl)sulfonyl-2-piperidinyl]methoxy]-4-024] pyrimidinyl]-4-piperidinyl]-1H-benzimidazol-2-on
[G_CC- 8-[2-[[1-(4-Methoxy-2,6-dimethylphenyl)sulfonyl-2-piperidinyl]methoxy]-4-pyrimidinyl]-4-025] phenyl-2,4,8-triazaspiro[4.5]decan-1-on
[G_CC- 2-[[1-(4-Methoxy-2,6-dimethylphenyl)sulfonyl-2-piperidinyl]methoxy]-4-[4-[2-(1-026] piperidyl)ethyl]-1-piperidinyl]pyrimidin
[G_CC- 2-[[1-(4-Methoxy-2,6-dimethylphenyl)sulfonyl-3-piperidinyl]oxy]-4-[4-[2-(1-piperidyl)ethyl]-1-027] piperidinyl]pyrimidin
[G_CC- N-[2-[[4-(3-Benzyl-3,7-diazaspiro[4.4]nonan-7-yl)-2-pyrimidinyl]oxy]ethyl]-4-methoxy-N,2,6-028] trimethylbenzolsulfonamid
[G_CC- 4-Methoxy-N,2,6-trimethyl-N-[2-[[4-(1'-spiro[1H-isobenzofuran-3,4'-piperidin]yl)-2-029] pyrimidinyl]oxy]ethyl]benzolsulfonamid
[G_CC- 4-Methoxy-N,2,6-trimethyl-N-[2-[[4-(1-oxo-4-phenyl-2,4,8-triazaspiro[4.5]decan-8-yl)-2-030] pyrimidinyl]oxy]ethyl]benzolsulfonamid
[G_CC- 4-Methoxy-N,2,6-trimethyl-N-[2-[[4-[4-[2-(1-piperidyl)ethyl]-1-piperidinyl]-2-031] pyrimidinyl]oxy]ethyl]benzolsulfonamid
[G_CC- 3-Benzyl-7-[2-[[(2S)-1-(4-methoxy-2,6-dimethylphenyl)sulfonyl-2-pyrrolidinyl]methoxy]-4-032] pyrimidinyl]-3,7-diazaspiro[4.4]nonan
[G_CC- 1'-[2-[[(2S)-1-(4-Methoxy-2,6-dimethylphenyl)sulfonyl-2-pyrrolidinyl]methoxy]-4-033] pyrimidinyl]spiro[1H-isobenzofuran-3,4'-piperidin]
[G_CC- 6-Chlor-3-[1-[2-[[(2S)-1-(4-methoxy-2,6-dimethylphenyl)sulfonyl-2-pyrrolidinyl]methoxy]-4-034] pyrimidinyl]-4-piperidinyl]-1H-benzimidazol-2-on
[G_CC- 8-[2-[[(2S)-1-(4-Methoxy-2,6-dimethylphenyl)sulfonyl-2-pyrrolidinyl]methoxy]-4-pyrimidinyl]-4-035] phenyl-2,4,8-triazaspiro[4.5]decan-1-on
[G_CC- 2-[[(2S)-1-(4-Methoxy-2,6-dimethylphenyl)sulfonyl-2-pyrrolidinyl]methoxy]-4-[4-(1-methyl-4-036] piperidinyl)-1-piperazinyl]pyrimidin
[G_CC- 2-[[(2S)-1-(4-Methoxy-2,6-dimethylphenyl)sulfonyl-2-pyrrolidinyl]methoxy]-4-[4-[2-(1-037] piperidyl)ethyl]-1-piperidinyl]pyrimidin
[G_CC- 3-(4-Fluorphenyl)-8-[2-[[1-(4-methoxy-2,6-dimethylphenyl)sulfonyl-2-piperidinyl]methoxy]-4-038] pyrimidinyl]-3,8-diazaspiro[4.5]decan-4-on
[G_CC- 3-[(4-Fluorphenyl)methyl]-8-[2-[[1-(4-methoxy-2,6-dimethylphenyl)sulfonyl-2-039] piperidinyl]methoxy]-4-pyrimidinyl]-3,8-diazaspiro[4.5]decan-4-on
[G_CC- 3-Benzyl-8-[2-[[1-(4-methoxy-2,6-dimethylphenyl)sulfonyl-2-piperidinyl]methoxy]-4-040] pyrimidinyl]-3,8-diazaspiro[4.5]decan-4-on
[G_CC- 9-[2-[[1-(4-Methoxy-2,6-dimethylphenyl)sulfonyl-3-piperidinyl]oxy]-4-pyrimidinyl]-3-(4-pyridyl)-041] 3,9-diazaspiro[5.5]undecan
[G_CC- N-[2-[[4-[3-(4-Fluorphenyl)-4-oxo-3,8-diazaspiro[4.5]decan-8-yl]-2-pyrimidinyl]oxy]ethyl]-4-042] methoxy-N,2,6-trimethylbenzolsulfonamid
[G_CC- 4-Methoxy-N,2,6-trimethyl-N-[2-[[4-[4-oxo-1-[3-(trifluormethyl)phenyl]-3,8-043] diazaspiro[4.5]decan-8-yl]-2-pyrimidinyl]oxy]ethyl]benzolsulfonamid
[G_CC- N-[2-[[4-[1-(4-Fluorphenyl)-3-methyl-4-oxo-3,8-diazaspiro[4.5]decan-8-yl]-2-044] pyrimidinyl]oxy]ethyl]-4-methoxy-N,2,6-trimethylbenzolsulfonamid
[G_CC- N-[2-[[4-[3-[(4-Fluorphenyl)methyl]-4-oxo-3,8-diazaspiro[4.5]decan-8-yl]-2-045] pyrimidinyl]oxy]ethyl]-4-methoxy-N,2,6-trimethylbenzolsulfonamid
[G_CC- N-[2-[[4-(3-Benzyl-4-oxo-3,8-diazaspiro[4.5]decan-8-yl)-2-pyrimidinyl]oxy]ethyl]-4-methoxy-046] N,2,6-trimethylbenzolsulfonamid
[G_CC- 4-Methoxy-N,2,6-trimethyl-N-[2-[[4-[3-(4-pyridyl)-3,9-diazaspiro[5.5]undecan-9-yl]-2-047] pyrimidinyl]oxy]ethyl]benzolsulfonamid
[G_CC- 3-(4-Fluorphenyl)-8-[2-[[(2S)-1-(4-methoxy-2,6-dimethylphenyl)sulfonyl-2-048] pyrrolidinyl]methoxy]-4-pyrimidinyl]-3,8-diazaspiro[4.5]decan-4-on
[G_CC- 8-[2-[[(2S)-1-(4-Methoxy-2,6-dimethylphenyl)sulfonyl-2-pyrrolidinyl]methoxy]-4-pyrimidinyl]-1-049] [3-(trifluormethyl)phenyl]-3,8-diazaspiro[4.5]decan-4-on
[G_CC- 1-(4-Fluorphenyl)-8-[2-[[(2S)-1-(4-methoxy-2,6-dimethylphenyl)sulfonyl-2-050] pyrrolidinyl]methoxy]-4-pyrimidinyl]-3-methyl-3,8-diazaspiro[4.5]decan-4-on
[G_CC- 3-[(4-Fluorphenyl)methyl]-8-[2-[[(2S)-1-(4-methoxy-2,6-dimethylphenyl)sulfonyl-2-051] pyrrolidinyl]methoxy]-4-pyrimidinyl]-3,8-diazaspiro[4.5]decan-4-on
[G_CC- 2-[[(2S)-1-(4-Methoxy-2,6-dimethylphenyl)sulfonyl-2-pyrrolidinyl]methoxy]-4-[4-(4-pyridyloxy)-052] 1-piperidinyl]pyrimidin
[G_CC- 3-Benzyl-8-[2-[[(2S)-1-(4-methoxy-2,6-dimethylphenyl)sulfonyl-2-pyrrolidinyl]methoxy]-4-053] pyrimidinyl]-3,8-diazaspiro[4.5]decan-4-on
[G_CC- N-[[1-[2-[[(2R)-1-(4-Methoxy-2,6-dimethylphenyl)sulfonyl-2-pyrrolidinyl]methoxy]-4-054] pyrimidinyl]-4-(4-methyl-1-piperazinyl)-4-piperidinyl]methyl]-4-pyridincarboxamid
[G_CC- 9-[2-[[(2S)-1-(4-Methoxy-2,6-dimethylphenyl)sulfonyl-2-pyrrolidinyl]methoxy]-4-pyrimidinyl]-3-055] (4-pyridyl)-3,9-diazaspiro[5.5]undecan
[G_CC- 5-[2-[[1-(4-Methoxy-2,6-dimethylphenyl)sulfonyl-2-piperidinyl]methoxy]-4-pyrimidinyl]-2-(4-056] pyridylmethyl)-2,5-diazabicyclo[2.2.1]heptan
[G_CC- 5-[2-[[1-(4-Methoxy-2,6-dimethylphenyl)sulfonyl-3-piperidinyl]oxy]-4-pyrimidinyl]-2-(4-057] pyridylmethyl)-2,5-diazabicyclo[2.2.1]heptan
[G_CC- 5-[2-[[(2S)-1-(4-Methoxy-2,6-dimethylphenyl)sulfonyl-2-pyrrolidinyl]methoxy]-4-pyrimidinyl]-2-058] (4-pyridylmethyl)-2,5-diazabicyclo[2.2.1]heptan
[G_CC- 4-Methoxy-N,2,6-trimethyl-N-[2-[[4-[4-(1-methyl-4-piperidinyl)-1-piperazinyl]-2-059] pyrimidinyl]oxy]-1-phenylethyl]benzolsulfonamid
[G_CC- N-[2-[[4-[3-[(4-Fluorphenyl)methyl]-4-oxo-3,8-diazaspiro[4.5]decan-8-yl]-2-pyrimidinyl]oxy]-1-060] phenylethyl]-4-methoxy-N,2,6-trimethylbenzolsulfonamid
[G_CC- N-[2-[[4-(3-Benzyl-4-oxo-3,8-diazaspiro[4.5]decan-8-yl)-2-pyrimidinyl]oxy]-1-phenylethyl]-4-061] methoxy-N,2,6-trimethylbenzolsulfonamid
[G_CC- 4-Methoxy-N,2,6-trimethyl-N-[1-phenyl-2-[[4-[4-(3-pyridyl)-4-(2-1-pyrrolidinylethoxy)-1-062] piperidinyl]-2-pyrimidinyl]oxy]ethyl]benzolsulfonamid
[G_CC- 4-Methoxy-N,2,6-trimethyl-N-[1-phenyl-2-[[4-[4-(4-pyridyloxy)-1-piperidinyl]-2-063] pyrimidinyl]oxy]ethyl]benzolsulfonamid
[G_CC- 4-Methoxy-N,2,6-trimethyl-N-[2-[[4-[4-[oxo-(3-pyridyl)methyl]-1-piperazinyl]-2-064] pyrimidinyl]oxy]-1-phenylethyl]benzolsulfonamid
[G_CC- N-[2-[[4-[2-[(4-Fluorphenyl)methyl]-2,5-diazabicyclo[2.2.1]heptan-5-yl]-2-pyrimidinyl]oxy]-1-065] phenylethyl]-4-methoxy-N,2,6-trimethylbenzolsulfonamid
[G_CC- 2-[[1-(4-Methoxy-2,6-dimethylphenyl)sulfonyl-2-piperidinyl]methoxy]-4-[4-(4-pyridyl)-1-066] piperazinyl]pyrimidin
[G_CC- 1-[2-[[1-(4-Methoxy-2,6-dimethylphenyl)sulfonyl-2-piperidinyl]methoxy]-4-pyrimidinyl]-4-(3-067] pyridyl)-4-piperidinol
[G_CC- N-[2-[[4-(3-Benzyl-3,7-diazaspiro[4.4]nonan-7-yl)-2-pyrimidinyl]oxy]-1-phenylethyl]-4-068] methoxy-N,2,6-trimethylbenzolsulfonamid
G_CC- [4-Butyl-1-[2-[[1-[(4-methoxy-2,6-dimethyl-phenyl)sulfonyl]-piperidin-2-yl]-methoxy]-pyrimidin-069 4-yl]-piperidin-4-yl]-dimethyl-amin
G_CC- [1-[2-[[1-[(4-Methoxy-2,6-dimethyl-phenyl)sulfonyl]-piperidin-2-yl]-methoxy]-pyrimidin-4-yl]-4-070 thiophen-2-yl-piperidin-4-yl]-dimethyl-amin
G_CC- [2-[[1-[(4-Methoxy-2,6-dimethyl-phenyl)sulfonyl]-piperidin-2-yl]-methoxy]-pyrimidin-4-yl]-073 methyl-[2-(4-phenyl-4-pyrrolidin-1-yl-piperidin-1-yl)-ethyl]-amin
G_CC- 2-(4-Butyl-4-dimethylamino-piperidin-1-yl)-ethyl-[2-[[1-[(4-methoxy-2,6-dimethyl-074 phenyl)sulfonyl]-piperidin-2-yl]-methoxy]-pyrimidin-4-yl]-methyl-amin
G_CC- 3-(4-Dimethylamino-4-phenyl-piperidin-1-yl)-propyl-[2-[[(2S)-1-[(4-methoxy-2,6-dimethyl-075 phenyl)sulfonyl]-pyrrolidin-2-yl]-methoxy]-pyrimidin-4-yl]-methyl-amin
G_CC- 3-(4-Dimethylamino-4-phenyl-piperidin-1-yl)-propyl-[2-[[(2S)-1-[(4-methoxy-2,6-dimethyl-076 phenyl)sulfonyl]-2,3-dihydro-1H-indol-2-yl]-methoxy]-pyrimidin-4-yl]-methyl-amin
G_CC- 2-[1-[(4-Methoxy-2,6-dimethyl-phenyl)sulfonyl]-azetidin-3-yl]oxy-4-[4-[(1-methyl-piperidin-4-077 yl)-methyl]-piperazin-1-yl]-pyrimidin
G_CC- 2-[[1-[(4-Methoxy-2,6-dimethyl-phenyl)sulfonyl]-piperidin-2-yl]-methoxy]-4-(4-pyridin-2-yloxy-078 piperidin-1-yl)-pyrimidin
G_CC- 2-[[1-[(4-Methoxy-2,6-dimethyl-phenyl)sulfonyl]-piperidin-2-yl]-methoxy]-4-(4-pyrazin-2-yloxy-079 piperidin-1-yl)-pyrimidin
G_CC- 2-[[1-[(4-Methoxy-2,6-dimethyl-phenyl)sulfonyl]-piperidin-2-yl]-methoxy]-4-[4-pyridin-3-yl-4-(3-080 pyrrolidin-1-yl-propyl)-piperidin-1-yl]-pyrimidin
G_CC- 2-[[1-[(4-Methoxy-2,6-dimethyl-phenyl)sulfonyl]-piperidin-2-yl]-methoxy]-4-[2-(pyridin-2-yl-081 methyl)-pyrrolidin-1-yl]-pyrimidin
G_CC- 1-[2-[[1-[(4-Methoxy-2,6-dimethyl-phenyl)sulfonyl]-piperidin-2-yl]-methoxy]-pyrimidin-4-yl]-4-082 pyridin-2-yl-piperidin-4-ol
G_CC- 1-[2-[[(2S)-1-[(4-Methoxy-2,6-dimethyl-phenyl)sulfonyl]-pyrrolidin-2-yl]-methoxy]-pyrimidin-4-083 yl]-4-pyridin-2-yl-piperidin-4-ol
G_CC- 2-[[(2R)-1-[(4-Methoxy-2,6-dimethyl-phenyl)sulfonyl]-pyrrolidin-2-yl]-methoxy]-4-[2-(pyridin-2-084 yl-methyl)-pyrrolidin-1-yl]-pyrimidin
G_CC- 5-[1-[2-[[(2S)-1-[(4-Methoxy-2,6-dimethyl-phenyl)sulfonyl]-pyrrolidin-2-yl]-methoxy]-pyrimidin-085 4-yl]-piperidin-4-yl]-3-pyridin-4-yl-[1,2,4]oxadiazol
G_CC- 4-[4-(3-Fluorphenyl)-4-(2-pyrrolidin-1-yl-ethoxy)-piperidin-1-yl]-2-[[(2S)-1-[(4-methoxy-2,6-086 dimethyl-phenyl)sulfonyl]-pyrrolidin-2-yl]-methoxy]-pyrimidin
G_CC- [4-Butyl-1-[2-[[(2S)-1-[(4-methoxy-2,6-dimethyl-phenyl)sulfonyl]-pyrrolidin-2-yl]-methoxy]-087 pyrimidin-4-yl]-piperidin-4-yl]-dimethyl-amin
G_CC- 2-(4-Dimethylamino-4-phenyl-piperidin-1-yl)-ethyl-[2-[[(2S)-1-[(4-methoxy-2,6-dimethyl-088 phenyl)sulfonyl]-pyrrolidin-2-yl]-methoxy]-pyrimidin-4-yl]-methyl-amin
G_CC- N-[2-[4-(4-Butyl-4-dimethylamino-piperidin-1-yl)-pyrimidin-2-yl]oxy-ethyl]-4-methoxy-N,2,6-089 trimethyl-benzolsulfonsäure amid
G_CC- [2-[[(2S)-1-[(4-Methoxy-2,6-dimethyl-phenyl)sulfonyl]-pyrrolidin-2-yl]-methoxy]-pyrimidin-4-yl]-091 methyl-[2-(4-phenyl-4-pyrrolidin-1-yl-piperidin-1-yl)-ethyl]-amin
G_CC- (2S)-1-[(4-Methoxy-2,6-dimethyl-phenyl)sulfonyl]-2-[[4-(4-pyrazin-2-yloxy-piperidin-1-yl)-092 pyrimidin-2-yl]oxy-methyl]-2,3-dihydro-1H-indol
G_CC- [4-Butyl-1-[2-[[(2S)-1-[(4-methoxy-2,6-dimethyl-phenyl)sulfonyl]-2,3-dihydro-1H-indol-2-yl]-093 methoxy]-pyrimidin-4-yl]-piperidin-4-yl]-dimethyl-amin
G_CC- 2-(4-Dimethylamino-4-phenyl-piperidin-1-yl)-ethyl-[2-[[(2S)-1-[(4-methoxy-2,6-dimethyl-094 phenyl)sulfonyl]-2,3-dihydro-1H-indol-2-yl]-methoxy]-pyrimidin-4-yl]-methyl-amin
G_CC- [2-[[(2S)-1-[(4-Methoxy-2,6-dimethyl-phenyl)sulfonyl]-2,3-dihydro-1H-indol-2-yl]-methoxy]-096 pyrimidin-4-yl]-methyl-[2-(4-phenyl-4-pyrrolidin-1-yl-piperidin-1-yl)-ethyl]-amin
G_CC- 2-(4-Butyl-4-dimethylamino-piperidin-1-yl)-ethyl-[2-[[(2S)-1-[(4-methoxy-2,6-dimethyl-097 phenyl)sulfonyl]-2,3-dihydro-1H-indol-2-yl]-methoxy]-pyrimidin-4-yl]-methyl-amin
G_CC- N-[2-[4-[2-(4-Dimethylamino-4-phenyl-piperidin-1-yl)-ethyl-methyl-amino]pyrimidin-2-yl]oxy-098 ethyl]-4-methoxy-N,2,6-trimethyl-benzolsulfonsäure amid
G_CC- N-[2-[4-[3-(4-Dimethylamino-4-phenyl-piperidin-1-yl)-propyl-methyl-amino]-pyrimidin-2-yl]oxy-100 ethyl]-4-methoxy-N,2,6-trimethyl-benzolsulfonsäure amid
G_CC- 3-(4-Dimethylamino-4-phenyl-piperidin-1-yl)-propyl-[2-[[1-[(4-methoxy-2,6-dimethyl-101 phenyl)sulfonyl]-piperidin-2-yl]-methoxy]-pyrimidin-4-yl]-methyl-amin
G_CC- 5-[1-[2-[[1-[(4-Methoxy-2,6-dimethyl-phenyl)sulfonyl]-piperidin-2-yl]-methoxy]-pyrimidin-4-yl]-103 piperidin-4-yl]-3-pyridin-4-yl-[1,2,4]oxadiazol
G_CC- 4-[4-(3-Fluorphenyl)-4-(4-methyl-piperazin-1-yl)-piperidin-1-yl]-2-[[1-[(4-methoxy-2,6-104 dimethyl-phenyl)sulfonyl]-piperidin-2-yl]-methoxy]-pyrimidin
G_CC- (1S,5R)-8-[2-[[1-[(4-Methoxy-2,6-dimethyl-phenyl)sulfonyl]-piperidin-2-yl]-methoxy]-pyrimidin-105 4-yl]-3-pyridin-3-yloxy-8-azabicyclo[3.2.1]octan
G_CC- 1-[2-[[2-[[1-[(4-Methoxy-2,6-dimethyl-phenyl)sulfonyl]-piperidin-2-yl]-methoxy]-pyrimidin-4-yl]-106 methyl-amino]-ethyl]-4-pyridin-3-yl-piperidin-4-ol
G_CC- 4-Methoxy-N,2,6-trimethyl-N-[1-phenyl-2-[4-[(1S,5R)-3-pyridin-3-yloxy-8-107 azabicyclo[3.2.1]octan-8-yl]-pyrimidin-2-yl]oxy-ethyl]-benzolsulfonsäure amid
G_CC- 4-Methoxy-N,2,6-trimethyl-N-[1-phenyl-2-[4-[4-pyridin-3-yl-4-(3-pyrrolidin-1-yl-propyl)-108 piperidin-1-yl]-pyrimidin-2-yl]oxy-ethyl]-benzolsulfonsäure amid
G_CC- 7-[2-[[(2S)-1-[(4-Methoxy-2,6-dimethyl-phenyl)sulfonyl]-pyrrolidin-2-yl]-methoxy]-pyrimidin-4-109 yl]-2-(piperidin-1-yl-methyl)-5,6,7,8-tetrahydro-imidazo[1,2-a]pyrazin
G_CC- 1-[2-[[1-[(4-Methoxy-2,6-dimethyl-phenyl)sulfonyl]-piperidin-2-yl]-methoxy]-pyrimidin-4-yl]-4-111 pyridin-4-yl-piperidin-4-of
G_CC- N-[2-[4-(4-Hydroxy-4-pyridin-4-yl-piperidin-1-yl)-pyrimidin-2-yl]oxy-1-phenyl-ethyl]-4-methoxy-112 N,2,6-trimethyl-benzolsulfonsäure amid
G_CC- [1-[2-[[1-[(4-Methoxy-2,6-dimethyl-phenyl)sulfonyl]-piperidin-2-yl]-methoxy]-pyrimidin-4-yl]-4-113 phenyl-piperidin-4-yl]-dimethyl-amin
G_CC- 2-[1-[(4-Methoxy-2,6-dimethyl-phenyl)sulfonyl]-piperidin-3-yl]oxy-4-(4-phenyl-4-pyrrolidin-1-114 yl-piperidin-1-yl)-pyrimidin
G_CC- 2-[[(2S)-1-[(4-Methoxy-2,6-dimethyl-phenyl)sulfonyl]-pyrrolidin-2-yl]-methoxy]-4-(4-phenyl-4-115 pyrrolidin-1-yl-piperidin-1-yl)-pyrimidin
G_CC- 4-Methoxy-N,2,6-trimethyl-N-[2-[4-(4-phenyl-4-pyrrolidin-1-yl-piperidin-1-yl)-pyrimidin-2-116 yl]oxy-ethyl]-benzolsulfonsäure amid
G_CC- N-[2-[4-(4-Dimethylamino-4-phenyl-piperidin-1-yl)-pyrimidin-2-yl]oxy-ethyl]-4-methoxy-N,2,6-117 trimethyl-benzolsulfonsäure amid
G_CC- N-[2-[4-(4-Dimethylamino-4-thiophen-2-yl-piperidin-1-yl)-pyrimidin-2-yl]oxy-ethyl]-4-methoxy-118 N,2,6-trimethyl-benzolsulfonsäure amid
G_CC- 4-Methoxy-N,2,6-trimethyl-N-[2-[4-[methyl-[2-(4-phenyl-4-pyrrolidin-1-yl-piperidin-1-yl)-ethyl]-119 amino]-pyrimidin-2-yl]oxy-ethyl]-benzolsulfonsäure amid
G_CC- N-[2-[4-[2-(4-Butyl-4-dimethylamino-piperidin-1-yl)-ethyl-methyl-amino]-pyrimidin-2-yl]oxy-120 ethyl]-4-methoxy-N,2,6-trimethyl-benzolsulfonsäure amid
G_CC- 2-[[1-[(4-Methoxy-2,6-dimethyl-phenyl)sulfonyl]-piperidin-2-yl]-methoxy]-4-(4-phenyl-4-121 pyrrolidin-1-yl-piperidin-1-yl)-pyrimidin
G_CC- 2-[1-[(4-Methoxy-2,6-dimethyl-phenyl)sulfonyl]-azetidin-3-yl]oxy-4-(4-phenyl-4-pyrrolidin-1-yl-122 piperidin-1-yl)-pyrimidin
G_CC- 2-(4-Dimethylamino-4-phenyl-piperidin-1-yl)-ethyl-[2-[1-[(4-methoxy-2,6-dimethyl-123 phenyl)sulfonyl]-azetidin-3-yl]oxy-pyrimidin-4-yl]-methyl-amin
G_CC- [2-[1-[(4-Methoxy-2,6-dimethyl-phenyl)sulfonyl]-azetidin-3-yl]oxy-pyrimidin-4-yl]-methyl-[2-(4-124 phenyl-4-pyrrolidin-1-yl-piperidin-1-yl)-ethyl]-amin
G_CC- [1-[2-[[(2S)-1-[(4-Methoxy-2,6-dimethyl-phenyl)sulfonyl]-2,3-dihydro-1H-indol-2-yl]-methoxy]-125 pyrimidin-4-yl]-4-thiophen-2-yl-piperidin-4-yl]-dimethyl-amin
G_CC- 3-(4-Dimethylamino-4-phenyl-piperidin-1-yl)-propyl-[2-[1-[(4-methoxy-2,6-dimethyl-126 phenyl)sulfonyl]-azetidin-3-yl]oxy-pyrimidin-4-yl]-methyl-amin
G_CC- 3-[4-[2-[1-[(4-Methoxy-2,6-dimethyl-phenyl)sulfonyl]-azetidin-3-yl]oxy-pyrimidin-4-yl]-127 piperazin-1-yl]-propyl-dimethyl-amin
G_CC- 1-[2-[1-[(4-Methoxy-2,6-dimethyl-phenyl)sulfonyl]-azetidin-3-yl]oxy-pyrimidin-4-yl]-4-pyridin-3-128 yl-piperidin-4-ol
G_CC- 2-[1-[(4-Methoxy-2,6-dimethyl-phenyl)sulfonyl]-azetidin-3-yl]oxy-4-[4-(2-piperidin-1-yl-ethyl)-129 piperidin-1-yl]-pyrimidin
G_CC- (2S)-2-[[4-[2-[(4-Fluorphenyl)-methyl]-2,5-diazabicyclo[2.2.1]heptan-5-yl]-pyrimidin-2-yl]oxy-130 methyl]-1-[(4-methoxy-2,6-dimethyl-phenyl)sulfonyl]-2,3-dihydro-1H-indol
G_CC- 4-[1-[2-[[1-[(4-Methoxy-2,6-dimethyl-phenyl)sulfonyl]-piperidin-2-yl]-methoxy]-pyrimidin-4-yl]-131 4-methyl-piperidin-4-yl]-morpholin
G_CC- 4-Methoxy-N,2,6-trimethyl-N-[1-phenyl-2-[4-[2-(pyridin-2-yl-methyl)-pyrrolidin-1-yl]-pyrimidin-133 2-yl]oxy-ethyl]-benzolsulfonsäure amid
G_CC- 4-Methoxy-N,2,6-trimethyl-N-[1-phenyl-2-[4-[4-(3-pyridin-4-yl-[1,2,4]oxadiazol-5-yl)-piperidin-134 1-yl]-pyrimidin-2-yl]oxy-ethyl]-benzolsulfonsäure amid
G_CC- N-[2-[4-[4-(3-Fluorphenyl)-4-(4-methyl-piperazin-1-yl)-piperidin-1-yl]-pyrimidin-2-yl]oxy-1-135 phenyl-ethyl]-4-methoxy-N,2,6-trimethyl-benzolsulfonsäure amid
G_CC- 4-Methoxy-N,2,6-trimethyl-N-[1-phenyl-2-[4-(4-pyridin-2-yloxy-piperidin-1-yl)-pyrimidin-2-136 yl]oxy-ethyl]-benzolsulfonsäure amid
G_CC- 4-Methoxy-N,2,6-trimethyl-N-[2-[4-(4-methyl-4-morpholin-4-yl-piperidin-1-yl)-pyrimidin-2-137 yl]oxy-1-phenyl-ethyl]-benzolsulfonsäure amid
G_CC- N-[2-[4-[2-(4-Hydroxy-4-pyridin-3-yl-piperidin-1-yl)-ethyl-methyl-amino]-pyrimidin-2-yl]oxy-1-138 phenyl-ethyl]-4-methoxy-N,2,6-trimethyl-benzolsulfonsäure amid
[H_CC- 4-[[1-(4-Methoxy-2,6-dimethylphenyl)sulfonyl-2-piperidinyl]methoxy]-2-[4-(1-methyl-4-001] piperidinyl)-1-piperazinyl]pyrimidin
[H_CC- 4-[[1-(4-Methoxy-2,6-dimethylphenyl)sulfonyl-2-piperidinyl]methoxy]-2-[4-(2-1-002] pyrrolidinylethyl)-1-piperidinyl]pyrimidin
[H_CC- 4-[[1-(4-Methoxy-2,6-dimethylphenyl)sulfonyl-2-piperidinyl]methoxy]-2-(4-methyl-1-003] piperazinyl)pyrimidin
[H_CC- 4-[[1-(4-Methoxy-2,6-dimethylphenyl)sulfonyl-2-piperidinyl]methoxy]-2-[4-(2-pyrimidinyl)-1-004] piperazinyl]pyrimidin
**[H_CC-** 4-[[1-(4-Methoxy-2,6-dimethylphenyl)sulfonyl-2-piperidinyl]methoxy]-2-[4-(4-pyridyl)-1-005] piperazinyl]pyrimidin
**[H_CC-** 4-[[1-(4-Methoxy-2,6-dimethylphenyl)sulfonyl-2-piperidinyl]methoxy]-2-[4-[(1-methyl-4-006] piperidinyl)methyl]-1-piperazinyl]pyrimidin
[H_CC- 1-[4-[[1-(4-Methoxy-2,6-dimethylphenyl)sulfonyl-2-piperidinyl]methoxy]-2-pymidinyl]-4-(3-007] pyridyl)-4-piperidinol
**[H_CC-** 4-[[(2S)-1-(4-Methoxy-2,6-dimethylphenyl)sulfonyl-2-pyrrolidinyl]methoxy]-2-(4-methyl-1-008] piperazinyl)pyrimidin
[H_CC- 4-[[(2S)-1-(4-Methoxy-2,6-dimethylphenyl)sulfonyl-2-pyrrolidinyl]methoxy]-2-[4-(2-1-009] pyrrolidinylethyl)-1-piperidinyl]pyrimidin
[H_CC- 4-[[(2S)-1-(4-Methoxy-2,6-dimethylphenyl)sulfonyl-2-pyrrolidinyl]methoxy]-2-[4-(4-methyl-1-010] piperazinyl)-1-piperidinyl]pyrimidin
[H_CC- 4-[[(2S)-1-(4-Methoxy-2,6-dimethylphenyl)sulfonyl-2-pyrrolidinyl]methoxy]-2-[4-(2-pyrimidinyl)-011] 1-piperazinyl]pyrimidin
[H_CC- 4-[[(2S)-1-(4-Methoxy-2,6-dimethylphenyl)sulfonyl-2-pyrrolidinyl]methoxy]-2-[4-[(1-methyl-4-012] piperidinyl)methyl]-1-piperazinyl]pyrimidin
[H_CC- 1-[4-[[(2S)-1-(4-Methoxy-2,6-dimethylphenyl)sulfonyl-2-pyrrolidinyl]methoxy]-2-pyrimidinyl]-4-013] (3-pyridyl)-4-piperidinol
[H_CC- 4-[[1-(4-Methoxy-2,6-dimethylphenyl)sulfonyl-3-azetidinyl]oxy]-2-[4-(2-1-pyrrolidinylethyl)-1-014] piperidinyl]pyrimidin
[H_CC- 4-[[1-(4-Methoxy-2,6-dimethylphenyl)sulfonyl-3-azetidinyl]oxy]-2-[4-(4-methyl-1-piperazinyl)-1-015] piperidinyl]pyrimidin
[H_CC- (2S)-1-(4-Methoxy-2,6-dimethylphenyl)sulfonyl-2-[[2-(4-methyl-1-piperazinyl)-4-016] pyrimidinyl]oxymethyl]indolin
[H_CC- (2S)-1-(4-Methoxy-2,6-dimethylphenyl)sulfonyl-2-[[2-[4-(2-1-pyrrolidinylethyl)-1-piperidinyl]-4-017] pyrimidinyl]oxymethyl]indolin
[H_CC- (2S)-1-(4-Methoxy-2,6-dimethylphenyl)sulfonyl-2-[[2-[4-(4-methyl-1-piperazinyl]-1-piperidinyl]-018] 4-pyrimidinyl]oxymethyl]indolin
[H_CC- (2S)-1-(4-Methoxy-2,6-dimethylphenyl)sulfonyl-2-[[2-[4-(4-pyridyl)-1-piperazinyl]-4-019] pyrimidinyl]oxymethyl]indolin
[H_CC- (2S)-1-(4-Methoxy-2,6-dimethylphenyl)sulfonyl-2-[[2-[4-[(1-methyl-4-piperidinyl)methyl]-1-020] piperazinyl]-4-pyrimidinyl]oxymethyl]indolin
[H_CC- 3-Benzyl-7-[4-[[1-(4-methoxy-2,6-dimethylphenyl)sulfonyl-2-piperidinyl]methoxy]-2-021] pyrimidinyl]-3,7-diazaspiro[4.4]nonan
[H_CC- 4-[[1-(4-Methoxy-2,6-dimethylphenyl)sulfonyl-2-piperidinyl]methoxy]-2-[4-[2-(1-piperidyl)ethyl]-022] 1-piperidinyl]pyrimidin
[H_CC- 3-Benzyl-7-[4-[[(2S)-1-(4-methoxy-2,6-dimethylphenyl)sulfonyl-2-pyrrolidinyl]methoxy]-2-023] pyrimidinyl]-3,7-diazaspiro[4.4]nonan
[H_CC- 8-[4-[[(2S)-1-(4-Methoxy-2,6-dimethylphenyl)sulfonyl-2-pyrrolidinyl]methoxy]-2-pyrimidinyl]-4-024] phenyl-2,4,8-triazaspiro[4.5]decan-1-on
[H_CC- 4-[[(2S)-1-(4-Methoxy-2,6-dimethylphenyl)sulfonyl-2-pyrrolidinyl]methoxy]-2-[4-(1-methyl-4-025] piperidinyl)-1-piperazinyl]pyrimidin
[H_CC- 4-[[(2S)-1-(4-Methoxy-2,6-dimethylphenyl)sulfonyl-2-pyrrolidinyl]methoxy]-2-[4-[2-(1-026] piperidyl)ethyl]-1-piperidinyl]pyrimidin
[H_CC- (2S)-1-(4-Methoxy-2,6-dimethylphenyl)sulfonyl-2-[[2-[4-(1-methyl-4-piperidinyl)-1-piperazinyl]-027] 4-pyrimidinyl]oxymethyl]indolin
[H_CC- (2S)-1-(4-Methoxy-2,6-dimethylphenyl)sulfonyl-2-[[2-[4-[2-(1-piperidyl)ethyl]-1-piperidinyl]-4-028] pyrimidinyl]oxymethyl]indolin
[H_CC- 4-Methoxy-N,2,6-trimethyl-N-[2-[[2-[3-(4-pyridyl)-3,9-diazaspiro[5.5]undecan-9-yl]-4-029] pyrimidinyl]oxy]ethyl]benzolsulfonamid
[H_CC- 3-[(4-Fluorphenyl)methyl]-8-[4-[(1-(4-methoxy-2,6-dimethylphenyl)sulfonyl-2-030] piperidinyl]methoxy]-2-pyrimidinyl]-3,8-diazaspiro[4.5]decan-4-on
[H_CC- N-[[1-[4-[[1-(4-Methoxy-2,6-dimethylphenyl)sulfonyl-2-piperidinyl)methoxy]-2-pyrimidinyl)-4-(4-031] methyl-1-piperazinyl)-4-piperidinyl]methyl]-4-pyridincarboxamid
[H_CC- 9-[4-[[1-(4-Methoxy-2,6-dimethylphenyl)sulfonyl-2-piperidinyl]methoxy)-2-pyrimidinyl]-3-(4-032] ]pyridyl)-3,9-diazaspiro[5.5]undecan
[H_CC- 4-Methoxy-N,2,6-trimethyl-N-[2-[[2-[4-(3-pyridyl)-4-(2-1-pyrrolidinylethoxy)-1-piperidinyl]-4-033] pyrimidinyl)oxy]ethyl]benzolsulfonamid
[H_CC- 4-Methoxy-N,2,6-trimethyl-N-[2-[[2-[2-(4-pyridylmethyl)-2,5-diazabicyclo[2.2.1]heptan-5-yl]-4-034] pyrimidinyl]oxy]ethyl]benzolsulfonamid
[H_CC- 4-[[1-(4-Methoxy-2,6-dimethylphenyl)sulfonyl-2-piperidinyl]methoxy]-2-[4-(3-pyridyl)-4-(2-1-035] pyrrolidinylethoxy)-1-piperidinyl]pyrimidin
[H_CC- 4-Methoxy-N,2,6-trimethyl-N-[1-phenyl-2-[[2-[4-(4-pyridyl)-1-piperazinyl]-4-036] pyrimidinyl]oxy]ethyl]benzolsulfonamid
[H_CC- 4-Methoxy-N,2,6-trimethyl-N-[1-phenyl-2-[[2-[3-(4-pyridyl)-3,9-diazaspiro[5.5]undecan-9-yl]-4-037] pyrimidinyl]oxy]ethyl]benzolsulfonamid
[H_CC- N-Methyl-N-[1-phenyl-2-[[2-[4-(3-pyridyl)-4-(2-1-pyrrolidinylethoxy)-1-piperidinyl]-4-038] pyrimidinyl]oxy]ethyl]-2-naphthalinsulfonamid
[H_CC- N-[2-[[2-[3-[(4-Fluorphenyl)methyl]-4-oxo-3,8-diazaspiro[4.5]decan-8-yl]-4-039] pyrimidinyl]oxy]ethyl]-4-methoxy-N,2,6-trimethylbenzolsulfonamid
[H_CC- 4-[[(2S)-1-(4-Methoxy-2,6-dimethylphenyl)sulfonyl-2-pyrrolidinyl]methoxy]-2-[4-(3-pyridyl)-4-040] (2-1-pyrrolidinylethoxy)-1-piperidinyl]pyrimidin
[H_CC- 4-[[1-(4-Methoxy-2,6-dimethylphenyl)sulfonyl-3-piperidinyl]oxy]-2-[4-[2-(1-piperidyl)ethyl]-1- - 041] piperidinyl]pyrimidin
H_CC- 1-[4-[[1-[(4-Methoxy-2,6-dimethyl-phenyl)sulfonyl]-piperidin-2-yl]-methoxy]-pyrimidin-2-yl]-4-042 (pyridin-2-yl-methyl)-[1,4]diazepan
H_CC- 1-[4-[[(2R)-1-[(4-Methoxy-2,6-dimethyl-phenyl)sulfonyl]-pyrrolidin-2-yl]-methoxy]-pyrimidin-2-043 yl]-4-(pyridin-2-yl-methyl)-[1,4]diazepan
H_CC- 2-(4-Dimethylamino-4-phenyl-piperidin-1-yl)-ethyl-[4-[[1-[(4-methoxy-2,6-dimethyl-044 phenyl)sulfonyl]-piperidin-2-yl]-methoxy]-pyrimidin-2-yl]-methyl-amin
H_CC- 2-(4-Butyl-4-dimethylamino-piperidin-1-yl)-ethyl-[4-[[1-[(4-methoxy-2,6-dimethyl-045 phenyl)sulfonyl]-piperidin-2-yl]-methoxy]-pyrimidin-2-yl]-methyl-amin
H_CC- 3-(4-Dimethylamino-4-phenyl-piperidin-1-yl)-propyl-[4-[[(2S)-1-[(4-methoxy-2,6-dimethyl-046 phenyl)sulfonyl]-pyrrolidin-2-yl]-methoxy]-pyrimidin-2-yl]-methyl-amin
H_CC- 1-[4-[[(2S)-1-[(4-Methoxy-2,6-dimethyl-phenyl)sulfonyl]-pyrrolidin-2-yl]-methoxy]-pyrimidin-2-047 yl]-4-pyridin-2-yl-piperidin-4-ol
H_CC- 5-[1-[4-[[(2S)-1-[(4-Methoxy-2,6-dimethyl-phenyl)sulfonyl]-pyrrolidin-2-yl]-methoxy]-pyrimidin-048 2-yl]-piperidin-4-yl]-3-pyridin-4-yl-[1,2,4]oxadiazol
H_CC- [4-[[1-[(4-Methoxy-2,6-dimethyl-phenyl)sulfonyl]-piperidin-2-yl]-methoxy]-pyrimidin-2-yl]-049 methyl-[2-(4-pyridin-4-yloxy-piperidin-1-yl)-ethyl]-amin
H_CC- (1S,5R)-8-[4-[[1-[(4-Methoxy-2,6-dimethyl-phenyl)sulfonyl]-piperidin-2-yl]-methoxy]-pyrimidin-050 2-yl]-3-pyridin-4-yloxy-8-azabicyclo[3.2.1]octan
H_CC- [4-[[(2S)-1-[(4-Methoxy-2,6-dimethyl-phenyl)sulfonyl]-pyrrolidin-2-yl]-methoxy]-pyrimidin-2-yl]-051 methyl-[2-(4-pyridin-4-yloxy-piperidin-1-yl)-ethyl]-amin
H_CC- (1 S,5R)-8-[4-[[(2S)-1-[(4-Methoxy-2,6-dimethyl-phenyl)sulfonyl]-pyrrolidin-2-yl]-methoxy]-052 pyrimidin-2-yl]-3-pyridin-4-yloxy-8-azabicyclo[3.2.1]octan
H_CC- 2-(4-Butyl-4-dimethylamino-piperidin-1-yl)-ethyl-[4-[1-[(4-methoxy-2,6-dimethyl-053 phenyl)sulfonyl]-piperidin-3-yl]oxy-pyrimidin-2-yl]-methyl-amin
H_CC- 2-(4-Dimethylamino-4-phenyl-piperidin-1-yl)-ethyl-[4-[[(2S)-1-[(4-methoxy-2,6-dimethyl-054 phenyl)sulfonyl]-pyrrolidin-2-yl]-methoxy]-pyrimidin-2-yl]-methyl-amin
H_CC- [4-[[(2S)-1-[(4-Methoxy-2,6-dimethyl-phenyl)sulfonyl]-pyrrolidin-2-yl]-methoxy]-pyrimidin-2-yl]-055 methyl-[2-(4-phenyl-4-pyrrolidin-1-yl-piperidin-1-yl)-ethyl]-amin
H_CC- 2-(4-Butyl-4-dimethylamino-piperidin-1-yl)-ethyl-[4-[[(2S)-1-[(4-methoxy-2,6-dimethyl-056 phenyl)sulfonyl]-pyrrolidin-2-yl]-methoxy]-pyrimidin-2-yl]-methyl-amin
H_CC- 2-(4-Dimethylamino-4-phenyl-piperidin-1-yl)-ethyl-[4-[[(2S)-1-[(4-methoxy-2,6-dimethyl-057 phenyl)sulfonyl]-2,3-dihydro-1H-indol-2-yl]-methoxy]-pyrimidin-2-yl]-methyl-amin
H_CC- 2-(4-Butyl-4-dimethylamino-piperidin-1-yl)-ethyl-[4-[[(2S)-1-[(4-methoxy-2,6-dimethyl-058 phenyl)sulfonyl]-2,3-dihydro-1H-indol-2-yl]-methoxy]-pyrimidin-2-yl]-methyl-amin
H_CC- 4-Methoxy-N,2,6-trimethyl-N-[2-[2-[methyl-[2-(4-phenyl-4-pyrrolidin-1-yl-piperidin-1-yl)-ethyl]-059 amino]-pyrimidin-4-yl]oxy-ethyl]-benzolsulfonsäure amid
H_CC- N-[2-[2-[2-(4-Butyl-4-dimethylamino-piperidin-1-yl)-ethyl-methyl-amino]-pyrimidin-4-yl]oxy-060 ethyl]-4-methoxy-N,2,6-trimethyl-benzolsulfonsäure amid
H_CC- 3-(4-Dimethylamino-4-phenyl-piperidin-1-yl)-propyl-[4-[[1-[(4-methoxy-2,6-dimethyl-061 phenyl)sulfonyl]-piperidin-2-yl]-methoxy]-pyrimidin-2-yl]-methyl-amin
H_CC- [4-Butyl-1-[4-[[1-[(4-methoxy-2,6-dimethyl-phenyl)sulfonyl]-piperidin-2-yl]-methoxy]-pyrimidin-062 2-yl]-piperidin-4-yl]-dimethyl-amin
H_CC- [4-[[1-[(4-Methoxy-2,6-dimethyl-phenyl)sulfonyl]-piperidin-2-yl]-methoxy]-pyrimidin-2-yl]-063 methyl-[2-(4-phenyl-4-pyrrolidin-1-yl-piperidin-1-yl)-ethyl]-amin
H_CC- [1-[4-[1-[(4-Methoxy-2,6-dimethyl-phenyl)sulfonyl]-piperidin-3-yl]oxy-pyrimidin-2-yl]-4-phenyl-064 piperidin-4-yl]-dimethyl-amin
H_CC- 2-(4-Dimethylamino-4-phenyl-piperidin-1-yl)-ethyl-[4-[1-[(4-methoxy-2,6-dimethyl-065 phenyl)sulfonyl]-piperidin-3-yl]oxy-pyrimidin-2-yl]-methyl-amin
H_CC- [4-[1-[(4-Methoxy-2,6-dimethyl-phenyl)sulfonyl]-piperidin-3-yl]oxy-pyrimidin-2-yl]-methyl-[2-(4-066 phenyl-4-pyrrolidin-1-yl-piperidin-1-yl)-ethyl]-amin
H_CC- N-[2-[2-[2-(4-Dimethylamino-4-phenyl-piperidin-1-yl)-ethyl-methyl-amino]-pyrimidin-4-yl]oxy-067 ethyl]-4-methoxy-N,2,6-trimethyl-benzolsulfonsäure amid
H_CC- [4-Butyl-1-[4-[[(2S)-1-[(4-methoxy-2,6-dimethyl-phenyl)sulfonyl]-pyrrolidin-2-yl]-methoxy]-068 pyrimidin-2-yl]-piperidin-4-yl]-dimethyl-amin
H_CC- 2-(4-Dimethylamino-4-phenyl-piperidin-1-yl)-ethyl-[4-[1-[(4-methoxy-2,6-dimethyl-069 phenyl)sulfonyl]-azetidin-3-yl]oxy-pyrimidin-2-yl]-methyl-amin
H_CC- [4-[1-[(4-Methoxy-2,6-dimethyl-phenyl)sulfonyll-azetidin-3-yl]oxy-pyrimidin-2-yl]-methyl-[2-(4-070 phenyl-4-pyrrolidin-1-yl-piperidin-1-yl)-ethyl]-amin
H_CC- 2-(4-Butyl-4-dimethylamino-piperidin-1-yl)-ethyl-[4-[1-[(4-methoxy-2,6-dimethyl-071 phenyl)sulfonyl]-azetidin-3-yl]oxy-pyrimidin-2-yl]-methyl-amin
H_CC- [4-[[(2S)-1-[(4-Methoxy-2,6-dimethyl-phenyl)sulfonyl]-2,3-dihydro-1H-indol-2-yl]-methoxy]-072 pyrimidin-2-yl]-methyl-[2-(4-phenyl-4-pyrrolidin-1-yl-piperidin-1-yl)-ethyl]-amin
H_CC- 3-(4-Dimethylamino-4-phenyl-piperidin-1-yl)-propyl-[4-[1-[(4-methoxy-2,6-dimethyl-073 phenyl)sulfonyl]-piperidin-3-yl]oxy-pyrimidin-2-yl]-methyl-amin
H_CC- 3-(4-Dimethylamino-4-phenyl-piperidin-1 -yl)-propyl-[4-[1-[(4-methoxy-2,6-dimethyl-074 phenyl)sulfonyl]-azetidin-3-yl]oxy-pyrimidin-2-yl]-methyl-amin
H_CC- 3-(4-Dimethylamino-4-phenyl-piperidin-1-yl)-propyl-[4-[[(2S)-1-[(4-methoxy-2,6-dimethyl-075 phenyl)sulfonyl]-2,3-dihydro-1H-indol-2-yl]-methoxy]-pyrimidin-2-yl]-methyl-amin
H_CC- 1-[4-[1-[(4-Methoxy-2,6-dimethyl-phenyl)sulfonyl]-azetidin-3-yl]oxy-pyrimidin-2-yl]-4-pyridin-3-076 yl-piperidin-4-ol
H_CC- 1-[4-[1-[(4-Methoxy-2,6-dimethyl-phenyl)sulfonyl]-azetidin-3-yl]oxy-pyrimidin-2-yl]-4-(pyridin-2-077 yl-methyl)-[1,4]diazepan
H_CC- 4-[[1-[(4-Methoxy-2,6-dimethyl-phenyl)sulfonyl]-piperidin-2-yl]-methoxy]-2-[2-(pyridin-2-yl-078 methyl)-pyrrolidin-1-yl]-pyrimidin
H_CC- 4-[[1-[(4-Methoxy-2,6-dimethyl-phenyl)sulfonyl]-piperidin-2-yl]-methoxy]-2-(4-pyridin-2-yloxy-079 piperidin-1-yl)-pyrimidin
H_CC- 4-[[1-[(4-Methoxy-2,6-dimethyl-phenyl)sulfonyl]-piperidin-2-yl]-methoxy]-2-(4-pyrazin-2-yloxy-080 piperidin-1-yl)-pyrimidin
H_CC- 4-[1-[4-[[1-[(4-Methoxy-2,6-dimethyl-phenyl)sulfonyl]-piperidin-2-yl]-methoxy]-pyrimidin-2-yl]-4-081 methyl-piperidin-4-yl]-morpholin
H_CC- 4-[[1-[(4-Methoxy-2,6-dimethy,-phenyl)sulfonyl]-piperidin-2-yl]-methoxy]-2-[4-pyridin-3-yl-4-(3-082 pyrrolidin-1-yl-propyl)-piperidin-1-yl]pyrimidin
H_CC- 1-[4-[[1-[(4-Methoxy-2,6-dimethyl-phenyl)sulfonyl]-piperidin-2-yl]-methoxy]-pyrimidin-2-yl]-4-083 pyridin-2-yl-piperidin-4-ol
H_CC- 4-[[(2R)-1-[(4-Methoxy-2,6-dimethyl-phenyl)sulfonyl]-pyrrolidin-2-yl]-methoxy]-2-[2-(pyridin-2-084 yl-methyl)-pyrrolidin-1-yl]pyrimidin
H_CC- [1-[4-[[(2S)-1-[(4-Methoxy-2,6-dimethyl-phenyl)sulfonyl]-pyrrolidin-2-yl]-methoxy]-pyrimidin-2-085 yl]-4-phenyl-piperidin-4-yl]-dimethyl-amin
H_CC- [1-[4-[[(2S)-1-[(4-Methoxy-2,6-dimethyl-phenyl)sulfonyl]-pyrrolidin-2-yl]-methoxy]-pyrimidin-2-086 yl]-4-thiophen-2-yl-piperidin-4-yl]-dimethyl-amin
H_CC- 4-Methoxy-N,2,6-trimethyl-N-[2-[2-[2-(pyridin-2-yl-methyl)-pyrrolidin-1-yl]-pyrimidin-4-yl]oxy-087 ethyl]-benzotsulfonsäure amid
H_CC- 4-[[(2S)-1-[(4-Methoxy-2,6-dimethyl-phenyl)sulfonyl]-pyrrolidin-2-yl]-methoxy]-2-[2-(pyridin-2-088 yl-methyl)-pyrrolidin-1-yl]-pyrimidin
H_CC- 4-Methoxy-N,2,6-trimethyl-N-[2-[2-[methyl-[2-(4-pyridin-4-yloxy-piperidin-1-yl)-ethyl]-amino]-089 pyrimidin-4-yl]oxy-ethyl]-benzolsulfonsäure amid
H_CC- N-[2-[2-[4-(3-Fluorphenyl)-4-(2-pyrrolidin-1-yl-ethoxy)-piperidin-1-yl]pyrimidin-4-yl]oxy-ethyl]-090 4-methoxy-N,2,6-trimethyl-benzolsulfonsäure amid
H_CC- 2-[4-(3-Fluorphenyl)-4-(4-methyl-piperazin-1-yl)-piperidin-1-yl]-4-[[1-[(4-methoxy-2,6-dimethyl-091 phenyl)sulfonyl]-piperidin-2-yl]-methoxy]-pyrimidin
H_CC- 2-[4-(3-Fluorphenyl)-4-(2-pyrrolidin-1-yl-ethoxy)-piperidin-1-yl]-4-[[(2S)-1-[(4-methoxy-2,6-092 dimethyl-phenyl)sulfonyl]-pyrrolidin-2-yl]-methoxy]-pyrimidin
H_CC- 4-Methoxy-N,2,6-trimethyl-N-[1-phenyl-2-[2-[(1S,5R)-3-pyridin-4-yloxy-8-093 azabicyclo[3.2.1]octan-8-yl]-pyrimidin-4-yl]oxy-ethyl]-benzolsulfonsäure amid

Die oben verwendete Nummerierung der einzelnen Ausführungsformen der erfindungsgemäßen Verbindungen wird in den nachfolgenden Erläuterungen der vorliegenden Erfindung, insbesondere in der Beschreibung der Beispiele, beibehalten.

Gemäß einem Aspekt der vorliegenden Erfindung weisen die erfindungsgemäßen Verbindungen vorzugweise eine antagonistische Wirkung am humanen B1 R-Rezeptor oder dem B1R-Rezeptor der Ratte auf. In einer bevorzugten Ausführungsform der Erfindung weisen die erfindungsgemäßen Verbindungen eine antagonistische Wirkung sowohl am humanen B1 R- Rezeptor (hB1 R) als auch am B1R-Rezeptor der Ratte (rB1 R) auf.

In einer bevorzugten Ausführungsform der vorliegenden Erfindung weisen die erfindungsgemäßen Verbindungen im FLIPR-Assay bei einer Konzentration von 10 µM am humanen B1 R-Rezeptor und/ oder am B1 R-Rezeptor der Ratte eine Inhibition von mindestens 15%, 25%, 50%, 70%, 80% oder 90 % auf. Ganz besonders bevorzugt sind Verbindungen, die eine Inhibition am humanen B1 R-Rezeptor und am B1 R-Rezeptor der Ratte von mindestens 70%, insbesondere von mindestens 80% und insbesondere bevorzugt von mindestens 90% bei einer Konzentration von 10 µM aufweisen.

Die agonistische bzw. antagonistische Wirkung von Substanzen kann am Bradykinin Rezeptor 1 (B1R) der Spezies Mensch und Ratte mit ektopisch exprimierenden Zelllinien (CHO K1 Zellen) und mit Hilfe eines Ca²⁺-sensitiven Farbstoffs (Fluo-4) im Fluorescent Imaging Plate Reader (FLIPR) quantifiziert werden. Die Angabe in % Aktivierung wird bezogen auf das Ca²⁺-Signal nach Zugabe von Lys-Des-Arg⁹-Bradykinin (0,5 nM), bzw. Des-Arg⁹-Bradykinin (100 nM). Antagonisten führen zu einer Unterdrückung des Ca²⁺-Einstroms nach der Zugabe des Agonisten. Angegeben werden % Inhibition im Vergleich zu der maximal erreichbaren Inhibition.

Vorzugsweise wirken die erfindungsgemäßen Substanzen beispielsweise auf den im Zusammenhang mit verschiedenen Erkrankungen relevanten B1R, sodass sie sich als pharmazeutischer Wirkstoff in Arzneimitteln eignen. Ein weiterer Gegenstand der Erfindung sind daher Arzneimittel enthaltend wenigstens ein erfindungsgemäßes substituiertes Pyrimidin- und/oder Triazin-Derivat, sowie gegebenenfalls geeignete Zusatz- und/oder Hilfsstoffe und/ oder gegebenenfalls weitere Wirkstoffe.

Die erfindungsgemäßen Arzneimittel enthalten neben mindestens einem erfindungsgemäßen substituierten Pyrimidin- und/oder Triazin-Derivat gegebenenfalls geeignete Zusatz- und/ oder Hilfsstoffe, so auch Trägermaterialien, Füllstoffe, Lösungsmittel, Verdünnungsmittel, Farbstoffe und/oder Bindemittel und können als flüssige Arzneiformen in Form von Injektionslösungen, Tropfen oder Säfte, als halbfeste Arzneiformen in Form von Granulaten, Tabletten, Pellets, Patches, Kapseln, Pflaster/ Sprühpflaster oder Aerosolen verabreicht werden. Die Auswahl der Hilfsstoffe etc. sowie die einzusetzenden Mengen derselben hängen davon ab, ob das Arzneimittel oral, peroral, parenteral, intravenös, intraperitoneal, intradermal, intramuskulär, nasal, buccal, rektal oder topisch, zum Beispiel auf die Haut, die Schleimhäute oder in die Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße substituierte Pyrimidin- und/oder Triazine in einem Depot, in gelöster Form oder in einem Pflaster, gegebenenfalls unter Zusatz von die Haupenetration fördernden Mitteln, sind geeignete perkutane Applikationszubereitungen. Oral oder perkutan anwendbare Zubereitungsformen können die erfindungsgemäßen substituierten Pyrimidin- und/oder Triazin-Derivate verzögert freisetzen. Die erfindungsgemäßen substituierten Pyrimindin- und/oder Triazin-Derivate können auch in parenteralen Langzeitdepotformen wie z. B. Implantaten oder implantierten Pumpen angewendet werden. Prinzipiell können den erfindungsgemäßen Arzneimitteln andere dem Fachmann bekannte weitere Wirkstoffe zugesetzt werden.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 0,00005 bis 50 mg/kg, bevorzugt 0,01 bis 5 mg/kg wenigstens eines erfindungsgemäßen substituierten Pyrimidin- und/oder Triazin-Derivats appliziert. In einer bevorzugten Form des Arzneimittels liegt ein enthaltenes erfindungsgemäßes substituiertes Pyrimidin- und/oder Triazin-Derivat als reines Diastereomer und/ oder Enantiomer, als Razemat oder als nicht-äquimolare oder äquimolare Mischung der Diastereomere und/ oder Enantiomere vor.

B1 R ist insbesondere am Schmerzgeschehen beteiligt. Entsprechend können die erfindungsgemäßen substituierten Pyrimidin- und/oder Triazin-Derivate zur Herstellung eines Arzneimittels zur Behandlung von Schmerz, insbesondere von akutem, viszeralem, neuropathischem oder chronischem Schmerz, verwendet werden.

Ein weiterer Gegenstand der Erfindung ist ein Arzneimittel enthaltend zumindest eines der erfindungsgemäßen substituierten Pyrimidin- und/oder Triazin-Derivate.

Ebenso ist die Verwendung der erfindungsgemäßen substituierten Pyrimidin- und/oder Triazin-Derivate als Medikament ein Gegenstand der Erfindung.

Ein weiterer Gegenstand der Erfindung ist daher die Verwendung eines erfindungsgemäßen substituierten Pyrimidin- und/oder Triazin-Derivats zur Herstellung eines Arzneimittels zur Behandlung von Schmerz, insbesondere von akutem, viszeralem, neuropathischem oder chronischem Schmerz. Eine bestimmte Ausführungsform der vorliegenden Erfindung ist die Verwendung zumindest eines der erfindungsgemäßen Substituierten Pyrimidin- und/oder Triazin-Derivats zur Herstellung eines Arzneimittels zur Behandlung von Entzündungsschmerz.

Ein weiterer Gegenstand der Erfindung ist die Verwendung eines erfindungsgemäßen substituierten Pyrimidin- und/oder Triazin-Derivats zur Herstellung eines Arzneimittels zur Behandlung von Diabetes, Erkrankungen der Atemwege, zum Beispiel Asthma bronchiale, Allergien, COPD/chronic-obstruktive pulmonary disease oder zystische Fibrose; entzündlichen Darmerkrankungen, beispielsweise Ulcerative Colitis oder CD/Crohn's disease; neurologischen Erkrankungen, beispielsweise Multiple Sklerose oder Neurodegeneration; Entzündungen der Haut, beispielsweise atopische Dermatitis, Psoriasis oder bakterielle Infektionen; rheumatischen Erkrankungen, beispielsweise rheumatoide Arthritis oder Osteoarthritis; septischem Schock; Reperfusionssyndrom, zum Beispiel nach Herzinfarkt oder Schlaganfall, Fettleibigkeit; und als Angiognese-Inhibitor.

Dabei kann es in einer der vorstehenden Verwendungen bevorzugt sein, wenn ein verwendetes substituiertes Pyrimidin- und/oder Triazin-Derivat als reines Diastereomer und/ oder Enantiomer, als Razemat oder als nicht-äquimolare oder äquimolare Mischung der Diastereomere und/ oder Enantiomere vorliegt.

Ein weiterer Gegenstand der Erfindung ist ein Verfahren zur Behandlung, insbesondere in einer der vorgenannten Indikationen, eines nichthumanen Säugetieres oder Menschen, das oder der eine Behandlung von Schmerzen, insbesondere chronischer Schmerzen, benötigt, durch Verabreichung einer therapeutisch wirksamen Dosis eines erfindungsgemäßen substituierten Pyrimidin- und/oder Triazin-Derivats, oder eines erfindungsgemäßen Arzneimittels.

Ein weiterer Gegenstand der Erfindung ist ein Verfahren zur Herstellung der erfindungsgemäßen substituierten Pyrimidin- und/oder Triazin-Derivats, insbesondere wie in der folgenden Beschreibung, Beispielen und Ansprüchen ausgeführt.

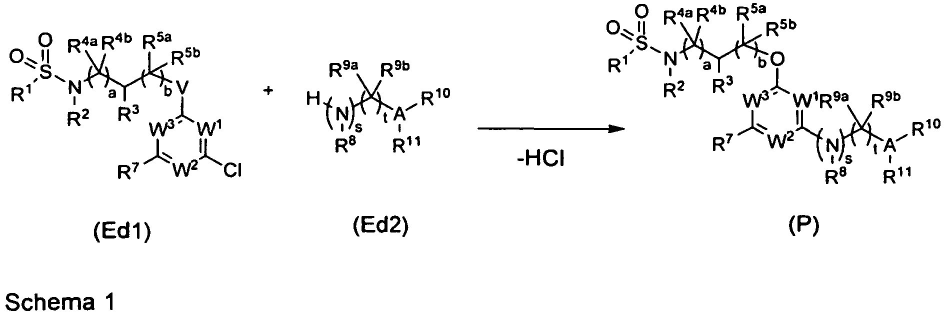

Das erfindungsgemäße Verfahren ist in Schema 1 dargestellt. Dabei wird zumindest eine Verbindung der allgemeinen Struktur Ed1 in Gegenwart eines Lösungsmittels sowie einer Base mit einer Verbindung der allgemeinen Struktur Ed2 zu den erfindungsgemäßen Produkten P umgesetzt. Besonders geeignete Lösemittel, Basen sowie sonstige Reaktionsbedingungen sind nachfolgend im Zusammenhang mit Schritt 3 der in Schema 2 gezeigten besonderen Ausführungsform des erfindungsgemäßen Verfahrens beschrieben.

Im Folgenden wird die Erfindung anhand von Beispielen erläutert, ohne den allgemeinen Erfindungsgedanken einzuschränken

### Allgemeine Syntheseverfahren

In den nachfolgenden Beispielen werden folgende Abkürzungen verwendet:
AAV = Allgemeine Arbeitsvorschrift
Äquiv. = Äquivalent
Boc = tert.-Butyloxycarbonyl
Bu = Butyl
Cbz = Benzyloxycarbonyl
DC = Dünnschichtchromatographie
DCM = Dichlormethan
Et = Ethyl
EtOAc = Ethylacetat
IPA = Isopropylamin
LAH = Lithiumaluminiumhydrid
LC = Flüssigchromatographie
LC-Ms = Flüssigchromatographie-Massenspektrometrie
Me = Methyl
THF = Tetrahydrofuran

Dem Fachmann ist ersichtlich, dass in einigen Fällen die Abfolge der Reaktionschritte ggf. abgeändert werden kann.

Die Trennung von Diastereomeren und/oder Enantiomeren erfolgt nach üblichen, dem Fachmann bekannten Methoden, beispielsweise durch Umkristallisation, Chromatographie oder insbesondere HPLC-Chromatographie bzw. Kristallisation mit einer ggf. chiralen Säure oder Base und Trennung der Salze oder chirale HPLC-Chromatographie (Fogassy et al., Optical Resolution Methods, Org. Biomol. Chem 2006, 4, 3011-3030).

Die eingesetzten Chemikalien und Lösungsmittel wurden kommerziell bei den herkömmlichen Anbietern (z.B. Acros, Avocado, Aldrich, Bachem, Fluka, Lancaster, Maybridge, Merck, Sigma, TCI, etc.) bezogen, nach den für den Fachmann bekannten Methoden oder den nachfolgend beschriebenen Verfahren synthetisiert.

Als stationäre Phase für die Säulenchromatographie wurden kommerziell erhältliche Materialien, beispielsweise Al₂O₃ oder Kieselgel eingesetzt [beispielsweise von der Firma E. Merck, Darmstadt, Deutschland]. Die dünnschicht-chromatographischen Untersuchungen wurden mit kommerziell erhältlichen HPTLC-Fertigplatten (beispielsweise Kieselgel 60 F 254, der Firma E. Merck, Darmstadt) durchgeführt. Die Mischungsverhältnisse von Lösemitteln, Laufmitteln oder für chromatographische Untersuchungen sind, insofern nicht anders angezeigt, stets in VolumenNolumen angegeben.

Die Analytik erfolgte soweit nicht anders angegeben durch Massenspektroskopie (ESI-MS).

### Allgemeine Verfahren zur Darstellung der Zielstrukturen G, H, I und J

Ein bevorzugtes Verfahren zur Herstellung der erfindungsgemäßen Verbindungen ist im folgendem Schema 2 wiedergegeben:

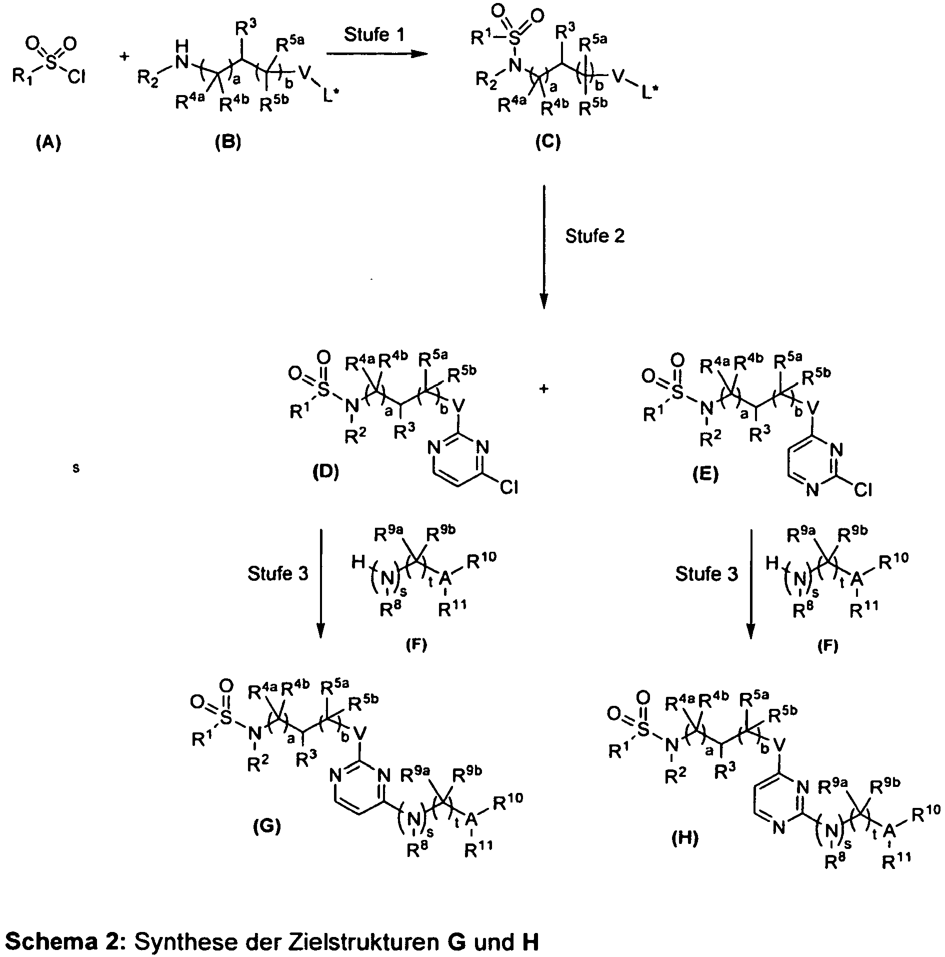

Aus Verbindungen der allgemeinen Formel **(A)** und **(B)** lassen sich, wie in Schema 2 dargestellt, Verbindungen der Formel **(G)** und **(H)** herstellen. Dabei haben die in Schema 2 verwendeten zur Beschreibung der jeweiligen chemischen Verbindungen verwendeten Reste, Variabeln und Indices die gleiche Bedeutung wie zuvor oben im Zusammenhang mit den erfindungsgemäßen Verbindungen beschrieben. Die Gruppe L* steht für eine reaktive Gruppe, die im Zuge der Bindungsknüpfung an den heteroaromatischen Kern abgespalten wird. Im Falle, dass V für O oder NR^{6c} steht kann L* für H oder ein Metallion, insbesondere für H, stehen.

In Stufe 1 werden Sulfonylchlorid der allgemeinen Formel **(A)** in wenigstens einem Lösungsmittel, vorzugsweise ausgewählt aus der Gruppe bestehend aus Dichlormethan, Acetonitril, Dimethylformamid, Diethylether, Dioxan, Tetrahydrofuran, Methanol, Ethanol und Isopropanol mit Verbindung **(B),** z.B. einem Aminoalkohol, in Gegenwart wenigstens einer anorganischen Base, vorzugsweise ausgewählt aus der Gruppe bestehend aus Kaliumcarbonat und Cäsiumcarbonat, oder einer organischen Base, vorzugsweise ausgewählt aus der Gruppe bestehend aus Triethylamin, Diisopropylethylamin und Pyridin und ggf. unter Zusatz von 4-(Dimethylamino)pyridin oder 1-Hydroxybenzotriazol, bei Temperaturen von vorzugsweise -15°C bis 50°C zu Verbindungen mit der allgemeinen Formel **(C)** umgesetzt.

In Stufe 2 werden die Verbindungen mit der allgemeinen Formel **(C),** beispielsweise sulfonylierte Aminoalkohole, in wenigstens einem Lösungsmittel, vorzugsweise ausgewählt aus der Gruppe bestehend aus Dichlormethan, Acetonitril, Dimethylformamid, Diethylether, Dioxan, und Tetrahydrofuran mit 2,4-Dichloropyrimidin, in Gegenwart wenigstens einer anorganischen Base, vorzugsweise ausgewählt aus der Gruppe bestehend aus Kaliumhydrid, Natriumhydrid, Kaliumcarbonat und Cäsiumcarbonat, oder einer organischen Base, vorzugsweise ausgewählt aus der Gruppe bestehend aus 2,3,4,6,7,8,9,10-Octahydropyrimidol[1,2-a]azepine, 1,8-Bis(dimethylamino)Naphthalin, Triethylamin, Düsopropylethylamin, Pyridin und Dimethylaminopyridin bei Temperaturen von vorzugsweise -25°C bis 100°C zu Verbindungen mit der allgemeinen Formel **(D)** und **(E)** umgesetzt.

Die Verbindungen der allgemeinen Formel **(D)** und **(E)** können in der weiteren Synthese als Gemisch oder, beispielsweise nach säulenchromatographischer Trennung, einzeln verwendet werden.

In Stufe 3 werden Pyrimidinbausteine der allgemeinen Formel **(D)** und **(E)** in wenigstens einem Lösungsmittel, vorzugsweise ausgewählt aus der Gruppe bestehend aus Dichlormethan, Acetonitril, Dimethylformamid, Diethylether, Dioxan, Tetrahydrofuran, Methanol, Ethanol und Isopropanol mit Amin **(F),** worin in Gegenwart wenigstens einer anorganischen Base, vorzugsweise ausgewählt aus der Gruppe bestehend aus Kaliumcarbonat und Cäsiumcarbonat, oder einer organischen Base, vorzugsweise ausgewählt aus der Gruppe bestehend aus 2,3,4,6,7,8,9,10-Octahydropyrimidol[1,2-a]azepine, 1,8-Bis(dimethylamino)naphthalin, Triethylamin, Diisopropylethylamin und Pyridin und ggf. unter Zusatz von 4-(Dimethylamino)pyridin oder 1-Hydroxybenzotriazol, bei Temperaturen von vorzugsweise 0°C bis 100°C zu Verbindungen mit der allgemeinen Formel **(G)** und **(H)** umgesetzt.

Um 4-,6-substituierte Pyrimidinderivate vom unten gezeigten Typ (I) zu erhalten kann in der Stufe 2 anstatt 2-,4- Dichlorpyrimidin 4-, 6-, Dichlorpyrimidin verwendet werden. In analoger Weise kann man die Triazinderivate (J) erhalten, wenn in Stufe 2 anstatt 2-,4-Dichlorpyrimidin 2-,4-Dichlor-1,3,5-Triazin eingesetzt wird.

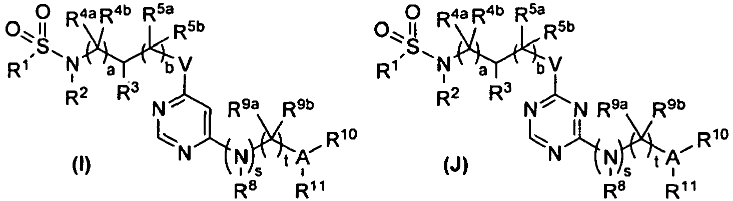

Um Verbindungen vom Typ **(G), (H), (I)** oder **(J)** zu erhalten, in denen V eine CR^{6a}R^{6b}-Gruppe darstellt, kann die Bindung an den heteroaromatischen Kern mittels einer Grignard-Reaktion erfolgen, analog den von B.Scheiper et al. in J.Org. Chem. 2004, 69, 3943-3949 beschriebenen Verfahren. L* stellt in diesem Fall ein Halogenatom dar, dass im Zuge der Grignard-Reaktion aus der Verbindung (C) abgespalten wird.

### Bausteinsynthesen

### 1) Synthese der Sulfonylchloride A

### Sulfonylchlorid A-01: 4-Methoxy-2,6-dimethylphenyl-1-sulfonyl chlorid

Zu einer Lösung 3,5-Dimethylanisol (1.632 g, 11.982 mmol) in Dichlormethan (15 ml) wurde Chlorsulfonsäure (1.83 ml, 2.3 Äquiv.) in Dichlormethan (10 ml) über 20 min bei 0 °C hinzugetropft. Anschließend wurde das Reaktionsgemisch 10 min bei Raumtemperatur gerührt. Das Reaktionsgemisch wurde auf Eiswasser (3 ml, 5 Äquiv. bezüglich Chlorsulfonsäure) gegeben und die wässrige Phase mit Dichlormethan (3 x 100 ml) extrahiert. Die organische Phase wurde getrocknet (Na₂SO₄) und unter Vakuum eingeengt.
Ausbeute: 2.6 g (92 %)

**Sulfonylchlorid A-03: Naphthalin-2-sulfonyl chlorid** [93-11-8] kommerziell erhältlich bei z.B. Aldrich. **Sulfonylchlorid A-04: 2-(Trifluoromethyl)phenyl-1-sulfonyl chlorid** [776-04-5] kommerziell erhältlich bei z.B. Aldrich. **Sulfonylchlorid A-05: 2-Chloro-6-methylphenyl-1-sulfonyl chlorid** [25300-37-2] kommerziell erhältlich bei z.B. Fluorochem.

### Sulfonylchlorid A-06: 2,6-Dichlor-3-methylbenzol-1-sulfonyl chlorid

Zu einer Lösung aus Salzsäure (240 mmol, 4 Äquiv.) und Eisessig (10.8 mmol, 1.8 Äquiv.) wurde 2,6-Dichlor-3-methylanilin (10.56 mmol, 1 Äquiv.) gegeben. Die Suspension wurde auf -10°C gekühlt und wässrige Natriumnitritlösung (65 mmol, 1.08 Äquiv., Wasser 360 mmol, 6 Äquiv.) wurde über 30 min zugetropft. Bei gleichbleibender Temperatur wurde weitere 45 min gerührt. Das Reaktionsgemisch wurde portionsweise zu mit Schwefeldioxid gesättigter Essigsäure (1080 mmol, 18 Äquiv.) gegeben und das Gemisch 30 min bei 0°C gerührt. Das Reaktionsgemisch wurde auf Eis / dest. Wasser (200 ml) gegossen und das angefallene Öl abgetrennt. Die wässrige Phase wurde mit Ether (3 x 20 ml) gewaschen. Die vereinigten organischen Phasen wurden mit dest. Wasser (1 x 50 ml), mit ges. Natriumhydrogencarbonatlösung (1 x 50 ml) und erneut mit dest. Wasser (1 x 50 ml) gewaschen, über Magnesiumsulfat getrocknet und konzentriert. Die Rohsubstanz wurde säulenchromatographisch (Hexan / Ethylacetat 10:1) gereinigt.
Ausbeute: 9.1 g (58%)

### 2) Synthese der Aminoalkohole B

**Aminoalkohol B-01: 2-(Methylamino)ethanol** [109-83-1] kommerziell erhältlich bei z.B. Aldrich. **Aminoalkohol B-02: Piperidin-2-ylmethanol** [3433-37-2] kommerziell erhältlich bei z.B. ABCR. **Aminoalkohol B-03: Piperidin-3-ol** [6859-99-0] kommerziell erhältlich bei z.B. Acros. **Aminoalkohol B-04: (S)-Pyrrolidin-2-ylmethanol** [23356-96-9] kommerziell erhältlich bei z.B. ACROS. **Aminoalkohol** B-**05: Azetidin-3-ol** [18621-18-6] kommerziell erhältlich bei z.B. Aldrich. **Aminoalkohol B-06: (S)-Indolin-2-ylmethanol** [27640-33-1] kommerziell erhältlich bei z.B. Aldrich.

### Aminoalkohol B-07: 2-(Methylamino)-2-phenylethanol

(i) Eine 0°C kalte Lösung aus Phenylglycine (132 mmol, 1 Äquiv.) in 1 N Natronlauge wurde mit Natriumcarbonat (66 mmol, 0.5 Äquiv.) versetzt und für 30 min. gerührt. Ethyl chloroformate (132 mmol, 1 Äquiv.) wurde zugegeben, die Reaktionsmischung für 1 h bei Raumtemperatur gerührt, Dichlormethan zugegeben und für eine weitere Stunde bei Raumtemperatur gerührt. Phasen wurden getrennt, die wässrige mit verdünnter Salzsäure neutralisiert und mit Dichlormethan extrahiert. Die vereinigten organischen Phasen wurden mit ges. Natriumhydrogencarbonatlösung und mit Wasser gewaschen, über Natriumsulfat getrocknet und unter vermindertem Druck konzentriert. Das gewünschte Produkt wurde in einer Ausbeute von 71% erhalten.
(ii) Das soeben erhaltene Produkt (89.6 mmol) wurde in THF gelöst zu einer 0°C kalten Lösung von Lithiumaluminiumhydrid (358 mmol, 4 Äqiuv.) in THF getropft und dabei eine Reaktionstemperatur von 0-5°C eingehalten. Die Reaktionsmischung wurde für 15 min bei Raumtemperatur und anschließend 12 h unter Rückfluß gerührt. Zur Aufarbeitung wurde auf 0°C abgekühlt und mit 15% NaOH-Lsg. (~ 40 ml) versetzt Nach Zugabe von ca. 20 ml der Natronlauge wurde mit 250 ml THF verdünnt und unter schwenken die restlichen 20 ml Natronlauge zugegeben. Nachdem für eine Stund bei Raumtemperatur gerührt wurde, wurde der Niederschlag abfiltriert und mit Ethylacetat nachgewaschen. Das Lösungsmittel wurde unter vermindertem Druck konzentriert und das gewünschte Produkt als gelbes Öl in einer Ausbeute von 88% erhalten.

### Aminoalkohol B-08: 2-(Cyclopropylamino)ethanol Hydrobromid

Cyclopropylamin (7 ml, 100.8 mmol) und 2-Bromethanol (5 g, 40.32 mmol) wurden in Ethanol (47 ml) 16 h bei 50°C gerührt. Das Lösungsmittel wurde unter Vakuum entfernt, der Rückstand in Toluol (3 x 40 ml) aufgenommen und unter Vakuum getrocknet. Ausbeute: 6.99 g (95 %)

### Aminoalkohol B-09: ((2S,4R)-4-Fluoropyrrolidin-2-yl)methanol hydrochlorid

(i) (2S,4R)-N-Boc-4-fluoropyrrolidine-2-carboxylic acid (2 g, 8.58 mmol) wurde in Tetrahydrofuran (31 ml) gelöst, gekühlt und bei 0°C langsam mit Borwasserstoff-Tetrahydrofuran-Komplex (1 mol/l, 12.87 ml) versetzt. Das Reaktionsgemisch erwärmte sich langsam auf Raumtemperatur, nach 30 min rühren wurde es wieder auf 0°C gekühlt. Wasser (3.9 ml) wurde langsam zugetropft, anschließend Kaliumcarbonat (2 g, 14.59 mmol) langsam zugegeben 30 min bei RT gerührt. Es wurde mit Wasser (10 ml) verdünnt und die Phasen getrennt. Die wässrige Phase wurde mit Ethylacetat (3 x 20 ml) extrahiert, die vereinigten organischen Phasen wurden über Natriumsulfat getrocknet und unter Vakuum eingeengt. Das Rohprodukt wurde säulenchromatographisch (Kieselgel, Diethylether / Dichlormethan / Hexan, 2:1:1) gereinigt. Ausbeute: 1.5 g (80%)
(ii) Das so erhaltene (2S,4R)-N-Boc-4-fluoropyrrolidin-2-yl-methanol (1.5 g, 6.845 mmol) wurde mit Chlorwasserstoff in Methanol (27 ml, 1.25 mol/l) versetzt und refluxiert. Nach 30 min wurde auf RT gekühlt und unter Vakuum eingeengt. Der Rückstand wurde in Etanol (10 ml) aufgenommen, mit Aceton (20 ml) versetzt und 30 min im Eisbad gerührt. Der Niederschlag wurde abgesogen, mit Diethylether gewaschen und unter Vakuum getrocknet um die gewünschte Zielverbindung zu erhalten. Ausbeute: 0,93 g (87%)

### Aminoalkohol B-10: 2-Amino-2-methyl-propan-1-ol [124-68-5] kommerziell erhältlich bei z.B. Aldrich

### Aminoalkohol B-11: 3-(Cyclopropylamino)propan-1-ol

3-Brompropanol (26.26 mmol, 1.0 Äquiv.) wurde zu einer Lösung aus Cyclopropylamin (52.53 mmol, 2.0 Äquiv.) in Ethanol (150 ml) geben und das gemisch 14 h refluxiert. Das Lösemittel wurde unter vermindertem Druck konzentriert und das so erhaltene Rohprodukt ohne weitere Aufreinigung im nächsten Schritt eingesetzt.

### Aminoalkohol B-12: (S)-Piperidin-2-ylmethanol

BH₃-DMS (62.0 mmol, 4.0 Äquiv.) und BF₃-Et₂O (15.5 mmol, 1.0 Äquiv.) wurden bei 0°C zu einer Lösung aus (2S)-Piperidin-2-carbonsäure (15.5 mmol, 1.0 Äquiv.) in THF (50 ml) gegeben und das Gemisch für 14 h refluxiert. Das Lösemittel wurde unter vermindertem Druck konzentriert und anschließend MeOH (40 ml) tropfenweise bei 0°C zugegeben. Konz. HCl (5 ml) zur Reaktionsmischung zugegeben und es wurde weitere 2 h refluxiert. Das Lösemittel wurde konzentriert, der Rückstand 15 min in 10% Isopropanol in DCM gerührt und filtriert. Das Filtrat wurde bis zur Trockene konzentriert und man erhielt einen weißen Feststoff.
Ausbeute: 80%

### 3) Synthese der sulfonylierten Aminoalkohole C:

### Allgemeine Methode zur Synthese der sulfonylierten Aminoalkohole C

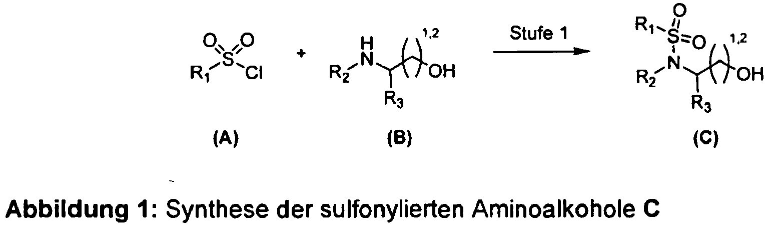

Allgemeine Arbeitsvorschrift AAV I: Zu einer Lösung von Aminoalkohol (B) (5 Äquiv.) in Dichlormethan wurde bei Raumtemperatur eine Lösung des Sulfonylchlorids (A) (1 Äquiv.) in Dichlormethan gegeben. Das erhaltene Reaktionsgemisch wurde für 12 h bei Raumtemperatur gerührt und anschließend 3x mit einer 5%igen HCl-Lösung gewaschen. Die organische Phase wurde über Magnesiumsulfat getrocknet und unter vermindertem Druck konzentriert. Der so erhaltene sulfonylierte Aminoalkohol (C) wurde ohne weitere Aufreinigung in der nächsten Stufe eingesetzt.

Allgemeine Arbeitsvorschrift AAV II: Zu einer 0°C kalten Lösung von Aminoalkohol (B) (1 Äquiv.) und Triethylamin (2.5 Äquiv.) in Dichlormethan wurde nach 30 min. eine Lösung des Sulfonylchlorids (A) (1 Äquiv.) in Dichlormethan gegeben. Das erhaltene Reaktionsgemisch wurde für 1-6 h bei Raumtemperatur gerührt, mit Dichlormethan verdünnt und anschließend mit 1 N HCl-Lösung und Wasser gewaschen. Die organische Phase wurde über Natriumsulfat getrocknet und unter vermindertem Druck konzentriert. Der erhaltene sulfonylierte Aminoalkohol (C) wurde säulenchromatographisch gereinigt.

Allgemeine Arbeitsvorschrift AAV III: Das Hydrochlorid bzw. Hydrobromid des Aminoalkohols (B) (1 Äquiv.) wurde in Dichlormethan und Triethylamin (2 Äquiv.) gelöst und 15 min gerührt, dabei mit einem Eisbad gekühlt. Anschließend wurde das Sulfonylchlorid (A) (1.5 Äquiv.), gelöst in Dichlormethan, bei 0°C langsam zugegeben. Das Kühlbad wurde entfernt und das Reaktionsgemisch 15 h gerührt. Das Gemisch wurde mit gesättigter Natriumhydrogencarbonatlösung versetzt und die Phasen getrennt. Die wässrige Phase wurde mit Dichlormethan extrahiert, die vereinigten organischen Phasen mit gesättigter Natriumchlorid-Lösung gewaschen, über Natriumsulfat getrocknet und unter Vakuum eingeengt. Der erhaltene sulfonylierte Aminoalkohol (C) wurden ggf. säulenchromatographisch (Kieselgel) gereinigt. In der nachfolgenden Tabelle I werden die nach diesen allgemeinen Arbeitsvorschriften synthetisierten sulfonierten Aminoalkohole (C) aufgelistet.

### Synthese von Aminoalkohol C-15: N-(1-(Hydroxymethyl)cyclobutyl)-4-methoxy-2,6-dimethylbenzolsulfonamid

### Stufe-1: Methyl 1-aminocyclobutancarboxylat

Konzentrierte H₂SO₄ (10 mL) wurde zu einer Lösung aus 1-(tert-Butoxycarbonylamino)-cyclobutan-1-carbonsäure (9.29 mmol, 1 Äquiv.) in MeOH (30 ml) bei 0 °C gegeben und die Reaktion wurde 2 h bei 0°C refluxiert. Das Lösungsmittel wurde unter vermindertem Druck entfernt. Der Rückstand wurde in dest. Wasser aufgenommen, mit gesättigter Natriumhydrogencarbonatlösung auf pH 8-9 eingestellt und mit Ethylacetat (2 x 200 ml) extrahiert. Die vereinigten org. Phasen wurden mit dest. Wasser (2 x 150 ml) und gesättigter NaCl-Lösung (2 x 50 ml) gewaschen und über Natriumsulfat getrocknet. Das Lösungsmittel wurde unter vermindertem Druck konzentriert um das gewünschte Produkt als weißen Feststoff zu erhalten.
Ausbeute: 75%

### Stufe-2: Methyl 1-(4-methoxy-2,6-dimethylphenylsulfonam ido)cyclobutancarboxylat

Zu einer Lösung aus Methyl 1-aminocyclobutancarboxylat (6,97 mmol, 1 Äquiv.) in Dichlormethan p.a. (20 ml) wurde bei 0°C Triethylamin (13,9 mmol, 2.0 Äquiv.) zugegeben und das Gemisch für 10 Minuten gerührt. 4-Methoxy-2, 6-dimethylphenylsulfonylchlorid (8.36 mmol, 1.2 Äquiv.), in DCM (5 ml) gelöst, wurde langsam bei gleicher Temperatur zugetropft und das Gemisch bei 25°C 4 h gerührt. Das Reaktionsgemisch wurde mit Dichlormethan (100 ml) verdünnt, mit dest. Wasser (2 x 50 ml) und ges. Natriumchloridlösung (2 x 50 ml) gewaschen und über Natriumsulfat getrocknet. Das Lösemittel wurde konzentriert und das Rohprodukt säulenchromatographisch (15% Ethylacetat in Hexan) gereinigt um das gewünschte produkt als weißen Feststoff zu erhalten.
Ausbeute: 66%

### Stufe-3: N-(1-(Hydroxymethyl)cyclobutyl)-4-methoxy-2,6-dimethylbenzolsulfonamid (C-15)

Methyl 1-(4-methoxy-2,6-dimethylphenylsulfonamido)cyclobutancarboxylat (4.58 mmol, 1.0 Äquiv.) wurde in THF p.a. (40 ml) gelöst und langsam unter Stickstoff bei 0°C in eine Suspension von LiAlH₄ (11.4 mmol, 2.5 Äquiv.) in THF p.a. (20 ml) getropft. Das Reaktionsgemisch wurde 1 h bei 25°C gerührt, danach mit gesättigter Na₂SO₄-Lösung versetzt und über Celite abfiltriert. Das Filtrat wurde konzentriert und das gewünschte Produkt wurde als weißer Feststoff aus Hexan ausgefällt. Ausbeute: 92%

**Tabelle 1: Synthese der sulfonylierten Aminoalkohole C**

| **Alkohol Nr.** | **Struktur** | **Name** | **Sulfonylchlorid (A)** | **Alkohol (B)** | **Synthese nach** | **Ausbeute** |
|---|---|---|---|---|---|---|
| C-01 | 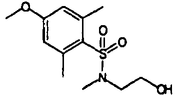 | N-(2-Hydroxyethyl)-4-methoxy-N,2,6-trimethylphenylsulfonamid (C-01) | 4-Methoxy-2,6-dimethylphenyl-1-sulfonyl chlorid (A-01) | 2-(Methylamino)ethanol (B-01) | AAV I | 91%, (30.36 mmol) |
| C-02 | 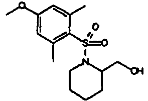 | (1-(4-Methoxy-2,6-dimethylphenylsulfonyl)pip eridin-2-yl)methanol (C-02) | 4-Methoxy-2,6-dimethylphenyl-1-sulfonyl chlorid (A-01) | Piperidin-2-ylmethanol (B-02) | AAV I | 63%, (21.1 mmol) |
| C-03 | 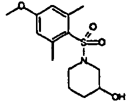 | 1-(4-Methoxy-2,6-dimethylphenylsulfonyl)pip eridin-3-ol (C-03) | 4-Methoxy-2,6-dimethylphenyl-1-sulfonyl chlorid (A-01) | Piperidin-3-ol (B-03) | AAV I | 99%, (56.45 mmol) |
| C-04 ^{(a)} | 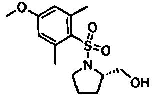 | (S)-(1-(4-Methoxy-2,6-dimethylphenylsulfonyl)pyrr olidin-2-yl)methanol (C-04) | 4-Methoxy-2,6-dimethylphenyl-1-sulfonyl chlorid (A-01) | (S)-Pyrrolidin-2-ylmethanol (B-04) | AAV I | 100%, (35.4 mmol) |
| C-06 | 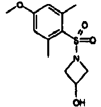 | 1-(4-Methoxy-2,6-dimethylphenylsulfonyl)aze tidin-3-ol (C-06) | 4-Methoxy-2,6-dimethylphenyl-1-sulfonyl chlorid (A-01) | Azetidin-3-ol (B-05) | AAV I | 94%, (14.26 mmol) |
| C-07 ^{(b)} | 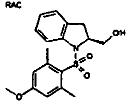 | (S)-(1-(4-Methoxy-2,6-dimethylphenylsulfonyl)ind olin-2-yl)methanol (C-07) | 4-Methoxy-2,6-dimethylphenyl-1-sulfonyl chlorid (A-01) | (S)-Indolin-2-ylmethanol (B-06) | AAV I | 86%, (9.6 mmol) |
| C-08 | 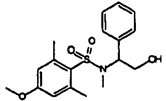 | N-(2-Hydroxy-1-phenylethyl)-4-methoxy-N,2,6-trimethylphenylsulfonamid (C-08) | 4-Methoxy-2,6-dimethylphenyl-1-sulfonyl chlorid (A-01) | 2-(Methylamino)-2-phenylethanol (B-07) | AAV II | 71% (93 mmol) |
| C-09 | 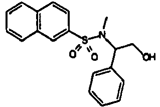 | N-(2-Hydroxy-1-phenylethyl)-N-methylNaphthalin-2-sulfonamid (C-09) | Naphthalin-2-sulfonyl chlorid (A-03) | 2-(Methylamino)-2-phenylethanol (B-07) | AAV II | 58% (46 mmol) |
| C-10 | 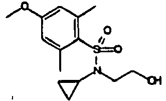 | N-Cyclopropyl-N-(2-hydroxyethyl)-4-methoxy-2,6-dimethylphenylsulfonamid (C-10) | 4-Methoxy-2,6-dimethylphenyl-1-sulfonyl chlorid (A-01) | 2-(Cyclopropylamino)etha nol Hydrobromid (B-08) | AAV III | 51% (2.53 g) |
| C-11 | 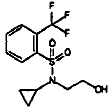 | N-Cyclopropyl-N-(2-hydroxyethyl)-2-(trifluoromethyl)phenylsulfo namid (C-11) | 2-(Trifluoromethyl)phenyl-1-sulfonyl chlorid (A-04) | 2-(Cyclopropylamino)etha nol Hydrobromid (B-08) | AAV III | 31% (0.91 g) |
| C-12 | 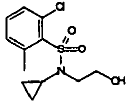 | 2-Chloro-N-cydopropyl-N-(2-hydroxyethyl)-6-methylphenylsulfonamid (C-12) | 2-Chloro-6-methylphenyl-1-sulfonyl chlorid (A-05) | 2-(Cyclopropylamino)etha nol Hydrobromid (B-08) | AAV III | 36% (1.17g) |
| C-13 | 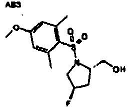 | ((2S,4R)-4-Fluoro-1-(4-methoxy-2,6-dimethylphenylsulfonyl)pyrr olidin-2-yl)methanol *(C-13) | 4-Methoxy-2,6-dimethylphenyl-1-sulfonyl chlorid (A-01) | ((2S,4R)-4-Fluoropyrrolidin-2-yl)methanol Hydrochlorid (B-09) | AAV III | 91% (1.71 g) |
| C-14 | 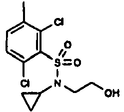 | 2,6-Dichlor-N-cyclopropyl-N-(2-hydroxy-ethyl)-3-methyl-benzolsulfonamid (C-14) | 2,6-Dichlor-3-methylbenzol-1-sulfonyl chlorid (A-06) | (Cyclopropylamino)ethanol Hydrobromid (B-08) | AAV III | 46% (1,05g) |
| C-15^{(c)} | 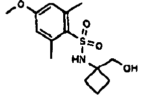 | N-[1-(Hydroxymethyl)-cyclobutyl]-4-methoxy-2,6-dimethyl-benzolsulfonamid (C-15) | | | | |
| C-16 | 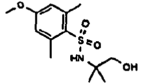 | N-(2-Hydroxy-1,1-dimelhyl-ethyl)-4-melhoxy-2,6-dimethyl-benzolsulfonamid (C-16) | 4-Methoxy-2,6-dimethylphenyl-1-sulfonyl chlorid (A-01) | 2-Amino-2-methyl-propan-1-ol (B-10) | AAV II | 26% |
| C-17 | 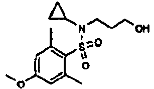 | N-Cyclopropyl-N-(3-hydroxy-propyl)-4-methoxy-2,6-dimethyl-benzolsulfonamid (C-17) | 4-Methoxy-2,6-dimethylphenyl-1-suffonyl chlorid (A-01) | 3-(Cyclopropylamino)prop an-1-ol (B-11) | AAV II | 34% |
| C-18 | 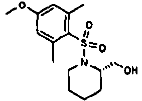 | [(2S)-1-[(4-Methoxy-2,6-dimethyl-phenyl)sulfonyl]-piperidin-2-yl]-methanol (C-18) | 4-Methoxy-2,6-dimethylphenyl-1-sulfonyl chlorid (A-01) | (S)-Piperidin-2-ylmethanol (B-12) | AAV II | 30% |

| | | | | | | |
|---|---|---|---|---|---|---|
| (a) Unter Verwendung von 3 Äqiv. L-Prolinol nach AAV (I) synthetisiert. (b) Säulenchromatographisch aufgereinigt (Silica, EtOAc/Hexan). | | | | | | |

### 4) Synthese der Pyrimidinbausteine D & E:

### Allgemeine Methode zur Synthese der Pyrimidinbausteine D & E

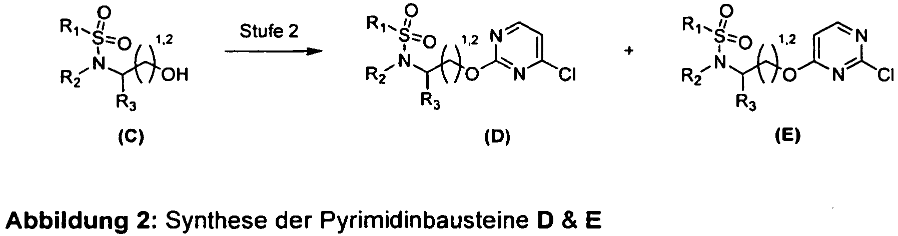

Allgemeine Arbeitsvorschrift AAV IVa: Eine Lösung des sulfonylierten Aminoalkohols (C) (1 Äquiv.) in Tetrahydrofuran wurde bei 0°C mit Natriumhydrid (1.1 Äquiv.) versetzt und für 30 min bei 0°C gerührt. Anschließend wurde dieses Reaktionsgemisch mit einer Lösung von 2,4-Dichloropyrimidin (1 Äquiv.) in Tetrahydrofuran versetzt und für 2-6 h bei Raumtemperatur gerührt. Zur Aufarbeitung wurde die Reaktionslösung auf Wasser gegossen, 3x mit Ethylacetat extrahiert, die vereinigten organischen Phasen über Magnesiumsulfat getrocknet und unter vermindertem Druck konzentriert. Nach säulenchromatographischer Aufreinigung (Silika; Ethylacetat / Hexan) wurden beide Regioisomere (D & E) hinreichend rein erhalten.

Allgemeine Arbeitsvorschrift AAV IVb: Der sulfonylierte Aminoalkohol (C) (1 Äquiv.) wurde unter Schutzgas in Tetrahydrofuran gelöst und gekühlt. Bei 0°C wurde Natriumhydrid (1.1 Äquiv.) zugegeben, es wurde 30 min bei gleicher Temperatur gerührt, und anschließend 2,4-Dichlorpyrimidin (1 Äquiv.), gelöst in Tetrahydrofuran, langsam zugetropft. Die Reaktionsmischung wurde 15 h gerührt und dabei auf Raumtemperatur enrvärmt. Nach dünnschichtchromatographischer Kontrolle wurde ggf. weitere 2 h bei 40°C gerührt und ggf. anschließend nochmal weitere 22 h bei Raumtemperatur. Gesättigte Natriumhydrogencarbonat-Lösung und Ethylacetat wurden zugegeben, die Phasen wurden getrennt und die wässrige Phase mit Ethylacetat extrahiert. Die vereinigten organischen Phasen wurden über Magnesiumsulfat getrocknet und unter Vakuum eingeengt. Das Rohprodukt wurde säulenchromatographisch (Kieselgel) gereinigt, dabei wurden die beiden Regioisomere (D & E) des Pyrimidinbausteins, sofern beide angefallen waren, zumindestens teilweise getrennt.

Allgemeine Arbeitsvorschrift AAV Va: Der sulfonylierte Aminoalkohol (C) (1 Äquiv.) wurde unter Schutzgas in trockenem Tetrahydrofuran gelöst und gekühlt. Bei 0°C wurde Natriumhydrid (2 Äquiv.) zugegeben, es wurde 30 min bei gleicher 25°C gerührt, und anschließend auf -78°C gekühlt. 4-Chlor-2-(methylsulfonyl)pyrimidin (1 Äquiv.), gelöst in Tetrahydrofuran, wurde langsam über 1 h zugetropft. Die Reaktionsmischung wurde auf 25°C erwärmt und mit Wasser gequencht. Es wurde mit Ethylacetat 2 x extrahiert und die vereinigten organischen Phasen mit Wasser (2 x) und ges. NaCl-Lösung (2 x) extrahiert. Dann wurde über Natriumsulfat getrocknet und unter Vakuum eingeengt. Das Rohprodukt des Pyrimidinbausteins (D) wurde direkt in die nachfolgende Stufe eingesetzt.

Allgemeine Arbeitsvorschrift AAV Vb: Der sulfonylierte Aminoalkohol (C) (1 Äquiv.) wurde unter Schutzgas in trockenem Tetrahydrofuran gelöst und gekühlt. Bei 0°C wurde Natriumhydrid (2 Äquiv.) zugegeben, es wurde 30 min bis 1 h bei 25°C gerührt, und anschließend auf -78°C gekühlt. 4-Chlor-2-(methylsulfonyl)pyrimidin (1 Äquiv.), gelöst in Tetrahydrofuran, wurde langsam über 45 min zugetropft. Dann wurde die Reaktionsmischung mit Wasser gequencht, auf 25°C erwärmt und anschließend mit Ethylacetat extrahiert. Die vereinigten organischen Phasen wurden über Natriumsulfat getrocknet und unter Vakuum eingeengt. Nach säulenchromatographischer Aufreinigung wurde der Pyrimidinbaustein (D) hinreichend rein erhalten.

Allgemeine Arbeitsvorschrift AAV Vc: Der sulfonylierte Aminoalkohol (C) (1 Äquiv.) wurde unter Schutzgas in trockenem Tetrahydrofuran gelöst und gekühlt. Bei 0°C wurde Natriumhydrid (2 Äquiv.) zugegeben, es wurde 30 min bei 25°C gerührt, und anschließend auf -78°C gekühlt. 4-Chlor-2-(methylsulfonyl)pyrimidin (1 Äquiv.), gelöst in Tetrahydrofuran, wurde langsam zugetropft. Das Gemisch wurde 1 h bei derselben Temperatur gerührt. Dann wurde die Reaktionsmischung mit Wasser gequencht und anschließend mit Ethylacetat extrahiert. Die vereinigten organischen Phasen wurden über Natriumsulfat getrocknet und unter Vakuum eingeengt. Nach säulenchromatographischer Aufreinigung wurde der Pyrimidinbaustein (D) hinreichend rein erhalten.

Allgemeine Arbeitsvorschrift AAV Vd: Der sulfonylierte Aminoalkohol (C) (1 Äquiv.) wurde unter Schutzgas in trockenem Tetrahydrofuran gelöst und gekühlt. Bei 0°C wurde Natriumhydrid (2 Äquiv.) zugegeben, es wurde 1 h bei 25°C gerührt, und anschließend auf -78°C gekühlt. 4-Chlor-2-(methylsulfonyl)pyrimidin (1 Äquiv.), gelöst in Tetrahydrofuran, wurde langsam zugetropft. Das Gemisch wurde 1 h bei - 30°C gerührt. Dann wurde die Reaktionsmischung mit Eis gequencht und anschließend mit Ethylacetat extrahiert. Die vereinigten organischen Phasen wurden über Natriumsulfat getrocknet und unter Vakuum eingeengt. Nach säulenchromatographischer Aufreinigung wurde der Pyrimidinbaustein (D) hinreichend rein erhalten.

In der nachfolgenden Tabelle 2 sind die nach den oben beschriebenen allgemeinen Arbeitsvorschriften hergestellten Pyrimidinbausteine aufgelistet.

**Tabelle 2: Synthese der Pyrimidinbausteine D & E**

| **Pyrimidin Nr.** | **Pyrimidin Struktur** | **Pyrimidin** | **Sulfonylierter Aminoalkohol** | **Synthese nach** | **Ausbeute** |
|---|---|---|---|---|---|
| E-01 | 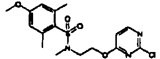 | N-(2-(2-Chloropyrimidin-4-yloxy)ethyl)-4-methoxy-N,2,6-trimethylphenylsulfonamid (E-01) | N-(2-Hydroxyethyl)-4-methoxy-N,2,6-trimethylphenylsulfonamid (C-01) | AAV IVa | 46%, (7.07 mmol) |
| D-01 | 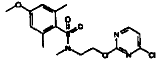 | N-(2-(4-Chloropyrimidin-2-yloxy)ethyl)-4-methoxy-N,2,6-trimethylphenylsulfonamid (D-01) | N-(2-Hydroxyethyl)-4-methoxy-N,2,6-trimethylphenylsulfonamid (C-01) | AAV IVa | 23%, (3.5 mmol) |
| E-02 | 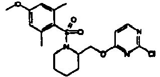 | 2-Chloro-4-((1-(4-methoxy-2,6-dimethylphenylsulfonyl)piperidin-2-yl)methoxy)pyrimidin (E-02) | (1-(4-Methoxy-2,6-dimethylphenylsulfonyl)piperidin-2-yl)methanol (C-02) | AAV IVa | 42%, (8.87 mmol) |
| D-02 | 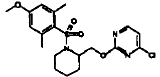 | 4-Chloro-2-((1-(4-methoxy-2,6-dimethylphenylsulfonyl)piperidin-2-yl)methoxy)pyrimidin (D-02) | (1-(4-Methoxy-2,6-dimethylphenylsulfonyl)piperidin-2-yl)methanol (C-02) | AAV IVa | 49%, (10.33 mmol) |
| E-03 | 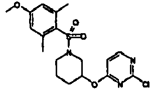 | 2-Chloro-4-(1-(4-methoxy-2,6-dimethylphenylsulfonyl)piperidin-3-yloxy)pyrimidin (E-03) | 1-(4-Methoxy-2,6-dimethylphenylsulfonyl)piperidin-3-ol (C-03) | AAV IVa | 36%, (6.04 mmol) |
| D-03 | 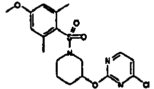 | 4-Chloro-2-(1-(4-methoxy-2,6-dimethylphenylsulfonyl)piperidin-3-yloxy)pyrimidin (D-03) | 1-(4-Methoxy-2,6-dimethylphenylsulfonyl)piperidin-3-ol (C-03) | AAV IVa | 18%, (3.01 mmol) |
| E-04 | 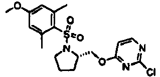 | (S)-2-Chloro-4-((1-(4-methoxy-2,6-dimethylphenylsulfonyl)pyrrolidin-2-yl)methoxy)pyrimidin (E-04) | (S)-(1-(4-Methoxy-2,6-dimethylphenylsulfonyl)pyrrolidin-2-yl)methanol (C-04) | AAV IVa | 57%, (8.67 mmol) |
| D-04 | 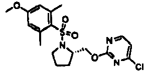 | (S)-4-Chloro-2-((1-(4-methoxy-2,6-dimethylphenylsulfonyl)pyrrolidin-2-yl)methoxy)pyrimidin (D-04) | (S)-(1-(4-Methoxy-2,6-dimethylphenylsulfonyl)pyrrolidin-2-yl)methanol (C-04) | AAV IVa | 43%, (6.5 mmol) |
| E-06 | 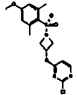 | 2-Chloro-4-(1-(4-methoxy-2,6-dimethylphenylsulfonyl)azetidin-3-yloxy)pyrimidin (E-06) | 1-(4-Methoxy-2,6-dimethylphenylsulfonyl)azetidin-3-ol (C-06) | AAV IVa | 0.12%, (1.69 mmol) |
| D-06/ E-06^{(e)} | 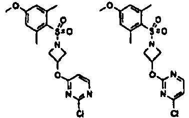 | 4-Chloro-2-(1-(4-methoxy-2,6-dimethylphenylsulfonyl)azetidin-3-yloxy)pyrimidin (D-06) / 2-chloro-4-(1-(4-methoxy-2,6-dimethylphenylsulfonyl)azetidin-3-yloxy)pyrimidin (E-06) | 1-(4-Methoxy-2,6-dimethylphenylsulfonyl)azetidin-3-ol (C-06) | AAV IVa | 69% (9.9 mmol) |
| E-07^{(a)}' | 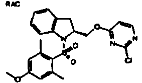 | (S)-2-((2-Chloropyrimidin-4-yloxy)methyl)-1-(4-methoxy-2,6-dimethylphenylsulfonyl)indoline (E-07) | (S)-(1-(4-Methoxy-2,6-dimethylphenylsulfonyl)indolin-2-yl)methanol (C-07) | AAV IVa | 28%, (2.7mmol) |
| E-08^{(b)} | 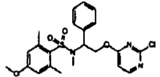 | N-(2-(2-Chloropyrimidin-4-yloxy)-1-phenylethyl)-4-methoxy-N,2,6-trimethylphenylsulfonamid (E-08) | N-(2-Hydroxy-1-phenylethyl)-4-methoxy-N,2,6-trimethylphenylsulfonamid (C-08) | AAV IVa | 15%, (1.5 mmol) |
| D-08^{(b)} | 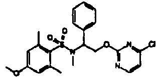 | N-(2-(4-Chloropyrimidin-2-yloxy)-1-phenylethyl)-4-methoxy-N,2,6-trimethylphenylsulfonamid (D-08) | N-(2-Hydroxy-1-phenylethyl)-4-methoxy-N,2,6-trimethylphenylsulfonamid (C-08) | AAV IVa | 25%, (2.5 mmol) |
| E-09^{(b)} | 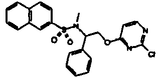 | N-(2-(2-Chloropyrimidin-4-yloxy)-1-phenylethyl)-N-methylnaphthalin-2-sulfonamid (E-09) | N-(2-Hydroxy-1-phenylethyl)-N-methynaphthalin-2-sulfonamid (C-09) | AAV IVa | 15%, (3.45 mmol) |
| D-09^{(b)} | 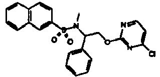 | N-(2-(4-Chloropyrimidin-2-yloxy)-1-phenylethyl)-N-methylnaphthalin-2-sulfonamid (D-09) | N-(2-Hydroxy-1-phenylethyl)-N-methylnaphthalin-2-sulfonamid (C-09) | AAV IVa | 20%, (4.6 mmol) |
| D-10^{(c)} | 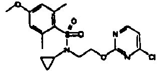 | N-(2-(4-Chloropyrimidin-2-yloxy)ethyl)-N-cyclopropyl-4-methoxy-2,6-dimethylphenylsulfonamid (D-10) | N-Cyclopropyl-N-(2-hydroxyethyl)-4-methoxy-2,6-dimethylphenylsulfonamid (C-10) | AAV IVb | 7% (0.38 g (D-10) (plus 1.94 g (D & E)) |
| E-10^{(c)} | 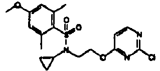 | N-(2-(2-Chloropyrimidin-4-yloxy)ethyl)-N-cyclopropyl-4-methoxy-2,6-dimethylphenylsulfonamid (E-10) | N-Cyclopropyl-N-(2-hydroxyethyl)-4-methoxy-2,6-dimethylphenylsulfonamid (C-10) | AAV IVb | 23% (1.21 g (E-10)) (plus 1.94 g (D & E)) |
| D-11^{(c)} | 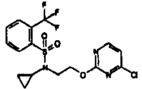 | N-(2-(4-Chloropyrimidin-2-yloxy)ethyl)-N-cyclopropyl-2-(trifluoromethyl)phenylsulfonamid (D-11) | N-Cyclopropyl-N-(2-hydroxyethyl)-2-(trifluoromethyl)phenylsulfonamid (C-11) | AAV IVb | 9% (0.11 g (D-11)) (plus 0.49 g (E-11) und 0.45 g (D & E)) |
| D-12^{(c),(d1)} | 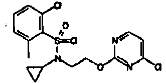 | 2-Chloro-N-(2-(4-chloropyrimidin-2-yloxy)ethyl)-N-cyclopropyl-6-methylphenylsulfonamid (D-12) | 2-Chloro-N-cydopropyl-N-(2-hydroxyethyl)-6-methylphenylsulfonamid (C-12) | AAV IVb | 16% (0.19 g (D-12)) (plus 0.31g (D & E)) |
| E-12^{(c)(e)} | 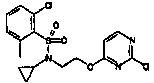 | 2-Chloro-N-(2-(2-chloropyrimidin-4-yloxy)ethyl)-N-cyclopropyl-6-methylphenylsulfonamid (E-12) | 2-Chloro-N-cyclopropyl-N-(2-hydroxyethyl)-6-methylphenylsulfonamid (C-12) | AAV IVb | 35% (0.41 g (E-12)) (plus 0.31 g (D & E)) |
| D-13^{(c)} | 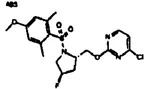 | 4-Chloro-2-(((2S,4R)-4-fluoro-1-(4-methoxy-2,6-dimethylphenylsulfonyl)pyrrolidin-2-yl)methoxy)pyrimidin (D-13) | ((2S,4R)-4-Fluoro-1-(4-methoxy-2,6-dimethylphenylsulfonyl)pyrrolidin-2-yl)methanol (C-13) | AAV IVb | 7% (0.15 g (D-13)) (plus 1.88 g (D & E)) |
| D-14^{(c)} | 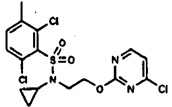 | 2,6-Dichlor-N-[2-(4-chlor-pyrimidin-2-yl)oxy-ethyl]-N-cyclopropyl-3-methyl-benzolsulfonamid (D-14) | 2,6-Dichlor-N-cyclopropyl-N-(2-hydroxy-ethyl)-3-methyl-benzolsulfonamid (C-14) | AAV IVb | 16% (0.22g) (D-14) |
| D-15 | 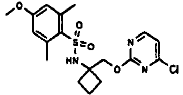 | N-[1-[(4-Chlor-pyrimidin-2-yl)oxy-methyl]-cyclobutyl]-4-methoxy-2,6-dimethyl-benzolsulfonamid (D-15) | N-[1-(Hydroxymethyl)-cyclobuty]-4-methoxy-2,6-dimethyl-benzolsulfonamid (C-15) | AAV Va | 84% (D-15) |
| D-16 | 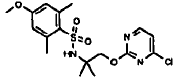 | N-[2-(4-Chlor-pyrimidin-2-yl)oxy-1,1-dimethyl-ethyl]-4-methoxy-2,6-dimethyl-benzolsulfonamid (D-16) | N-(2-Hydroxy-1,1-dimethyl-ethyl)-4-methoxy-2,6-dimethyl-benzolsulfonamid (C-16) | AAV Vb | 27% (D-16) |
| D-17 | 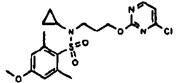 | N-[3-(4-Chlor-pyrimidin-2-yl)oxy-propyl]-N-cyclopropyl-4-methoxy-2,6-dimethyl-benzolsulfonamid (D-17) | N-Cyclopropyl-N-(3-hydroxy-propyl)-4-methoxy-2,6-dimethyl-benzolsulfonamid (C-17) | AAV Vc | 10% (D-17) |
| D-18 | 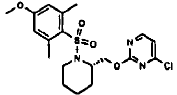 | 4-Chlor-2-[[(2S)-1-[(4-methoxy-2,6-dimethyl-phenyl)sulfonyl)-piperidin-2-yl]-methoxy]-pyrimidin (D-18) | [(2S)-1-[(4-Methoxy-2,6-dimethyl-phenyl)sulfonyl]-piperidin-2-yl]-methanol (C-18) | AAV Vd | 43% (D-18) |

| | | | | | |
|---|---|---|---|---|---|
| (a) Das Reaktionsgemisch wurde für 12 h bei Raumtemperatur gerührt. Da die Umsetzung unvollständig war, wurde für 12 h auf 50°C erhitzt. (b) Die Synthese wurde unter Verwendung von 2.5 Äquiv. NaH durchgeführt. (c) Die Regiochemie wurde auf dieser und / oder der nachfolgenden Synthesestufe via 1H NMR zugeordnet. (d) 1H NMR (400 MHz, DMSO-*d*₆) d ppm 0.26 - 0.34 (m, 2 H) 0.53 - 0.62 (m, 2 H) 2.58 - 2.63 (m, 1H) 2.64 (s, 3 H) 3.81 (t, *J*=5.52 Hz, 2 H) 4.58 (t, *J*=5.52 Hz, 2 H) 7.35 (d, *J*=5.02 Hz, 1H) 7.37 - 7.42 (m, 1H) 7.47 - 7.52 (m, 2 H) 8.60 (d, *J*=5.02 Hz, 1H) (e) 1H NMR (400 MHz, DMSO-*d*₆) d ppm 0.20 - 0.34 (m, 2 H) 0.59 (dd, *J*=7.03, 2.01 Hz, 2 H) 2.56 - 2.63 (m, 1H) 2.64 (s, 3 H) 3.80 (t, *J*=5.52 Hz, 2 H) 4.61 (t, *J*=5.52 Hz, 2 H) 6.95 (d, *J*=5.52 Hz, 1H) 7.33 - 7.44 (m, 1H) 7.45 - 7.56 (m, 2 H) 8.48 (d, J=5.52 Hz, 1H) (e) Die Produkte D-06 und E-06 konnten säulenchromatographisch nicht getrennt werden. | | | | | |

### 5) Synthese der Aminbausteine F:

Nachfolgend sind in der Tabelle 3 die zur Synthese der speziellen Ausführungformen der erfindungsgemäßen Verbindungen eingesetzten Aminbausteine F aufgelistet, sowie, insofern diese nicht kommerziell erhältlich sind, deren Herstellung beschrieben.

**Tabelle 3: Aminbausteine F**

| **Beispiel Nr.** | **Struktur** | **Name** |
|---|---|---|
| F-01 | 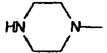 | 1-Methylpiperazin (F-01) |
| F-02 | 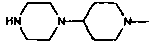 | 1-(1-Methylpiperidin-4-yl)piperazin (F-02) |
| F-03 | 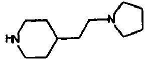 | 4-(2-(Pyrrolidin-1-yl)ethyl)piperidin (F-03) |
| F-04 | 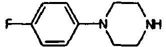 | 1-(4-Fluorophenyl)piperazin (F-04) |
| F-05 | 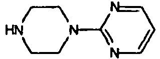 | 2-(Piperazin-1-yl)pyrimidin (F-05) |
| F-06 | 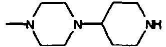 | 1-Methyl-4-(piperidin-4-yl)piperazin (F-06) |
| F-07 | 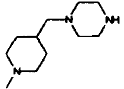 | 1-((1-Methylpiperidin-4-yl)methyl)piperazin (F-07) |
| F-08 | 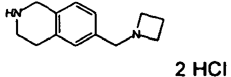 | 6-(Azetidin-1-ylmethyl)-1,2,3,4-tetrahydroisoquinolin dihydrochlorid (F-08) |
| F-09 | 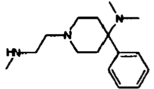 | N,N-Dimethyl-1-(2-(methylamino)ethyl)-4-phenylpiperidin-4-amin (F-09) |
| F-10 | 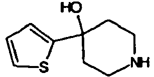 | 4-(Thiophen-2-yl)piperidin-4-ol (F-10) |
| F-11 | 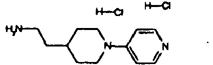 | 2-(1-(Pyridin-4-yl)piperidin-4-yl)ethanamin Dihydrochlorid (F-11) |
| F-12 | 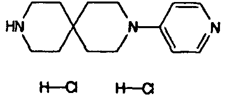 | 3-(Pyridin-4-yl)-3,9-diazaspiro[5.5]undecan Dihydrochlorid (F-12) |
| F-13 | 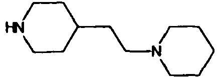 | 1,4'-(Ethan-1,2-diyl)dipiperidin (F-13) |
| F-14 | 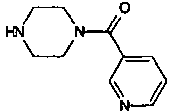 | Piperazin-1-yl(pyridin-3-yl)methanon (F-14) |
| F-15 | 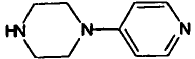 | 1-(Pyridin-4-yl)piperazine (F-15) |
| F-16 | 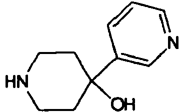 | 4-(Pyridin-3-yl)piperidin-4-ol (F-16) |
| F-17 | 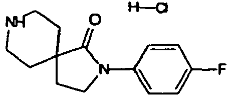 | 2-(4-Fluorophenyl)-2,8-diazaspiro[4.5]decan-1-on Hydrochlorid (F-17) |
| F-18 | 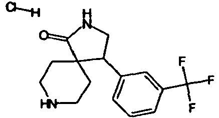 | 4-(3-(Trifluoromethyl)phenyl)-2,8-diazaspiro[4.5]decan-1-on Hydrochlorid (F-18) |
| F-19 | 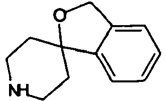 | 3H-Spiro[isobenzofuran-1,4'-piperidin] (F-19) |
| F-20 | 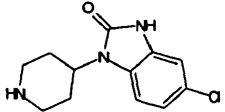 | 5-Chloro-1-(piperidin-4-yl)-1H-benzo[d]imidazol-2(3H)-on (F-20) |
| F-21 | 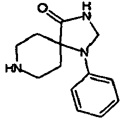 | 1-Phenyl-1,3,8-triazaspiro[4.5]decan-4-on (F-21) |
| F-22 | 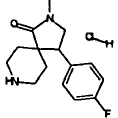 | 4-(4-Fluorophenyl)-2-methyl-2,8-diazaspiro[4.5]decan-1-on Hydrochlorid (F-22) |
| F-23 | 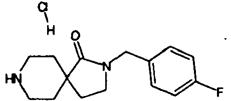 | 2-(4-Fluorobenzyl)-2,8-diazaspiro[4.5]decan-1-on Hydrochloride (F-23) |
| F-24 | 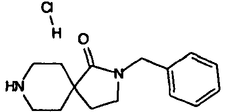 | 2-Benzyl-2,8-diazaspiro[4.5]decan-1-on Hydrochloride (F-24) |
| F-25 | 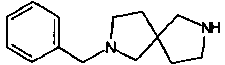 | 2-Benzyl-2,7-diazaspiro[4.4]nonan (F-25) |
| F-26 | 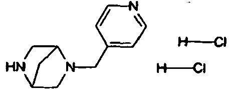 | 2-(Pyridin-4-ylmethyl)-2,5-diazabicyclo[2.2.1]heptan Dihydrochloride (F-26) |
| F-27 | 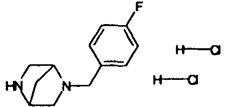 | 2-(4-Fluorobenzyl)-2,5-diazabicyclo[2.2.1]heptan Dihydrochloride (F-27) |
| F-28 | 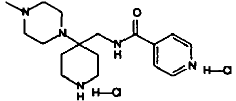 | N-((4-(4-Methylpiperazin-1-yl)piperidin-4-yl)methyl)isonicotinamid Dihydrochlorid (F-28) |
| F-31 | 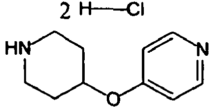 | 4-(Piperidin-4-yloxy)pyridin dihydrochlorid (F-31) |
| F-37 | 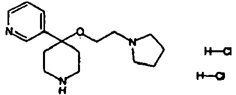 | 3-(4-(2-(Pyrrolidin-1-yl)ethony)piperidin-4-yl)pyridin Dihydrochlorid (F-37) |
| F-38 | 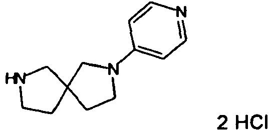 | 2-(Pyridin-4-yl)-2,7-diazaspiro[4.4]nonan dihydrochlorid (F-38) |
| F-39 | 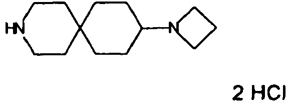 | 9-(Azetidin-1-yl)-3-azaspiro[5.5]undecan dihydrochlorid (F-39) |
| F-40 | 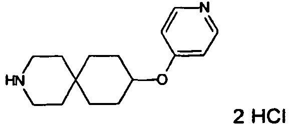 | 9-(Pyridin-4-yloxy)-3-azaspiro[5.5]undecan dihydrochlorid (F-40) |
| F-41 | 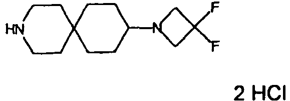 | 9-(3,3-Difluoroazetidin-1-yl)-3-azaspiro[5.5]undecan dihydrochlorid ( F-41) |
| F-42 | 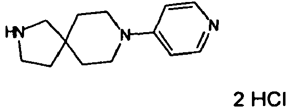 | 8-(Pyridin-4-yl)-2,8-diazaspiro[4.5]decan dihydrochlorid (F-42) |
| F-43 | 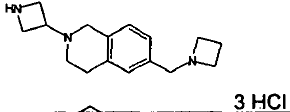 | 6-(Azetidin-1-ylmethyl)-2-(azetidin-3-yl)-1,2,3,4-tetrahydroisoquinolin trihydrochlorid (F-43) |
| F-44 | 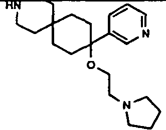 | 3-Pyridin-3-yl-3-(2-pyrrolidin-1-yl-ethoxy)-9-azaspiro[5.5]undecan (F-44) |
| F-45 | 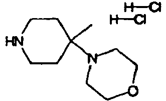 | 4-(4-Methyl-piperidin-4-yl)-morpholin dihydrochlorid (F-45) |
| F-46 | 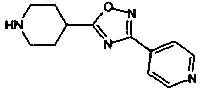 | 5-Piperidin-4-yl-3-pyridin-4-yl-[1,2,4]oxadiazol (F-46) |
| F-47 | 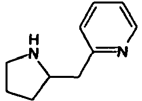 | 2-(Pyrrolidin-2-yl-methyl)-pyridin (F-47) |
| F-48 | 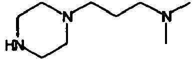 | Dimethyl-(3-piperazin-1-yl-propyl)-amin (F-48) |
| F-49 | 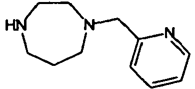 | 1-(Pyridin-2-yl-methyl)-[1,4]diazepan (F-49) |
| F-50 | 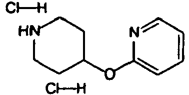 | 2-Piperidin-4-yloxy-pyridin dihydrochlorid (F-50) |
| F-51 | 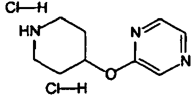 | 2-Piperidin-4-yloxy-pyrazin dihydrochlorid (F-51) |
| F-52 | 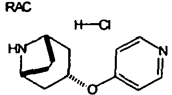 | (1S,5R)-3-Pyridin-4-yloxy-8-azabicyclo[3.2.1]octan hydrochlorid (F-52) |
| F-53 | 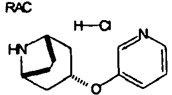 | (1S,5R)-3-Pyridin-3-yloxy-8-azabicyclo[3.2.1]octan hydrochlorid (F-53) |
| F-54 | 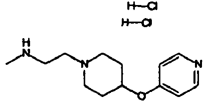 | Methyl-[2-(4-pyridin-4-yloxy-piperidin-1-yl)-ethyl]-amin dihydrochlorid (F-54) |
| F-55 | 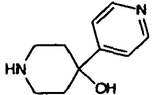 | 4-Pyridin-4-yl-piperidin-4-ol (F-55) |
| F-56 | 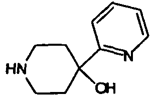 | 4-Pyridin-2-yl-piperidin-4-ol (F-56) |
| F-57 | 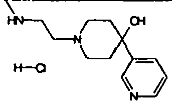 | 1-(2-Methylamino-ethyl)-4-pyridin-3-yl-piperidin-4-ol hydrochlorid (F-57) |
| F-58 | 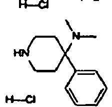 | Dimethyl-(4-phenyl-piperidin-4-yl)-amin dihydrochlorid (F-58) |
| F-59 | 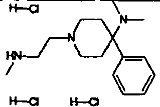 | Dimethyl-[1-(2-methylamino-ethyl)-4-phenyl-piperidin-4-yl]-amin trihydrochlorid (F-59) |
| F-60 | 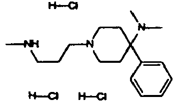 | Dimethyl-[1-(3-methylamino-propyl)-4-phenyl-piperidin-4-yl]-amin trihydrochlorid (F-60) |
| F-61 | 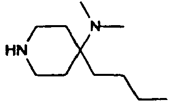 | (4-Butyl-piperidin-4-yl)-dimethyl-amin (F-61) |
| F-62 | 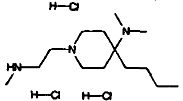 | [4-Butyl-1-(2-methylamino-ethyl)-piperidin-4-yl]-dimethyl-amin trihydrochlorid (F-62) |
| F-63 | 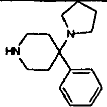 | 4-Phenyl-4-pyrrolidin-1-yl-piperidin (F-63) |
| F-64 | 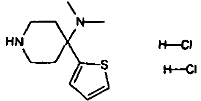 | Dimethyl-(4-thiophen-2-yl-piperidin-4-yl)-amin dihydrochlorid (F-64) |
| F-65 | 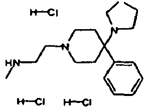 | Methyl-[2-(4-phenyl-4-pyrrolidin-1-yl-piperidin-1-yl)-ethyl]-amin trihydrochlorid (F-65) |
| F-66 | 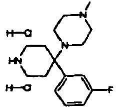 | 1-[4-(3-Fluorphenyl)-piperidin-4-yl]-4-methyl-piperazin dihydrochlorid (F-66) |
| F-67 | 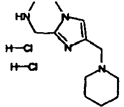 | 2-(Piperidin-1-yl-methyl)-5,6,7,8-tetrahydro-imidazo[1,2-a]pyrazin dihydrochlorid (F-67) |
| F-68 | 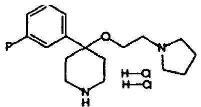 | 4-(3-Fluorphenyl)-4-(2-pyrrolidin-1-yl-ethoxy)-piperidin dihydrochlorid (F-68) |
| F-69 | 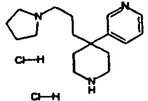 | 3-[4-(3-Pyrrolidin-1-yl-propyl)-piperidin-4-yl]-pyridin dihydrochlorid (F-69) |

**Amin F-01: 1-Methylpiperazin** [109-01-3] kommerziell erhältlich bei z.B. Aldrich. **Amin F-02: 1-(1-Methylpiperidin-4-yl)piperazin** [23995-88-2] kommerziell erhältlich bei z.B. Fluka. **Amin F-03: 4-(2-(Pyrrolidin-1-yl)ethyl)piperidin** [14759-08-1] kommerziell erhältlich bei z.B. ABCR. **Amin F-04: 1-(4-Fluorophenyl)piperazin** [16141-90-5] kommerziell erhältlich bei z.B. Aldrich. **Amin F-05: 2-(Piperazin-1-yl)pyrimidin** [20980-22-7] kommerziell erhältlich bei z.B. Aldrich. **Amin F-06: 1-Methyl-4-(piperidin-4-yl)piperazin** [436099-90-0] kommerziell erhältlich bei z.B. ABCR. **Am in F-07: 1 1-((1-Methylpiperidin-4-yl)methyl)piperazin** [735262-46-1] kommerziell erhältlich bei z.B. Otava.

### Amin F-08: 6-(Azetidin-1-ylmethyl)-1,2,3,4-tetrahydroisoquinolin dihydrochlorid

(i): 2-(2,2,2-Trifluoracetyl)-1,2,3,4-tetrahydroisoquinolin-6-carbaldehyd (2 g, 7.78 mmol) und Azetidin (532 mg, 9.33 mmol) wurden in 1,2-Dichlorethan (37 ml) vorgelegt und mit Natriumtriacetoxyborhydrid (2.31 g, 10.89 mmol) versetzt. Das Reaktionsgemisch wurde 15 h gerührt, anschließend mit Dichlormethan verdünnt und mit gesättigter Natriumhydrogencarbonatlösung (100 ml) versetzt. Nach Phasentrennung wurde die wässrige Phase mit Dichlormethan (3 x 100 ml) extrahiert. Die vereinigten organischen Phasen wurden mit gesättigter Natriumchloridlösung (50 ml) gewaschen, über Magnesiumsulfat getrocknet und unter Vakuum eingeengt. Das Rohprodukt wurde säulenchromatographisch (Kieselgel, Ethylacetat / Dichlormethan / Methanol / Hexan, 300:100:20:10) gereinigt. Ausbeute: 1.55 g (66%)
(ii): 1-(6-(Azetidin-1-ylmethyl)-3,4-dihydroisoquinolin-2(1H)-yl)-2,2,2-trifluorethanon (1.54 g, 5.162 mmol) wurde in Methanol (21 ml) vorgelegt, Kaliumcarbonat (1.42 g, 10.32 mmol) wurde zugegeben und das Reaktionsgemisch 15 h bei Raumtemperatur gerührt. Das Lösungsmittel wurde anschließend unter Vakuum entfernt, der Rückstand in Dichlormethan aufgenommen und mit Wasser gewaschen. Die wässrige Phase wurde mit Dichlormethan extrahiert, die vereinigten organischen Phasen über Natriumsulfat getrocknet und unter Vakuum eingeengt. Der Rückstand wurde in einem Ethanol / Diethylether Gemisch aufgenommen und mit Chlorwasserstoff in Diethylether (4 Äquiv., 2 mol/l) das Hydrochlorid gefällt, welches abgesogen unter Vakuum getrocknet wurde.
Ausbeute: 1.03 g (72%)

### Amin F-09: N,N-Dimethyl-1-(2-(methylamino)ethyl)-4-phenylpiperidin-4-amin

### Stufe 1: tert-Butyl 2-(4-(dimethylamino)-4-phenylpiperidin-1-yl)ethyl(methyl)carbamat

Zu einer Lösung von 6,5 g (1,5 Äquiv.) *tert*-Butyl methyl(2-oxoethyl)carbamat in 60 ml Methanol wurden portionsweise 7 g (1 Äquiv.) *N,N*-Dimethyl-4-phenylpiperidin-4-amin gegeben. Diese Reaktionsmischung wurde auf 0°C abgekühlt, portionsweise mit 3,97 g (2,5 Äquiv.) Natriumcyanoborhydrid versetzt und anschließend für 10 min bei Raumtemperatur gerührt. Das erhaltene Reaktionsgemisch wurde mittels Essigsäure auf einen pH ~ 5 eingestellt und für 12 h bei Raumtemperatur gerührt. Der Reaktionsverlauf wurde mittels Dünnschichtchromatographie (20% MeOH/CHCl₃) kontrolliert. Da die Umsetzung noch nicht vollständig war wurden 1,5 g Natriumcyanoborhydrid und Essigsäure zugegeben und die Reaktionsmischung für weitere 30 - 45 min gerührt.

Nach nun vollständigem Umsatz wurde das Methanol abdestilliert, 100 ml ges. NaHCO₃-Lsg. zugegeben und die erhaltene Mischung mit Chloroform (2x 200 ml) extrahiert und die vereinigten organischen Phasen wurden über Na₂SO₄ getrocknet. Nach Entfernen des Lösungsmittels unter vermindertem Druck wurde säulenchromatographisch (Kieselgel; 5% MeOH/CHCl₃) gereinigt. 8 g (64%) Produkt wurden in Form eines Öls erhalten.

### Stufe 2: N,N-Dimethyl-1-(2-(methylamino)ethyl)-4-phenylpiperidin-4-amin tris hydrochlorid

Durch eine Lösung von 9 g (1 Äquiv.) *tert*-Butyl 2-(4-(dimethylamino)-4-phenylpiperidin-1-yl)ethyl(methyl)carbamat in 600 ml CH₃Cl wurde für 30 min HCl-Gas geleitet. Der Reaktionsverlauf wurde mittels Dünnschichtchromatographie (20% MeOH/CHCl₃) kontrolliert. Nach vollständigem Umsatz wurde für weitere 30 min HCl-Gas geleitet und die vollständige Umsetzung abermals mittels Dünnschichtchromatographie (20% MeOH/CHCl₃) kontrolliert. Nach nun vollständigem Umsatz wurde das Lösungsmittel unter vermindertem Druck entfernt und das gewünschte Produkt 7,2 g (96%) in Form eines weißen Feststoffs erhalten. Die freie Base wurde nach Lösen des Hydrochlorids in wässriger Natriumhydroxid-Lösung und Extraktion dieser mit Dichlormethan erhalten.

### Amin F-10: 4-(Thiophen-2-yl)piperidin-4-ol

(i) Thiopene (10 g) wurde in trockenem THF (500 ml) gelöst, auf -78°C gekühlt und langsam mit n-BuLi (66 ml) bei -78°C versetzt. Die Reaktionsmischung wurde für 1 Stunde gerührt und anschließend mit N-Cbz-4-Piperidon (25 g) in 50 ml THF versetzt. Die erhaltene Reaktionsmischung wurde für 1 Stunde gerührt, auf Raumtemperatur erwärmt und mit gesättigter NH₄Cl (250 ml) gequencht. Die Phasen wurden getrennt, die wässrige Phase 3x mit Essigester extrahiert, die vereinigten organischen Phasen über Natriumsulfat getrocknet und unter vermindertem Druck konzentriert. Der Rückstand wurde aus EtOAc/n-Hexan umkristallisiert. Der erhaltene Feststoff wurde abfiltriert und mit EtOAc/n-Hexan gewaschen. Es wurden 22.4 g (66%) Produkt in Form eines farblosen Feststoffes erhalten.
(ii) Zu einer Lösung des soeben erhaltenen Feststoffs (10 g) in Ethanol (100 ml) wurde eine Lösung von KOH (2.7 g) in Wasser (10 ml) gegeben und das erhaltene Reaktionsgemisch für 24 Stunden zum Rückfluss erhitzt. Nach vollständigem Umsatz wurde das Reaktionsgemisch unter vermindertem Druck konzentriert, mit 30 ml Wasser versetzt und 4x mit IPA/CHCl₃ extrahiert. Die vereinigten organischen Phasen wurden über Natriumsulfat getrocknet und unter vermindertem Druck konzentriert. Der Rückstand wurde aus EtOAc/n-Hexan umkristallisiert. Es wurden 3.2g (55%) Produkt in Form eines blassbraunen Feststoffes erhalten.

### Amin F-11: 2-(1-(Pyridin-4-yl)piperidin-4-yl)ethanamin dihydrochlorid

(i): tert-butyl 2-(Piperidin-4-yl)ethylcarbamat (0.2 g, 0.876 mmol), 4-Chlorpyridiniumchlorid (0.197 g, 1.314 mmol) und N-Ethyl-diisopropylamin (0.37 ml, 2.19 mmol) wurden in 2-Propanol (10 ml) 15 h refluxiert. Gesättigte Natriumhydrogencarbonatlösung (20 ml) und Ethylacetat (50 ml) wurden zugegeben, Phasen getrennt und die wässrige Phase mit Ethylacetat (2 x 50 ml) extrahiert. Die vereinigten organischen Phasen wurden über Magnesiumsulfat getrocknet und unter Vakuum eingeengt. Das Rohprodukt wurde säulenchromatographisch (Kieselgel, Ethylacetat / Dichlormethan / Methanol / Ammoniak (25% aq), 400:100:50:1) gereinigt.
   Ausbeute: 80 mg (30%)
(ii): Zu einer Lösung tert-butyl 2-(1-(Pyridin-4-yl)piperidin-4-yl)ethylcarbamat (0.12 g, 0.393 mmol) in Methanol (3 ml) wurde bei Raumtemperatur Chlorwasserstoff (1.25 M Lösung in Methanol, 1.25 ml) hinzugegeben und das Reaktionsgemisch 1 h refluxiert. Das Lösungsmittel wurde unter Vakuum entfernt und der Rückstand getrocknet.
Ausbeute: quantitativ

### Amin F-12: 3-(Pyridin-4-yl)-3,9-diazaspiro[5.5]undecan dihydrochlorid

(i): tert-butyl 3,9-Diazaspiro[5.5]undecan-3-carboxylat (1 g, 3.931 mmol), 4-Chlorpyridiniumchlorid (1.765 g, 11.794 mmol) und Triethylamin (2.2 ml, 15.725 mmol) wurden in 1-Butanol (50 ml) 15 h refluxiert. Gesättigte Natriumhydrogencarbonatlösung (30 ml) und Ethylacetat (80 ml) wurden zugegeben, Phasen getrennt und die wässrige Phase mit Ethylacetat (2 x 80 ml) extrahiert. Die vereinigten organischen Phasen wurden über Magnesiumsulfat getrocknet und unter Vakuum eingeengt. Das Rohprodukt wurde säulenchromatographisch (Kieselgel, Ethylacetat / Hexan / Methanol / Ammoniak (25% aq), 400:40:40:1) gereinigt. Ausbeute: 0.52 g (39%)
   [Diese Umsetzung wurde anstelle von 1-Butanol als Lösungsmittel teilweise auch in 2-Propanol durchgeführt und 15 h bei 90 °C gerührt.] (ii): tert-butyl 9-(Pyridin-4-yl)-3,9-diazaspiro[5.5]undecan-3-carboxylat (0.52 g, 1.569 mmol) wurde mit Chlorwasserstoff in Methanol (1.25 mol/l, 6.3 ml) versetzt und 1 h refluxiert. Das Lösungsmittel wurde unter Vakuum entfernt, der Rückstand in Ethanol (3 ml) aufgenommen und gekühlt. Aceton (80 ml) wurde zugegeben und 30 min im Eisbad gerührt. Der Niederschlag wurde abgesogen, mit Diethylether gewaschen und unter Vakuum getrocknet.
   Ausbeute: 0.4 g (83%)

Amin F-13: 1,4'-(Ethan-1,2-diyl)dipiperidin [14759-09-2] kommerziell erhältlich bei z.B. Fluorochem. **Amin F-14: Piperazin-1-yl(pyridin-3-yl)methanon** [39640-08-9] kommerziell erhältlich bei z.B. Fluorochem. **Amin F-15: 1-(Pyridin-4-yl)piperazin** [1008-91-9] kommerziell erhältlich bei z.B. ABCR.

### Amin F-16: 4-(Pyridin-3-yl)piperidin-4-ol

(i) (Apparatur: 1 I-Dreihalskolben mit Stickstoff-Ballon) Magnesium (5.7 g) wurde in wasserfreiem Ether (125 ml) vorgelegt, 1,1-Dibromethan (0.5 g) und Isopropylchlorid (17.3 ml) wurden zugetropft und es wurde 15 min gerührt um das Magnesium zu initiieren. Eine Lösung aus 3-Brompyridin (25 g) in wasserfreiem Tetrahydrofuran (400 ml) wurde über 20 min bei 40°C zugetropft, dann wurde 2 h refluxiert. Abschliessend wurde über 20 min bei 40°C eine Lösung aus 1-Benzylpiperidin-4-on (30 g) in wasserfreiem Tetrahydrofuran (100 ml) zugetropft und über Nacht bei Raumtemperatur gerührt. Dünnschichtchromatographische Kontrolle: 10% Methanol in Chloroform. Das Reaktionsgemisch wurde bei 0°C mit Wasser (50 ml) hydrolysiert und über Celite filtriert. Es wurde mit Dichlormethan (2 x 100 ml) extrahiert, die vereinigten organischen Phasen wurden mit Wasser (50 ml) gewaschen, über Natriumsulfat getrocknet und unter Vakuum eingeengt. Das Rohprodukt wurde säulenchromatographisch (Alox neutral) mit 5% Methanol in Chloroform gereinigt. Ausbeute: 8.2 g (19.3%)
(ii) (Apparatur: 1 I-Dreihalskolben mit Kühler) Zu einer Lösung aus 1-Benzyl-4-(pyridin-3-yl)piperidin-4-ol (32 g) in Methanol (220 ml) wurde Palladium auf Kohle (10%, katalytische Menge) gegeben, gefolgt von Ammoniumformat-Lösung (22.7 g in 50 ml Wasser). Das Reaktionsgemisch wurde über Nacht bei 68°C refluxiert. Dünnschichtchromatographische Kontrolle: 20% Methanol in Chloroform. Es wurde über Celite filtriert, das Filtrat wurde unter Vakuum eingeengt. Der Rückstand wurde mit Aceton (100 ml) gewaschen um die gewünschte Verbindung sauber zu erhalten.
Ausbeute: 17.3 g (81.3%)

Amin F-17: 2-(4-Fluorophenyl)-2,8-diazaspiro[4.5]decan-1-one hydrochlorid (MDL No.: MFCD05861564) kommerziell erhältlich bei z.B. ASW MedChem. Amin F-**18: 4-(3-(Trifluoromethyl)phenyl)-2,8-diazaspiro[4.5]decan-1-one hydrochlorid** kommerziell erhältlich bei z.B. ASW MedChem. **Amin F-19: 3H-Spiro[isobenzofuran-1,4'-piperidin]** [38309-60-3] kommerziell erhältlich bei z.B. Chem Impex. **Amin F-20: 3H 5-Chloro-1-(piperidin-4-yl)-1H-benzo[d]imidazol-2(3H)-one** [53786-28-0] kommerziell erhältlich bei z.B. Aldrich. **Amin F-21: 1-Phenyl-1,3,8-triazaspiro[4.5]decan-4-on** [1021-25-6] kommerziell erhältlich bei z.B. ABCR. **Amin F-22: 4-(4-Fluorophenyl)-2-methyl-2,8-diazaspiro[4.5]decan-1-on hydrochlorid** (MDL No: MFCD08460813) kommerziell erhältlich bei z.B. ASW MedChem. **Amin F-23: 2-(4-Fluorobenzyl)-2,8-diazaspiro[4.5]decan-1-on hydrochlorid** (MDL No: MFCD08461093) kommerziell erhältlich bei z.B. ASW MedChem. **Amin F-24: 2-Benzyl-2,8-diazaspiro[4.5]decan-1-one hydrochlorid** (MDL No: MFCD02179153) kommerziell erhältlich bei z.B. ASW MedChem. **Amin F-25: 2-Benzyl-2,7-diazaspiro[4.4]nonan (MDL No: MFCD04115133) kommerziell** erhältlich bei z. B. Tyger.

### Amin F-26: 2-(Pyridin-4-ylmethyl)-2,5-diazabicyclo[2.2.1]heptan dihydrochlorid

(i): tert-Butyl 2,5-diazabicyclo[2.2.1]heptan-2-carboxylat (5 g, 25,214 mmol) und Pyridin-4-carbaldehyd (2,97 g, 27,74 mmol) wurden in Dichlormethan (650 ml) vorgelegt, mit Natriumtriacetoxyborhydrid (10,6 g, 50,43 mmol) und Eisessig (0,14 ml, 2,521 mmol) versetzt und das Reaktionsgemisch für 15 h bei Raumtemperatur gerührt. Anschließend wurde mit gesättigter Natriumhydrogencarbonat-Lösung hydrolysiert, die Phasen getrennt und die wässrige Phase 2x mit Diethylether extrahiert: Die vereinigten organischen Phasen wurden mit gesättigter Natriumchlorid-Lösung gewaschen, über Magnesiumsulfat getrocknet und unter vermindertem Druck konzentriert. Das Rohprodukt wurde säulenchromatographisch gereinigt (Kieselgel; Dichlormethan/Methanol).
   Ausbeute: 5,8 g, 79%
(ii): tert-butyl 5-(pyridin-4-ylmethyl)-2,5-diazabicyclo[2.2.1]heptane-2-carboxylate (5.8 g, 20.0 mmol) wurde in Methanol (50 ml) gelöst, im Eisbad gekühlt und mit Acetylchlorid (7.1 ml) versetzt. Die Reaktionsmischung wurde 15 h bei Raumtemperatur gerührt und anschließend unter vermindertem Druck konzentriert. Der Rückstand wurde in Wasser aufgenommen, die wässrige Phase 2x mit Dichlormethan gewaschen, eingefroren und das Wasser unter Gefriertrocknung entfernt. Ausbeute: 5.2 g, 99%

Das in der nachfolgenden Tabelle aufgeführte Amin **F-27** wurden aus *tert*-Butyl 2,5-diazabicyclo[2.2.1]heptane-2-carboxylat durch Umsetzung mit dem entsprechenden Aldehyd und anschließender Schutzgruppenabspaltung analog zu Amin **F-38** hergestellt.

| **Amin** | **Aldehyd** | **Ausbeute** *(nach 2 Stufen)* |
|---|---|---|
| 2-(4-Fluorobenzyl)-2,5-diazabicyclo[2.2.1]heptan dihydrochlorid (F-27) | 4-Fluor-benzaldehyd | 77% |

### Amin F-28: N-((4-(4-Methylpiperazin-1-yl)piperidin-4-yl)methyl)isonicotinamid dihydrochlorid

(i) Eine Mischung aus N-Boc-Piperidon (10.0g, 50.2 mmol), 1-Methylpiperazin (5.57 ml, 50.2 mmol), Wasser (1.18 ml, 65.2 mmol) und Essigsäure (3.18 ml, 55.2 mmol) in Methanol (20 ml) wurde bei RT unter Stickstoffatmosphäre gerührt. KCN (3.44g, 52.8 mmol) wurde zugegeben und der Reaktionsansatz wurde bei RT gerührt. Nach 30 min fiel ein Feststoff aus. Zu der Reaktionsmischung wurde wäßrige NH₄OH-Lösung (35 %, 300 ml) und Eis (100 g) zugeben. Der Ferststoff wurde filtriert, getrocknet und ohne weitere Aufreinigung weiter eingesetzt.
(ii) Eine Lösung LAH (1.0 M in Diethylether, 34.6 ml, 34.6 mmol) wurde auf 0°C abgekühlt und das Produkt aus Stufe (i), gelöst in Diethylether (150 ml), wurde tropfenweise zugegeben. Anschließend wurde für 2 h bei 0°C gerührt. Anschließend wurde bei 0°C Na₂SO₄·10 H₂O zugegeben, bis keine Gasentwicklung mehr festgestellt werden konnte. Die Reaktionsmischung wurde filtriert und mit DCM gewaschen. Das Lösungsmittel wurde entfernt und das Rohprodukt ohne weitere Aufreinigung weiter eingesetzt.
(iii) Zu einer Lösung des Rohprodukts aus Stufe (ii) (max. 9.89 mmol) in DCM (125 ml) wurde bei RT Triethylamin (4.2 ml, 29.7 mmol) und Isonicotinoylchlorid Hydrochlorid (1.20g, 6.74 mmol) gegeben. Der Reaktionsansatz wurde 3 h bei RT gerührt und anschließend zur Trockne eingeengt. Das Rohprodukt wurde säulenchromatographisch aufgereinigt (Kieselgel, DCM, 7 M NH₃ in Methanol, 95 : 5)
(iv) Zu einer Lösung des Produktes aus Stufe (iii) (490 mg, 1.17 mmol) in Dioxan (10 ml) wurde HCl (4 M in Dioxan, 2.35 ml, 9.4 mmol) gegeben und 3 h bei RT gerührt. Das Lösungsmittel wurde entfernt und das Rohprodukt ohne weitere Aufreinigung weiter eingesetzt.

### Amin F-31: 4-(Piperidin-4-yloxy)pyridin dihydrochlorid

(i) Zu einer Lösung von 4-Hydroxypyridin (3 g, 31.546 mmol) in Tetrahydrofuran (50 ml) wurden bei Raumtemperatur tert-Butyl-4-hydroxypiperidin-1-carboxylat (6.348 g, 31.546 mmol) und Triphenylphosphin (10.256 g, 39.432 mmol) hinzugegeben. Anschließend wurde Diisopropyl-azodicarboxylat (7.66 ml, 39.432 mmol) hinzugetropft und das Gemisch dann 15 h bei 55 °C gerührt. Das Reaktionsgemisch wurde mit gesättigter Natriumhydrogencarbonatlösung versetzt (50 ml) und mit Ethylacetat (4 x 80 ml) extrahiert. Die vereinigten organischen Phasen wurden mit ges. Natriumchlorid-Lösung (20 ml) gewaschen, getrocknet (Na₂SO₄) und unter Vakuum eingeengt. Anschließend wurde das Rohprodukt säulenchromatographisch (Kieselgel, Ethylacetat / Hexan, 4:1) gereinigt.
   Ausbeute: 4.11 g (46%)
(ii) Zu einer Lösung tert-Butyl 4-(pyridin-3-yloxy)piperidin-1-carboxylat (4.1 g, 14.727 mmol) in Methanol (10 ml) wurde bei Raumtemperatur Chlorwasserstoff (47 ml, 59 mmol, 1.25 M Lösung in Methanol) hinzugegeben und das Reaktionsgemisch 30 min refluxiert. Das Lösungsmittel wurde unter Vakuum entfernt und der Rückstand in ein wenig Ethanol aufgenommen und mit Diethylether versetzt. Anschließend wurde 30 min im Eisbad gekühlt und der entstandene Feststoff abfiltriert und getrocknet. Ausbeute: 3.46 g (93%)

### Am in F-37: 3-(4-(2-(Pyrrolidin-1-yl)ethoxy)piperidin-4-yl)pyridin dihydrochlorid

(i) Zu einer Lösung von 3-Brompyridin (7.94 g, 1Äquiv.) in trockenem THF (1600 ml) wurde bei -70°C n-Butyllithium (2 Äquiv.) gegeben und bei dieser Temperatur für 1 h gerührt. Anschließend wurde bei -70°C eine Lösung aus N-Boc-Piperidon (10 g, 1 Äquiv.) in THF (400 ml) zugegeben und bei dieser Temperatur für 2 h gerührt (DC-Kontrolle). Nach beendeter Reaktion wurde mit gesättigter Ammoniumchlorid-Lösung hydrolysiert und anschließend langsam auf RT erwärmt. Es wurde mit Ethylacetat verdünnt. Die organische Phase wurde mit Natriumchlorid-Lösung gewaschen und über Natriumsulfat getrocknet. Das Lösungsmittel wurde am Rotationsverdampfer entfernt und das erhaltene Rohprodukt säulenchromatographisch (Kieselgel, DCM /Methanol, 9:1) aufgereinigt.
(ii) Der Alkohol (2 g) wurde in Benzol (20 ml) gelöst, bei 25°C mit Natriumamid (10 Äquiv.) versetzt und 15 min bei dieser Temperatur gerührt. Es wurde anschließend 1-(2-Chlorethyl)pyrrolidin (1.2 Äquiv.) zugegeben und 16 h unter Rückfluß erhitzt. Nach beendeter Reaktion (DC-Kontrolle) wurde auf 0°C abgekühlt und mit Eis hydrolysiert. Die wäßrige Phase wurde mit Ethylacetat extrahiert. Die organische Phase wurde mit Wasser und gesättigter NaCl-Lösung gewaschen und über Na₂SO₄ getrocknet. Das Lösungsmittel wurde am Rotationsverdampfer entfernt und das erhaltene Rohprodukt säulenchromatographisch (Kieselgel, DCM / Methanol, 95:5) aufgereinigt. (iii) tert-Butyl 4-(pyridin-3-yl)-4-(2-(pyrrolidin-1-yl)ethoxy)piperidin-1-carboxylat (12.7 g, 33.82 mmol) wurde in Methanol (80 ml) gelöst, im Eisbad gekühlt und mit Acetylchlorid (12 ml, 169.1 mmol) versetzt. Nach 3 h war die Reaktion laut DC-Kontrolle (Dichlormethan / Methanol, 9:1) beendet, das Lösungsmittel wurde unter Vakuum entfernt und der Rückstand in Wasser / Dichlormethan aufgenommen. Die Phasen wurden getrennt, die wässrige Phase wurde mit Dichlormethan (zweimal) gewaschen und an der Gefriertrocknung getrocknet.
   Ausbeute: quantitativ

### Amin F-38: 2-(Pyridin-4-yl)-2,7-diazaspiro[4.4]nonane

Die Herstellung erfolgte in 2 Stufen analog der Synthese des Amin F-12 aus tert-Butyl 2,7-diazaspiro[4.4]nonan-2-carboxylat und 4-Chlorpyridiniumchlorid.

### Amin F-39: 9-(Azetidin-1-yl)-3-azaspiro[5.5]undecan dihydrochlorid

(i): tert-Butyl 9-oxo-3-azaspiro[5.5]undecane-3-carboxylate (1 g, 3.74 mmol) und Azetidin (0.25 ml, 3.74 mmol) wurden in 1,2-Dichlorethan (15 ml) vorgelegt und mit Natriumtriacetoxyborhydrid (1.1 g, 5.23 mmol) versetzt. Das Reaktionsgemisch wurde 3 d bei Raumtemepartur gerührt und anschließend mit gesättigter Natriumhydrogencarbonatlösung versetzt. Nach Phasentrennung wurde die wässrige Phase mit Dichlormethan (2 x) extrahiert. Die vereinigten organischen Phasen wurden mit gesättigter Natriumchloridlösung (1 x) gewaschen, über Magnesiumsulfat getrocknet und unter Vakuum eingeengt. Das Rohprodukt wurde säulenchromatographisch (Kieselgel, Ethylacetat / Methanol / Ammoniak (25% aq), 100:10:1) gereinigt.
   Ausbeute: 1 g (89%)
(ii): tert-Butyl 9-(azetidin-1-yl)-3-azaspiro[5.5]undecane-3-carboxylat (1 g, 3.24 mmol) wurde mit Chlorwasserstoff in Methanol (1.25 mol/l, 15.5 ml) versetzt und 45 min refluxiert. Das Lösungsmittel wurde unter Vakuum entfernt und der Rückstand in einer geringen Menge Ethanol gelöst. Anschließend wurde durch Zugabe von Aceton ein Feststoff ausgefällt, schließlich Diethylether hinzugegeben und der entstandene Niederschlag abgesogen.
Ausbeute: 0.87 g (95%)

### Amin F-40: 9-(Pyridin-4-yloxy)-3-azaspiro[5.5]undecan Dihydrochlorid

### Stufe (i): 1-(Benzyloxycarbonyl)piperidin-4-carbonsäure

Zu Piperidin-4-carbonsäure (25 g) in THF (75 ml) wurde Wasser (75 ml) gegeben, gefolgt von Natriumbicarbonat (30.8 g). Das Gemisch wurde auf to 0°C gekühlt und Cbz chlorid (38.9 ml) hinzugetropft. Anschließend wurde das Reaktionsgemisch 5 h bei Raumtemperatur gerührt (DC-Kontrolle). Nach vollständiger Umsetzung wurde das organische Lösungsmittel abdestilliert und der Rückstand in Wasser (200 ml) aufgenommen, mit Ethylacetat (2 x 150 ml) gewaschen. Die wässrige Phase wurde mit verdünnter wässriger HCl Lösung sauer gestellt und mit Ethylacetat extrahiert. Die organische Phase wurde getrocknet (Na₂SO₄) und unter Vakuum aufkonzentriert.
Ausbeute: 48.5 g (96%)

### Stufe (ii): 1-Benzyl 4-methyl piperidin-1,4-dicarboxylat

1-(Benzyloxycarbonyl)piperidin-4-carbonsäure (48.5 g) in Methanol (485 ml) wurde auf 0°C gekühlt und Thionylchlorid (13.34 ml) hinzugetropft. Das Gemisch wurde anschließend 20 min refluxiert (DC-Kontrolle). Nach vollständiger Umsetzung wurde das Methanol abdestilliert, der Rückstand in Wasser (15 ml) aufgenommen und mit Ethylacetat (2 x 150 ml). Die vereinigten organischen Phasen wurden mit Wasser und ges. Natriumchloridlösung extrahiert, getrocknet (Na₂SO₄) und unter Vakuum aufkonzentriert.
Ausbeute: 38 g (67%)

### Stufe (iii): Benzyl 4-formylpiperidin-1-carboxylat

Eine Lösung 1-Benzyl 4-methyl piperidin-1,4-dicarboxylat (10 g) in Toluol (100 ml) unter Stickstoff wurde auf -78°C gekühlt. Anschließend wurde DIBAL-H (60.9 ml) bei -78°C hinzugetropft und das Gemisch 1 h bei dieser Temperatur gerührt (DC-Kontrolle). Aufgrund unvollständiger Umsetzung wurde weitere 0.2 Äq DIBAL-H hinzugegeben und weitere 30 min gerührt (DC-Kontrolle: etwas Edukt und der entsprechende Alkohol waren zu erkennen). Zu dem Reaktionsgemisch wurden langsam bei -78°C Methanol (40 ml), gefolgt von ges. Natriumchloridlösung (40 ml) hinzugegeben. Das Gemisch wurde über Celite filtriert, das Lösungsmittel unter Vakuum entfernt. Der Rückstand wurde mit Ethylacetat (3 x 75 ml) extrahiert, getrocknet (Na₂SO₄) und unter Vakuum aufkonzentriert. Das so erhaltene Rohprodukt wurde säulenchromatographisch aufgereinigt (Kieselgel, 20% Ethylacetat / Hexan).
Ausbeute: 4.3 g (49%)

### Stufe (iv): Benzyl 9-oxo-3-azaspiro[5.5]undec-7-en-3-carboxylat

Methylvinylketon (1.64 ml), Ethanol (5 ml) and Wasser (5 ml) wurden zu Benzyl 4-formylpiperidin-1-carboxylat (5 g) gegeben. Anschließend wurde das Gemisch zu einer kochenden Lösung Kaliumhydroxid (0,22 g) in Ethanol (10 ml) gegeben und das resultierende Reaktionsgemisch 1 h refluxiert (DC-Kontrolle). Nach vollständiger Umsetzung wurde das Gemisch auf Wasser (25 ml) gegeben und mit Ethylacetat (2 x 50 ml) extrahiert. Die vereinigten organischen Phasen wurden getrocknet (Na₂SO₄) und unter Vakuum aufkonzentriert. Das so erhaltene Rohprodukt wurde säulenchromatographisch aufgereinigt (Kieselgel, 25% Ethylacetat / Hexan).
Ausbeute: 2.8 g (46%)

### Stufe (v): tert-Butyl 9-oxo-3-azaspiro[5.5]undecan-3-carboxylat

Boc-Anhydrid (9.4 ml) und Kaliumcarbonat (7.56 g) wurden zu Benzyl 9-oxo-3-azaspiro[5.5]undec-7-en-3-carboxylat (8.2 g) in EtOH / Wasser (9:1) (200 ml) gegeben. Anschließend wurde Pd/C (1 g) hinzugegeben und 4 h bei 80 psi hydrogenolysiert (DC-Kontrolle). Nach vollständiger Umsetzung wurde das Gemisch über Celite filtriert und mit Ethanol und Ethylacetat nachgespült. Das Filtrat wurde getrocknet (Na₂SO₄) und unter Vakuum aufkonzentriert. Der Rückstand wurde in Ethylacetat und Wasser aufgenommen und die wässrige Phase mit Ethylacetat extrahiert. Die vereinigten organischen Phasen wurden getrocknet (Na₂SO₄) und unter Vakuum aufkonzentriert. Das so erhaltene Rohprodukt wurde säulenchromatographisch aufgereinigt (Kieselgel, 20% Ethylacetat / Hexan).
Ausbeute: 2.92 g (40%)

### Stufe (vi): tert-Butyl 9-hydroxy-3-azaspiro[5.5]undecan-3-carboxylat

tert-Butyl 9-oxo-3-azaspiro[5.5]undecan-3-carboxylat (1.5 g) wurde in THF (7.5 ml) gelöst und auf -5°C gekühlt. Anschließend wurde NaBH₄ (0.212 g) hinzugegeben und das Gemisch 1 h bei Raumtemperatur gerührt (DC- Kontrolle). Nach vollständiger Umsetzung wurde zu dem Gemisch Essigsäure gegeben und das Methanol anschließend abdestilliert. Der Rückstand wurde in Wasser (50 ml) aufgenommen und mit Ethylacetat (2 x 50 ml) extrahiert. Die vereinigten organischen Phasen wurden getrocknet (Na₂SO₄) und unter Vakuum aufkonzentriert. Das so erhaltene Rohprodukt wurde säulenchromatographisch aufgereinigt (Kieselgel, 30% Ethylacetat / Hexan).
Ausbeute: 1.2 g (80%)

### Stufe (vii): tert-Butyl 9-(pyridin-4-yloxy)-3-azaspiro[5.5]undecan-3-carboxylat

Zu Natriumhydrid (0.89 g) in DMSO (20 ml) wurde 4-Chlorpyridin Hydrochlorid (1.3 g) gegeben und das Gemisch 10 min gerührt. Anschließend wurde tert-Butyl 9-hydroxy-3-azaspiro[5.5]undecan-3-carboxylat (2.0 g) in DMSO (20 ml) langsam hinzugegeben und das Gemisch über Nacht gerührt (DC-Kontrolle: Umsetzung ca. 30 - 35 %). Eine katalytische Menge Natriumiodid wurde hinzugegeben und das Reaktionsgemisch 8 h bei 80°C gerührt (DC-Kontrolle). Zu dem Reaktionsgemisch wurde Methanol und NaHCO₃ Lösung gegeben und 20 min gerührt. Dann wurde mit Ethylacetat extrahiert und wiederum mit NaHCO₃ Lösung und kaltem Wasser gewaschen. Die organische Phase wurde getrocknet (Na₂SO₄) und unter Vakuum aufkonzentriert. Das so erhaltene Rohprodukt wurde säulenchromatographisch aufgereinigt (Kieselgel, 70% Ethylacetat / Hexan).
Ausbeute: 1.0 g (40%)

### Stufe (viii): 9-Pyridin-4-yloxy-3-azaspiro[5.5]undecan Dihydrochlorid

tert-Butyl 9-(pyridin-4-yloxy)-3-azaspiro[5.5]undecan-3-carboxylat (1 g, 2.886 mmol) wurde in Methanol (2 ml) gelöst und mit Chlorwasserstoff in Methanol (1.25 mol/l, 11.5 ml) versetzt und 30 min refluxiert. Das Lösungsmittel wurde unter Vakuum entfernt und der Rückstand in einer geringen Menge Ethanol gelöst. Anschließend wurde Aceton (ca. 25 ml) hinzugegeben, das Gemisch 30 min bei 0°C gerührt und schließlich der entstandene Feststoff abgesogen.
Ausbeute: 0.96 g (>99%)

### Amin F-41: 9-(3,3-Difluoroazetidin-1-yl)-3-azaspiro[5.5]undecan dihydrochlorid

Die Herstellung erfolgte in 2 Stufen analog der Synthese des Amin F-39 aus tert-Butyl 9-oxo-3-azaspiro[5.5]undecane-3-carboxylat und 3,3-Difluoroazetidin hydrochlorid unter Zugabe von Triethylamin (1 Äquiv.) bzw. wie folgt:

### Stufe (i): tert-Butyl 9-(3,3-difluorazetidin-1-yl)-3-azaspiro[5.5]undecan-3-carboxylat

Zu 3,3-Difluorazetidin Hydrochlorid (0.484 g, 3.74 mmol) und Triethylamin (0.52 ml, 3.74 mmol) in 1,2-Dichlorethan (15 ml) wurde tert-Butyl 9-oxo-3-azaspiro[5.5]undecan-3-carboxylat (Stufe (iv) **Amin F-40)** (1 g, 3.74 mmol) hinzugegeben. Das Gemisch wurde 5 min gerührt und anschließend mit Natriumtriacetoxyborhydrid (1.1 g, 5.23 mmol) versetzt und 3 d bei Raumtemepartur gerührt. Es wurde gesättigte Natriumhydrogencarbonatlösung hinzugegeben und nach Phasentrennung wurde die wässrige Phase mit Dichlormethan (2 x) extrahiert. Die vereinigten organischen Phasen wurden mit gesättigter Natriumchloridlösung (1 x) gewaschen, über Magnesiumsulfat getrocknet und unter Vakuum eingeengt.
Ausbeute: 1.26 g (98%)

### Stufe (ii): 9-(3,3-Difluor-azetidin-1-yl)-3-azaspiro[5.5]undecan Dihydrochlorid

tert-Butyl 9-(3,3-difluorazetidin-1-yl)-3-azaspiro[5.5]undecan-3-carboxylat (1.26 g, 3.66 mmol) wurde in Chlorwasserstoff in Methanol (1.25 mol/l, 29 ml) gelöst und 45 min refluxiert. Das Lösungsmittel wurde unter Vakuum entfernt und der Rückstand in einer geringen Menge Ethanol gelöst. Anschließend wurde durch Zugabe von Aceton ein Feststoff ausgefällt. Das Gemisch wurde 10 min bei Raumtemperatur gerührt, dann Diethylether hinzugegeben und weitere 30 min bei Raumtemperatur gerührt. Der entstandene Niederschlag wurde abgesogen, mit Diethylether gewaschen und unter Vakuum getrocknet.
Ausbeute: 1.1 g (95%)

### Amin F-42: 8-(Pyridin-4-yl)-2,8-diazaspiro[4.5]decan dihydrochlorid

Die Herstellung erfolgte in 2 Stufen analog der Synthese des Amin F-12 aus tert-Butyl 2,8-diazaspiro[4.5]decane-2-carboxylat und 4-Chlorpyridiniumchlorid bzw. wie folgt:

### Stufe (I): tert-Butyl 8-(pyridin-4-yl)-2,8-diazaspiro[4.5]decan-2-carboxylat

tert-Butyl-2,8-diazaspiro[4.5]decane-2-carboxylat (10.403 mmol, 1 Äquiv.) und N-Ethyl-diisopropylamin (41.608 mmol, 4 Äquiv.) wurden in 2-Propanol (20 ml) vorgelegt. 4-Chorpyridin (31.206 mmol, 3 Äquiv.) wurde dazugegeben und das Gemisch für 16 h auf 90°C erhitzt. Dann wurde ges. Natriumhyrogencarbonatlösung (50 ml) zugegeben und die Phasen getrennt. Die wässrige Phase wurde mit Ethylacetat extrahiert (4 x 50 ml), die vereingten org. Phasen mit ges. NaCl-Lösung (50 ml) gewaschen und über Magnesiumsulfat getrocknet. Nach säulenchromatischer Reinigung (Ethylacetat / DCM / Methanol / Ammoniak (25% aq), 100:100:25:1) erhielt man das gewünschte produkt als gelbes Öl.
Ausbeute: 1.8 g (55%)

### Stufe (II): 8-(Pyridin-4-yl)-2,8-diazaspiro[4.5]decan Dihydrochlorid

Es wurde tert-Butyl 8-(pyridin-4-yl)-2,8-diazaspiro[4.5]decan-2-carboxylat (5.671 mmol, 1 Äquiv.) in Ethanol p.a. (20 ml) vorgelegt. Dann wurde Acetylchlorid (28,355 mmol, 3 Äquiv.) bei 0°C zugegeben und das resultierende Gemisch 16 h bei 25°C gerührt. Das Lösemittel wurde unter Vakuum eingeengt und der Rückstand im Hochvakuum getrocknet um das gewünschte produkt zu erhalten.
Ausbeute: 1.48 g (90%)

### Amin F-43: 6-(Azetidin-1-ylmethyl)-2-(azetidin-3-yl)-1,2,3,4-tetrahydroisoquinolin trihydrochlorid

(i): 6-(Azetidin-1-ylmethyl)-1,2,3,4-tetrahydroisoquinolin dihydrochlorid (F-08) (0.7 g, 2.54 mmol), Triethylamin (0.7 ml, 5.09 mmol) und 1-Boc-3-azetidinon (433 mg, 2.54 mmol) wurden in 1,2-Dichlorethan (10 ml) vorgelegt und mit Natriumtriacetoxyborhydrid (747 mg, 3.56 mmol) versetzt. Das Reaktionsgemisch wurde 15 h gerührt und anschließend mit gesättigter Natriumhydrogencarbonatlösung versetzt. Nach Phasentrennung wurde die wässrige Phase mit Dichlormethan (2 x) extrahiert. Die vereinigten organischen Phasen wurden mit gesättigter Natriumchloridlösung (1 x) gewaschen, über Magnesiumsulfat getrocknet und unter Vakuum eingeengt. Das Rohprodukt wurde säulenchromatographisch (Kieselgel, Ethylacetat / Hexan / Methanol / Amoniak (25% aq), 600:100:100:5) gereinigt.
   Ausbeute: 0.74 g (81 %)
(ii): tert-Butyl 3-(6-(azetidin-1-ylmethyl)-3,4-dihydroisoquinolin-2(1H)-yl)azetidine-1-carboxylate (0.73 g, 2.04 mmol) wurde mit Chlorwasserstoff in Methanol (1.25 mol/l, 16 ml) versetzt und 30 min refluxiert. Das Lösungsmittel wurde unter Vakuum entfernt und der Rückstand in Ethanol / Aceton (20 ml) aufgenommen. Anschließend wurde Diethylether (20 ml) hinzugegeben und der entstandene Niederschlag abgesogen.
   Ausbeute: 0.76 g (>99%)

Alternativ kann die Herstellung wie folgt erfolgen:

### Stufe (i): 1-[6-(Azetidin-1-yl-methyl)-1,2,3,4-tetrahydro-isochinolin-2-yl]-2,2,2-trifluor-ethanon

2-(2,2,2-Trifluor-acetyl)-1,2,3,4-tetrahydro-isochinolin-6-carbaldehyd (7.776 mmol, 1 Äquiv.) und Azetidin (9.331 mmol, 1.2 Äquiv.) wurden in 1,2-Dichlorethan (37 ml) gelöst und das Gemisch 10 min bei 25°C rührt. Natriumtriacetoxyborhydrid (10.89 mmol, 1.4 Äquiv.) wurde zugegeben und das Reaktionsgemisch 16 h bei 25°C gerührt. Danach wurde ges. Natriumhydrogencarbonatlösung (50 ml) zugegeben, die Phasen getrennt und die wässrige Phase mit Dichlormethan (4 x 20 ml) extrahiert. Die vereinigten org. Phasen wurden mit ges. NaCl-Lösung (1 x 50 ml) gewaschen, über Natriumsulfat getrocknet und im Vakuum konzentriert. Das Rohprodukt wurde säulenchromatographisch (Ethylacetat / Dichlormethan / Hexan / Methanol /Ammoniak (25% aq), 30:10:10:2:0.02) gereinigt.
Ausbeute: 1.55 g (67%)

### Stufe (ii): 6-(Azetidin-1-yl-methyl)-1,2,3,4-tetrahydro-isochinolin Dihydrochlorid

1-[6-(Azetidin-1-yl-methyl)-1,2,3,4-tetrahydro-isochinolin-2-yl]-2,2,2-trifluor-ethanon (5.162 mmol, 1 Äquiv.) wurde in Methanol (20 ml) gelöst, K₂CO₃ (10.32 mmol, 2 Äquiv.) zugegeben und das Gemisch 1 h bei 25°C gerührt. Methanol wurde im Vakuum konzentriert, der Rückstand in Dichlormethan (30 ml) aufgenommen und mit dest. Wasser (5 ml) gewaschen. Die wässrige Phase wurde mit DCM (20 ml) extrahiert und die vereinigten org. Phasen über Natriumsulfat getrocknet und im Vakuum eingeengt. Zur Reinigung wurde das Rohprodukt in Diethylether (50 ml) gelöst und mit 2 M HCl in Diethylether-Lösung (3 Äquiv.) wurde ein Hydrochlorid gefällt. Dieses wurde abfiltriert, mit Diethylether gewaschen und im Vakuum getrocknet um das gewünschte produkt zu erhalten.
Ausbeute: 1.03 g (73%)

### Stufe (iii): 3-[6-(Azetidin-1-yl-methyl)-1,2,3,4-tetrahydro-isochinolin-2-yl]-azetidin-1-carbonsäure tert-butyl ester

6-(Azetidin-1-yl-methyl)-1,2,3,4-tetrahydro-isochinolin Dihydrochlorid (2.455 mmol, 1 Äquiv.) wurde in 1,2-Dichlorethan (10 ml) und Triethylamin (5.088 mmol, 2 Äquiv.) gelöst und 1-Boc-3-Azetidin (2.544 mmol, 1 Äquiv.) zugegeben. Nach 5 min wurde Natriumtriacetoxyborhydrid (2.652 mmol, 1.4 Äquiv.) zugegeben und das Reaktionsgemisch 16 h bei RT unter Stickstoff gerührt. Zum Reaktionsgemisch wurde ges. Natriumhydrogencarbonatlösung zugegeben und mit Dichlormethan (2 x 50 ml) extrahiert. Die vereinigten org. Phasen wurden mit ges. NaCl-Lösung gewaschen, über Magnesiumsulfat getrocknet und unter vermindertem Druck eingeengt. Das Rohprodukt wurde säulenchromatographisch (Ethylacetat / Hexan /Methanol / Ammoniak (25% aq), 60:10:10:0,1) gereinigt.
Ausbeute: 81 %

### Stufe (iv): 6-(Azetidin-1-ylmethyl)-2-(azetidin-3-yl)-1,2,3,4-tetrahydroisochinolin Trihydrochlorid

3-[6-(Azetidin-1-yl-methyl)-1,2,3,4-tetrahydro-isochinolin-2-yl]-azetidin-1-carbonsäure tert-butyl ester (2.042 mmol, 2 Äquiv.) wurde in Chlorwasserstoff in Methanol (1.25 M, 10 Äquiv., 16 ml) gelöst und 30 min bei Siedetemperatur gerührt. Nach dünnschichtchromatographischer Kontrolle wurde das Methanol entfernt und der Rückstand in Ethanol / Aceton (1:5, 20 ml) gelöst. Mit Diethylether (50 ml) wurde das Hydrochlorid ausgefällt, filtriert, mit Diethylether gewaschen und im Vakuum getrocknet. Um das gewünschte Produkt zu erhalten.
Ausbeute: 100%

### Amin F-44: 3-Pyridin-3-yl-3-(2-pyrrolidin-1-yl-ethoxy)-9-azaspiro[5.5]undecan

### Stufe-1: 3-Hydroxy-3-pyridin-3-yl-9-azaspiro[5.5]undecan-9-carbonsäure tert-butyl ester

3-Brompyridin (14.9 mmol, 1 Äquiv.) wurde in Ether (50 ml) gelöst und bei -78°C in eine Lösung aus n-BuLi (16.5 mmol, 1.17 M solution in Ether, 1.1 Äquiv.) getropft. Das Gemisch wurde 20 min gerührt. Anschließend wurde tert-Butyl 9-oxo-3-azaspiro[5.5]undecan-3-carboxylat (Stufe (iv) Amin F-40) (7.49 mmol) wurde in Ether (90 ml) gelöst und langsam zugegeben. Das Reaktionsgemisch wurde für eine weitere Stunde bei -78°C gerührt. Dest. Wasser wurde zugegeben und das Gemisch auf 25°C aufgetaut. Die Phasen wurden getrennt, die org. Phase mit ges. NaCl-Lösung gewaschen (30 ml), über Na₂SO₄ getrocknet und konzentriert. Das Rohprodukt wurde in Ethylacetat (10 ml) aufgenommen und mit Hexan (30 ml) wurde ein Feststoff ausgefällt. Dieser wurde abfiltriert und im Hochvakuum getrocknet um das gewünschte produkt zu erhalten.
Ausbeute: 50%

### Stufe-2: 3-Pyridin-3-yl-3-(2-pyrrolidin-1-yl-ethoxy)-9-azaspiro[5.5]undecan-9-carbonsäure tert-butyl ester

Eine Mischung aus 3-Hydroxy-3-pyridin-3-yl-9-azaspiro[5.5]undecan-9-carbonsäure tert-butyl ester (0.867 mmol), 1-(2-Chlor-ethyl)-pyrrolidin Hydrochlorid (1.3 mmol, 1.5 Äquiv.), trockenem KOH Pulver (4.33 mmol, 5 Äquiv.) und katalytischen Mengen von 18-Krone-6 in Toluol (20 ml) wurde für 16 h auf Siedetemperatur erhitzt. Das Reaktionsgemisch wurde mit Ethylacetat (25 ml) verdünnt und mit dest. Wasser (10 ml) und ges. Natriumchloridlösung (10 ml) gewaschen. Die org. Phase wurde über Natriumsulfat getrocknet und konzentriert. Anschließend wurde das Rohprodukt säulenchromatographisch (5% MeOH in DCM) gereinigt.
Ausbeute: 34%

### Stufe-3: 3-Pyridin-3-yl-3-(2-pyrrolidin-1-yl-ethoxy)-9-azaspiro[5,5]undecan

3-Pyridin-3-yl-3-(2-pyrrolidin-1-yl-ethoxy)-9-azaspiro[5.5]undecan-9-carbonsäure tert-butyl ester (0.282 mmol, 1 Äquiv.) wurde in DCM (3ml) gelöst. Bei 0°C wurde Trifluoressigsäure (1.5 ml) zugegeben und das Gemisch 2 h bei 25°C gerührt. Das Lösemittel wurde unter vermindertem Druck konzentriert, der Rückstand in DCM (2 x 10 ml) aufgenommen und getrocknet um das gewünschte produkt zu erhalten.

### Amin F-45: 4-(4-Methyl-piperidin-4-yl)-morpholin dihydrochlorid,

[MFCD09743690] kommerziell erhältlich bei SynChem Inc. **Amin F-46: 5-Piperidin-4-yl-3-pyridin-4-yl-[1,2,4]oxadiazol,** [276237-03-7] kommerziell erhältlich bei Fluorochem. **Amin F-47: 2-(Pyrrolidin-2-yl-methyl)-pyridin,** [MFCD04966889] kommerziell erhältlich bei ACB Blocks. **Amin F-48: Dimethyl-(3-piperazin-1-yl-propyl)-amin,** [877-96-3] kommerziell erhältlich bei ABCR. **Amin F-49: 1-(Pyridin-2-yl-methyl)-[1,4]diazepan,** [247118-06-5] kommerziell erhältlich bei Matrix. **Amin F-50: 2-Piperidin-4-yloxy-pyridin dihydrochlorid,** [28033-37-6] kommerziell erhältlich bei Interchim BB. **Amin F-51: 2-Piperidin-4-yloxy-pyrazin dihydrochlorid,** [MFCD03840122] kommerziell erhältlich bei Interchim BB.

### Amin F-52: (1S,5R)-3-Pyridin-4-yloxy-8-azabicyclo[3.2.1]octan hydrochlorid

### Stufe 1: (1R,3r,5S)-tert-Butyl 3-hydroxy-8-azabicyclo[3.2.1]octan-8-carboxylat

Nortropin (9,099 g, 71,541 mmol) wurde in Dichlormethan (140 ml) vorgelegt und die Lösung auf 0°C gekühlt. Bei dieser Temperatur wurden Triethylamin (19,82 ml, 143,08 mmol) und Di-tert-butyldicarbonat (18,73 g, 85,85 mmol) zugegeben. Das Reaktionsgemisch wurde langsam auf Raumtemperatur erwärmt und 15 Std gerührt. Nach Hydrolyse mit Wasser wurden die Phasen getrennt, die organische Phase wurde mit gesättigter Zitronensäure-Lösung, Wasser und gesättigter Natriumchlorid-Lösung gewaschen, über Natriumsulfat getrocknet und unter Vakuum eingeengt. Das Rohprodukt wurde ohne weitere Reinigung verwendet.
Ausbeute: 14,36 g, 88%

### Stufe 2: (1R,3r,5S)-tert-Butyl 3-(pyridin-4-yloxy)-8-azabicyclo[3.2.1]octan-8-carboxylat

(1 R,3r,5S)-tert-Butyl 3-hydroxy-8-azabicyclo[3.2.1]octan-8-carboxylat (0,61 g, 2,685 mmol), 4-Hydroxypyridin (255 mg, 2,685 mmol) und Triphenylphosphin (873 mg, 3,357 mmol) wurden in Tetrahydrofuran (11 ml) vorgelegt und mit Diisopropylazodicarboxylat (0,65 ml, 3,357 mmol) versetzt. Die Reaktionsmischung wurde auf 55°C erwärmt und bei dieser Temperatur 15 Std. gerührt. Tetrahydrofuran wurde unter Vakuum entfernt, der Rückstand wurde in Ethylacetat (50 ml) gelöst und mit Chlorwasserstoff-Lösung (1 mol/l, 2 x 30 ml) gewaschen. Die wässrige Phase wurde mit verdünnter Natronlauge alkalisiert (pH = 8) und mit Ethylacetat (3 x 70 ml) extrahiert. Diese organischen Phasen wurden vereinigt, über Natriumsulfat getrocknet und unter Vakuum eingeengt. Das Rohprodukt wurde säulenchromatographisch (Kieselgel) mit Ethylacetat / Hexan 3/1 gereinigt.
Ausbeute: 0,45 g, 55%

### Stufe 3: (1S,5R)-3-Pyridin-4-yloxy-8-azabicyclo[3.2.1]octan hydrochlorid

(1 R,3r,5S)-tert-Butyl 3-(pyridin-4-yloxy)-8-azabicyclo[3.2.1]octan-8-carboxylat (4,6 g, 15,123 mmol) wurde in Methanol (37 ml) gelöst, im Eisbad gekühlt und mit Acetylchlorid (5,36 ml) versetzt. Die Reaktionsmischung wurde 15 Std bei Raumtemperatur gerührt, dann unter Vakuum eingeengt. Der Rückstand wurde in Wasser und Dichlormethan aufgenommen, die Phasen getrennt, die wässrige Phase mit Dichlormethan (2 mal) gewaschen und an der Gefriertrocknung getrocknet.
Ausbeute: 3,47 g, 95%

### Amin F-53: (1S,5R)-3-Pyridin-3-yloxy-8-azabicyclo[3.2.1]octan hydrochlorid

Die Synthese wurde in Analogie zu **F-52** unter Verwendung von 3-Pyridinol durchgeführt.

### Amin F-54: (Methyl-[2-(4-pyridin-4-yloxy-piperidin-1-yl)-ethyl]-amin dihydrochlorid

**Stufe 1:** Zu einer Lösung von 4-Hydroxypyridin (1.89 g, 19.8 mmol) in 70 ml trockenem THF wurde bei Raumtemperatur N-Boc-4-Hydroxy-Piperidin (5.0 g, 24.8 mmol) gegeben. Es wurden Triphenylphosphin (6.49 g, 24.77 mmol) und Diisopropylazodicarboxylat (DIAD, 4.91 ml) zugegeben, das Reaktionsgemisch für 12 Stundn auf 55°C erhitzt und abschließend unter vermindertem Druck konzentriert. Der Rückstand wurde mit 30 ml 1M HCl versetzt und mit Dichlormethan gewaschen. Die vereinigten organischen Phasen wurden mit 1 M HCl und Wasser gewaschen. Die vereinigten wässrigen Phasen wurden mit 1 M NaOH-Lsg. auf pH 12 eingestellt und mit Dichlormethan extrahiert. Die vereinigten organischen Phasen wurden mit ges. NaCl-Lsg. gewaschen, über Natriumsulfat getrocknet und unter vermindertem Druck konzentriert. Der Rückstand wurde aus Pentan / Ether 3:1 umkristallisiert.
Ausbeute: 50%

**Stufe 2:** Zu einer Lösung des soeben in Stufe 1 erhaltenen Produkts (50 g) in 400 ml Dichlormethan wurden bei 0°C 100 ml TFA gegeben und das Reaktionsgemisch für 3 Stunden bei Raumtemperatur gerührt. Anschließend wurde das Lösungsmittel abdestilliert, der Rückstand in Ethanol aufgenommen und mit Amberlyst A21 Harz (25g) versetzt. Das Harz wurde abfiltriert und das Lösungsmittel unter vermindertem Druck entfernt um das gewünschte Amin zu erhalten.

**Stufe 3:** Zu einer Lösung des soeben erhaltenen Amins in Dichlormethan (5 ml/mmol) wurde Methyl-(2-oxo-ethyl)-carbaminsäure tert-butyl ester (2 Äquiv.), Essigsäure (3 Äquiv.) und Triacetoxyborhydrid (3 Äquiv.) gegeben, 12 Stunden bei Raumtemperatur gerührt und anschließend mit Dichlormethan verdünnt: Das Reaktionsgemisch wurde mit gesättigter Natriumhydrogencarbonat-Lsg. und ges. NaCl-Lsg. gewaschen, über Natriumsulfat getrocknet und unter vermindertem Druck konzentriert. Der Rückstand wurde säulenchromatographisch gereinigt (10% Methanol in Dichlormethan).

**Stufe 4:** Zu einer Lösung des soeben erhaltenen Produkts in Methanol wurden bei 0°C 10 Äqiv. Acetylchlorid gegeben. Der Reaktionsverlauf wurde mittels Dünnschichtchromatographie (10% MeOH/CHCl₃) kontrolliert. Nach vollständigem Umsatz wurde das Lösungsmittel unter vermindertem Druck entfernt und das gewünschte Produkt in Form eines Feststoffs erhalten.

**Amin F-55: 4-Pyridin-4-yl-piperidin-4-ol, [233261-75-1] kommerziell erhältlich bei** ABCR. **Amin F-56: 4-Pyridin-2-yl-piperidin-4-ol,** [50461-56-8] kommerziell erhältlich bei Apollo.

### Amin F-57: 1-(2-Methylamino-ethyl)-4-pyridin-3-yl-piperidin-4-ol hydrochlorid

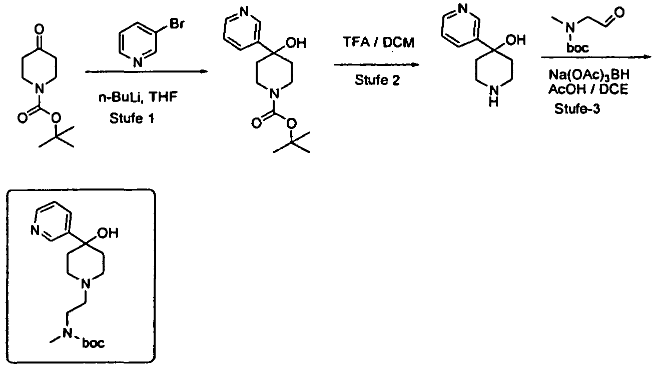

**Stufe 1:** Zu einer Lösung von **F-16** in Dichlormethan (5 ml/mmol) wurde Methyl-(2-oxo-ethyl)-carbaminsäure tert-butyl ester (2 Äquiv.), Essigsäure (3 Äquiv.) und Triacetoxyborhydrid (3 Äquiv.) gegeben, 12 Stunden bei Raumtemperatur gerührt und anschließend mit Dichlormethan verdünnt: Das Reaktionsgemisch wurde mit gesättigter Natriumhydrogencarbonat-Lsg. und ges. NaCl-Lsg. gewaschen, über Natriumsulfat getrocknet und unter vermindertem Druck konzentriert. Der Rückstand wurde säulenchromatographisch gereinigt (10% Methanol in Dichlormethan).

**Stufe 2:** Zu einer Lösung des soeben erhaltenen Produkts in Methanol wurden bei 0°C 10 Äqiv. Acetylchlorid gegeben. Der Reaktionsverlauf wurde mittels Dünnschichtchromatographie (10% MeOH/CHCl₃) kontrolliert. Nach vollständigem Umsatz wurde das Lösungsmittel unter vermindertem Druck entfernt und das gewünschte Produkt in Form eines Feststoffs erhalten.

### Amin F-58: Dimethyl-(4-phenyl-piperidin-4-yl)-amin dihydrochlorid

### Stufe 1: 1-Benzyl-N,N-dimethyl-4-phenylpiperidin-4-amin

Zu einer Mischung aus 34,5 g (3,5 Äquiv.) Magnesium und 100 ml trockenem Diethylether wurde zunächst etwas Jod und anschließend über einen Zeitraum von 10 min 10 g (0,15 Äquiv.) Brombenzol gegeben und für weitere 10 min gerührt. Nach dem Anspringen der Reaktion wurden 183 g (2,85 Äquiv.) Brombenzol gelöst in 500 ml Diethylether tropfenweise über einen Zeitraum von 2 h zugegeben und für 15 min nachgerührt. Das soeben hergestellte Grignardreagenz wurde über einen Zeitraum von 2 h mit 100 g (1 Äquiv.) 1-Benzyl-4-(dimethylamino)piperidin-4-carbonitril gelöst in 900 ml Diethylether versetzt und abschließend für 12 h auf 80°C erhitzt. Der Reaktionsverlauf wurde mittels Dünnschichtchromatographie (10% MeOH/CHCl₃) kontrolliert.

Nach vollständigem Umsatz wurde die Reaktionslösung auf 0°C abgekühlt, mit gesättigter NH₄Cl-Lösung versetzt, mit Ethylacetat extrahiert (3x300 ml) und die vereinigten organischen Phasen mit Na₂SO₄ getrocknet. Nach Entfernen des Lösungsmittels unter vermindertem Druck wurde säulenchromatographisch (Kieselgel; 1% MeOH/CHCl₃) gereinigt. 30 g (35%) Produkt wurden in Form eines gelben Feststoffs erhalten.

### Stufe 2: Benzyloxycarbonyl-4-(dimethylamino)-4-phenylpiperidin

Zu 50 g (1 Äquiv.) 1-Benzyl-*N,N*-dimethyl-4-phenylpiperidin-4-amin wurden über einen Zeitraum von 1 h tropfenweise 500 ml (10 Äquiv.) Cbz-Chlorid gegeben und das erhalte Reaktionsgemisch für 2 h bei Raumtemperatur gerührt. Der Reaktionsverlauf wurde mittels Dünnschichtchromatographie (10% MeOH/CHCl₃) kontrolliert. Nach vollständigem Umsatz wurde auf 0°C abgekühlt und mit ges. Natriumhydrogencarbonatlösung basich gestellt und das Reaktionsgemisch 3x mit 300 ml EtOAc extrahiert. Die vereinigten organischen Phasen wurden mit Na₂SO₄ getrocknet. Nach Entfernen des Lösungsmittels unter vermindertem Druck wurde säulenchromatographisch (Kieselgel; 50% EtOAc/Heptan) gereinigt. 12 g (21 %) Produkt wurden in Form eines Öls erhalten.

### Stufe 3: tert-Butyloxycarbonyl-4-(dimethylamino)-4-phenylpiperidin

Zu einer Lösung von 12 g (1 Äquiv.) Benzyloxycarbonyl-4-(dimethylamino)-4-phenylpiperidin in 120 ml Ethanol wurden 12,2 g KOH gegeben und die Reaktionsmischung für 48 h zum Rückfluß erhitzt. Der Reaktionsverlauf wurde mittels Dünnschichtchromatographie (20% MeOH/CHCl₃) kontrolliert.

Nach vollständigem Umsatz wurde das Lösungsmittel vollständig abdestilliert, der Rückstand in Ethylacetat suspendiert, filtriert und die organische Phase über Natriumsulfat getrocknet. Nach Entfernen des Lösungsmittels unter vermindertem Druck wurde das Rohprodukt mit in Dioxan gelöst, mit gesättigter Natriumhydrogencarbonatlösung und 11,9 g (1,5 Äquiv.) Boc-Anhydrid versetzt und für 30 min bei Raumtemperatur gerührt. Nach vollständigem Umsatz wurde die Reaktionsmischung mit 3x200 ml Ethylacetat extrahiert und die vereinigten organischen Phasen über Na₂SO₄ getrocknet. Nach Entfernen des Lösungsmittels unter vermindertem Druck wurden 8,5 g (77%) Rohprodukt in Form eines farblosen Feststoffs erhalten.

### Stufe 4: Dimethyl-(4-phenyl-piperidin-4-yl)-amin dihydrochlorid

Zu einer Lösung von *tert*-Butyloxycarbonyl-4-(dimethylamino)-4-phenylpiperidin in Methanol wurden bei 0°C 10 Äqiv. Acetylchlorid gegeben.

Der Reaktionsverlauf wurde mittels Dünnschichtchromatographie (10% MeOH/CHCl₃) kontrolliert. Nach vollständigem Umsatz wurde das Lösungsmittel unter vermindertem Druck entfernt und das Produkt in Form eines Feststoffs erhalten.

### Amin F-59: Dimethyl-[1-(2-methylamino-ethyl)-4-phenyl-piperidin-4-yl]-amin trihydrochlorid

Vgl. **AMN-09.**

### Amin F-60: Dimethyl-[1-(3-methylamino-propyl)-4-phenyl-piperidin-4-yl]-amin trihydrochlorid

### Stufe 1: tert-Butyl 3-(4-(dimethylamino)-4-phenylpiperidin-1-yl)propyl(methyl)carbamat

Zu einer Lösung von 11 g (1 Äquiv.) *N,N*-Dimethyl-4-phenylpiperidin-4-amin dihydrochlorid in 110 ml Methanol wurden bei 0°C 11,1 g (1,3 Äquiv.) tert-Butyl methyl(3-oxopropyl)carbamat gegeben und die Reaktionsmischung für 15 min bei 0°C gerührt. Anschließend wurden portionsweise 6,2 g (3 Äquiv.) Natriumcyanoborhydrid zugegeben und für 30 min bei Raumtemperatur gerührt. Das erhaltene Reaktionsgemisch wurde mittels Essigsäure auf pH 5-6 eingestellt und für 12 h bei Raumtemperatur gerührt. Der Reaktionsverlauf wurde mittels Dünnschichtchromatographie (20% MeOH/CHCl₃) kontrolliert. Da die Umsetzung noch nicht vollständig war wurden 2,4 g Natriumcyanoborhydrid zugegeben, das erhaltene Reaktionsgemisch mittels Essigsäure auf pH 5-6 eingestellt und für 60 min bei Raumtemperatur gerührt.

Nach nun vollständigem Umsatz wurde das Methanol abdestilliert, mit ges. NaHCO₃-Lsg. basisch gestellt, die erhaltene Mischung mit Chloroform (3x 100 ml) extrahiert und die vereinigten organischen Phasen wurden über Na₂SO₄ getrocknet. Nach Entfernen des Lösungsmittels unter vermindertem Druck wurde säulenchromatographisch (Kieselgel; 5% MeOH/CHCl₃) gereinigt. 9 g (60%) Produkt wurden erhalten.

### Stufe 2: Dimethyl-[1-(3-methylamino-propyl)-4-phenyl-piperidin-4-yl]-amin trihydrochlorid

Durch eine 0°C kalte Lösung von 9 g (1 Äquiv.) *tert*-Butyl 3-(4-(dimethylamino)-4-phenylpiperidin-1-yl)propyl(methyl)carbamat in 100 ml Chloroform wurde für 1 h HCl-Gas geleitet. Der Reaktionsverlauf wurde mittels Dünnschichtchromatographie (20% MeOH/CHCl3) kontrolliert.

Nach vollständigem Umsatz wurde das Lösungsmittel unter vermindertem Druck entfernt und 10 g (100%) Produkt wurden nach verreiben mit Diethylether in Form eines weißen Feststoffs erhalten.

### Amin F-61: (4-Butyl-piperidin-4-yl)-dimethyl-amin

### Stufe 1: 1-Benzyl-4-(dimethylamino)piperidin-4-carbonitril

Zu einer Lösung von 150 g (1 Äquiv.) 1-Benzylpiperidin-4-on in 300 ml Methanol wurde 208 g (3 Äquiv.) *N,N*-Dimethyl amin hydrochlorid, 154 g (3 Äquiv.) Kaliumcyanid in 154 ml Wasser und 1050 ml (7 Äquiv.) einer 40%igen wässrigen Dimethylamin Lösung gegeben und auf 0°C abgekühlt. Anschließend wurden bei 0°C 75 ml (0,5 Äquiv.) konzentrierte Salzsäure zugegeben und das Reaktionsgemisch 24 h bei Raumtemperatur gerührt. Der Reaktionsverlauf wurde mittels Dünnschichtchromatographie (20% EtOAc/Hexan) kontrolliert.

Nach vollständigem Umsatz wurde der gebildete Feststoff abfiltriert und mit Eiswasser (4 I) gewaschen. Der erhaltene Feststoff wurde anschließend in Ethylacetat gelöst, mit Na₂SO₄ getrocknet. Nach Entfernen des Lösungsmittels unter vermindertem Druck wurden 165 g (85%) Rohprodukt wurden in Form eines Feststoffs erhalten.

### Stufe 2: 1-Benzyl-4-butyl-N,N-dimethylpiperidin-4-amin

Zu einer Mischung aus 17,7 g (6 Äquiv.) Magnesium und 50 ml trockenem Ether wurde zunächst etwas Jod und anschließend über einen Zeitraum von 1 h 100 g (6 Äquiv.) Brombutan gelöst in 100 ml trockenem Ether gegeben. Diese Reaktionsmischung wurde für 1 h bei Raumtemperatur gerührt. Das soeben hergestellte Grignardreagenz wurde über einen Zeitraum von 20 min zu einer Lösung von 30 g (1 Äquiv.) 1-Benzyl-4-(dimethylamino)piperidin-4-carbonitril gelöst in 210 ml trockenem THF gegeben und das erhaltene Reaktionsgemisch anschließend für 12 h bei Raumtemperatur gerührt. Der Reaktionsverlauf wurde mittels Dünnschichtchromatographie (10% MeOH/CHCl₃) kontrolliert.

Nach vollständigem Umsatz wurde die Reaktionslösung auf 0°C abgekühlt, mit gesättigter NH₄Cl-Lösung versetzt, über Celite filtriert, mit Ethylacetat extrahiert (3x200 ml) und die vereinigten organischen Phasen über Na₂SO₄ getrocknet. Nach Entfernen des Lösungsmittels unter vermindertem Druck wurde säulenchromatographisch (Aluminiumoxid Neutral; Hexan) gereinigt. 18,2 g (53%) Produkt wurden in Form eines Öls erhalten.

### Stufe 3: 4-Butyl-N,N-dimethylpiperidin-4-amin bis hydrochlorid

Zu einer Lösung von 10 g (1 Äquiv.) 1-Benzyl-4-butyl-N,N-dimethylpiperidin-4-amin in 100 ml MeOH wurden 1,5 g 20%Pd(OH)₂/C und 6,95 g (3 Äquiv.) Ammoniumformat gegebn. Das erhaltene Reaktionsgemisch wurde für 30 min zum Rückfluß erhitzt. Der Reaktionsverlauf wurde mittels Dünnschichtchromatographie (20% MeOH/CHCl₃) kontrolliert.

Nach vollständigem Umsatz wurde die Reaktionslösung auf Raumtemperatur abgekühlt, über Celite filtriert und mit Methanol nachgewaschen. Das Methanol wird abdestilliert, der Rückstand in Ethylacetat/Hexan aufgenommen, das Lösungsmittel abdekantiert und Toluol zugegeben. Die so erhaltene organische Phase wurde unter vermindertem Druck konzentriert und der Rückstand in 150 ml Dichlormethan aufgenommen. Durch die Dichlormethan Lösung wurde für 20 min HCl-Gas geleitet, das Lösungsmittel abdestilliert und so 7 g (74%) Produkt als weißer Feststoff erhalten. Die freie Base wurde nach Lösen des Hydrochlorids in wässriger Natriumhydroxid-Lösung und Extraktion dieser mit Dichlormethan erhalten.

### Amin F-62: [4-Butyl-1-(2-methylamino-ethyl)-piperidin-4-yl]-dimethyl-amin trihydrochlorid

### Stufe 1: tert-Butyl 2-(4-butyl-4-(dimethylamino)piperidin-1-yl)ethyl(methyl)carbamat

Zu einer Lösung von 7 g (1 Äquiv.) 4-Butyl-*N,N*-dimethylpiperidin-4-amin bis hydrochlorid in 50 ml Methanol wurde bei Raumtemperatur eine Lösung von 4,73 g (1 Äquiv.) tert-Butyl methyl(2-oxoethyl)carbamat in 20 ml Methanol gegeben und das erhaltene Reaktionsgemisch für 50 min bei Raumtemperatur gerührt. Diese Reaktionsmischung wurde portionsweise mit 3,43 g (2 Äquiv.) Natriumcyanoborhydrid versetzt und anschließend für 12 h bei Raumtemperatur gerührt. Der Reaktionsverlauf wurde mittels Dünnschichtchromatographie (20% MeOH/CHCl₃) kontrolliert.

Nach vollständigem Umsatz wurde das Reaktionsgemisch auf 0°C abgekühlt und mittels Essigsäure auf einen pH ~ 5 eingestellt. Es wurden abermals 2 g tert-Butyl formylmethylmethylcarbamat und 1,7 g Natriumcyanoborhydrid zugegeben und die Reaktionsmischung für weitere 60 min bei Raumtemperatur gerührt. Abschließend wurde das Methanol abdestilliert, 100 ml ges. NaHCO₃-Lsg. zugegeben und die erhaltene Mischung mit Ethylacetat (2x 200 ml) extrahiert und die vereinigten organischen Phasen wurden über Na₂SO₄ getrocknet. Nach Entfernen des Lösungsmittels unter vermindertem Druck wurden 10,5 g Rohprodukt in Form eines blassgelben Öls erhalten.

### Stufe 2: 4-Butyl-N,N-dimethyl-1-(2-(methylamino)ethyl)piperidin-4-amin tris hydrochlorid

Durch eine 0°C kalte Lösung von 10,5 g (1 Äquiv.) *tert*-Butyl 2-(4-butyl-4-(dimethylamino)piperidin-1-yl)ethyl(methyl)carbamat in 1000 ml Chloroform wurde für ~ 1 h HCl-Gas geleitet. Anschließend wurde das Reaktionsgemisch für 12 Stunden bei Raumtemperatur gerührt. Der Reaktionsverlauf wurde mittels Dünnschichtchromatographie (20% MeOH/CHCl₃) kontrolliert.

Nach vollständigem Umsatz wurde das Lösungsmittel unter vermindertem Druck entfernt und der Rückstand mit Hexan (3x50 ml) und Ethylacetat (3x50 ml) gewaschen und getrocknet. 9 g (87%) Produkt wurden in Form eines weißen Feststoffs erhalten.

### Amin F-63: 4-Phenyl-4-pyrrolidin-1-yl-piperidin

### Stufe 1: 1-Benzyl-4-(pyrrolidin-1-yl)piperidin-4-carbonitril

Zu einer Lösung von 50 g (1 Äquiv.) 1-Benzylpiperidin-4-on in 250 ml Ethanol wurde 100 g (5 Äquiv.) Pyrrolidin gegeben und für 10 min bei Raumtemperatur gerührt. Das Reaktionsgemisch wurde anschließend, tropfenweise, über einen Zeitraum von 10 min mit 25 ml (0,5 Äquiv.) Salzsäure versetzt und für 30 min bei Raumtemperatur gerührt. Zu dieser Reaktionsmischung wurden 55 g (3 Äquiv.) Kaliumcyanid gelöst in 250 ml Wasser gegeben und für drei Tage bei Raumtemperatur gerührt. Der Reaktionsverlauf wurde mittels Dünnschichtchromatographie (50% EtOAc/Heptan) kontrolliert.

Nach vollständigem Umsatz wurde der gebildete Feststoff abfiltriert und mit Eiswasser (3x150 ml) gewaschen. Der erhaltene Feststoff wurde anschließend in Ethylacetat suspendiert, mit Na₂SO₄ getrocknet. Nach Entfernen des Lösungsmittels unter vermindertem Druck wurden 70 g Rohprodukt wurden in Form eines Feststoffs erhalten.

### Stufe 2: 1-Benzyl-4-phenyl-4-(pyrrolidin-1-yl)piperidin

Zu einer Mischung aus 31,2 g (5 Äquiv.) Magnesium und 100 ml trockenem THF wurde zunächst etwas Jod und anschließend über einen Zeitraum von 10 min 10 g (0,25 Äquiv.) Brombenzol gegeben und für weitere 10 min gerührt. Nach dem Anspringen der Reaktion wurden 194,2 g (4,75 Äquiv.) Brombenzol gelöst in 500 ml THF tropfenweise über einen Zeitraum von 2 h zugegeben und für 15 min nachgerührt. Das soeben hergestellte Grignardreagenz wurde über einen Zeitraum von 2 h mit 70 g (1Äquiv.) 1-Benzyl-4-(pyrrolidin-1-yl)piperidin-4-carbonitril gelöst in 450 ml THF versetzt und abschließend für 12 h auf 80°C erhitzt. Der Reaktionsverlauf wurde mittels Dünnschichtchromatographie (10% MeOH/CHCl₃) kontrolliert.

Nach vollständigem Umsatz wurde die Reaktionslösung auf 0°C abgekühlt, mit gesättigter NH₄Cl-Lösung versetzt, mit Ethylacetat extrahiert (3x200 ml) und die vereinigten organischen Phasen mit Na₂SO₄ getrocknet. Nach Entfernen des Lösungsmittels unter vermindertem Druck wurden 33 g (40%) Rohprodukt wurden in Form eines Öls erhalten.

### Stufe 3: Benzyloxycarbonyl-4-phenyl-4-(pyrrolidin-1-yl)piperidin

Zu einer Lösung von 33 g (1 Äquiv.) 1-Benzyl-4-phenyl-4-(pyrrolidin-1-yl)piperidin in 330 ml Chloroform wurden über einen Zeitraum von 10 min tropfenweise 60 g (3,5 Äquiv.) Cbz-Chlorid gegeben und das erhalte Reaktionsgemisch für 30 min bei Raumtemperatur gerührt. Der Reaktionsverlauf wurde mittels Dünnschichtchromatographie (Ethylacetat) kontrolliert.

Nach vollständigem Umsatz wurde das Lösungsmittel vollständig abdestilliert der Rückstand mit 10% HCl-Lösung auf ~pH 6 eingestellt und 3x mit 100 ml EtOAc gewaschen. Im Eisbad wurde die wässrige Lösung mit NaOH-Lösung auf ~pH 9 eingestellt und anschließend 3x mit 100 ml Chloroform extrahiert. Die vereinigten organischen Phasen wurden mit Na₂SO₄ getrocknet. Nach Entfernen des Lösungsmittels unter vermindertem Druck wurde säulenchromatographisch (Kieselgel; 20% EtOAc/Heptan) gereinigt. 11 g (29%) Produkt wurden in Form eines gelben Feststoffs erhalten.

### Stufe 4: 4-Phenyl-4-(pyrrolidin-1-yl)piperidin

Zu einer Lösung von 7,3 g (1 Äquiv.) Benzyloxycarbonyl-4-phenyl-4-(pyrrolidin-1-yl)piperidin in 100 ml Ethanol wurden 11 g KOH gegeben und die Reaktionsmischung für 24 h zum Rückfluß erhitzt. Der Reaktionsverlauf wurde mittels Dünnschichtchromatographie (20% MeOH/CHCl₃) kontrolliert.

Nach vollständigem Umsatz wurde das Lösungsmittel vollständig abdestilliert der Rückstand mit 100 ml Wasser versetzt und 3x mit 100 ml CHCl₃ extrahiert. Die vereinigten organischen Phasen wurden mit Na₂SO₄ getrocknet. Nach Entfernen des Lösungsmittels unter vermindertem Druck wurden 7 g Rohprodukt in Form eines Öls erhalten.

### Stufe 5: 4-Phenyl-4-(pyrrolidin-1-yl)piperidin bishydrochlorid

Durch eine Lösung von 9 g (1 Äquiv.) 4-Phenyl-4-(pyrrolidin-1-yl)piperidin in 180 ml Chloroform wurde für ~ 30 min HCl-Gas geleitet, bis die Reaktionsmischung einen pH von ~2 erreichte.

Der Reaktionsverlauf wurde mittels Dünnschichtchromatographie (10% MeOH/CHCl₃) kontrolliert. Nach vollständigem Umsatz wurde das Lösungsmittel unter vermindertem Druck entfernt, der Rückstand mit Ethylacetat gewaschen (3x100 ml) und getrocknet. 9 g (76%) Produkt wurden in Form eines Feststoffs erhalten.

### Amin F-64: Dimethyl-(4-thiophen-2-yl-piperidin-4-yl)-amin dihydrochlorid

### Stufe 1: tert-Butyloxycarbonyl-4-cyano-4-(dimethylamino)piperidin

Zu einer Lösung von 50 g (1 Äquiv.) *tert*-Butyloxycarbonyl-4-oxopiperidin in 100 ml Methanol wurden 500 ml (10 Äquiv.) Dimethylamin-Lösung und 109,9 g (5 Äquiv.) Dimethylaminhydrochlorid gegeben und auf 5°C abgekühlt. Das Reaktionsgemisch wurde anschließend, tropfenweise, über einen Zeitraum von 10 min mit 5 ml (0,1 Äquiv.) Salzsäure versetzt und für 60 min bei Raumtemperatur gerührt. Zu dieser Reaktionsmischung wurden portionsweise 48,9 g (3 Äquiv.) Kaliumcyanid für 24 h bei Raumtemperatur gerührt. Der Reaktionsverlauf wurde mittels Dünnschichtchromatographie (50% EtOAc/Hexan) kontrolliert.

Nach vollständigem Umsatz wurde die Reaktionsmischung mit 150 ml Wasser versetzt und 3x mit 100 ml Ethylacetat extrahiert. Die vereinigten organischen Phasen wurden mit Na₂SO₄ getrocknet. Nach Entfernen des Lösungsmittels unter vermindertem Druck wurde Rohprodukt erhalten welches aus Hexan umkristallisiert wurde. 57 g (90%) Produkt wurden in Form eines farblosen Feststoffs erhalten.

### Stufe 2: tert-Butyloxycarbonyl-4-(dimethylamino)-4-(thiophen-2-yl)piperidin

Zu einer Mischung aus 5,6 g (3 Äquiv.) Magnesium und 20 ml trockenem Diethylether wurde zunächst etwas Jod und anschließend über einen Zeitraum von 10 min 5 g 2-Bromthiophen gegeben und für weitere 10 min gerührt. Nach dem Anspringen der Reaktion wurden 33,5 g (2,6 Äquiv.) 2-Bromthiophen gelöst in 80 ml Diethylether tropfenweise zugegeben und über einen Zeitraum von 2 h bei Raumtemperatur gerührt. Das soeben hergestellte Grignardreagenz wurde tropfenweise zu einer Lösung von 20 g (1 Äquiv.) tert-Butyloxycarbonyl-4-cyano-4-(dimethylamino)piperidin gelöst in 200 ml THF gegeben und über Nacht bei Raumtemperatur gerührt. Der Reaktionsverlauf wurde mittels Dünnschichtchromatographie (50% EtOAc/Hexan) kontrolliert.

Nach vollständigem Umsatz wurde die Reaktionslösung auf 0°C abgekühlt, mit gesättigter NH₄Cl-Lösung versetzt, mit Ethylacetat extrahiert (3x100 ml) und die vereinigten organischen Phasen mit Na₂SO₄ getrocknet. Nach Entfernen des Lösungsmittels unter vermindertem Druck wurde säulenchromatographisch (Alox Neutral; 30% EtOAc/Hexan) gereinigt. 6,1 g (25%) Produkt wurden in Form eines weißen Feststoffs erhalten.

### Stufe 3: N,N-Dimethyl-4-(thiophen-2-yl)piperidin-4-amin

Durch eine 0°C kalte Lösung von 10 g (1 Äquiv.) tert-Butyloxycarbonyl-4-(dimethylamino)-4-(thiophen-2-yl)piperidin in Chloroform wurde für ~ 1 h HCl-Gas geleitet.

Der Reaktionsverlauf wurde mittels Dünnschichtchromatographie (75% EtOAc/Hexan) kontrolliert.

Nach vollständigem Umsatz wurde das Reaktionsgemisch mit 200 ml Wasser versetzt, mit Na₂CO₃ auf pH ~8 eingestellt und abschließend mit 15% IPA/CHCl₃ extrahiert. Die vereinigten organischen Phasen wurden über Na₂SO₄ getrocknet. Nach Entfernen des Lösungsmittels unter vermindertem Druck wurden 6 g (89%) Produkt in Form eines weißen Feststoffs erhalten.

### Amin F-65: Methyl-[2-(4-phenyl-4-pyrrolidin-1-yl-piperidin-1-yl)-ethyl]-amin trihydrochlorid

### Stufe 1: tert-Butyl methyl(2-(4-phenyl-4-(pyrrolidin-1-yl)piperidin-1-yl)ethyl)carbamat

Zu einer Lösung von 4,4 g (1,1 Äquiv.) *tert*-Butyl-methyl(2-oxoethyl)carbamat in 70 ml Methanol wurden unter Stickstoffatmosphäre 7 g (1 Äquiv.) 4-Phenyl-4-(pyrrolidin-1-yl)piperidin bishydrochlorid gegeben und die Reaktionsmischung für 10 min bei 0°C gerührt. Anschließend wurden 3,62 g (2,5 Äquiv.) Natriumcyanoborhydrid zugegeben und für 30 min bei Raumtemperatur gerührt. Das erhaltene Reaktionsgemisch wurde mittels Essigsäure auf einen pH 5-6 eingestellt und für 14 h bei Raumtemperatur gerührt. Der Reaktionsverlauf wurde mittels Dünnschichtchromatographie (10% MeOH/CHCl₃) kontrolliert.

Nach vollständigem Umsatz wurde das Methanol abdestilliert, mit ges. NaHCO₃-Lsg. versetzt und die erhaltene Mischung mit Chloroform (3x 50 ml) extrahiert und die vereinigten organischen Phasen wurden über Na₂SO₄ getrocknet. Nach Entfernen des Lösungsmittels unter vermindertem Druck wurde säulenchromatographisch (Kieselgel; 50% EtOAc/Heptan) gereinigt. 8 g (89%) Produkt wurden in Form eines roten Öls erhalten.

### Stufe 2: tert-Butyl methyl(2-(4-phenyl-4-(pyrrolidin-1-yl)piperidin-1-yl)ethyl)carbamat tris hydrochlorid

Durch eine 0°C kalte Lösung von 8 g (1 Äquiv.) *tert.* Butyl methyl(2-(4-phenyl-4-(pyrrolidin-1-yl)piperidin-1-yl)ethyl)carbamat in 160 ml Chloroform wurde für ~ 30 min HCl-Gas geleitet, bis die Reaktionsmischung einen pH von ~2 erreichte. Anschließend wurde das Reaktionsgemisch bei Raumtemperatur für 4 Stunden gerührt. Der Reaktionsverlauf wurde mittels Dünnschichtchromatographie (10% MeOH/CHCl₃) kontrolliert.

Nach vollständigem Umsatz wurde das Lösungsmittel unter vermindertem Druck entfernt und 8 g (97%) Produkt wurden in Form eines weißen Feststoffs erhalten.

### Amin F-66: 1-[4-(3-Fluorphenyl)-piperidin-4-yl]-4-methyl-piperazin dihydrochlorid

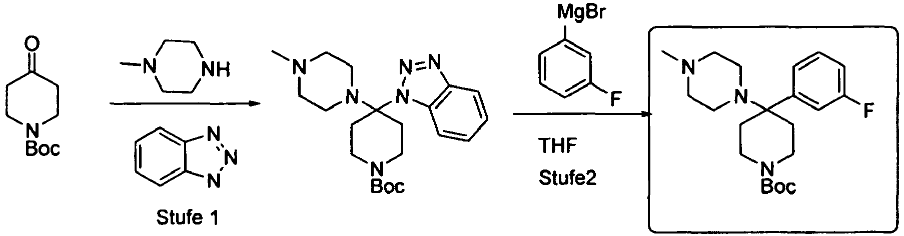

**Stufe 1:** Zu einer THF-Lösung des entsprechenden Grignard Reagenzes (60 mmol) wurde bei 0°C das soeben in Stufe 1 erhaltene Benzotriazol Addukt, gelöst in trockenem THF gegeben und das erhaltene Reaktionsgemisch für 16 Stunden bei 25°C gerührt. Anschließend wurde auf 0°C abgekühlt, mit gesättigter Ammoniumchlorid-Lsg. gequencht und mit Ethylacetat extrahiert. Die vereinigten organischen Phasen wurden mit Wasser und ges. Natriumchlorid-Lsg. gewaschen, über Natriumsulfat getrocknet und unter vermindertem Druck konzentriert. Der Rückstand wurde säulenchromatographisch aufgereinigt (2% Methanol in Dichlormethan).

**Stufe 2:** Zu einer Lösung des soeben erhaltenen Produkts in Methanol wurden bei 0°C 10 Äqiv. Acetylchlorid gegeben. Der Reaktionsverlauf wurde mittels

Dünnschichtchromatographie (10% MeOH/CHCl₃) kontrolliert. Nach vollständigem Umsatz wurde das Lösungsmittel unter vermindertem Druck entfernt und das gewünschte Produkt in Form eines Feststoffs erhalten.

### Amin F-67: 2-(Piperidin-1-yl-methyl)-5,6,7,8-tetrahydro-imidazo[1,2-a]pyrazin dihydrochlorid

Stufe 1: Zu einer Lösung des Ethyl 5,6,7,8-tetrahydroimidazo[1,2-a]pyrazin-2-carboxylat Hydrochlorids (1.09 g, 4.70 mmol) in DCM (100 ml) wurde erst DMAP (0.75 g, 6.12 mmol) und dann Boc₂O (1.34 g, 6.12 mmol) gegeben. Der Reaktionsansatz wurde 18 h bei RT gerührt. Da die Reaktion noch nicht vollständig war, wurde weiteres Boc₂O (0.12 g, 0.53 mmol) zugegeben und erneut über Nacht gerührt. Nach abgeschlossener Reaktion wurde mit wässriger HCl-Lösung (1 M, 100 ml) gewaschen, die organische Phase über Natriumsulfat getrocknet und im Vakuum eingeengt. Das Rohprodukt wurde säulenchromatographisch (Kieselgel, Ethylacetat) aufgereinigt. Ausbeute: 300 mg, 21 %

Stufe 2: Eine Lösung 7-tert-Butyl 2-ethyl 5,6-dihydroimidazo[1,2-a]pyrazin-2,7(8H)-dicarboxylat (300 mg, 1.02 mmol) in THF (15 ml) wurde auf -78°C abgekühlt und unter N₂-Atmosphäre langsam mit DIBAL-H (1 M in Hexan, 2.0 ml, 2.0 mmol) versetzt. Der Reaktionsansatz wurde 1 h bei dieser Temperatur gerührt und anschließend mit Na₂SO₄ x 10 H₂O versetzt bis keine Gasentwicklung mehr beobachtet werden konnte. Es wurde weiteres Natriumsulfat zugegeben, filtriert und der Rückstand mit DCM (25 ml) gewaschen. Das Filtrat wurde aufkonzentriert und das erhaltene Rohprodukt (450 mg) ohne weitere Aufreinigung in die nächste Stufe eingesetzt.

Stufe 3: Das tert-Butyl 2-formyl-5,6-dihydroimidazo[1,2-a]pyrazin-7(8H)-carboxylat (400 mg, max. 0.91 mmol) und Piperidin (158 µl, 1.59 mmol) wurde in DCM (8 ml) gelöst und mit NaBH(OAc)₃ (506 mg, 2.39 mmol) portionsweise versetzt. Der Reaktionsansatz wurde 2 h bei RT gerührt und anschließend mit gesättigter Natriumhydrogencarbonat-Lösung (25 ml) hydrolysiert. Die Phasen wurden getrennt und die wäßrige Phase erneut mit DCM (25 ml) extrahiert. Die vereinigten organischen Phasen wurden mit gesättigter Natriumchlorid-Lösung gewaschen und über Natriumsulfat getrocknet und im Vakuum eingeengt. Ausbeute: 260 mg, 90 % über 2 Stufen.

Stufe 4: Zu einer Lösung des tert-Butyl 2-(piperidin-1-ylmethyl)-5,6-dihydroimidazo[1,2-a]pyrazin-7(8H)-carboxylats (235 mg, 0.73 mmol) in DCM (10 ml) wurde TFA (2.83 ml, 36.7 mmol) gegeben und bei RT 3 - 4h gerührt (DC-Kontrolle). Nach abgeschlossener Reaktion wurde zunächst das Lösungsmittel entfernt, mit DCM versetzt und erneut zur Trockne eingeengt. Das Produkt wurde ohne weitere Aufreinigung für weitere Reaktionen verwendet.

### Amin F-68: 4-(3-Fluorphenyl)-4-(2-pyrrolidin-1-yl-ethoxy)-piperidin dihydrochlorid

**Stufe-1:** Zu einer Lösung von 3-Fluorophenyl magnesium bromide (15.075 mmol, 0.5 M) in THF wurde bei 0 °C eine Lösung von N-boc piperidone (10.05 mmol) in THF (10 mmol) gegeben. Nach vollständiger Zugabe wurde die Reaktion für 2 h bei derselben Temperatur (DC-Kontrolle) gerührt. Dann wurde mit ges. wässriger NH₄Cl-Lösung gequencht, das Reaktionsgemisch wurde mit Ethylacetat verdünnt und die organische Phase wurde nacheinander mit Wasser und ges. NaCl-Lösung gewaschen. Die organische Phase wurde über Na₂SO₄ getrocknet und zuletzt im Vakuum eingeengt, um das Rohprodukt zu erhalten, welches durch Säulenchromatographie (50 % Ethylacetat in Hexan) aufgereinigt wurde.
Ausbeute : 40%

**Stufe-2:** Zu einer Benzollösung (200 mL) des Pyridinderivats das in Stufe-1 erhalten wurde (9.84g, 35.3 mmol) wurde trocknes, pulverisiertes KOH (9.9 g) 18-crown-6 (1.06g) und 2-chloroethyl pyrrolidine hydrochloride (1.5 eqv.) gegeben und das resultierende Gemisch wurde für 16 h refluxiert. Dann wurde auf 25°C abgekühlt, mit Ethylacetat verdünnt und die organische Phase wurde nacheinander mit Wasser und ges. NaCl-Lösung gewaschen und zuletzt über Na₂SO₄ getrocknet. Das Einengen der organischen Phase im Vakuum ergab das Rohprodukt, welches durch Säulenchromatographie (5 % Methanol in Dichlormethan) aufgereinigt wurde.
Ausbeute: 50%

**Stufe 3:** Das soeben erhaltene Produkt wurde in Methanol gelöst, bei 0°C mit Acetylchlorid (3 Äq) versetzt und 16 Stunden bei 25°C gerührt. Danach das Lösemittel unter vermindertem Druck entfernt und der Rückstand im Hochvakuum getrocknet.

### Amin F-69: 3-[4-(3-Pyrrolidin-1-yl-propyl)-piperidin-4-yl]-pyridin dihydrochlorid

Stufe-1: Zu einer Lösung von 3-pyridyl acetate (2g) in THF (36 mL) wurde bei 25 °C bis-(2-chloro-ethyl)-carbamic acid tert-butyl ester (1.5 eqv), NaH (3 eqv), 18-crown-6 (0.2 eqv) und DMF (2 ml) gegeben und das resultierende Reaktionsgemisch wurde bei dieser Temperatur für 4 h (DC-Kontrolle) gerührt. Die Reaktion wurde auf 0°C abgekühlt, mit zerstoßenem Eis gequencht und mit Ethylacetat verdünnt. Die organische Phase wurde nacheinander mit Wasser und ges. NaCL-Lösung gequencht und zuletzt über Na₂SO₄ getrocknet. Das Einengen der organischen Phase im Vakuum ergab das Rohprodukt, welches durch Säulenchromatographie aufgereinigt wurde.
Ausbeute: 25%

**Stufe-2:** Zu einer kalten (0 °C) Suspension von LAH (1.2 eqv) in THF (3ml/mmol) wurde unter Argon-Atmosphäre eine Lösung des Esters (1 eqv) aus Stufe-1 in THF (2 mL/mmol) getropft. Nach vollständiger Zugabe wurde das Reaktionsgemisch für 2 h, nach dieser Zeit war das Edukt vollständig umgesetzt (DC-Kontrolle), bei dieser Temperatur gerührt. Die Reaktion wurde vorsichtig mit ges. wässriger Na2SO4-Lösung gequencht und über Celite filtriert. Der Rückstand wurde mit Ethylacetat gewaschen, die vereinigten organischen Phasen wurden über Na₂SO₄ getrocknet und im Vakuum eingeengt, um den Rohalkohol zu erhalten, welcher ohne weitere Aufreinigung direkt in die nächste Stufe eingesetzt wurde.
Ausbeute: 90 %

**Stufe-3:** Zu einer Lösung von Oxalyl chloride (1.1 eqv) in Dichlormethan (3 mL/mmol) wurde bei - 78°C unter Argon-Atmosphäre DMSO (2 eqv) gegeben und das resultierende Reaktionsgemisch wurde bei dieser Temperatur für 15 min gerührt. Zu diesem kalten Reaktionsgemisch wurde der Alkohol der in Stufe-2 erhalten wurde gelöst in Dichlormethan (3 mL/mmol) tropfenweise zugegeben und es wurde für eine weitere Stunde bei dieser Temperatur gerührt. Dann wurde Triethyl amine (5 eqv) zu der Reaktion gegeben und das Gemisch wurde langsam auf Raumtemperatur gebracht und für 1 h bei dieser Temperatur gerührt. Das Reaktionsgemisch wurde mit Dichlormethan verdünnt, die organische Phase wurde nacheinander mit ges. wässriger NH₄Cl-Lösung, Wasser und ges. NaCl-Lösung gewaschen und zuletzt über Na₂SO₄ getrocknet- Das Einengen der organischen Phase im Vakuum ergab das Rohprodukt, welches ohne weitere Aufreinigung direkt in die nächste Stufe eingesetzt wurde.
Ausbeute: 90% (roh)

**Stufe-4:** Zu einer kalten Suspension (0°C) von 60 % NaH (1.1 eqv) in abs. THF (5 ml/mmol) wurde langsam eine Lösung von triethyl phosphonoacetate (1.1eqv) in THF (5ml/mmol) gegeben und das resultierende Reaktionsgemisch wurde bei 25°C für 30 min gerührt. Dann wurde auf 0°C abgekühlt und der Aldehyd aus Stufe-3 gelöst in abs. THF (5 mL/mmo)) wurde bei gleichbleibender Temperatur zugetropft und das Reaktionsgemisch wurde dann für 16 h bei 25°C gerührt (nach dieser Zeit waren die Edukte vollständig umgesetzt). Es wurde mit Eis und ges. NaCl-Lösung gequencht, die wässrige Phase wurde mit Ethylacetat extrahiert und die organische Phase wurde nacheinander mit Wasser und ges. NaCl-Lösung gewaschen. Die organische Phase wurde über Na₂SO₄ getrocknet und im Vakuum eingeengt, um das Rohprodukt zu erhalten, welches durch Säulenchromatographie (50 % Ethylacetat in Hexan) aufgereinigt wurde.
Ausbeute: 50 %

**Stufe-5:** Eine Lösung des Esters (1 eqv) der aus Stufe-4 erhalten wurde in Methanol (5 mL/mmol) wurde für 15 min mit Argon entgast und dann mit 10% Pd/C (50% by weight) versetzt und das resultierende Reaktionsgemisch wurde unter Atmosphärendruck für 1 h hydriert ( Überwachung durch LCMS). Es wurde über - Celite abfiltriert, der Rückstand wurde mit Methanol gewaschen und die vereinigten organischen Phasen wurden vollständig eingeengt, um das Rohprodukt zu erhalten, welches ohne weitere Aufreinigung direkt in die nächste Stufe eingesetzt wurde.
Ausbeute: 95 % (crude)

**Stufe-6**: Zu einer kalten (0 °C) Suspension von LAH (1.2 eqv) in THF (3ml/mmol) wurde unter Argon Atmosphäre tropfenweise der Ester, der in Stufe-5 gewonnen wurde, gelöst in THF (2 mL/mmol) zugegeben. Nach vollständiger Zugabe wurde das Reaktionsgemisch bei dieser Temperatur für 2 h (nach dieser Zeit waren die Edukte vollständig umgesetzt→ DC-Kontrolle) gerührt. Die Reaktion wurde vorsichtig mit ges. wässriger Na₂SO₄-Lösung gequencht und über Celite filtriert. Der Rückstand wurde mit Ethylacetat gewaschen, die vereinigten organischen Phasen wurden über Na₂SO₄ getrocknet und im Vakuum eingeengt, um den Rohalkohol zu erhalten, welcher ohne weitere Aufreinigung direkt in die nächste Stufe eingesetzt wurde.
Ausbeute: 90 %

**Stufe-7**: Zu einer Lösung des Alkohols aus Stufe-6 (5.3 mmol) in Dichlormethan (22 mL) wurden bei 0 °C TEA (21.2 mmol) und Methane sulfonyl chloride (7.95 mmol) zugegeben und das erhaltene Reaktionsgemisch wurde bei dieser Temperatur für 2 h gerührt (DC-Kontrolle). Die Reaktion wurde mit Dichlormethan verdünnt, nacheinander mit Wasser und ges. NaCl gewaschen und zuletzt über Na₂SO₄ getrocknet. Das Einengen der organischen Phase im Vakuum ergab das Rohprodukt, welches direkt in die nächste Stufe eingesetzt wurde.
Ausbeute: quantitativ

**Stufe-8**: Zu einer Lösung des Mesylderivats aus Stufe-7 in Toluol (30 mL) wurden Kaliumcarbonat (26.5 mmol) und Pyrrolidine (6.36 mmol) gegeben und das resultierende Reaktionsgemisch wurde für 16 h refluxiert. Dann wurde dieses auf 25 °C abgekühlt, mit Ethylacetat verdünnt und die organische Phase wurde nacheinander mit Wasser und ges. NaCl-Lösung gewaschen. Nach dem Trocknen über Na₂SO₄, wurde die organische Phase im Vakuum eingeengt, um das Rohprodukt zu erhalten, welches durch Säulenchromatographie (5 % Methanol in Dichlormethan) aufgereinigt wurde.
Ausbeute: 50%

### Einzelsubstanzsynthesen

### 6a) Synthese der Pyrimidinderivate G & H:

### Allgemeine Methode zur Synthese der Pyrimidinderivate G & H

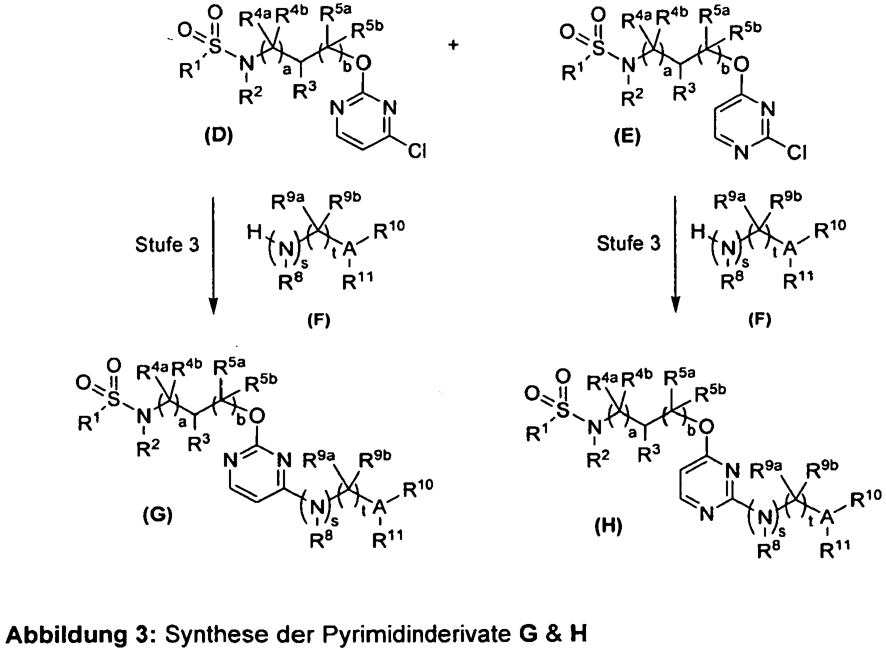

Allgemeine Arbeitsvorschrift AAV VI: Eine Lösung des Pyrimidinbausteins (**D** bzw. **E**) (1 Äquiv.) und Diisopropylethylamin (1.5 Äquiv.) in Isopropanol wurde mit dem entsprechenden Amin (**F**) (1.5 Äquiv.) versetzt und für 2-5 h zum Rückfuß erhitzt. Zur Aufarbeitung wurde das Reaktionsgemisch mit ges. Natriumhydrogencarbonat-Lsg. versetzt, 3x mit Ethylacetat extrahiert, die vereinigten organischen Phasen über Magnesiumsulfat getrocknet und unter vermindertem Druck konzentriert. Nach säulenchromatographischer Aufreinigung (Alox Neutral; Ethylacetat / Hexan) wurde das gewünschte Endprodukt (G bzw. H) erhalten. Bei Verwendung von Amin Hydrochloridn (**F** + **n HCl**) wurde, wurde die Menge des Diisopropylamins auf 1.5 Äquiv. + n Äquiv. erhöht.

Allgemeine Arbeitsvorschrift AAV VII: Der Pyrimidinbaustein (**D** bzw. **E**) (1 Äquiv.) wurde in 2-Propanol gelöst, N-Ethyl-diisopropylamin (1.5-4 Äquiv.) und das Amin (**F**) (1.5 Äquiv.), ggf. in Form des entsprechenden Hydrochlorids (xHCl), wurden zugegeben und das Gemisch wurde 15 h refluxiert. Anschließend wurde die Reaktionsmischung auf Raumtemperatur gekühlt, gesättigte Natriumhydrogencarbonat-Lösung und Ethylacetat zugegeben und die Phasen wurden getrennt. Die wässrige Phase wurde mit Ethylacetat extrahiert, die vereinigten organischen Phasen über Magnesiumsulfat getrocknet und unter Vakuum eingeengt. Das Rohprodukt wurde säulenchromatographisch (Kieselgel) gereinigt und die erfindungsgemäßen Verbindungen (**G** bzw. H) erhalten.

Allgemeine Arbeitsvorschrift AAV VIII: Der Pyrimidinbaustein ((**D** bzw. **E**) (1 Äquiv.) wurde in 1,4-Dioxan gelöst, Cs₂CO₃ (2 Äquiv.) und das Amin (**F**) (1.5 Äquiv.), ggf. in Form des entsprechenden Hydrochlorids (xHCl), wurden zugegeben und das Gemisch wurde 16 h refluxiert. Anschließend wurde die Reaktionsmischung auf Raumtemperatur gekühlt, gesättigte Natriumhydrogencarbonat-Lösung und Ethylacetat zugegeben und die Phasen wurden getrennt. Die wässrige Phase wurde mit Ethylacetat extrahiert. Die vereinigten organischen Phasen wurden mit Wasser und gesättigter NaCl-Lösung gewaschen, über Natriumsulfat getrocknet und unter Vakuum eingeengt. Das Rohprodukt wurde säulenchromatographisch (Kieselgel) gereinigt und die erfindungsgemäßen Verbindungen (G bzw. H) erhalten.

In den nachfolgend gezeigten Tabellen 4 und 5 sind nach diesen allgemeinen Arbeitsvorschriften hergestellte, spezielle Ausführungsformen der erfindungsgemäßen Verbindungen aufgelistet.

In Tabelle 6 sind entsprechende Daten für die Verbindungen vom Typ **(I)** angegeben.

**Tabelle 4: Synthese der Pyrimidinderivate G**

| **Beispiel Nr.** | **Struktur** | **Name** | **Pyrimidin (D bzw. E)** | **Amin (F)** | **Ausbeute** | **Analytik (LC/MS)** | **Synthetisiert nach** |
|---|---|---|---|---|---|---|---|
| G-001 | 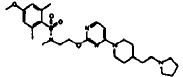 | 4-Methoxy-N,2.6-trimethyl-N-(2-(4-(4-(2-(pyrrolidin-1-yl)ethyl)piperidin-1-yl)pyrimidin-2-yloxy)ethyl)phenylsulfonamid (G-001) | N-(2-(4-Chloropyrimidin-2-yloxy)ethyl)-4-methoxy-N,2,6-trimethylphenylsulfonamid (D-01) | 4-(2-(Pyrrolidin-1-yl)ethyl)piperidin (F-03) | 61%, (0.17 mmol) | Rₜ = 2.0 min; Reinheit (UV 200-400 nm) 93%; *m*/*z* = 532.2 [MH]^{*} | AAV VI |
| G-002 | 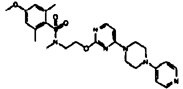 | 4-Methoxy-N,2,6-trimethyl-N-(2-(4-(4-(pyridin-4-yppiperaz(n-1-yppyrimidirr 2-yloxy)ethyl)phenylsulfonamid (G-002) | N-(2-(4-Chloropyrimidin-2-yloxy)ethyl)-4-methoxy-N,2,6-trimethylphenylsulfonamid (D-01) | 1-(Pyridin-4-yl)piperazin (F-15) | 81%, (0.314 mmol) | R, = 2.1 min; Reinheit (UV 200-400 nm) 99%; *m*/*z* = 513.2 [MH]⁺ | AAV VI |
| G-003 | 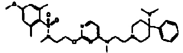 | N-(2-(4-((2-(4-(Dimethylamino)-4-phenylpiperidin-1-yl)(methyl)amino)pydmidin-2-yloxy)ethyl)-4-methoxy-N,2,6-trimethylphenylsulfonamid (G-003) | N-(2-(4-Chloropyrimidin-2-yloxy)ethyl)-4-methoxy-N,2,6-trimethylphenylsulfonamid (D-01) | N,N-Dimethyl-1-(2-(methylamino)ethyl)-4-phenylpiperidin-4-amin (F-09) | 88%, (0.342 mmol) | Rₜ = 1.9 min; Reinheit (UV 200-400 nm) *97%; m*/*z =* 611.2 [MH]⁺ | AAV VI |
| G-004 | 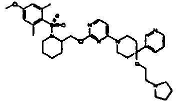 | 2-((1-(4-Methoxy-2,6-dimethylphenylsulfonyl)piperidin-2-yl)methoxyl-4-(4-(pyridin-3-yl)-4-(2-(pyrrolidin-1-yl)elhoxy)pipefidin-i, yl)pyrimidin (G-004) | 4-Chloro-2-((1-(4-methoxy-2,6-dimethylphenylsulfonyl)piperi din-2-yl)methoxy)pyrimidin (D-02) | 3-(4-(2-(Pyrrolidin-1-yl)ethoxy)piperidin-4-yl)pyridin dihydrochlorid (F-37) | 23%, (0.11 mmol) | Rₜ = 2.1 min; Reinheit (UV 200-400 nm) 91%; *m*/*z* = 665.2 [MH]⁺ | AAV VI ^{(a)} |
| G-005 | 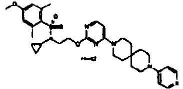 | N-Cyclopropyl-4-methoxy-2,6-dimethyl-N-(2-(4-(9-(pyridin-4-yl)-3,9-diazaspiro[5.5]undecan-3-yl)pyrimidin-2-yloxy)ethyl)phenylsldfonamid hydrochlorid (G-005) | N-(2-(4-Chloropy_{d}midiri-2-yloxy)ethyl)-N-cydopropyl-4-methoxy-2,6-dimethylphenylsulfonamid (D-10) | 3-(Pyridin-4-yl)-3,9-diazaspiro[5.5]undec an dihydrochlorid (F-12) | 51% (0.08 g) | Rₜ = 2.5 min; mh = 607.1 [MH]⁺ | AAV VII ^{(b)} |
| G-006 | 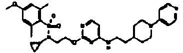 | N-Cyclopropyl-4-methoxy-2.6-dimethyl-N-(2-(4-(2-(1-(pyridin-4-yl)piperidin-4-yl)ethylamino)pyrimidin-2-yloxy)ethyl)phenylsulfonamid (G-006) | N-(2-(4-Chloropyrimidin-2-yloxy)ethyl)-N-cyclo propyl-4-methoxy-2,6-dimethylphenylsulfonamid (D-10) | 2-(1-(Pyridin-4-yl)piperidin-4-yl)ethanamin dihydrochlorid (F-11) | 24% (0.04 g) | R, = 2.6 min; *m*/*z* = 581.1 [MH]⁺ | AAV VII |
| G-007 | 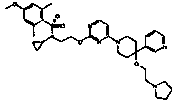 | N-Cyclopropyl-4-Methoxy-2,6-dimethyl-N-(2-(4-(4-(pyridin-3-yl)-4-(2-(pyrrolidin-1-yl)ethoxy)piperidin-1-yl)pyrimidin-2-yloxy)ethyl)phenylsulfonamid (G-007) | N-(2-(4-Chloropyrimidin-2-yloxy)ethyl)-N-cydopropyl-4-methoxy-2,6-dimethylphenylsulfonamid (D-10) | 3-(4-(2-(Pyrrolidin-1-yl)ethoxy)piperidin-4-yl)pyridin dihydrochlorid (F-37) | 80% (0.24 g) | Rₜ = 2.2 min; *m*/*z* = 651.1 [MH]⁺ | AAV VII |
| G-008 | 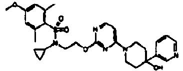 | N-Cyclopropyl-N-(2-(4-(4-hydroxy-4-(pyridin-3-yl)piperidin-1 -yl)pyrimidin-2-yloxy)ethyl)-4-methoxy-2,6-dimethylphenylsulfonamid (G-008) | N-(2-(4-Chloropyrimidin-2-yloxy)ethyl)-N-cydopropyl-4-methoxy-2,6-dimethylphenylsulfonamid (D-10) | 4-(Pyridin-3-yl)piperidin-4-ol (F-16) | 74% (0.18 g) | R, = 2.3 min; *mlz* = 554.0 [MH]⁺ | AAV VII |
| G-009 | 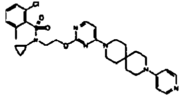 | 2-Chloro-N-cyclopropyl-6-methyl-N-(2-(4-(9-(pyridin-4-yl)-3,9-diazaspiro[5.5]undecan-3-yl)pyrimidin-2-yloxy)ethyl)phenylsulfonamid (G-009) | 2-Chloro-N-(2-(4-chloropyrimidin-2-yloxy)ethyl)-N-cyclopropyl-6-methylphenylsulfonamid (D-12) | 3-(Pyridin-4-yl)-3,9-diazaspiro[5.5]undec an dihydrochlorid (F-12) | 71% (0.19 g) | **Rₜ** = 2.6 min; *mlz* = 597.2 [MH]⁺ | AAV VII ^{(c)} |
| G-010 | 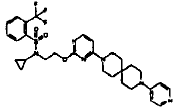 | N-Cyclopropyl-N-(2-(4-(9-(pyridin-4-yl)-3,9-diazaspiro[5.5]undecan,3, yl)pyrimidin-2-yloxy)ethyl)-2-(trifluoromethyl)phenylsulfonamid (G-010) | N-(2-(4-Chloropyrimidin-2-yloxy)ethyl)-N-cy_{d}opropyl-2-(trifluoromethyl)phenylsufforia mid (D-11) | 3-(Pyridin-4-yl)-3,9-diazaspiro[5.5]undec an dihydrochlorid (F-12) | 99% (0.16 g) | Rₜ = 2.5 min; *m*/*z* = 617.4 [MH]⁺ | AAV VII |
| G-011 | 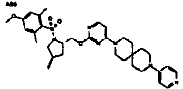 | 3-(2-(((2S,4R)-4-Fluoro-1-(4-methoxy-2,6-dimethylphenylsulfonyl)pyrrolidin-2-yl)methoxy)pyrimidin-4-yl)-9-(pyridin-4-yl)-3,9-diazaspiro[5.5]undecan (G-011) | 4-Chloro-2-(((2S,4R)-4-fluoro-1-(4-methoxy-2,8-dimethylphenylsulfonyl)pyrroli din-2-yl)methoxy)pyrimidin (D-13) | 3-(Pyridin-4-yl)-3,9-diazaspiro[5.5]undec an dihydrochlorid (F-12) | 79% (0.16 g) | Rₜ = 2.5 min; *m*/*z* = 625.4 [MH]⁺ | AAV VII |
| G-012 | 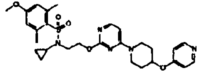 | N-Cyclopropyl-4-methoxy-2,6-dimethyl-N-(2-(4-(pyridin-4-yloxy)piperidin-1-yl)pyrlmldin-2-yloxy)ethyl)phenylsulfonamid (G-012) | N-(2-(4-Chloropyrimidin-2-yloxy)ethyl)-N-cyclopropyl-4-methoxy-2,6-dimethylphenylsulfonamid (D-10) | 4-(Piperidin-4-yloxy)pyridin dihydrochlorid (F-31) | 64% (0.18 g) | Rₜ = 2.2 min; *m*/*z* = 554.0 [MH]⁺ | AAV VII |
| G-013 | 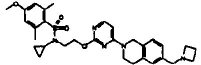 | N-(2-(4-(6-(Azetidin-1-ylmethyl)-3,4-dihydroisoquinolin-2(1H)-yl)pyrimidin-2-yloxy)ethyl)-N-cyclopropyl-4-methoxy-2.6-dimethylphenylsulfonamid (G-013) | N-(2-(4-Chloropyrimidin-2-yloxy)ethyl)-N-cyGopropyl-4-methoxy-2,6 dimethylphenylsulfonamid (D-10) | 6-(Azetidin-1-ylmethyl)-1,2,3,4-tetrahydroisoquinolin dihydrochlod (F-08) | 51% (0.05 g) | Rₜ = 2.5 min; *m*/*z* = 578.4 [MH]⁺ | AAV VII |
| G.014 | 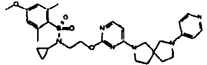 | N-cyclopropyl-4-methoxy-2,6-dimethyl-N-(2-(4-(7-(pyridin-4-yl)-2,7-diazaspiro[4.4]nonan-2-yl)pyrimidin-2-yloxy)ethyl)benzenesulfonamid (G-014) | N-(2-(4-Chloropyrimidin-2-yloxy)ethyl)-N-cydopropyl-4-methoxy-2,6-dimethylbenzenesutfonamid (D-10) | 2-(Pyridin-4-yl)-2,7-diazaspiro[4.4]nonan dihydrochlorld (F-38) | 91% (0.09 g) | Rₜ = 2.5 min; *m*/*z* = 579.4 [MH]⁺ | AAV VII |
| G-015 | 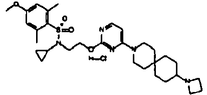 | N-(2-(4-(9-(azetidin-1-yl)-3-azaspiro[5.5]undecan-3-yl)pyrimidin-2-yloxy)ethyl)-N-cyclopropyl-methoxy-2,6-dimethylbenzenesulfonamid hydrochloride (G-015) | N-(2-(4-Chloropyrimidin-2-yloxy)ethyl)-N-cydapropyl-4-methoxy-2,6-dimethylbenzenesulfonamid (D-10) | 9-(Azetidin-1-yl)-3-azaspiro[5.5]undeca n dihydrochlorid (F-39) | 88% (0.1 g) | Rₜ 2.5 min; *mlz* = 584.4 [MH]⁺ | AAV VII^{(b)} |
| G-016 | 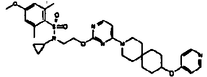 | N-cyclopropyl-4-methoxy-2,6-dimethyl-N-(2-(4-(9-pyridin-4-yloxy)-3-azaspiro[5.5]undecan-3-yl)pyrimidin-2-yloxy)ethyl)benzenesulfonamid (G-016) | N-(2-(4-Chloropyrimidin-2-yloxy)ethyl)-N-cyGopropyl-4-methoxy-2,6-dimethylbenzenesulfonamid (D-10) | 9-(Pyridin-4-yloxy)-3-azaspiro[5.5]undeca n dihydrochlorid (F-40) | > 99% (0.12 g) | Rₜ = 2.8 min; *m*/*z* = 622.4 [MH]⁺ | AAV VII |
| G-017 | 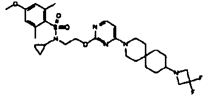 | N-cyclopropyl-N-(2-(4-(9-(3,3-difluoroazetidin-1-yl)-3-azaspiro[5.5]undecan-3-yl)pyrimidin-2-yloxy)ethyl)-4-methoxy-2,6-dimethylbenzenesulfonamid (G-017) | N-(2-(4-Chloropyrimidin-2-yloxy)ethyl)-N-cydopropyt-4-methoxy-2,6-dimethylbenzenesulfonamid (D-10) | 9-(3,3-Difluoroazetidin-1-yl)-3-azaspiro[5.5]undeca n dihydrochlorid (F-41) | 62% (0.07 g) | Rₜ = 2.7 min; *m*/*z* = 620.5 [MH]⁺ | AAV VII |
| G-018 | 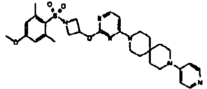 | 3-(2-(1-(4-methoxy-2,6-dimethylphenylsulfonyl)azetidin-3-yloxy)pyrimidin-4-yl)-9-(pyridin-4-yl)-3,9-diazaspiro[5.5]undecan (G-018) | 4-Chloro-2-(1-(4-methoxy-2,6-dimethylphenylsulfonyl)azetid in-3-yloxy)pyrimidin (D-06) / 2-Chloro-4-(1-(4-niethoxy-2,6-dimethylphenylsulfonyl)azetid in-3-yloxy)pyrimidin (E-06) | 3-(Pyridin-4-yl)-3.9-diazaspiro(5.51undec an dihydrochloride (F-12) | 15% (0.11 g) | Rₜ = 2.7 min; *m*/*z* = 579.4 [MH]⁺ | AAV VII^{(d)} |
| G-019 | 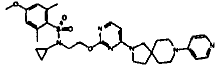 | N-cyclopropyl-4-methoxy-2,6-dimethyl-N-(2-(4-(8-(pyridin-4-yl)-2,8-diazaspiro[4.5]decan-2-yl)pyrimidin-2-yloxy)ethyl)benzenesuifonamid (G-019) | N-(2-(4-Chloropyrimidin-2-yloxy)ethyl)-N-cyclopropyl-4-methoxy-2,6-dimethylbenzenesulfonamid (D-10) | 8-(Pyridin-4-yl)-2,8-diazaspiro[4.5]decan dihydrochlorid (F-42) | 79% (0.08 g) | Rₜ = 2.6 min; *m*/*z* = 593.4 [MH]⁺ | AAV VII |
| G-020 | 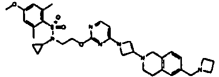 | N-(2-(4-(3-(6-(azetidin-1-ylmethyl)-3,4-dihydroisoquinolin-2(I H)-yl)azetidin-1-yl)pyrimidin-2-yloxy)ethyl)-N-cyclopropyl-4-methoxy-2,6-dimethylbenzenesulfonamid (G-020) | N-(2-(4-Chloropyrimidin-2-yloxy)ethyl)-N-cyclopropyl-4-methoxy-2,6-dimethylbenzenesulfonamid (D-10) | 6-(Azetidin-1-ylmethyl)-2-(azetidin-3-yl)-1,2,3,4-tetrahydroisoquinolin trihydrochlorid (F-43) | 74% (0.08 g) | Rₜ = 2.3 min; m/z = 633.5 [MH]⁺ | AAV VII |
| G-021 | 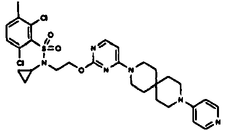 | 2,6-Dichlor-N-cyclopropyl-3-methyl-N-[2-[4-(9-pyridin-4-yl-3,9-diazaspiro[5.5]undecan-3-yl)-pyrimidin-2-yl]oxy-ethyl]-benzolsulfonamid (G-021) | 2,6-Dichlor-N-[2-(4-chlor-pyrimidin-2-yl)o^{q}ithyl]-N-cyclopropyl-3-methyl-benzolsulfonamid (D-14) | 3-(Pyridin-4-yl)-3,9-diazaspiro(5.S)undec an Dihydrochlorid (F-12) | 61% (0.15 g) | Rₜ = 3.7 min; *m*/*z* =631.2 [MHI' | AAV VII |
| G-022 | 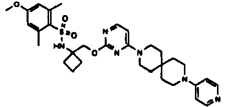 | 4-Methoxy-2,6-dimethyl-N-[1-[[4-(9-pyridin4-yl-3,9-diazaspiro[5.5]undecan-3-yl)-pyrimidin-2-yl]oxy-methyl]-cyclobutyl]-benzolsulfonamid (G-022) | N-[1-[(4-Chlor-pyrimidin-2-yl)oxy-methyl]-cyclobutyl]-4-methoxy-2,6-dimethyl-benzolsulfonamid (D-15) | 3-(Pyridin-4-yl)-3,9-diazaspiro[5.5]undec an Dihydrochlorid (F-12) | 23% | Rₜ = 2.5 min; m/z = 607.2 [MH]⁺ | AAV VIII |
| G-023 | 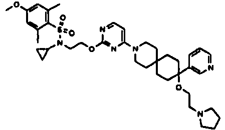 | N-Cyclopropyl-4-methoxy-2,6-dimethyl-N-[2-[4-[9-pyridin-3-yl-9-(2-pyrrolidin-1-yl-ethoxy)-3-azaspiro[5.5]undecan-3-yl]-pyrimidin-2-yl]oxy-ethyl]-benzolsultonamid (G-023) | N-(2-(4-Chloropyrimidin-2-yloxy)ethyl)-N-cycopropyl-4-methoxy-2,6-dimethylphenylsulfonamid (D-10) | 3-Pyridin-3-yl-3-(2-pyrrolidin-1-yl-ethoxy)-9-azaspiro[5.5]undeca n (F-44) | 49% | Rₜ = 3.0 min; m/z = 719.5 [MH]⁺ | AAV VIII |
| G-024 | 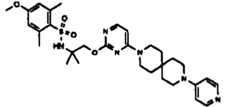 | N-(1,1-Dimethyl-2-[4-(9-pyridin-4-yl-3,9-diazaspiro[5.5]undecan-3-yl)-pyrimidin-2-yl]oxy-ethyl)-4-methoxy-2,6-dimethyl-benzolsulfonamid (G-024) | N-[2-(4-Chlor-pyrimidin-2-yl)oxy-1,1-dimethyl-ethyl]-4-methoxy-2,6-dimethyl-benzolsulfonamid (D-16) | 3-(Pyridin-4-yi)-3,9-diazaspiro[5.5]undec an Dihydrochlorid (F-12) | 64% | Rₜ = 2.7 min; *m*/*z* = 595.3 [MH]⁺ | AAV VIII |
| G-025 | 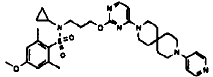 | N-Cyclopropyl-4-methoxy-2,6-dimethyl-N-[3-[4-(9-pyridin-4-yl-3,9-diazaspiro[5.5]undecan-3-yl)-pyrimidin-2-yl]oxy-propyl]-benzolsulfonamid (G-025) | N-[3-(4-Chlor-pyrimidin-2-yl)oxy-propyl]-N-cyclopropyl-4-methoxy-2,6-dimethyl-benzolsulfonamid (D-17) | 3-(Pyridin-4-yl)-3,9-diazaspiro[5.5]undec an Dihydrochlorid (F-12) | 62% | Rₜ = 3.3 min; *m*/*z* = 621.4 [MH]⁺ | AAV VIII |
| G-026 | 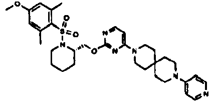 | 3-[2-[[(2S)-1-[(4-Methoxy-2.6-dimethyl-phenyl)sulfonyl]-piperidin-2-y]-methoxy]-pyrimidin-4-yl]-9-pyridin-4-yl-3,9-diazaspiro[5.5]undecan (G-026) | 4-Chlor-2-[[(2S)-1-[(4-methoxy-2,6-dimethyl-phenyl)sulfonyl]-piperidin-2-yl]-methoxy]-pyrimidin (D-18) | 3-(Pyridin-4-yl)-3,9-diazaspiro[5.5]undec an Dihydrochlorid (F-12) | 50% | Rₜ - 3.0 min; *m*/*z* = 621.4 [MH]⁺ | AAV VIII |

| | | | | | | | |
|---|---|---|---|---|---|---|---|
| Kommentar zu Tabelle 4: (a) Das Reaktionsgemisch wurde vor Aufarbeitung für 12 h zum Rückfluss erhitzt. (b) Die Endverbindung (G) wurde unter Einwirkung von Chlorwasserstoff, bevorzugterweise 2M HCl in Diethylether (bis zu 4 Äquiv.), in einem organischem Lösungsmittel bzw. Löungsmittelgemisch, bevorzugterweise Aceton / Diethylether, in das entsprechende Hydrochlorid überführt. (c) Die Regiochemie wurde via NMR bestimmt: 1 H NMR (600 MHz, DMSO-d₆) d ppm 0.28 - 0.36 (m, 2 H) 0.53 - 0.60 (m, 2 H) 1.47 - 1.54 (m, 4 H) 1.53 -1.60 (m, 4 H) 2.57 - 2.63 (m, 1 H) 2.65 (s, 3 H) 3.33 - 3.41 (m, 4 H) 3.55 - 3.67 (m, 4 H) 3.71 - 3.77 (m, 3 H) 4.44 (t, J=6.04 Hz, 2 H) 6.47 (d, J=6.04 Hz, 1 H) 6.77 - 6.84 (m, 2 H) 7.36 - 7.42 (m, 1 H) 7.46 - 7.55 (m, 2 H) 7.96 (d, J=6.04 Hz, 1 H)8.12(d,J=6.80Hz,2H) (d) Als Edukt eingesetzt wurde ein Gemisch aus D-06/E-06; die Trennung der Isomere erfolgte säulenchromatographisch und via päparativer HPLC:[15% G-018], [8% H-007] [18% G-018, verunreinigt mit H-007]. | | | | | | | |

**Tabelle 5: Synthese der Pyrimidinderivate H**

| **Beispiel Nr.** | **Struktur** | **Name** | **Pyrimidin (D bzw. E)** | **Amin (F)** | **Ausbeute** | **Analytik (LC/MS)^{[Fetterit Textmarke nichte definierte]}** | **Sythetisiert nach** |
|---|---|---|---|---|---|---|---|
| H-001 | 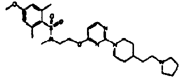 | 4-Methoxy-N,2,6-trimethyl-N-(2-(2-(4-(2-(pyrrolidin-1-yl)ethyl)piperidin-1-yl)pyrimidin-4-yloxy)ethyl)phenylsulfonamid (H-001) | N-(2-(2-Chloropyrimidin-4-yloxy)ethyl)-4-methoxy-N,2,6-trimethylphenylsulfonamid (E-01) | 4-(2-(Pyrrolidin-1-yl)ethyl)piperidin (F-03) | 61% (0.22 mmol) | Rₜ = 2.2 min; Reinheit (UV 200-400 nm) 93%; *m*/*z* = 532.3 [MH]⁺ | AAV VI |
| H-002 | 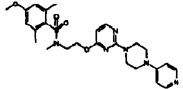 | 4-Methoxy-N,2,6-trlmethyl-N-(2.(2-(4-(pyridin-4-yl)piperazin-1-yl)pyrimidin-4-yloxy)ethyl)phenylsulfonamid (H-002) | N-(2-(2-Chloropyrimidin-4-yloxy)ethyl)-4-methoxy-N,2,6-trimethylphenylsulfonamid (E-01) | 1-(Pyridin-4-yl)piperazin (F-15) | 58.2% (0.23 mmol) | Rₜ = 2.8 min; Reinheit (UV 200-400 nm) 99%; *m*/*z* 513.2 [MH]⁺ | AAV VI |
| H-003 | 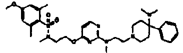 | N-(2-(2-((2-(4-(Dimethylamino)-4-phenylpiperidin-1-yl)ethyl)(methyl)amino)pyrlmldin-4-yloxy)ethyl)-4-methoxy-N,2,6-trimethylphenylsulfonamid (H-003) | N-(2-(2-Chloropyrimidin-4-yloxy)ethyl)-4-methoxy-N,2,6-trimethylphenylsulfonamid (E-01) | N,N-Dimethyl-1-(2-(methylamino)ethyl)-4-phenylpiperidin-4-amin (F-09) | 73% (0.28 mmol) | Rₜ = 2.5 min; Reinheit (UV 200-400 nm) 91%; *m*/*z* 611.1 [MH]⁺ | AAV VI |
| H-004 | 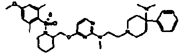 | N-(2-(4-(Dimethylamino)-4-phenylpiperidin-1-yl)ethyl)-4-((1-(4-methoxy-2,6-dimethylphenylsulfonyl)piperidin-2-yl)methoxy)-N-methylpyrimidin-2-amin (H-004) | 2-Chloro-4-((1-(4-methoxy-2,6-dimethylphenylsulfonyl)piper idin-2-yl)methoxy)pyrimidin (E-02) | N,N-Dimethyl-1-(2-(methylamino)ethyl)-4-phenylpiperidin-4-amin (F-09) | 71.6% (0.25 mmol) | Rₜ = 2.7 min; Reinheit (UV 200-400 nm) 99%; *m*/*z* = 651.3 [MH]⁺ | AAV VI |
| H-005 | 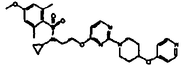 | N-Cycopropyl-4-methoxy-2,6-dimethyl-N-(2-(2-(4-(pyridim4-yloxy)piperidin-1-yl)pyrimidin-4-yloxy)ethyl)phenylsulfonamid (H-005) | N-(2-(2-Chloropyrimidin-4-yloxy)ethyl)-N-cyclopropyl-4-methoxy-2,6 dimethylphenylsulfonamid (E-10) | 4-(Piperidin-4-yloxy)pyridin dihydrochlorid (F-31) | 89% (0.18 g) | Rₜ = 2.8 min; *m*/*z* = 554.1 [MH]⁺ | AAV VII |
| H-006 | 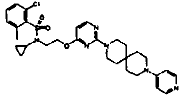 | 2-Chloro-N-cyclopropyl-6-melhyl-N-(2-(2-(9-(pyridin-4-yl)-3,9-diazaspiro[5.5]undecan-3-yl)pyrimidin-4-yloxy)ethyl)phenylsulfonamid (H-006) | N-(2-(2-Chloropyrimidin-4-yloxy)ethyl)-N-cyclopropyl-4-methoxy-2,6-dimethylphenosulfonamid (E-10) | 3-(Pyridin-4-yl)-3,9-diazaspiro[5.Siundec an dihydrochlorid (F-12) | 76% (0,17 g) | Rₜ =3.0min: *m*/*z* = 597.2 [MH]⁺ | AAV VII^{(a)} |
| H-007 | 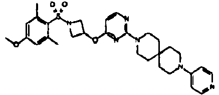 | 3-(4-(1-(4-Methoxy-2,6-dimethylphenylsulfonyl)azetidin-3-yloxy)pyrimidin-2-yl)-9-(pyridin-4-yl)-3,9-diazaspiro[5.5]undecan (H-007) | 4-Chloro-2-(1-(4-methoxy-2,6-dimethylphenylsulfonyl)azeti din-3-yloxy)pyrimidin (D-06) / 2-Chloro-4-(1-(4-methoxy-2,6-dimethylphenylsulfonyl)azeti din-3-yloxy)pyrimidin (E-06) | 3-(Pyridin-4-yl)-3,9-diazaspiro[5.5]undec an dihydrochloride (F-12) | 8% (0.06 g) | Rₜ = 3.3 min; *m*/*z* = 579.4 [MH]⁺ | AAV VII^{(b)} |

| | | | | | | | |
|---|---|---|---|---|---|---|---|
| (a) Die Regiochemie wurde via NMR bestimmt: 1 H NMR (600 MHz, DMSO-d₆) d ppm 0.24 - 0.33 (m, 2 H) 0.51 - 0.65 (m, 2 H) 1.43 - 1.53 (m, 4 H) 1.53 - 1.61 (m, 4 H) 2.64 (s, 1 H) 2.65 (s, 3 H) 3.32 - 3.38 (m, 4 H) 3.73 - 3.77 (m, 6 H) 4.51 (t, J=5.67 Hz, 2 H) 5.97 (d, J=6.04 Hz, 1 H) 6.79 (d, J=6.80 Hz, 2 H) 7.37 - 7.41 (m, 1 H) 7.45 - 7.54 (m, 2 H) 8.07 (d, J=5.29 Hz, 1 H) 8.12 (d, J=6.04 Hz, 2 H) (b) Als Edukt eingesetzt wurde ein Gemisch aus D-06/E-06; die Trennung der Isomere erfolgte säulenchromatographisch und via päparativer HPLC:[15% G-018], [8% H-007] [18% G-018, verunreinigt mit H-007]. | | | | | | | |

### 6b) Synthese der Pyrimidinderivate I:

### Beispiel I-001: N-Cyclopropyl-4-methoxy-2,6-dimethyl-N-(2-(6-(9-(pyridin-4-yl)-3,9-diazaspiro[5.5]undecan-3-yl)pyrimidin-4-yloxy)ethyl)benzenesulfonamid

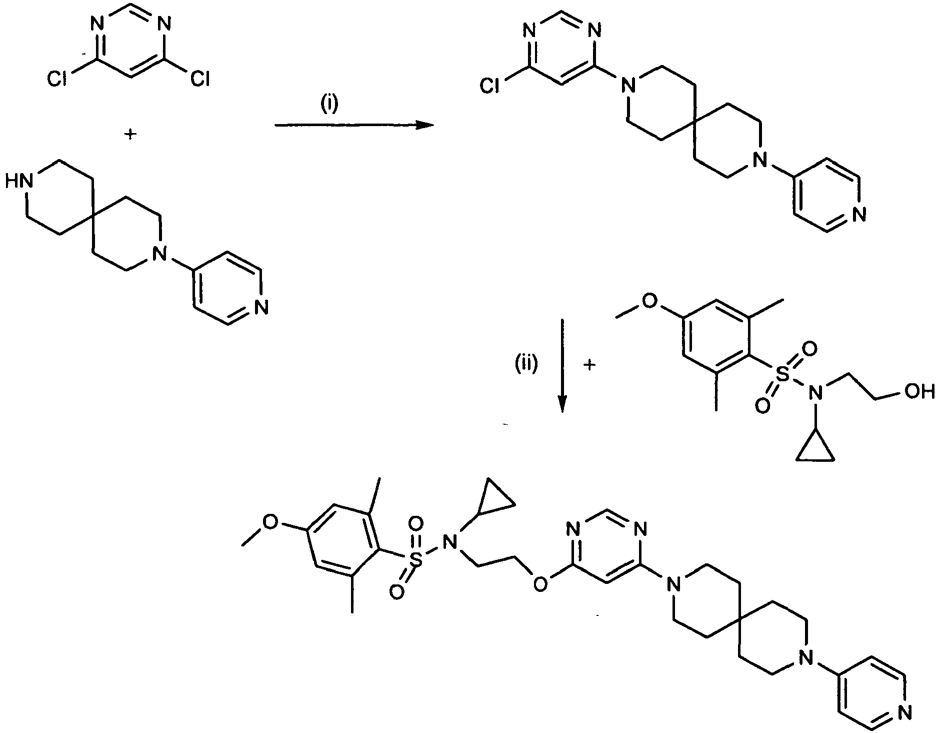

(i) Zu einer Lösung 4,6-Dichloropyrimidin (1.35 mmol, 1 Äquiv.) in Aceton wurde Kaliumcarbonat ( 4 Äquiv.) gegeben, gefolgt von 3-(Pyridin-4-yl)-3,9-diazaspiro[5.5]undecan (F-12, freie Base) (1 Äquiv.). Die resultierende Lösung wurde über Nacht refluxiert und anschließend das Lösungsmittel unter Vakuum entfernt.
   Der Rückstand wurde in Dichlormethan aufgenommen und mit Wasser und ges. Natriumchloridlösung gewaschen. Die organische Phase wurde über Magnesiumsulfat getrocknet und unter Vakuum eingeengt und das Rohprodukt säulenchromatographisch (Alox-neutral) aufgereinigt.
   Ausbeute: 49 %
(ii) Zu einer Suspension Natriumhydrid (7.28 mmol, 10 Äquiv.) in Tetrahydrofuran (10 ml) wurde bei 0 °C N-Cyclopropyl-N-(2-hydroxyethyl)-4-methoxy-2,6-dimethylphenylsulfonamid (C-10) (0.8mmol, 1.1 Äquiv.) gegeben, gefolgt von 3-(6-Chlorpyrimidin-4-yl)-9-(pyridin-4-yl)-3,9-diazaspiro[5.5]undecan (0.73 mmol, 1Äquiv.). Das Reaktionsgemisch wurde über Nacht bei Raumtemperatur gerührt, anschließend mit Wasser versetzt und mit Ethylacetat verdünnt. Die organische Phase wurde mit Wasser und ges. Natriumchloridlösung extrahiert, getrocknet (Na₂SO₄) und im Vakuum eingeengt. Das Rohprodukt wurde säulenchromatographisch aufgereinigt (Alox-neutral).
   Ausbeute: 7 %
   Rₜ = 3.4 min; *m*/*z* = 607.5 [MH]⁺

### Beispiel I-002: N-Cyclopropyl-4-methoxy-2,6-dimethyl-N-[2-[methyl-[6-(9-pyridin-4-yl-3,9-diazaspiro[5.5]undecan-3-yl)-pyrazin-2-yl]-amino]-ethyl]-benzolsulfonsäure amid (I-002)

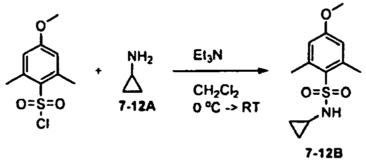

Eine Lösung von Dimethylmethoxysulfonylchlorid (32,2 g; 137 mmol) in Dichlormethan (150 ml) wurde bei 0°C mit einer Lösung von **7-12A** und Triethylamin (40,2 ml, 288 mmol) in Dichlormethan (150 ml) versetzt und über Nacht bei Raumtemperatur gerührt. Die Reaktionsmischung wurde mit wässriger 1 M KHSO₄-Lösung und ges. NaCl-Lösung gewaschen, über Natriumsulfat getrocknet und unter vermindertem Druck konzentriert. Man erhielt **7-12B** (33,3 g, 95%)

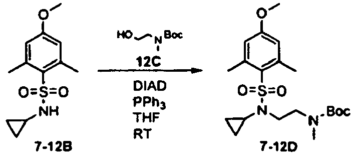

Eine Lösung von PPh₃ (12.33 g, 47.0 mmol) in THF (60 mL) wurde mit DIAD (8.22 mL, 42.3 mmol) versetzt und für 5 Minuten gerührt. Anschließend wurden **12C** (4.94 g, 28.2 mmol) in THF (60 mL) und **7-12B** (6.00 g, 23.5 mmol) in THF (60 mL) zugegeben und über Nacht bei Raumtemperatur gerührt. Die Reaktionsmischung wurde unter vermindertem Druck konzentriert und das Rohprodukt säulenchromatographisch (Silika (∼500 g); Heptan/EtOAc, 4:1 -> 3:1) gereinigt.
Ausbeute: 10,81 g

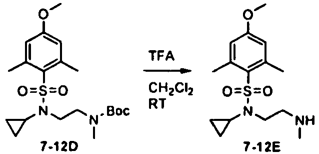

Eine Lösung von **7-12D** (10,81 g, 23,5 mmol) in Dichlormethan (100 ml) wurde mit Trifluoressigsäure (10 ml, 130 mmol) versetzt und über Nacht bei Raumtemperatur gerührt. Die Reaktionsmischung wurde unter vermindertem Druck konzentriert und das Rohprodukt säulenchromatographisch (Silika; CH₂Cl₂/(7 M NH₃ in MeOH), 98:2 - > 95:5) gereinigt. Man erhielt **7-12E** (6,35 g, 86%)

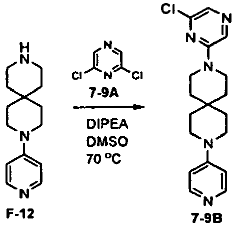

**F-12** (1,00 g, 4,23 mmol) und DIPEA (1,51 ml, 8,65 mmol) wurden zu einer Lösung von **7-9A** (0,708g, 4,75 mmol) in DMSO (3 ml) gegeben und für 5 Stunden auf 70°C erhitzt. Das Reaktionsgemisch wurde unter vermindertem Druck konzentriert und säulenchromatogaphisch säulenchromatographich (Silika; CH₂Cl₂/(7 M NH₃ in MeOH), 98:2 -> 9:1) gereinigt.
Ausbeute: 319 mg (21 %)

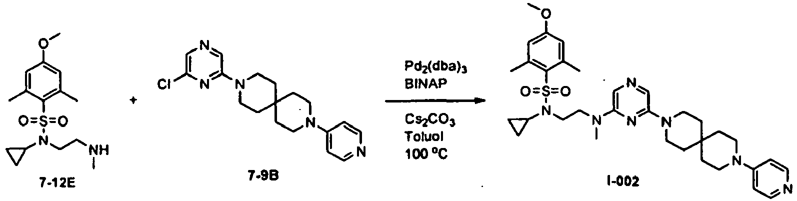

7-9B (400 mg, 1.16 mmol) und **7-12E** (400 mg, 1.28 mmol) wurden in Toluol (5 ml) gelöst, Pd₂(dba)₃ (43 mg, 0.047 mmol) und BINAP (44 mg, 0.070 mmol) zugegeben und das Reaktionsgemisch für 10 Minuten mit Argon überspült. Anschließend wurde auf 100°C erhitzt, 10 Minuten bei dieser Temperatur gerührt, auf Raumtemperatur abgekühlt, Cs₂CO₃ (57 mg, 0.17 mmol) zugegeben und für 5 Stunden bei 100°C gerührt. Nach Abkühlung auf Raumtemperatur wurde über Celite filtriert, das Filtrat konzentriert und der Rückstand säulenchromatographisch (Silika; CH₂Cl₂/(7 M NH₃ in MeOH), 95:5 -> 96.5:3.5) gereinigt, gefolgt von präparativer LC-MS. Die resultierenden Fraktionen wurden gesammelt, gefriergetrocknet, in DCM aufgenommen und konzentriert (2x), in DCM aufgenommen und über Natriumsulfat getrocknet und konzentriert.
Ausbeute: 137 mg (19%)

### Beispiel I-003: N-Cyclopropyl-4-methoxy-2,6-dimethyl-N-[2-[methyl-[6-(9-pyridin-4-yl-3,9-diazaspiro[5.5]undecan-3-yl)-pyrimidin-4-yl]-amino]-ethyl]-benzolsulfonsäure amid (I-003)

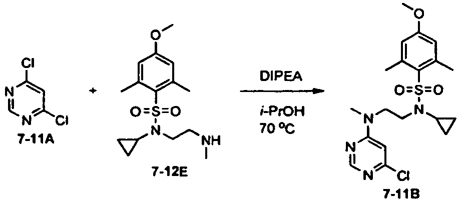

Zu einer Lösung von **7-11A** (0.477 g, 3.20 mmol) und **7-12E** (1.00 g, 3.20 mmol) in i-PrOH (2.5 mL) wurde DIPEA (0.839 mL, 4.80 mmol) gegeben und über Nacht bei 70°C gerührt. Das Reaktionsgemisch wurde auf Raumtemperatur abgekühlt, filtriert, mit 2-Propanol gewaschen und getrocknet.
Ausbeute: 0,893g, (66%)

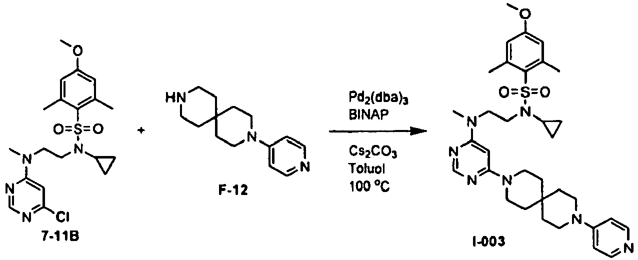

In Toluol (5ml) wurden nacheinander **7-11B** (400 mg, 0.941 mmol), **F-12** (218 mg, 0.941 mmol), Pd₂(dba)₃ (35 mg, 0.038 mmol) und BINAP (35 mg, 0.056 mmol) gelöst und 10 Minuten mit Argon überspült. Das Reaktionsgemisch wurde auf 100°C erhitzt, 10 Minuten bei dieser Temperatur gerührt, auf Raumtemperatur abgekühlt, mit Cs₂CO₃ (368 mg, 1,13 mmol) versetzt und unter Argon über Nacht bei 100°C gerührt. Nach Abkühlung auf Raumtemperatur wurde unter vermindertem Druck konzentriert. Der Rückstand wurde säulenchromatographisch (Silika; CH₂Cl₂/(7 M NH₃ in MeOH), 98:2 -> 95:5) gereinigt. Ausbeute: 192 mg (33%)

### Beispiel I-004: N-Cyclopropyl-4-methoxy-2,6-dimethyl-N-[2-[methyl-[2-(9-pyridin-4-yl-3,9-diazaspiro[5.5]undecan-3-yl)-pyrimidin-4-yl]-amino]-ethyl]-benzolsulfonsäure amid (I-004)

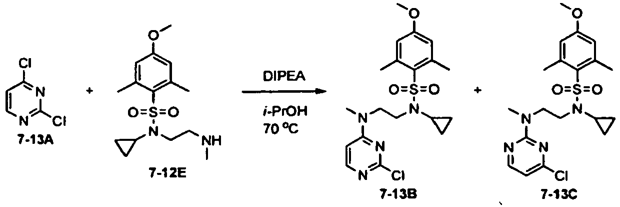

Zu einer Lösung von **7-13A** (0.477 g, 3.20 mmol) und **7-12E** (1.00 g, 3.20 mmol) in i-PrOH (2.5 mL) wurde DIPEA (0.839 mL, 4.80 mmol) gegeben und über Nacht bei 70°C gerührt. Das Reaktionsgemisch wurde auf Raumtemperatur abgekühlt, konzentriert und das Rohprodukt säulenchromatographisch (Silika; Heptan/EtOAc, 1:1) gereinigt und in **7-13B** (889 mg, 65%) und **7-13C** (nicht sauber) getrennt. **7-13C** wurde erneut säulenchromatographisch (Silika; Heptan/EtOAc, 3:1) gereinigt (114 mg, 8%).
Ausbeute: 0,889g; (65%); 7-13B, 0,114g; 8%; 7-13C

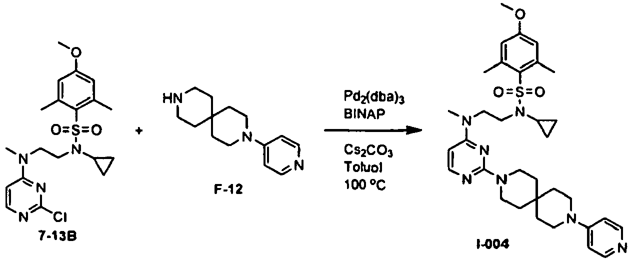

In Toluol (5ml) wurden nacheinander **7-13B** (400 mg, 0.941 mmol), **F-12** (218 mg, 0.941 mmol), Pd₂(dba)₃ (35 mg, 0.038 mmol) und BINAP (35 mg, 0.056 mmol) gelöst und 10 Minuten mit Argon überspült. Das Reaktionsgemisch wurde auf 100°C erhitzt, 10 Minuten bei dieser Temperatur gerührt, auf Raumtemperatur abgekühlt, mit Cs₂CO₃ (368 mg, 1,13 mmol) versetzt und unter Argon über Nacht bei 100°C gerührt. Nach Abkühlung auf Raumtemperatur wurde unter vermindertem Druck konzentriert. Der Rückstand wurde in DCM/ 7M NH₃ in MeOH aufgenommen, filtriert und konzentriert. Das Rohprodukt wurde säulenchromatographisch (Silika; CH₂Cl₂/(7 M NH₃ in MeOH), 98:2 -> 95:5) gereinigt.
Ausbeute: 308 mg, (53%)

### Beispiel I-005: N-Cyclopropyl-4-methoxy-2,6-dimethyl-N-[2-[methyl-[4-(9-pyridin-4-yl-3,9-diazaspiro[5.5]undecan-3-yl)-pyrimidin-2-yl]-amino]-ethyl]-benzolsulfonsäure amid (I-005)

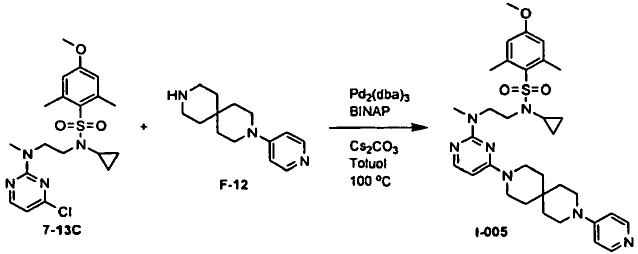

In Toluol (1ml) wurden nacheinander **7-13C** (165 mg, 0.165 mmol), **F-12** (38 mg, 0.165 mmol), Pd₂(dba)₃ (6 mg, 0.07 mmol) und BINAP (6 mg, 0.07 mmol) gelöst und 10 Minuten mit Argon überspült. Das Reaktionsgemisch wurde auf 100°C erhitzt, 10 Minuten bei dieser Temperatur gerührt, auf Raumtemperatur abgekühlt, mit Cs₂CO₃ (64 mg, 0,198 mmol) versetzt und unter Argon über Nacht bei 100°C gerührt. Nach Abkühlung auf Raumtemperatur wurde unter vermindertem Druck konzentriert. Der Rückstand wurde über präparative DC (Silika; CH₂Cl₂/(7 M NH₃ in MeOH), 95:5) gereinigt.
Ausbeute: 51 mg (50%)

### Beispiel I-006: N-Cyclopropyl-4-methoxy-2,6-dimethyl-N-[3-[6-(9-pyridin-4-yl-3,9-diazaspiro[5.5]undecan-3-yl)-pyrazin-2-yl]-propyl]-benzolsulfonsäure amid (I-006)

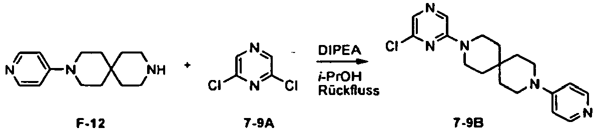

Zu einer Lösung von **F-12** (7.61 g, 32.9 mmol) in i-PrOH (20 mL) wurde DIPEA (5.75 mL, 32.9 mmol) und 2,6-Dichlorpyrazin (**7-9A**, 4.90 g, 32.9 mmol) gegeben und über Nacht zum Rückfluss erhitzt. Anschließend wurde nochmals 2,6-Dichlorpyrazin **7-9A** (1.17 g, 7.8 mmol) und DIPEA (1.4 mL, 8.0 mmol) zugegeben und weitere 4 Stunden refluxiert. Die Reaktionslösung wurde unter vermindertem Druck bis zur Trockene eingeengt und säulenchromatographisch (Silika, CH₂Cl₂/(7 M NH₃ in MeOH, 98:2) gereinigt.
Ausbeute: 5,6 g (50%)

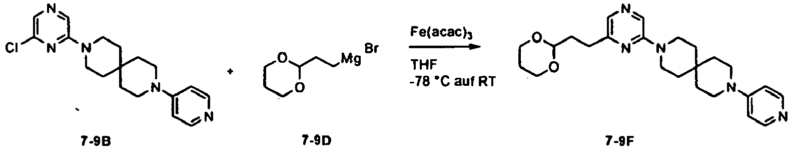

Eine Lösung von **7-9B** (4.70 g, 13.67 mmol) und Fe(acac)₃ (483 mg, 1.37 mmol) in THF (200 mL) wurde auf -78°C gekühlt und mit einer Lösung von (2-(1,3-Dioxan-2-yl)ethyl)magnesium bromid (**7-9D,** 0.5 M in THF 137 mL, 68.3 mmol) versetzt. Die Reaktionsmischung wurde langsam auf Raumtemperatur erwärmt, 3 Stunden gerührt und erneut auf -78°C gekühlt. (2-(1,3-Dioxan-2-yl)ethyl)magnesium bromid (**7-9D,** 0.5 M in THF, 82 mL, 41.0 mmol) wurde tropfenweise nachdosiert und die Mischung wurde abermals langsam auf Raumtemperatur erwärmt. Ges. NH₄Cl (600 mL) wurde zugegeben und das Reaktionsgemisch mit Dichlormethan (500 ml) extrahiert. Die vereinigten org. Phasen wurden mit ges.NaCl-Lösung gewaschen und die vereinigten wässrigen Phasen mit DCM (2 x 200 ml) extrahiert. Die vereinigten org. Phasen wurden über Natriumsulfat getrocknet, unter vermindertem Druck konzentriert und säulenchromatographisch (Silika, CH₂Cl₂/(7 M NH₃ in MeOH), 98:2) gereinigt.
Ausbeute: 4,92 g, 85% 7-9F

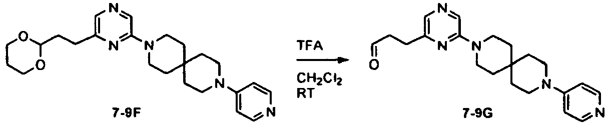

Eine Lösung von Acetal **7-9F** (1.0 g, 2.36 mmol) in TFA (148 g, 1.3 mol) und H₂O (20 mL) wurde 2 Stunden bei Raumtemperatur gerührt und anschließend bis zur Trockene eingeengt. Der Rückstand wurde jeweils in H₂O (50 mL), Toluol (2x 50 mL) und CH₂Cl₂ (3x 50 mL) aufgenommen und wieder konzentriert. Das Rohprodukt wurde in CH₂Cl₂ (100 mL) aufgenommen, mit ges. NaHCO₃-Lösung gewaschen, über Natriumsulfat getrocknet und bis zur Trockene unter vermindertem Druck konzentriert.
Ausbeute: 761 mg (88%)

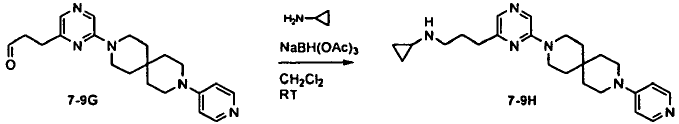

Eine Lösung von Aldehyd **7-9G** (761 mg, 2.08 mmol) in CH₂Cl₂ (2 mL) wurde mit Cyclopropylamin (587 µl, 8,33 mmol) und NaBH(OAC)₃ (353 mg, 1.67 mmol) versetzt und bei Raumtemperatur über Nacht gerührt. Das Reaktionsgemisch wurde unter vermindertem Druck bis zur Trockene konzentriert und das Rohprodukt säulenchromatographisch (Silika, CH₂Cl₂/(7 M NH₃ in MeOH), 98:2) gereinigt. Ausbeute: 313 mg (37%)

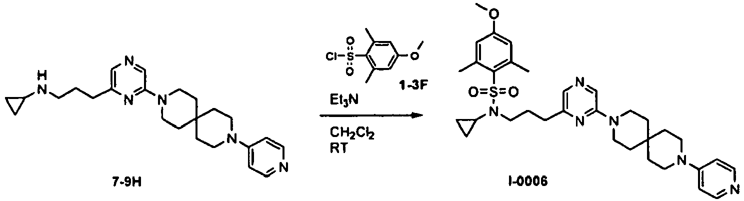

Eine Lösung von Amin **7-9H** (313 mg, 0.77 mmol) in CH₂Cl₂ (5 mL) und Et₃N (268 µL, 1.92 mmol) wurde mit einer Lösung von Sulfonylchlorid **1-3F** (271 mg, 1.155 mmol) in CH₂Cl₂ (5 mL)versetzt und über Nacht bei Raumtemperatur gerührt. Das Reaktionsgemisch wurde bis zur Trockene konzentriert und säulenchromatographisch (Silika, CH₂Cl₂/(7 M NH₃ in MeOH), 99:1) gereinigt.
Ausbeute: 417 mg (90%)

### Beispiel I-007: N-Cyclopropyl-4-methoxy-2,6-dimethyl-N-[3-[2-(9-pyridin-4-yl-3,9-diazaspiro[5.5]undecan-3-yl)-pyrimidin-4-yl]-propyl]-benzolsulfonsäure amid (I-007)

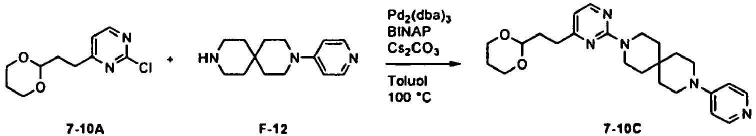

Zu einer Lösung von **7-10A** (1.90 g, 8.31 mmol), **F-12** (1.92 g, 8.31 mmol) in trockenem Toluol (50 ml) wurden nacheinander, Cs₂CO₃ (3.25 g, 9.97 mmol), BINAP (310 mg, 499 µmol) and Pd₂(dba)₃ (304 mg, 332 µmol) gegeben und 15 Minuten mit Argon überspült. Das Reaktionsgemisch wurde auf 100°C erhitzt, 3 Stunden bei dieser Temperatur gerührt und über Nacht auf Raumtemperatur abgekühlt. Die Reaktionsmischung wurde über Natriumsulfat filtriert und mit Dichlormethan gewaschen. Das Filtrat wurde mit ges. NaHCO₃-Lösung und ges. NaCl-Lösung gewaschen, über Natriumsulfat getrocknet und konzentriert. Das Rohprodukt wurde säulenchromatographisch (Silika, CH₂Cl₂/(7 M NH₃ in MeOH), 98:2) gereinigt.
Ausbeute: 750 mg (21 %)

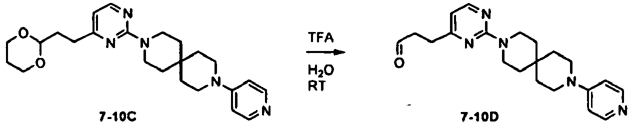

Eine Lösung von Acetal **7-10C** (750 mg, 1.77 mmol) in TFA (50 mL, 673 mmol) und H₂O (10 mL) wurde 2 Stunden bei Raumtemperatur gerührt und anschließend bis zur Trockene eingeengt. Der Rückstand wurde jeweils in Dichlormethan (2 x 50 mL), Toluol (50 mL) und Dichlormethan (50 mL) aufgenommen und wieder konzentriert. Das Rohprodukt wurde in DCM (30 mL) aufgenommen und mit ges. NaHCO₃-Lösung (100 ml) gewaschen, über Natriumsulfat getrocknet und bis zur Trockene unter vermindertem Druck konzentriert.
Ausbeute: 780 mg (100%)

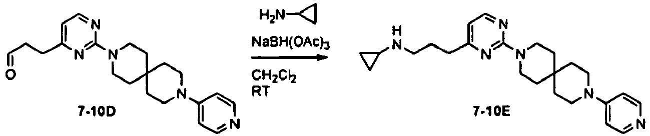

Zu einer Lösung von Aldehyd **7-10D** (780 mg, 1.77 mmol) in CH₂Cl₂ (10 ml) wurde Cyclopropylamin (1,25 ml, 17,7 mmol) und NaBH(OAc)₃ (751 mg, 3,54 mmol) gegeben und bei Raumtemperatur 2 Stunden gerührt. Das Reaktionsgemisch wurde über wenig Natriumsulfat filtriert, das Filtrat unter vermindertem Druck bis zur Trockene konzentriert und das Rohprodukt säulenchromatographisch (Silika, CH₂Cl₂/(7 M NH₃ in MeOH), 98:2) gereinigt.
Ausbeute: 313 mg (37%)

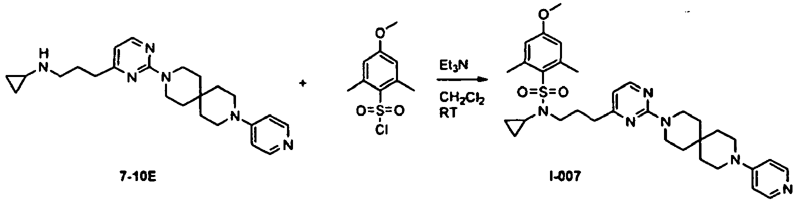

Eine Lösung von Amin **7-10E** (460 mg, 1.13 mmol) und Et₃N (394 µL, 2.83 mmol) in CH₂Cl₂ (15 mL) wurde bei 0°C mit einer Lösung von 4-Methoxy-2,6-dimethylbenzolsulfonsäurechlorid (398 mg, 1,70 mmol) in CH₂Cl₂ (10 mL).versetzt und über Nacht bei Raumtemperatur gerührt. Das Reaktionsgemisch wurde bis zur Trockene konzentriert und säulenchromatographisch (Silika, CH₂Cl₂/(7 M NH₃ in MeOH), 98:2) gereinigt.
Ausbeute: 325 mg (48%)

### Beispiel I-008: N-Cyclopropyl-N-[3-[2-(9-pyridin-4-yl-3,9-diazaspiro[5.5]undecan-3-yl)-pyrimidin-4-yl]-propyl]-3-(trifluormethyl)-benzolsulfonsäure amid (I-008)

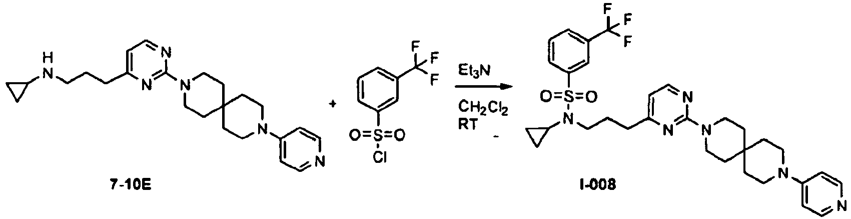

Eine Lösung von Amin **7-10E** (138 mg, 339 µmol) und Et₃N (118 µL, 849 µmol) in CH₂Cl₂ (5 mL) wurde bei 0°C mit einer Lösung von 3-(Trifluoromethyl)phenyl-1-sulfonyl chlorid (82 µL, 509 µmol) in CH₂Cl₂ (5 mL) versetzt und für 2 Stunden bei Raumtemperatur gerührt. Das Reaktionsgemisch wurde bis zur Trockene konzentriert und säulenchromatographisch (Silika, CH₂Cl₂/(7 M NH₃ in MeOH), 99:1) gereinigt.
Ausbeute: 61 mg (30%)

**Tabelle 6: Synthese der Derivate I**

| **Beispiel Nr.** | **Struktur** | **Name** | **Amin (F) -** | **Ausbeute letzte Stufe** | **Analytik (LC/MS)^{[Fehte ri Textmarke nicht definiert.]}** |
|---|---|---|---|---|---|
| I-001 | 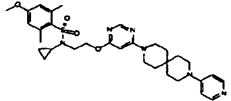 | N-Cyclopropyl-4-ethoxy-2.6-dimethyl-N-[2-[[6-[9-(4-pyridyl)-3,9-diazaspiro[5.5]undecan-3-yl]-4-pyrimidinyl)oxy)ethyl]phenylsulfonamid (I-001) | 3-(Pyridin-4-yl)-3,9-diazaspiro[5.5]undeca n (F-12) | 7% | Rₜ = 3.4 min; *m*/*z* = 607.5 [MH]⁺ |
| I-002 | 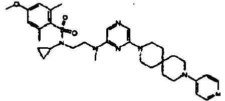 | N-Cyclopropyl-4-methoxy-2,6-dimethyl-N-[2-(methyl-[6-(9-pyridin-4-yl-3,9-diazaspiro[5.5]undecan-3-yl)-pyrazin-2-yl]-amino]-ethyl]-benzolsulfonsäure amid (I-002) | 3-(Pyridin-4-yl)-3,9-diazaspiro[5.5]undeca n (F-12) | 137 mg (19%) | Rₜ = 3.4 min; ***m*/*z*** = 620.4 [MH]⁺ |
| I-003 | 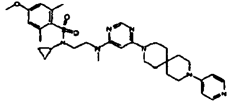 | N-Cyclopropyl-4-methoxy-2,6-dimethyl-N-[2-[methyl-(6-(9-pyridin-4-y1-3,9-diazaspiro[5.5]undecan-3-yl)-pyümidin-4-yl]-amino]-ethyl]-benzolsulfonsäure amid (I-003) | 3-(Pyridin-4-yl)-3.9-diazaspiro[5.5]undeca n (F-12) | 192 mg (33%) | Rₜ = 3.3 min; *m*/*z* = 620.4 [MH]⁺ |
| I-004 | 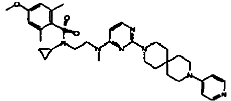 | N-Cyclopropyl-4-methoxy-2,6-dimethyl-N-[2-methyl-2-(9-pyridin-4-yl-3.9-diazaspiro[5.5]undecan-3-yl)-pyrimidin-4-yl]-amino]-ethyl]-benzolsulfonsaüre amid (I-004) | 3-(Pyridin-4-yl)-3.9-diazaspiro[5.5]undeca n (F-12) | 308 mg (53%) | Rₜ = 3.2 min; *mlz* = 620.4 [MH]⁺ |
| I-005 | 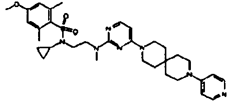 | N-Cyclopropyl-4-methoxy-2,8-dimethyl-N-[2-[methyl-(4-(9-pyridin-4-yl-3,9-diazaspiro[5.5]undecan-3-yl)-pyrimidin-2-yl]-amino]-ethyl]-benzolsulfonsäure amid (I-005) | 3-(Pyridin-4-yl)-3,9-diazaspiro[5.5]undeca n (F-12) | 51 mg (50%) | Rₜ=3.0min; *m*/*z* = 620.4 [MH]⁺ |
| I-006 | 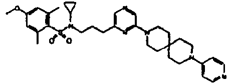 | N-Cyclopropyl-4-methoxy-2,6-dimethyl-N-[3-[6-(9-pyridin-4-yl-3,9-diazaspiro[5.5]undecan-3-yl)-pyrazin-2-yl]-propyl]-benzolsulfonsäure amid (I006) | 3-(Pyridin-4-yl)-3,9-diazaspiro[5.5]undeca n (F-12) | 417 mg (90%) | Rₜ = 3.3 min; *m*/*z* = 605.5 [MH]⁺ |
| I-007 | 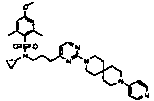 | N-Cyclopropyl-4-methoxy-2,6-dimethyl-N-[3-[2-(9-pyridin-4-yl-3,9-diazaspiro[5.5]undecan-3-yl)-pyrimidin-4-yl]-propyl]-benzolsulfönsäure amid (I-007) | 3-(Pyridin-4-yl)-3,9-diazaspiro[5.5]undeca n (F-12) | 325 mg (48%) | Rₜ = 4.0 min; *m*/*z* = 605.5 [MH]⁺ |
| I-008 | 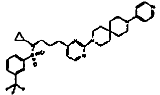 | N-Cyclopropyl-N-[3-[2-(9-pyridin-4-yl-3,9-diazaspiro[5.5]undecan-3-yl)-Pyrimidin-4-yl]-propyl]-3-(trifluormethyl)-benzolsulfonsäure amid (I-008) | 3-(Pyridin-4-yl)-3,9-diazaspiro[5.5]undeca n (F-12) | 61 mg (30%) | Rₜ = 4.0 min; *mlz* = 615.4 [MH]⁺ |

### Parallelsynthesen

### 7) Parallelsynthese der Pyrimidinderivate G_CC & H_CC

### Parallel Methode zur Synthese der Pyrimidinderivate G_CC & H_CC

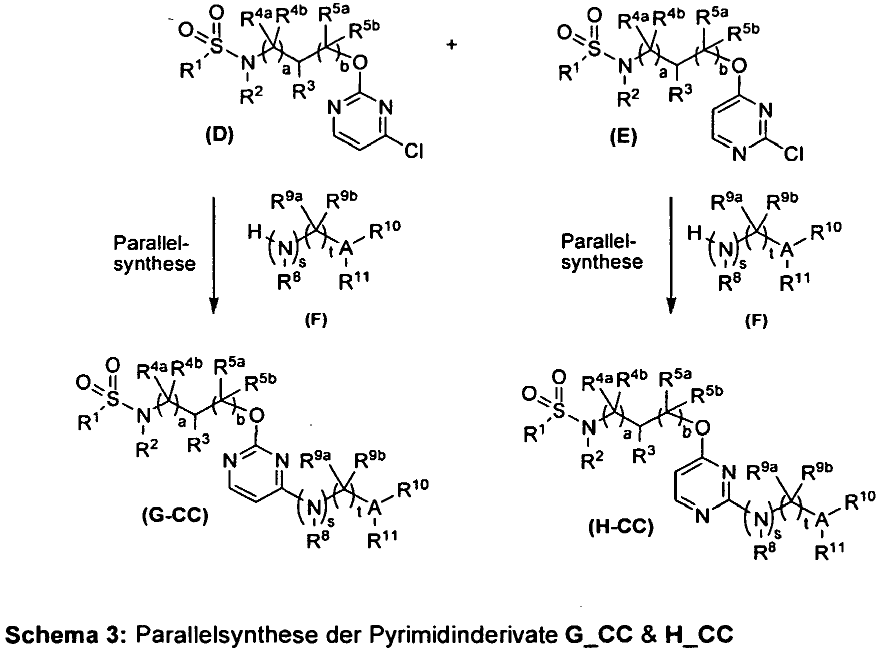

Nach obiger Abbildung wurden sowohl die Pyrimidinbausteine **D** als auch die Pyrimidinbausteine **E** parallelsynthetisch mit Aminn **F** zu den Pyrimidinderivaten **G_CC** und **H_CC** umgesetzt. Die Korrelation von Produkt zu Reagenz und Bausteinen ist der Synthesematrix zu entnehmen.

Die Rohprodukte der Parallelsynthese wurden durch HPLC-MS analysiert und anschließend mittels Reverse Phase HPLC-MS aufgereinigt. Die Identität der Produkte konnte durch analytische HPLC-MS Messungen nachgewiesen werden. Es wurde zugrunde gelegt, dass aus den Regioisomeren **D** nach Umsetzung mit **F** die Pyrimidin-Regioisomere **G_CC** erhalten werden. Entsprechendes gilt für die Überführung der Regioisomere E in die Pyrimidin-Regioisomere **H_CC.** Für den Fall, dass im Rahmen der Parallelsynthese, Mischungen aus **G_CC** und **H_CC** erhalten wurden (z.B. Aufgrund der Verwendung von **D** & **E** Gemischen als Edukt in der Parallelsynthese), wurde angenommen, dass **G_CC** immer das Regioisomer mit der kürzeren Retentionszeit ist.

### Parallelsynthese:

### Synthesevorschrift zur Herstellung der Pyrimidinderivate G_CC & H_CC

Eine Lösung des Chlorpyrimidins **D** (bzw. **E**) (125 µM) in 1 ml iso-Propanol wird mit einer Lösung von Diisopropylethylamin (187 µM) in 1 ml iso-Propanol versetzt und bei Raumtemperatur für 15 min geschüttelt. Anschließend wir eine Lösung des Amins **F** (187 µM) in 1 ml iso-Propanol zugegeben und das Reaktionsgemisch für 10 h bis 15 h bei 60°C geschüttelt. Bei Verwendung von Amin-Hydrochloriden **(F + n HCl)** wurde, wurde die Menge des Diisopropylamins auf 1.5 Äquiv. + n Äquiv. erhöht. Zur Aufarbeitung wurden die Ansätze mit 2 ml einer 2M Natriumhydroxid-Lsg., 1 ml einer ges. Natriumchlorid-Lsg. und 1 ml Ethylacetat versetzt. Die weitere Aufarbeitung erfolgte auf einem Myriad-Allex-Aufarbeitungssystem (Firma: Mettler-Toledo). Nach Durchmischung wurde die organische Phase separiert, die wässrige mit 2x mit 3 ml Ethylacetat extrahiert und die organischen Phasen vereinigt. Die Entfernung des Lösungsmittels erfolgte unter Vakuum in Vakuumzentrifugen (Firma GeneVac). Die finale Aufreinigung erfolgte über HPLC-MS. Die finale Analytik wurde mittels LC-MS durchgeführt.

### Geräte und Methoden für die HPLC-MS Analytik:

Parallelsynthese Methode: HPLC: Waters Alliance 2795 mit PDA Waters 996; MS: ZQ 2000 MassLynx Single Quadrupol MS Detektor; Säule: Atlantis dC18 30 x 2.1 mm, 3 µm; Säulentemperatur: 40 °C, Eluent A: Wasser + 0.1 % Ameisensäure; Eluent B: Methanol + 0.1% Ameisensäure; Gradient: 0% B zu 100% B in 2.3 min, 100% B für 0.4 min, 100% B zu 0% B in 0.01 min, 0% B für 0.8 min; Fluß: 1.0 mL/min; Ionisation: ES+, 25V; Make up: 100µL/min 70% Methanol + 0.2% Ameisensäure; UV: 200 - 400 nm

Geräte und Methoden für die HPLC-MS Aufreinigung: *Prep Pump:* Waters 2525; *Make up Pump:* Waters 515; *Auxilary Detektor:* Waters DAD 2487; *MS Detektor:* Waters Micromass ZQ; *Injector* / *Fraktionssammler:* Waters Sample Manager 2767; *Gradient: Initial:* 50% Water / 50% Methanol -> 2-20 *min:* 0% Water 100% Methanol; *Fluß:* 35 ml/min *Säule: Phenomenex Gemini,* C18, 100x21,2 mm, Axia, 110A, 5µ.

In der nachfolgenden Tabelle 6 sind die Verbindungen, die mittels der oben beschriebenen Parallelsynthesen hergestellt wurden, sowie die dazu verwendeten Synthesebausteine aufgeführt.

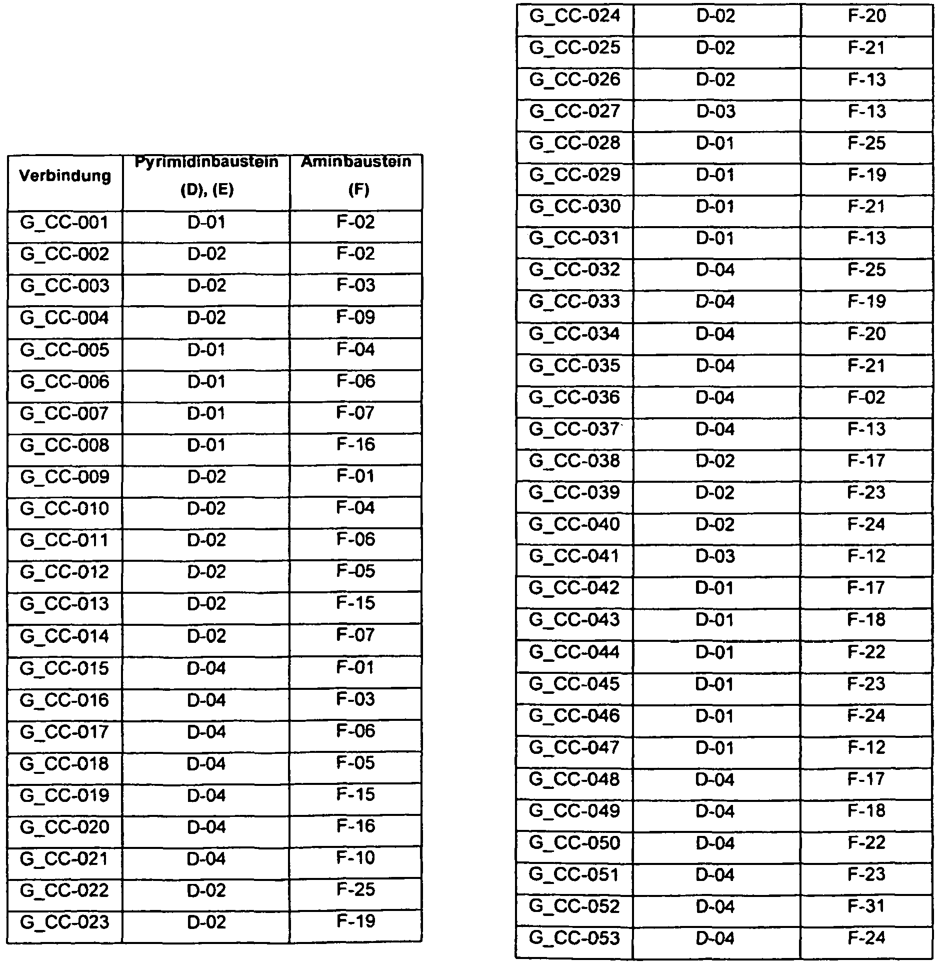

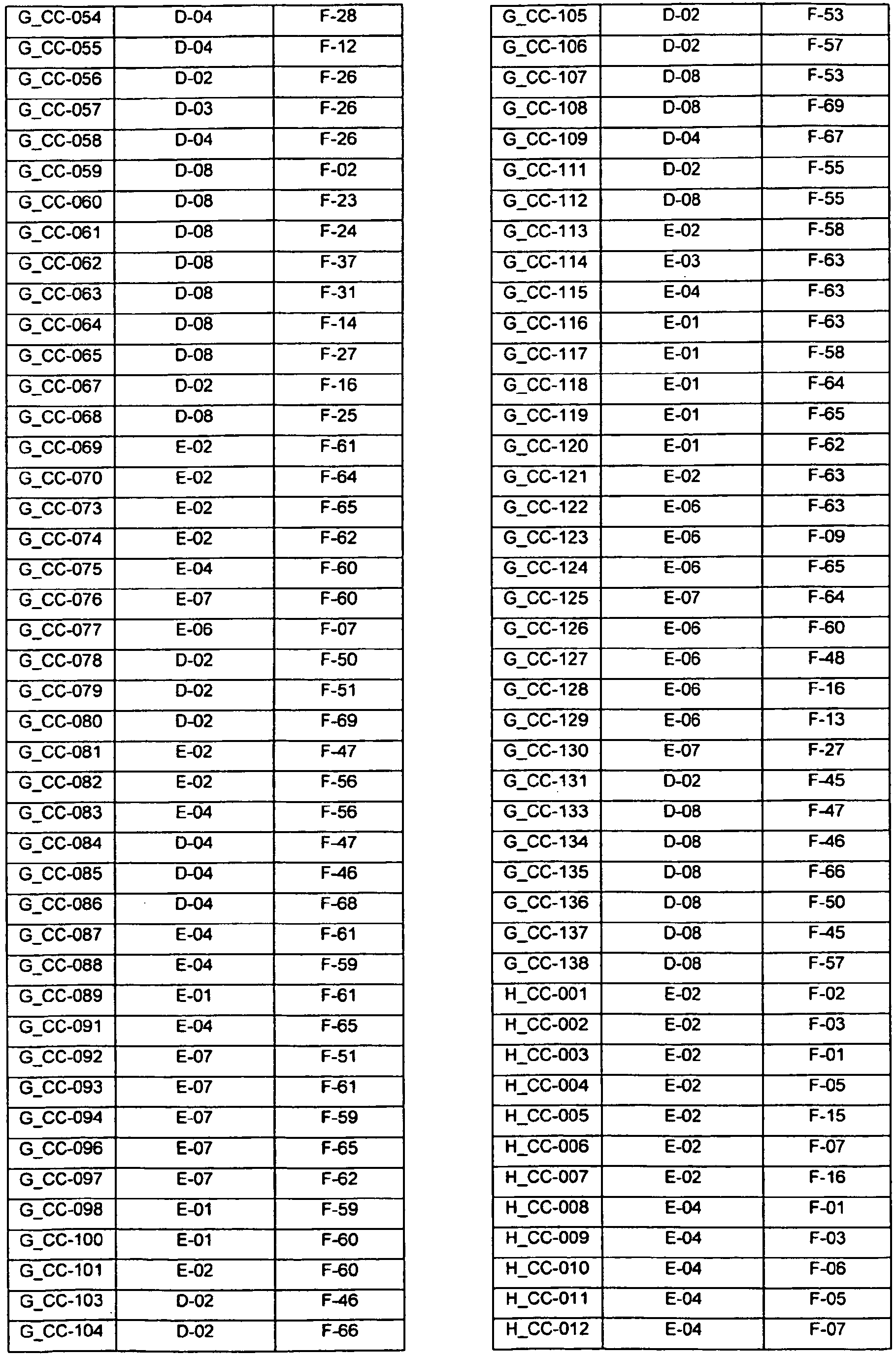

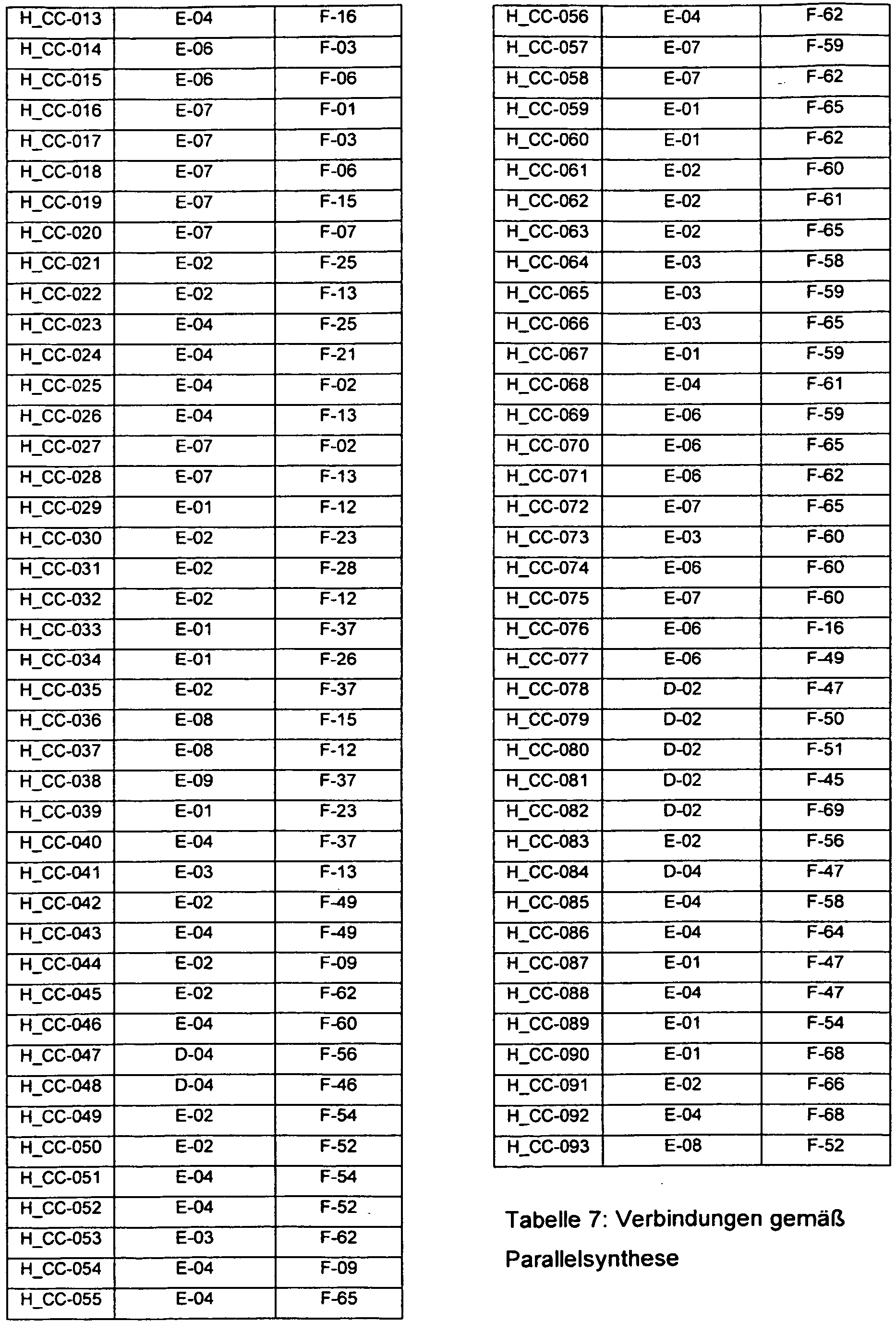

### 1 Funktionelle Untersuchung am Bradykinin Rezeptor 1 (B1R)

Die agonistische bzw. antagonistische Wirkung von Substanzen kann am Bradykinin Rezeptor 1 (B1R) der Spezies Mensch und Ratte mit folgendem Assay bestimmt werden. Gemäß diesem Assay wird der Ca²⁺-Einstrom durch den Kanal mit Hilfe eines Ca²⁺-sensitiven Farbstoffs (Typ Fluo-4, Molecular Probes Europe BV, Leiden Niederlande) im Fluorescent Imaging Plate Reader (FLIPR, Molecular Devices, Sunnyvale, USA) quantifiziert.

### 2 Methode:

Es werden Chinese Hamster Ovary Zellen (CHO K1 Zellen) verwendet, die stabil mit dem humanen B1 R-Gen (hB1 R-Zellen) bzw. dem B1 R-Gen der Ratte (rB1 R-Zellen), transfiziert sind. Für funktionelle Untersuchungen werden diese Zellen auf schwarze 96-Loch-Platten mit klarem Boden (BD Biosciences, Heidelberg, Deutschland oder Greiner, Frickenhausen, Deutschland) in einer Dichte von 20.000 - 35.000 Zellen/Loch ausplattiert. Über Nacht werden die Zellen bei 37 °C und 5 % CO₂ in Kulturmedium (hB1R-Zellen: Nutrient Mixture Ham's F12, Gibco Invitrogen GmbH, Karlsruhe, Deutschland oder DMEM, Sigma-Aldrich, Taufkirchen, Deutschland; rB1R-Zellen: D-MEM/F12, Gibco Invitrogen GmbH, Karlsruhe, Deutschland) mit 10 Vol-% FBS (Fetal bovine serum, Gibco Invitrogen GmbH, Karlsruhe, Deutschland oder PAN Biotech GmbH, Aidenbach, Deutschland) belassen.

Am folgenden Tag werden die Zellen mit 2,13 µM Fluo-4 (Molecular Probes Europe BV, Leiden Niederlande) in HBSS-Puffer (Hank's buffered saline solution, Gibco Invitrogen GmbH, Karlsruhe, Deutschland) mit 2,5 mM Probenecid (Sigma-Aldrich, Taufkirchen, Deutschland) und 10 mM HEPES (Sigma-Aldrich, Taufkirchen, Deutschland) für 60 min bei 37 °C beladen. Anschließend werden die Platten 2 x mit HBSS-Puffer gewaschen und mit HBSS-Puffer versetzt, der zusätzlich 0,1 % BSA (bovines Serumalbumin; Sigma-Aldrich, Taufkirchen, Deutschland), 5,6 mM Glucose und 0,05 % Gelatine (Merck KGaA, Darmstadt, Deutschland) enthält. Nach einer weiteren Inkubation von 20 Minuten bei Raumtemperatur werden die Platten zur Ca²⁺-Messung im FLIPR eingesetzt.

Alternativ wird mit Puffer A (15 mM HEPES, 80 mM NaCl, 5 mM KCl, 1,2 mM CaCl₂, 0,7 mM MgSO₄, 2g/L Glucose, 2,5 mM Probenecid) gewaschen und mit Puffer A versetzt mit 2,5 µM Fluo-4 und 0,025 % Pluronic F127 (Sigma-Aldrich, Taufkirchen, Deutschland) beladen. Danach werden die Zellen 2 x mit Puffer A gewaschen und für 30 Minuten mit Puffer A, der zusätzlich 0,05 % BSA und 0,05 % Gelatine enthält bei Raumtemperatur inkubiert und danach zur Ca²+-Messung im FLIPR eingesetzt.

Die Ca²⁺-abhängige Fluoreszenz wird dabei vor und nach Zugabe von Substanzen gemessen (λₑₓ = 488 nm, λₑₘ = 540 nm). Die Quantifizierung erfolgt durch die Messung der höchsten Fluoreszenzintensität (FC, Fluorescence Counts) über die Zeit.

### 3 FLIPR-Assay:

Das FLIPR-Protokoll besteht aus 2 Substanzzugaben. Zunächst werden Testsubstanzen (10 µM) auf die Zellen pipettiert und der Ca²⁺-Einstrom mit der Kontrolle (hB1R: Lys-Des-Arg⁹-Bradykinin >= 50 nM; rB1R: Des-Arg⁹-Bradykinin 10 µM) verglichen. Daraus ergibt sich die Angabe in % Aktivierung bezogen auf das Ca²⁺-Signal nach Zugabe von Lys-Des-Arg⁹-Bradykinin (>= 50 nM), bzw. Des-Arg⁹-Bradykinin (10 µM).

Nach 10-20 Minuten Inkubation werden Lys-Des-Arg⁹-Bradykinin (hB1 R) bzw. Des-Arg⁹-Bradykinin (rB1 R) in der Konzentration des EC₈₀ appliziert und ebenfalls der Einstrom von Ca²⁺ ermittelt.

Antagonisten führen zu einer Unterdrückung des Ca²⁺-Einstroms. Es werden % Inhibition im Vergleich zu der maximal erreichbaren Inhibition berechnet.

Zur Bestimmung des IC₅₀-Wertes werden die Substanzen in verschiedenen Konzentrationen zugegeben. Es werden Zweifach- oder Dreifach-Bestimmungen (n=2 oder n=3) durchgeführt und diese in mindestens einem weiteren unabhängigen Experiment wiederholt (N>=2).

Vorzugsweise weisen die Verbindungen eine B1 R antagonistische Wirkung am humanen Rezeptor und/oder am Ratten-Rezeptor auf. Beispielhaft werden in der nachfolgenden Tabelle 8 die folgenden Daten angegeben: (Dabei steht "% Inh. (rat B1 R) 10 µM" für" % Inhibition Ratte B1R bei 10 M" und "% Inh. (hum. B1 R) 10 µM" für" % Inhibition human B1R bei 10 µM"

**Tabelle 8: % Inhibition am B1R der Ratte sowie am humanen B1R (10 µM) [*] und [**]: Nur die Daten für Verbindungen aus der Parallelsynthese sind in dieser Tabelle aufgeführt. Für die Verbindungen aus den Einzelsynthesen sind diese Daten in den Tabellen 4, 5 und 6 aufgeführt.**

| **Verbindung** | **[M+] gefunden*** | **Rt [min]**** | **% Inh. (rat B1R) 10 µM** | **% Inh. (hum. B1R) 10 µM** |
|---|---|---|---|---|
| G-001 | | | 99 | 84 |
| G-002 | | | 109 | 97 |
| G-003 | | | 112 | 101 |
| G-004 | | | 73 | 99 |
| G-005 | | | 96 | 99 |
| G-006 | | | 94 | 99 |
| G-007 | | | 97 | 100 |
| G-008 | | | 98 | 77 |
| G-009 | | | 91 | 97 |
| G-010 | | | 89 | 98 |
| G-011 | | | 99 | 99 |
| G-012 | | | 97 | 99 |
| G-013 | | | 102 | 92 |
| G-014 | | | 102 | 100 |
| G-015 | | | 95 | 99 |
| G-016 | | | 99 | 96 |
| G-017 | | | 103 | 22 |
| G-018 | | | 93 | 99 |
| G-019 | | | 102 | 100 |
| G-020 | | | 101 | 95 |
| G-021 | | | 86 | 90 |
| G-022 | | | 103 | 100 |
| G-023 | | | 107 | 94 |
| G-024 | | | 113 | 99 |
| G-025 | | | 104 | 92 |
| G-026 | | | 101 | 98 |
| H-001 | | | 99 | 44 |
| H-002 | | | 51 | 81 |
| H-003 | | | 71 | 90 |
| H-004 | | | 93 | 99 |
| H-005 | | | 97 | 98 |
| H-006 | | | 92 | 64 |
| H-007 | | | 80 | 11 |
| I-001 | | | 88 | 98 |
| I-002 | | | 109 | 94 |
| I-003 | | | 108 | 96 |
| I-004 | | | 102 | 85 |
| I005 | | | 111 | 96 |
| I006 | | | 97 | 94 |
| I007 | | | | |
| I-08 | | | 94 | |
| G_CC-001 | 533.2 | 1.30 | 97 | 98 |
| G_CC-002 | 573.3 | 1.46 | 101 | 99 |
| G_CC-003 | 572.3 | 1.56 | 102 | 100 |
| G_CC-004 | 651.3 | 1.61 | 100 | 100 |
| G_CC-005 | 530.2 | 2.10 | 81 | |
| G_CC-006 | 533.3 | 1.33 | 97 | |
| G_CC-007 | 547.3 | 1.22 | 95 | |
| G_CC-008 | 528.2 | 1.50 | 59 | |
| G_CC-009 | 490.2 | 1.56 | 63 | |
| G_CC-010 | 570.2 | 2.24 | 70 | |
| G_CC-011 | 573.3 | 1.51 | 96 | 91 |
| G_CC-012 | 554.2 | 2.06 | 76 | |
| G_CC-013 | 553.2 | 1.60 | 102 | 100 |
| G_CC-014 | 587.3 | 1.42 | 105 | 94 |
| G_CC-015 | 476.1 | 1.48 | 83 | |
| G_CC-016 | 558.2 | 1.53 | 100 | 96 |
| G_CC-017 | 559.2 | 1.47 | 98 | |
| G_CC-018 | 540.1 | 2.00 | 97 | |
| G_CC-019 | 539.1 | 1.55 | 104 | 100 |
| G_CC-020 | 554.1 | 1.62 | 102 | |
| G_CC-021 | 559.1 | 1.99 | 105 | 36 |
| G_CC-022 | 606.2 | 1.72 | 104 | |
| G_CC-023 | | | 50 | |
| G_CC-024 | 641.1 | 2.17 | 73 | |
| G_CC-025 | 621.1 | 2.13 | 95 | |
| G_CC-026 | 586.2 | 1.60 | 100 | 97 |
| G_CC-027 | 572.2 | 1.49 | 63 | |
| G_CC-028 | 566.1 | 1.57 | 104 | |
| G_CC-029 | 539.1 | 2.11 | 52 | |
| G_CC-030 | 581.1 | 1.96 | 79 | |
| G_CC-031 | 546.2 | 1.49 | 104 | |
| G_CC-032 | 592.1 | 1.68 | 105 | 86 |
| G_CC-033 | 565.1 | 2.18 | 68 | |
| G_CC-034 | 627.1 | 2.15 | 93 | |
| G_CC-035 | 607.2 | 2.04 | 99 | -1 |
| G_CC-036 | 559.2 | 1.40 | 102 | 75 |
| G_CC-037 | 572.2 | 1.55 | 102 | |
| G_CC-038 | 638.2 | 2.16 | 94 | |
| G_CC-039 | 652.2 | 2.14 | 99 | 58 |
| G_CC-040 | 634.2 | 2.12 | 99 | 57 |
| G_CC-041 | 607.2 | 1.63 | 101 | 96 |
| G_CC-042 | 598.2 | 2.00 | 87 | |
| G_CC-043 | 648.2 | 2.06 | 70 | |
| G_CC-044 | 612.2 | 1.97 | 68 | |
| G_CC-045 | 612.2 | 2.01 | 96 | |
| G_CC-046 | 594.2 | 2.00 | 96 | |
| G_CC-047 | 581.2 | 1.60 | 99 | 100 |
| G_CC-048 | 624.2 | 2.10 | 96 | 28 |
| G_CC-049 | 674.2 | 2.12 | 72 | |
| G_CC-050 | 638.2 | 2.08 | 73 | |
| G_CC-051 | 638.2 | 2.12 | 98 | 38 |
| G_CC-052 | 554.2 | 1.60 | 99 | 74 |
| G_CC-053 | 620.2 | 2.10 | 96 | |
| G_CC-054 | 693.3 | 1.59 | 80 | |
| G_CC-055 | 607.2 | 1.72 | 101 | 97 |
| G_CC-056 | 579.2 | 1.66 | 98 | |
| G_CC-057 | 565.2 | 1.53 | 90 | |
| G_CC-058 | 565.2 | 1.61 | 93 | |
| G_CC-059 | 609.2 | 1.65 | 98 | |
| G_CC-060 | 688.3 | 2.25 | 88 | 17 |
| G_CC-061 | 670.3 | 2.25 | 81 | |
| G_CC-062 | 701.4 | 1.74 | 78 | |
| G_CC-063 | 604.3 | 1.72 | 101 | 28 |
| G_CC-064 | 617.3 | 2.05 | 55 | |
| G_CC-065 | 632.3 | 1.97 | 58 | |
| G_CC-067 | 568.2 | 1.69 | 104 | |
| G_CC-068 | 642.2 | 1.83 | 68 | |
| G_CC-069 | 574.5 | 1.72 | 102 | |
| G_CC-070 | 600.4 | 1.77 | 95 | |
| G_CC-073 | 677.6 | 1.66 | 101 | |
| G_CC-074 | 631.6 | 1.66 | 102 | |
| G_CC-075 | 651.5 | 1.47 | 104 | |
| G_CC-076 | 699.6 | 1.63 | 105 | |
| G_CC-077 | 545.4 | 1.53 | 92 | |
| G_CC-078 | 568.4 | 2.07 | 98 | |
| G_CC-079 | 569.4 | 2.02 | 98 | |
| G_CC-080 | 663.6 | 1.58 | 103 | |
| G_CC-081 | 552.4 | 1.96 | 88 | |
| G_CC-082 | 568.4 | 1.87 | 73 | |
| G_CC-083 | 554.4 | 1.82 | 62 | |
| G_CC-084 | 538.4 | 1.86 | 83 | |
| G_CC-085 | 606.4 | 2.03 | 101 | |
| G_CC-086 | 668.5 | 1.88 | 105 | |
| G_CC-087 | 560.4 | 1.68 | 101 | |
| G_CC-088 | 637.5 | 1.36 | 100 | |
| G_CC-089 | 534.4 | 1.58 | 93 | |
| G_CC-091 | 663.6 | 1.57 | 101 | |
| G_CC-092 | 603.4 | 2.14 | 85 | |
| G_CC-093 | 608.5 | 1.87 | 91 | |
| G_CC-094 | 685.6 | 1.72 | 102 | |
| G_CC-096 | 711.6 | 1.75 | 101 | |
| G_CC-097 | 665.6 | 1.74 | 101 | |
| G_CC-098 | 611.5 | 1.47 | 79 | |
| G_CC-100 | 625.5 | 1.37 | 89 | |
| G_CC-101 | 665.6 | 1.52 | 105 | |
| G_CC-103 | 620.4 | 2.08 | 93 | |
| G_CC-104 | 667.5 | 1.85 | 101 | |
| G_CC-105 | 594.4 | 1.87 | 101 | |
| G_CC-106 | 625.5 | 1.61 | 95 | |
| G_CC-107 | 630.4 | 1.98 | 94 | |
| G_CC-108 | 699.6 | 1.75 | 102 | |
| G_CC-109 | 596.5 | 1.66 | 98 | |
| G_CC-111 | 568.4 | 1.64 | 90 | |
| G_CC-112 | 604.4 | 1.77 | 88 | |
| G_CC-113 | 594.5 | 1.78 | 79 | |
| G_CC-114 | 606.5 | 1.66 | 54 | |
| G_CC-115 | 606.4 | 1.75 | 81 | |
| G_CC-116 | 580.4 | 1.62 | 64 | |
| G_CC-117 | 554.4 | 1.61 | 56 | |
| G_CC-118 | 560.4 | 1.60 | 86 | |
| G_CC-119 | 637.5 | 1.54 | 79 | |
| G_CC-120 | 591.5 | 1.49 | 86 | |
| G_CC-121 | 620.5 | 1.79 | 75 | |
| G_CC-122 | 578.4 | 1.95 | 51 | |
| G_CC-123 | 609.5 | 1.66 | 81 | |
| G_CC-124 | 635.5 | 1.69 | 57 | |
| G_CC-125 | 634.4 | 1.92 | 58 | |
| G_CC-126 | 623.5 | 1.55 | 77 | |
| G_CC-127 | 519.4 | 1.55 | 54 | |
| G_CC-128 | 526.3 | 1.72 | 57 | |
| G_CC-129 | 544.4 | 1.65 | 73 | |
| G_CC-130 | 630.4 | 1.91 | 61 | |
| G_CC-131 | 574.4 | 1.54 | 88 | |
| G_CC-133 | 588.4 | 2.02 | 78 | |
| G_CC-134 | 656.4 | 2.14 | 88 | |
| G_CC-135 | 703.6 | 1.89 | 53 | |
| G_CC-136 | 604.4 | 2.16 | 81 | |
| G_CC-137 | 610.5 | 1.67 | 77 | |
| G_CC-138 | 661.5 | 1.75 | 56 | |
| H_CC-001 | 573.3 | 1.75 | 65 | |
| H_CC-002 | 572.3 | 1.82 | 77 | |
| H_CC-003 | 490.2 | 1.97 | 58 | |
| H_CC-004 | 554.2 | 2.54 | 57 | |
| H_CC-005 | 553.2 | 2.00 | 94 | |
| H_CC-006 | 587.3 | 1.74 | 61 | |
| H_CC-007 | 568.2 | 2.01 | 71 | |
| H_CC-008 | 476.1 | 1.87 | 95 | |
| H_CC-009 | 558.2 | 1.75 | 85 | |
| H_CC-010 | 559.2 | 1.75 | 100 | |
| H_CC-011 | 540.1 | 2.39 | 56 | |
| H_CC-012 | 573.2 | 1.70 | 93 | |
| H_CC-013 | 554.1 | 1.87 | 79 | |
| H_CC-014 | 530.2 | 1.91 | 53 | |
| H_CC-015 | 531.2 | 1.89 | 56 | |
| H_CC-016 | 524.1 | 2.08 | 67 | |
| H_CC-017 | 606.2 | 2.00 | 93 | |
| H_CC-018 | 607.2 | 2.03 | 91 | |
| H_CC-019 | 587.1 | 2.16 | 98 | |
| H_CC-020 | 621.2 | 1.87 | 93 | |
| H_CC-021 | 606.2 | 1.96 | 62 | |
| H_CC-022 | 586.2 | 1.83 | 77 | |
| H_CC-023 | 592.1 | 1.89 | 76 | |
| H_CC-024 | 607.1 | 2.35 | 97 | |
| H_CC-025 | 559.2 | 1.72 | 65 | |
| H_CC-026 | 572.2 | 1.78 | 75 | |
| H_CC-027 | 607.2 | 1.91 | 91 | |
| H_CC-028 | 620.2 | 2.02 | 83 | |
| H_CC-029 | 581.2 | 1.79 | 101 | 92 |
| H_CC-030 | | | 49 | |
| H_CC-031 | 707.3 | 1.91 | 56 | |
| H_CC-032 | 621.2 | 1.96 | 101 | 77 |
| H_CC-033 | 625.2 | 1.80 | 82 | |
| H_CC-034 | 539.2 | 1.79 | 74 | |
| H_CC-035 | 665.3 | 2.01 | 86 | |
| H_CC-036 | 589.2 | 2.12 | 71 | |
| H_CC-037 | 657.3 | 2.08 | 102 | 54 |
| H_CC-038 | 693.4 | 2.12 | 70 | |
| H_CC-039 | 612.2 | 2.23 | 50 | |
| H_CC-040 | 651.3 | 1.95 | 51 | |
| H_CC-041 | | | 48 | |
| H_CC-042 | 581.2 | 2.03 | 61 | |
| H_CC-043 | 567.2 | 1.98 | 80 | |
| H_CC-044 | 651.5 | 1.85 | 102 | |
| H_CC-045 | 631.6 | 1.86 | 96 | |
| H_CC-046 | 651.5 | 1.67 | 89 | |
| H_CC-047 | 554.4 | 1.97 | 85 | |
| H_CC-048 | 606.4 | 2.38 | 63 | |
| H_CC-049 | 625.5 | 1.81 | 94 | |
| H_CC-050 | 594.4 | 1.99 | 74 | |
| H_CC-051 | 611.5 | 1.74 | 99 | |
| H_CC-052 | 653.5 | 2.09 | 72 | |
| H_CC-053 | 617.5 | 1.77 | 95 | |
| H_CC-054 | 637.9 | 1.79 | 83 | |
| H_CC-055 | 663.6 | 1.80 | 92 | |
| H_CC-056 | 617.5 | 1.78 | 98 | |
| H_CC-057 | 685.6 | 1.92 | 98 | |
| H_CC-058 | 665.6 | 1.93 | 97 | |
| H_CC-059 | 637.5 | 1.72 | 91 | |
| H_CC-060 | 591.5 | 1.66 | 102 | |
| H_CC-061 | 665.6 | 1.76 | 105 | |
| H_CC-062 | 574.5 | 2.12 | 73 | |
| H_CC-063 | 677.6 | 1.85 | 82 | |
| H_CC-064 | 580.4 | 2.03 | 61 | |
| H_CC-065 | 637.5 | 1.75 | 52 | |
| H_CC-066 | 663.6 | 1.77 | 56 | |
| H_CC-067 | 611.5 | 1.65 | 59 | |
| H_CC-068 | 560.4 | 2.05 | 65 | |
| H_CC-069 | 609.5 | 1.79 | 52 | |
| H_CC-070 | 635.5 | 1.80 | 53 | |
| H_CC-071 | 589.5 | 1.80 | 55 | |
| H_CC-072 | 711.6 | 1.96 | 83 | |
| H_CC-073 | 651.5 | 1.68 | 55 | |
| H_CC-074 | 623.5 | 1.75 | 59 | |
| H_CC-075 | 699.6 | 1.85 | 75 | |
| H_CC-076 | 526.3 | 1.99 | 69 | |
| H_CC-077 | 539.4 | 2.00 | 58 | |
| H_CC-078 | 552.4 | 2.09 | 77 | |
| H_CC-079 | 568.4 | 2.38 | 73 | |
| H_CC-080 | 604.4 | 1.96 | 59 | |
| H_CC-081 | 574.4 | 1.96 | 61 | |
| H_CC-082 | 663.5 | 1.94 | 72 | |
| H_CC-083 | 568.4 | 2.03 | 59 | |
| H_CC-084 | 538.4 | 2.01 | 55 | |
| H_CC-085 | 580.4 | 2.10 | 70 | |
| H_CC-086 | 586.4 | 2.11 | 66 | |
| H_CC-087 | 512.4 | 1.85 | 57 | |
| H_CC-088 | 538.4 | 2.00 | 67 | |
| H_CC-089 | 585.4 | 1.61 | 58 | |
| H_CC-090 | 642.5 | 2.02 | 83 | |
| H_CC-091 | 667.5 | 2.12 | 73 | |
| H_CC-092 | 668.5 | 2.16 | 50 | |
| H_CC-093 | 630.4 | 2.10 | 63 | |

## Patentansprüche

1. Verbindung gemäß der allgemeinen Formel I 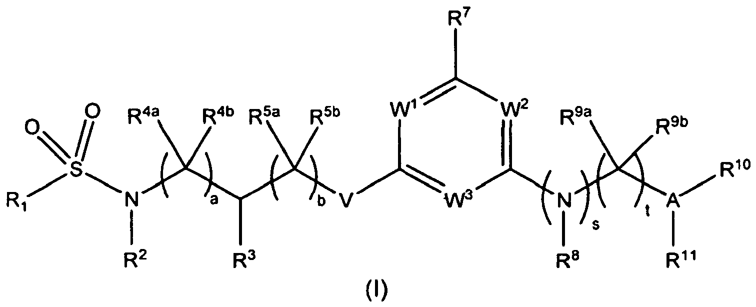 worin
a für 0, 1 oder 2 steht;
b für 0, 1, oder 2 steht;
R¹ für Aryl, Heteroaryl CH(Aryl)₂ oder ein über eine C₁₋₃-Alkylengruppe gebundenes Aryl oder Heteroaryl steht;
R² und R³ wie unter (i) oder (ii) beschrieben definiert sind:
(i) R² für H, C₁₋₆-Alkyl, C₃₋₈-Cycloalkyl, Aryl oder Heteroaryl steht; oder R² ein über eine C₁₋₆-Alkylengruppe, C₂₋₆-Alkenylengruppe oder C₂₋₆-Alkinylengruppe gebundenes C₃₋₈-Cycloalkyl, Aryl oder Heteroaryl bedeutet;
R³ für H, F, Cl, Br, I, -CF₃, -OCF₃, OH, O-C₁₋₆-Alkyl, C₁₋₆-Alkyl, C₃₋₈Cycloalkyl, Aryl, Heteroaryl steht; oder R³ ein über eine C₁₋₆-Alkylengruppe, C₂₋₆-Alkenylengruppe oder C₂₋₆-Alkinylengruppe gebundenes C₃₋₈Cycloalkyl, Aryl oder Heteroaryl bedeutet;
oder
(ii) R² und R³ zusammen mit der sie verbindenden Gruppe -N-(CR^{4a}R^{4b})ₐ-CH-einen Heterocyclus bilden, der an einem oder mehreren seiner Kohlenstoff-Ringgliedern mit einem oder mehreren Resten unabhängig voneinander ausgewählt aus der Gruppe bestehend aus F, Cl, Br, I, -CF₃, =O, -O-CF₃, -OH, -SH, -O-C₁₋₆-Alkyl, C₁₋₆-Alkyl, C₃₋₈Cycloalkyl, Aryl und Heteroaryl substituiert und/oder mit einem Aryl- oder Heteroaryl anelliert sein kann und/oder zwei seiner Kohlenstoffringglieder über eine C1-3-Alkylenbrücke miteinander verbunden sind,
worin der Heterocyclus gesättigt oder wenigstens einfach ungesättigt, nicht aber aromatisch ist, 4-, 5-, 6- oder 7-gliedrig ist, neben dem N-Heteratom, an welches der Rest R² gebunden ist, ein oder mehrere Heteroatome oder Heteroatomgruppen unabhänig voneinander ausgewählt aus der Gruppe bestehend aus N, NR⁵⁰, O, S, S=O oder S(=O)₂ enthalten kann; wobei der Rest R⁵⁰ H, C₁₋₆-Alkyl, -C(=O)-R⁵¹, C₃₋₈-Cycloalkyl, Aryl, Heteroaryl oder ein über eine C₁₋₃-Alkylengruppe gebundenes C₃₋₈-Cycloalkyl, Aryl oder Heteroaryl bedeutet, und R⁵¹ C₁₋₆-Alkyl, C₃₋₈-Cycloalkyl, Aryl, Heteroaryl oder ein über eine C₁₋₃-Alkylengruppe gebundenes C₃₋₈-Cycloalkyl, Aryl oder Heteroaryl bedeutet;
V für C(R^{6a})(R^{6b}), NR^{6c}, O oder eine Einfachbindung steht,
wobei R^{6c} für einen Rest aus der Gruppe H, C₁₋₆-Alkyl, C₃₋₈-Cycloalkyl, Aryl, Heteroaryl oder für ein über eine C₁₋₆-Alkylengruppe, C₂₋₆-Alkenylengruppe oder C₂₋₆-Alkinylengruppe gebundenes C₃₋₈Cycloalkyl, Aryl oder Heteroaryl steht,
R^{4a}, R^{4b}, R^{5a}, R^{5b}, R^{6a} R^{6b}, unabhängig voneinander, für H, F, Cl, Br, I, -CF₃, -OCF₃, OH, SH, O-C₁₋₆-Alkyl, C₁₋₆-Alkyl, C₃₋₈Cycloalkyl, Aryl, oder Heteroaryl stehen; oder für ein über eine C₁₋₆-Alkylengruppe oder C₂₋₆-Alkenylengruppe gebundenes C₃₋₈Cycloalkyl, Aryl oder Heteroaryl stehen; und R^{6a} und R^{6b} zusätzlich zusammen =O bedeuten können;
und/oder
R^{4a} und R^{4b} gemeinsam mit dem sie verbindenden C-Atom einen gesättigten Cyclus bilden, der unsubstituiert oder an einem oder mehreren, beispielsweise 1, 2, 3 oder 4, seiner Kohlenstoff-Ringglieder mit einem oder mehreren, beispielsweise 1, 2, 3 oder 4, Substituenten unabhängig voneinander ausgewählt aus der Gruppe bestehend aus F, CF₃, C₁₋₆-Alkyl, O-C₁₋₆-Alkyl, OH, OCF₃, Aryl und Heteroaryl substituiert ist, wobei der Cyclus 3,- 4-, 5- oder 6-gliedrig ist, und ggf. ein oder mehr, beispielsweise 1 oder 2, Sauerstoffatome enthalten kann;
R⁷ für einen Substituenten aus der Gruppe H, C₁₋₆-Alkyl, -CN, -CF₃, OH, C₁₋₆-Alkoxy, -O-CF₃ steht;
W¹, W² und W³ unabhängig voneinander für N oder CR⁶⁰ stehen, mit der Maßgabe, dass von W¹, W² und W³ mindestens zwei N darstellen, und R⁶⁰ für H, C₁₋₆-Alkyl, Halogen, -CN, CF₃, OH, C₁₋₆-Alkoxy oder -O-CF₃ steht;
s = 0 oder 1 ist,
t = 0, 1, 2 oder 3 ist,
R⁸ für H; C₁₋₆-Alkyl; C₃₋₈-Cycloalkyl; Aryl oder Heteroaryl; über eine C₁₋₆-Alkylengruppe gebundenes C₃₋₈-Cycloalkyl, Aryl oder Heteroaryl, steht;
R^{9a} und R^{9b} jeweils unabhängig voneinander H; F; Cl; OH; C₁₋₆-Alkyl; O-C₁₋₆-Alkyl; C₃₋₈-Cycloalkyl; Aryl oder Heteroaryl; über eine C₁₋₆-Alkylengruppe gebundenes C₃₋₈-Cycloalkyl, Aryl oder Heteroaryl, bedeuten;
A für N oder CH steht,
mit der Maßgabe, dass wenn s für 1 und t für 0 steht, A für CH steht; und
mit der Maßgabe, dass wenn s und t jeweils für 0 stehen, A für N steht;
die Reste R¹⁰ und R¹¹ unter Einschluss von A eine spirocyclische oder cyclische Gruppe gemäß einer der allgemeinen Formeln II oder III darstellen, 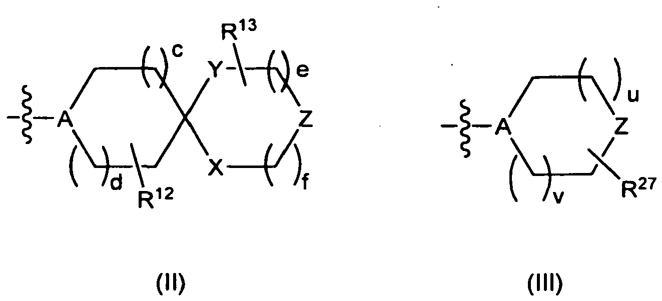 wobei
c, d, e, f, u und v jeweils unabhängig voneinander 0, 1 oder 2 bedeuten;
R¹², R¹³ und R²⁷ jeweils unabhängig voneinander für 0 bis 4 Substituenten stehen, die jeweils unabhängig voneinander ausgewählt sind aus F; Cl; OH; =O; C₁₋₆-Alkyl; O-C₁₋₆-Alkyl; C₃₋₈-Cycloalkyl; Aryl oder Heteroaryl; über eine C₁₋₆-Alkylengruppe gebundenes C₃₋₈-Cycloalkyl, Aryl oder Heteroaryl;
und/oder jeweils zwei der 0-4 Substituenten R²⁷ zusammen eine C₁₋₃-Alkylenbrücke darstellen, so dass der in der allgemeinen Formel III dargestellte Cyclus eine bicyclisch verbrückte Form annimmt;
und/oder zwei benachbarte der 0-4 Substituenten R¹³ ein anelliertes Aryl oder Heteroaryl bilden;
und/oder zwei benachbarte der 0-4 Substituenten R²⁷ ein anelliertes Aryl oder Heteroaryl bilden;
X für CR^{14a}R^{14b}, NR¹⁵ oder O steht;
Y für CR^{16a}R^{16b}, NR¹⁷ oder O steht;
mit der Maßgabe, dass X nicht NR¹⁵ bedeutet, wenn Y NR¹⁷ bedeutet; und
mit der Maßgabe, dass X und Y nicht gleichzeitig O bedeuten;
wobei
R^{14a}, R^{14b}, R^{16a} und R^{16b} jeweils unabhängig voneinander H; F; Cl; OH; C₁₋₆-Alkyl; O-C₁₋₆-Alkyl; C₃₋₈-Cycloalkyl; Aryl oder Heteroaryl; über eine C₁₋₆-Alkylengruppe gebundenes C₃₋₈-Cycloalkyl, Aryl und Heteroaryl; bedeuten
und/oder jeweils R^{14a} und R^{14b} gemeinsam für =O und/oder jeweils R^{16a} und R^{16b} gemeinsam für =O stehen können;
R¹⁵ und R¹⁷ jeweils unabhängig voneinander für H; C₁₋₆-Alkyl; C₃₋₈-Cycloalkyl, Aryl oder Heteroaryl; über eine C₁₋₆-Alkylengruppe gebundenes C₃₋₈-Cycloalkyl, Aryl oder Heteroaryl stehen;
Z in der allgemeinen Formel II für CR^{18a}R^{18b}, NR¹⁹ oder O steht;
oder
Z in der allgemeinen Formel II, in dem Fall, dass X für O und f für 0 steht, - (C(R¹²⁴)-C(R¹²⁵))-bedeutet, wobei
R¹²⁴ und R¹²⁵ gemeinsam mit den sie verbindenden Kohlenstoffatomen ein ankondensiertes Aryl oder Heteroaryl bilden; oder
Z in der allgemeinen Formel II, in dem Fall dass X für O und f für 0 steht, =(N(CR¹²⁶))- bedeutet, wobei das N-Atom einfach an das O-Atom gebunden ist, und
R¹²⁶ für H; C₁₋₆Alkyl; C₃₋₈Cycloalkyl; Aryl oder Heteroaryl; über eine C₁₋₆-Alkylengruppe gebundenes C₃₋₈Cycloalkyl, Aryl oder Heteroaryl steht;
Z in der allgemeinen Formel III für CR^{18a}R^{18b}, NR¹⁹, O, S, S(=O) oder S(=O)₂ steht;
wobei
R^{18a} für H; C₁₋₆-Alkyl; C₃₋₈-Cycloalkyl; Aryl oder Heteroaryl; über eine C₁₋₆-Alkylengruppe gebundenes C₃₋₈-Cycloalkyl, Aryl oder Heteroaryl, steht;
oder R^{18a} für eine Gruppe gemäß der allgemeinen Formel IV steht, 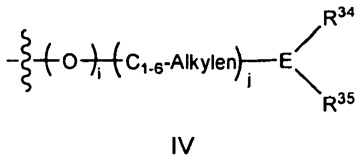 worin
i und j jeweils unabhängig voneinander für 0 oder 1 stehen;
E für N oder CH steht, mit der Maßgabe, dass wenn i für 1 und j für 0 steht, E für CH steht
R³⁴ und R³⁵ jeweils unabhängig voneinander H; C₁₋₆-Alkyl; C₃₋₈Cycloalkyl; Aryl oder Heteroaryl; über eine C₁₋₃-Alkylengruppe gebundenes Aryl, Heteroaryl oder C₃₋₈Cycloalkyl bedeuten;
oder R³⁴ und R³⁵ unter Einschluss von E ein5- oder 6-gliedriges Aryl oder Heteroaryl bilden;
oder R³⁴ und R³⁵ unter Einschluss von E einen gesättigten Heterocyclus gemäß der allgemeinen Formel V bilden, 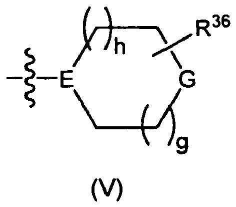 wobei
h und g unabhängig voneinander 0, 1 oder 2 bedeuten;
G für CR^{37a}R^{37b}, NR³⁸, O, S, S=O oder S(=O)₂ steht, mit der Maßgabe, dass wenn E für CH steht, G nicht für CR^{37a}R^{37b} steht;
R³⁶ für 0 bis 4 Substituenten steht, die jeweils unabhängig voneinander ausgewählt sind aus F; Cl; Br; I; OH; SH; =O; O-C₁₋₆-Alkyl; C₁₋₆-Alkyl; C₃₋₈-Cycloalkyl; Aryl oder Heteroaryl; über eine C₁₋₆-Alkylengruppe gebundenes C₃₋₈-Cycloalkyl, Aryl oder Heteroaryl;
und/oder zwei benachbarte Substituenten R³⁶ zusammen ein ankondensiertes Aryl oder Heteroaryl darstellen;
R^{37a} und R^{37b} jeweils unabhängig voneinander H; F; Cl; Br; I; OH; SH; =O; O-C₁₋₆-Alkyl; C₁₋₆-Alkyl; C₃₋₈-Cycloalkyl; Aryl oder Heteroaryl; über eine C₁₋₆-Alkylengruppe gebundenes C₃₋₈-Cycloalkyl, Aryl und Heteroaryl bedeuten;
R³⁸ für H; C₁₋₆Alkyl; C₃₋₈Cycloalkyl; Aryl oder Heteroaryl; über eine C₁₋₃-Alkylengruppe gebundenes Aryl, Heteroaryl oder C₃₋₈-Cycloalkyl steht;
wobei
R^{18b} für H; OH; C₁₋₆-Alkyl; C₃₋₈-Cycloalkyl; O- C₁₋₆-Alkyl; O-(C₃₋₈-Cycloalkyl); (C₁₋₆-Alkylen)-O-C₁₋₆Alkyl; (C₁₋₆Alkylen)-O-(C₃₋₈-Cycloalkyl); Aryl oder Heteroaryl; O-Aryl oder O-Heteroaryl; über C₁₋₆-Alkylen gebundenes Aryl, O-Aryl, Heteroaryl oder O-Heteroaryl steht;
oder R^{18b} für eine Gruppe gemäß der allgemeinen Formel VI steht, 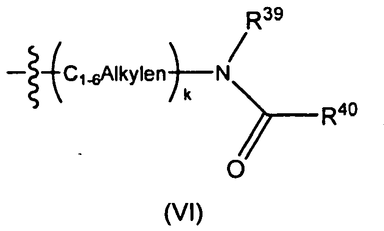 worin
k für 0 oder 1 steht;
R³⁹ für H; C₁₋₆-Alkyl; C₃₋₈-Cycloalkyl; Aryl oder Heteroaryl; über eine C₁₋₃-Alkylengruppe gebundenes C₃₋₈-Cycloalkyl, Aryl oder Heteroaryl; steht;
R⁴⁰ für C₁₋₆-Alkyl; C₃₋₈-Cycloalkyl; Aryl oder Heteroaryl; über eine C₁₋₆-Alkylengruppe gebundenes C₃₋₈-Cycloalkyl, Aryl oder Heteroaryl; steht;
oder
R³⁹ und R⁴⁰ gemeinsam mit der sie verbindenden N-C(=O)-Gruppe einen Ring gemäß der allgemeinen Formel VII bilden, 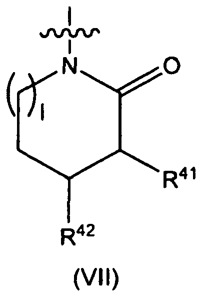 worin
I für 0, 1 oder 2 steht
und R⁴¹ und R⁴² gemeinsam mit den sie verbindenden Kohlenstoffatomen ein anelliertes Aryl oder Heteroaryl bilden;
wobei R¹⁹ für H; oder (P)_{z}-R²² steht,
wobei
z für 0 oder 1 steht;
P für (C=O), S(=O)₂ oder C(=O)-N(R²⁴) steht; wobei das N-Atom in der Gruppe C(=O)-N(R²⁴) mit R²² verknüpft ist,wobei
R²⁴ für H; C₁₋₆-Alkyl; C₃₋₈Cycloalkyl; über eine C₁₋₃-Alkylengruppe gebundenes Aryl, Heteroaryl oder C₃₋₈Cycloalkyl steht;
R²² für C₁₋₆-Alkyl; Aryl oder Heteroaryl; über eine C₁₋₆-Alkylengruppe gebundenes Aryl oder Heteroaryl steht; oder
R²² für eine Gruppe gemäß der allgemeinen Formel VIII steht, worin 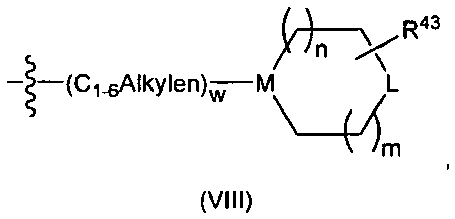
n für 0, 1 oder 2 steht;
m für 0, 1 oder 2 steht;
w für 0 oder 1 steht,
M für CH oder N steht;
mit der Maßgabe, dass wenn P für C(=O)-NR²⁴ und w für 0 steht, M für CH steht; und
mit der Maßgabe, dass wenn z und w gleichzeitig für 0 stehen, M für CH steht;
L für CR^{44a}R^{44b}, NR⁴⁵, O, S, S=O oder S(=O)₂ steht;
R⁴³ für 0 bis 4 Substituenten steht, die jeweils unabhängig voneinander ausgewählt sind aus F; Cl; OH; =O; C₁₋₆-Alkyl; O-C₁₋₆-Alkyl; C₃₋₈-Cycloalkyl;
Aryl oder Heteroaryl; über eine C₁₋₆-Alkylengruppe gebundenes C₃₋₈-Cycloalkyl, Aryl oder Heteroaryl;
und/oder zwei benachbarte der 0-4 Reste R⁴³ zusammen ein anelliertes Aryl oder Heteroaryl darstellen;
R^{44a} und R^{44b} jeweils unabhängig voneinander für H; F; Cl; Br; I; OH; C₁-₆-Alkyl; O-C₁₋₆-Alkyl; C₃₋₈-Cycloalkyl; Aryl oder Heteroaryl; über eine C₁-₆-Alkylengruppe gebundenes C₃₋₈-Cycloalkyl, Aryl oder Heteroaryl stehen;
oder R^{44a} und R^{44b} gemeinsam für =O stehen können;
R⁴⁵ für H; C₁₋₆Alkyl; C₃₋₈Cycloalkyl; Aryl oder Heteroaryl; über eine C₁₋₃-Alkylengruppe gebundenes Aryl, Heteroaryl oder C₃₋₈-Cycloalkyl steht;
wobei die oben genannten Reste C₁₋₆-Alkyl, C₁₋₃-Alkylen, C₁₋₆-Alkylen, C₂₋₆-Alkenylen, C₂₋₆-Alkinylen, C₃₋₆-Cycloalkyl, C₃₋₈-Cycloalkyl, Aryl und Heteroaryl jeweils unsubstituiert oder einfach oder mehrfach mit gleichen oder verschiedenen Resten substituiert sein können; die oben angegebenen Reste C₁₋₆-Alkyl, C₁₋₃-Alkylen, C₁₋₆-Alkylen, C₂₋₆-Alkenylen und C₂₋₆-Alkinylen, jeweils verzweigt oder unverzweigt sein können;
in Form eines einzelnen Enantiomers oder eines einzelnen Diastereomers, des Razemats, der Enantiomere, der Diastereomere, Mischungen der Enantiomere und/oder Diastereomere, sowie jeweils in Form ihrer Basen und/ oder physiologisch verträglichen Salze.

2. Verbindung gemäß Anspruch 1,worin
W¹ und W³ für N stehen und W² für CR⁶⁰ steht; oder
W¹ und W² für N stehen und W³ für CR⁶⁰ steht, oder
W¹, W² und W³ für N stehen.

3. Verbindung gemäß Anspruch 1 oder 2, worin
V für O steht.

4. Verbindung gemäß einem der Ansprüche 1 bis 3, worin
R¹ für Phenyl, Naphthyl, Indolyl, Benzofuranyl, Benzothiophenyl (Benzothienyl); Benzooxazolyl, Benzooxadiazolyl, Pyrrolyl, Furanyl, Thienyl, Pyridinyl, Pyridazinyl, Pyrimidinyl, Pyrazinyl, Imidazothiazolyl, Carbazolyl, Dibenzofuranyl, Dibenzothiophenyl, (Dibenzothienyl) oder CH(Phenyl)₂, bevorzugt für Phenyl, Naphthyl, Benzothiophenyl, Benzooxadiazolyl, Thiophenyl, Pyridinyl, Imidazothiazolyl oder Dibenzofuranyl, besonders bevorzugt für Phenyl oder Naphthyl steht, jeweils unsubstituiert oder einfach oder mehrfach, gleich oder verschieden, substituiert, wobei die Substituenten vorzugweise ausgewählt sind aus -O-C₁-₃-Alkyl, C₁₋₆-Alkyl, F, Cl, Br, I, CF₃, OCF₃, OH, SH, Phenyl, Naphthyl, Furyl, Thiazolyl, Thienyl und Pyridinyl.

5. Verbindung gemäß einem der Ansprüche 1 bis 4, wobei in der allgemeinen Formel I die Teilstruktur (Ac I): 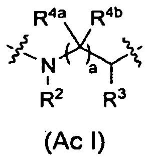 für 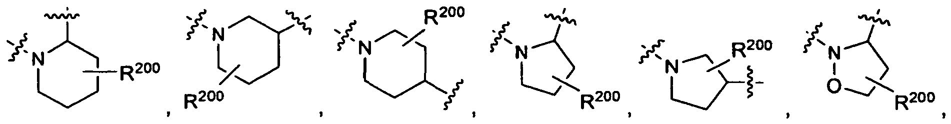 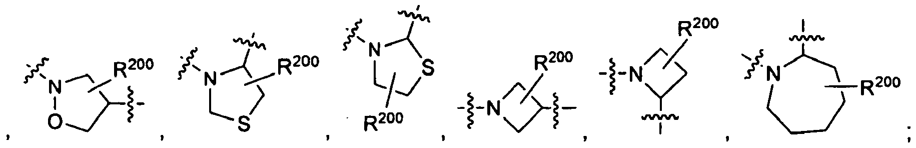 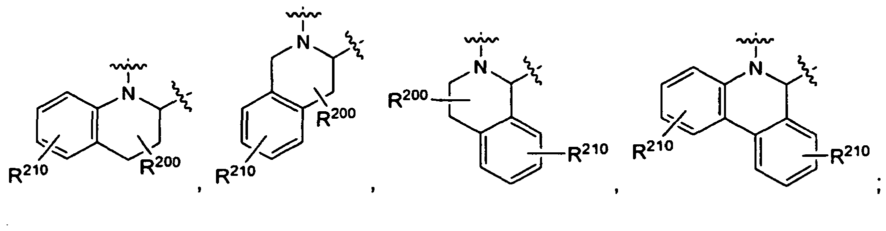 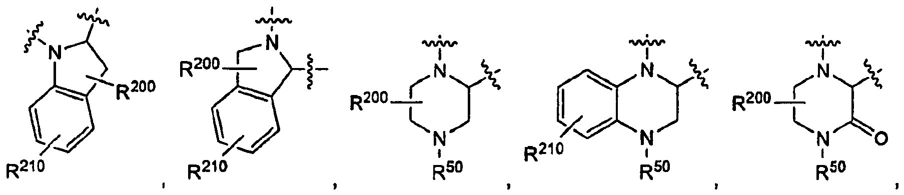 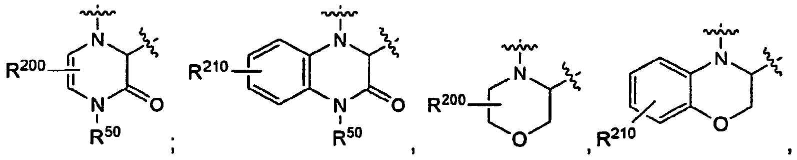 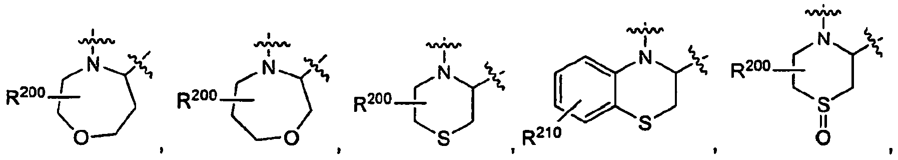 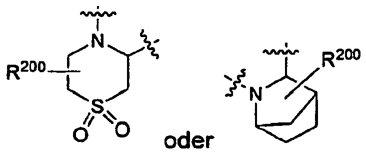 steht,
worin
R²⁰⁰ für 0-4 Substituenten steht, die unabhängig voneinander ausgesucht sind aus aus F, Cl, -CF₃, =O, -O-CF₃, -OH, -O-C₁₋₆-Alkyl oder C₁₋₆-Alkyl, vorzugsweise für F oder CF₃ steht oder zwei der Reste R²⁰⁰ ein anelliertes Aryl, insbesondere eine Benzogruppe, darstellen;
R²¹⁰ für 0-4 Substituenten steht, die unabhängig voneinander ausgesucht sind aus -O-C₁₋₃-Alkyl, C₁₋₆-Alkyl, F; Cl, Br, I, CF₃, OCF₃, OH, SH, Phenyl, Naphthyl, Furyl, Thienyl und Pyridinyl, vorzugsweise Methyl, Methoxy, O-CF₃, CF₃, F, Cl und Br.

6. Verbindung gemäß einem der Ansprüche 1 bis 5, wobei
R² für H, C₁₋₆-Alkyl, C₃₋₆-Cycloalkyl, Aryl oder ein über eine C₁₋₃-Alkylengruppe gebundenes C₃₋₆-Cycloalkyl oder Aryl, insbesondere für H, Methyl, Ethyl, n-Propyl, i-Propyl, n-Butyl, i-Butyl, t-Butyl, Cyclopropyl, Phenyl, Pyridinyl oder ein über eine C₁₋₃-Alkylengruppe gebundenes Phenyl oder Pyridinyl steht; jeweils unsubstituiert oder einfach oder mehrfach substituiert mit gleichen oder verschiedenen Substituenten;
und
R³ für H, F, Cl, -CF₃, -OH, -O-C₁₋₆-Alkyl, C₁₋₆-Alkyl oder Aryl steht; oder ein über eine C₁₋₃-Alkylengruppe gebundenes Aryl bedeutet, jeweils unsubstituiert oder einfach oder mehrfach mit gleichen oder verschiedenen Resten substituiert.

7. Verbindung gemäß einem der Ansprüche 1 bis 6, wobei
a+b=1 1 gilt.

8. Substituierte Verbindungen gemäß einem oder mehreren der Ansprüche 1 bis 7,
(a1) die allgemeine Formel II die folgende Teilstruktur IIa annimmt: 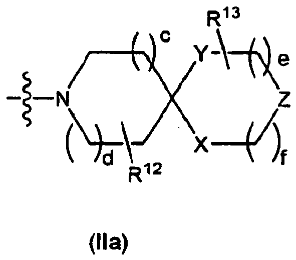 oder
(a2) die allgemeine Formel III eine der folgenden Teilstrukturen IIIa oder IIIb annimmt:

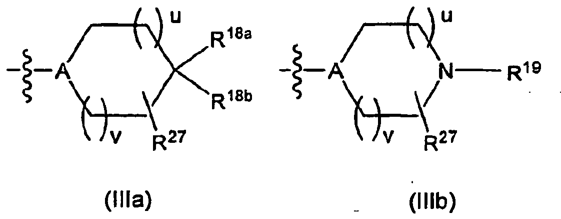

9. Substituierte Verbindungen gemäß Anspruch 8, worin
(a1) die Teilstruktur der Formel IIa die folgende Teilstruktur IIb annimmt: 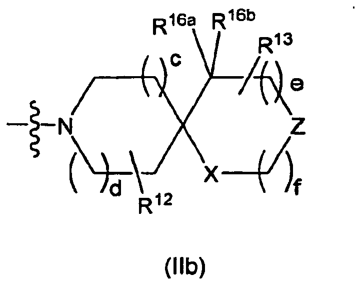 oder
(a2) die Teilstrukturen der Formeln IIIa und IIIb eine der folgenden Teilstrukturen IIIc, IIId oder IIIe annehmen:

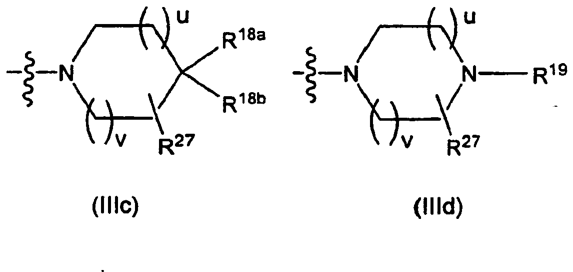

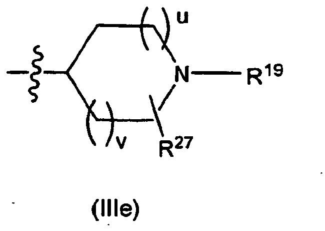

10. Substituierte Verbindungen gemäß Anspruch 9, worin
(a1) die Teilstruktur der Formel IIa die Teilstruktur IIb annimmt,
R⁸ für H, C₁₋₆-Alkyl, C₃₋₆-Cycloalkyl, jeweils unsubstituiert oder einfach oder mehrfach substituiert mit gleichen oder verschiedenen Substituenten, steht und
R^{9a} und R^{9b} jeweils für H stehen;
und/oder
(a2) die Teilstrukturen der Formeln IIIa und IIIb eine der Teilstrukturen IIIc oder IIId annehmen,und s und t jeweils für 0 stehen;
und/oder
(a3) die Teilstrukturen der Formeln IIIa und IIIb eine der Teilstrukturen IIIc oder IIId annehmen und zwei der Substituenten R²⁷ zusammen eine C₁₋₃-Alkylenbrücke darstellen, so dass der in der Teilstruktur IIIc oder IIId dargestellte-Cyclus eine bicyclisch verbrückte Form annimmt, und
s und t jeweils = 0 sind;
und/oder
(a4) die Teilstrukturen der Formeln IIIa und IIIb eine der Teilstrukturen IIIc oder IIIe annehmen, s für 1 und t für 1, 2 oder 3 stehen und R⁸ für H, C₁₋₆-Alkyl oder C₃₋₆-Cycloalkyl jeweils unsubstituiert oder einfach oder mehrfach substituiert mit gleichen oder verschiedenen Substituenten, steht;

11. Substituierte Verbindungen nach Anspruch 10, worin
(a1) Teilstruktur IIb die folgende Teilstruktur IIc annimmt: 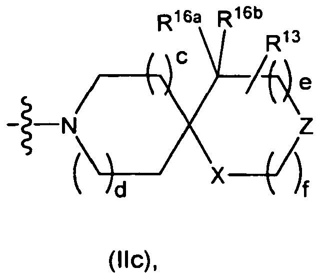 und worin s und t jeweils 0 bedeuten;
und/oder
(a2) die Teilstrukturen IIIc oder IIId eine der folgenden Teilstrukturen IIIf oder IIIg annehmen, 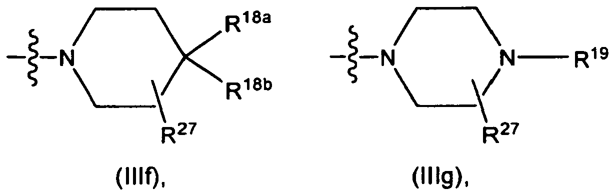 worin
R²⁷ für H oder Methyl steht und/oder zwei benachbarte Substituenten R²⁷ ein anelliertes Aryl oder Heteroaryl, insbesondere eine Benzogruppe, bilden;
und/oder
(a3) in den Verbindungendie Teilstrukturen IIIc oder IIId einen der folgenden Reste Reste A bis H darstellen, 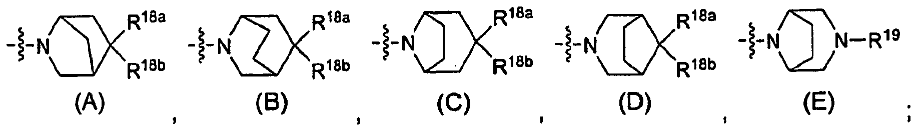 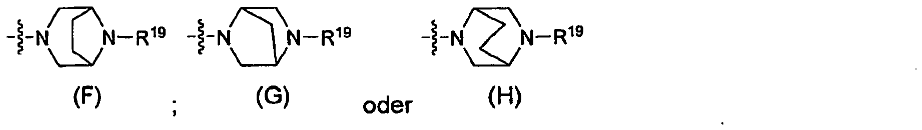 und/oder
(a4) in den Verbindungen die Teilstrukturen IIIc oder IIIe eine Gruppe gemäß einer der Formeln IIIh oder IIIi darstellen, 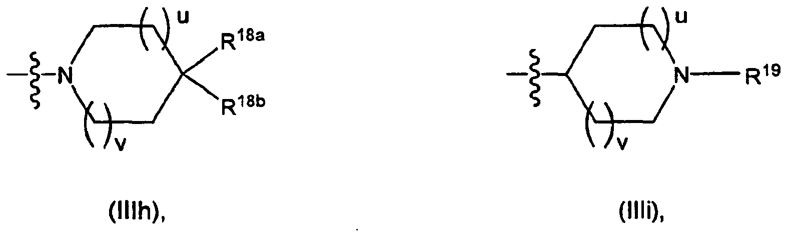 und R^{9a} und R^{9b} jeweils für H stehen;

12. Substituierte Verbindungen nach Anspruch 11, worin
(a1) in der Teilstruktur IIc die Reste R^{16a} und R^{16b} jeweils für H stehen oder zusammen =O bilden;
R¹³ für Aryl oder Heteroaryl, steht und/oder zwei der Substituenten R¹³ zusammen =O bilden
und/oder zwei benachbarte Substituenten R¹³ ein anelliertes Aryl oder Heteroaryl, insbesondere eine Benzogruppe, bilden,
und/oder
(a2) in den Teilstrukturen IIIf oder IIIg
R^{18a} für H; C₁₋₆-Alkyl; C₃₋₈-Cycloalkyl, -NH(C₁₋₆Alkyl), -N(C₁₋₆Alkyl)₂, Phenyl, Pyridyl, Pyrimidinyl, Thiazolyl, Imidazolyl, Triazolyl oder Thienyl, jeweils unsubstituiert oder einfach oder mehrfach substituiert; über eine -(O)₀₋₁-C₁₋₆-Alkylengruppe gebundenes Phenyl, Pyridyl, Pyrimidinyl, Imidazolyl, Triazolyl oder Thienyl, jeweils unsubstituiert oder einfach oder mehrfach substituiert, steht;
oder
R^{18a} für den Rest gemäß der allgemeinen Formel VIIa 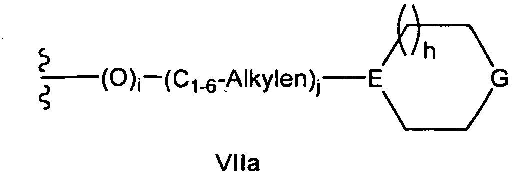 steht, worin
i für 0 oder 1 steht;
j für 0 oder 1 steht;
h für 0 oder 1 steht;
E für N oder CH steht; mit der Maßgabe, dass wenn i für 1 und j für 0 steht, E für CH steht;
G für CR^{37a}R^{37b} oder NR³⁸ steht;
wobei R^{37a} und R^{37b} unabhängig voneinander für H; F, oder C₁₋₆-Alkyl, jeweils unsubstituiert oder einfach oder mehrfach substituiert mit gleichen oder verschiedenen Substituenten, stehen;
R³⁸ für H; C₁₋₆-Alkyl, C₃₋₆-Cycloalkyl oder Pyridyl steht;
R^{18b} für H; OH; C₁₋₆-Alkyl; Phenyl, Pyridyl, Pyrimidinyl, Imidazolyl, Triazolyl, Thienyl oder Thiazolyl; jeweils unsubstituiert oder einfach oder mehrfach substituiert mit gleichen oder verschiedenen Substituenten; über eine C₁₋₆-Alkylengruppe gebundenes Phenyl, Pyridyl, Pyrimidinyl, O-Phenyl, O-Pyridyl, Imidazolyl, Triazolyl, Thienyl oder Thiazolyl, jeweils unsubstituiert oder einfach oder mehrfach substituiert mit gleichen oder verschiedenen Substituenten; über C₁₋₆-Alkylen-NH(C=O) verbrücktes Phenyl, Pyridyl, Pyrimidinyl, Imidazolyl, Triazolyl, Thiazolyl oder Thienyl, jeweils unsubstituiert oder einfach oder mehrfach substituiert mit gleichen oder verschiedenen Substituenten, steht
R¹⁹ für H; C₁₋₆-Alkyl; C₃₋₈-Cycloalkyl, oder für über (C=O)₀₋₁ verbundenes C₁₋₆**-**Alkyl; Phenyl, Pyridyl, Thienyl, Thiazolyl, Triazolyl, Pyrimidinyl oder Imidazolyl; jeweils unsubstituiert oder einfach oder mehrfach substituiert mit gleichen oder verschiedenen Substituenten; über eine C₁₋₆-Alkylengruppe gebundenes Phenyl, Pyridyl, Thienyl, Thiazolyl, Pyrimidinyl, Triazolyl oder Imidazolyl; jeweils unsubstituiert oder einfach oder mehrfach substituiert mit gleichen oder verschiedenen Substituenten, steht;
oder für den Rest gemäß der allgemeinen Formel VIIIa 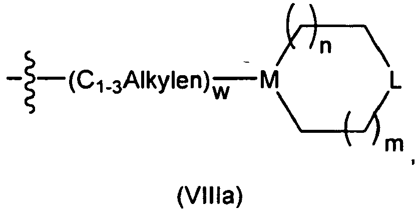 steht, worin
w für 0 oder 1 steht;
n für 0 oder 1 steht;
m für 0 oder 1 steht;
M für CH oder N steht, mit der Maßgabe, dass wenn w für 0 steht, M für CH steht;
L für CR^{44a}R^{44b} oder NR⁴⁵ steht;
wobei R^{44a} und R^{44b} unabhängig voneinander für H; F oder C₁₋₆-Alkyl, jeweils unsubstituiert oder einfach oder mehrfach substituiert mit gleichen oder verschiedenen Substituenten, stehen;
R⁴⁵ für H; C₁₋₆-Alkyl, C₃₋₆-Cycloalkyl oder Pyridyl steht;
und/oder
(a3) in den Verbindungen die Teilstrukturen IIIc oder IIId eine der folgenden Gruppen A bis H darstellen, 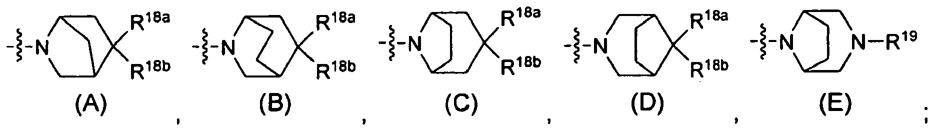 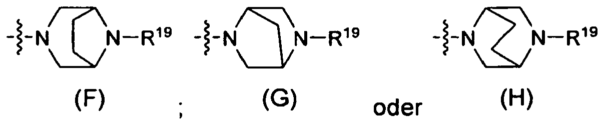 und worin
R^{18a} für H; C₁₋₆-Alkyl; C₃₋₈-Cycloalkyl, N(C₁₋₆-Alkyl)₂; NH(C₁₋₆-Alkyl); Azetidinyl; Pyrrolidinyl, Piperidinyl, 4-(C₁₋₆Alkyl)-Piperazinyl; Phenyl, Pyridyl, Pyrimidinyl, Imidazolyl, Triazolyl, Thiazolyl oder Thienyl, jeweils unsubstituiert oder einfach oder mehrfach substituiert mit gleichen oder verschiedenen Substituenten; über eine -(O)₀-₁-C₁₋₆-Alkylengruppe gebundenes N(C₁-₆-Alkyl)₂; NH(C₁-₆-Alkyl); Azetidinyl; Pyrrolidinyl, Piperidinyl, 4-(C₁₋₆Alkyl)-Piperazinyl; Phenyl, Imidazolyl, Triazolyl, Thienyl, Thiazolyl, Pyrimidinyl oder Pyridyl, jeweils unsubstituiert oder einfach oder mehrfach substituiert mit gleichen oder verschiedenen Substituenten, steht;
R^{18b} für H; OH; C₁-₆-Alkyl; Phenyl, Pyridyl, Pyrimidinyl, Imidazolyl, Triazolyl, Thiazolyl oder Thienyl jeweils unsubstituiert oder einfach oder mehrfach substituiert mit gleichen oder verschiedenen Substituenten; über eine C₁₋₆-Alkylengruppe gebundenes Phenyl, Pyridyl, Pyrimidinyl, Imidazolyl, Triazolyl, Thiazolyl oder Thienyl jeweils unsubstituiert oder einfach oder mehrfach substituiert mit gleichen oder verschiedenen Substituenten, steht;
R¹⁹ für H; C₁₋₆-Alkyl; C₃₋₈-Cycloalkyl, Phenyl, Pyridyl, Pyrimidinyl, Thienyl, Imidazolyl, Thiazolyl, oder Triazolyl, jeweils unsubstituiert oder einfach oder mehrfach substituiert mit gleichen oder verschiedenen Substituenten; über eine C₁₋₆-Alkylengruppe oder eine (C=O)-Gruppe gebundenes Phenyl, Pyridyl, Pyrimidinyl, Thienly, Imidazolyl, Thiazolyl, oder Triazolyl, jeweils unsubstituiert oder einfach oder mehrfach substituiert mit gleichen oder verschiedenen Substituenten; steht; und/oder
(a4) in den Teilstrukturen IIIh oder IIIi
R^{18a} für H; C₁₋₆-Alkyl; C₃₋₈-Cycloalkyl, N(C₁₋₆-Alkyl)₂; NH(C₁₋₆-Alkyl), Azetidinyl; Pyrrolidinyl, Piperidinyl, 4-(C₁₋₆Alkyl)-Piperazinyl; Phenyl, Pyridyl, Pyrimidinyl, Thienyl, Imidazolyl, Thiazolyl, oder Triazolyl, jeweils unsubstituiert oder einfach oder mehrfach substituiert mit gleichen oder verschiedenen Substituenten; über eine - (O)_{0/1}-C₁₋₆-Alkylengruppe gebundenes N(C₁₋₆-Alkyl)₂; NH(C₁₋₆-Alkyl), Azetidinyl; Pyrrolidinyl, Piperidinyl, 4-(C₁₋₆Alkyl)-Piperazinyl; Phenyl, Pyridyl, Pyrimidinyl, Thienyl, Imidazolyl, Thiazolyl, oder Triazolyl, jeweils unsubstituiert oder einfach oder mehrfach substituiert mit gleichen oder verschiedenen Substituenten, steht;
R^{18b} für H; OH; C₁₋₆-Alkyl; Phenyl, Pyridyl, Pyrimidinyl, Thienyl, Imidazolyl, Thiazolyl, oder Triazolyl, jeweils unsubstituiert oder einfach oder mehrfach substituiert mit gleichen oder verschiedenen Substituenten; über eine C₁₋₆-Alkylengruppe gebundenes Phenyl, Pyridyl, Pyrimidinyl, Thienyl, Imidazolyl, Thiazolyl, oder Triazolyl, jeweils unsubstituiert oder einfach oder mehrfach substituiert mit gleichen oder verschiedenen Substituenten, steht;
R¹⁹ für H; C₁₋₆-Alkyl; C₃₋₈-Cycloalkyl, Phenyl, Pyridyl, Pyrimidinyl, Thienyl, Imidazolyl, Thiazolyl, oder Triazolyl , jeweils unsubstituiert oder einfach oder mehrfach substituiert mit gleichen oder verschiedenen Substituenten;über eine C₁₋₆-Alkylengruppe oder (C=O)-Gruppe gebundenes Phenyl, Pyridyl, Pyrimidinyl, Thienyl, Imidazolyl, Thiazolyl, oder Triazolyl, jeweils unsubstituiert oder einfach oder mehrfach substituiert mit gleichen oder verschiedenen Substituenten , steht.

13. Substituierte Verbindungen nach Anspruch 12, worin
(a1) die Teilstruktur der Formel IIc die folgenden Teilstrukturen SP1 bis SP34 annehmen kann: 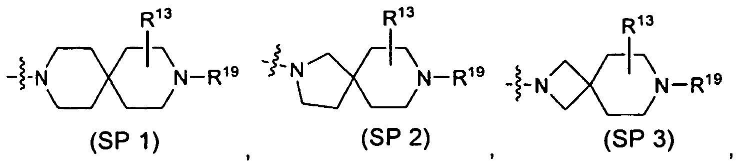 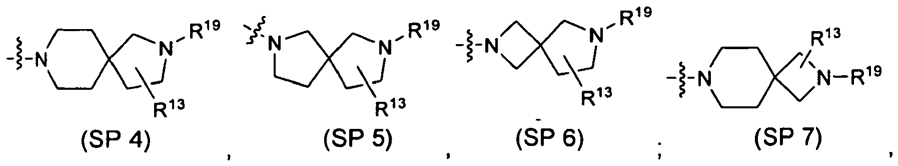 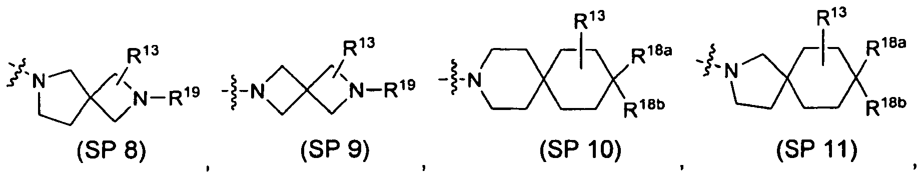 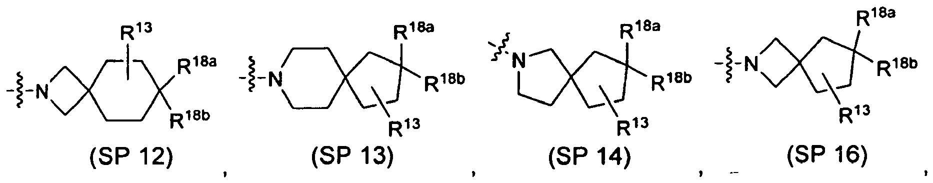 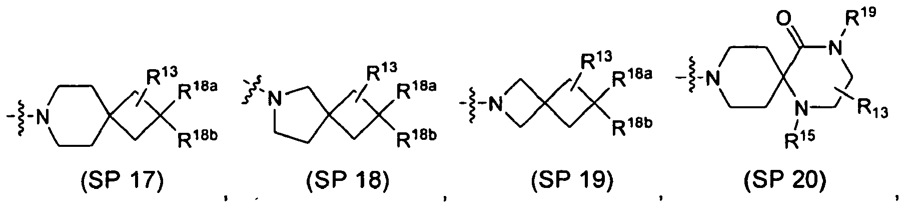 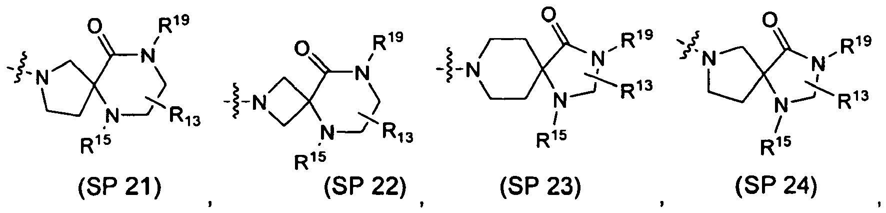 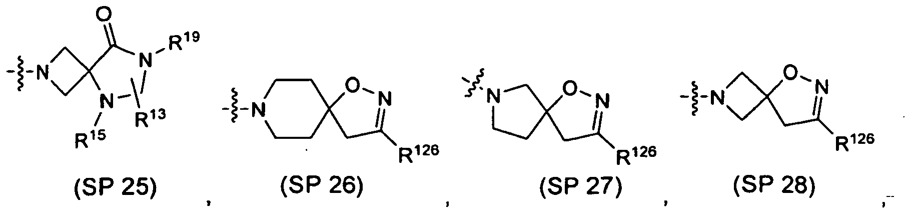 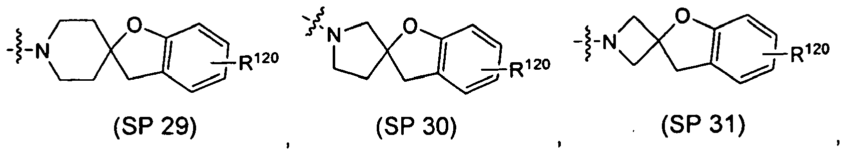 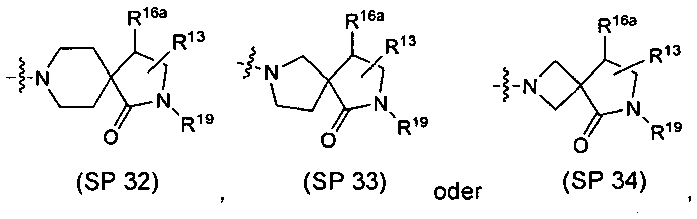 wobei
R¹³ für H oder Phenyl, jeweils unsubstituiert oder einfach oder mehrfach substituiert mit gleichen oder verschiedenen Substituenten, steht; und/oder zwei der Substituenten R¹³ zusammen =O bilden
und/oder zwei benachbarte Substituenten R¹³ ein anelliertes Aryl oder Heteroaryl, insbesondere eine Benzogruppe, bilden,
R¹⁵ H; C₁₋₆-Alkyl; C₃₋₈-Cycloalkyl, Phenyl, Pyridyl, Pyrimidinyl, Imidazolyl, Triazolyl, Thiazolyl oder Thienyl, jeweils unsubstituiert oder einfach oder mehrfach substituiert mit gleichen oder verschiedenen Substituenten; über eine C₁₋₆-Alkylengruppe gebundenes Phenyl, Pyridyl, Pyrimidinyl, Imidazolyl, Triazolyl, Thiazolyl oder Thienyl, jeweils unsubstituiert oder einfach oder mehrfach substituiert mit gleichen oder verschiedenen Substituenten, steht;
R^{16a} für H, C₁₋₆-Alkyl, Phenyl, Pyridyl, Pyrimidinyl, Imidazolyl, Triazolyl, Thiazolyl oder Thienyl, jeweils unsubstituiert oder einfach oder mehrfach substituiert mit gleichen oder verschiedenen Substituenten, steht;
R^{18a} für H; C₁₋₆-Alkyl; C₃₋₈-Cycloalkyl, N(C₁₋₆-Alkyl)₂; NH(C₁₋₆-Alkyl), Azetidinyl; Pyrrolidinyl, Piperidinyl, 4-(C₁₋₆Alkyl)-Piperazinyl; Phenyl, Pyridyl, Pyrimidinyl, Imidazolyl, Triazolyl, Thiazolyl oder Thienyl, jeweils jeweils unsubstituiert oder einfach oder mehrfach substituiert mit gleichen oder verschiedenen Substituenten; über eine -(O)_{0/1}C₁₋₆-Alkylengruppe gebundenes N(C₁₋₆-Alkyl)₂; NH(C₁₋₆-Alkyl),
Azetidinyl; Pyrrolidinyl, Piperidinyl, 4-(C₁₋₆Alkyl)-Piperazinyl; Phenyl, Pyridyl, Pyrimidinyl, Imidazolyl, Triazolyl, Thiazolyl oder Thienyl, jeweils unsubstituiert oder einfach oder mehrfach substituiert mit gleichen oder verschiedenen Substituenten, steht;
R^{18b} für H; OH; C₁₋₆-Alkyl, Phenyl, Pyridyl, Pyrimidinyl, Imidazolyl, Triazolyl, Thiazolyl oder Thienyl, jeweils unsubstituiert oder einfach oder mehrfach substituiert mit gleichen oder verschiedenen Substituenten; über eine C₁₋₆-Alkylengruppe gebundenes Phenyl, Pyridyl, Pyrimidinyl, Imidazolyl, Triazolyl, Thiazolyl oder Thienyl, jeweils unsubstituiert oder einfach oder mehrfach substituiert mit gleichen oder verschiedenen Substituenten; steht;
R¹⁹ für H; C₁₋₆-Alkyl; C₃₋₈-Cycloalkyl, Phenyl, Pyridyl, Pyrimidinyl, Imidazolyl, Triazolyl, Thiazolyl oder Thienyl, jeweils unsubstituiert oder einfach oder mehrfach substituiert mit gleichen oder verschiedenen Substituenten; über eine C₁₋₆-Alkylengruppe oder (C=O)-Gruppe gebundenes Phenyl, Pyridyl, Pyrimidinyl, Imidazolyl, Triazolyl, Thiazolyl oder Thienyl, jeweils unsubstituiert oder einfach oder mehrfach substituiert mit gleichen oder verschiedenen Substituenten, steht;
R¹²⁰ für H; F; Cl, ; OH; OCH₃, C₁-₆-Alkyl; Phenyl, jeweils unsubstituiert oder einfach oder mehrfach substituiert mit gleichen oder verschiedenen Substituenten , steht R¹²⁶ für H; C₁₋₆Alkyl; C₃₋₆Cycloalkyl; Phenyl, Pyridyl, Pyrimidinyl, Imidazolyl, Triazolyl, Thiazolyl oder Thienyl; über eine C₁₋₃-Alkylengruppe gebundenes C₃₋₆Cycloalkyl, Phenyl oder Pyridyl, jeweils unsubstituiert oder einfach oder mehrfach substituiert mit gleichen oder verschiedenen Substituenten, steht.

14. Verbindung gemäß einem der vorherigen Ansprüche, worin
in der der allgemeinen Formel I die Teilstruktur (B) 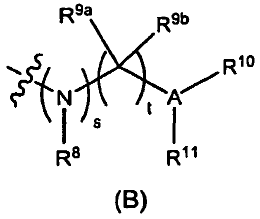 ausgesucht ist aus 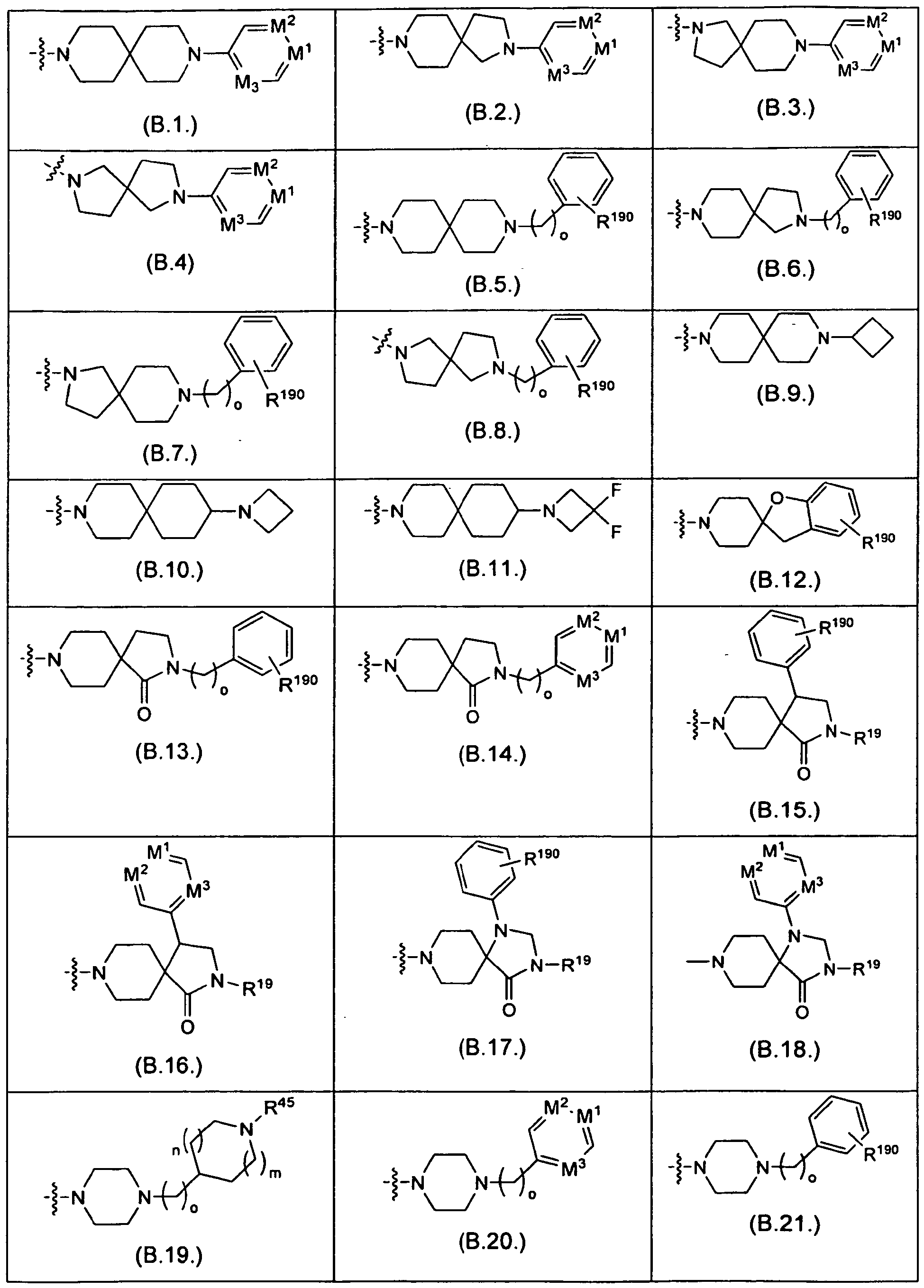 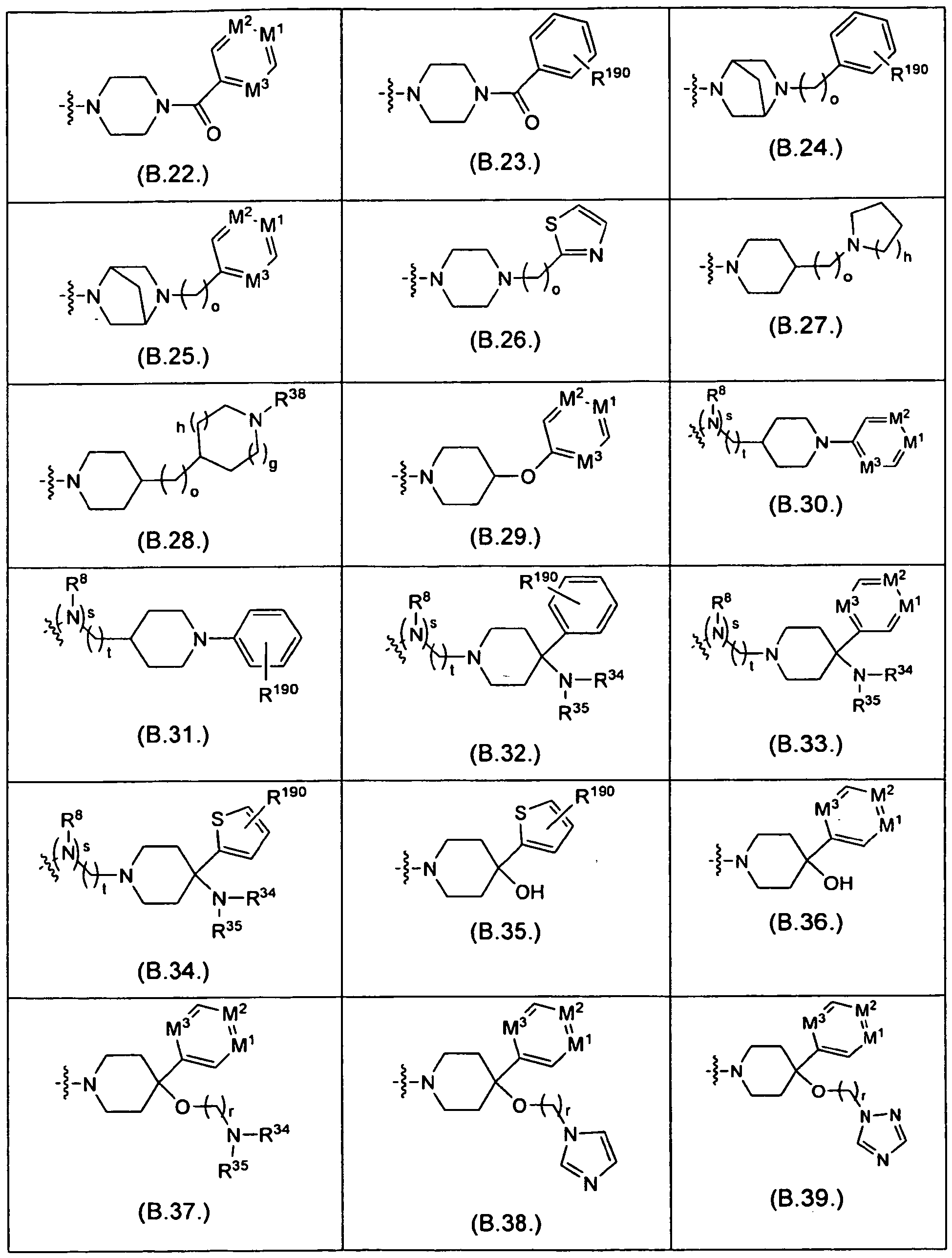 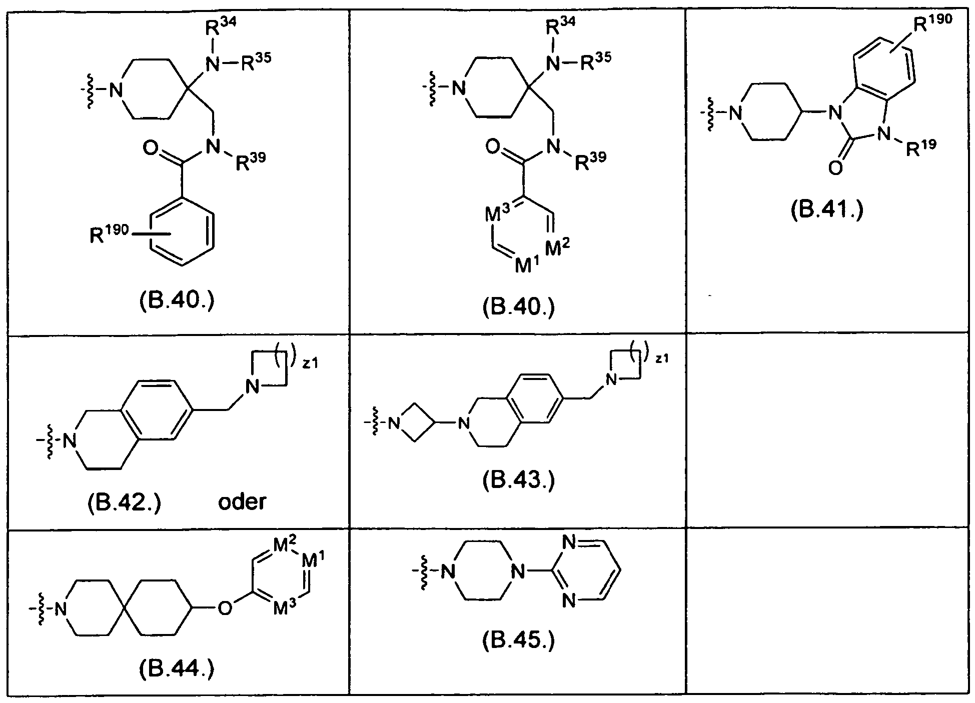 worin
h = 0 oder 1;
g = 0 oder 1;
m = 0 oder 1;
n = 0 oder 1,
o = 0, 1, 2 oder 3;
r = 1, 2 oder 3, insbesondere 1 oder 2;
s = 0 oder 1;
t = 0, 1, 2 oder 3, insbesondere 0, 1, oder 2, mit der Maßgabe, dass wenn s für 0 steht, t ebenfalls für 0 steht;
z1 = 0, 1, 2, oder 3 steht, insbesondere 1
M¹, M² und M³ unabhängig voneinander jeweils für N oder CH stehen können, wobei eine Variabel aus M¹, M² und M³ für N und die anderen beiden für CH stehen;
R⁸ für H; C₁₋₆Alkyl, insbesondere Methyl, Ethyl, n-Propyl, iso-Propyl, n-Butyl, sec-Butyl, iso-Butyl und tert-Butyl; C₃-₆Cycloalkyl, insbesondere Cyclopropyl, jeweils unsubstituiert oder einfach oder mehrfach substituiert mit gleichen oder verschiedenen Substituenten;
R¹⁹ ausgesucht ist aus H; C₁₋₆Alkyl, insbesondere Methyl, Ethyl, n-Propyl, iso-Propyl, n-Butyl, sec-Butyl, iso-Butyl und tert-Butyl; C₃₋₆Cycloalkyl, insbesondere Cyclopropyl; jeweils unsubstituiert oder einfach oder mehrfach substituiert mit gleichen oder verschiedenen Substituenten;
R³⁴ und R³⁵ vorzugsweise unabhängig voneinander Methyl oder Ethyl sind oder zusammen mit dem sie verbindenden N-Atom eine Azetidinyl-; Pyrrolidinyl-, Piperidinyl-, 4-(C₁₋₆Alkyl)-Piperazinyl-Gruppe bilden, jeweils unsubstituiert oder einfach oder mehrfach substituiert mit gleichen oder verschiedenen Substituenten; R³⁸ für H, C₁₋₆-Alkyl, C₃₋₆-Cycloalkyl oder Pyridyl steht;
R³⁹ ausgesucht ist aus H; C₁₋₆Alkyl, insbesondere Methyl, Ethyl, n-Propyl, iso-Propyl, n-Butyl, sec-Butyl, iso-Butyl und tert-Butyl; C₃₋₆Cycloalkyl, insbesondere Cyclopropyl, jeweils unsubstituiert oder einfach oder mehrfach substituiert mit gleichen oder verschiedenen Substituenten; und
R⁴⁵ für H, C₁₋₆-Alkyl, C₃₋₆-Cycloalkyl oder Pyridyl steht;
R¹⁹⁰ 0-4 Substituenten darstellt, die unabhängig voneinander ausgesucht sind aus F, Cl, O-CF₃, CF₃ oder CN.

15. Verbindungen gemäß einem der vorherigen Ansprüche, die durch die im Folgenden gezeigten allgemeinen Formel C1 bis C21 dargestellt werden: 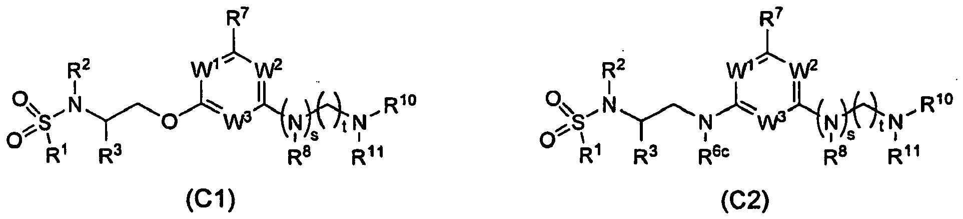 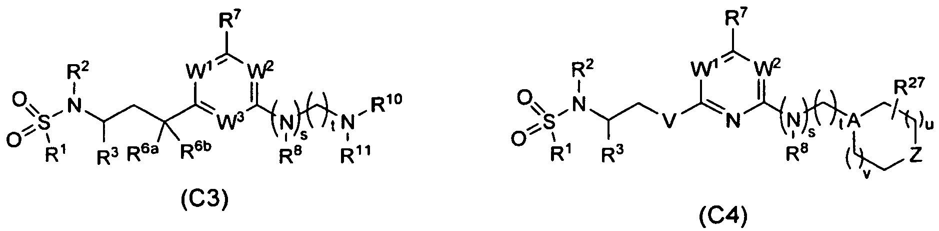 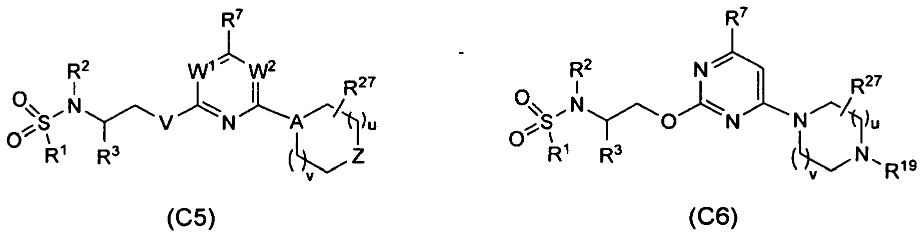 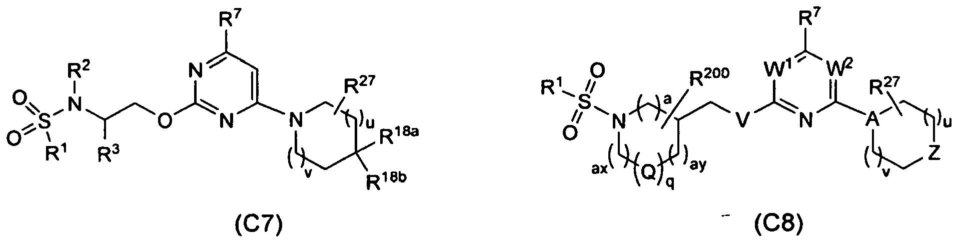 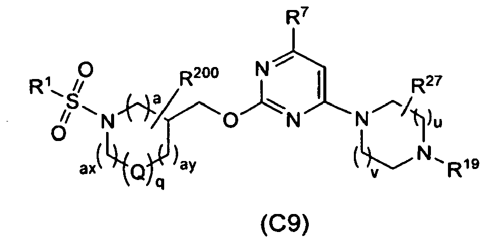 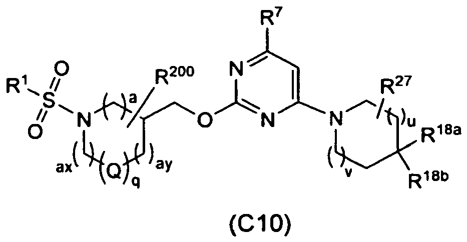 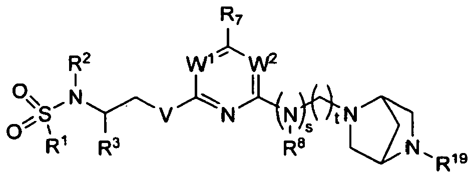 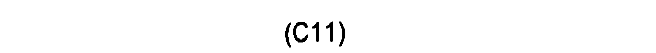 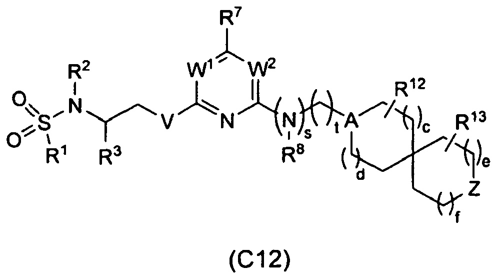 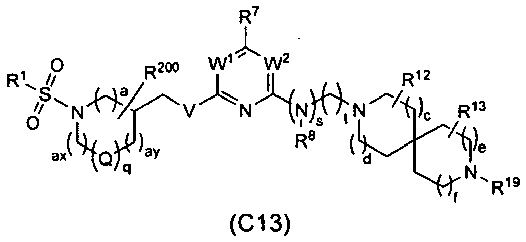 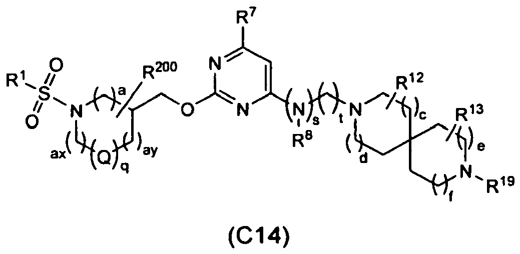 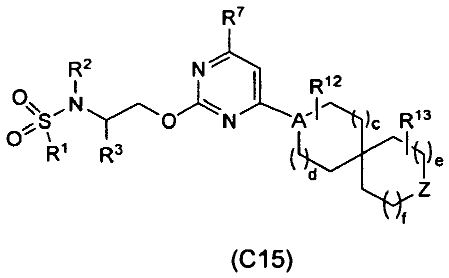 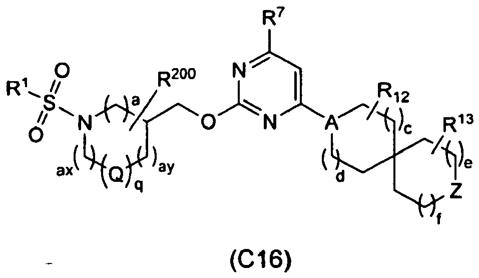 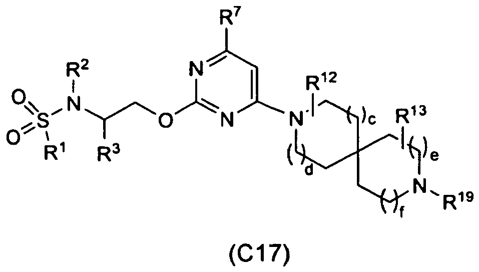 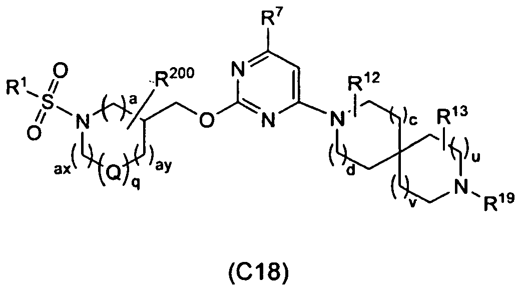 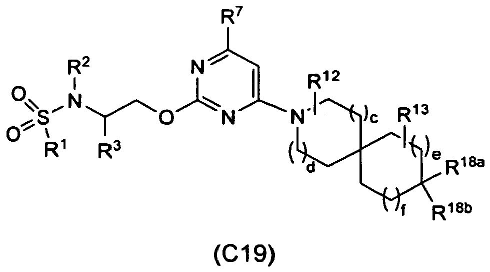 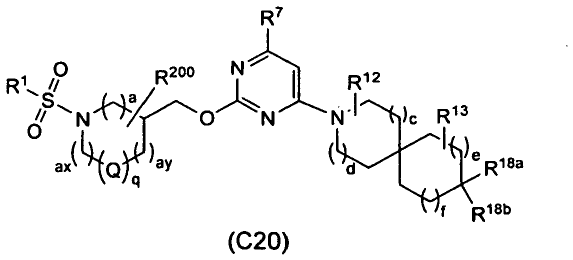 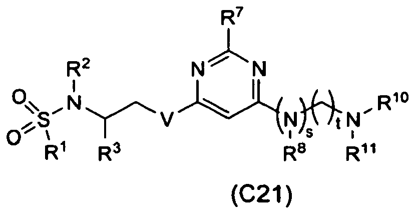 und worin
q für 0 oder 1 steht,
a für 0, 1 oder 2 steht;
ax für 0, 1, 2 oder 3 steht;
ay für 0, 1 oder 2;
q für 0 oder 1 steht;
mit der Maßgabe, dass a + ax + ay + q ≥ 2;
Q für CH₂, NR⁵⁰, O, S, S=O oder S(=O)₂ steht,
und alle weiteren Reste, Variabeln und Indices die in den vorhergehenden Ansprüchen 1 bis 13 beschriebenen Bedeutungen haben.

16. Verbindung gemäß einem der vorherigen Ansprüche, worin die Verbindung ausgewählt ist aus:
4-Methoxy-N,2,6-trimethyl-N-[2-[[4-[4-(2-1-pyrrolidinylethyl)-1-piperidinyl]-2-pyrimidinyl]oxy]ethyl]benzolsulfonamid,
4-Methoxy-N,2,6-trimethyl-N-[2-[[4-[4-(4-pyridyl)-1-piperazinyl]-2-pyrimidinyl]oxy]ethyl]benzolsulfonamid,
N-[2-[[4-[2-(4-Dimethylamino-4-phenyl-1-piperidinyl)ethyl-methylamino]-2-pyrimidinyl]oxy]ethyl]-4-methoxy-N,2,6-trimethylbenzolsulfonamid,
2-[[1-(4-Methoxy-2,6-dimethylphenyl)sulfonyl-2-piperidinyl]methoxy]-4-[4-(3-pyridyl)-4-(2-1-pyrroildinylethoxy)-1-piperidinyl]pyrimidin
N-Cyclopropyl-4-methoxy-2,6-dimethyl-N-[2-[[4-[9-(4-pyridyl)-3,9-diazaspiro[5.5]undecan-3-yl]-2-pyrimidinyl]oxy]ethyl]benzolsulfonamid hydroclorid.
N-Cyclopropyl-4-methoxy-2,6-dimethyl-N-[2-[[4-[2-[1-(4-pyridyl)-4-piperidinyl]ethylamino]-2-pyrimidinyl]oxy]ethyl]benzolsulfonamid,
N-Cyclopropyl-4-methoxy-2,6-dimethyl-N-[2-[[4-[4-(3-pyridyl)-4-(2-1-pyrrolldinylethoxy)-1-piperidinyl)-2-pyrimidinyl]oxy]ethyl]benzolsulfonamid,
N-Cyclopropyl-N-[2-[[4-[4-hydroxy-4-(3-pyridyl)-1-piperidinyl]-2-pyrimidinyl]oxy]ethyl]-4-methoxy-2,6-dimethylbenzolsulfonamid,
2-Chlor-N-cyclopropyl-6-methyl-N-[2-[[4-[9-(4-pyridyl)-3.9-diazaspiro[5.5]undecan-3-yl]-2-pyrimidinyl]oxylethyl]benzolsulfonamid,
N-Cyclopropyl-N-[2-[[4-[9-(4-pyridyl)-3,9-diazaspiro[5.5]undecan-3-yl]-2-pyrimidinyl]oxy]ethyl]-2-(trifluormethyl)benzolsulfonamid,
3-[2-([(2S,4R)-4-Fluor-1-(4-methoxy-2,6-dimethylphenyl)sulfonyl-2-pyrrylidinyl]methoxy]-4-pyrimidinyl]-9-(4-pyridyl)-3,9-diazaspiro[5.5]undecan,
N-Cyclopropyl-4-methoxy-2,6-dimethyl-N-[2-[[4-[4-(4-pyridyloxy)-1-piperidinyl]-2-pyrimidinyl]oxy]ethyl]benzolsulfonamid,
N-[2-[[4-[6-(1-Azetidinylmethyl)-3,4-dihydro-1H-isoquinolin-2-yl-2-pyrimidinyl]oxy]ethyl]-N-cyclopropyl-4-methoxy-2,6-dimethylbenzolsulfonamid,
N-Cyclopropyl-4-methoxy-2,6-dimethyl-N-[2-[[4-(8-(4-pyridyl)-3,8-diazaspiro[4.4]nonan-3-yl]-2-pyrimidinyl]oxy]ethyl]benzolsulfonamid,
N-[2-[[4-[9-(1-Azetidinyl)-3-azaspiro[5.5]undecan-3-yl]-2-pyrimidinyl]oxy]ethyl]-N-cyclopropyl-4-methoxy-2,6-dimethylbenzolsulfonamid hydroclorid,
N-Cyclopropyl-4-methoxy-2,6-dimethyl-N-[2-[[4-[9-(4-pyridyloxy)-3-azaspiro[5.5]undecan-3-yl]-2-pyrimidinyl]oxy]ethyl]benzolsulfonamid,
N-Cyclopropyl-N-[2-[[4-[9-(3,3-difluor-1-azetidinyl)-3-azaspiro[5.5]undecan-3-yl]-2-pyrimidinyl]oxy]ethyl]-4-methoxy-2,6-dimethylbenzolsulfonamid,
3-[2-[[1-(4-Methoxy-2,6-dimethylphenyl)sulfonyl-3-azetidinyl]oxy]-4-pyrimidinyl]-9-(4-pyridyl)-3,9-diazaspiro[5.5]undecan,
N-Cyclopropyl-4-methoxy-2,6-dimethyl-N-[2-[[4-[8-(4-pyridyl)-3,8-diazaspiro[4.5]decan-3-yl]-2-pyrimidinyl]oxy]ethyl]benzolsulfonamid,
N-[2-[[4-[3-[6-(1-Azetidinylmethyl)-3,4-dihydro-1H-isoquinolin-2-yl]-1-azetidinyl]-2-pyrimidinyl]oxylethyl]-N-cyclopropyl-4-methoxy-2,6-dimethylbenzolsulfonamid,
2,6-Dichlor-N-cyclopropyl-3-methyl-N-[2-[4-(9-pyridin-4-yl-3,9-diazaspiro[5.5]undecan-3-yl)-pyrimidin-2-yl]oxy-ethyl]-benzolsulfonsäure amid
4-Methoxy-2,6-dimethyl-N-[1-[[4-(9-pyridin-4-yl-3,9-diazaspiro[5.5]undecan-3-yl)-pyrimidin-2-yl]oxy-methyl]-cyclobutyl]-benzolsulfonsäure amid
N-Cyclopropyl-4-methoxy-2,6-dimethyl-N-[2-[4-[9-pyridin-3-yl-9-(2-pyrrolidin-1-yl-ethoxy)-3-azaspiro[5.5]undecan-3-yl]-pyrimidin-2-yl]oxy-ethyl]-benzolsulfonsäure amid
N-[1,1-Dimethyl-2-[4-(9-pyridin-4-yl-3,9-diazaspiro[5.5]undecan-3-yl)-pyrimidin-2-yl]oxy-ethyl]-4-methoxy-2,6-dimethyl-benzolsulfonsäure amid
N-Cyclopropyl-4-methoxy-2,6-dimethyl-N-[3-[4-(9-pyridin-4-yl-3,9-diazaspiro[5.5]undecan-3-yl)-pyrimidin-2-ylloxy-propyl]-benzolsulfonsäure amid
3-[2-[[(2S)-1-[(4-Methoxy-2,6-dimethyl-phenyl)sulfonyl]-piperidin-2-yl]-methoxy]-pyrimidin-4-yl]-9-pyridin-4-yl-3,9-diazaspiro[5.5]undecan
4-Methoxy-N,2,6-trimethyl-N-[2-[[2-[4-(2-1-pyrrolidinytethyl)-1-piperidinyl]-4-pyrimidinyl]oxy]ethyl]benzolsulfonamid,
4-Methoxy-N,2,6-trimethyl-N-[2-[[2-[4-(4-pyridyl)-1-piperazinyl]-4-pyrimidinyl]oxy]ethyl]benzolsulfonamid,
N-[2-[[2-[2-(4-Dimethylamino-4-phenyl-1-piperidinyl)ethyl-methylamino]-4-pyrimidinyl]oxy]ethyl]-4-methoxy-N,2,6-trimethylbenzolsulfonamid,
N-[2-(4-Dimethylamino-4-phenyl-1-piperidinyl)ethyl]-4-[[1-(4-methoxy-2,6-dimethylphenyl)sulfonyl-2-piperidinyl]methoxy]-N-methyl-2-pyrimidinamin,
N-Cyclopropyl-4-methoxy-2,6-dimethyl-N-[2-[[2-[4-(4-pyridyloxy)-1-piperidinyl]-4-pyrimidinyl]oxy]ethyl]benzolsulfonamid,
2-Chlor-N-cyclopropyl-6-methyl-N-[2-[[2-[9-(4-pyridyl)-3,9-diazaspiro[5.5]undecan-3-yl}-4-pyrimidinyl]oxy]ethyl]benzolsulfonamid.
3-{4-[[1-(4-Methoxy-2,6-dimethylphenyl)sulfonyl-3-azetidinyl]oxy]-2-pyrimidinyl]-9-(4-pyridyl)-3,9-diazaspiro[5.5]undecan,
N-Cyclopropyl-4-methoxy-2,6-dimethyl-N-12-[[6-19-(4-pyridyl)-3,9-diazaspiro[5.5]undecan-3-yl]-4-pyrimidinyl]oxy]ethyl]benzolsulfonamid,
N-Cyclopropyl-4-methoxy-2,6-dimethyl-N-[2-[methyl-[6-(9-pyridin-4-yl-3,9-diazaspiro[5.5]undecan-3-yl)-pyrazin-2-yl]-amino]-ethyl]-benzolsulfonsäure amid
N-Cyctopropyl-4-methoxy-2,6-dimethyl-N-[2-[methyl-[6-(9-pyridin-4-yl-3,9-diazaspiro[5.5]undecan-3-yl)-pyrimidin-4-yl]-amino]-ethyl]-benzolsulfonsäure amid
N-Cyclopropyl-4-methoxy-2,6-dimethyl-N-[2-[methyl-[2-(9-pyridin-4-yl-3,9-diazaspiro[5.5]undecan-3-yl)-pyrimidin-4-yl]-amino]-ethyl]-benzolsulfonsäure amid
N-Cyclopropyl-4-methoxy-2,6-dimethyl-N-[2-[methyl-[4-(9-pyridin-4-yl-3,9-diazaspiro[5.5]undecan-3-yl]pyrimidin-2-yl]-amino]-ethyl]-benzolsulfonsäure amid
N-Cyclopropyl-4-methoxy-2,6-dimethyl-N-[3-[6-(9-pyridin-4-yl-3,9-diazaspiro[5.5]undecan-3-yl)-pyrazin-2-yl]-propyl]benzolsulfonsäure amid
N-Cyclopropyl-4-methoxy-2,6-dimethyl-N-[3-[2-(9-pyridin-4-yl-3,9-diazaspiro[5.5]undecan-3-yl)-pyrimidin-4-yl]-propyl]-benzolsulfonsäure amid
N-Cyclopropyl-N-[3-[2-(9-pyridin-4-yl-3,9-diazaspiro[5.5]undecan-3-yl)-pyrimidin-4-yl]-propyl]-3-(trifluormethyl)-benzolsulfonsäure amid
4-Methoxy-N,2,6-trimethyl-N-[2-[[4-[4-(1-methyl-4-piperidinyl)-1-piperazinyl]-2-pyrimidinyl]oxy]ethyl]benzolsulfonamid,
2-[[1-(4-Methoxy-2.6-dimethylphenyl)sulfonyl-2-piperidinyl]methoxy]-4-[4-(1-methy)-4-piperidinyl)-1-piperazinyl]pyrimidin,
2-[[1-(4-Methoxy-2,6-dimethylphenyl)sulfonyl-2-piperidinyl]methoxy]-4-(4-(2-1-pyrrolidinylethyl)-1-piperidinyl]pyrimidin,
N-[2-(4-Dimethylamino-4-phenyl-1-piperidinyl)ethyl]-2-[[1-(4-methoxy-2,6-dimethylphenyl)sulfonyl-2-piperidinyl]methoxy]-N-methyl-4-pyrimidinamin,
N-[2-[[4-[4-(4-Fluorphenyl)-1-piperazinyl]-2-pyrimidinyl]oxy]ethyl]-4-methoxy-N,2,6-trimethylbenzolsulfonamid,
4-Methoxy-N,2,6-trimethyl-N-[2-[[4-[4-(4-methyl-1-piperazinyl)-1-piperidinyl]-2-pyrimidinyl]oxy]ethyl]benzolsulfonamid,
4-Methoxy-N,2,6-trimethyl-N-[2-[[4-[4-[(1-methyl-4-piperidinyl)methyl]-1-piperazinyl]-2-pyrimidinyl]oxy]ethyl]benzolsulfonamid,
N-[2-[[4-[4-Hydroxy-4-(3-pyridyl)-1-piperidinyl]-2-pyrimidinyl)oxylethyl]-4-methoxy-N,2,6-trimethylbenzolsulfonamid,
2-[[1-(4-Methoxy-2,6-dimethylphenyl)sulfonyl-2-piperidinyl]methoxy]-4-(4-methyl-1-piperazinyl)pyrimidin,
4-[4-(4-Fluorphenyl)-1-piperazinyl]-2-[[1-(4-methoxy-2,6-dimethylphenyl)sulfonyl-2-piperidinyl]methoxy]pyrimidin.
2-[[1-(4-Methoxy-2,6-dimethylphenyl)sulfonyl-2-piperidinyl]methoxy]-4-[4-(4-methyl-1-piperazinyl)-1-piperidinyl]pyrimidin,
2-[[1-(4-Methoxy-2,6-dimethylphenyl)sulfonyl-2-piperidinyl]methoxy]-4-[4-(2-pyrimidinyl)-1-piperazinyl]pyrimidin,
2-[[1-(4-Methoxy-2,6-dimethylphenyl)sulfonyl-2-piperidinyl]methoxyl-4-[4-(4-pyridyl)-1-piperazinyl]pyrimidin,
2-[[1-(4-Methoxy-2,6-dimethylphenyl)sulfonyl-2-piperidinyl]methoxy]-4-[4-[(1-methyl-4-piperidinyl)methyl]-1-piperazinyl]pyrimidin.
2-[[(2S)-1-(4-Methoxy-2,6-dimethylphenyl)sulfonyl-2-pyrrolidinyl]methoxy]-4-(4-methyl-1-piperazinyl)pyrimidin,
2-[[(2S)-1-(4-Methoxy-2,6-dimethylphenyl)sulfonyl-2-pyrrolidinyl]methoxy]-4-[4-(2-1-pyrrolidinylethyl)-1-piperidinyl]pyrimidin.
2-[[(2S)-1-(4-Methoxy-2,6-dimethylphenyl)sulfonyl-2-pyrrolidinyl]methoxy]-4-[4-(4-methyl-1-piperazinyl)-1-piperidinyl]pyrimidin,
2-[[(2S)-1-(4-Methoxy-2,6-dimethylphenyl)sulfonyl-2-pyrrolidinyl]methoxy]-4-[4-(2-pyrimidinyl)-1-piperazinyl]pyrimidin,
2-[[(2S)-1-(4-Methoxy-2,6-dimethylphenyl)sulfonyl-2-pyrrolidinyl]methoxy]-4-[4-(4-pyridyl)-1-piperazinyl]pyrimidin,
1-[2-[[(2S)-1-(4-Methoxy-2,6-dimethylphenyl)sulfonyl-2-pyrrolidinyl]methoxy]-4-pyrimidinyl]-4-(3-pyridyl)-4-piperidinol,
1-[2-[[(2S)-1-(4-Methoxy-2,6-dimethylphenyl)sulfonyl-2-pyrrolidinyl]methoxy]-4-pyrimidinyl]-4-(2-thienyl)-4-piperidinol,
3-benzyl-7-[2-[[1-(4-Methoxy-2,6-dimethylphenyl)sulfonyl-2-piperidinyl]methoxy]-4-pyrimidinyl]-3,7-diazaspiro[4.4]nonan,
1'-[2-[[1-(4-Methoxy-2,6-dimethylphenyl)sulfonyl-2-piperidinyl]methoxy]-4-pyrimidinyl]spiro[1 H-isobenzofuran-3,4'-piperidin],
6-chlor-3-[1-[2-[[1-(4-Methoxy-2,6-dimethylphenyl)sulfonyl-2-piperidinyl]methoxy]-4-pyrimidinyl]-4-piperidinyl]-1H-benzimidazol-2-on,
8-[2-[[1-(4-Methoxy-2,6-dimethylphenyl)sulfonyl-2-piperidinyl]methoxy]-4-pyrimidinyl]-4-phenyl-2,4,8-triazaspiro[4.5]decan-1-on,
2-[[1-(4-Methoxy-2,6-dimethylphenyl)sulfonyl-2-piperidinyl]methoxy]-4-[4-[2-(1-piperidyl)ethyl]-1-piperidinyl]pyrimidin,
2-[[1-(4-Methoxy-2,6-dimethylphenyl)sulfonyl-3-piperidinyl]oxy]-4-[4-[2-(1-piperidyl)ethyl]-1-piperidinyl]pyrimidin,
N-[2-[[4-(3-Benzyl-3,7-diazaspiro[4.4]nonan-7-yl)-2-pyrimidinyl]oxy]ethyl]-4-methoxy-N,2,6-trimethylbenzolsulfonamid,
4-Methoxy-N,2,6-trimethyl-N-[2-[[4-(1'-spiro[1H-isobenzofuran-3,4'-piperidin]yl)-2-pyrimidinyl]oxy]ethyl]benzolsulfonamid,
4-Methoxy-N,2,6-trimethyl-N-[2-[[4-(1-oxo-4-phenyl-2,4,8-triazaspiro[4.5]decan-8-yl)-2-pyrimidinyl]oxy]ethyl]benzolsulfonamid,
4-Methoxy-N,2,6-trimethyl-N-[2-[[4-[4-[2-(1-piperidyl)ethyl]-1-piperidinyl]-2-pyrimidinyl]oxy]ethyl]benzolsulfonamid,
3-Benzyl-7-[2-[[(2S)-1-(4-methoxy-2,6-dimethylphenyl)sulfonyl-2-pyrrolidinyl]methoxy]-4-pyrimidinyl]-3,7-diazaspiro[4.4]nonan,
1'-[2-[[(2S)-1-(4-Methoxy-2,6-dimethylphenyl)sulfonyl-2-pyrrolidinyl]methoxy]-4-pyrimidinyl]spiro[1H-isobenzofuran-3,4'-piperidin],
6-Chlor-3-(1-[2-[[(2S)-1-(4-methoxy-2,6-dimethylphenyl)sulfonyl-2-pyrrolidinyl]methoxy]-4-pyrimidinyl]-4-piperidinyl]-1H-benzimidazol-2-on,
8-[2-[[(2S)-1-(4-Methoxy-2,6-dimethylphenyl)sulfonyl-2-pyrrolidinyl]methoxy]-4-pyrimidinyl)-4-phenyl-2.4,8-triazaspiro[4.5]decan-1-on.
2-[[(2S)-1-(4-Methoxy-2,6-dimethylphenyl)sulfonyl-2-pyrrolidinyl]methoxy]-4-[4-(1-methyl-4-piperidinyl)-1-piperazinyl]pyrimidin,
2-[[(2S)-1-(4-Methoxy-2,6-dimethylphenyl)sulfonyl-2-pyrrolidinyl]methoxy]-4-[4-[2-(1-piperidyl)ethyl]-1-piperidinyl]pyrimidin,
3-(4-Fluorphenyl)-6-[2-[[1-(4-methoxy-2,6-dimethylphenyl)sulfonyl-2-piperidinyl]methoxy]-4-pyrimidinyl]-3,8-diazaspiro[4.5]decan-4-on,
3-[(4-Fluorphenyl)methyl]-8-[2-[[1-(4-methoxy-2,6-dimethylphenyl)sulfonyl-2-piperidinyl]methoxy]-4-pyrimidinyl]-3,8-diazaspiro[4.5]decan-4-on,
3-Benzyl-8-[2-[[1-(4-methoxy-2,6-dimethylphenyl)sulfonyl-2-piperidinyl]methoxy]-4-pyrimidinyl]-3,8-diazaspiro[4.5]decan-4-on,
9-[2-[[1-(4-Methoxy-2,6-dimethylphenyl)sulfonyl-3-piperidinyl]oxy]-4-pyrimidinyl]-3-(4-pyridyl)-3,9-diazaspiro[5.5]undecan,
N-[2-[[4-[3-(4-Fluorphenyl)-4-oxo-3,8-diazaspiro[4.5]decan-8-yl]-2-pyrimidinyl]oxy]ethyl]-4-methoxy-N,2,6-trimethylbenzolsulfonamid,
4-Methoxy-N,2,6-trimethyl-N-2-[[4-[4-oxo-1-[3-(trifluormethyl)phenyl]-3,8-diazaspiro[4.5]decan-8-yl]-2-pyrimidinyl]oxy]ethyl]senzolsulfonamid,
N-[2-[[4-[1-(4-Fluorphenyl)-3-methyl-4-oxo-3,8-diazaspiro[4.5]decan-8-yl]-2-pyrimidinyl]oxy]ethyl]-4-methoxy-N,2,6-trimethylbenzolsulfonamid,
N-[2-[[4-[3-[(4-Fluorphenyl)methyl]-4-oxo-3,8-diazaspiro[4.5]decan-8-yl]-2-pyrimidinyl]oxy]ethyl]-4-methoxy-N,2,6-trimethylbenzolsulfonamid,
N-[2-[[4-(3-Benzyl-4-oxo-3,8-diazaspiro[4.5]decan-8-yl)-2-pyrimidinyl]oxy]ethyl]-4-methoxy-N,2,6-trimethylbenzolsulfonamid,
4-Methoxy-N,2,6-trimethyl-N-[2-[[4-[3-(4-pyridyl)-3,9-diazaspiro[5.5]undecan-9-yl]-2-pyrimidinyl]oxy]ethyl]benzolsulfonamid,
3-(4-Fluorphenyl)-8-[2-[[(2S)-1-(4-methoxy-2,6-dimethylphenyl)sulfonyl-2-pyrrolidinyl]methoxy]-4-pyrimidinyl]-3,8-diazaspiro[4.5]decan-4-on,
8-[2-[[(2S)-1-(4-Methoxy-2,6-dimethylphenyl)sulfonyl-2-pyrrolidinyl]methoxy]-4-pyrimidinyl]-1-[3-(trifluormethyl)phenyl]-3,8-diazaspiro[4.5]decan-4-on,
1-(4-Fluorphenyl)-8-[2-[[(2S)-1-(4-methoxy-2,6-dimethylphenyl)sulfonyl-2-pyrrolidinyl]methoxy]-4-pyrimidinyl]-3-methyl-3,8-diazaspiro[4.5]decan-4-on,
3-[(4-Fluorphenyl)methyl]-8-[2-[[(2S)-1-(4-methoxy-2,6-dimethylphenyl)sulfonyl-2-pyrrolidinyl]methoxy]-4-pyrimidinyl]-3,8-diazaspiro[4.5]decan-4-on,
2-[[(2S)-1-(4-Methoxy-2,6-dimethylphenyl)sulfonyl-2-pyrrolidinyl]methoxy]-4-[4-(4-pyridyloxy)-1-piperidinyl]pyrimidin,
3-Benzyl-8-[2-[[(2S)-1-(4-methoxy-2,6-dimethylphenyl)sulfonyl-2-pyrrolidinyl]methoxy]-4-pyrimidinyl]-3,8-diazaspiro[4.5]decan-4-on,
N-[[1-[2-[[(2R)-1-(4-Methoxy-2,6-dimethylphenyl)sulfonyl-2-pyrrolidinyl]methoxy]-4-pyrimidinyl]-4-(4-methyl-1-piperazinyl)-4-piperidinyl]methyl]-4-pyridincarboxamid,
9-[2-[[(2S)-1-(4-Methoxy-2,6-dimethylphenyl)sulfonyl-2-pyrrolidinyl]methoxy]-4-pyrimidinyl]-3-(4-pyridyl)-3,9-diazaspiro[5.5]undecan,
5-[2-[[1-(4-Methoxy-2,6-dimethylphenyl)sulfonyl-2-piperidinyl]methoxy]-4-pyrimidinyl]-2-(4-pyridylmethyl)-2,5-diazabicyclo[2.2.1]heptan,
5-[2-[[1-(4-Methoxy-2,6-dimethylphenyl)sulfonyl-3-piperidinyl]oxy]-4-pyrimidinyl]-2-(4-pyridylmethyl)-2,5-diazabicyclo[2.2.1]heptan,
5-[2-[[(2S)-1-(4-Methoxy-2,6-dimethylphenyl)sulfonyl-2-pyrrolidinyl]methoxy]-4-pyrimidinyl]-2-(4-pyridylmethyl)-2,5-diazabicyclo[2.2.1]heptan,
4-Methoxy-N,2,6-trimethyl-N-[2-[[4-[4-(1-methyl-4-piperidinyl)-1-piperazinyl]-2-pyrimidinyl]oxy]-1-phenylethyl]benzolsulfonamid,
N-[2-[[4-[3-[(4-Fluorphenyl)methyl]-4-oxo-3,8-diazaspiro[4.5]decan-8-yl]-2-pyrimidinyl]oxy]-1-phenylethyl]-4-methoxy-N,2,6-trimethylbenzolsulfonamid,
N-[2-[[4-(3-Benzyl-4-oxo-3,8-diazaspiro[4.5]decan-8-yl)-2-pyrimidinyl]oxy]-1-phenylethyl]-4-methoxy-N,2,6-trimethylbenzolsulfonamid,
4-Methoxy-N,2,6-trimethyl-N-[1-phenyl-2-[[4-[4-(3-pyridyl)-4-(2-1-pyrrolidinylethoxy)-1-piperidinyl]-2-pyrimidinyl]oxy]ethyl]benzolsulfonamid,
4-Methoxy-N,2,6-trimethyl-N-[1-phenyl-2-[[4-[4-(4-pyridyloxy)-1-piperidinyl]-2-pyrimidinyl]oxy]ethyl]benzolsulfonamid,
4-Methoxy-N,2,6-trimethyl-N-[2-[[4-[4-[oxo-(3-pyridyl)methyl]-1-piperazinyl]-2-pyrimidinyl]oxy]-1-phenylethyl]benzolsulfonamid,
N-[2-[[4-[2-[(4-Fluorphenyl)methyl]-2,5-diazabicyclo[2.2.1]heptan-5-yl]-2-pyrimidinyl]oxy]-1-phenylethyl]-4-methoxy-N,2,6-trimethylbenzolsulfonamid,
2-[[1-(4-Methoxy-2,6-dimethylphenyl)sulfonyl-2-piperidinyl]methoxyl-4-[4-(4-pyridyl)-1-piperazinyl]pyrimidin,
1-[2-[[1-(4-Methoxy-2,6-dimethylphenyl)sulfonyl-2-piperidinyl]methoxy]-4-pyrimidinyl]-4-(3-pyridyl)-4-piperidinol,
N-[2-[[4-(3-Benzyl-3,7-diazaspiro[4.4]nonan-7-yl)-2-pyrimidinyl]oxy]-1-phenylethyl]-4-methoxy-N,2,6-trimethylbenzolsulfonamid,
[4-Butyl-1-[2-[[1-[(4-methoxy-2,6-dimethyl-phenyl)sulfonyl]-piperidin-2-yl]-methoxy]-pyrimidin-4-yl]-piperidin-4-yl]-dimethyl-amin
[1-[2-[[1-[(4-Methoxy-2,6-dimethyl-phenyl)sulfonyl]-piperidin-2-yl]-methoxy]-pyrimidin-4-yl]-4-thiophen-2-yl-piperidin-4-yl]-dimethyl-amin
[2-[[1-[(4-Methoxy-2,6-dimethyl-phenyl)sulfonyl]-piperidin-2-yl]-methoxy]-pyrimidin-4-yl]-methyl-[2-(4-phenyl-4-pyrrolidin-1-yl-piperidin-1-yl)-ethyl]-amin
2-(4-Butyl-4-dimethylamino-piperidin-1-yl)-ethyl-[2-[[1-[(4-methoxy-2,6-dimethylphenyl)sulfonyl]-piperidin-2-yl]-methoxy]-pyrimidin-4-yl]-methyl-amin
3-(4-Dimethylamino-4-phenyl-piperidin-1-yl)-propyl-[2-[[(2S)-1-[(4-methoxy-2,6-dimethylphenyl)sulfonyl]-pyrrolidin-2-yl]-methoxy]-pyrimidin-4-yl]-methyl-amin
3-(4-Dimethylamino-4-phenyl-piperidin-1-yl)-propyl-[2-[[(2S)-1-[(4-methoxy-2,6-dimethylphenyl)sulfonyl]-2,3-dihydro-1H-indol-2-yl]-methoxy]-pyrimidin-4-yl]-methyl-amin
2-[1-[(4-Methoxy-2,6-dimethyl-phenyl)sulfonyl]-azetidin-3-yl]oxy-4-[4-[(1-methyl-piperidin-4-y))-methyl]-piperazin-1-yl)-pyrimidin
2-[[1-[(4-Methoxy-2,6-dimethyl-phenyl)sulfonyl)-piperidin-2-yl]-methoxy]-4-(4-pyridin-2-yloxy-piperidin-1-yl)-pyrimidin
2-[[1-[(4-Methoxy-2,6-dimethyl-phenyl)sulfonyl)-piperidin-2-yl]-methoxy]-4-(4-pyrazin-2-yloxy-piperidin-1-yl)-pyrimidin
2-[[1-[(4-Methoxy-2,6-dimethyl-phenyl)sulfonyl]-piperidin-2-yl]-methoxy]-4-[4-pyridin-3-yl-4-(3-pyrrolidin-1-yl-propyl)-piperidin-1-yl]-pyrimidin
2-[[1-[(4-Methoxy-2,6-dimethyl-phenyl)sulfonyl]-piperidin-2-yl]-methoxy]-4-[2-(pyridin-2-yl-methyl)-pyrrolidin-1-yl]-pyrimidin
1-[2-[[1-[(4-Methoxy-2,6-dimethyl-phenyl)sulfonyl]-piperidin-2-yl]-methoxyrpyrimidin-4-yl]-4-pyridin-2-yl-piperidin-4-ol
1-[2-[[(2S)-1-[(4-Methoxy-2,6-dimethyl-phenyl)sulfonyl]-pyrrolidin-2-yl]-methoxy]-pyrimidin-4-yl]-4-pyridin-2-yl-piperidin-4-ol
2-[[(2R)-1-[(4-Methoxy-2,6-dimethyl-phenyl)sulfonyl]-pyrrolidin-2-yl]-methoxy]-4-[2-(pyridin-2-yl-methyl)-pyrrolidin-1-yl]-pyrimidin
5-[1-[2-[[(2S)-1-[(4-Methoxy-2,6-dimethyl-phenyl)sulfonyl]-pyrrolidin-2-yl]-methoxy]-pyrimidin-4-yl]-piperidin-4-yl]-3-pyrid in-4-yl-[1,2,4]oxadiazol
4-[4-(3-Fluorphenyl)-4-(2-pyrrolidin-1-yl-ethoxy)-piperidin-1-yl]-2-[[(2S)-1-[(4-methoxy-2,6-dimethyl-phenyl)sulfonyl]-pyrrolidin-2-yl]-methoxy]-pyrimidin
[4-Butyl-1-[2-[[(2S)-1-[(4-methoxy-2,6-dimethyl-phenyl)sulfonyl]-pyrrolidin-2-yl]-methoxy]-pyrimidin-4-yl]-piperidin-4-yl]-dimethyl-amin
2-(4-Dimethylamino-4-phenyl-piperidin-1-yl)-ethyl-[2-[[(2S)-1-[(4-methoxy-2,6-dimethylphenyl)sulfonyl]-pyrrolidin-2-yl]-methoxy]-pyrimidin-4-yl]-methyl-amin
N-[2-[4-(4-Butyl-4-dimethylamino-piperidin-1-yl)-pyrimidin-2-yl]oxy-ethyl]-4-methoxy-N,2,6-trimethyl-benzolsulfonsäure amid
[2-[[(2S)-1-[(4-Methoxy-2,6-dimethyl-phenyl)sulfonyl]-pyrrolidin-2-yl]-methoxy]-pyrimidin-4-yl]-methyl-[2-(4-phenyl-4-pyrrolidin-1-yl-piperidin-1-yl)-ethyl]-amin (2S)-1-[(4-Methoxy-2,6-dimethyl-phenyl)sulfonyl]-2-[[4-(4-pyrazin-2-yloxy-piperidin-1-yl)-pyrimidin-2-yl]oxy-methyl]-2,3-dihydro-1H-indol
[4-Butyl-1-[2-[[(2S)-1-[(4-methoxy-2,6-dimethyl-phenyl)sulfonyl]-2,3-dihydro-1H-indol-2-yl]-methoxy]-pyrimidin-4-yl]-piperidin-4-yl]-dimethyl-amin
2-(4-Dimethylamino-4-phenyl-piperidin-1-yl)-ethyl-[2-[[(2S)-1-((4-methoxy-2,6-dimethylphenyl)sulfonyl]-2,3-dihydro-1H-indol-2-yl]-methoxy]-pyrimidin-4-yl]-methyl-amin
[2-[[(2S)-1-[(4-Methoxy-2,6-dimethyl-phenyl)sulfonyl]-2,3-dihydro-1H-indol-2-yl]-methoxy]-pyrimidin-4-yl]-methyl-[2-(4-phenyl-4-pyrrolidin-1-yl-piperidin-1-yl)-ethyl]-amin
2-(4-Butyl-4-dimethylamino-piperidin-1-yl)-ethyl-[2-[[(2S)-1-[(4-methoxy-2,6-dimethylphenyl)sulfonyl]-2,3-dihydro-1H-indol-2-yl]-methoxy]-pyrimidin-4-yl]-methyl-amin
N-[2-[4-[2-(4-Dimethylamino-4-phenyl-piperidin-1-yl)-ethyl-methyl-amino]-pyrimidin-2-yl]oxy-ethyl]-4-methoxy-N,2,6-trimethyl-benzolsulfonsäure amid
N-[2-[4-[3-(4-Dimethylamino-4-phenyl-piperidin-1-yl)-propyl-methyl-amino]-pyrimidin-2-yl]oxy-ethyl]-4-methoxy-N,2,6-trimethyl-benzolsulfonsäure amid
3-(4-Dimethylamino-4-phenyl-piperidin-1-yl)-propyl-[2-[[1-[(4-methoxy-2,6-dimethylphenyl)sulfonyl]-piperidin-2-yl]-methoxy]-pyrimidin-4-yl]-methyl-amin
5-[1-[2-[[1-[(4-Methoxy-2,6-dimethyl-phenyl)sulfonyl]-piperidin-2-yl]-methoxy]-pyrimidin-4-yl]-piperidin-4-yl]-3-pyridin-4-yl-[1,2,4]oxadiazol
4-[4-(3-Fluorphenyl)-4-(4-methyl-piperazin-1-yl)-piperidin-1-yl]-2-[[1-[(4-methoxy-2,6-dimethyl-phenyl)sulfonyl]-piperidin-2-yl]-methoxy]-pyrimidin
(1S,5R)-8-[2-[[1-[(4-Methoxy-2,6-dimethyl-phenyl)sulfonyl]-piperidin-2-yl]-methoxy]-pyrimidin-4-yl]-3-pyridin-3-yloxy-8-azabicyclo[3.2.1]octan
1-[2-[[2-[[1-[(4-Methoxy-2,6-dimethyl-phenyl)sulfonyl]-piperidin-2-yl]-methoxy]-pyrimidin-4-yl]-methyl-amino]-ethyl]-4-pyridin-3-yl-piperidin-4-ol
4-Methoxy-N,2,6-trimethyl-N-[1-phenyl-2-[4-[(1S,5R)-3-pyridin-3-yloxy-8-azabicyclo[3.2.1]octan-8-yl]-pyrimidin-2-yl]oxy-ethyl]-benzolsulfonsäure amid
4-Methoxy-N,2,6-trimethyl-N-[1-phenyl-2-[4-[4-pyridin-3-yl-4-(3-pyrrolidin-1-yl-propyl) piperidin-1-yl]-pyrimidin-2-yl]oxy-ethyl]-benzolsulfonsäure amid
7-[2-[[(2S)-1-[(4-Methoxy-2,6-dimethyl-phenyl)sulfonyl]-pyrrolidin-2-yl]methoxy]-pyrimidin-4-yl]-2-(piperidin-1-yl-methyl)-5,6,7,8-tetrahydro-imidazo[1,2-a]pyrazin
1-[2-[[1-[(4-Methoxy-2,6-dimethyl-phenyl)sulfonyl]-piperidin-2-yl]-methoxy]-pyrimidin-4-yl]-4-pyridin-4-yl-piperidin-4-ol
N-[2-[4-(4-Hydroxy-4-pyridin-4-yl-piperidin-1-yl)-pyrimidin-2-yl]oxy-1-phenyl-ethyl]-4-methoxy-N,2,6-trimethyl-benzolsulfonsäure amid
[1-[2-[[1-[(4-Methoxy-2,6-dimethyl-phenyl)sulfonyl]-piperidin-2-yl]-methoxy]-pyrimidin-4-yl]-4-phenyl-piperidin-4-yl]-dimethyl-amin
2-[1-[(4-Methoxy-2,6-dimethyl-phenyl)sulfonyl]-piperidin-3-yl]oxy-4-(4-phenyl-4-pyrrolidin-1-yl-piperidin-1-yl)-pyrimidin
2-[[(2S)-1-[(4-Methoxy-2,6-dimethyl-phenyl)sulfonyl]-pyrrolidin-2-yl]-methoxy]-4-(4-phenyl-4-pyrrolidin-1-yl-piperidin-1-yl)-pyrimidin
4-Methoxy-N,2,6-trimethyl-N-[2-[4-(4-phenyh4-pyrrolidin-1-yl-piperidin-1-yl)-pyrimidin-2-yl]oxy-ethyl]-benzolsulfonsäure amid
N-[2-[4-(4-Dimethylamino-4-phenyl-piperidin-1-yl)-pyrimidin-2-yl]oxy-ethyl]-4-methoxy-N,2,6-trimethyl-benzolsulfonsäure amid
N-[2-[4-(4-Dimethylamino-4-thiophen-2-yl-piperidin-1-yl)-pyrimidin-2-yl]oxy-ethyl]-4-methoxy-N,2,6-trimethyl-benzolsulfonsäure amid
4-Methoxy-N,2,6-trimethyl-N-[2-[4-[methyl-[2-(4-phenyl-4-pyrrolidin-1-yl-piperidin-1-yl)-ethyl]amino]-pyrimidin-2-yl]oxy-ethyl]-benzolsulfonsäure amid N-[2-[4-[2-(4-Butyl-4-dimethylamino-piperidin-1-yl)-ethyl-methyl-amino]-pyrimidin-2-yl]oxy-ethyl]-4-methoxy-N,2,6-trimethyl-benzolsulfonsäure amid
2-[[1-[(4-Methoxy-2,6-dimethyl-phenyl)sulfonyl]-piperidin-2-yl]-methoxy]-4-(4-phenyl-4-pyrrolidin-1-yl-piperidin-1-yl)-pyrimidin
2-[1-[(4-Methoxy-2,6-dimethyl-phenyl)sulfonyl]-azetidin-3-yl]oxy-4-(4-phenyl-4-pyrrolidin-1-yl-piperidin-1-yl)-pyrimidin
2-(4-Dimethylamino-4-phenyl-piperidin-1-yl)-ethyl-[2-[1-[(4-methoxy-2,6-dimethylphenyl)sulfonyl]-azetidin-3-yl]oxy-pyrimidin-4-yl]-methyl-amin
[2-[1-[(4-Methoxy-2,6-dimethyl-phenyl)sulfonyl]-azetidin-3-yl]oxy-pyrimidin-4-yl]-methyl-[2-(4-phenyl-4-pyrrolidin-1-yl-piperidin-1-yl)-ethyl]-amin
[1-[2-[[(2S)-1-[(4-Methoxy-2,6-dimethyl-phenyl)sulfonyl]-2,3-dihydro-1H-indol-2-yl]-methoxy]-pyrimidin-4-yl]-4-thiophen-2-yl-piperidin-4-yl]-dimethyl-amin
3-(4-Dimethylamino-4-phenyl-piperidin-1-yl)-propyl-[2-[1-[(4-methoxy-2,6-dimethylphenyl)sulfonyl]-azetidin-3-yl]oxy-pyrimidin-4-yl]-methyl-amin
3-[4-[2-[1-[(4-Methoxy-2,6-dimethyl-phenyl)sulfonyl]-azetidin-3-yl]oxy-pyrimidin-4-yl]-piperazin-1-yl]-propyl-dimethyl-amin
1-[2-[1-[(4-Methoxy-2,6-dimethyl-phenyl)sulfonyl]-azetidin-3-yl]oxy-pyrimidin-4-yl]-4-pyridin-3-yl-piperidin-4-ol
2-[1-[(4-Methoxy-2,6-dimethyl-phenyl)sulfonyl]-azetidin-3-yl]oxy-4-[4-(2-piperidin-1-yl-ethyl)-piperidin-1-yl]-pyrimidin
(2S)-2-[[4-[2-[(4-Fluorphenyl)-methyl]-2,5-diazabicyclo[2.2.1]heptan-5-yl]-pyrimidin-2-yl]oxymethyl]-1-[(4-methoxy-2,6-dimethyl-phenyl)sulfonyl]-2,3-dihydro-1H-indol
4-[1-[2-[[1-[(4-Methoxy-2,6-dimethyl-phenyl)sulfonyl-piperidin-2-yl]-methoxy]-pyrimidin-4-yl]-4-methyl-piperidin-4-yl]-morpholin
4-Methoxy-N,2,6-trimethyl-N-[1-phenyl-2-[4-[2-(pyridin-2-yl-methyl)-pyrrolidin-1-yl]-pyrimidin-2-yl]oxy-ethyl]-benzolsulfonsäure amid
4-Methoxy-N,2,6-trimethyl-N-[1-phenyl-2-[4-[4-(3-pyridin-4-yl-[1,2,4]oxadiazol-5-yl)-piperidin-1-yl]-pyrimidin-2-yl]oxy-ethyl]-benzolsulfonsäure amid
N-[2-[4-[4-(3-Fluorphenyl)-4-(4-methyl-piperazin-1-yl)-piperidin-1-yl]-pyrimidin-2-yl]oxy-1-phenyl-ethyl]-4-methoxy-N,2,6-trimethyl-benzolsulfonsäure amid
4-Methoxy-N,2,6-trimethyl-N-[1-phenyl-2-[4-(4-pyridin-2-yloxy-piperidin-1-yl)-pyrimidin-2-yl]oxy-ethyl]-benzolsulfonsäure amid
4-Methoxy-N,2,6-trimethyl-N-[2-[4-(4-methyl-4-morpholin-4-yl-piperidin-1-yl)-pyrimidin-2-yl]oxy-1-phenyl-ethyl]-benzolsulfonsäure amid
N-[2-[4-[2-(4-Hydroxy-4-pyridin-3-yl-piperidin-1-yl)-ethyl-methyl-amino]-pyrimidin-2-yl]oxy-1-phenyl-ethyl]-4-methoxy-N,2,6-trimethyl-benzolsulfonsäure amid
4-[[1-(4-Methoxy-2,6-dimethylphenyl)sulfonyl-2-piperidinyl]methoxy]-2-[4-(1-methyl-4-piperidinyl)-1-piperazinyl]pyrimidin,
4-[[1-(4-Methoxy-2,6-dimethylphenyl)sulfonyl-2-piperidinyl]methoxy]-2-[4-(2-1-pyrrolidinylethyl)-1-piperidinyl]pyrimidin,
4-[[1-(4-Methoxy-2,6-dimethylphenyl)sulfonyl-2-piperidinyl]methoxy]-2-(4-methyl-1-piperazinyl)pyrimidin,
4-[[1-(4-Methoxy-2,6-dimethylphenyl)sulfonyl-2-piperidinyl]methoxy]-2-[4-(2-pyrimidinyl)-1-piperazinyl]pyrimidin,
4-[[1-(4-Methoxy-2,6-dimethylphenyl)sulfonyl-2-piperidinyl]methoxy]-2-[4-(4-pyridyl)-1-piperazinyl]pyrimidin,
4-[[1-(4-Methoxy-2,6-dimethylphenyl)sulfonyl-2-piperidinyl]methoxy]-2-[4-[(1-methyl-4-piperidinyl)methyl]-1-piperazinyl]pyrimidin,
1-[4-[[1-(4-Methoxy-2,6-dimethylphenyl)sulfonyl-2-piperidinyl]methoxy]-2-pyrimidinyl]-4-(3-pyridyl)-4-piperidinol.
4-[[(2S)-1-(4-Methoxy-2,6-dimethylphenyl)sulfonyl-2-pyrrolidinyl]methoxy]-2-(4-methyl-1-piperazinyl)pyrimidin,
4-[[(2S)-1-(4-Methoxy-2,6-dimethylphenyl)sulfonyl-2-pyrrolidinyl]methoxy]-2-[4-(2-1-pyrrolidinylethyl)-1-piperidinyl]pyrimidin,
4-[[(2S)-1-(4-Methoxy-2,6-dimethylphenyl)sulfonyl-2-pyrrolidinyl]methoxy]-2-[4-(4-methyl-1-piperazinyl)-1-piperidinyl]pyrimidin,
4-[[(2S)-1-(4-Methoxy-2,6-dimethylphenyl)sulfonyl-2-pyrrolidinyl]methoxy]-2-[4-(2-pyrimidinyl)-1-piperazinyl]pyrimidin,
4-[[(2S)-1-(4-Methoxy-2,6-dimethylphenyl)sulfonyl-2-pyrrolidinyl]methoxy]-2-[4-[(1-methyl-4-piperidinyl)methyl]-1-piperazinyl]pyrimidin,
1-[4-[[(2S)-1-(4-Methoxy-2,6-dimethylphenyl)sulfonyl-2-pyrrolidinyl]methoxy]-2-pyrimidinyl]-4-(3-pyridyl)-4-piperidinol,
4-[[1-(4-Methoxy-2,6-dimethylphenyl)sulfonyl-3-azetidinylloxyl-2-(4-(2-1-pyrrolidinylethyl)-1-piperidinyl]pyrimidin,
4-[[1-(4-Methoxy-2,6-dimethylphenyl)sulfonyl-3-azetidinyl]oxy]-2-[4-(4-methyl-1-piperazinyl)-1-piperidinyl]pyrimidin,
(2S)-1-(4-Methoxy-2,6-dimethylphenyl)sulfonyl-2-[[2-(4-methyl-1-piperazinyl)-4-pyrimidinyl]oxymethyl]indolin,
(2S)-1-(4-Methoxy-2,6-dimethylphenyl)sulfonyl-2-[[2-[4-(2-1-pyrrolidinylethyl)-1-piperidinyl]-4-pyrimidinyl]oxymethyl]indolin,
(2S)-1-(4-Methoxy-2,6-dimethylphenyl)sulfonyl-2-[[2-[4-(4-methyl-1-piperazinyl)-1 - piperidinyl]-4-pyrimidinyl]oxymethyl]indolin,
(2S)-1-(4-Methoxy-2,6-dimethylphenyl)sulfonyl-2-[[2-[4-(4-pyridyl)-1-piperazinyl]-4-pyrimidinyl]oxymethyl]indolin,
(2S)-1-(4-Methoxy-2,6-dimethylphenyl)sulfonyl-2-[[2-[4-[(1-methyl-4-piperidinyl)methyl]-1-piperazinyl]-4-pyrimidinyl]oxymethyl]indoli,n
3-Benzyl-7-[4-[[1-(4-methoxy-2,6-dimethylphenyl)sulfonyl-2-piperidinyl]methoxy]-2-pyrimidinyl]-3,7-diazaspiro[4.4]nonan,
4-[[1-(4-Methoxy-2,6-dimethylphenyl)sulfonyl-2-piperidinyl]methoxy]-2-[4-[2-(1-piperidyl)ethyl]-1-piperidinyl]pyrimidin.
3-Benzyl-7-[4-[[(2S)-1-(4-methoxy-2,6-dimethylphenyl)sulfonyl-2-pyrrolidinyl]methoxy]-2-pyrimidinyl]-3,7-diazaspiro[4.4]nonan,
8-[4-[[(2S)-1-(4-Methoxy-2,6-dimethylphenyl)sulfonyl-2-pyrrolidinyl]methoxy]-2-pyrimidinyl]-4-phenyl-2,4,8-triazaspiro[4.5]decan-1-on,
4-[[(2S)-1-(4-Methoxy-2,6-dimethylphenyl)sulfonyl-2-pyrrolidinyl]methoxy]-2-[4-(1-methyl-4-piperidinyl)-1-piperazinyl]pyrimidin,
4-[[(2S)-1-(4-Methoxy-2,6-dimethylphenyl)sulfonyl-2-pyrrolidinyl]methoxy]-2-[4-[2-(1-piperidyl)ethyl]-1-piperidinyl]pyrimidin,
(2S)-1-(4-Methoxy-2,6-dimethylphenyl)sulfonyl-2-[[2-[4-(1-methyl-4-piperidinyl)-1-piperazinyl]-4-pyrimidinyl]oxymethyl]indolin,
(2S)-1-(4-Methoxy-2,6-dimethylphenyl)sulfonyl-2-[[2-(4-[2-(1-piperidyl)ethyl]-1-piperidinyl]-4-pyrimidinyl]oxymethyl]indolin,
4-Methoxy-N,2,6-trimethyl-N-[2-[[2-[3-(4-pyridyl)-3,9-diazaspiro[5.5]undecan-9-yl]-4-pyrimidinyl]oxy]ethyl]benzolsulfonamid,
3-[(4-Fluorphenyl)methyl]-8-[4-[[1-(4-methoxy-2,6-dimethylphenyl)sulfonyl-2-piperidinyl]methoxy]-2-pyrimidinyl]-3,8-diazaspiro[4.5]decan-4-on,
N-[[1-[4-[[1-(4-Methoxy-2,6-dimethylphenyl)sulfonyl-2-piperidinyl]methoxy]-2-pyrimidinyl]-4-(4-methyl-1-piperazinyl)-4-piperidinyl]methyl]-4-pyridincarboxamid,
9-[4-[[1-(4-Methoxy-2,6-dimethylphenyl)sulfonyl-2-piperidinyl]methoxy]-2-pyrimidinyl]-3-(4-]pyridyl)-3,9-diazaspiro[5.5]undecan,
4-Methoxy-N,2,6-trimethyl-N-[2-[[2-[4-(3-pyridyl)-4-(2-1-pyrrolidinylethoxy)-1-piperidinyl]-4-pyrimidinyl]oxy]ethyl]benzolsulfonamid.
4-Methoxy-N,2,6-trimethyl-N-[2-[[2-[2-(4-pyridylmethyl)-2,5-diazabicyclo[2.2.1]heptan-5-yl]-4-pyrimidinyl]oxy]ethyl]benzolsulfonamid,
4-[[1-(4-Methoxy-2,6-dimethylphenyl)sulfonyl-2-piperidinyl]methoxy]-2-[4-(3-pyridyl)-4-(2-1-pyrrolidinylethoxy)-1-piperidinyl]pyrimidin,
4-Methoxy-N,2,6-trimethyl-N-[1-phenyl-2-[[2-(4-(4-pyridyl)-1-piperazinyl]-4-pyrimidinyl]oxy]ethyl]benzolsulfonamid,
4-Methoxy-N,2,6-trimethyl-N-[1-phenyl-2-[[2-[3-(4-pyridyl)-3,9-diazaspiro[5.5]undecan-9-yl)-4-pyrimidinyl]oxy]ethyl]benzolsulfonamid,
N-Methyl-N-[1-phenyl-2-[[2-[4-(3-pyridyl)-4-(2-1-pyrrolidinylethoxy)-1-piperidinyl]-4-pyrimidinyl]oxyJethyl]-2-naphthalinsulfonamid,
N-[2-[[2-[3-[(4-Fluorphenyl)methyl]-4-oxo-3,8-diazaspiro[4.5]decan-8-yl]-4-pyrimidinyl]oxy]ethyl]-4-methoxy-N,2,6-trimethylbenzolsulfonamid,
4-[[(2S)-1-(4-Methoxy-2,6-dimethylphenyl)sulfonyl-2-pyrrolidinyl]methoxy]-2-[4-(3-pyridyl)-4-(2-1-pyrrolidinylethoxy)-1-piperidinyl]pyrimidin, oder
4-[[1-(4-Methoxy-2,6-dimethylphenyl)sulfonyl-3-piperidinyl]oxy]-2-[4-[2-(1-piperidyl)ethyl]-1-piperidinyl]pyrimidin.
1-[4-[[1-[(4-Methoxy-2,6-dimethyl-phenyl)sulfonyl]-piperidin-2-yl]-methoxy]-pyrimidin-2-yl]-4-(pyridin-2-yl-methyl)-[1,4]diazepan
1-[4-[[(2R)-1-[(4-Methoxy-2,6-dimethyl-phenyl)sulfonyl]-pyrrolidin-2-yl]-methoxy]-pyrimidin-2-yl]-4-(pyridin-2-yl-methyl)-[1,4]diazepan
2-(4-Dimethylamino-4-phenyl-piperidin-1-yl)-ethyl-[4-[[1-[(4-methoxy-2,6-dimethylphenyl)sulfonyl]-piperidin-2-yl]-methoxy]-pyümidin-2-yl]-methyl-amin
2-(4-Butyl-4-dimethylamino-piperidin-1-yl)-ethyl-[4-[[1-[(4-methoxy-2,6-dimethylphenyl)sulfonyl]-piperidin-2-yl]-methoxy]-pyrimidin-2-yl]-methyl-amin
3-(4-Dimethylamino-4-phenyl-piperidin-1-yl)-propyl-[4-[[(2S)-1-[(4-methoxy-2,6-dimethylphenyl)sulfonyl]-pyrrolidin-2-yl]-methoxy]-pyrimidin-2-yl]-methyl-amin
1-[4-[[(2S)-1-[(4-Methoxy-2,6-dimethyl-phenyl)sulfonyl]-pyrrolidin-2-yl]-methoxy]-pyrimidin-2-yl]-4-pyridin-2-yl-piperidin-4-ol
5-[1-[4-[[(2S)-1-[(4-Methoxy-2,6-dimethyl-phenyl)sulfonyl]-pyrrolidin-2-yl)-methoxy]-pyrimidin-2-yl]-piperidin-4-yl]-3-pyridin-4-yl-[1,2,4]oxadiazol
[4-[[1-[(4-Methoxy-2,6-dimethyl-phenyl)sulfonyl]-piperidin-2-yl]-methoxy]-pyrimidin-2-yl]-methyl-[2-(4-pyridin-4-yloxy-piperidin-1-yl)-ethyl]-amin
(1S,5R)-8-[4-[[1-[(4-Methoxy-2,6-dimethyl-phenyl)sulfonyl]-piperidin-2-yl]-methoxy]-pyrimidin-2-yl]-3-pyridin-4-yloxy-8-azabicyclo[3.2.1]octan
[4-[[(2S)-1-[(4-Methoxy-2,6-dimethyl-phenyl)sulfonyl]-pyrrolidin-2-yl]-methoxy]-pyrimidin-2-yl]-methyl-[2-(4-pyridin-4-yloxy-piperidin-1-yl)-ethyl]-amin
(1S,5R)-8-[4-[[(2S)-1-[(4-Methoxy-2,6-dimethyl-phenyl)sulfonyl]-pyrrolidin-2-yl]-methoxy]-pyrimidin-2-yl]-3-pyridin-4-yloxy-8-azabicyclo[3.2.1]octan
2-(4-Butyl-4-dimethylamino-piperidin-1-yl)-ethyl-[4-[1-[(4-methoxy-2,6-dimethylphenyl)sulfonyl]-piperidin-3-yl]oxy-pyrimidin-2-yl]-methyl-amin
2-(4-Dimethylamino-4-phenyl-piperidin-1-yl)-ethyl-[4-[[(2S)-1-[(4-methoxy-2,6-dimethylphenyl)sulfonyl]-pyrrolidin-2-yl]-methoxy]-pyrimidin-2-yl]-methyl-amin
[4-[[(2S)-1-[(4-Methoxy-2,6-dimethyl-phenyl)sulfonyl]-pyrrolidin-2-yl]-methoxy]-pyrimidin-2-yl]-methyl-[2-(4-phenyl-4-pyrrolidin-1-yl-piperidin-1-yl)-ethyl]-amin
2-(4-Butyl-4-dimethylamino-piperidin-1-yl)-ethyl-[4-[[(2S)-1-[(4-methoxy-2,6-dimethylphenyl)sulfonyl]-pyrrolidin-2-yl]-methoxy]-pyrimidin-2-yl]-methyl-amin
2-(4-Dimethylamino-4-phenyl-piperidin-1-yl)-ethyl-[4-[[(2S)-1-[(4-methoxy-2,6-dimethylphenyl)sulfonyl]-2,3-dihydro-1H-indol-2-yl]-methoxy]-pyrimidin-2-yl]-methyl-amin
2-(4-Butyl-4-dimethylamino-piperidin-1-yl)-ethyl-[4-[[(2S)-1-[(4-methoxy-2,6-dimethylphenyl)sulfonyl]-2,3-dihydro-1H-indol-2-yl]-methoxy]-pyrimidin-2-yl]-methyl-amin
4-Methoxy-N,2,6-trimethyl-N-[2-[2-[methyl-[2-(4-phenyl-4-pyrrolidin-1-yl-piperidin-1-yl)-ethyl]-amino]-pyrimidin-4-yl]oxy-ethyl}-benzolsulfonsäure amid
N-[2-[2-(2-(4-Butyl-4-dimethylamino-piperidin-1-yl)-ethyl-methyl-amino]-pyrimidin-4-yl]oxy-ethyl]-4-methoxy-N,2,6-trimethyl-benzolsulfonsäure amid
3-(4-Dimethylamino-4-phenyl-piperidin-1-yl)-propyl-[4-[[1-[(4-methoxy-2,6-dimethylphenyl)sulfonyl]-piperidin-2-yl]-methoxy]-pyrimidin-2-yl]-methyl-amin
[4-Butyl-1-[4-[[1-[(4-methoxy-2,6-dimethyl-phenyl)sulfonyl]-piperidin-2-yl]-methoxy]-pyrimidin-2-yl]-piperidin-4-yl]-dimethyl-amin
[4-[[1-[(4-Methoxy-2,6-dimethyl-phenyl)sulfonyl]-piperidin-2-yl]-methoxy]-pyrimidin-2-yl]-methyl-[2-(4-phenyl-4-pyrrolidin-1-yl-piperidin-1-yl)-ethyl]-amin
[1-[4-[1-[(4-Methoxy-2,6-dimethyl-phenyl)sulfonyl]-piperidin-3-yl]oxy-pyrimidin-2-yl]-4-phenyl-piperidin-4-yl]-dimethyl-amin
2-(4-Dimethylamino-4-phenyl-piperidin-1-yl)-ethyl-[4-[1-[(4-methoxy-2,6-dimethylphenyl)sulfonyl]-piperidin-3-yl]oxy-pyrimidin-2-yl]-methyl-amin
[4-[1-[(4-Methoxy-2,6-dimethyl-phenyl)sulfonyl]-piperidin-3-yl]oxy-pyrimidin-2-yl]-methyl-[2-(4-phenyl-4-pyrrolidin-1-yl-piperidin-1-yl)-ethyl]-amin
N-[2-[2-[2-(4-Dimethylamino-4-phenyl-piperidin-1-yl)-ethyl-methyl-amino]-pyrimidin-4-yl]oxy-ethyl]-4-methoxy-N,2,6-trimethyl-benzolsulfonsäure amid
[4-Butyl-1-[4-[[(2S)-1-[(4-methoxy-2,6-dimethyl-phenyl)sulfonyl]-pyrrolidin-2-yl]-methoxy]-pyrimidin-2-yl]-piperidin-4-yl]-dimethyl-amin
2-(4-Dimethylamino-4-phenyl-piperidin-1-yl)-ethyl-[4-[1-[(4-methoxy-2,6-dimethylphenyl)sulfonyl]-azetidin-3-yl]oxy-pyrimidin-2-yl]-methyl-amin
[4-[1-[(4-Methoxy-2,6-dimethyl-phenyl)sulfonyl]-azetidin-3-yl]oxy-pyrimidin-2-yl]-methyl-[2-(4-phenyl-4-pyrrolidin-1-yl-piperidin-1-yl)-ethyl]-amin
2-(4-Butyl-4-dimethylamino-piperidin-1-yl)-ethyl-[4-[1-[(4-methoxy-2,6-dimethylphenyl)sulfonyl]-azetidin-3-yl]oxy-pyrimidin-2-yl]-methyl-amin
[4-[[(2S)-1-[(4-Methoxy-2,6-dimethyl-phenyl)sulfonyl]-2,3-dihydro-1H-indol-2-yl]-methoxy]-pyrimidin-2-yl]-methyl-[2-(4-phenyl-4-pyrrolidin-1-yl-piperidin-1-yl)-ethyl]-amin
3-(4-Dimethylamino-4-phenyl-piperidin-1-yl)-propyl-[4-[1-[(4-methoxy-2,6-dimethylphenyl)sulfonyl]-piperidin-3-yl]oxy-pyrimidin-2-yl]-methyl-amin
3-(4-Dimethylamino-4-phenyl-piperidin-1-yl)-propyl-[4-[1-[(4-methoxy-2,6-dimethylphenyl)sulfonyl]-azetidin-3-yl]oxy-pyrimidin-2-yl]-methyl-amin
3-(4-Dimethylamino-4-phenyl-piperidin-1-yl)-propyl-[4-[[(2S)-1-[(4-methoxy-2,6-dimethylphenyl)sulfonyl]-2,3-dihydro-1H-indol-2-yl]-methoxy]-pyrimidin-2-yl]-methyl-amin
1-[4-[1-[(4-Methoxy-2,6-dimethyl-phenyl)sulfonyl]-azetidin-3-yl]oxy-pyrimidin-2-yl]-4-pyridin-3-yl-piperidin-4-ol
1-[4-[1-[(4-Methoxy-2,6-dimethyl-phenyl)sulfonyl]-azetidin-3-yl]oxy-pyrimidin-2-yl]-4-(pyridin-2-yl-methyl)-[1,4]diazepan
4-[[1-[(4-Methoxy-2,6-dimethyl-phenyl)sulfonyl]-piperidin-2-yl]-methoxy]-2-[2-(pyridin-2-yl-methyl)-pyrrolidin-1-yl]-pyrimidin
4-[[1-[(4-Methoxy-2,6-dimethyl-phenyl)sulfonyl]-piperidin-2-yl]-methoxy]-2-(4-pyridin-2-yloxy-piperidin-1-yl)-pyrimidin
4-[[1-[(4-Methoxy-2,6-dimethyl-phenyl)sulfonyl]-piperidin-2-yl]-methoxy]-2-(4-pyrazin-2-yloxy-piperidin-1-yl)-pyrimidin
4-[1-[4-[[1-[(4-Methoxy-2,6-dimethyl-phenyl)sulfonyl]-piperidin-2-yl]-methoxy]-pyrimidin-2-yl]-4-methyl-piperidin-4-yl]-morpholin
4-[[1-[(4-Methoxy-2,6-dimethyl-phenyl)sulfonyl]-piperidin-2-yl]-methoxy]-2-[4-pyridin-3-yl-4-(3-pyrrolidin-1-yl-propyl)-piperidin-1-yl]-pyrimidin
1-[4-[[1-[(4-Methoxy-2,6-dimethyl-phenyl)sulfonyl]-piperidin-2-yl]-methoxy]-pyrimidin-2-yl]-4-pyridin-2-yl-piperidin-4-ol
4-[[(2R)-1-[(4-Methoxy-2,6-dimethyl-phenyl)sulfonyl]-pyrrolidin-2-yl]-methoxy]-2-[2-(pyridin-2-yl-methyl)-pyrrolidin-1-yl]-pyrimidin
[1-[4-[[(2S)-1-[(4-Methoxy-2,6-dimethyl-phenyl)sulfonyl]-pyrrolidin-2-yl]-methoxy]-pyrimidin-2-yl]-4-phenyl-piperidin-4-yl]-dimethyl-amin
[1-[4-[[(2S)-1-[(4-Methoxy-2,6-dimethyl-phenyl)sulfonyl]-pyrrolidin-2-yl]-methoxy]-pyrimidin-2-yl]-4-thiophen-2-yl-piperidin-4-yl]-dimethyl-amin
4-Methoxy-N,2,6-trimethyl-N-[2-[2-[2-(pyridin-2-yl-methyl)-pyrrolidin-1-yl]-pyrimidin-4-yl]oxy-ethyl]-benzolsulfonsäure amid
4-[[(2S)-1-[(4-Methoxy-2,6-dimethyl-phenyl)sulfonyl]-pyrrolidin-2-yl]-methoxy]-2-[2-(pyridin-2-yl-methyl)-pyrrolidin-1-yl]-pyrimidin
4-Methoxy-N,2,6-trimethyl-N-[2-[2-[methyl-[2-(4-pyridin-4-yloxy-piperidin-1-yl)-ethyl]-amino]-pyrimidin-4-yl]oxy-ethyl]-benzolsulfonsäure amid
N-[2-[2-[4-(3-Fluorphenyl)-4-(2-pyrrolidin-1-yl-ethoxy)-piperidin-1-yl]-pyrimidin-4-yl]oxy-ethyl]-4-methoxy-N,2,6-trimethyl-benzolsulfonsäure amid
2-[4-(3-Fluorphenyl)-4-(4-methyl-piperazin-1-yl)-piperidin-1-yl]-4-[[1-[(4-methoxy-2,6-dimethyl-phenyl)sulfonyl]-piperidin-2-yl]-methoxy]-pyrimidin
2-[4-(3-Fluorphenyl)-4-(2-pyrrolidin-1-yl-ethoxy)-piperidin-1-yl]-4-[[(2S)-1-[(4-methoxy-2,6-dimethyl-phenyl)sulfonyl]-pyrrolidin-2-yl]-methoxy]-pyrimidin
4-Methoxy-N,2,6-trimethyl-N-[1-phenyl-2-[2-[(1S,5R)-3-pyridin-4-yloxy-8-azabicyclo[3.2.1]octan-8-yl]-pyrimidin-4-yl]oxy-ethyl]-benzolsulfonsäure amid

17. Arzneimittel enthaltend wenigstens eine Verbindung gemäß einem oder mehreren der Ansprüche 1 bis 15, gegebenenfalls enthaltend geeignete Zusatzstoffe und/oder Hilfsstoffe und/oder weitere Wirkstoffe.

18. Verwendung wenigstens einer Verbindung gemäß einem der Ansprüche 1 bis 15 zur Herstellung eines Arzneimittels zur Behandlung von Schmerz, insbesondere von akutem Schmerz, viszeralem Schmerz, neuropathischem Schmerz, und/oder chronischem Schmerz, Entzündungsschmerz, Migräne, Diabetes, Erkrankungen der Atemwege, entzündlichen Darmerkrankungen, neurologischen Erkrankungen, Entzündungen der Haut, rheumatischen Erkrankungen, septischem Schock, Reperfusionssyndrom, Fettleibigkeit und als Angiognese-Inhibitor.

## Claims

1. A compound of the general formula I 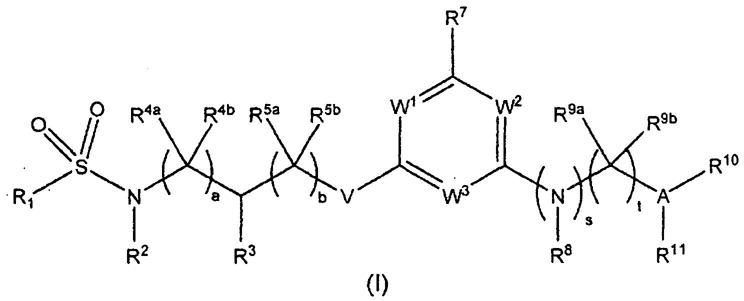 wherein
a represents 0, 1 or 2;
b represents 0, 1 or 2;
R¹ represents aryl, heteroaryl CH(aryl)₂, or an aryl or heteroaryl bonded via a C₁₋₃-alkylene group;
R² and R³ are defined as described under (i) or (ii):
(i) R² represents H, C₁₋₆-alkyl, C₃₋₈-cydoalkyl, aryl or heteroaryl; or R² denotes a C₃₋₈-cycloalkyl, aryl or heteroaryl bonded *via* a C₁₋₆-alkylene group, C₂₋₆-alkenylene group or C₂₋₆-alkynylene group;
R³ represents H, F, Cl, Br, I, -CF₃, -OCF₃, OH, O-C₁₋₆-alkyl, C₁₋₆-alkyl, C₃₋₈-cycloalkyl, aryl, heteroaryl; or R³ denotes a C₃₋₈-cycloalkyl, aryl or heteroaryl bonded *via* a C₁₋₆-alkylene group, C₂₋₆-alkenylene group or C₂₋₆-alkynylene group;
or
(ii) R² and R³, together with the group -N-(CR^{4a}R^{4b})ₐ-CH- linking them, form a heterocycle which can be substituted on one or more of its carbon ring members by one or more radicals selected independently of one another from the group consisting of F, Cl, Br, I, -CF₃, =O, -O-CF₃, -OH, -SH, -O-C₁₋₆-alkyl, C₁₋₆-alkyl, C₃₋₈-cycloalkyl, aryl and heteroaryl and/or can be fused to an aryl or heteroaryl and/or two of its carbon ring members are linked together *via* a C₁₋₃-alkylene bridge, wherein the heterocycle is saturated or at least monounsaturated but is not aromatic, is 4-, 5-, 6- or 7-membered and can contain, in addition to the N heteroatom to which the radical R² is bonded, one or more heteroatoms or heteroatom groups selected independently of one another from the group consisting of N, NR⁵⁰, O, S, S=O and S(=O)₂; wherein the radical R⁵⁰ denotes H, C₁₋₆-alkyl, -C(=O)-R⁵¹, C₃₋₈-cycloalkyl, aryl, heteroaryl, or a C₃₋₈-cycloalkyl, aryl or heteroaryl bonded *via* a C₁₋₃-alkylene group, and R⁵¹ denotes C₁₋₆-alkyl, C₃₋₈-cycloalkyl, aryl, heteroaryl, or a C₃₋₈-cycloalkyl, aryl or heteroaryl bonded *via* a C₁₋₃-alkylene group;
V represents C(R^{6a})(R^{6b}), NR^{6c}, O or a single bond,
wherein R^{6c} represents a radical from the group H, C₁₋₆-alkyl, C₃₋₈-cycloalkyl, aryl, heteroaryl, or represents a C₃₋₈-cycloalkyl, C₃₋₈-cyclalkyl, aryl or heteroaryl bonded *via* a C₁₋₆-alkylene group, C₂₋₆-alkenylene group or C₂₋₆-alkynylene group,
R^{4a}, R^{4b}, R^{5a}, R^{5b}, R^{6a}, R^{6b} independently of one another represent H, F, Cl, Br, I, -CF₃, -OCF₃, OH, SH, O-C₁₋₆-alkyl, C₁₋₆-alkyl, C₃₋₈-cycloalkyl, aryl or heteroaryl; or represent a C₃₋₈-cycloalkyl, aryl or heteroaryl bonded *via* a C₁₋₆-alkylene group or C₂₋₆-alkenylene group; and R^{6a} and R^{6b} can additionally together denote =O;
and/or
R^{4a} and R^{4b}, together with the carbon atom linking them, form a saturated ring which is unsubstituted or substituted on one or more, for example 1, 2, 3 or 4, of its carbon ring members by one or more, for example 1, 2, 3 or 4, substituents selected independently of one another from the group consisting of F, CF₃, C₁₋₆-alkyl, O-C₁₋₆-alkyl, OH, OCF₃, aryl and heteroaryl, wherein the ring is 3-, 4-, 5- or 6-membered and can contain one or more, for example 1 or 2, oxygen atoms;
R⁷ represents a substituent from the group H, C₁₋₆-alkyl, -CN, -CF₃, OH, C₁₋₆-alkoxy, -O-CF₃;
W¹, W² and W³ independently of one another represent N or CR⁶⁰, with the proviso that at least two of W¹, W² and W³ represent N, and R⁶⁰ represents H, C₁₋₆-alkyl, halogen, -CN, CF₃, OH, C₁₋₆-alkoxy or -O-CF₃;
s is 0 or 1,
t is 0, 1, 2 or 3,
R⁸ represents H; C₁₋₆-alkyl; C₃₋₈-cycloalkyl; aryl or heteroaryl; C₃₋₈-cycloalkyl, aryl or heteroaryl bonded *via* a C₁₋₆-alkylene group;
R^{9a} and R^{9b} each independently of the other denotes H; F; Cl; OH; C₁₋₆-alkyl; O-C₁₋₆-allkyl; C₃₋₈-cycloalkyl; aryl or heteroaryl; C₃₋₈-cycloalkyl, aryl or heteroaryl bonded *via* a C₁₋₆-alkylene group;
A represents N or CH,
with the proviso that when s represents 1 and t represents 0, A represents CH; and
with the proviso that when s and t each represents 0, A represents N;
the radicals R¹⁰ and R¹¹, with the inclusion of A, represent a spirocyclic or cyclic group of one of the general formulae II and III: 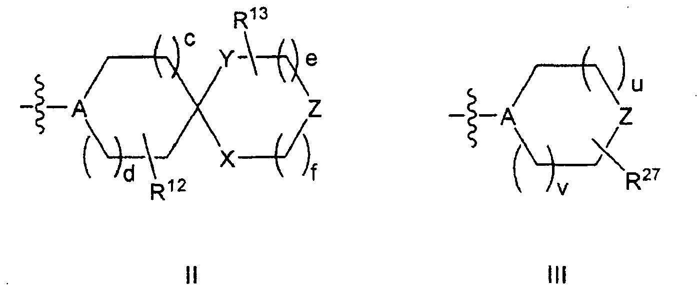 wherein
c, d, e, f, u and v each independently of the others denotes 0, 1 or 2;
R¹² , R¹³ and R²⁷ each independently of the others represents from 0 to 4 substituents each selected independently of any others from F; Cl; OH; =O; C₁₋₆-alkyl; O-C₁₋₆-alkyl; C₃₋₈-cycloalkyl; aryl or heteroaryl; C₃₋₈-cycloalkyl, aryl or heteroaryl bonded *via* a C₁₋₆-alkylene group;
and/or in each case two of the 0 to 4 substituents R²⁷ together represent a C₁₋₃-alkylene bridge, so that the ring shown in the general formula III assumes a bicyclically bridged form;
and/or two of the 0 to 4 substituents R¹³ that are adjacent form a fused aryl or heteroaryl;
and/or two of the 0 to 4 substituents R²⁷ that are adjacent form a fused aryl or heteroaryl;
X represents CR^{14a}R^{14b}, NR¹⁵ or O;
Y represents CR^{16a}R^{16b}, NR¹⁷ or O;
with the proviso that X does not denote NR¹⁵ when Y denotes NR¹⁷; and with the proviso that X and Y do not simultaneously denote O;
wherein
R^{14a}, R^{14b}, R^{16a} and R^{16b} each independently of the others denotes H; F; Cl; OH; C₁₋₆-alkyl; O-C₁₋₆-alkyl; C₃₋₈-cycloalkyl; aryl or heteroaryl; C₃₋₈-cycloalkyl, aryl or heteroaryl bonded *via* a C₁₋₆-alkylene group;
and/or in each case R^{14a} and R^{14b} together can represent =O and/or in each case R^{16a} and R^{16b} together can represent =O;
R¹⁵ and R¹⁷ each independently of the other represents H; C₁₋₆-alkyl; C₃₋₈-cycloalkyl, aryl or heteroaryl; C₃₋₈-cycloalkyl, aryl or heteroaryl bonded *via* a C₁₋₆-alkylene group;
Z in the general formula II represents CR^{18a}R^{18b}, NR¹⁹ or O;
or
Z in the general formula II, in the case where X represents O and f represents 0, denotes -(C(R¹²⁴)-C(R¹²⁵))-, wherein
R¹²⁴ and R¹²⁵, together with the carbon atoms linking them, form a fused aryl or heteroaryl; or
Z in the general formula II, in the case where X represents O and f represents , denotes =(N-(CR¹²⁶))-, wherein the N atom is singly bonded to the O atom, and
R¹²⁶ represents H; C₁₋₆-alkyl; C₃₋₈-cycloalkyl; aryl or heteroaryl; C₃₋₈-cycloalkyl, aryl or heteroaryl bonded *via* a C₁₋₆-alkylene group;
Z in the general formula III represents CR^{18a}R^{18b}, NR¹⁹, O, S, S(=O) or S(=O)₂;
wherein
R^{18a} represents H; C₁₋₆-alkyl; C₃₋₈-cycloalkyl; aryl or heteroaryl; C₃₋₈-cycloalkyl, aryl or heteroaryl bonded *via* a C₁₋₆-alkylene group;
or R^{18a} represents a group of the general formula IV: 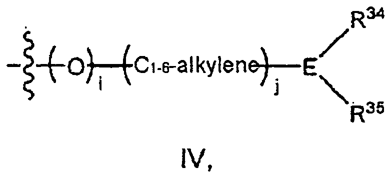 wherein
i and j each independently of the other represents 0 or 1;
E represents N or CH,
with the proviso that when i represents 1 and j represents 0, E represents CH;
R³⁴ and R³⁵ each independently of the other denotes H; C₁₋₆-alkyl; C₃₋₈-cycloalkyl; aryl or heteroaryl; aryl, heteroaryl or C₃₋₈-cycloalkyl bonded *via* a C₁₋₃-alkylene group;
or R³⁴ and R³⁵, with the inclusion of E, form a 5- or 6-membered aryl or heteroaryl;
or R³⁴ and R³⁵, with the inclusion of E, form a saturated heterocycle of the general formula V: 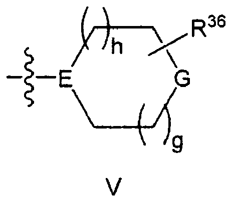 wherein
h and g independently of one another denote 0, 1 or 2;
G represents CR^{37a}R^{37b}, NR³⁸, O, S, S=O or S(=O)₂, with the proviso that when E represents CH, G does not represent CR^{17a}R^{37b};
R³⁶ represents from 0 to 4 substituents each selected independently of any others from F; Cl; Br; I; OH; SH; =O; O-C₁₋₆-alkyl; C₁₋₆-alkyl; C₃₋₈-cycloalkyl; aryl or heteroaryl; C₃₋₈-cycloalkyl, aryl or heteroaryl bonded *via* a C₁₋₆-alkylene group;
and/or two adjacent substituents R³⁶ together represent a fused aryl or heteroaryl;
R^{37a} and R^{37b} each independently of the other denotes H; F; Cl; Br; I; OH; SH; =O; O-C₁₋₆-alkyl; C₁₋₆-alkyl; C₃₋₈-cycloalkyl; aryl or heteroaryl; C₃₋₈-cycloalkyl, aryl or heteroaryl bonded *via* a C₁₋₆-alkylene group;
R³⁸ represents H; C₁₋₆-alkyl; C₃₋₈-cycloalkyl; aryl or heteroaryl; aryl, heteroaryl or C₃₋₈-cycloalkyl bonded *via* a C₁₋₃-alkylene group;
wherein
R^{18b} represents H; OH; C₁₋₆-alkyl; C₃₋₈-cycloalkyl; O-C₁₋₆-alkyl; O-(C₃₋₈-cycloalkyl); (C₁₋₆-alkylene)-O-C₁₋₆-alkyl; (C₁₋₆-alkylene)-O-(C₃₋₈-cycloalkyl); aryl or heteroaryl; O-aryl or O-heteroaryl; aryl, O-aryl, heteroaryl or O-heteroaryl bonded *via* C₁₋₆-alkylene;
or R^{18b} represents a group of the general formula VI: 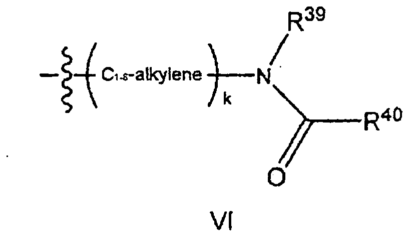 wherein
k represents 0 or 1;
R³⁹ represents H; C₁₋₆-alkyl; C₃₋₈-cycloalkyl; aryl or heteroaryl; C₃₋₈-cycloalkyl, aryl or heteroaryl bonded via a C₁₋₃-alkylene group;
R⁴⁰ represents C₁₋₆-alkyl; C₃₋₈-cycloalkyl; aryl or heteroaryl; C₃₋₈-cycloalkyl, aryl or heteroaryl bonded *via* a C₁₋₆-alkylene group;
or
R³⁹ and R⁴⁰, together with the N-C(=O) group linking them, form a ring of the general formula VII: 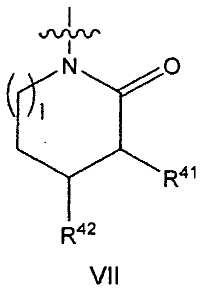 wherein
I represents 0, 1 or 2 and R⁴¹ and R⁴², together with the carbon atoms linking them, form a fused aryl or heteroaryl;
wherein R¹⁹ represents H; or (P)_{z}-R²²,
wherein
z represents 0 or 1;
P represents (C=O), S(=O)₂ or C(=O)-N(R²⁴); wherein the nitrogen atom in the C(=O)-N(R²⁴) group is linked to R²²; wherein
R²⁴ represents H; C₁₋₆-alkyl; C₃₋₈-cycloalkyl; aryl, heteroaryl or C₃₋₈-cycloalkyl bonded *via* a C₁₋₃-alkylene group;
R²² represents C₁₋₆-alkyl; aryl or heteroaryl; aryl or heteroaryl bonded *via* a C₁₋₆-alkylene group; or
R²² represents a group of the general formula VIII: 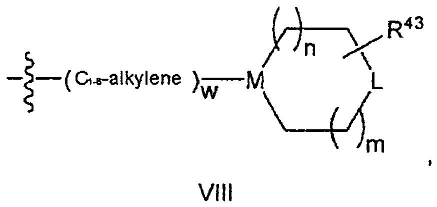 wherein
n represents 0, 1 or 2;
m represents 0, 1 or 2;
w represents 0 or 1,
M represents CH or N;
with the proviso that when P represents C(=O)-NR²⁴ and w represents 0, M represents CH; and
with the proviso that when z and w simultaneously represent 0, M represents CH;
L represents CR^{44a}R^{44b}, NR⁴⁵,O, S, S=O or S(=O)₂;
R⁴³ represents from 0 to 4 substituents each selected independently of any others from F; Cl; OH; =O; C₁₋₆-alkyl; O-C₁₋₆-alkyl; C₃₋₈-cycloalkyl; aryl or heteroaryl; C₃₋₈-cycloalkyl, aryl or heteroaryl bonded *via* a C₁₋₆-alkylene group;
and/or two of the 0 to 4 radicals R⁴³ that are adjacent together represent a fused aryl or heteroaryl;
R^{44a} and R^{44b} each independently of the other represents H; F; Cl; Br; I; OH; C₁₋₆-alkyl; O-C₁₋₆-alkyl; C₃₋₈-cycloalkyl; aryl or heteroaryl; C₃₋₈-cycloalkyl, aryl or heteroaryl bonded *via* a C₁₋₆-alkylene group;
or R^{44a} and R^{44b} together can represent =O;
R⁴⁵ represents H; C₁₋₆-alkyl; C₃₋₈-cycloalkyl; aryl or heteroaryl; aryl, heteroaryl or C₃₋₈-cycloalkyl bonded via a C₁₋₃-alkylene group;
wherein the above-mentioned radicals C₁₋₆-alkyl, C₁₋₃-alkylene, C₁₋₆-alkylene, C₂₋₆-alkenylene, C₂₋₆-alkynylene, C₃₋₆-cycloalkyl, C₃₋₈-cycloalkyl, aryl and heteroaryl can in each case be unsubstituted or mono- or poly-substituted by identical or different radicals; the above-mentioned radicals C₁₋₆-alkyl, C₁₋₃-alkylene, C₁₋₆-alkylene, C₂₋₆-alkenylene and C₂₋₆-alkynylene can in each case be branched or unbranched;
in the form of an individual enantiomer or of an individual diastereoisomer, of the racemate, of the enantiomers, of the diastereoisomers, mixtures of the enantiomers and/or diastereoisomers, as well as in each case in the form of their bases and/or physiologically acceptable salts.

2. The compound as claimed in claim 1, wherein
W¹ and W³ represent N and W² represents CR⁶⁰; or
W¹ and W² represent N and W³ represents CR⁶⁰, or
W¹, W² and W³ represent N.

3. The compound as claimed in claim 1 or 2, wherein
V represents O.

4. The compound as claimed in any one of claims 1 to 3, wherein
R¹ represents phenyl, naphthyl, indolyl, benzofuranyl, benzothiophenyl (benzothienyl); benzooxazolyl, benzooxadiazolyl, pyrrolyl, furanyl, thienyl, pyridinyl, pyridazinyl, pyrimidinyl, pyrazinyl, imidazothiazolyl, carbazolyl, dibenzofuranyl, dibenzothiophenyl (dibenzothienyl) or CH(phenyl)₂, preferably phenyl, naphthyl, benzothiophenyl, benzooxadiazolyl, thiophenyl, pyridinyl, imidazothiazolyl or dibenzofuranyl, particularly preferably phenyl or naphthyl, in each case
unsubstituted or mono- or poly-substituted by identical or different substituents, wherein the substituents are preferably selected from -O-C₁₋₃-alkyl, C₁₋₆-alkyl, F, Cl, Br, I, CF₃, OCF₃, OH, SH, phenyl, naphthyl, furyl, thiazolyl, thienyl and pyridinyl.

5. The compound as claimed in any one of claims 1 to 4, wherein in the general formula I the partial structure (Ac I): 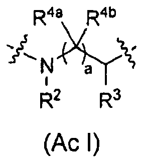 represents: 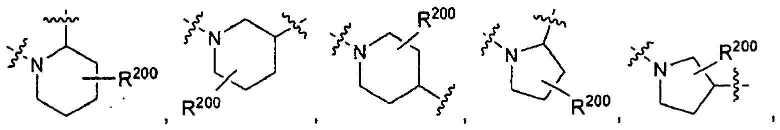 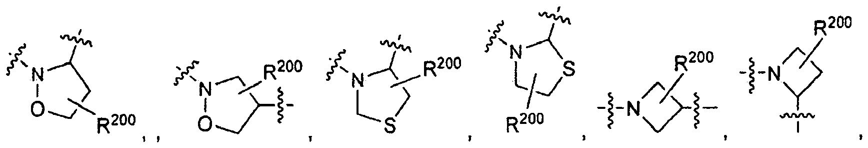 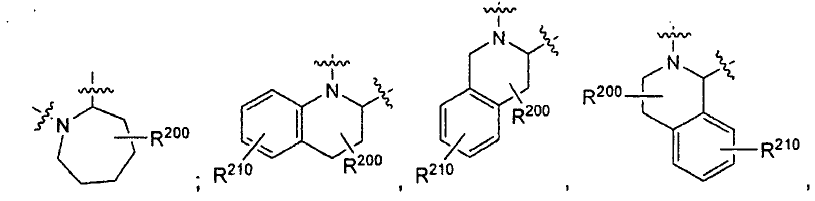 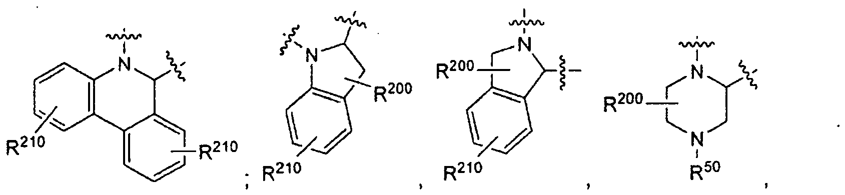 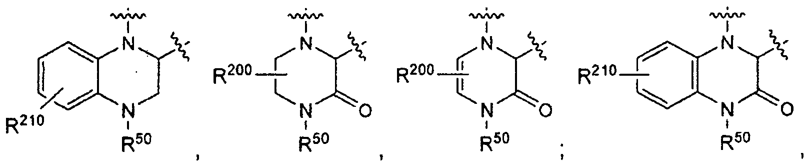 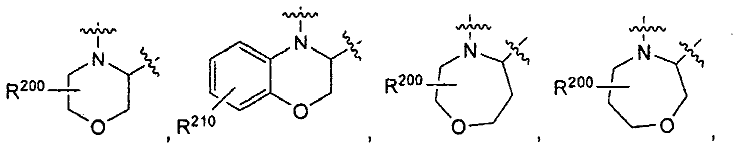 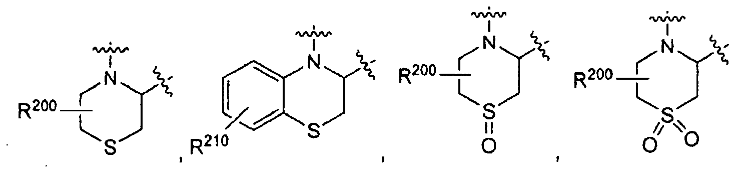 or 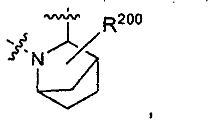 wherein
R²⁰⁰ represents from 0 to 4 substituents selected independently of one another from F, Cl, -CF₃, =O, -O-CF₃, -OH, -O-C₁₋₆-alkyl and C₁₋₆-alkyl, preferably represents F or CF ₃, or two of the radicals R²⁰⁰ represent a fused aryl, in particular a benzo group,
R²¹⁰ represents from 0 to 4 substituents selected independently of one another from -O-C₁₋₃-alkyl, C₁₋₆-alkyl, F, Cl, Br, I, CF₃, OCF₃, OH, SH, phenyl, naphthyl, furyl, thienyl and pyridinyl, preferably methyl, methoxy, O-CF₃, CF₃, F, Cl and Br.

6. The compound as claimed in any one of claims 1 to 5 wherein
R² represents H, C₁₋₆-alkyl, C₃₋₆-cycloalkyl, aryl, or a C₃₋₆-cycloalkyl or aryl bonded *via* a C₁₋₃-alkylene group, in particular H, methyl, ethyl, n-propyl, isopropyl, n-butyl, isobutyl, tert-butyl, cyclopropyl, phenyl, pyridinyl, or phenyl or pyridinyl bonded *via* a C₁₋₃-alkylene group; in each case unsubstituted or mono- or poly-substituted by identical or different substituents;
and
R³ represents H, F, Cl, -CF₃, -OH, -O-C₁₋₆-alkyl, C₁₋₆-alkyl or aryl; or an aryl bonded *via* a C₁₋₃-alkylene group, in each case unsubstituted or mono- or poly-substituted by identical or different radicals.

7. The compound as claimed in any one of claims 1 to 6, wherein
a+b=1.

8. A substituted compound as claimed in one or more of claims 1 to 7,
(a1) the general formula II assumes the following partial structure IIa: 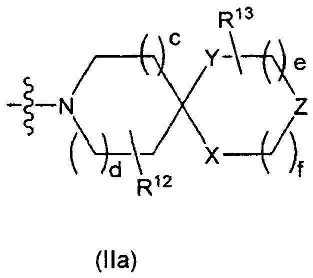 or
(a2) the general formula III assumes one of the following partial structures IIIa or IIIb:

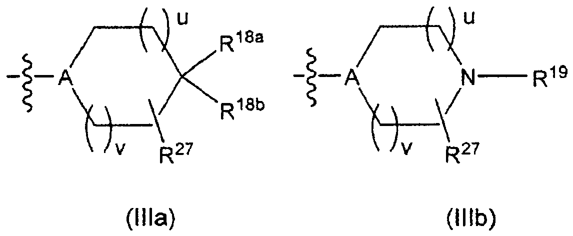

9. A substituted compound as claimed in claim 8, wherein
(a1) the partial structure of formula IIa assumes the following partial structure IIb: 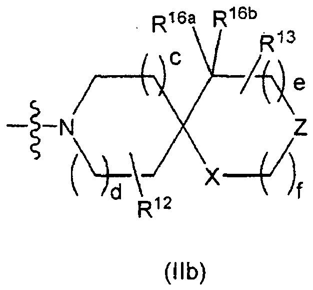 or
(a2) the partial structures of formulae IIIa and IIIb assume one of the following partial structures IIIc, IIId or IIIe:

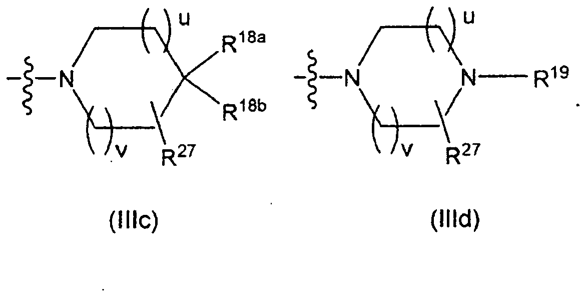

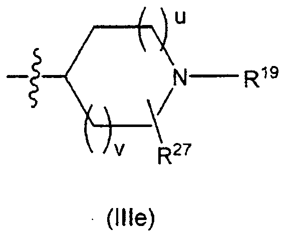

10. A substituted compound as claimed in claim 9, wherein
(a1) the partial structure of formula IIa assumes the partial structure IIb,
R⁸ represents H, C₁₋₆-alkyl, C₃₋₆-cycloalkyl, in each case unsubstituted or mono- or poly-substituted by identical or different substituents, and
R^{9a} and R^{9b} each represents H;
and/or
(a2) the partial structures of formulae IIIa and IIIb assume one of the partial structures IIIc or IIId, and s and t each represents 0;
and/or
(a3) the partial structures of formulae IIIa and IIIb assume one of the partial structures IIIc or IIId and two of the substituents R²⁷ together represent a C₁₋₃-alkylene bridge, so that the ring shown in partial structure IIIc or IIId assumes a bicyclically bridged form, and
each of s and t is 0;
and/or
(a4) the partial structures of formulae IIIa and IIIb assume one of the partial structures IIIc or IIIe, s represents 1 and t represents 1, 2 or 3, and R⁸ represents H, C₁₋₆-alkyl or C₃₋₆-cycloalkyl, in each case unsubstituted or mono- or poly-substituted by identical or different substituents.

11. A substituted compound as claimed in claim 10, wherein
(a1) partial structure IIb assumes the following partial structure IIc: 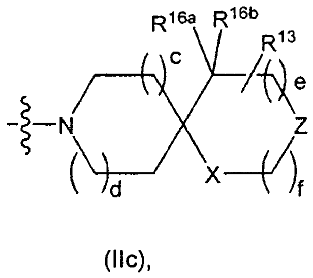 and wherein s and t each denotes 0;
and/or
(a2) the partial structures IIIc or IIId assume one of the following partial structures IIIf or IIIg: 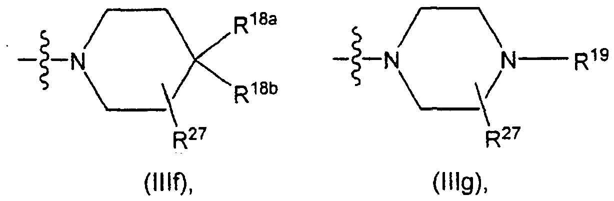 wherein
R²⁷ represents H or methyl and/or two adjacent substituents R²⁷ form a fused aryl or heteroaryl, in particular a benzo group;
and/or
(a3) in the compounds the partial structures IIIc or IIId represent one of the following radicals A to H: 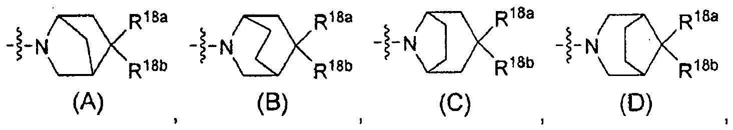 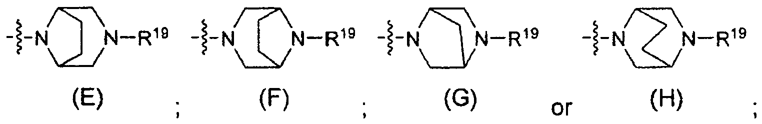 and/or
(a4) in the compounds the partial structures IIIc or IIIe represent a group of one of formulae IIIh or IIIi: 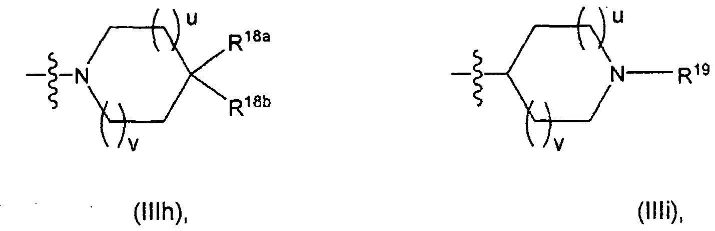
and R^{9a} and R^{9b} each represents H.

12. A substituted compound as claimed in *claim 11, wherein
(a1) in the partial structure IIc the radicals R^{16a} and R^{16b} each represents H or together form =O;
R¹³ represents aryl or heteroaryl, and/or two of the substituents R¹³ together form =O
and/or two adjacent substituents R¹³ form a fused aryl or heteroaryl, in particular a benzo group,
and/or
(a2) in the partial structures IIIf or IIIg
R^{18a} represents H; C₁₋₆-alkyl; C₃₋₈-cydoalkyl, -NH(C₁₋₆-alkyl), -N(C₁₋₆-alkyl)₂, phenyl, pyridyl, pyrimidinyl, thiazolyl, imidazolyl, triazolyl or thienyl, in each case unsubstituted or mono- or poly-substituted; phenyl, pyridyl, pyrimidinyl, imidazolyl, triazolyl or thienyl bonded *via* a -(O)₀₋₁-C₁₋₆-alkylene group and in each case unsubstituted or mono- or poly-substituted;
or
R^{18a} represents the radical of the general formula VIIa 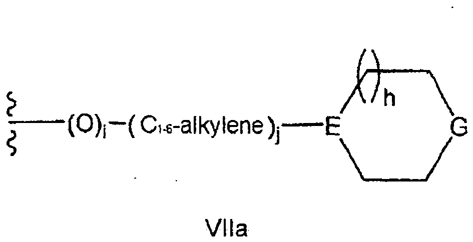 wherein
i represents 0 or 1;
j represents 0 or 1;
h represents 0 or 1;
E represents N or CH; with the proviso that when i represents 1 and j represents 0, E represents CH;
G represents CR^{37a}R^{37b} or NR³⁸;
wherein R^{37a} and R^{37b} independently of one another represent H; F or C₁₋₆-alkyl, in each case unsubstituted or mono- or poly-substituted by identical or different substituents;
R³⁸ represents H; C₁₋₆-alkyl, C₃₋₆-cycloalkyl or pyridyl;
R^{18b} represents H; OH; C₁₋₆-alkyl; phenyl, pyridyl, pyrimidinyl, imidazolyl, triazolyl, thienyl or thiazolyl; in each case unsubstituted or mono- or poly-substituted by identical or different substituents; phenyl, pyridyl, pyrimidinyl, O-phenyl, O-pyridyl, imidazolyl, triazolyl, thienyl or thiazolyl bonded *via* a C₁₋₆-alkylene group and in each case unsubstituted or mono- or poly-substituted by identical or different substituents; phenyl, pyridyl, pyrimidinyl, imidazolyl, triazolyl, thiazolyl or thienyl bridged via C₁₋₆₋alkylene-NH(C=O) and in each case unsubstituted or substituted by identical or different substituents;
R¹⁹ represents H; C₁₋₆-alkyl; C₃₋₈-cycloalkyl, or C₁₋₆-alkyl bonded *via* (C=O)₀₋₁ ; phenyl, pyridyl, thienyl, thiazolyl, triazolyl, pyrimidinyl or imidazolyl, in each case unsubstituted or mono- or poly-substituted by identical or different substituents; phenyl, pyridyl, thienyl, thiazolyl, pyrimidinyl, triazolyl or imidazolyl bonded *via* a C₁₋₆-alkylene group and in each case unsubstituted or mono- or poly-substituted by identical or different substituents;
or represents the radical of the general formula VIIIa 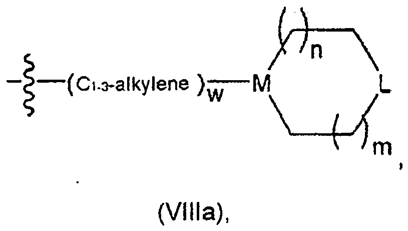 wherein
w represents 0 or 1;
n represents 0 or 1;
m represents 0 or 1;
M represents CH or N, with the proviso that when w represents 0, M represents CH;
L represents CR^{44a}R^{44b} or NR⁴⁵;
wherein R^{44a} and R^{44b} independently of one another represent H; F or C₁₋₆-alkyl, in each case unsubstituted or mono- or poly-substituted by identical or different substituents;
R⁴⁵ represents H; C₁₋₆-alkyl, C₃₋₆-cycloalkyl or pyridyl;
and/or
(a3) in the compounds the partial structures IIIc or IIId represent one of the following groups A to H: 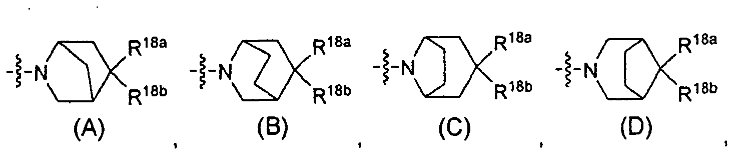 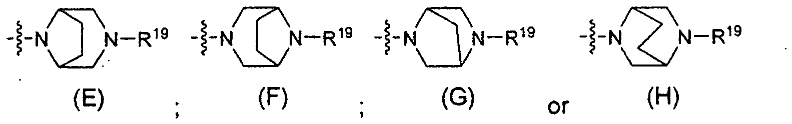 and wherein
R^{18a} represents H; C₁₋₆-alkyl; C₃₋₈-cycloalkyl, N(C₁₋₆-alkyl)₂; NH(C₁₋₆-alkyl); azetidinyl; pyrrolidinyl, piperidinyl, 4-(C₁₋₆-alkyl)-piperazinyl; phenyl, pyridyl, pyrimidinyl, imidazolyl, triazolyl, thiazolyl or thienyl, in each case unsubstituted or mono- or poly-substituted by identical or different substituents; N(C₁₋₆-alkyl)₂; NH(C₁₋₆-alkyl); azetidinyl; pyrrolidinyl, piperidinyl, 4-(C₁₋₆-alkyl)-piperazinyl; phenyl, imidazolyl, triazolyl, thienyl, thiazolyl, pyrimidinyl or pyridyl bonded via a -(O)₀₋₁-C₁₋₆-alkylene group and in each case unsubstituted or mono- or poly-substituted by identical or different substituents;
R^{18b} represents H; OH; C₁₋₆-alkyl; phenyl, pyridyl, pyrimidinyl, imidazolyl, triazolyl, thiazolyl or thienyl, in each case unsubstituted or mono- or poly-substituted by identical or different substituents; phenyl, pyridyl, pyrimidinyl, imidazolyl, triazolyl, thiazolyl or thienyl bonded *via* a C₁₋₆-alkylene group and in each case unsubstituted or mono- or poly-substituted by identical or different substituents;
R¹⁹ represents H; C₁₋₆-alkyl; C₃₋₈-cycloalkyl, phenyl, pyridyl, pyrimidinyl, thienyl, imidazolyl, thiazolyl or triazolyl, in each case unsubstituted or mono- or poly-substituted by identical or different substituents; phenyl, pyridyl, pyrimidinyl, thienyl, imidazolyl, thiazolyl or triazolyl bonded *via* a C₁₋₆-alkylene group or a (C=O) group and in each case unsubstituted or mono- or poly-substituted by identical or different substituents;
and/or
(a4) in the partial structures IIIh or IIIi
R^{18a} represents H; C₁₋₆-alkyl; C₃₋₈-cycloalkyl, N(C₁₋₆-alkyl)₂; NH(C₁₋₆-alkyl), azetidinyl; pyrrolidinyl, piperidinyl, 4-(C₁₋₆-alkyl)-piperazinyl; phenyl, pyridyl, pyrimidinyl, thienyl, imidazolyl, thiazolyl or triazolyl, in each case unsubstituted or mono- or poly-substituted by identical or different substituents; N(C₁₋₆-alkyl)₂; NH(C₁₋₆-alkyl), azetidinyl; pyrrolidinyl, piperidinyl, 4-(C₁₋₆-alkyl)-piperazinyl; phenyl, pyridyl, pyrimidinyl, thienyl, imidazolyl, thiazolyl or triazolyl bonded via a -(O)_{0/1}-C₁₋₆-alkylene group and in each case unsubstituted or mono- or poly-substituted by identical or different substituents;
R^{18b} represents H; OH; C₁₋₆-alkyl; phenyl, pyridyl, pyrimidinyl, thienyl, imidazolyl, thiazolyl or triazolyl, in each case unsubstituted or mono- or poly-substituted by identical or different substituents; phenyl, pyridyl, pyrimidinyl, thienyl, imidazolyl, thiazolyl or triazolyl bonded *via* a C₁₋₆-alkylene group and in each case unsubstituted or mono- or poly-substituted by identical or different substituents;
R¹⁹ represents H; C₁₋₆-alkyl; C₃₋₈-cycloalkyl, phenyl, pyridyl, pyrimidinyl, thienyl, imidazolyl, thiazolyl or triazolyl, in each case unsubstituted or mono- or poly-substituted by identical or different substituents; phenyl, pyridyl, pyrimidinyl, thienyl, imidazolyl, thiazolyl or triazolyl bonded *via* a C₁₋₆-alkylene group or (C=O) group and in each case unsubstituted or mono- or poly-substituted by identical or different substituents.

13. A substituted compound as claimed in claim 12, wherein
(a1) the partial structure of formula IIc can assume the following partial structures SP1 to SP34: 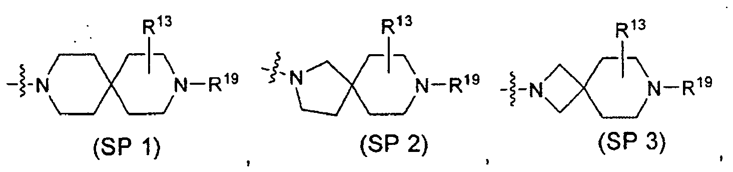 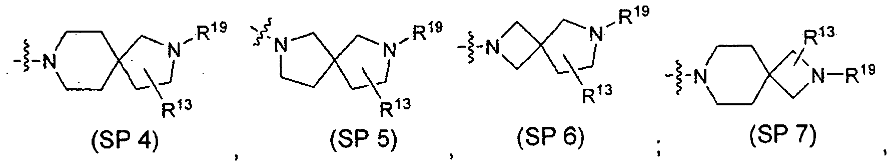 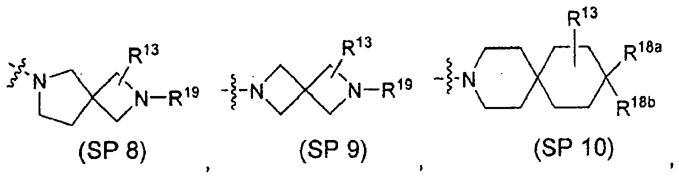 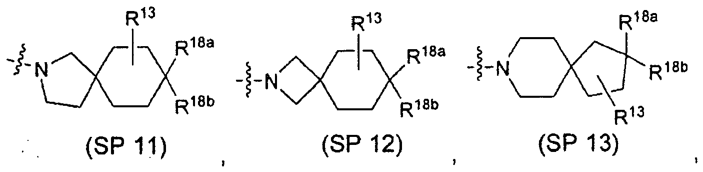 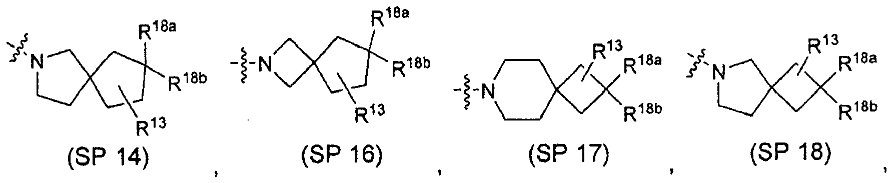 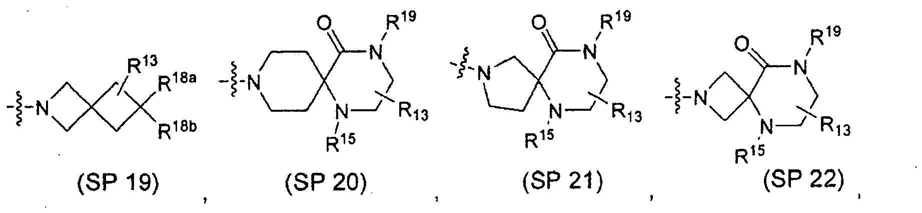 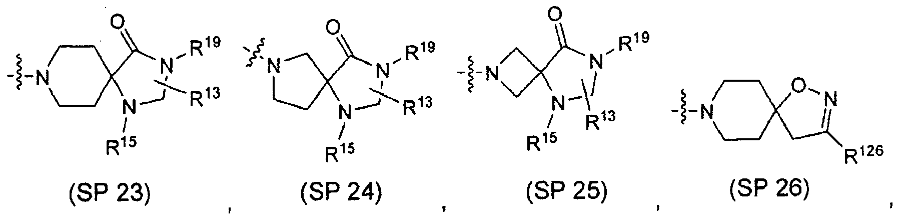 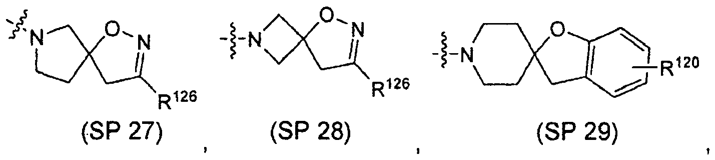 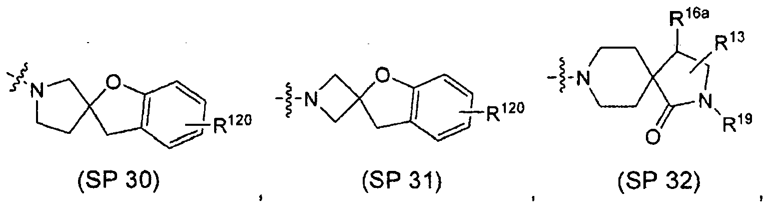 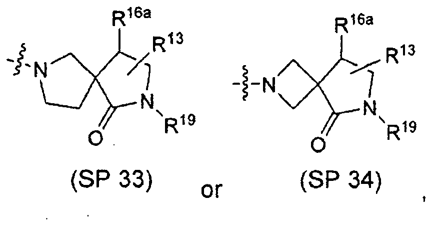 wherein
R¹³ represents H or phenyl, in each case unsubstituted or mono- or poly-substituted by identical or different substituents; and/or two of the substituents R¹³ together form =O
and/or two adjacent substituents R¹³ together form a fused aryl or heteroaryl, in particular a benzo group,
R¹⁵ represents H; C₁₋₆-alkyl; C₃₋₈-cycloalkyl, phenyl, pyridyl, pyrimidinyl, imidazolyl, triazolyl, thiazolyl or thienyl, in each case unsubstituted or mono- or poly-substituted by identical or different substituents; phenyl, pyridyl, pyrimidinyl, imidazolyl, triazolyl, thiazolyl or thienyl bonded *via* a C₁₋₆-alkylene group and in each case unsubstituted or mono- or poly-substituted by identical or different substituents;
R^{16a} represents H, C₁₋₆-alkyl, phenyl, pyridyl, pyrimidinyl, imidazolyl, triazolyl, thiazolyl or thienyl, in each case unsubstituted or mono- or poly-substituted by identical or different substituents;
R^{18a} represents H; C₁₋₆-alkyl; C₃₋₈-cycloalkyl, N(C₁₋₆-alkyl)₂; NH(C₁₋₆-alkyl), azetidinyl; pyrrolidinyl, piperidinyl, 4-(C₁₋₆-alkyl)-piperazinyl; phenyl, pyridyl, pyrimidinyl, imidazolyl, triazolyll, thiazolyl or thienyl, in each case unsubstituted or mono- or poly-substituted by identical or different substituents; N(C₁₋₆-alkyl)₂; NH(C₁₋₆-alkyl), azetidinyl; pyrrolidinyl, piperidinyl, 4-(C₁₋₆-alkyl)-piperazinyl; phenyl, pyridyl, pyrimidinyl, imidazolyl, triazolyl, thiazolyl or thienyl bonded via a -(O)_{0/1}-C₁₋₆-alkylene group and in each case unsubstituted or mono- or poly-substituted by identical or different substituents;
R^{18b} represents H; OH; C₁₋₆-alkyl; phenyl, pyridyl, pyrimidinyl, imidazolyl, triazolyl, thiazolyl or thienyl, in each case unsubstituted or mono- or poly-substituted by identical or different substituents; phenyl, pyridyl, pyrimidinyl, imidazolyl, triazolyl, thiazolyl or thienyl bonded *via* a C₁₋₆-alkylene group and in each case unsubstituted or mono- or poly-substituted by identical or different substituents;
R¹⁹ represents H; C₁₋₆-alkyl; C₃₋₈-cydoalkyl, phenyl, pyridyl, pyrimidinyl, imidazolyl, triazolyl, thiazolyl or thienyl, in each case unsubstituted or mono- or poly-substituted by identical or different substituents; phenyl, pyridyl, pyrimidinyl, imidazolyl, triazolyl, thiazolyl or thienyl bonded *via* a C₁₋₆-alkylene group or (C=O) group and in each case unsubstituted or mono- or poly-substituted by identical or different substituents;
R¹²⁰ represents H; F; Cl; OH; OCH₃, C₁₋₆-alkyl; phenyl, in each case unsubstituted or mono- or poly-substituted by identical or different substituents;
R¹²⁶ represents H; C₁₋₆-alkyl; C₃₋₆-cycloalkyl; phenyl, pyridyl, pyrimidinyl, imidazolyl, triazolyl, thiazolyl or thienyl; C₃₋₆-cycloalkyl, phenyl or pyridyl bonded *via* a C₁₋₃-alkylene group and in each case unsubstituted or mono- or poly-substituted by identical or different substituents.

14. A compound as claimed in any one of the preceding claims, wherein
in the general formula I the partial structure (B): 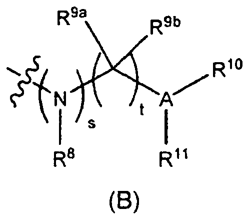 is selected from 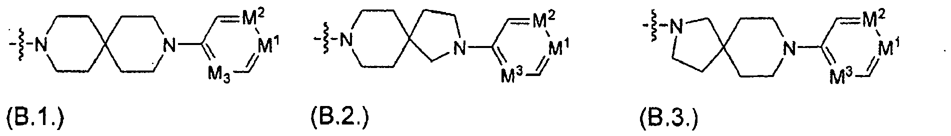 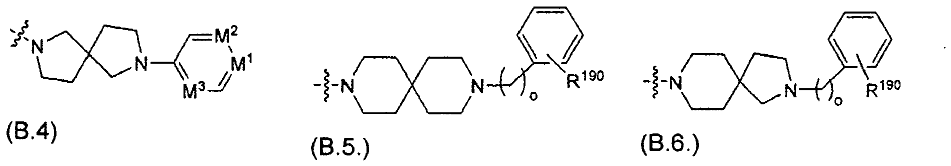 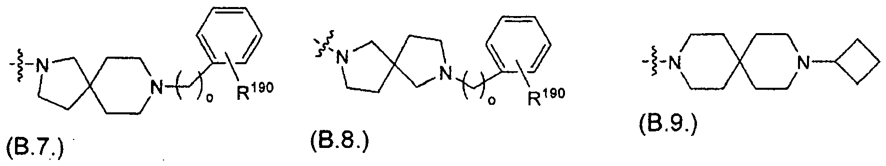 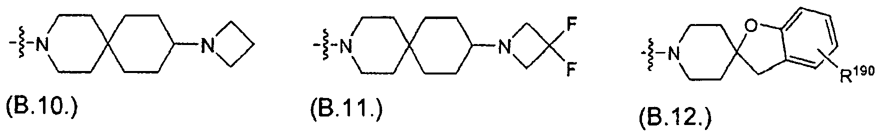 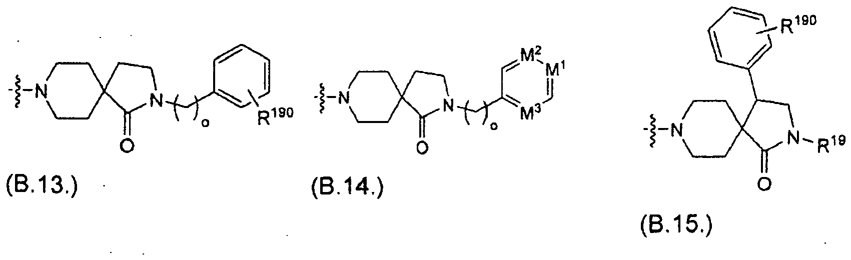 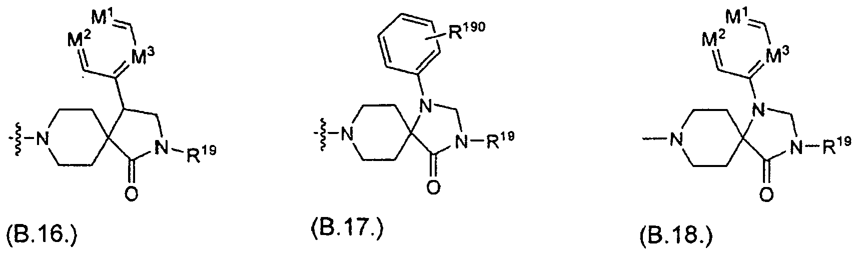 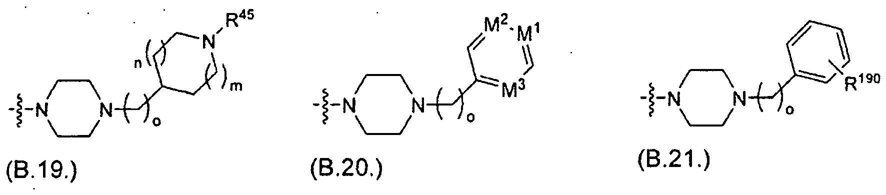 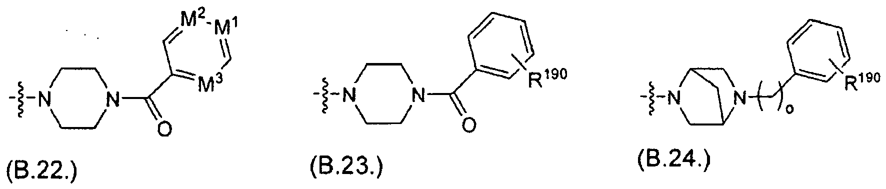 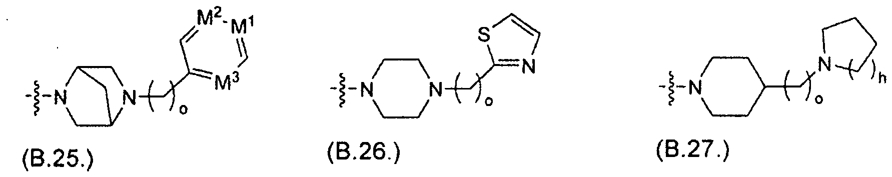 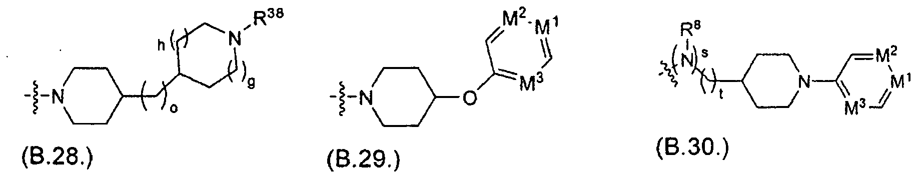 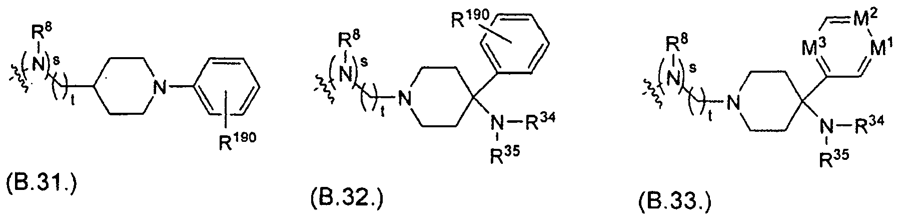 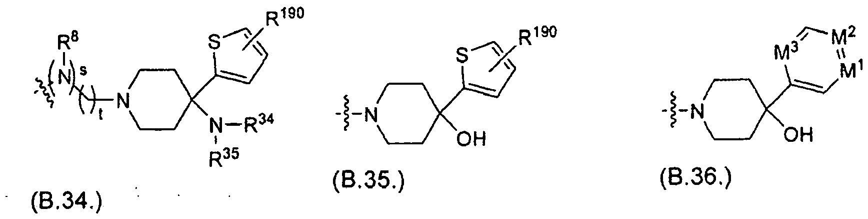 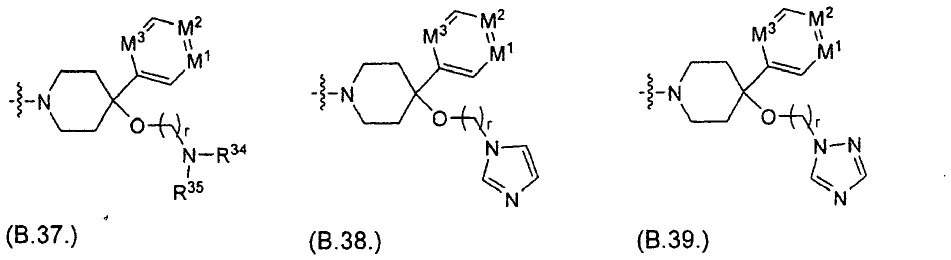 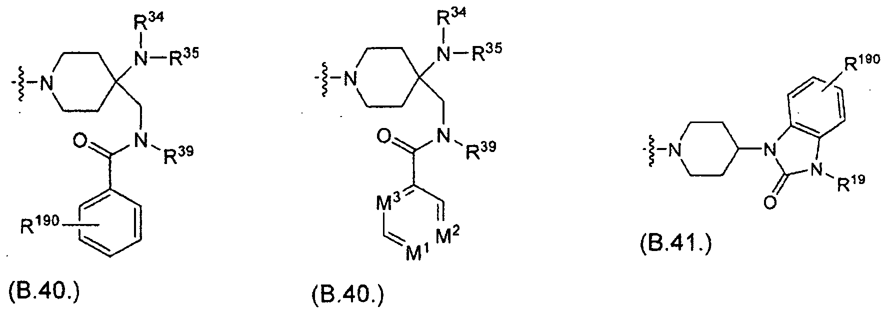 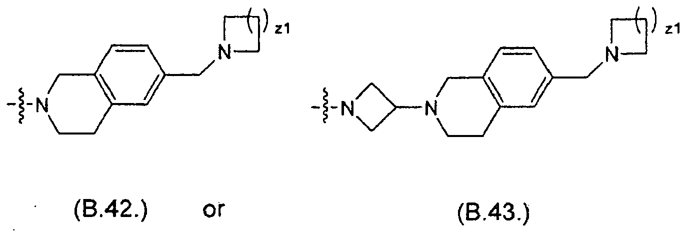 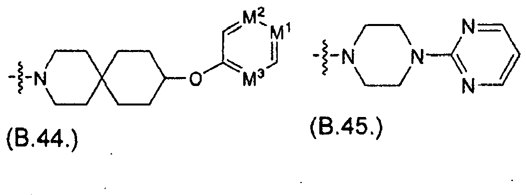 wherein
h = 0 or 1;
g = 0 or 1;
m = 0 or 1;
n = 0 or 1;
o = 0, 1, 2 or 3;
r = 1, 2 or 3, in particular 1 or 2;
s = 0 or 1;
t = 0, 1, 2 or 3, in particular 0, 1 or 2, with the proviso that when s represents 0, t likewise represents 0;
z1 = 0, 1, 2 or 3, in particular 1;
M¹, M² and M³ each independently of the others can represent N or CH, wherein one variable from M¹, M² and M³ represents N and the other two represent CH;
R⁸ represents H; C₁₋₆-alkyl, in particular methyl, ethyl, n-propyl, isopropyl, n-butyl, sec-butyl, isobutyl and tert-butyl; C₃₋₆-cycloalkyl, in particular cyclopropyl; in each case unsubstituted or mono- or poly-substituted by identical or different substituents;
R¹⁹ is selected from H; C₁₋₆-alkyl, in particular methyl, ethyl, n-propyl, isopropyl, n-butyl, sec-butyl, isobutyl and tert-butyl; C₃₋₆-cycloalkyl, in particular cyclopropyl; in each case unsubstituted or mono- or poly-substituted by identical or different substituents.
R³⁴ and R³⁵ are independently of one another preferably methyl or ethyl or, together with the N atom linking them, form an azetidinyl; pyrrolidinyl, piperidinyl, 4-(C₁₋₆-alkyl)-piperazinyl group; in each case unsubstituted or mono- or poly-substituted by identical or different substituents;
R³⁸ represents H, C₁₋₆-alkyl, C₃₋₆-cycloalkyl or pyridyl;
R³⁹ is selected from H; C₁₋₆-alkyl, in particular methyl, ethyl, n-propyl, isopropyl, n-butyl, sec-butyl, isobutyl and tert-butyl; C₃₋₆-cycloalkyl, in particular cyclopropyl, in each case unsubstituted or mono- or poly-substituted by identical or different substituents; and
R⁴⁵ represents H, C₁₋₆-alkyl, C₃₋₆-cycloalkyl or pyridyl;
R¹⁹⁰ represents from 0 to 4 substituents selected independently of one another from F, Cl, O-CF₃, CF₃ and CN.

15. A compound as claimed in any one of the preceding claims, represented by the general formulae C1 to C21 shown hereinbelow: 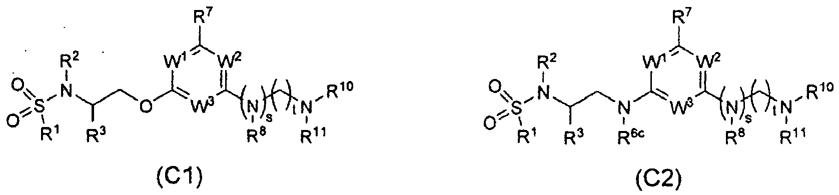 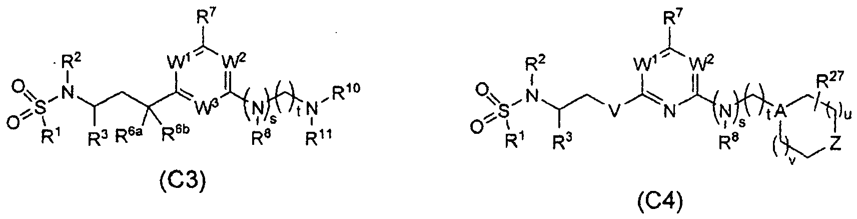 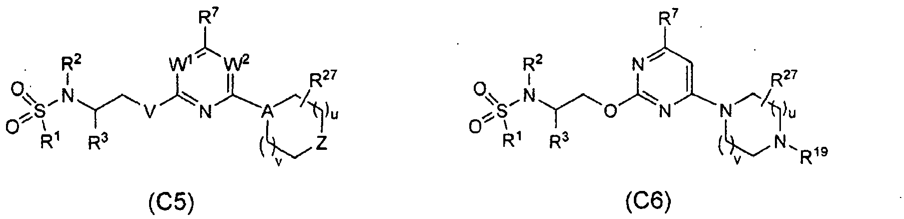 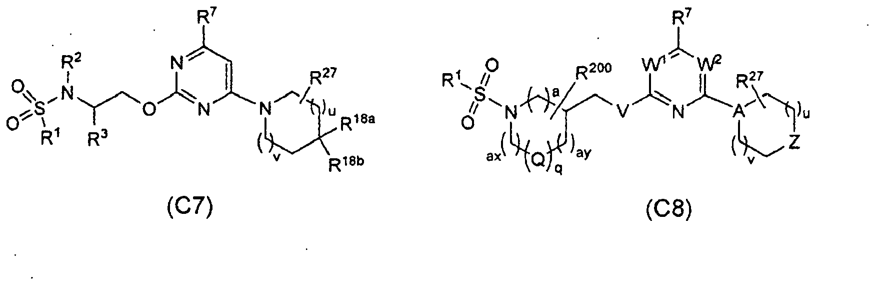 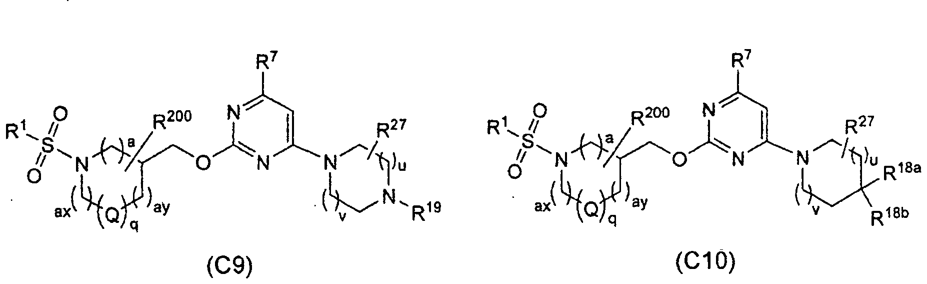 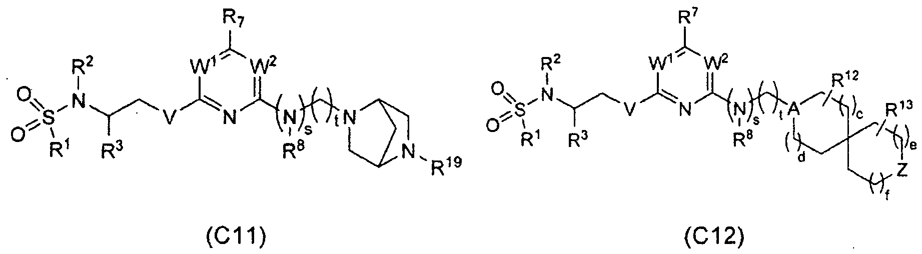 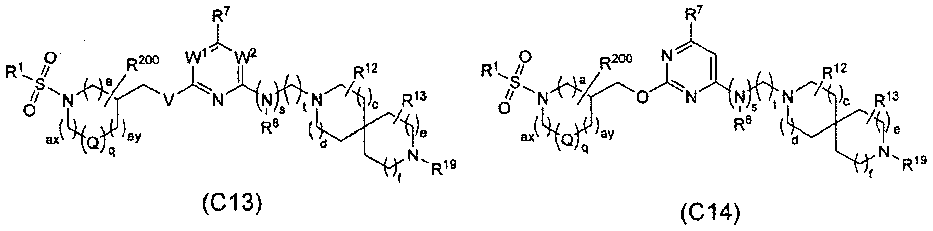 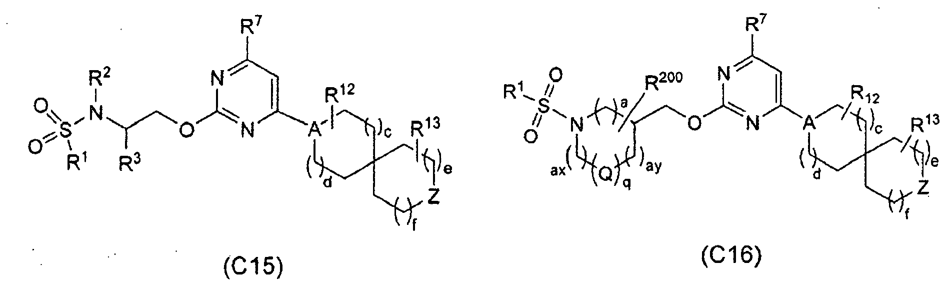 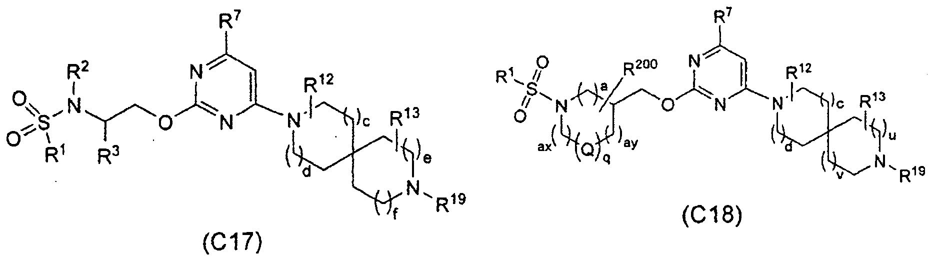 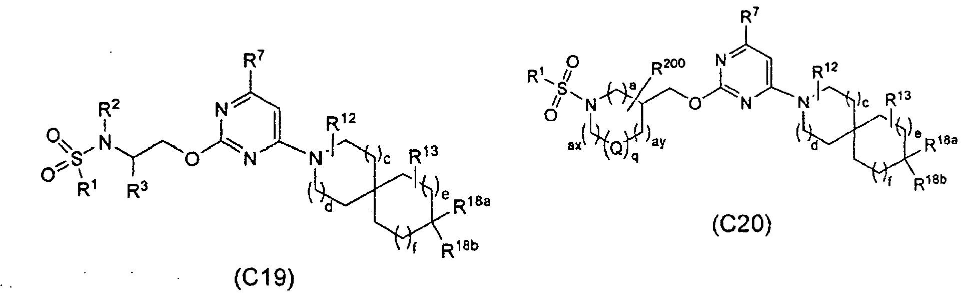 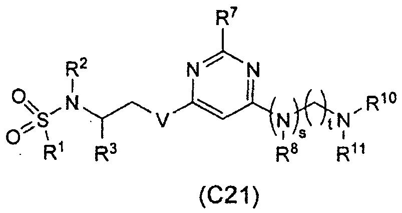 and wherein
q represents 0 or 1,
a represents 0, 1 or 2;
ax represents 0, 1, 2 or 3;
ay represents 0, 1 or 2;
q represents 0 or 1;
with the proviso that a + ax + ay + q ≥ 2;
Q represents CH₂, NR⁵⁰, O, S, S=O or S(=O)₂,
and all other radicals, variables and indices have the meanings described in claims 1 to 13 hereinbefore.

16. A compound as claimed in any one of the preceding claims, wherein the compound is selected from:
4-methoxy-N,2,6-trimethyl-N-[2-[[4-[4-(2-1-pyrrolidinylethyl)-1-piperidinyl]-2-pyrimidinyl]oxy]ethyl]benzenesulfonamide,
4-methoxy-N,2,6-trimethyl-N-[2-[[4-[4-(4-pyridyl)-1-piperazinyl]-2-pyrimidinyl]oxy]ethyl]benzenesulfonamide,
N-[2-[[4-[2-(4-dimethylamino-4-phenyl-1-piperidinyl)ethyl-methylamino]-2-pyrimldinyl]oxy]ethyl]-4-methoxy-N,2,6-trimethylbenzenesulfonamide,
2-[[1-(4-methoxy-2,6-dimethylphenyl)sulfonyl-2-piperidinyl]methoxy]-4-[4-(3-pyridyl)-4-(2-1-pyrrolidinylethoxy)-1-piperidinyl]pyrimidine,
N-cyclopropyl-4-methoxy-2,6-dimethyl-N-[2-[[4-[9-(4-pyridyl)-3,9-diazaspiro[5.5]undecan-3-yl]-2-pyrimidinyl]oxy]ethyl]benzenesulfonamide hydrochloride.
N-cyclopropyl-4-methoxy-2,6-dimethyl-N-[2-[[4-[2-[1-(4-pyridyl)-4-piperidinyl]ethylamino]-2-pyrimidinyl]oxy]ethyl]benzenesulfonamide,
N-cyclopropyl-4-methoxy-2,6-dimethyl-N-[2-[[4-[4-(3-pyridyl)-4-(2-1-pyrrolidinylethoxy)-1-piperidinyl]-2-pyrimidinyl]oxy]ethyl]benzenesulfonamide,
N-cyclopropyl-N-[2-[[4-[4-hydroxy-4-(3-pyridyl)-1-piperidinyl]-2-pyrimidinyl]oxy]ethy]-4-methoxy-2,6-dimethylbenzenesulfonamide,
2-chloro-N-cyclopropyl-6-methyl-N-[2-[[4-[9-(4-pyridyl)-3,9-diazaspiro[5.5]undecan-3-yl]-2-pyrimidinyl]oxy]ethyl]benzenesulfonamide,
N-cyclopropyl-N-[2-[[4-[9-(4-pyridyl)-3,9-diazaspiro[5.5]undecan-3-yl]-2-pyrimidinyl]oxy]ethyl]-2-(trifluoromethyl)benzenesulfonamide,
3-[2-[[(2S,4R)-4-fluoro-1-(4-methoxy-2,6-dimethylphenyl)sulfonyl-2-pyrrolidinyl]methoxy]-4-pyrimidinyl]-9-(4-pyridyl)-3,9-diazaspiro[5.5]undecane,
N-cyclopropyl-4-methoxy-2,6-dimethyl-N-[2-[[4-[4-(4-pyridyloxy)-1-piperidinyl]-2-pyrimidinyl]oxy]ethyl]benzenesulfonamide,
N-[2-[[4-[6-(1-azetidinylmethyl)-3,4-dihydro-1H-isoquinolin-2-yl]-2-pyrimidinyl]oxy]ethyl]-N-cyclopropyl-4-methoxy-2,6-dimethylbenzenesulfonamide,
N-cyclopropyl-4-methoxy-2,6-dimethyl-N-[2-[[4-[8-(4-pyridyl)-3,8-diazaspiro[4.4]nonan-3-yl]-2-pyrimidinyl]oxy]ethyl]benzenesulfonamide,
N-[2-[[4-[9-(1-azetidinyl)-3-azaspiro[5.5]undecan-3-yl]-2-pyrimidinyl]oxy]ethyl]-N-cyclopropyl-4-methoxy-2,6-dimethylbenzenesulfonamide hydrochloride,
N-cyclopropyl-4-methoxy-2,6-dimethyl-N-[2-[[4-[9-(4-pyridyloxy)-3-azaspiro[5.5]undecan-3-yl]-2-pyrimidinyl]oxy]ethyl]benzenesulfonamide,
N-cyclopropyl-N-[2-[[4-[9-(3,3-difluoro-1-azetidinyl)-3-azaspiro[5.5]undecan-3-yl]-2-pyrimidinyl]oxy]ethyl]-4-methoxy-2,6-dimethylbenzenesulfonamide,
3-[2-[[1-(4-methoxy-2,6-dimethylphenyl)sulfonyl-3-azetidinyl]oxy]-4-pyrimidinyl]-9-(4-pyridyl)-3,9-diazaspiro[5.5]undecane,
N-cyclopropyl-4-methoxy-2,6-dimethyl-N-[2-[[4-[8-(4-pyridyl)-3,8-diazaspiro[4.5]decan-3-yl]-2-pyrimidinyl]oxy]ethyl]benzenesulfonamide,
N-[2-[[4-[3-[6-(1-azetidinylmethyl)-3,4-dihydro-1H-isoquinolin-2-yl]-1-azetidinyl]-2-pyrimidinyl]oxy]ethyl]-N-cyclopropyl-4-methoxy-2,6-dimethylbenzenesulfonamide, 2,6-dichloro-N-cyclopropyl-3-methyl-N-[2-[4-(9-pyridin-4-yl-3,9-diazaspiro[5.5]undecan-3-yl)-pyrimidin-2-yl]oxy-ethy]-benzenesulfonic acid amide
4-methoxy-2,6-dimethyl-N-[1-[[4-(9-pyridin-4-yl-3,9-diazaspiro[5.5]undecan-3-yl)-pyrimidin-2-yl]oxy-methyl]-cyclobutyl]-benzenesulfonic acid amide
N-cyclopropyl-4-methoxy-2,6-dimethyl-N-[2-[4-[9-pyridin-3-yl-9-(2-pyrrolidin-1-yl-ethoxy)-3-azaspiro[5.5]undecan-3-yl]-pyimidin-2-yl]oxy-ethyl]-benzenesulfonic acid amide
N-[1,1-dimethyl-2-[4-(9-pyridin-4-yl-3,9-diazaspiro[5.5]undecan-3-yl)-pyrimidin-2-yl]oxy-ethyl]-4-methoxy-2,6-dimethyl-benzenesulfonic acid amide
N-cyclopropyl-4-methoxy-2,6-dimethyl-N-[3-[4-(9-pyridin-4-yl-3,9-diazaspiro[5.5]undecan-3-yl)-pyrimidin-2-yl]oxy-propyl]-benzenesulfonic acid amide
3-[2-[[(2S)-1-[(4-methoxy-2,6-dimethyl-phenyl)sulfonyl]-piperidin-2-yl]-methoxy]-pyrimidin-4-yl]-9-pyridin-4-yl-3,9-diazaspiro[5.5]undecane
4-methoxy-N,2,6-trimethyl-N-[2-[[2-[4-(2-1-pyrrolidinylethyl)-1-piperidinyl]-4-pyrimidinyl]oxy]ethyl]benzenesulfonamide,
4-methoxy-N,2,6-trimethyl-N-[2-[[2-[4-(4-pyridyl)-1-piperazinyl]-4-pyrimidinyl]oxy]ethyl]benzenesulfonamide,
N-[2-[[2-[2-(4-dimethylamino-4-phenyl-1-piperidinyl)ethyl-methylamino]-4-pyrimidinyl]oxy]ethyl]-4-methoxy-N,2,6-trimethylbenzenesulfonamide,
N-[2-(4-dimethylamino-4-phenyl-1-piperidinyl)ethyl]-4-[[1-(4-methoxy-2,6-dimethylphenyl)sulfonyl-2-piperidinyl]methoxy]-N-methyl-2-pyrimidineamine,
N-cyclopropyl-4-methoxy-2,6-dimethyl-N-[2-[[2-[4-(4-pyridyloxy)-1-piperidinyl]-4-pyrimidinyl]oxy]ethyl]benzenesulfonamide,
2-chloro-N-cyclopropyl-6-methyl-N-[2-[[2-(9-(4-pyridyl)-3,9-diazaspiro[5.5]undecan-3-yl]-4-pyrimidinyl]oxy]ethyl]benzenesulfonamide,
3-[4-[[1-(4-methoxy-2,6-dimethylphenyl)sulfonyl-3-azetidinyl]oxy]-2-pydmidinyl]-9-(4-pyridyl)-3,9-diazaspiro[5.5]undecane,
N-cyclopropyl-4-methoxy-2,6-dimethyl-N-[2-[[6-[9-(4-pyridyl)-3,9-diazaspiro[5.5]undecan-3-yl]-4-pyrimidinyl]oxy]ethyl]benzenesulfonamide,
N-cyclopropyl-4-methoxy-2,6-dimethyl-N-[2-[methyl-[6-(9-pyridin-4-yl-3,9-diazaspiro[5.5]undecan-3-yl)-pyrazin-2-yl]-amino]-ethyl]-benzenesulfonic acid amide
N-cyclopropyl-4-methoxy-2,6-dimethyl-N-[2-[methyl-[6-(9-pyridin-4-yl-3,9-diazaspiro[5.5]undecan-3-yl)-pyrimidin-4-yl]-amino]-ethyl]-benzenesulfonic acid amide
N-cyclopropyl-4-methoxy-2,6-dimethyl-N-[2-[methyl-[2-(9-pyridin-4-yl-3,9-diazaspiro[5.5]undecan-3-yl)-pyrimidin-4-yl)-amino]-ethyl)-benzenesulfonic acid amide
N-cyclopropyl-4-methoxy-2,6-dimethyl-N-[2-[methyl-[4-(9-pyridin-4-yl-3,9-diazaspiro[5.5]undecan-3-yl)-pyrimidin-2-yl]-amino]-ethyl]-benzenesulfonic acid amide
N-cyclopropyl-4-methoxy-2,6-dimethyl-N-[3-[6-(9-pyridin-4yl-3,9-diazaspiro[5.5]undecan-3-yl)-pyrazin-2-yl]-propyl]-benzenesulfonic acid amide
N-cyclopropyl-4-methoxy-2,6-dimethyl-N-[3-[2-(9-pyridin-4-yl-3,9-diazaspiro[5.5]undecan-3-yl)-pyrimidin-4-yl]-propyl]-benzenesulfonic acid amide
N-cyclopropyl-N-[3-[2-(9-pyridin-4-yl-3,9-diazaspiro[5.5]undecan-3-yl)-pyrimidin-4-yl]-propyl]-3-(trifluoromethyl)-benzenesulfonic acid amide
4-methoxy-N,2,6-trimethyl-N-[2-[[4-[4-(1-methyl-4-piperidinyl)-1-piperazinyl]-2-pyrimidinyl]oxy]ethyl]benzenesulfonamide,
2-[[1-(4-methoxy-2,6-dimethylphenyl)sulfonyl-2-piperidinyl]methoxy]-4-[4-(1-methyl-4-piperidinyl)-1-piperazinyl]pyrimidine,
2-[[1-(4-methoxy-2,6-dimethylphenyl)sulfonyl-2-piperidinyl]methoxy]-4-[4-(2-1-pyrrolidinylethyl)-1-piperidinyl]pyrimidine,
N-[2-(4-dimethylamino-4-phenyl-1-piperidinyl)ethyl]-2-[[1-(4-methoxy-2,6-dimethylphenyl)sulfonyl-2-piperidinyl]methoxy]-N-methyl-4-pyrimidineamine, N-[2-[[4-[4-(4-fluorophenyl)-1-piperazinyl]-2-pyrimidinyl]oxy]ethyl]-4-methoxy-N,2,6-trimethylbenzenesulfonamide,
4-methoxy-N,2,6-trimethyl-N-[2-[[4-[4-(4-methyl-1-piperazinyl)-1-piperidinyl]-2-pyrimidinyl]oxy]ethyl]benzenesulfonamide,
4-methoxy-N,2,6-trimethyl-N-[2-[[4-[4-[(1-methy)-4-piperidinyl)methyl]-1-piperazinyl]-2-pyrimidinyl]oxy]ethyl]benzenesulfonamide,
N-[2-[[4-[4-hydroxy-4-(3-pyridyl)-1-piperidinyl]-2-pyrimidinyl]oxy]ethyl]-4-methoxy-N,2,6-trimethylbenzenesulfonamide,
2-[[1-(4-methoxy-2,6-dimethylphenyl)sulfonyl-2-piperidinyl]methoxy]-4-(4-methyl-1-piperazinyl)pyrimidine,
4-[4-(4-fluorophenyl)-1-piperazinyl]-2-[[1-(4-methoxy-2,6-dimethylphenyl)sulfonyl-2-piperidinyl]methoxy]pyrimidine,
2-[[1-(4-methoxy-2,6-dimethylphenyl)sulfonyl-2-pipeidinyl]methoxy]-4-[4-(4-methyl-1-piperazinyl)-1-piperidinyl]pyrimidine,
2-[[1-(4-methoxy-2,6-dimethylphenyl)sulfonyl-2-piperidinyl]methoxy]-4-[4-(2-pyrimidinyl)-1-piperazinyl]pyrimidine,
2-[[1-(4-methoxy-2,6-dimethylphenyl)sulfonyl-2-piperidinyl]methoxy]-4-[4-(4-pyridyl)-1-piperazinyl]pyrimidine,
2-[(1-(4-methoxy-2,6-dimethylphenyl)sulfonyl-2-piperidinyl]methoxy]-4-[4-[(1-methyl-4-piperidinyl)methyl]-1-piperazinyl]pyrimidine,
2-[[(2S)-1-(4-methoxy-2,6-dimethylphenyl)sulfonyl-2-pyrrolidinyl]methoxy]-4-(4-methyl-1-piperazinyl)pyrimidine,
2-[[(2S)-1-(4-methoxy-2,6-dimethylphenyl)sulfonyl-2-pyrrolidinyl]methoxy]-4-[4-(2-1-pyrrolidinylethyl)-1-piperidinyl]pyrimidine,
2-[[(2S)-1-(4-methoxy-2,6-dimethylphenyl)sulfonyl-2-pyrrolidinyl]methoxy]-4-[4-(4-methyl-1-piperazinyl)-1-piperidinyl]pyrimidine,
2-[[(2S)-1-(4-methoxy-2,6-dimethylphenyl)sulfonyl-2-pyrrolidinyl)methoxy]-4-[4-(2-pyrimidinyl)-1-piperazinyl]pyrimidine,
2-[[(2S)-1-(4-methoxy-2,6-dimethylphenyl)sulfonyl-2-pyrrolidinyl]methoxy]-4-[4-(4-pyridyl)-1-piperazinyl]pyrimidine,
1-[2-[[(2S)-1-(4-methoxy-2,6-dimethylphenyl)sulfonyl-2-pyrrolidinyl]methoxy]-4-pyrimidinyl]-4-(3-pyridyl)-4-piperidinol,
1-[2-[[(2S)-1-(4-methoxy-2,6-dimethylphenyl)sulfonyl-2-pyrrolidinyl]methoxy]-4-pyrimidinyl]-4-(2-thienyl)-4-piperidinol,
3-benzyl-7-[2-[[1-(4-methoxy-2,6-dimethylphenyl)sulfonyl-2-piperidinyl]methoxy]-4-pyrimidinyl]-3,7-diazaspiro[4.4]nonane,
1'-[2-[[1-(4-methoxy-2,6-dimethylphenyl)sulfonyl-2-piperidinyl]methoxy]-4-pyrimidinyl]spiro[1H-isobenzofuran-3,4'-piperidine],
6-chloro-3-[1-[2-[[1-(4-methoxy-2,6-dimethylphenyl)sulfonyl-2-piperidinyl]methoxy]-4-pyrimidinyl]-4-piperidinyl]-1H-benzimidazol-2-one,
8-[2-[[1-(4-methoxy-2,6-dimethylphenyl)sulfonyl-2-piperidinyl]methoxy]-4-pyrimidinyl]-4-phenyl-2,4,8-triazaspiro[4.5]decan-1-one,
2-[[1-(4-methoxy-2,6-dimethylphenyl)sulfonyl-2-piperidinyl]methoxy]-4-[4-[2-(1-piperidyl)ethyl]-1-piperidinyl]pyrimidine,
2-[[1-(4-methoxy-2,6-dimethylphenyl)sulfonyl-3-piperidinyl]oxy]-4-[4-[2-(1-piperidyl)ethyl]-1-piperidinyl]pyrimidine.
N-[2-[[4-(3-benzyl-3,7-diazaspiro[4.4]nonan-7-yl)-2-pyrimidinyl]oxy]ethyl]-4-methoxy-N,2,6-trimethylbenzenesulfonamide,
4-methoxy-N,2,6-trimethyl-N-[2-[[4-(1'-spiro[1H-isobenzofuran-3,4'-piperidin]yl)-2-pyrimidinyl]oxy]ethyl]benzenesulfonamide,
4-methoxy-N,2,6-trimethyl-N-[2-[[4-(1-oxo-4-phenyl-2,4,8-triazaspiro[4.5)decan-8-yl)-2-pyrimidinyl]oxy]ethyl]benzenesulfonamide,
4-methoxy-N,2,6-trimethyl-N-[2-[[4-[4-[2-(1-piperidyl)ethyl]-1-piperidinyl]-2-pyrimidinyl]oxy]ethyl]benzenesulfonamide,
3-benzyl-7-[2-[[(2S)-1-(4-methoxy-2,6-dimethylphenyl)sulfonyl-2-pyrrolidinyl]methoxy]-4-pyrimidinyl]-3,7-diazaspiro[4.4]nonane,
1'-[2-[[(2S)-1-(4-methoxy-2,6-dimethylphenyl)sulfonyl-2-pyrrolidinyl]methoxy]-4-pyrimidinyl]spiro[1H-isobenzofuran-3,4'-piperidine,]
6-chloro-3-[1-[2-[[(2S)-1-(4-methoxy-2,6-dimethylphenyl)sulfonyl-2-pyrrolidinyl]methoxy]-4-pyrimidinyl]-4-piperidinyl]-1H-benzimidazol-2-one,
8-[2-[[(2S)-1-(4-methoxy-2,6-dimethylphenyl)sulfonyl-2-pyrrolidinyl]methoxy]-4-pyrimidinyl]-4-phenyl-2,4,8-triazaspiro[4.5]decan-1-one,
2-[[(2S)-1-(4-methoxy-2,6-dimethylphenyl)sulfonyl-2-pyrrolidinyl]methoxy]-4-[4-(1-methyl-4-piperidinyl)-1-piperazinyl]pyrimidine,
2-[[(2S)-1-(4-methoxy-2,6-dimethylphenyl)sulfonyl-2-pyrrolidinyl]methoxy]-4-[4-[2-(1-piperidyl)ethyl}-1-piperidinyl]pyrimidine.
3-(4-fluorophenyl)-8-[2-[[1-(4-methoxy-2,6-dimethylphenyl)sulfonyl-2-piperidinyl]methoxy]-4-pyrimidinyl]-3,8-diazaspiro[4.5]decan-4-one,
3-[(4-fluorophenyl)methyl]-8-[2-[[1-(4-methoxy-2,6-dimethylphenyl)sulfonyl-2-piperidinyl]methoxy]-4-pyrimidinyl]-3,8-diazaspiro[4.5]decan-4-one, 3-benzyl-8-[2-[[1-(4-methoxy-2,6-dimethylphenyl)sulfonyl-2-piperidinyl]methoxy]-4-pyrimidinyl]-3,8-diazaspiro[4.5]decan-4-one,
9-[2-[[1-(4-methoxy-2,6-dimethylphenyl)sulfonyl-3-piperidinyl]oxy]-4-pyrimidinyl]-3-(4-pyridyl)-3,9-diazaspiro[5.5]undecane,
N-[2-[[4-[3-(4-fluorophenyl)-4-oxo-3,8-diazaspiro[4.5]decan-8-yl]-2-pyrimidinyl]oxy]ethyl]-4-methoxy-N,2,6-trimethylbenzenesulfonamide,
4-methoxy-N,2,6-trimethyl-N-[2-[[4-[4-oxo-1-[3-(trifluoromethyl)phenyl]-3,8-diazaspiro[4.5]decan-8-yl]-2-pyrimidinyl]oxy]ethyl]benzenesulfonamide,
N-[2-[[4-[1-(4-fluorophenyl)-3-methyl-4-oxo-3,8-diazaspiro[4.5]decan-8-yl]-2-pyrimidinyl]oxy]ethyl]-4-methoxy-N,2,6-trimethylbenzenesulfonamide,
N-[2-[[4-[3-[(4-fluorophenyl)methyl]-4-oxo-3,8-diazaspiro[4.5]decan-8-yl]-2-pyrimidinyl]oxy]ethyl]-4-methoxy-N,2,6-trimethylbenzenesulfonamide,
N-[2-[[4-(3-benzyl-4-oxo-3,8-diazaspiro[4.5]decan-8-yl)-2-pyrimidinyl]oxy]ethyl]-4-methoxy.-N,2,6-trimethylbenzenesulfonamide,
4-methoxy-N,2,6-trimethyl-N-[2-[[4-[3-(4-pyridyl)-3,9-diazaspiro[5.5]undecan-9-yl]-2-pyrimidinyl]oxy]ethyl]benzenesulfonamide,
3-(4-fluorophenyl)-8-[2-[[(2S)-1-(4-methoxy-2,6-dimethylphenyl)sulfonyl-2-pyrrolidinyl]methoxy]-4-pyrimidinyl]-3,8-diazaspiro[4.5]decan-4-one,
8-[2-[[(2S)-1-(4-methoxy-2,6-dimethylphenyl)sulfonyl-2-pyrrolidinyl]methoxy]-4-pyrimidinyl]-1-[3-(trifluoromethyl)phenyl]-3,8-diazaspiro[4.5]decan-4-one,
1-(4-fluorophenyl)-8-[2-[[(2S)-1-(4-methoxy-2,6-dimethylphenyl)sulfonyl-2-pyrrolidinyl]methoxy]-4-pyrimidinyl]-3-methyl-3,8-diazaspiro[4.5]decan-4-one,
3-[(4-fluorophenyl)methyl]-8-[2-[[(2S)-1-(4-methoxy-2,6-dimethylphenyl)sulfonyl-2-pyrrolidinyl]methoxy]-4-pyrimidinyl]-3,8-diazaspiro[4.5]decan-4-one,
2-[((2S)-1-(4-methoxy-2,6-dimethylphenyl)sulfonyl-2-pyrrolidinyl]methoxy]-4-[4-(4-pyridyloxy)-1-piperidinyl]pyrimidine,
3-benzyl-8-[2-[[(2S)-1-(4-methoxy-2,6-dimethylphenyl)sulfonyl-2-pyrrolidinyl]methoxy]-4-pyrimidinyl]-3,8-diazaspiro[4.5]decan-4-one,
N-[[1-[2-[[(2R)-1-(4-methoxy-2,6-dimethylphenyl)sulfonyl-2-pyrrolidinyl]methoxy]-4-pyrimidinyl]-4-(4-methyl-1-piperazinyl)-4-piperidinyl]methyl]-4-pyridinecarboxamide,
9-[2-[[(2S)-1-(4-methoxy-2,6-dimethylphenyl)sulfonyl-2-pyrrolidinyl]methoxy]-4-pyrimidinyl]-3-(4-pyridyl)-3,9-diazaspiro[5.5]undecane,
5-[2-[[1-(4-methoxy-2,6-dimethylphenyl)sulfonyl-2-piperidinyl]methoxy]-4-pyrimidinyl]-2-(4-pyridylmethyl)-2,5-diazabicyclo[2.2.1]heptane,
5-[2-[[1-(4-methoxy-2,6-dimethylphenyl)sulfonyl-3-piperidinyl]oxy]-4-pyrimidinyl]-2-(4-pyridylmethyl)-2,5-diazabicyclo[2.2.1]heptane,
5-[2-[[(2S)-1-(4-methoxy-2,6-dimethylphenyl)sulfonyl-2-pyrrolidinyl]methoxy]-4-pyrimidinyl]-2-(4-pyridylmethyl)-2,5-diazabicyclo[2.2. 1]heptane,
4-methoxy-N,2,6-trimethyl-N-[2-[[4-[4-(1-methyl-4-piperidinyl)-1-piperazinyl]-2-pyrimidinyl]oxy]-1-phenylethyl]benzenesulfonamide,
N-[2-[[4-[3-[(4-fluorophenyl)methyl]-4-oxo-3,8-diazaspiro[4.5]decan-8-yl]-2-pyimidinyl]oxy]-1-phenylethyl]-4-methoxy-N,2,6-trimethylbenzenesulfonamide,
N-[2-[[4-(3-benzyl-4-oxo-3,8-diazaspiro[4.5]decan-8-yl)-2-pyrimidinyl]oxy]-1-phenylethyl]-4-methoxy-N,2,6-trimethylbenzenesulfonamide,
4-methoxy-N,2,6-trimethyl-N-[1-phenyl-2-[[4-[4-(3-pyridyl)-4-(2-1-pyrrolidinylethoxy)-1-piperidinyl]-2-pyrimidinyl]oxy]ethyl]benzenesulfonamide,
4-methoxy-N,2,6-trimethyl-N-[1-phenyl-2-[[4-[4-(4-pyridyloxy)-1-piperidinyl]-2-pyrimidinyl]oxy]ethyl]benzenesulfonamide,
4-methoxy-N,2,6-trimethyl-N-[2-[[4-[4-[oxo-(3-pyridyl)methyl]-1-piperazinyl]-2-pyrimidinyl]oxy]-1-phenylethyl]benzenesulfonamide,
N-[2-[[4-[2-[(4-fluorophenyl)methyl]-2,5-diazabicyclo[2.2.1]heptan-5-yl]-2-pyrimidinyl]oxy]-1-phenylethyl]-4-methoxy-N,2,6-trimethylbenzenesulfonamide,
2-[[1-(4-methoxy-2,6-dimethylphenyl)sulfonyl-2-piperidinyl]methoxy]-4-[4-(4-pyridyl)-1-piperazinyl]pyrimidine.
1-[2-[[1-(4-methoxy-2,6-dimethylphenyl)sulfonyl-2-piperidinyl]methoxy]-4-pyrimidiny]-4-(3-pyridyl)-4-piperidinol,
N-[2-[[4-(3-benzyl-3,7-diazaspiro[4.4]nonan-7-yl)-2-pyrimidinyl]oxy]-1-phenylethyl]-4-methoxy-N,2,6-trimethylbenzenesulfonamide,
[4-butyl-1-[2-[[1-[(4-methoxy-2,6-dimethyl-phenyl)sulfonyl]-piperidin-2-yl]-methoxy]-pyrimidin-4-yl]-piperidin-4-yl]-dimethyl-amine
[1-[2-[[1-[(4-methoxy-2,6-dimethyl-phenyl)sulfonyl]-piperidin-2-yl]-methoxy]-pyrimidin-4-yl]-4-thiophen-2-yl-piperidin-4-yl]-dimethyl-amine
[2-[[1-[(4-methoxy-2,6-dimethyl-phenyl)sulfonyl]-piperidin-2-yl-methoxy]-pyrimidin-4-yl]-methyl-[2-(4-phenyl-4-pyrrolidin-1-yl-piperidin-1-yl)-ethyl]-amine
2-(4-butyl-4-dimethylamino-piperidin-1-yl)-ethyl-[2-[[1-[(4-methoxy-2,6-dimethylphenyl)sulfonyl]-piperidin-2-yl]-methoxy]-pyrimidin-4-yl]-methyl-amine
3-(4-dimethylamino-4-phenyl-piperidin-1-yl)-propyl-[2-[[(2S)-1-[(4-methoxy-2,6-dimethylphenyl)sulfonyl]-pyrrolidin-2-yl]-methoxy]-pyrimidin-4-yl]-methyl-amine
3-(4-dimethylamino-4-phenyl-piperidin-1-yl)-propyl-[2-[[(2S)-1-[(4-methoxy-2,6-dimethylphenyl)sulfonyl]-2,3-dihydro-1H-indol-2-yl]-methoxy]-pyrimidin-4-yl]-methyl-amine
2-[1-[(4-methoxy-2,6-dimethyl-phenyl)sulfonyl]-azetidin-3-yl]oxy-4-[4-[(1-methyl-piperidin-4-yl)-methyl]-piperazin-1-yl]-pyrimidine
2-[[1-[(4-methoxy-2,6-dimethyl-phenyl)sulfonyl]-piperidin-2-yl]-methoxy]-4-(4-pyridin-2-yloxy-piperidin-1-yl)-pyrimidine
2-[[1-[(4-methoxy-2,6-dimethyl-phenyl)sulfonyl]-piperidin-2-yl]-methoxy]-4-(4-pyrazin-2-yloxy-piperidin-1-yl)-pyrimidine
2-[[1-[(4-methoxy-2,6-dimethyl-phenyl)sulfonyl]-piperidin-2-yl]-methoxy]-4-[4-pyridin-3-yl-4-(3-pyrrolidin-1-yl-propyl)-piperidin-1-yl]-pyrimidine
2-[[1-[(4-methoxy-2,6-dimethyl-phenyl)sulfonyl]-piperidin-2-yl]-methoxy]-4-[2-(pyridin-2-yl-methyl)-pyrrolidin-1-yl]-pyrimidine
1-[2-[[1-[(4-methoxy-2,6-dimethyl-phenyl)sulfonyl]-piperidin-2-yl]-methoxy]-pyrimidin-4-yl]-4-pyridin-2-yl-piperidin-4-ol
1-[2-[[(2S)-1-[(4-methoxy-2,6-dimethyl-phenyl)sulfonyl]-pyrrolidin-2-yl]-methoxy]-pyrimidin-4-yl]-4-pyridin-2-yl-piperidin-4-ol
2-[[(2R)-1-[(4-methoxy-2,6-dimethyl-phenyl)sulfonyl]-pyrrolidin-2-yl]-methoxy]-4-[2-(pyridin-2-yl-methyl)-pyrrolidin-1-yl]-pyrimidine
5-[1-[2-[[(2S)-1-[(4-methoxy-2,6-dimethyl-phenyl)sulfonyl]-pyrrolidin-2-yl]-methoxy]-pyrimidin-4-yl]-piperidin-4-yl]-3-pyridin-4-yl-[1,2,4]oxadiazole
4-[4-(3-fluorophenyl)-4-(2-pyrrolidin-1-yl-ethoxy)-piperidin-1-yl]-2-[[(2S)-1-[(4-methoxy-2,6-dimethyl-phenyl)sulfonyl]-pyrrolidin-2-yl]-methoxy]-pyrimidine
[4-butyl-1-[2-[[(2S)-1-[(4-methoxy-2,6-dimethyl-phenyl)sulfonyl]-pyrrolidin-2-yl]-methoxy]-pyrimidin-4-yl]-piperidin-4-yl]-dimethyl-amine
2-(4-dimethylamino-4-phenyl-piperidin-1-yl)-ethyl-[2-[[(2S)-1-[(4-methoxy-2,6-dimethylphenyl)sulfonyl]-pyrrolidin-2-yl]-methoxy]-pyrimidin-4-yl]-methyl-amine
N-[2-[4-(4-butyl-4-dimethylamino-piperidin-1-yl)-pyrimidin-2-yl]oxy-ethyl]-4-methoxy-N,2,6-trimethyl-benzenesulfonic acid amide
[2-[[(2S)-1-[(4-methoxy-2,6-dimethyl-phenyl)sulfonyl]-pyrrolidin-2-yl]-methoxy]-pyrimidin-4-yl]-methyl-[2-(4-phenyl-4-pyrrolidin-1-yl-piperidin-1-yl)-ethyl]-amine
(2S)-1-[(4-methoxy-2,6-dimethyl-phenyl)sulfonyl]-2-[[4-(4-pyrazin-2-yloxy-piperidin-1-yl)-pyrimidin-2-yl]oxy-methyl]-2,3-dihydro-1H-indole
[4-butyl-1-[2-[[(2S)-1-[(4-methoxy-2,6-dimethyl-phenyl)sulfonyl]-2,3-dihydro-1H-indol-2-yl]-methoxy]-pyrimidin-4-yl]-piperidin-4-yl]-dimethyl-amine
2-(4-dimethylamino-4-phenyl-piperidin-1-yl)-ethyl-[2-[[(2S)-1-[(4-methoxy-2,6-dimethylphenyl)sulfonyl]-2,3-dihydro-1H-indol-2-yl]-methoxy]-pyrimidin-4-yl]-methyl-amine
[2-[[(2S)-1-[(4-methoxy-2,6-dimethyl-phenyl)sulfonyl]-2,3-dihydro-1H-indol-2-yl]-methoxy]-pyrimidin-4-yl]-methyl-[2-(4-phenyl-4-pyrrolidin-1-yl-piperidin-1-yl)-ethyl]-amine
2-(4-butyl-4-dimethylamino-piperidin-1-yl)-ethyl-[2-[[(2S)-1-[(4-methoxy-2,6-dimethylphenyl)sulfonyl]-2,3-dihydro-1H-indol-2-yl]-methoxy]-pyrimidin-4-yl]-methyl-amine
N-[2-[4-[2-(4-dimethylamino-4-phenyl-piperidin-1-yl)-ethyl-methyl-amino]-pyrimidin-2-yl]oxyethyl]-4-methoxy-N,2,6-trimethyl-benzenesulfonic acid amide
N-[2-[4-[3-(4-dimethylamino-4-phenyl-piperidin-1-yl)-propyl-methyl-amino]-pyrimidin-2-yl]oxyethyl]-4-methoxy-N,2,6-trimethyl-benzenesulfonic acid amide
3-(4-dimethylamino-4-phenyl-piperidin-1-yl)-propyl-[2-[[1-[(4-methoxy-2,6-dimethylphenyl)sulfonyl]-piperidin-2-yl]-methoxy]-pyrimidin-4-yl]-methyl-amine
5-[1-[2-[[1-[(4-methoxy-2,6-dimethyl-phenyl)sulfonyl]-piperidin-2-yl]-methoxy]-pyrimidin-4-yl]-piperidin-4-yl]-3-pyridin-4-yl-[1,2,4]oxadiazole
4-[4-(3-fluorophenyl)-4-(4-methyl-piperazin-1-yl)-piperidin-1-yl]-2-[[1-[(4-methoxy-2,6-dimethyl-phenyl)sulfonyl]-piperidin-2-yl]-methoxy]-pyrimidine
(1S,5R)-8-[2-[[1-[(4-methoxy-2,6-dimethyl-phenyl)sulfonyl]-piperidin-2-yl]-methoxy]-pyrimidin-4-yl]-3-pyridin-3-yloxy-8-azabicyclo[3.2.1]octane
1-[2-[[2-[[1-[(4-methoxy-2,6-dimethyl-phenyl)sulfonyl]-piperidin-2-yl]-methoxy]-pyrimidin-4-yl]-methyl-amino]-ethyl]-4-pyridin-3-yl-piperidin-4-ol
4-methoxy-N,2,6-trimethyl-N-[1-phenyl-2-[4-[(1S,5R)-3-pyridin-3-yloxy-8-azabicyclo[3.2.1]octan-8-yl]-pyrimidin-2-yl]oxy-ethyl]-benzenesulfonic acid amide
4-methoxy-N,2,6-timethyl-N-[1-phenyl-2-[4-[4-pyridin-3-yl-4-(3-pyrrolidin-1-yl-propyl)-piperidin-1-yi]-pyrimidin-2-yl]oxy-ethyl]-benzenesulfonic acid amide
7-[2-[[(2S)-1-[(4-methoxy-2,6-dimethyl-phenyl)sulfonyl]-pyrrolidin-2-yl]-methoxy]-pyrimidin-4-yl]-2-(piperidin-1-yl-methyl)-5,6,7,8-tetrahydro-imidazo[1,2-a]pyrazine
1-[2-[[1-[(4-methoxy-2,6-dimethyl-phenyl)sulfonyl]-piperidin-2-yl]-methoxy]-pyrimidin4-yl]-4-pyridin-4-yl-piperidin-4-ol
N-[2-[4-(4-hydroxy-4-pyridin-4-yl-piperidin-1-yl)-pyrimidin-2-yl]oxy-1-phenyl-ethyl]-4-methoxy-N,2,6-trimethyl-benzenesulfonic acid amide
[1-[2-[[1-[(4-methoxy-2,6-dimethyl-phenyl)sulfonyl]-piperidin-2-yl]-methoxy]-pyrimidin-4-yl]-4-phenyl-piperidin-4-yl]-dimethyl-amine
2-[1-[(4-methoxy-2,6-dimethyl-phenyl)sulfonyl]-piperidin-3-yl]oxy-4-(4-phenyl-4-pyrrolidin-1-yl-piperidin-1-yl)-pyrimidine
2-[[(2S)-1-[(4-methoxy-2,6-dimethyl-phenyl)sulfonyl]-pyrrolidin-2-yl]-methoxy]-4-(4-phenyl-4-pyrrolidin-1-yl-piperidin-1-yl)-pyrimidine
4-methoxy-N,2,6-trimethyl-N-[2-[4-(4-phenyl-4-pyrrolidin-1-yl-piperidin-1-yl)-pyrimidin-2-yl]oxy-ethyl]-benzenesulfonic acid amide
N-[2-[4-(4-dimethylamino-4-phenyl-piperidin-1-yl)-pyrimidin-2-yl]oxy-ethyl]-4-methoxy-N,2,6-trimethyl-benzenesulfonic acid amide
N-[2-[4-(4-dimethylamino-4-thiophen-2-y1-piperidin-1-yl)-pyrimidin-2-yl]oxy-ethyl]-4-methoxy-N,2,6-trimethyl-benzenesulfonic acid amide
4-methoxy-N,2,6-trimethyl-N-[2-[4-[methyl-[2-(4-phenyl-4-pyrrolidin-1-yl-piperidin-1-yl)-ethyl]-amino]-pyrimidin-2-yl]oxy-ethyl]-benzenesulfonic acid amide
N-[2-[4-[2-(4-butyl-4-dimethylamino-piperidin-1-yl)-ethyl-methyl-amino]-pyrimidin-2-yl]oxyethyl]-4-methoxy-N,2,6-trimethyl-benzenesulfonic acid amide
2-[[1-[(4-methoxy-2,6-dimethyl-phenyl)sulfonyl]-piperidin-2-yl]-methoxy]-4-(4-phenyl-4-pyrrolidin-1-yl-piperidin-1-yl)-pyrimidine
2-[1-[(4-methoxy-2,6-dimethyl-phenyl)sulfonyl]-azetidin-3-yl]oxy-4-(4-phenyl-4-pyrrolidin-1-yl-piperidin-1-yl)-pyrimidine
2-(4-dimethylamino-4-phenyl-piperidin-1-yl)-ethyl-[2-[1-[(4-methoxy-2,6-dimethylphenyl)sulfonyl]-azetidin-3-yl]oxy-pyrimidin-4-yl]-methyl-amine
[2-[1-[(4-methoxy-2,6-dimethyl-phenyl)sulfonyl]-azetidin-3-yl]oxy-pyrimidin-4-yl]-methyl-[2-(4-phenyl-4-pyrrolidin-1-yl-piperidin-1-yl)-ethyl]-amine
[1-[2-[[(2S)-1-[(4-methoxy-2,6-dimethyl-phenyl)sulfonyl]-2,3-dihydro-1H-indol-2-yl]-methoxy]-pyrimidin-4-yl]-4-thiophen-2-yl-piperidin-4-yl]-dimethyl-amine
3-(4-dimethylamino-4-phenyl-piperidin-1-yl)-propyl-[2-[1-[(4-methoxy-2,6-dimethylphenyl)sulfonyl]-azetidin-3-yl]oxy-pyrimidin-4-yl]-methyl-amine
3-[4-[2-[1-[(4-methoxy-2,6-dimethyl-phenyl)sulfonyl]-azetidin-3-yl]oxy-pyrimidin-4-yl]-piperazin-1-yl]-propyl-dimethyl-amine
1-[2-[1-[(4-methoxy-2,6-dimethyl-phenyl)sulfonyl]-azetidin-3-yl]oxy-pyrimidin-4-yl]-4-pyridin-3-yl-piperidin-4-ol
2-[1-[(4-methoxy-2,6-dimethyl-phenyl)sulfonyl]-azetidin-3-yl]oxy-4-[4-(2-piperidin-1-yl-ethyl)-piperidin-1-yl]-pyrimidine
(2S)-2-[[4-[2-[(4-fluorophenyl)-methyl]-2,5-diazabicyclo[2.2.1]heptan-5-yl]-pyrimidin-2-yl]oxymethyl]-1-[(4-methoxy-2,6-dimethyl-phenyl)sulfonyl]-2,3-dihydro-1H-indole
4-[1-[2-[[1-[(4-methoxy-2,6-dimethyl-phenyl)sulfonyl]-piperidin-2-yl]-methoxy]-pyrimidin-4-yl]-4-methyl-piperidin-4-yl]-morpholine
4-methoxy-N,2,6-trimethyl-N-[1-phenyl-2-[4-[2-(pyridin-2-yl-methyl)-pyrrolidin-1-yl]-pyrimidin-2-yl]oxy-ethyl]-benzenesulfonic acid amide
4-methoxy-N,2,6-trimethyl-N-[1-phenyl-2-[4-[4-(3-pyridin-4-yl-[1,2,4]oxadiazol-5-yl)-piperidin-1-yl]-pyrimidin-2-yl]oxy-ethyl]-benzenesulfonic acid amide
N-[2-[4-[4-(3-fluorophenyl)-4-(4-methyl-piperazin-1-yl)-piperidin-1-yl]-pyrimidin-2-yl]oxy-1-phenyl-ethyl]-4-methoxy-N,2,6-trimethyl-benzenesulfonic acid amide
4-methoxy-N,2,6-trimethyl-N-[1-phenyl-2-[4-(4-pyridin-2-yloxy-piperidin-1-yl)-pyrimidin-2-yl]oxy-ethyl]-benzenesulfonic acid amide
4-methoxy-N,2,6-trimethyl-N-[2-[4-(4-methyl-4-morpholin-4-yl-piperidin-1-yl)-pyrimidin-2-yl]oxy-1-phenyl-ethyl]-benzenesulfonic acid amide
N-[2-[4-[2-(4-hydroxy-4-pyridin-3-yl-piperidin-1-yl)-ethyl-methyl-amino]-pyrimidin-2-yl]oxy-1-phenyl-ethyl]-4-methoxy-N,2,6-trimethyl-benzenesulfonic acid amide
4-[[1-(4-methoxy-2,6-dimethylphenyl)sulfonyl-2-piperidinyl]methoxy]-2-[4-(1-methyl-4-piperidinyl)-1-piperazinyl]pyrimidine,
4-[[1-(4-methoxy-2,6-dimethylphenyl)sulfonyl-2-piperidinyl]methoxy]-2-[4-(2-1-pyrrolidinylethyl)-1-piperidinyl]pyrimidine,
4-[[1-(4-methoxy-2,6-dimethylphenyl)sulfonyl-2-piperidinyl]methoxy]-2-(4-methyl-1-piperazinyl)pyrimidine,
4-[[1-(4-methoxy-2,6-dimethylphenyl)sulfonyl-2-piperidinyl]methoxy]-2-[4-(2-pyrimidinyl)-1-piperazinyl]pyrimidine,
4-[[1-(4-methoxy-2,6-dimethylphenyl)sulfonyl-2-piperidinyl]methoxy]-2-[4-(4-pyridyl)-1-piperazinyl]pyrimidine,
4-[[1-(4-methoxy-2,6-dimethylphenyl)sulfonyl-2-piperidinyl]methoxy]-2-[4-[(1-methyl-4-piperidinyl)methyl-1-piperazinyl]pyrimidine,
1-[4-[[1-(4-methoxy-2,6-dimethylphenyl)sulfonyl-2-piperidinyl]methoxy]-2-pyrimidinyl]-4-(3-pyridyl)-4-piperidinol,
4-[[(2S)-1-(4-methoxy-2,6-dimethylphenyl)sulfonyl-2-pyrrolidinyl]methoxy]-2-(4-methyl-1-piperazinyl)pyrimidine,
4-[[(2S)-1-(4-methoxy-2,6-dimethylphenyl)sulfonyl-2-pyrrolidinyl]methoxy]-2-[4-(2-1-pyrrolidinylethyl)-1-piperidinyl]pyrimidine,
4-[[(2S)-1-(4-methoxy-2,6-dimethylphenyl)sulfonyl-2-pyrrolidinyl]methoxy]-2-[4-(4-methyl-1-piperazinyl)-1-piperidinyl]pyrimidine,
4-[[(2S)-1-(4-methoxy-2,6-dimethylphenyl)sulfonyl-2-pyrrolidinyl]methoxy]-2-[4-(2-pyrimidinyl) 1-piperazinyl]pyrimidine,
4-[[(2S)-1-(4-methoxy-2,6-dimethylphenyl)sulfonyl-2-pyrrolidinyl]methoxy]-2-[4-[(1-methyl-4-piperidinyl)methyl]-1-piperazinyl]pyrimidine,
1-[4-[[(2S)-1-(4-methoxy-2,6-dimethylphenyl)sulfonyl-2-pyrrolidinyl]methoxy]-2-pyrimidinyl]-4-(3-pyridyl)-4-piperidinol,
4-[[1-(4-methoxy-2,6-dimethylphenyl)sulfonyl-3-azetidinyl]oxy]-2-[4-(2-1-pyrrolidinylethyl)-1-piperidinyl]pyrimidine,
4-[[1-(4-methoxy-2,6-dimethylphenyl)sulfonyl-3-azetidinyl]oxy]-2-[4-(4-methyl-1-piperazinyl)-1-piperidinyl]pyrimidine,
(2S)-1-(4-methoxy-2,6-dimethylphenyl)sulfonyl-2-[[2-(4-methyl-1-piperazinyl)-4-pyrimidinyl]oxymethyl]indoline,
(2S)-1-(4-methoxy-2,6-dimethylphenyl)sulfonyl-2-[[2-[4-(2-1-pyrrolidinylethyl)-1-piperidinyl]-4-pyrimidinyl]oxymethyl]indoline,
(2S)-1-(4-methoxy-2,6-dimethylphenyl)sulfonyl-2-[[2-[4-(4-methyl-1-piperazinyl)-1-piperidinyl]-4-pyrimidinyl]oxymethyl]indoline,
(2S)-1-(4-methoxy-2,6-dimethylphenyl)sulfonyl-2-[[2-[4-(4-pyridyl)-1-piperazinyl]-4-pyrimidinyl]oxymethyl]indoline,
(2S)-1-(4-methoxy-2,6-dimethylphenyl)sulfonyl-2-[[2-[4-[(1-methyl-4-piperidinyl)methyl]-1-piperazinyl]-4-pyrimidinyl]oxymethyl]indoline.
3-benzyl-7-[4-[[1-(4-methoxy-2,6-dimethylphenyl)sulfonyl-2-piperidinyl]methoxy]-2-pyrimidinyl]-3,7-diazaspiro[4.4]nonane,
4-[[1-(4-methoxy-2,6-dimethylphenyl)sulfonyl-2-piperidinyl]methoxy]-2-[4-[2-(1-piperidyl)ethyl]-1-piperidinyl]pyrimidine,
3-benzyl-7-[4-[[(2S)-1-(4-methoxy-2,6-dimethylphenyl)sulfonyl-2-pyrrolidinyl]methoxy]-2-pyrimidinyl]-3,7-diazaspiro[4.4]nonane,
8-[4-[[(2S)-1-(4-methoxy-2,6-dimethylphenyl)sulfonyl-2-pyrrolidinyl]methoxy]-2-pyrimidinyl]-4-phenyl-2,4,8-triazaspiro[4.5]decan-1-one,
4-[[(2S)-1-(4-methoxy-2,6-dimethylphenyl)sulfonyl-2-pyrrolidinyl]methoxy]-2-[4-(1-methyl-4-piperidinyl)-1-piperazinyl]pyrimidine,
4-[[(2S)-1-(4-methoxy-2,6-dimethylphenyl)sulfonyl-2-pyrrolidinyl]methoxy]-2-[4-[2-(1-piperidyl)ethyl]-1-piperidinyl]pyrimidine.
(2S)-1-(4-methoxy-2,6-dimethylphenyl)sulfonyl-2-[[2-[4-(1-methyl-4-piperidinyl)-1-piperazinyl]-4-pyrimidinyl]oxymethyl]indoline,
(2S)-1-(4-methoxy-2,6-dimethylphenyl)sulfonyl-2-[[2-[4-[2-(1-piperidyl)ethyl]-1-piperidinyl]-4-pyrimidinyl]oxymethyl]indoline,
4-methoxy-N,2,6-trimethyl-N-[2-[[2-[3-(4-pyridyl)-3,9-diazaspiro[5.5]undecan-9-yl]-4-pyrimidinyl]oxy]ethyl]benzenesulfonamide,
3-[(4-fluorophenyl)methyl]-8-[4-[[1-(4-methoxy-2,6-dimethylphenyl)sulfonyl-2-piperidinyl]methoxy]-2-pyrimidinyl]-3,8-diazaspiro[4.5]decan-4-one,
N-[[1-[4-[[1-(4-methoxy-2,6-dimethylphenyl)sulfonyl-2-piperidinyl]methoxy]-2-pyrimidinyl]-4-(4-methyl-1-piperazinyl)-4-piperidinyl]methyl]-4-pyridinecarboxamide.
9-[4-[[1-(4-methoxy-2,6-dimethylphenyl)sulfonyl-2-piperidinyl]methoxy]-2-pyrimidinyl]-3-(4-]pyidyl)-3,9-diazaspiro[5.5]undecane,
4-methoxy-N,2,6-trimethyl-N-[2-[[2-[4-(3-pyridyl)-4-(2-1-pyrrolidinylethoxy)-1-piperidinyl]-4-pyrimidinyl]oxy]ethyl]benzenesulfonamide,
4-methoxy-N,2,6-trimethyl-N-[2-[[2-[2-(4-pyridylmethyl)-2,5-diazabicyclo[2.2.1]heptan-5-yl]-4-pyrimidinyl]oxy]ethyl]benzenesulfonamide,
4-[[1-(4-methoxy-2,6-dimethylphenyl)sulfonyl-2-piperidinyl]methoxy]-2-[4-(3-pyridyl)-4-(2-1-pyrrolidinylethoxy)-1-piperidinyl]pyrimidine,
4-methoxy-N,2,6-trimethyl-N-[1-phenyl-2-[[2-[4-(4-pyridyl)-1-piperazinyl]-4-pyrimidinyl]oxy]ethyl]benzenesulfonamide,
4-methoxy-N,2,6-trimethyl-N-[1-phenyl-2-[[2-[3-(4-pyridyl)-3,9-diazaspiro[5.5]undecan-9-yl]-4-pyrimidinyl]oxy]ethyl]benzenesulfonamide,
N-methyl-N-[1-phenyl-2-[[2-[4-(3-pyridyl)-4-(2-1-pyrrolidinylethoxy)-1-piperidinyl]-4-pyrimidinyl]oxy]ethyl-2-naphthalenesulfonamide,
N-[2-[[2-[3-[(4-fluorophenyl)methyl]-4-oxo-3,8-diazaspiro[4.5]decan-8-yl]-4-pyrimidinyl]oxy]ethyl]-4-methoxy-N,2,6-trimethylbenzenesulfonamide,
4-[[(2S)-1-(4-methoxy-2,6-dimethylphenyl)sulfonyl-2-pyrrolidinyl]methoxy]-2-[4-(3-pyridyl)-4-(2-1-pyrrolidinylethoxy)-1-piperidinyl]pyrimidine and
4-[[1-(4-methoxy-2,6-dimethylphenyl)sulfonyl-3-piperidinyl]oxy]-2-[4-[2-(1-piperidyl)ethyl]-1-piperidinyl]pyrimidine.
1-[4-[[1-[(4-methoxy-2,6-dimethyl-phenyl)sulfonyl]-piperidin-2-yl]-methoxy]-pyrimidin-2-yl]-4-(pyridin-2-yl-methyl)-[1,4]diazepan
1-[4-[[(2R)-1-[(4-methoxy-2,6-dimethyl-phenyl)sulfonyl]-pyrrolidin-2-yl]-methoxy]-pyrimidin-2-yl]-4-(pyridin-2-yl-methyl)-[1,4]diazepan
2-(4-dimethylamino-4-phenyl-piperidin-1-yl)-ethyl-[4-[[1-[(4-methoxy-2,6-dimethylphenyl)sulfonyl]-piperidin-2-yl]-methoxy]-pyrimidin-2-yl]-methyl-amine
2-(4-butyl-4-dimethylamino-piperidin-1-yl)-ethyl-[4-[[1-[(4-methoxy-2,6-dimethylphenyl)sulfonyl]-piperidin-2-yl]-methoxy]-pyrimidin-2-yl]-methyl-amine
3-(4-dimethylamino-4-phenyl-piperidin-1-yl)-propyl-[4-[[(2S)-1-[(4-methoxy-2,6-dimethylphenyl)sulfonyl]-pyrrolidin-2-yl]-methoxy]-pyrimidin-2-yl]-methyl-amine
1-[4-[[(2S)-1-[(4-methoxy-2,6-dimethyl-phenyl)sulfonyl]-pyrrolidin-2-yl]-methoxy]-pyrimidin-2-yl]-4-pyridin-2-yl-piperidin-4-ol
5-[1-[4-[[(2S)-1-[(4-methoxy-2,6-dimethyl-phenyl)sulfonyl]-pyrrolidin-2-yl]-methoxy]-pyrimidin-2-yl]-piperidin-4-yl]-3-pyridin-4-yl-[1,2,4]oxadiazole
[4-[[1-[(4-methoxy-2,6-dimethyl-phenyl)sulfonyl]-piperidin-2-yl]-methoxy]-pyrimidin-2-yl]-methyl-[2-(4-pyridin-4-yloxy-piperidin-1-yl)-ethyl]-amine
(1S,5R)-8-[4-[[1-[(4-methoxy-2,6-dimethyl-phenyl)sulfonyl]-piperidin-2-yl]-methoxy]-pyrimidin-2-yl]-3-pyridin-4-yloxy-8-azabicyclo[3.2.1]octane
[4-[[(2S)-1-[(4-methoxy-2,6-dimethyl-phenyl)sulfonyl]-pyrrolidin-2-yl]-methoxy]-pyrimidin-2-yl]-methyl-[2-(4-pyridin4-yloxy-piperidin-1-yl)-ethyl]-amine
(1S,5R)-8-[4-[[(2S)-1-[(4-methoxy-2,6-dimethyl-phenyl)sulfonyl]-pyrrolidin-2-yl]-methoxy]-pyrimidin-2-yl]-3-pyridin-4-yloxy-8-azabicyclo[3.2.1]octane
2-(4-butyl-4-dimethylamino-piperidin-1-yl)-ethyl-[4-[1-[(4-methoxy-2,6-dimethylphenyl)sulfonyl]-piperidin-3-yl]oxy-pyrimidin-2-yl]-methyl-amine
2-(4-dimethylamino-4-phenyl-piperidin-1-yl)-ethyl-[4-[[(2S)-1-[(4-methoxy-2,6-dimethylphenyl)sulfonyl]-pyrrolidin-2-yl]-methoxy]-pyrimidin-2-yl]-methyl-amine
[4-[[(2S)-1-[(4-methoxy-2,6-dimethyl-phenyl)sulfonyl]-pyrrolidin-2-yl]-methoxy]-pyrimidin-2-yl]-methyl-[2-(4-phenyl-4-pyrrolidin-1-yl-piperidin-1-yl)-ethyl]-amine
2-(4-butyl-4-dimethylamino-piperidin-1-yl)-ethyl-[4-[[(2S)-1-[(4-methoxy-2,6-dimethylphenyl)sulfonyl]-pyrrolidin-2-yl]-methoxy]-pyrimidin-2-yl]-methyl-amine
2-(4-dimethylamino-4-phenyl-piperidin-1-yl)-ethyl-[4-[[(2S)-1-[(4-methoxy-2,6-dimethylphenyl)sulfonyl]-2,3-dihydro-1H-indol-2-yl]-methoxy]-pyrimidin-2-yl]-methyl-amine
2-(4-butyl-4-dimethylamino-piperidin-1-yl)-ethyl-[4-[[(2S)-1-[(4-methoxy-2,6-dimethylphenyl)sulfonyl]-2,3-dihydro-1H-indol-2-yl]-methoxy]-pyrimidin-2-yl]-methyl-amine
4-methoxy-N,2,6-trimethyl-N-[2-[2-[methyl-[2-(4-phenyl-4-pyrrolidin-1-yl-piperidin-1-yl)-ethyl]-amino]-pyrimidin-4-yl]oxy-ethyl]-benzenesulfonic acid amide
N-[2-[2-[2-(4-butyl-4-dimethylamino-piperidin-1-yl)-ethyl-methyl-amino]-pyrimidin-4-yl]oxyethyl]-4-methoxy-N,2,6-trimethyl-benzenesulfonic acid amide
3-(4-dimethylamino-4-phenyl-piperidin-1-yl)-propyl-[4-[[1-[(4-methoxy-2,6-dimethylphenyl)sulfonyl]-piperidin-2-yl]-methoxy]-pyimidin-2-yl]-methyl-amine
[4-butyl-1-[4-[[1-[(4-methoxy-2,6-dimethyl-phenyl)sulfonyl]-piperidin-2-yl]-methoxy]-pyrimidin-2-yl]-piperidin-4-yl]-dimethyl-amine
[4-[[1-[(4-methoxy-2,6-dimethyl-phenyl)sulfonyl]-piperidin-2-yl]-methoxy]-pyrimidin-2-yl]-methyl-[2-(4-phenyl-4-pyrrolidin-1-yl-piperidin-1-yl)-ethyl]-amine
[1-[4-[1-[(4-methoxy-2,6-dimethyl-phenyl)sulfonyl]-piperidin-3-yl]oxy-pyrimidin-2-yl]-4-phenyl-piperidin-4-yl]-dimethyl-amine
2-(4-dimethylamino-4-phenyl-piperidin-1-yl)-ethyl-[4-[1-[(4-methoxy-2,6-dimethylphenyl)sulfonyl]-piperidin-3-yl]oxy-pyrimidin-2-yl]-methyl-amine
[4-[1-[(4-methoxy-2,6-dimethyl-phenyl)sulfonyl]-piperidin-3-yl]oxy-pyrimidin-2-yl]-methyl-[2-(4-phenyl-4-pyrrolidin-1-yl-piperidin-1-yl)-ethyl]-amine
N-[2-[2-[2-(4-dimethylamino-4-phenyl-piperidin-1-yl)-ethyl-methyl-amino]-pyrimidin-4-yl]oxyethyl]-4-methoxy-N,2,6-trimethyl-benzenesulfonic acid amide
[4-butyl-1-[4-[[(2S)-1-[(4-methoxy-2,6-dimethyl-phenyl)sulfonyl-pyrrolidin-2-yl]-methoxy]-pyrimidin-2-yl]-piperidin-4-yl]-dimethyl-amine
2-(4-dimethylamino-4-phenyl-piperidin-1-yl)-ethyl-[4-[1-[(4-methoxy-2,6-dimethylphenyl)sulfonyl]-azetidin-3-yl]oxy-pyrimidin-2-yl]-methyl-amine
[4-[1-[(4-methoxy-2,6-dimethyl-phenyl)sulfonyl]-azetidin-3-yl]oxy-pyrimidin-2-yl]-methyl-[2-(4-phenyl-4-pyrrolidin-1-yl-pipeidin-1-yl)-ethyl]-amine
2-(4-butyl-4-dimethylamino-piperidin-1-yl)-ethyl-[4-[1-[(4-methoxy-2,6-dimethylphenyl)sulfonyl]-azetidin-3-yl]oxy-pyrimidin-2-yl]-methyl-amine
[4-[[(2S)-1-[(4-methoxy-2,6-dimethyl-phenyl)sulfonyl]-2,3-dihydro-1H-indol-2-yl]-methoxy]-pyrimidin-2-yl]-methyl-[2-(4-phenyl-4-pyrrolidin-1-yl-piperidin-1-yl)-ethyl]-amine
3-(4-dimethylamino-4-phenyl-piperidin-1-yl)-propyl-[4-[1-[(4-methoxy-2,6-dimethylphenyl)sulfonyl]-piperidin-3-yl]oxy-pyrimidin-2-yl]-methyl-amine
3-(4-dimethylamino-4-phenyl-piperidin-1-yl)-propyl-[4-[1-[(4-methoxy-2,6-dimethylphenyl)sulfonyl]-azetidin-3-yl]oxy-pyrimidin-2-yl]-methyl-amine
3-(4-dimethylamino-4-phenyl-piperidin-1-yl)-propyl-[4-[[(2S)-1-[(4-methoxy-2,6-dimethylphenyl)sulfonyl]-2,3-dihydro-1H-indol-2-yl]-methoxy]-pyrimidin-2-yl]-methyl-amine
1-[4-[1-[(4-methoxy-2,6-dimethyl-phenyl)sulfonyl]-azetidin-3-yl]oxy-pyrimidin-2-yl]-4-pyridin-3-yl-piperidin-4-ol
1-[4-[1-[(4-methoxy-2,6-dimethyl-phenyl)sulfonyl]-azetidin-3-yl]oxy-pyrimidin-2-yl]-4-(pyridin-2-yl-methyl)-[1,4]diazepan
4-[[1-[(4-methoxy-2,6-dimethyl-phenyl)sulfonyl]-piperidin-2-yl]-methoxy]-2-[2-(pyridin-2-yl-methyl)-pyrrolidin-1-yl]-pyrimidine
4-[[1-[(4-methoxy-2,6-dimethyl-phenyl)sulfonyl]-piperidin-2-yl]-methoxy]-2-(4-pyridin-2-yloxy-piperidin-1-yl)-pyrimidine
4-[[1-[(4-methoxy-2,6-dimethyl-phenyl)sulfonyl]-piperidin-2-yl]-methoxy]-2-(4-pyrazin-2-yloxy-piperidin-1-yl)-pyrimidine
4-[1-[4-[[1-[(4-methoxy-2,6-dimethyl-phenyl)sulfonyl]-piperidin-2-yl]-methoxy]-pyrimidin-2-yl]-4-methyl-piperidin-4-yl]-morpholine
4-[[1-[(4-methoxy-2,6-dimethyl-phenyl)sulfonyl]-piperidin-2-yl]-methoxy]-2-[4-pyridin-3-yl-4-(3-pyrrolidin-1-yl-propyl)-piperidin-1-yl]-pyrimidine
1-[4-[[1-[(4-methoxy-2,6-dimethyl-phenyl)sulfonyl]-piperidin-2-yl]-methoxy]-pyrimidin-2-yl]-4-pyridin-2-yl-piperidin-4-ol
4-[[(2R)-1-[(4-methoxy-2,6-dimethyl-phenyl)sulfonyl]-pyrrolidin-2-yl]-methoxy]-2-[2-(pyridin-2-yl-methyl)-pyrrolidin-1-yl]-pyrimidine
[1-[4-[[(2S)-1-[(4-methoxy-2,6-dimethyl-phenyl)sulfonyl]-pyrrolidin-2-yl]-methoxy]-pyrimidin-2-yl]-4-phenyl-piperidin-4-yl]-dimethyl-amine
[1-[4[[(2S)-1-[(4-methoxy-2,6-dimethyl-phenyl)sulfonyl]-pyrrolidin-2-yl]-methoxy]-pyrimidin-2-yl]-4-thiophen-2-yl-piperidin-4-yl]-dimethyl-amine
4-methoxy-N,2,6-trimethyl-N-[2-[2-[2-(pyridin-2-yl-methyl)-pyrrolidin-1-yl]-pyrimidin-4-yl]oxyethyl]-benzenesulfonic acid amide
4-[[(2S)-1-[(4-methoxy-2,6-dimethyl-phenyl)sulfonyl]-pyrrolidin-2-yl]-methoxy]-2-[2-(pyridin-2-yl-methyl)-pyrrolidin-1-yl]-pyrimidine
4-methoxy-N,2,6-trimethyl-N-[2-[2-[methyl-[2-(4-pyridin-4-yloxy-piperidin-1-yl)-ethyl]-amino]-pyrimidin-4-yl]oxy-ethyl]-benzenesulfonic acid amide
N-[2-[2-[4-(3-fluorophenyl)-4-(2-pyrrolidin-1-yl-ethoxy)-piperidin-1-yl]-pyrimidin-4-yl]oxy-ethyl]-4-methoxy-N,2,6-trimethyl-benzenesulfonic acid amide
2-[4-(3-fluorophenyl)-4-(4-methyl-piperazin-1-yl)-piperidin-1-yl]-4-[[1-[(4-methoxy-2,6-dimethylphenyl)sulfonyl]-piperidin-2-yl]-methoxy]-pyrimidine
2-[4-(3-fluorophenyl)-4-(2-pyrrolidin-1-yl-ethoxy)-piperidin-1-yl]-4-[[(2S)-1-[(4-methoxy-2,6-dimethyl-phenyl)sulfonyl]-pyrrolidin-2-yl]-methoxyl-pyrimidine
4-methoxy-N,2,6-trimethyl-N-[1-phenyl-2-[2-[(1S,5R)-3-pyridin-4-yloxy-8-azabicyclo[3.2.1]octan-8-yl]-pyrimidin-4-yl]oxy-ethyl]-benzenesulfonic acid amide

17. A medicament comprising at least one compound as claimed in one or more of claims 1 to 15, optionally comprising suitable additives and/or auxiliary substances and/or further active ingredients.

18. The use of at least one compound as claimed in any one of claims 1 to 15 in the preparation of a medicament for the treatment of pain, in particular of acute pain, visceral pain, neuropathic pain and/or chronic pain, inflammatory pain, migraine, diabetes, respiratory diseases, inflammatory intestinal diseases, neurological diseases, inflammations of the skin, rheumatic diseases, septic shock, reperfusion syndrome, obesity, and as an angiogenesis inhibitor.

## Revendications

1. Composé selon la formule générale I 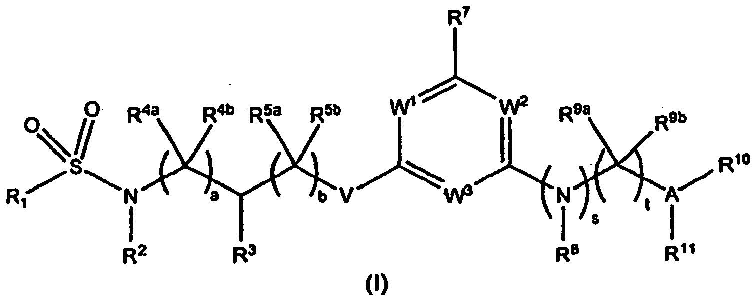 dans laquelle :
a vaut 0, 1 ou 2 ;
b vaut 0, 1 ou 2 ;
R¹ représente un groupe aryle, hétéroaryle, CH(aryle)₂ ou un groupe aryle ou hétéroaryle lié par l'intermédiaire d'un groupe alkylène en C₁ à C₃ ;
R² et R³ sont définis tel que décrit au point (i) ou (ii) :
(i) R² représente H, un groupe alkyle en C₁₋₆, cycloalkyle en C₃₋₈, aryle ou hétéroaryle ; ou R² représente un groupe cycloalkyle en C₃₋₈, aryle ou hétéroaryle lié par l'intermédiaire d'un groupe alkylène en C₁₋₆, alcénylène en C₂₋₆ ou alcynylène en C₂₋₆ ;
R³ représente un H, F, Cl, Br, I, un groupe -CF₃, -OCF₃, OH, O-(alkyle en C₁ à C₆), alkyle en C₁ à C₆, cycloalkyle en C₃ à C₈, aryle, hétéroaryle ; ou R³ représente un groupe cycloalkyle en C₃ à C₈, aryle ou hétéroaryle lié par l'intermédiaire d'un groupe alkylène en C₁ à C₆, alcénylène en C₂ à C₆ ou alcynylène en C₂ à C₆ ;
ou
(ii) R² et R³ forment, conjointement avec le groupe -N-(CR^{4a}R^{4b})ₐ-CH- par lequel ils sont liés, un hétérocycle qui peut être substitué au niveau d'un ou plusieurs de ses éléments du cycle carbone par un ou plusieurs groupes choisis indépendamment les uns des autres dans le groupe formé par F, Cl, Br, I, -CF₃, =O, -O-CF₃, -OH, -SH, -O-(alkyle en C₁ à C₆), alkyle en C₁ à C₆, cycloalkyle en C₃ à C₈, aryle et hétéroaryle et/ou condensé par un groupe aryle ou hétéroaryle et/ou deux de ses éléments du cycle carbone peuvent être reliés par l'intermédiaire d'un pont alkylène en C₁ à C₃,
où l'hétérocycle est saturé ou au moins mono-insaturé, mais pas aromatique, présente 4, 5, 6 ou 7 éléments, peut contenir, outre le N-hétéroatome auquel le groupe R² est lié, un ou plusieurs hétéroatomes ou groupes d'hétéroatomes choisis indépendamment les uns des autres dans le groupe formé par N, NR⁵⁰, O, S, S=O ou S(=O)₂ ; le groupe R⁵⁰ représentant H, un groupe alkyle en C₁ à C₆, -C(=O)-R⁵¹, cycloalkyle en C₃ à C₈, aryle, hétéroaryle ou cycloalkyle en C₃ à C₈, aryle ou hétéroaryle lié par l'intermédiaire d'un groupe alkylène en C₁ à C₃, et R⁵¹ représentant un groupe alkyle en C₁ à C₆, cycloalkyle en C₃ à C₈, aryle, hétéroaryle ou un groupe cycloalkyle en C₃ à C₈, aryle ou hétéroaryle lié par l'intermédiaire d'un groupe alkylène en C₁ à C₃ ;
V représente un groupe C(R^{6a}) (R^{6b}), NR^{6c}, O ou une liaison simple,
R^{6c} représentant un groupe choisi dans le groupe formé par H, alkyle en C₁ à C₆, cycloalkyle en C₃ à C₈, aryle, hétéroaryle ou un groupe cycloalkyle en C₃ à C₈, aryle ou hétéroaryle lié par l'intermédiaire d'un groupe alkylène en C₁ à C₆, alcénylène en C₂ à C₆ ou alcynylène en C₂ à C₆, R^{4a}, R^{4b}, R^{5a}, R^{5b}, R^{6a}, R^{6b} représentent, indépendamment les uns des autres, H, F, Cl, Br, I, -CF₃, -OCF₃, OH, SH, O-(alkyle en C₁ à C₆), alkyle en C₁ à C₆, cycloalkyle en C₃ à C₈, aryle, ou hétéroaryle ; ou un groupe cycloalkyle en C₃ à C₈, aryle ou hétéroaryle lié par l'intermédiaire d'un groupe alkylène en C₁ à C₆ ou alcénylène en C₂ à C₆ ; et R^{6a} et R^{6b} peuvent en outre représenter ensemble =O ;
et/ou
R^{4a} et R^{4b} forment un cycle saturé conjointement avec l'atome de carbone par lequel ils sont liés, lequel cycle peut être non substitué ou substitué au niveau d'un ou
plusieurs, par exemple 1, 2, 3 ou 4, de ses éléments du cycle carbone, par un ou plusieurs substituants, par exemple 1, 2, 3 ou 4 substituants choisis indépendamment les uns des autres dans le groupe formé par F, CF₃, alkyle en C₁ à C₆, O-(alkyle en C₁ à C₆), OH, OCF₃, aryle et
hétéroaryle, le cycle présentant 3, 4, 5 ou 6 éléments et
pouvant contenir éventuellement un ou plusieurs, par exemple 1 ou 2, atomes d'oxygène ;
R⁷ représente un substituant choisi dans le groupe formé par H, un groupe alkyle en C₁ à C₆, -CN, -CF₃, OH, alkoxy en C₁ à C₆, -O-CF₃ ;
W¹, W² et W³ représentent, indépendamment les uns des autres, N ou un groupe CR⁶⁰, à condition que parmi W¹, W² et
W³ au moins deux représentent N, et R⁶⁰ représente H, un groupe alkyle en C₁ à C₆, halogèno, -CN, CF₃, OH, alkoxy en C₁ à C₆ ou -O-CF₃ ;
s est égal à 0 ou 1,
t est égal à 0, 1, 2 ou 3,
R⁸ représente H ; un groupe alkyle en C₁ à C₆ ; cycloalkyle en C₃ à C₈ ; aryle ou hétéroaryle ; un groupe cycloalkyle en C₃ à C₈, aryle ou hétéroaryle lié par l'intermédiaire d'un groupe alkylène en C₁ à C₆ ;
R^{9a} et R^{9b} représentent, chacun indépendamment l'un de l'autre, un H ; F ; Cl ; un groupe OH ; alkyle en C₁ à C₆ ; O-(alkyle en C₁ à C₆) ; cycloalkyle en C₃ à C₈ ; aryle ou hétéroaryle ; un groupe cycloalkyle en C₃ à C₈, aryle ou hétéroaryle lié par l'intermédiaire d'un groupe alkylène en C₁₋₆ ;
A représente N ou un groupe CH,
à condition que, lorsque s vaut 1 et t vaut 0, A représente un groupe CH ; et
à condition que, lorsque s et t valent chacun 0, A représente N ;
les groupes R¹⁰ et R¹¹ représentent, conjointement avec A, un groupe spirocyclique ou cyclique selon l'une des formules générales II ou III, 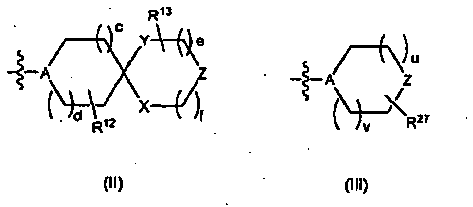 dans lesquelles :
c, d, e, f, u et v valent chacun, indépendamment les uns des autres, 0, 1 ou 2 ;
R¹², R¹³ et R²⁷ représentent chacun, indépendamment les uns des autres, 0 à 4 substituants choisis indépendamment les uns des autres parmi F ; Cl ; OH ; =O ; un groupe alkyle en C₁ à C₆ ; O-(alkyle en C₁ à C₆) ; cycloalkyle en C₃ à C₈ ; aryle ou hétéroaryle ;
un groupe cycloalkyle en C₃ à C₈, aryle ou hétéroaryle lié par l'intermédiaire d'un groupe alkylène en C₁ à C₆ ;
et/ou à chaque fois deux des 0 à 4 substituants R²⁷ forment ensemble un pont alkylène en C₁ à C₃, si bien que le cycle représenté par la formule générale III prend une forme pontée bicyclique ;
et/ou deux substituants adjacents des 0 à 4 substituants R¹³ forment un groupe aryle ou hétéroaryle condensé ;
et/ou deux substituants adjacents des 0 à 4 substituants R²⁷ forment un groupe aryle ou hétéroaryle condensé ;
X représente un groupe CR^{14a}R^{14b}, NR¹⁵ ou O ;
Y représente un groupe CR^{16a}R^{16b}, NR¹⁷ ou O ;
à condition que X ne représente pas un groupe NR¹⁵ lorsque Y représente un groupe NR¹⁷ ; et
à condition que X et Y ne représentent pas en même temps O ;
où
R^{14a}, R^{14b}, R^{16a} et R^{16b} représentent chacun, indépendamment les uns des autres, H ; F ; Cl ; un groupe OH ; alkyle en C₁ à C₆ ; O-(alkyle en C₁ à C₆) ; cycloalkyle en C₃ à C₈ ; aryle ou hétéroaryle ; un groupe cycloalkyle en C₃ à C₈, aryle et hétéroaryle lié par l'intermédiaire d'un groupe alkylène en C₁ à C₆ ;
et/ou R^{14a} et R^{14b} peuvent représenter ensemble =O et/ou R^{16a} et R^{16b} peuvent représenter ensemble =O ;
R¹⁵ et R¹⁷ représentent chacun, indépendamment l'un de l'autre, H ; un groupe alkyle en C₁ à C₆ ; un groupe cycloalkyle en C₃ à C₈, aryle ou hétéroaryle ; un groupe cycloalkyle en C₃ à C₈, aryle ou hétéroaryle lié par l'intermédiaire d'un groupe alkylène en C₁ à C₆ ;
Z dans la formule générale II, représente un groupe CR^{18a}R^{18b}, NR¹⁹ ou O ; ou
Z dans la formule générale II, dans le cas où X représente O et f vaut 0, représente un groupe -(C(R¹²⁴) -C(R¹²⁵)), où
R¹²⁴ et R¹²⁵ représentent conjointement avec les atomes de carbone par lesquels ils sont liés, un groupe aryle ou hétéroaryle condensé ; ou
Z dans la formule générale II, dans le cas où X représente O et f vaut 0, représente un groupe =(N(CR¹²⁶))-, l'atome de N étant lié par une liaison simple à l'atome de O, et
R¹²⁶ représente H ; un groupe alkyle en C₁ à C₆ ; cycloalkyle en C₃ à C₈ ; aryle ou hétéroaryle ; un groupe cycloalkyle en C₃ à C₈, aryle ou hétéroaryle lié par l'intermédiaire d'un groupe alkylène en C₁ à C₆ ;
Z dans la formule générale III, représente un groupe CR^{18a}R^{18b}, NR¹⁹, O, S, S(=O) ou S(=O)₂ ;
où
R^{18a} représente H ; un groupe alkyle en C₁ à C₆ ; cycloalkyle en C₃ à C₈ ; aryle ou hétéroaryle ; un groupe cycloalkyle en C₃ à C₈, aryle ou hétéroaryle lié par l'intermédiaire d'un groupe alkylène en C₁ à C₆ ;
ou R^{18a} représente un groupe selon la formule générale IV, 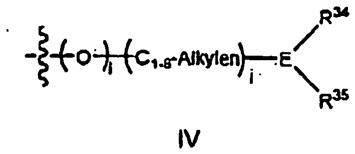 dans laquelle :
i et j valent chacun indépendamment l'un de l'autre 0 ou 1 ;
E représente N ou un groupe CH, à condition que, lorsque i vaut 1 et j vaut 0,
E représente un groupe CH ;
R³⁴ et R³⁵ représentent, chacun indépendamment l'un de l'autre, H ; un groupe alkyle en C₁ à C₆ ; cycloalkyle C₃ à C₈ ; aryle ou hétéroaryle ; un groupe cycloalkyle en C₃ à C₈, aryle ou hétéroaryle lié par l'intermédiaire d'un groupe alkylène en C₁ à C₃ ;
ou R³⁴ et R³⁵ forment, conjointement avec E, un groupe aryle ou hétéroaryle à 5 ou 6 éléments ;
ou R³⁴ et R³⁵ forment, conjointement avec E, un hétérocycle saturé selon la formule générale V, 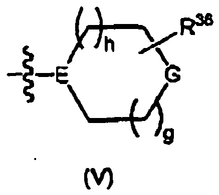
dans laquelle :
h et g valent indépendamment l'un de l'autre 0, 1 ou 2 ;
G représente un groupe CR^{37a}R^{37b}, NR³⁸, O, S, S=O ou S(=O)₂,
à condition que, lorsque E représente un groupe CH, G ne représente pas un groupe CR^{37a}R^{37b} ;
R³⁶ représente 0 à 4 substituants, choisis chacun indépendamment les uns des autres parmi F ; Cl ; Br ; I ; un groupe OH ; SH ; =O ; O-(alkyle en C₁ à C₆) ; un groupe alkyle en C₁ à C₆ ; cycloalkyle en C₃ à C₈ ; aryle ou hétéroaryle ; un groupe cycloalkyle en C₃ à C₈, aryle ou hétéroaryle lié par l'intermédiaire d'un groupe alkylène en C₁ à C₆ ;
et/ou deux substituants adjacents R³⁶ représentent ensemble un groupe aryle ou hétéroaryle condensé ;
R^{37a} et R^{37b} représentent, chacun indépendamment l'un de l'autre, H ; F ; Cl ; Br ; I ; un groupe OH ; SH ; =O ; O-(alkyle en C₁ à C₆) ; un groupe alkyle en C₁ à C₆ ; cycloalkyle en C₃ à C₈ ; aryle ou hétéroaryle ; un groupe cycloalkyle en C₃ à C₈, aryle ou hétéroaryle lié par l'intermédiaire d'un groupe alkylène en C₁ à C₆ ;
R^{3a} représente H ; un groupe alkyle en C₁ à C₆ ; cycloalkyle en C₃ à C₈ ; aryle ou hétéroaryle ; un groupe cycloalkyle en C₃ à C₈, aryle ou hétéroaryle lié par l'intermédiaire d'un groupe alkylène en C₁ à C₃ ;
où
R^{18b} représente H ; un groupe OH ; alkyle en C₁ à C₆ ; cycloalkyle C₃ à C₈ ; O-(alkyle en C₁ à C₆) ; O-(cycloalkyle en C₃ à C₈) ; (alkylène en C₁ à C₆)-O-(alkyle en C₁ à C₆); (alkylène en C₁ à C₆) - O-(cycloalkyle en C₃ à C₈) ; aryle ou hétéroaryle ; O-aryle ou O-hétéroaryle ; aryle, O-aryle, hétéroaryle ou O-hétéroaryle lié par l'intermédiaire d'un groupe alkylène en C₁ à C₆ ; ou R^{18b} représente un groupe selon la formule générale VI, 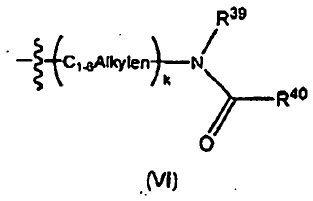 dans laquelle :
k vaut 0 ou 1 ;
R³⁹ représente H ; un groupe alkyle en C₁ à C₆ ; cycloalkyle en C₃ à C₈ ; aryle ou hétéroaryle ; un groupe cycloalkyle en C₃ à C₈, aryle ou hétéroaryle lié par l'intermédiaire d'un groupe alkylène en C₁ à C₃ ;
R⁴⁰ représente un groupe alkyle en C₁ à C₆ ; cycloalkyle en C₃ à C₈ ; aryle ou hétéroaryle ; un groupe cycloalkyle en C₃ à C₈, aryle ou hétéroaryle lié par l'intermédiaire d'un groupe alkylène en C₁ à C₆ ;
ou
R³⁹ et R⁴⁰ représentent conjointement avec le groupe N-C(=O) par lequel ils sont liés, un cycle selon la formule générale VII, 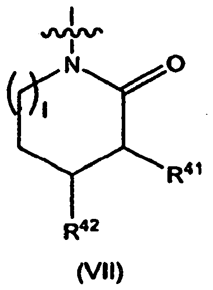
dans laquelle :
I vaut 0, 1 ou 2
et R⁴¹ et R⁴² représentent conjointement avec les atomes de carbone par lesquels ils sont liés, un groupe aryle ou hétéroaryle condensé ;
où R¹⁹ représente H ; ou un groupe (P)_{z}-R²², dans lequel :
z vaut 0 ou 1 ;
P représente un groupe (C=O), S(=O)₂ ou C(=O)-N(R²⁴); où l'atome de N dans le groupe C(=O)-N(R²⁴) est relié à R²², où
R²⁴ représente H ; un groupe alkyle en C₁ à C₆ ; cycloalkyle C₃ à C₈ ; un groupe aryle, hétéroaryle ou cycloalkyle en C₃ à C₈ lié par l'intermédiaire d'un groupe alkylène en C₁ à C₃ ;
R²² représente un groupe alkyle en C₁ à C₆ ; aryle ou hétéroaryle ; un groupe aryle ou hétéroaryle lié par l'intermédiaire d'un groupe alkylène en C₁ à C₆ ; ou
R²² représente un groupe selon la formule générale VIII, 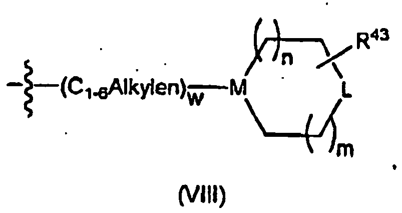 dans laquelle :
n vaut 0, 1 ou 2 ;
m vaut 0, 1 ou 2 ;
w vaut 0 ou 1,
M représente un groupe CH ou N ;
à condition que, lorsque P représente un groupe C(=O)-NR²⁴ et w vaut 0, M représente un groupe CH ; et
à condition que, lorsque z et w valent en même temps 0, M représente un groupe CH ;
L représente un groupe CR^{44a}R^{44b}, NR⁴⁵, O, S, S=O ou S (=O) ;
R⁴³ représente 0 à 4 substituants, choisis chacun indépendamment les uns des autres parmi F ; Cl ; un groupe OH ; =O ; alkyle en C₁ à C₆ ; O-(alkyle en C₁ à C₆) ; cycloalkyle en C₃ à C₈ ; aryle ou hétéroaryle ; un groupe cycloalkyle en C₃ à C₈, aryle ou hétéroaryle lié par l'intermédiaire d'un groupe alkylène en C₁ à C₆ ;
et/ou deux substituants adjacents des 0 à 4 substituants R⁴³ représentent ensemble un groupe aryle ou hétéroaryle condensé ;
R^{44a} et R^{44b} représentent chacun, indépendamment l'un de l'autre, H ; F ; Cl ; Br ; I ; un groupe OH ; alkyle en C₁ à C₆ ; O- (alkyle en C₁ à C₆) ;
cycloalkyle en C₃ à C₈ ; aryle ou hétéroaryle ; un groupe cycloalkyle en C₃ à C₈, aryle ou hétéroaryle lié par l'intermédiaire d'un groupe alkylène en C₁ à C₆ ;
ou R^{44a} et R^{44b} peuvent représenter ensemble =O ;
R⁴⁵ représente H ; un groupe alkyle en C₁ à C₆ ; cycloalkyle C₃ à C₈ ; aryle ou hétéroaryle ; un groupe aryle, hétéroaryle ou cycloalkyle en C₃ à C₈, lié par l'intermédiaire d'un groupe alkylène en C₁ à C₃ ;
où les autres groupes mentionnés alkyle en C₁ à C₆, alkylène en C₁ à C₃, alkylène en C₁ à C₆, alcénylène en C₂ à C₆, alcynylène en C₂ à C₆, cycloalkyle en C₃ à C₆, cycloalkyle en C₃ à C₈, aryle et hétéroaryle peuvent chacun être non substitué ou substitué une ou plusieurs fois par un ou plusieurs groupes identiques ou différents ; les groupes susmentionnés alkyle en C₁ à C₆ , alkylène en C₁ à C₃ , alkylène en C₁ à C₆ , alcénylène en C₂ à C₆ et alcynylène en C₂ à C₆, peuvent être chacun ramifié ou non ramifié ;
sous la forme d'un énantiomère individuel ou d'un diastéréomère individuel, du racémate, des énantiomères, des diastéréomères, de mélanges des énantiomères et/ou diastéréomères, ainsi qu'à chaque fois sous la forme de leurs bases et/ou de leurs sels physiologiquement acceptables.

2. Composé selon la revendication 1, dans lequel :
W¹ et W³ représentent N et W² représente un groupe CR⁶⁰ ; ou
W¹ et W² représentent N et W³ représente un groupe CR⁶⁰ , ou
W¹, W² et W³ représentent N.

3. Composé selon la revendication 1 ou 2, dans lequel :
V représente O.

4. Composé selon l'une quelconque des revendications 1 à 3, dans lequel :
R¹ représente un groupe phényle, naphtyle, indolyle, benzofuranyle, benzothiophényle (benzothiényle) ; benzooxazolyle, benzooxadiazolyle, pyrrolyle, furanyle, thiényle, pyridinyle, pyridazinyle, pyrimidinyle, pyrazinyle, imidazothiazolyle, carbazolyle, dibenzofuranyle, dibenzothiophényle, (dibenzothiényle) ou CH(phényle)₂, de préférence un groupe phényle, naphthyle, benzothiophényle, benzooxadiazolyle, thiophényle, pyridinyle, imidazothiazolyle ou dibenzofuranyle, de manière plus particulièrement préférée un groupe phényle ou naphtyle, à chaque fois non substitué ou substitué une ou plusieurs fois, de manière identique ou différente, les substituants étant choisis de préférence parmi des substituants -O-(alkyle en C₁ à C₃), alkyle en C₁ à C₆, F, Cl, Br, I, CF₃, OCF₃, OH, SH, phényle, naphtyle, furyle, thiazolyle, thiényle et pyridinyle.

5. Composé selon l'une quelconque des revendications 1 à 4, dans lequel, dans la formule générale I, la structure partielle (Ac I) 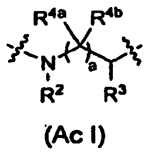 représente : 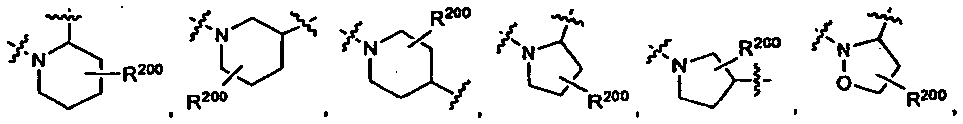 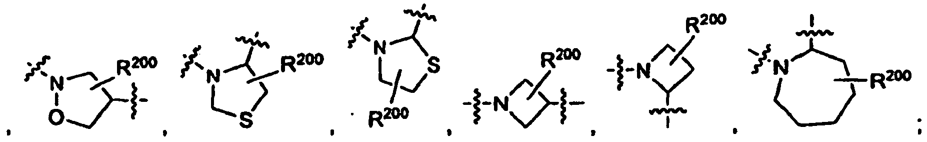 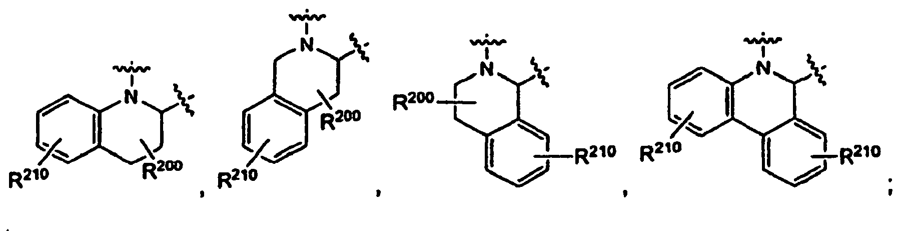 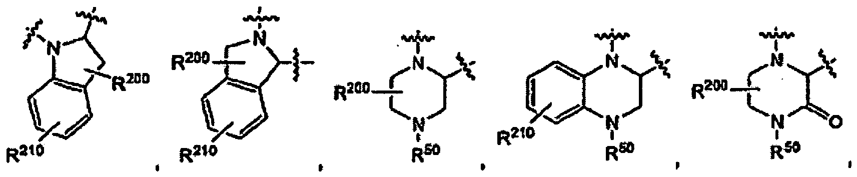 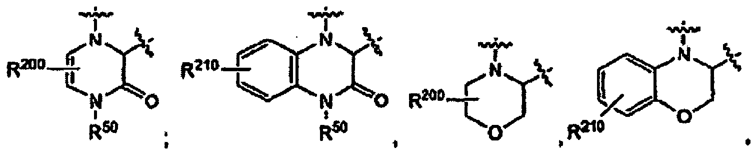 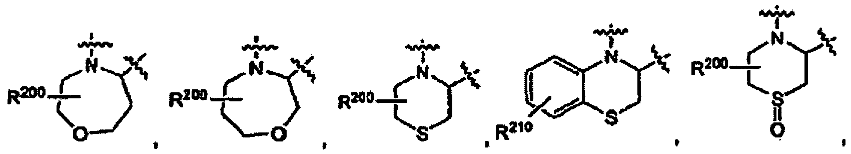 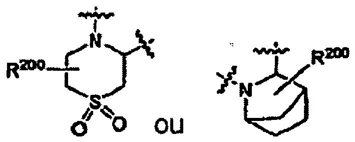 où :
R²⁰⁰ représente 0 à 4 substituants choisis indépendamment les uns des autres parmi des substituants F, Cl, -CF₃, =O, -O-CF₃, -OH, -O-(alkyle en C₁ à C₆) ou alkyle en C₁ à C₆ de préférence F ou CF₃ ou deux des groupes R²⁰⁰ représentent un groupe aryle condensé, en particulier un groupe benzo ;
R²¹⁰ représente 0 à 4 substituants choisis indépendamment les uns des autres parmi des substituants -O- (alkyle en C₁ à C₃), alkyle en C₁ à C₆, F ; Cl, Br, I, CF₃, OCF₃, OH, SH, phényle, naphtyle, furyle, thiényle et pyridinyle, de préférence méthyle, méthoxy, O-CF₃, CF₃, F, Cl et Br.

6. Composé selon l'une quelconque des revendications 1 à 5, dans lequel :
R² représente H, un groupe alkyle en C₁ à C₆, cycloalkyle en C₃ à C₆, aryle, ou cycloalkyle en C₃ à C₆ ou aryle lié par l'intermédiaire d'un groupe alkylène en C₁ à C₃, en particulier H, un groupe méthyle, éthyle, n-propyle, iso-propyle, n-butyle, iso-butyle, tertio-butyle, cyclopropyle, phényle, pyridinyle ou un groupe phényle ou pyridinyle lié par l'intermédiaire d'un groupe alkylène en C₁ à C₃ ; chacun non substitué ou substitué une ou plusieurs fois par des substituants identiques ou différents ;
et
R³ représente H, F, Cl, un groupe -CF₃, -OH, -O-(alkyle en C₁ à C₆), alkyle en C₁ à C₆ ou aryle ; ou un groupe aryle lié par l'intermédiaire d'un groupe alkylène en C₁ à C₃, à chaque fois non substitué ou substitué une ou plusieurs fois par des groupes identiques ou différents.

7. Composé selon l'une quelconque des revendications 1 à 6, dans lequel :
a + b = 1.

8. Composés substitués selon une ou plusieurs des revendications 1 à 7, dans lesquels :
(a1) la formule générale II présente la structure partielle IIa suivante : 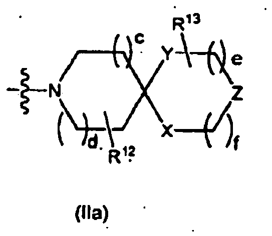 ou
(a2) la formule générale III présente l'une des structures partielles IIIa ou IIIb suivantes :

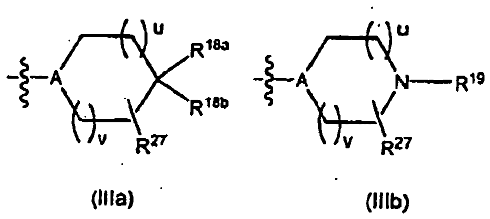

9. Composés substitués selon la revendication 8, dans lesquels :
(a1) la structure partielle de formule IIa présente la structure partielle IIb suivante: 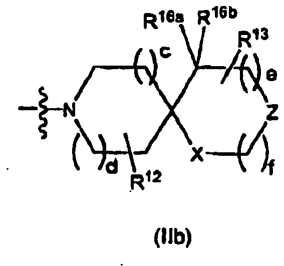 ou
(a2) les structures partielles de formules IIIa et IIIb présentent l'une des structures partielles IIIc, IIId ou IIIe suivantes :

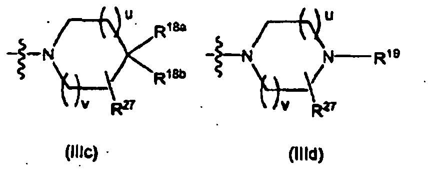

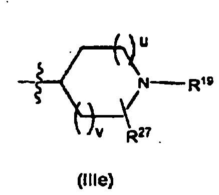

10. Composés substitués selon la revendication 9, dans lesquels :
(a1) la structure partielle de formule IIa présente la structure partielle IIb,
R⁸ représente H, un groupe alkyle en C₁ à C₆, cycloalkyle en C₃ à C₆, chacun non substitué ou substitué une ou plusieurs fois par des substituants identiques ou différents, et
R^{9a} et R^{9b} représentent chacun H ;
et/ou
(a2) les structures partielles des formules IIIa et IIIb représentent l'une des structures partielles IIIc ou IIId, et s et t valent chacun 0;
et/ou
(a3) les structures partielles des formules IIIa et IIIb représentent l'une des structures partielles IIIc ou IIId et deux des substituants R²⁷ représentent ensemble un pont alkylène en C₁ à C₃, de sorte que le cycle représenté dans la structure partielle IIIc ou IIId prend une forme pontée bicyclique, et
s et t sont chacun = 0 ;
et/ou
(a4) les structures partielles des formules IIIa et IIIb représentent l'une des structures partielles IIIc ou IIIe, s vaut 1 et t vaut 1, 2 ou 3 et R⁸ représente H, un groupe alkyle en C₁ à C₆ ou cycloalkyle en C₃ à C₆, chacun non substitué ou substitué une ou plusieurs fois par des substituants identiques ou différents.

11. Composés substitués selon la revendication 10, dans lesquels :
(a1) la structure partielle IIb représente la structure partielle IIc suivante : 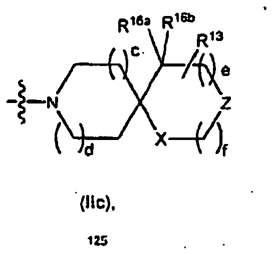 et s et t valant chacun 0 ;
et/ou
(a2) les structures partielles IIIc ou IIId représentent l'une des structures partielles IIIf ou IIIg suivantes, 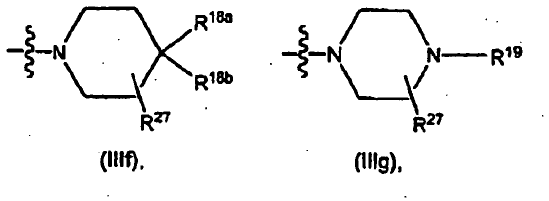 dans lesquelles :
R²⁷ représente H ou un groupe méthyle et/ou deux substituants adjacents R²⁷ forment un groupe aryle ou hétéroaryle condensé, en particulier un groupe benzo ;
et/ou
(a3) les structures partielles IIIc ou IIId représentent, dans les composés, l'un des groupes A à H suivants, 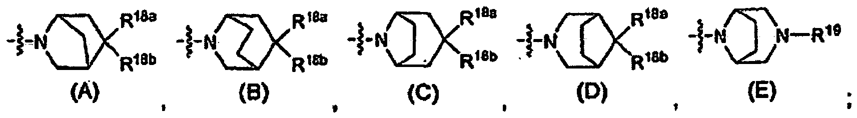 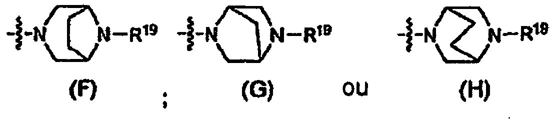 et/ou
(a4) les structures partielles IIIc ou IIIe représentent, dans les composés, un groupe de formules IIIh ou IIIi, 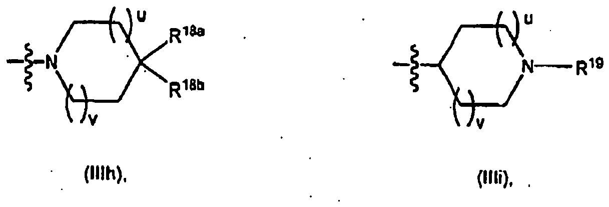
et R^{9a} et R^{9b} représentent chacun H .

12. Composés substitués selon la revendication 11, dans lesquels :
(a1) les groupes _{R}^{16a} et R^{16b}, dans la structure partielle IIc, représentent chacun H ou forment ensemble un groupe =O ;
R¹³ représente un groupe aryle ou hétéroaryle et/ou deux des substituants R¹³ forment ensemble un groupe =O,
et/ou deux substituants adjacents R¹³ forment un groupe aryle ou hétéroaryle condensé, en particulier un groupe benzo,
et/ou
(a2) dans les structures partielles IIIf ou IIIg R^{18a} représente H ; un groupe alkyle en C₁ à C₆ ; cycloalkyle en C₃ à C₈, -NH(alkyle en C₁ à C₆), -N(alkyle en C₁ à C₆)₂, phényle, pyridyle, pyrimidinyle, thiazolyle, imidazolyle, triazolyle ou thiényle, chacun non substitué ou substitué une ou plusieurs fois ; un groupe phényle, pyridyle, pyrimidinyle, imidazolyle, triazolyle ou thiényle lié par l'intermédiaire d'un groupe -(O)₀₋₁-alkylène en C₁ à C₆), chacun non substitué ou substitué une ou plusieurs fois ;
ou
R^{18a} représente le groupe selon la formule générale VIIa 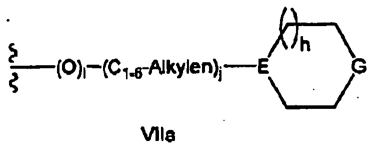 dans laquelle :
i vaut 0 ou 1 ;
j vaut 0 ou 1 ;
h vaut 0 ou 1 ;
E représente N ou un groupe CH, à condition que, lorsque i vaut 1 et j vaut 0, E représente un groupe CH ;
G représente un groupe CR^{37a}R^{37b} ou NR³⁸ ;
où R^{37a} et R^{37b} représentent, chacun indépendamment l'un de l'autre, H ; F, ou un groupe alkyle en C₁ à C₆, à chaque fois non substitué ou substitué une ou plusieurs fois par des substituants identiques ou différents ;
R³⁸ représente H ; un groupe alkyle en C₁ à C₆, cycloalkyle en C₃ à C₆ ou pyridyle ;
R^{18b} représente H ; un groupe OH ; un groupe alkyle en C₁ à C₆ ; phényle, pyridyle, pyrimidinyle, imidazolyle, triazolyle, thiényle ou thiazolyle ; chacun non substitué ou substitué une ou plusieurs fois par des substituants identiques ou différents ; un groupe phényle, pyridyle, pyrimidinyle, O-phényle, O-pyridyle, imidazolyle, triazolyle, thiényle ou thiazolyle lié par l'intermédiaire d'un groupe alkylène en C₁ à C₆, chacun non substitué ou substitué une ou plusieurs fois par des substituants identiques ou différents ; un groupe phényle, pyridyle, pyrimidinyle, imidazolyle, triazolyle, thiazolyle ou thiényle ponté par l'intermédiaire d'un groupe (alkylène en C₁ à C₆)-NH(C=O), chacun non substitué ou substitué une ou plusieurs fois par des substituants identiques ou différents
R¹⁹ représente H ; un groupe alkyle en C₁ à C₆ ; cycloalkyle en C₃ à C₈, ou un groupe alkyle en C₁ à C₆ lié par l'intermédiaire d'un groupe (C=O)₀₋₁ ; phényle, pyridyle, thiényle, thiazolyle, triazolyle, pyrimidinyle ou imidazolyle ; chacun non substitué ou substitué une ou plusieurs fois par des substituants identiques ou différents ; un groupe phényle, pyridyle, thiényle, thiazolyle, pyrimidinyle, triazolyle ou imidazolyle lié par l'intermédiaire d'un groupe alkylène en C₁ à C₆ ; chacun non substitué ou substitué une ou plusieurs fois par des substituants identiques ou différents ;
ou un groupe selon la formule générale VIIIa 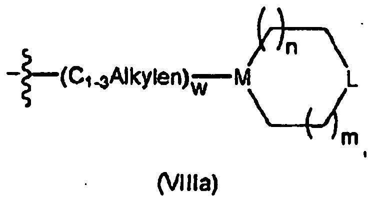 dans laquelle :
w vaut 0 ou 1 ;
n vaut 0 ou 1 ;
m vaut 0 ou 1 ;
M représente un groupe CH ou N, à condition que, lorsque w vaut 0, M représente un groupe CH ;
L représente un groupe CR^{44a}R^{44b} ou NR⁴⁵ ;
où R^{44a} et R^{44b} représentent chacun, indépendamment l'un de l'autre, H ; F ou un groupe alkyle en C₁ à C₆, à chaque fois non substitué ou substitué une ou plusieurs fois par des substituants identiques ou différents ;
R⁴⁵ représente H ; un groupe alkyle en C₁ à C₆, cycloalkyle en C₃ à C₆ ou pyridyle ;
et/ou
(a3) les structures partielles IIIc ou IIId représentent, dans les composés, l'un des groupes A à H suivants, 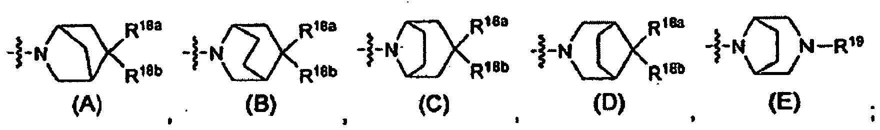 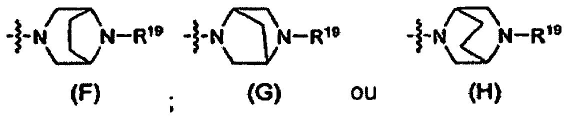 et où
R^{18a} représente H ; un groupe alkyle en C₁ à C₆ ; cycloalkyle en C₃ à C₈, N (alkyle en C₁ à C₆)₂ ; NH (alkyle en C₁ à C₆) ; azétidinyle ; pyrrolidinyle, pipéridinyle, 4-(alkyle en C₁ à C₆)-pipérazinyle ; phényle, pyridyle, pyrimidinyle, imidazolyle, triazolyle, thiazolyle ou thiényle, chacun non substitué ou substitué une ou plusieurs fois par des substituants identiques ou différents ; un groupe N (alkyle en C₁ à C₆)₂ ; NH (alkyle en C₁ à C₆) ; azétidinyle ; pyrrolidinyle, pipéridinyle, 4-(alkyle en C₁ à C₆)-pipérazinyle ; phényle, imidazolyle, triazolyle, thiényle, thiazolyle, pyrimidinyle ou pyridyle lié par l'intermédiaire d'un groupe - (O)₀₋₁₋(alkylène en C₁ à C₆), chacun non substitué ou substitué une ou plusieurs fois par des substituants identiques ou différents ;
R^{18b} représente H ; un groupe OH ; un groupe alkyle en C₁ à C₆ ; phényle, pyridyle, pyrimidinyle, imidazolyle, triazolyle, thiazolyle ou thiényle, chacun non substitué ou substitué une ou plusieurs fois par des substituants identiques ou différents ; un groupe phényle, pyridyle, pyrimidinyle, imidazolyle, triazolyle, thiazolyle ou thiényle lié par l'intermédiaire d'un groupe alkylène en C₁ à C₆, chacun non substitué ou substitué une ou plusieurs fois par des substituants identiques ou différents ;
R¹⁹ représente H ; un groupe alkyle en C₁ à C₆ ; cycloalkyle en C₃ à C₈, phényle, pyridyle, pyrimidinyle, thiényle, imidazolyle, thiazolyle, ou triazolyle, chacun non substitué ou substitué une ou plusieurs fois par des substituants identiques ou différents ; un groupe phényle, pyridyle, pyrimidinyle, thiényle, imidazolyle, thiazolyle, ou triazolyle lié par l'intermédiaire d'un groupe alkylène en C₁ à C₆ ou un groupe (C=O)-, chacun non substitué ou substitué une ou plusieurs fois par des substituants identiques ou différents ;
et/ou
(a4) dans les structures partielles IIIh ou IIIi
R^{18a} représente H _{;} un groupe alkyle en C₁ à C₆ ; cycloalkyle en C₃ à C₈, N (alkyle en C₁ à C₆)₂ ; NH (alkyle en C₁ à C₆), azétidinyle ; pyrrolidinyle, pipéridinyle, 4-(alkyle en C₁ à C₆)-pipérazinyle ; phényle, pyridyle, pyrimidinyle, thiényle, imidazolyle, thiazolyle ou triazolyle, chacun non substitué ou substitué une ou plusieurs fois par des substituants identiques ou différents ; un groupe N (alkyle en C₁ à C₆)₂ ; NH (alkyle en C₁ à C₆), azétidinyle ; pyrrolidinyle, pipéridinyle, 4-(alkyle en C₁ à C₆)-pipérazinyle ; phényle, pyridyle, pyrimidinyle, thiényle, imidazolyle, thiazolyle ou triazolyle lié par l'intermédiaire d'un groupe - (O)_{0/1-} (alkylène en C₁ à C₆), chacun non substitué ou substitué une ou plusieurs fois par des substituants identiques ou différents ;
R^{18b} représente H ; un groupe OH ; un groupe alkyle en C₁ à C₆ ; phényle, pyridyle, pyrimidinyle, thiényle, imidazolyle, thiazolyle ou triazolyle, chacun non substitué ou substitué une ou plusieurs fois par des substituants identiques ou différents ; un groupe phényle, pyridyle, pyrimidinyle, thiényle, imidazolyle, thiazolyle ou triazolyle lié par l'intermédiaire d'un groupe alkylène en C₁ à C₆, chacun non substitué ou substitué une ou plusieurs fois par des substituants identiques ou différents ;
R¹⁹ représente H ; un groupe alkyle en C₁ à C₆ ; cycloalkyle en C₃ à C₈, phényle, pyridyle, pyrimidinyle, thiényle, imidazolyle, thiazolyle, ou triazolyle, chacun non substitué ou substitué une ou plusieurs fois par des substituants identiques ou différents ; un groupe phényle, pyridyle, pyrimidinyle, thiényle, imidazolyle, thiazolyle, ou triazolyle lié par l'intermédiaire d'un groupe alkylène en C₁ à C₆ ou un groupe (C=O)-, chacun non substitué ou substitué une ou plusieurs fois par des substituants identiques ou différents.

13. Composés substitués selon la revendication 12, dans lesquels :
(a1) la structure partielle de formule IIc peut représenter les structures partielles SP1 à SP34 suivantes : 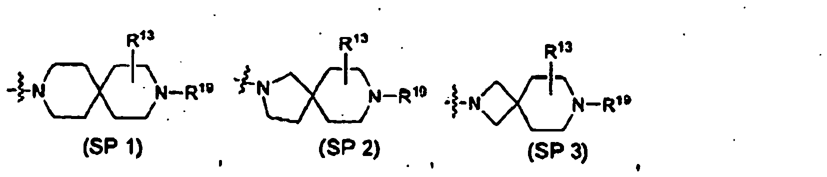 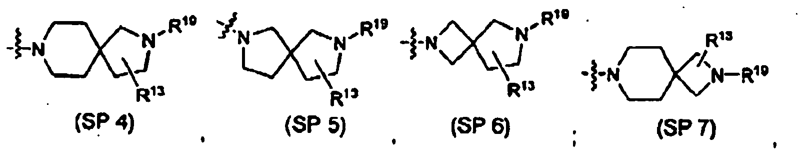 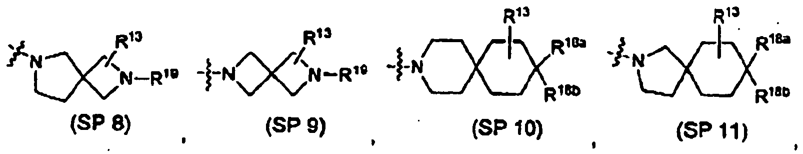 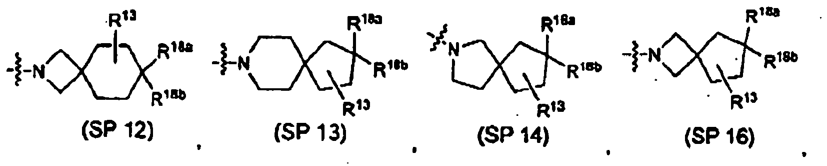 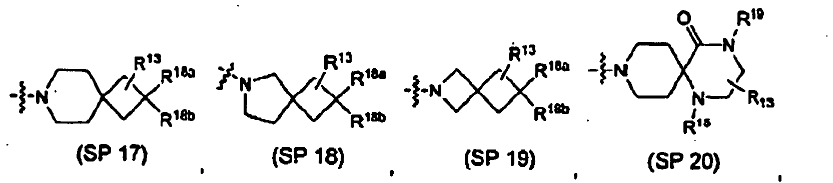 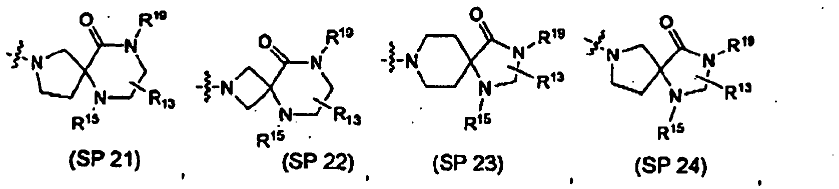 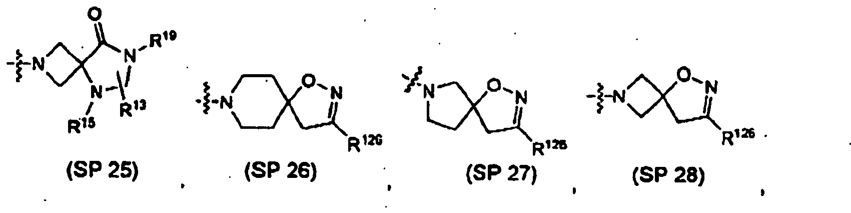 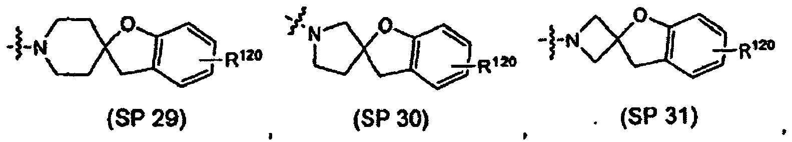 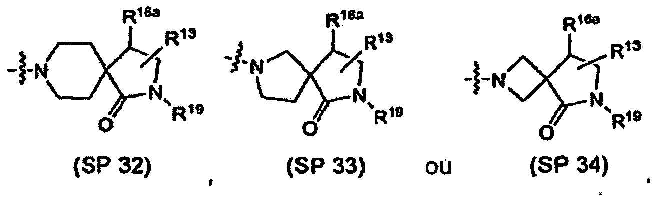 dans lesquelles :
R¹³ représente H ou un groupe phényle, à chaque fois non substitué ou substitué une ou plusieurs fois par des substituants identiques ou différents ; et/ou deux des substituants R¹³ forment ensemble un groupe =O,
et/ou deux substituants adjacents R¹³ forment un groupe aryle ou hétéroaryle condensé, en particulier un groupe benzo,
R¹⁵ représente H ; un groupe alkyle en C₁ à C₆ ; cycloalkyle en C₃ à C₈, phényle, pyridyle, pyrimidinyle, imidazolyle, triazolyle, thiazolyle ou thiényle, chacun non substitué ou substitué une ou plusieurs fois par des substituants identiques ou différents ; un groupe phényle, pyridyle, pyrimidinyle, imidazolyle, triazolyle, thiazolyle ou thiényle lié par l'intermédiaire d'un groupe alkylène en C₁ à C₆, chacun non substitué ou substitué une ou plusieurs fois par des substituants identiques ou différents ;
R^{16a} représente H, un groupe alkyle en C₁ à C₆, phényle, pyridyle, pyrimidinyle, imidazolyle, triazolyle, thiazolyle ou thiényle, chacun non substitué ou substitué une ou plusieurs fois par des substituants identiques ou différents ;
R^{18a} représente H ; un groupe alkyle en C₁ à C₆ ; cycloalkyle en C₃ à C₈, N (alkyle en C₁ à C₆)₂ ; NH (alkyle en C₁ à C₆), azétidinyle ; pyrrolidinyle, pipéridinyle, 4-(alkyle en C₁ à C₆)-pipérazinyle ; phényle, pyridyle, pyrimidinyle, imidazolyle, triazolyle, thiazolyle ou thiényle, chacun non substitué ou substitué une ou plusieurs fois par des substituants identiques ou différents ; un groupe N (alkyle en C₁ à C₆)₂ ; NH (alkyle en C₁ à C₆), azétidinyle ; pyrrolidinyle, pipéridinyle, 4-(alkyle en C₁ à C₆)-pipérazinyle ; phényle, pyridyle, pyrimidinyle, imidazolyle, triazolyle, thiazolyle ou thiényle lié par l'intermédiaire d'un groupe -(O)_{0/1}-(alkylène en C₁ à C₆), chacun non substitué ou substitué une ou plusieurs fois par des substituants identiques ou différents ;
R^{1ab} représente H ; un groupe OH ; un groupe alkyle en C₁ à C₆ ; phényle, pyridyle, pyrimidinyle, imidazolyle, triazolyle, thiazolyle ou thiényle, chacun non substitué ou substitué une ou plusieurs fois par des substituants identiques ou différents ; un groupe phényle, pyridyle, pyrimidinyle, imidazolyle, triazolyle, thiazolyle ou thiényle lié par l'intermédiaire d'un groupe alkylène en C₁ à C₆, chacun non substitué ou substitué une ou plusieurs fois par des substituants identiques ou différents ;
R¹⁹ représente H ; un groupe alkyle en C₁ à C₆ ; cycloalkyle en C₃ à C₈, phényle, pyridyle, pyrimidinyle, imidazolyle, triazolyle, thiazolyle ou thiényle, chacun non substitué ou substitué une ou plusieurs fois par des substituants identiques ou différents ; un groupe phényle, pyridyle, pyrimidinyle, imidazolyle, triazolyle, thiazolyle ou thiényle lié par l'intermédiaire d'un groupe alkylène en C₁ à C₆ ou un groupe (C=O)-, chacun non substitué ou substitué une ou plusieurs fois par des substituants identiques ou différents ;
R¹²⁰ représente H ; F ; Cl ; un groupe OH ; OCH₃, alkyle en C₁ à C₆ ; phényle, chacun non substitué ou substitué une ou plusieurs fois par des substituants identiques ou différents,
R¹²⁶ représente H ; un groupe alkyle en C₁ à C₆ ; cycloalkyle en C₃ à C₆ ; phényle, pyridyle, pyrimidinyle, imidazolyle, triazolyle, thiazolyle ou thiényle ; un groupe cycloalkyle en C₃ à C₆, phényle ou pyridyle lié par l'intermédiaire d'un groupe alkylène en C₁ à C₃, chacun non substitué ou substitué une ou plusieurs fois par des substituants identiques ou différents.

14. Composé selon l'une quelconque des revendications précédentes, dans lequel, dans la formule générale I, la structure partielle (B) 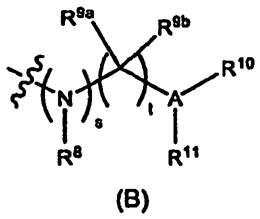 est choisie parmi 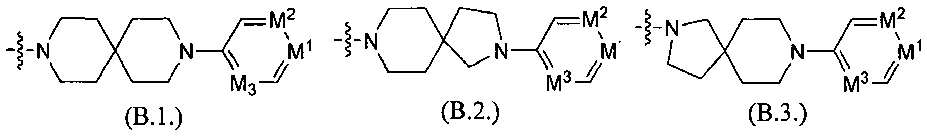 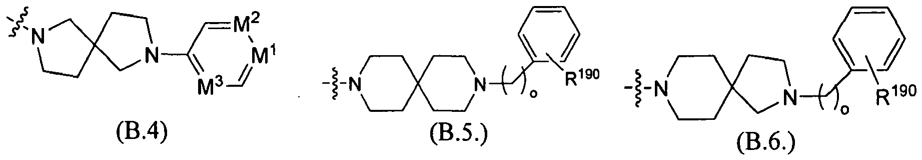 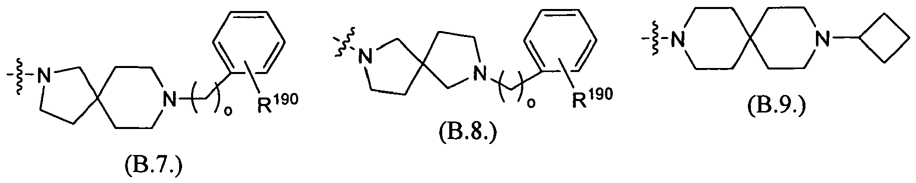 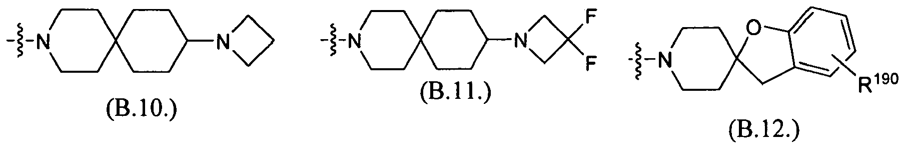 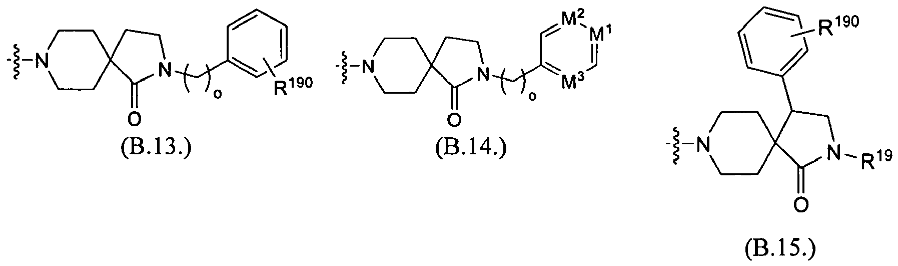 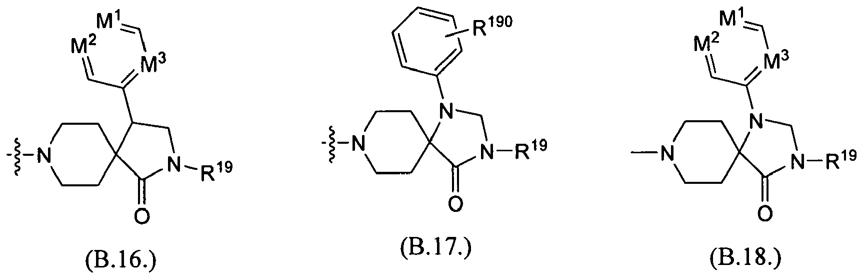 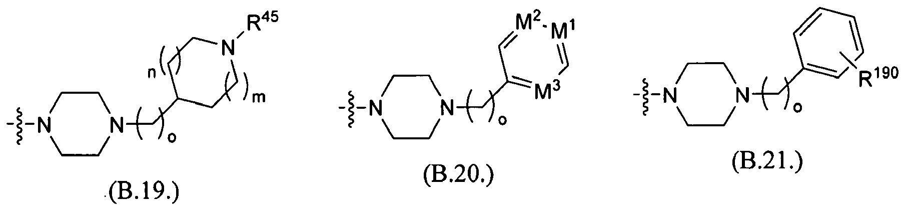 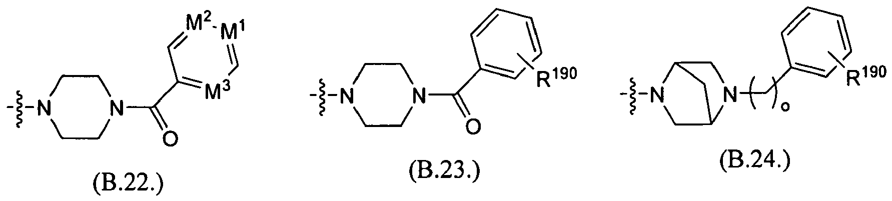 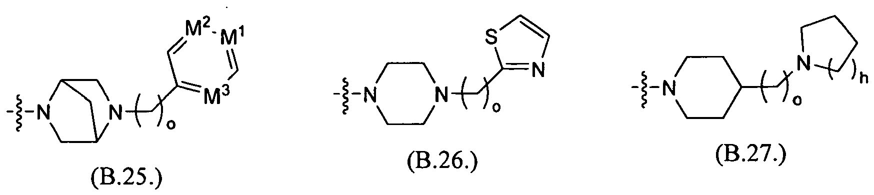 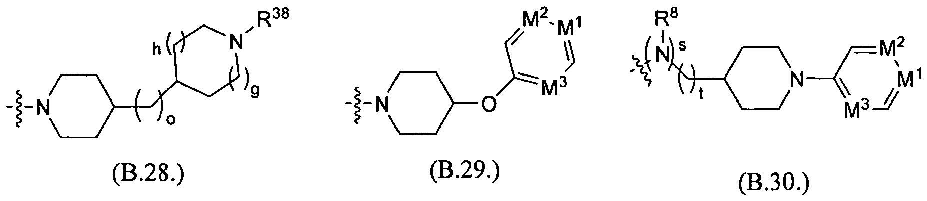 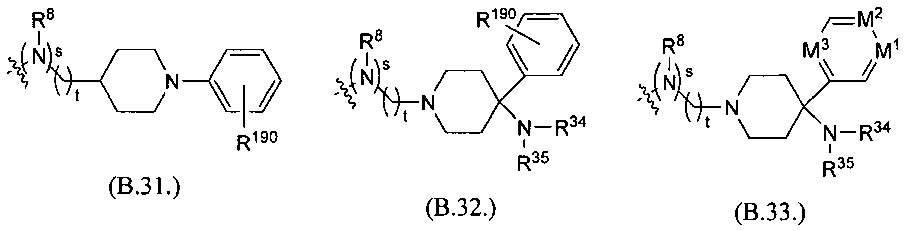 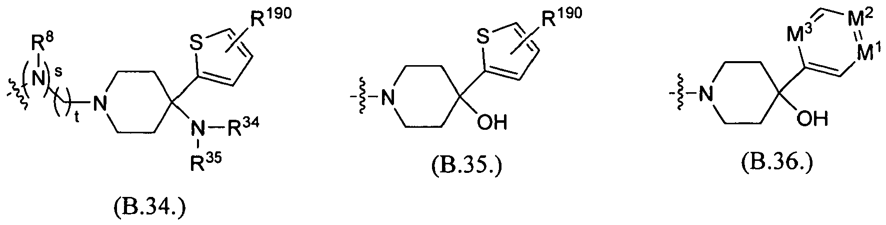 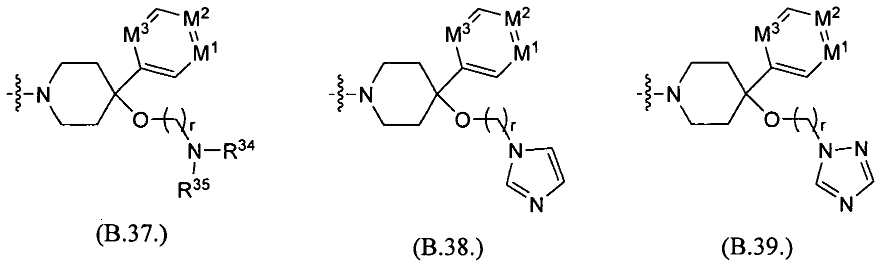 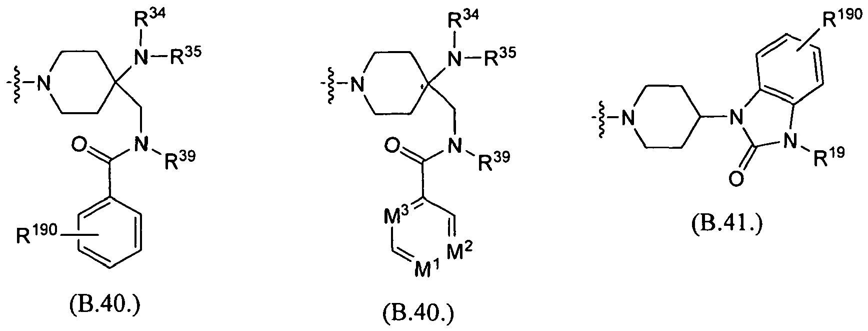 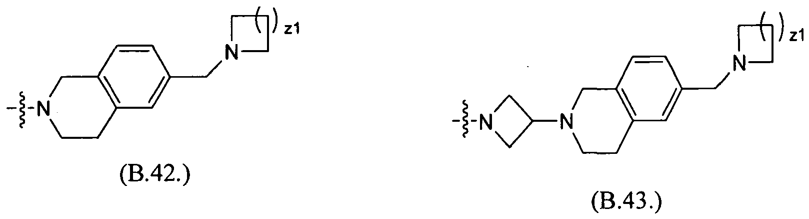 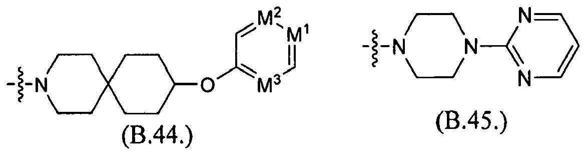 où :
h = 0 ou 1 ;
g = 0 ou 1 ;
m = 0 ou 1 ;
n = 0 ou 1 ;
o = 0, 1, 2 ou 3 ;
r = 1, 2 ou 3, en particulier 1 ou 2 ;
s = 0 ou 1 ;
t = 0, 1, 2 ou 3, en particulier 0, 1, ou 2, à condition que, lorsque s vaut 0, t vaut également 0 ;
z1 = 0, 1, 2 ou 3, en particulier 1,
M¹, M² et M³ représentent, indépendamment les uns des autres, N ou un groupe CH, une variable parmi M¹, M² et M³ représentant N et les deux autres un groupe CH ;
R⁸ représente H ; un groupe alkyle en C₁ à C₆, en particulier un groupe méthyle, éthyle, n-propyle, iso-propyle, n-butyle, sec-butyle, iso-butyle et tertio-butyle ; un groupe cycloalkyle en C₃ à C₆, en particulier cyclopropyle, chacun non substitué ou substitué une ou plusieurs fois par des substituants identiques ou différents ;
R¹⁹ est choisi parmi H _{;} un groupe alkyle en C₁ à C₆, en particulier un groupe méthyle, éthyle, n-propyle, iso-propyle, n-butyle, sec-butyle, iso-butyle et tertio-butyle ; cycloalkyle en C₃ à C₆, en particulier cyclopropyle ; chacun non substitué ou substitué une ou plusieurs fois par des substituants identiques ou différents ;
R³⁴ et R³⁵ représentent de préférence, indépendamment l'un de l'autre, un groupe méthyle ou éthyle ou représentent conjointement avec l'atome de N par lequel ils sont liés, un groupe azétidinyle ; pyrrolidinyle, pipéridinyle, 4-(alkyle en C₁ à C₆)-pipérazinyle, chacun non substitué ou substitué une ou plusieurs fois par des substituants identiques ou différents ;
R³⁸ représente H, un groupe alkyle en C₁ à C₆, cycloalkyle en C₃ à C₆ ou pyridyle ;
R³⁹ est choisi parmi H ; un groupe alkyle en C₁ à C₆, en particulier un groupe méthyle, éthyle, n-propyle, iso-propyle, n-butyle, sec-butyle, iso-butyle et tertio-butyle ; un groupe cycloalkyle en C₃ à C₆, en particulier cyclopropyle, chacun non substitué ou substitué une ou plusieurs fois par des substituants identiques ou différents ; et
R⁴⁵ représente H, un groupe alkyle en C₁ à C₆, cycloalkyle en C₃ à C₆ ou pyridyle ;
R¹⁹⁰ représente 0 à 4 substituants choisis indépendamment les uns des autres parmi des substituants F, Cl, O-CF₃, CF₃ ou CN.

15. Composés selon l'une quelconque des revendications précédentes, qui sont représentés par les formules générales C1 à C21 ci-dessous : 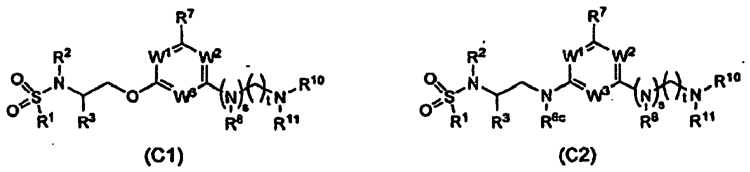 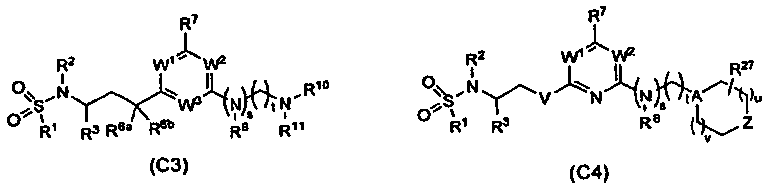 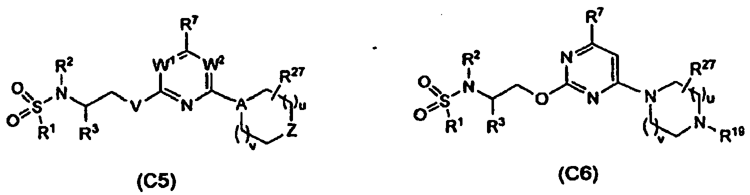 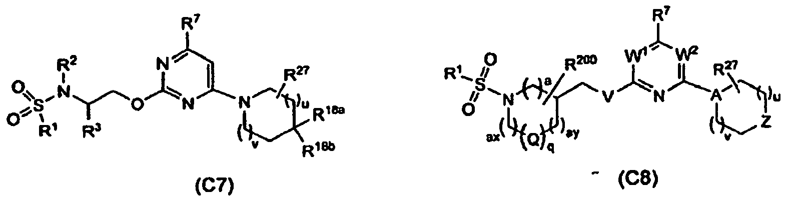 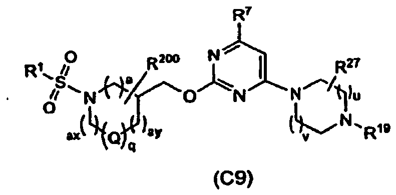 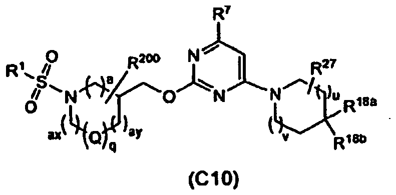 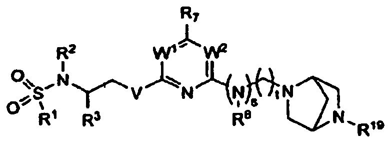 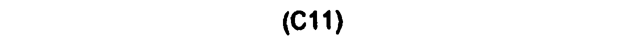 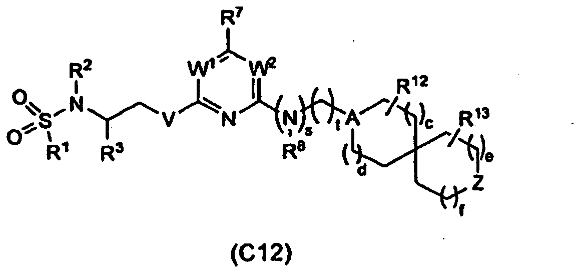 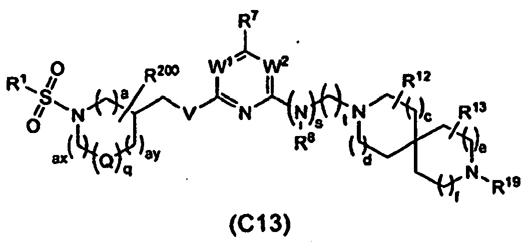 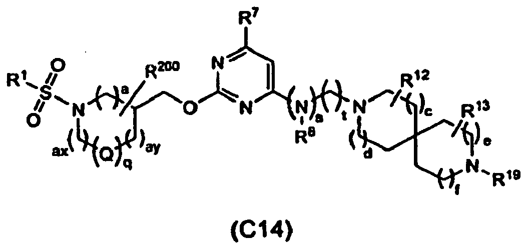 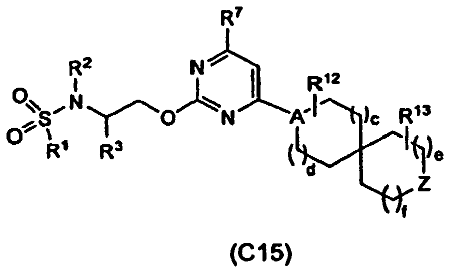 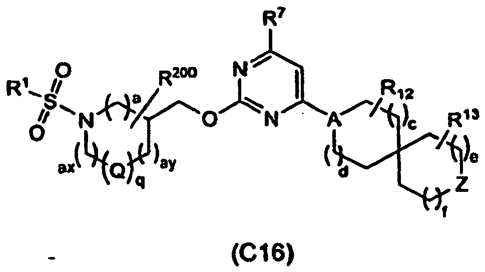 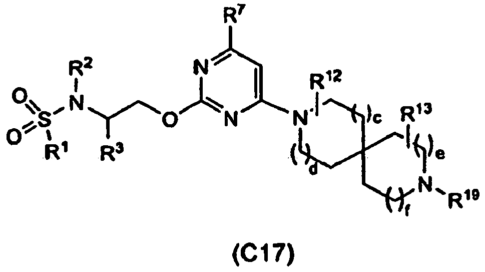 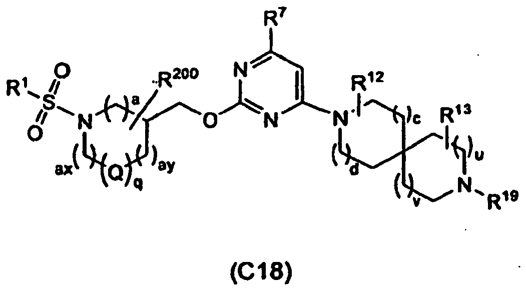 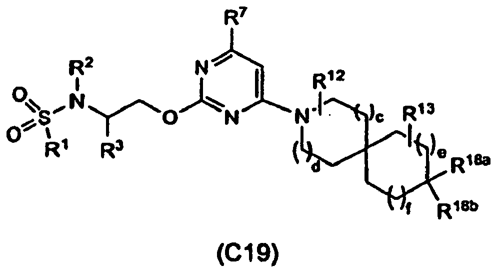 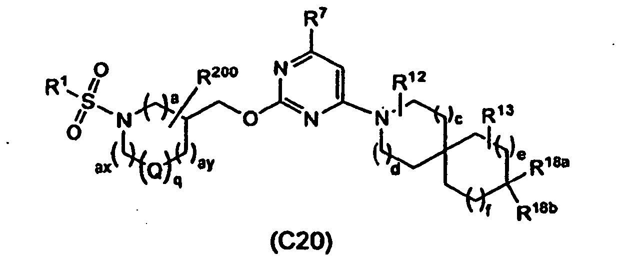 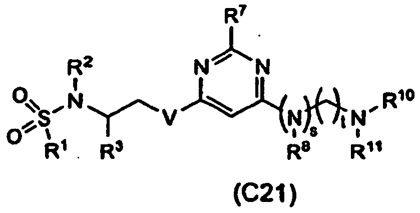
et dans lesquelles :
q vaut 0 ou 1,
a vaut 0, 1 ou 2 ;
ax vaut 0, 1, 2 ou 3 ;
ay vaut 0, 1 ou 2 ;
q vaut 0 ou 1 ;
à condition que a + ax + ay + q ≥ 2 ;
Q représente un groupe CH₂, NR⁵⁰, O, S, S=O ou S(=O)₂,
et tous les autres groupes, variables et indices ont les significations décrites dans les revendications 1 à 13 précédentes.

16. Composé selon l'une quelconque des revendications précédentes, le composé étant choisi parmi les suivants :
4-méthoxy-N,2,6-triméthyl-N-[2-[[4-[4-(2-1-pyrrolidinyl-éthyl)-1-pipéridinyl]-2-pyrimidinyl]oxy]éthyl]benzènesulfonamide,
4-méthoxy-N,2,6-triméthyl-N-[2-[[4-[4-(4-pyridyl)-l-pipérazinyl]-2-pyrimidinyl]oxy]éthyl]benzènesulfonamide,
N-[2-[[4-[2-(4-diméthylamino-4-phényl-1-pipéridinyl)-éthyl-méthylamino]-2-pyrimidinyl]oxyléthyl]-4-méthoxy-N,2,6-triméthylbenzènesulfonamide,
2- [[1-(4-méthoxy-2,6-diméthylphényl)sulfonyl-2-pipéridinyl] - méthoxy]-4-[4-(3-pyridyl)-4-(2-1-pyrrolidinyléthoxy)-1-pipéridinyl]pyrimidine,
chlorhydrate de N-cyclopropyl-4-méthoxy-2,6-diméthyl-N-[2-[[4-[9-(4-pyridyl)-3,9-diazaspiro[5.5]undécane-3-yl]-2-pyrimidinyl]oxy]éthyl]benzènesulfonamide,
N-cyclopropyl-4-méthoxy-2,6-diméthyl-N-[2-[[4-[2-[1-(4-pyridyl)-4-pipéridinyl]éthylamino]-2-pyrimidinyl]-oxy]éthyl]benzènesulfonamide,
N-cyclopropyl-4-méthoxy-2,6-diméthyl-N-[2-[[4-[4-(3-pyridyl)-4-(2-1-pyrrolidinyléthoxy)-1-pipéridinyl]-2-pyrimidinyl]oxy]éthyl]benzènesulfonamide,
N-cyclopropyl-N-[2-[[4-[4-hydroxy-4-(3-pyridyl)-1-pipéridinyl]-2-pyrimidinyl]oxy]éthyl]-4-méthoxy-2,6-diméthyl-benzènesulfonamide,
2-chloro-N-cyclopropyl-6-méthyl-N-[2-[[4-[9-(4-pyridyl)-3,9-diazaspiro[5.5]undécane-3-yl]-2-pyrimidinyl]-oxy]éthyl]benzènesulfonamide,
N-cyclopropyl-N-[2-[[4-[9-(4-pyridyl)-3,9-diazaspiro-[5.5]undécane-3-yl]-2-pyrimidinyl]oxy]éthyl)-2-(trifluoro-méthyl)benzènesulfonamide,
3-[2-[[(2S,4R)-4-fluoro-1-(4-méthoxy-2,6-diméthyl-phényl)sulfonyl-2-pyrrolidinyl]méthoxy]-4-pyrimidinyl]-9-(4-pyridyl)-3,9-diazaspiro[5.5]undécane,
N-cyclopropyl-4-méthoxy-2,6-diméthyl-N-[2-[[4-[4-(4-pyridyloxy)-1-pipéridinyl]-2-pyrimidinyl]oxy]éthyl]benzènesulfonamide,
N-[2-[[4-(6-(1-azétindinylméthyl]-3,4-dihydro-1H-iso-quinoléine-2-yl]-2-pyrimidinyl]oxy]éthyl]-N-cyclopropyl-4-méthoxy-2,6-diméthylbenzènesulfonamide,
N-cyclopropyl-4-méthoxy-2,6-diméthyl-N-[2-[[4-[8-(4-pyridyl)-3,8-diazaspiro[4.4]nonane-3-yl]-2-pyrimidinyl]-oxy]éthyl]benzènesulfonamide,
chlorhydrate de N-[2-[[4-[9-(1-azétidinyl)-3-aza-spiro[5.5]undécane-3-yl]-2-pyrimidinyl]oxy]éthyl]-N-cyclopropyl-4-méthoxy-2,6-diméthylbenzènesulfonamide,
N-cyclopropyl-4-méthoxy-2,6-diméthyl-N-[2-[[4-[9-(4-pyridyloxy)-3-azaspiro[5.5]undécane-3-yl]-2-pyrimidinyl]-oxy]éthyl]benzènesulfonamide,
N-cyclopropyl-N-[2-[[4-[9-(3,3-difluoro-1-azétidinyl)-3-azaspiro[5.5]undécane-3-yl]-2-pyrimidinyl]oxy]éthyl]-4-méthoxy-2,6-diméthylbenzènesulfonamide,
3-[2-[[1-(4-méthoxy-2,6-diméthylphényl)sulfonyl-3-azétidyl]oxy]-4-pyrimidinyl]-9-(4-pyridyl)-3,9-diaza-spiro[5.5]undécane,
N-cyclopropyl-4-méthoxy-2,6-diméthyl-N-[2-[[4-(8-(4-pyridyl)-3,8-diazaspiro[4.5]décane-3-yl]-2-pyrimidinyl]-oxy]éthyl]benzènesulfonamide,
N-[2-[[4-(3-[6-(1-azétidinylméthyl)-3,4-dihydro-1H-isoquinoléine-2-yl]-1-azétidinyl]-2-pyrimidinyl]oxy]éthyl]-N-cyclopropyl-4-méthoxy-2,6-diméthylbenzènesulfonamide,
amide d'acide 2,6-dichloro-N-cyclopropyl-3-méthyl-N-[2-[4-(9-pyridine-4-yl-3,9-diazaspiro[5.5]undécane-3-yl)-pyrimidine-2-yl]oxy-éthyl]benzènesulfonique,
amide d'acide 4-méthoxy-2,6-diméthyl-N-[1-[[4-(9-pyridine-4-yl-3,9-diazaspiro[5.5]undécane-3-yl)-pyrimidine-2-yl]oxyméthyl]cyclobutyl]benzènesulfonique,
amide d'acide N-cyclopropyl-4-méthoxy-2,6-diméthyl-N-[2-[4-[9-pyridine-3-yl-9-(2-pyrrolidine-1-yl-éthoxy)-3-azaspiro[5.5]undécane-3-yl]pyrimidine-2-yl]oxy-éthyl]benzènesulfonique,
amide d'acide N-[1,1-diméthyl-2-[4-(9-pyridine-4-yl-3,9-diazaspiro[5.5]undécane-3-yl)-pyrimidine-2-yl]-oxy-éthyl]-4-méthoxy-2,6-diméthyl-benzènesulfonique,
amide d'acide N-cyclopropyl-4-méthoxy-2,6-diméthyl-N-[3-[4-(9-pyridine-4-yl-3,9-diazaspiro[5.5]undécane-3-yl)-pyrimidine-2-yl]oxy-propyl]benzènesulfonique,
3-[2-[[(2S)-1-[(4-méthoxy-2,6-diméthyl-phényl)sulfonyl]-pipéridine-2-yl]méthoxy]pyrimidine-4-yl]-9-pyridine-4-yl-3,9-diazaspiro[5.5]undécane,
4-méthoxy-N,2,6-timéthyl-N-[2-[[2-[4-(2-1-pyrrolidinyl-éthyl)-1-pipéridinyl]-4-pyrimidinyl]oxy]éthyl]benzènesulfonamide,
4-méthoxy-N,2,6-triméthyl-N-[2-[[2-(4-(4-pyridyl)-1-pipérazinyl]-4-pyrimidyl]oxy]éthyl]benzènesulfonamide,
N-[2-[[2-[2-(4-diméthylamino-4-phényl-1-pipéridinyl)-éthyl-méthylamino]-4-pyrimidinyl]oxy]éthyl]-4-méthoxy-N,2,6-triméthylbenzènesulfonamide,
N-[2-(4-diméthylamino-4-phényl-1-pipéridinyl)éthyl]-4-[[1-(4-méthoxy-2,6-diméthylphényl)sulfonyl-2-pipéridinyl]méthoxy]-N-méthyl-2-pyrimidinamine,
N-cyclopropyl-4-méthoxy-2,6-diméthyl-N-[2-[[2-[4-(4-pyridyloxy)-1-pipéridinyl]-4-pyrimidinyl]oxy]éthyl]benzènesulfonamide,
2-chloro-N-cyclopropyl-6-méthyl-N-[2-[[2-[9-(4-pyridyl)-3,9-diazaspiro[5.5]undécane-3-yl]-4-pyrimidinyl]-oxy]éthyl]benzènesulfonamide,
3-[4-[[1-(4-méthoxy-2,6-diméthylphényl)sulfonyl-3-azétidinyl]oxy]-2-pyrimidinyl]-9-(4-pyridyl)-3,9-diaza-spiro[5.5]undécane
N-cyclopropyl-4-méthoxy-2,6-diméthyl-N-[2-[[6-[9-(4-pyridyl)-3,9-diazaspiro[5.5]undécane-3-yl]-4-pyrimidinyl]-oxy]éthyl]benzènesulfonamide,
amide d'acide N-cyclopropyl-4-méthoxy-2,6-diméthyl-N-[2-[méthyl-[6-(9-pyridine-4-yl-3,9-diazaspiro[5.5]undécane-3-yl)pyrazine-2-yl]amino]éthyl]benzènesulfonique,
amide d'acide N-cyclopropyl-4-méthoxy-2,6-diméthyl-N-[2-[méthyl-[6-(9-pyridine-4-yl-3,9-diazaspiro[5.5]undécane-3-yl)pyrimidine-4-yl]amino]éthyl]benzènesulfonique,
amide d'acide N-cyclopropyl-4-méthoxy-2,6-diméthyl-N-[2-[méthyl-[2-(9-pyridine-4-yl-3,9-diazaspiro[5.5]undécane-3-yl)pyrimidine-4-yl]amino]éthyl]benzènesulfonique,
amide d'acide N-cyclopropyl-4-méthoxy-2,6-diméthyl-N-[2-[méthyl-[4-(9-pyridine-4-yl-3,9-diazaspiro[5.5]undécane-3-yl)pyrimidine-2-yl]amino]éthyl]benzènesulfonique,
amide d'acide N-cyclopropyl-4-méthoxy-2,6-diméthyl-N-[3-[6-(9-pyridine-4-yl-3,9-diazaspiro[5.5]undécane-3-yl)-pyrazine-2-yl]propyl]benzènesulfonique,
amide d'acide N-cyclopropyl-4-méthoxy-2,6-diméthyl-N-[3-[2-(9-pyridine-4-yl-3,9-diazaspiro[5.5]undécane-3-yl)-pyrimidine-4-yl]propyl]benzènesulfonique,
amide d'acide N-cyclopropyl-N-[3-[2-(9-pyridine-4-yl-3,9-diazaspiro[5.5]undécane-3-yl)pyrimidine-4-yl]propyl]-3-(trifluorométhyl)benzènesulfonique,
4-méthoxy-N,2,6-triméthyl-N-[2-[[4-(4-(1-méthyl-4-pipéridinyl)-1-pipérazinyl]-2-pyrimidinyl]oxy]éthyl]-benzènesulfonamide,
2-[[1-(4-méthoxy-2,6-diméthylphényl)sulfonyl-2-pipéridinyl]méthoxy]-4-[4-(1-méthyl-4-pipéridinyl)-1-pipérazinyl]pyrimidine,
2-[[1-(4-méthoxy-2,6-diméthylphényl)sulfonyl-2-pipéridinyl]méthoxy]-4-[4-(2-1-pyrrolidinyléthyl)-1-pipéridinyl]pyrimidine,
N-[2-(4-diméthylamino-4-phényl-1-pipéridinyl)éthyl]-2-[[1-(4-méthoxy-2,6-diméthylphényl)sulfonyl-2-pipéridinyl]-méthoxy]-N-méthyl-4-pyrimidinamine,
N-[2-[[4-(4-(4-fluorophényl)-1-pipérazinyl]-2-pyrimidinyl]oxy]éthyl]-4-méthoxy-N,2,6-triméthylbenzènesulfonamide,
4-méthoxy-N,2,6-timéthyl-N-[2-[[4-[4-(4-méthyl-1-pipérazinyl)-1-pipéridinyl]-2-pyrimidinyl]oxy]éthyl]-benzènesulfonamide,
4-méthoxy-N,2,6-triméthyl-N-[2-[[4-(4-[(1-méthyl-4-pipéridinyl)méthyl]-1-pipérazinyl]-2-pyrimidinyl]oxy]-éthyl]benzènesulfonamide,
N-[2-[[4-[4-hydroxy-4-(3-pyridyl)-1-pipéridinyl]-2-pyrimidinyl]oxy]éthyl]-4-méthoxy-N,2,6-triméthylbenzènesulfonamide,
2-[[1-(4-méthoxy-2,6-diméthylphényl)sulfonyl-2-pipéridinyl]méthoxy]-4-(4-méthyl-1-pipérazinyl)pyrimidine,
4-[4-(4-fluorophényl)-1-pipérazinyl]-2-[[1-(4-méthoxy-2,6-diméthylphényl)sulfonyl-2-pipéridinyl]méthoxy]pyrimidine,
2-[[1-(4-méthoxy-2,6-diméthylphényl)sulfonyl-2-pipéridinyl]méthoxy]-4-[4-(4-méthyl-1-pipérazinyl)-1-pipéridinyl]pyrimidine,
2-[[1-(4-méthoxy-2,6-diméthylphényl)sulfonyl-2-pipéridinyl]méthoxy]-4-[4-(2-pyrimidinyl)-1-pipérazinyl]pyrimidine,
2-[[1-(4-méthoxy-2,6-diméthylphényl)sulfonyl-2-pipéridinyl]méthoxy]-4-[4-(4-pyridyl)-1-pipérazinyl]pyrimidine,
2-[[1-(4-méthoxy-2,6-diméthylphényl)sulfonyl-2-pipéridinyl]méthoxy]-4-[4-[(1-méthyl-4-pipéridinyl)méthyl]-1-pipérazinyl]pyrimidine,
2-[[(2S)-1-(4-méthoxy-2,6-diméthylphényl)sulfonyl-2-pyrrolidinyl]méthoxy]-4-(4-méthyl-1-pipérazinyl)pyrimidine,
2-[[(2S)-1-(4-méthoxy-2,6-diméthylphényl)sulfonyl-2-pyrrolidinyl]méthoxy]-4-[4-(2-1-pyrrolidinyléthyl)-1-pipéridinyl]pyrimidine,
2-[[(2S)-1-(4-méthoxy-2,6-diméthylphényl)sulfonyl-2-pyrrolidinyl]méthoxy]-4-[4-(4-méthyl-1-pipérazinyl)-1-pipéridinyl]pyrimidine,
2-[[(2S)-1-(4-méthoxy-2,6-diméthylphényl)sulfonyl-2-pyrrolidinyl]méthoxy]-4-[4-(2-pyrimidinyl)-1-pipérazinyl]-pyrimidine,
2-[[(2S)-1-(4-méthoxy-2,6-diméthylphényl)sulfonyl-2-pyrrolidinyl]méthoxy]-4-[4-(4-pyridyl)-1-pipérazinyl]-pyrimidine,
1-[2-[[(2S)-1-(4-méthoxy-2,6-diméthylphényl)sulfonyl-2-pyrrolidinyl]méthoxy]-4-pyrimidinyl]-4-(3-pyridyl)-4-pipéridinol,
1-[2-[[(2S)-1-(4-méthoxy-2,6-diméthylphényl)sulfonyl-2-pyrrolidinyl]méthoxy]-4-pyrimidinyl]-4-(2-thiényl)-4-pipéridinol,
3-benzyl-7-[2-[[1-(4-méthoxy-2,6-diméthylphényl)-sulfonyl-2-pipéridinyl]méthoxy]-4-pyrimidinyl]-3,7-diazaspiro[4,4]nonane,
1'-[2-[[1-(4-méthoxy-2,6-diméthylphényl)sulfonyl-2-pipéridinyl]méthoxy]-4-pyrimidinyl]spiro[1H-isobenzofurane-3,4'-pipéridine],
6-chloro-3-[1-[2-[[1-(4-méthoxy-2,6-diméthylphényl)-sulfonyl-2-pipéridinyl]méthoxy]-4-pyrimidinyl]-4-pipéridinyl]-1H-benzimidazole-2-one,
8-[2-[[1-(4-méthoxy-2,6-diméthylphényl)sulfonyl-2-pipéridinyl]méthoxy]-4-pyrimidinyl]-4-phényl-2,4,8-triaza-spiro[4.5]décane-1-one,
2-[[1-(4-méthoxy-2,6-diméthylphényl)sulfonyl-2-pipéridinyl]méthoxyl-4-[4-[2-(1-pipéridyl)éthyl]-1-pipéridinyl]pyrimidine,
2-[[1-(4-méthoxy-2,6-diméthylphényl)sulfonyl-3-pipéridinyl]oxy-4-[4-(2-(1-pipéridyl)éthyl]-1-pipéridinyl]-pyrimidine,
N-[2-[[4-(3-benzyl-3,7-diazaspiro[4.4]nonane-7-yl)-2-pyrimidinyl]oxy]éthyl]-4-méthoxy-N,2,6-triméthylbenzènesulfonamide,
4-méthoxy-N,2,6-triméthyl-N-[2-[[4-(1'-spiro[1H-isobenzofurane-3,4'-pipéridine]yl)-2-pyrimidinyl]oxy]-éthyl]benzènesulfonamide,
4-méthoxy-N,2,6-triméthyl-N-[2-[[4-(1-oxo-4-phényl-2,4,8-triazaspiro[4.5]décane-8-yl)-2-pyrimidinyl]oxyl-éthyl]benzènesulfonamide,
4-méthoxy-N,2,6-triméthyl-N-[2- [[4-[4- [2- (1-pipéridyl)-éthyl]-1-pipéridinyl]-2-pyrimidinyl]oxy]éthyl]benzènesulfonamide,
3-benzyl-7-[2-[[(2S)-1-(4-méthoxy-2,6-diméthylphényl)-sulfonyl-2-pyrrolidinyl]méthoxy]-4-pyrimidinyl]-3,7-diaza-spiro[4.4]nonane,
1'-[2-[[(2S)-1-(4-méthoxy-2,6-diméthylphényl)sulfonyl-2-pyrrolidinyl]méthoxy]-4-pyrimidinyl]spiro[1H-isobenzo-furane-3,4'-pipéridine],
6-chloro-3-[1-[2-[[(2S)-1-(4-méthoxy-2,6-diméthyl-phényl)sulfonyl-2-pyrrolidinyl]méthoxy]-4-pyrimidinyl]-4-pipéridinyl)-1H-benzimidazole-2-one,
8-[2-[[(2S)-1-(4-méthoxy-2,6-diméthylphényl)sulfonyl-2-pyrrolidinyl]méthoxy]-4-pyrimidinyl]-4-phényl-2,4,8-tri-azaspiro[4.5]décane-1-one,
2-[[(2S)-1-(4-méthoxy-2,6-diméthylphényl)sulfonyl-2-pyrrolidinyl]méthoxy]-4-[4-(1-méthyl-4-pipéridinyl)-1-pipérazinyl]pyrimidine,
2-[[(2S)-1-(4-méthoxy-2,6-diméthylphényl)sulfonyl-2-pyrrolidinyl]méthoxy]-4-[4-[2-(l-pipéridyl)éthyl]-1-pipéridinyl]pyrimidine,
3-(4-fluorophényl)-8-[2-[[1-(4-méthoxy-2,6-diméthyl-phényl)sulfonyl-2-pipéridinyl]méthoxy]-4-pyrimidinyl]-3,8-diazaspiro[4.5]décane-4-one,
3-[(4-fluorophényl)méthyl]-8-[2-[[1-(4-méthoxy-2,6-diméthylphényl) sulfonyl-2-pipéridinyl]méthoxy] -4-pyrimidinyl]-3,8-diazaspiro[4.5]décane-4-one,
3-benzyl-8-[2-[[1-(4-méthoxy-2,6-diméthylphényl)-sulfonyl-2-pipéridinyl]méthoxy]-4-pyrimidinyl]-3,8-diazaspiro[4.5]décane-4-one,
9-[2-[[1-(4-méthoxy-2,6-diméthylphényl)sulfonyl-3-pipéridinyl]oxy-4-pyrimidinyl]-3-(4-pyridyl)-3,9-diaza-spiro[5.5]undécane,
N-[2-[[4-[3-(4-fluorophényl)-4-oxo-3,8-diazaspiro-[4.5]décane-8-yl]-2-pyrimidinyl]oxy]éthyl]-4-méthoxy-N,2,6-triméthylbenzènesulfonamide,
4-méthoxy-N,2,6-triméthyl-N-[2-[[4-[4-oxo-1-[3-(trifluoro-méthyl)phényl]-3,8-diazaspiro[4.5]décane-8-yl]-2-pyrimidinyl]-oxy]éthyl]benzènesulfonamide,
N-[2-[[4-[1-(4-fluorophényl)-3-méthyl-4-oxo-3,8-diaza-spiro[4.5]décane-8-yl]-2-pyrimidinyl]oxy]éthyl]-4-méthoxy-N,2,6-triméthylbenzènesulfonamide,
N-[2-[[4-[3-[(4-fluorophényl)méthyl]-4-oxo-3,8-diaza-spiro[4.5]décane-8-yl]-2-pyrimidinyl]oxy]éthyl]-4-méthoxy-N,2,6-triméthylbenzènesulfonamide,
N-[2-[[4-(3-benzyl-4-oxo-3,8-diazaspiro[4.5]décane-8-yl)-2-pyrimidinyl]oxy]éthyl]-4-méthoxy-N,2,6-triméthylbenzènesulfonamide,
4-méthoxy-N,2,6-triméthyl-N-[2-[[4-(3-(4-pyridyl)-3,9-diazaspiro[5.5]undécane-9-yl]pyrimidinyl]oxy]éthyl]benzènesulfonamide,
3-(4-fluorophényl)-8-[2-[[(2S)-1-(4-méthoxy-2,6-diméthylphényl)sulfonyl-2-pyrrolidinyl]méthoxy]-4-pyrimidinyl]-3,8-diazaspiro[4.5]décane-4-one,
8-[2-[[(2S)-1-(4-méthoxy-2,6-diméthylphényl)sulfonyl-2-pyrrolidinyl]méthoxy]-4-pyrimidinyl]-1-[3-(trifluoro-méthyl)phényl]3,8-diazaspiro[4.5]décane-4-one,
1- (4-fluorophényl) -8- [2- [[(2S)-1-(4-méthoxy-2,6-diméthyl-phényl)sulfonyl-2-pyrrolidinyl]méthoxy]-4-pyrimidinyl]-3-méthyl-3,8-diazaspiro[4.5]décane-4-one,
3-[(4-fluorophényl]méthyl]-8-[2-[[(2S)-1-(4-méthoxy-2,6-diméthylphényl)sulfonyl-2-pyrrolidinyl]méthoxy]-4-pyrimidinyl]-3,8-diazaspiro[4.5]décane-4-one,
2-[[(2S)-1-(4-méthoxy-2,6-diméthylphényl)sulfonyl-2-pyrrolidinyl]méthoxy]-4-[4-(4-pyridyloxy)-1-pipéridinyl]pyrimidine,
3-benzyl-8-[2-[[(2S)-1-(4-méthoxy-2,6-diméthylphényl)-sulfonyl-2-pyrrolidinyl]méthoxy]-4-pyrimidinyl]-3,8-diaza-spiro[4.5]décane-4-one,
N-[[1-[2-[[(2R)-1-(4-méthoxy-2,6-diméthylphényl)-sulfonyl-2-pyrrolidinyl]méthoxy]-4-pyrimidinyl]-4-(4-mêthyl-1-pipérazinyl)-4-pipéridinyl]méthyl]-4-pyridine-carboxamide,
9-[2-[[(2S)-1-(4-méthoxy-2,6-diméthylphényl)sulfonyl-2-pyrrolidinyl]méthoxy]-4-pyrimidinyl]-3-(4-pyridyl)-3,9-diazaspiro(5.5]undécane,
5-[2-[[1-(4-méthoxy-2,6-diméthylphényl)sulfonyl-2-pipéridinyl]méthoxy)-4-pyrimidinyl]-2-(4-pyridylméthyl)-2,5-diazabicyclo[2.2.1]heptane,
5-[2-[[1-(4-méthoxy-2,6-diméthylphényl)sulfonyl-3-pipéridinyl]oxy-4-pyrimidinyl]-2-(4-pyridylméthyl)-2,5-diazabicyclo[2.2.1]heptane,
5-[2-[[(2S)-1-(4-méthoxy-2,6-diméthylphényl)sulfonyl-2-pyrrolidinyl]méthoxy]-4-pyrimidinyl]-2-(4-pyridylméthyl)-2,5-diazabicyclo[2.2.1]heptane,
4-méthoxy-N,2,6-triméthyl-N-[2-[[4-(4-(1-méthyl-4-pipéridinyl)-1-pipérazinyl]-2-pyrimidinyl]oxy]-1-phényl-éthyl]benzènesulfonamide,
N-[2-[[4-(3-[(4-fluorophényl)méthyl]-4-oxo-3,8-diaza-spiro[4.5]décane-8-yl]-2-pyrimidinyl]oxy-1-phényléthyl]-4-méthoxy-N,2,6-triméthylbenzènesulfonamide,
N-[2-[[4-(3-benzyl-4-oxo-3,8-diazaspiro[4.5]décane-8-yl)-2-pyrimidinyl]oxy-1-phényléthyl]-4-méthoxy-N,2,6-triméthylbenzènesulfonamide,
4-méthoxy-N,2,6-triméthyl-N-[1-phényl-2-[[4-[4-(3-pyridyl)-4-(2-1-pyrrolidinyléthoxy)-1-pipéridinyl]-2-pyrimidinyl]oxy]éthyl]benzènesulfonamide,
4-méthoxy-N,2,6-triméthyl-N-[1-phényl-2-[[4-[4-(4-pyridyloxy)-1-pipéridinyl]-2-pyrimidinyl]oxy]éthyl]benzènesulfonamide,
4-méthoxy-N,2,6-triméthyl-N- [2- [[4-[4- [oxo-(3-pyridyl)-méthyl]-1-pipérazinyl]-2-pyrimidinyl]oxy-1-phényléthyl]-benzènesulfonamide,
N-[2-[[4-[2-[(4-fluorophényl)méthyl]-2,5-diazabicyclo-[2.2.1]heptane-5-yl]-2-pyrimidinyl]oxy-1-phényléthyl]-4-méthoxy-N,2,6-triméthylbenzènesulfonamide,
2-[[1-(4-méthoxy-2,6-diméthylphényl)sulfonyl-2-pipéridinyl]méthoxy]-4-[4-(4-pyridyl)-1-pipérazinyl]-pyrimidine,
1-[2-[[1-(4-méthoxy-2,6-diméthylphényl)sulfonyl-2-pipéridinyl]méthoxy]-4-pyrimidinyl]-4-(3-pyridyl)-4-pipéridinol,
N-[2-[[4-(3-benzyl-3,7-diazaspiro[4.4]nonane-7-yl)-2-pyrimidinyl]oxy-1-phényléthyl]-4-méthoxy-N,2,6-triméthylbenzènesulfonamide,
[4-butyl-1-[2-[[1-[(4-méthoxy-2,6-diméthyl-phényl)-sulfonyl]pipéridine-2-yl]méthoxy]pyrimidine-4-yl]-diméthylamine,
[1-[2-[[1-[(4-méthoxy-2,6-diméthyl-phényl)sulfonyl]-pipéridine-2-yl]méthoxy]pyrimidine-4-yl]-4-thiophène-2-yl-pipéridine-4-yl]diméthylamine,
[2-[[1-[(4-méthoxy-2,6-diméthyl-phényl)sulfonyl]-pipéridine-2-yl]méthoxy]pyrimidine-4-yl]méthyl-[2-(4-phényl-4-pyrrolidine-1-yl-pipéridine-1-yl]éthyl]amine,
2-(4-butyl-4-diméthylamino-pipéridine-1-yl)éthyl-[2-[[1-[(4-méthoxy-2,6-diméthylphényl)sulfonyl]pipéridine-2-yl-méthoxy]pyrimidine-4-yl]méthylamine,
3-(4-diméthylamino-4-phényl-pipéridine-1-yl)propyl-[2-[[(2S)-1-[(4-méthoxy-2,6-diméthylphényl) sulfonyl] pyrrolidine-2-yl] méthoxy] pyrimidine-4-yl] méthylamine,
3-(4-diméthylamino-4-phényl-pipéridine-1-yl)propyl-[2-[[(2S)-1-[(4-méthoxy-2,6-diméthylphényl)sulfonyl]-2,3-dihydro-1H-indole-2-yl]méthoxy]pyrimidine-4-yl]méthylamine,
2- [1- [(4-méthoxy-2,6-diméthyl-phényl)sulfonyl]azétidine-3-yl] oxy-4-[4-[(1-méthyl-pipéridine-4-yl)méthyl]pipérazine-1-yl]pyrimidine,
2- [[1-[(4-méthoxy-2,6-diméthyl-phényl)sulfonyl]pipéridine-2-yl]méthoxy]-4-(4-pyridine-2-yloxy-pipéridine-1-yl)-pyrimidine,
2- [[1-[(4-méthoxy-2,6-diméthyl-phényl) sulfonyl]pipéridine-2-yl]méthoxy]-4-(4-pyrazine-2-yloxy-pipéridine-1-yl)-pyrimidine,
2-[[1-[(4-méthoxy-2,6-diméthyl-phényl)sulfonyl]-pipéridine-2-yl]méthoxy]-4-[4-pyridine-3-yl-4-(3-pyrrolidine-1-yl-propyl)-pipéridine-1-yl-pyrimidine,
2-[[1-[(4-méthoxy-2,6-diméthyl-phényl)sulfonyl]-pipéridine-2-yl]méthoxy]-4-[2-(pyridine-2-yl-méthyl)-pyrrolidine-1-yl]pyrimidine,
1-[2-[[1-[(4-méthoxy-2,6-diméthyl-phényl)sulfonyl]-pipéridine-2-yl]méthoxy]pyrimidine-4-yl]-4-pyridine-2-yl-pipéridine-4-ol,
1-[2-[[(2S)-1-[(4-méthoxy-2,6-diméthyl-phényl)-sulfonyl]pyrrolidine-2-yl]méthoxy]pyrimidine-4-yl]-4-pyridine-2-yl-pipéridine-4-ol,
2-[[(2R)-1-[(4-méthoxy-2,6-diméthyl-phényl)sulfonyl]-pyrrolidine-2-yl]méthoxy]-4-[2-(pyridine-2-yl-méthyl)-pyrrolidine-1-yl]pyrimidine,
5-[1-[2-[[(2S)-1-[(4-méthoxy-2,6-diméthyl-phényl)-sulfonyl]pyrrolidine-2-yl]méthoxy]pyrimidine-4-yl]pipéridine-4-yl]-3-pyridine-4-yl-[1,2,4]oxadiazole,
4-[4-(3-fluorophényl)-4-(2-pyrrolidine-1-yl-éthoxy)-pipéridine-1-yl]-2-[[(2S)-1-[(4-méthoxy-2,6-diméthyl-phényl)sulfonyl]pyrrolidine-2-yl]méthoxy]pyrimidine,
[4-butyl-1-[2-[[(2S)-1-[(4-méthoxy-2,6-diméthyl-phényl)sulfonyl]pyrrolidine-2-yl]méthoxy]pyrimidine-4-yl]pipéridine-4-yl]diméthylamine,
2-(4-diméthylamino-4-phényl-pipéridine-1-yl)-éthyl-[2-[[(2S)-1-[(4-méthoxy-2,6-diméthylphényl)sulfonyl]-pyrrolidine-2-yl]méthoxy]pyrimidine-4-yl]méthylamine,
amide d'acide N-[2-[4-(4-butyl-4-diméthylamino-pipéridine-1-yl)-pyrimidine-2-yl]oxy-éthyl]-4-méthoxy-N,2,6-triméthyl-benzènesulfonique,
[2-[[(2S)-1-[(4-méthoxy-2,6-diméthyl-phényl)sulfonyl]-pyrrolidine-2-yl]méthoxy]pyrimidine-4-yl]méthyl-[2-(4-phényl-4-pyrrolidine-1-yl-pipéridine-1-yl)éthyl]amine,
(2S)-1-[(4-méthoxy-2,6-diméthyl-phényl)sulfonyl]-2-[[4-(4-pyrazine-2-yloxy-pipéridine-1-yl)-pyrimidine-2-yl]oxy-méthyl]-2,3-dihydro-1H-indole,
[4-butyl-1-[2-[[(2S)-1-[(4-méthoxy-2,6-diméthyl-phényl)sulfonyl]-2,3-dihydro-1H-indole-2-yl]méthoxy]-pyrimidine-4-yl]pipéridine-4-yl]diméthylamine,
2-(4-diméthylamino-4-phényl-pipéridine-1-yl)éthyl-[2-[[(2S)-1-[(4-méthoxy-2,6-diméthylphényl)sulfonyl]-2,3-dihydro-1H-indole-2-yl]méthoxy]pyrimidine-4-yl]méthylamine,
[2-[[(2S)-1-[(4-méthoxy-2,6-diméthyl-phényl)sulfonyl]-2,3-dihydro-1H-indole-2-yl]méthoxy]pyrimidine-4-yl]méthyl-[2-(4-phényl-4-pyrrolidine-1-yl-pipéridine-1-yl)éthyl]-amine,
2-(4-butyl-4-diméthylamino-pipéridine-1-yl)éthyl-[2-[[(2S)-1-[(4-méthoxy-2,6-diméthylphényl)sulfonyl]-2,3-dihydro-1H-indole-2-yl]méthoxy]pyrimidine-4-yl]méthylamine,
amide d'acide N-[2-[4-[2-(4-diméthylamino-4-phényl-pipéridine-1-yl)-éthyl-méthylamino]pyrimidine-2-yl]oxy]-éthyl]-4-méthoxy-N,2,6-triméthyl-benzènesulfonique,
amide d'acide N-[2-[4-[3-(4-diméthylamino-4-phényl-pipéridine-1-yl)-propyl-méthylamino]pyrimidine-2-yl]oxy-éthyl]-4-méthoxy-N,2,6-triméthyl-benzènesulfonique,
3-(4-diméthylamino-4-phényl-pipéridine-1-yl)-propyl-[2-[[1-[(4-méthoxy-2,6-diméthylphényl)sulfonyl]pipéridine-2-yl]méthoxy]pyrimidine-4-yl]méthylamine,
5-[1-[2-[[1-[(4-méthoxy-2,6-diméthyl-phényl)sulfonyl]-pipéridine-2-yl]méthoxy]pyrimidine-4-yl]pipéridine-4-yl]-3-pyridine-4-yl-[1,2,4]oxadiazole,
4-[4-(3-fluorophényl)-4-(4-méthyl-pipérazine-1-yl)-pipéridine-1-yl]-2-[[1-[(4-méthoxy-2,6-diméthyl-phényl)-sulfonyl]piperidine-2-yl]méthoxy]pyrimidine,
(1S,5R)-8-[2-[[1-[(4-méthoxy-2,6-diméthyl-phényl)-sulfonyl]pipéridine-2-yl]méthoxy]pyrimidine-4-yl]-3-pyridine-3-yloxy-8-azabicyclo[3.2.1]octane,
1-[2-[[2-[[1-[(4-méthoxy-2,6-diméthyl-phényl)-sulfonyl)-pipéridine-2-yl]méthoxy]pyrimidine-4-yl]méthyl-amino]éthyl]-4-pyridine-3-yl-pipéridine-4-ol,
amide d'acide 4-méthoxy-N,2,6-triméthyl-N-[1-phényl-2-[4-[(1S,5R)-3-pyridine-3-yloxy-8-azabicyclo[3.2.1]octane-8-yl]pyrimidine-2-yl]oxy-éthyl]benzènesulfonique,
amide d'acide 4-méthoxy-N,2,6-triméthyl-N-[1-phényl-2-[4-[4-pyridine-3-yl-4-(3-pyrrolidine-1-yl-propyl)pipéridine-1-yl]pyrimidine-2-yl]oxy-éthyl]benzènesulfonique,
7-[2-[[(2S)-1-[(4-méthoxy-2,6-diméthyl-phényl)-sulfonyl]pyrrolidine-2-yl]méthoxy]pyrimidine-4-yl]-2-(pipéridine-1-yl-méthyl)-5,6,7,8-tetrahydro-imidazo[1,2-a]-pyrazine,
1-[2-[[1-[(4-méthoxy-2,6-diméthyl-phényl)sulfonyl]-pipéridine-2-yl]méthoxy]pyrimidine-4-yl]-4-pyridine-4-yl-pipéridine-4-ol,
amide d'acide N-[2-[4-(4-hydroxy-4-pyridine-4-yl-pipéridine-1-yl)-pyrimidine-2-yl]oxy-1-phényl-éthyl]-4-méthoxy-N,2,6-triméthyl-benzènesulfonique,
[1-[2-[[1-[(4-méthoxy-2,6-diméthyl-phényl)sulfonyl]-pipéridine-2-yl]méthoxy]pyrimidine-4-yl]-4-phényl-pipéridine-4-yl]diméthylamine,
2-[1-[(4-méthoxy-2,6-diméthyl-phényl)sulfonyl]-pipéridine-3-yl]oxy-4-(4-phényl-4-pyrrolidine-1-yl-pipéridine-1-yl)-pyrimidine,
2-[[(2S)-1-[(4-méthoxy-2,6-diméthyl-phényl)sulfonyl]-pyrrolidine-2-yl]méthoxy]-4-(4-phényl-4-pyrrolidine-1-yl-pipéridine-1-yl)-pyrimidine,
amide d'acide 4-méthoxy-N,2,6-triméthyl-N-[2-[4-(4-phényl-4-pyrrolidine-1-yl-pipéridine-1-yl)-pyrimidine-2-yl]oxy-éthyl]benzènesulfonique,
amide d'acide N-[2-[4-(4-diméthylamino-4-phényl-pipéridine-1-yl)-pyrimidine-2-yl]oxy-éthyl]-4-méthoxy-N,2,6-triméthyl-benzènesulfonique,
amide d'acide N-[2-[4-(4-diméthylamino-4-thiophène-2-yl-pipéridine-1-yl)-pyrimidine-2-yl]oxy-éthyl]-4-méthoxy-N,2,6-triméthyl-benzènesulfonique,
amide d'acide 4-méthoxy-N,2,6-triméthyl-N-[2-[4-[méthyl-[2-(4-phényl-4-pyrrolidine-1-yl-pipéridine-1-yl)-éthyl]amino]pyrimidine-2-yl]oxy-éthyl]benzènesulfonique,
amide d'acide N-[2-[4-[2-(4-butyl-4-diméthylamino-pipéridine-1-yl)-éthyl-méthylamino]pyrimidine-2-yl]oxy]-éthyl]-4-méthoxy-N,2,6-triméthyl-benzènesulfonique,
2-[[1-[(4-méthoxy-2,6-diméthyl-phényl)sulfonyl]pipéridine-2-yl]méthoxy]-4-(4-phényl-4-pyrrolidine-1-yl-pipéridine-1-yl)-pyrimidine,
2-[1-[(4-méthoxy-2,6-diméthyl-phényl)sulfonyl]-azétidine-3-yl]oxy-4-(4-phényl-4-pyrrolidine-1-yl-pipéridine-1-yl)-pyrimidine,
2-(4-diméthylamino-4-phényl-pipéridine-1-yl)-éthyl-[2-[1-[(4-méthoxy-2,6-diméthylphényl)sulfonyl]azétidine-3-yl]oxy-pyrimidine-4-yl]méthylamine,
[2-[1-[(4-méthoxy-2,6-diméthyl-phényl)sulfonyl]-azétidine-3-yl]oxy-pyrimidine-4-yl]méthyl-[2-(4-phényl-4-pyrrolidine-1-yl-pipéridine-1-yl)-éthyl]amine,
[1-[2-[[(2S)-1-[(4-méthoxy-2,6-diméthyl-phényl)-sulfonyl]-2,3-dihydro-1H-indole-2-yl]méthoxy]pyrimidine-4-yl]-4-thiophène-2-yl-pipéridine-4-yl]-diméthylamine,
3-(4-diméthylamino-4-phényl-pipéridine-1-yl)-propyl-[2-[1-[(4-méthoxy-2,6-diméthylphényl)sulfonyl]azétidine-3-yl]oxy-pyrimidine-4-yl]méthylamine,
3-[4-[2-[1-[(4-méthoxy-2,6-diméthyl-phényl)sulfonyl]-azétidine-3-yl]oxy-pyrimidine-4-yl]pipérazine-1-yl]propyl-diméthylamine,
1-[2-[1-[(4-méthoxy-2,6-diméthyl-phényl)sulfonyl]-azétidine-3-yl]oxy-pyrimidine-4-yl]-4-pyridine-3-yl-pipéridine-4-ol,
2-[1-[(4-méthoxy-2,6-diméthyl-phényl)sulfonyl]-azétidine-3-yl]-oxy-4-[4-(2-pipéridine-1-yl-éthyl)-pipéridine-1-yl]pyrimidine,
(2S)-2-[[4-[2-[(4-fluorophényl)-méthyl]-2,5-diaza-bicyclo[2.2.1]heptane-5-yl]pyrimidine-2-yl]oxyméthyl]-1-[(4-méthoxy-2,6-diméthyl-phényl)sulfonyl]-2,3-dihydro-1H-indole,
4-[1-[2-[[1-[(4-méthoxy-2,6-diméthyl-phényl)sulfonyl]-pipéridine-2-yl]méthoxy]pyrimidine-4-yl]-4-méthyl-pipéridine-4-yl]morpholine,
amide d'acide 4-méthoxy-N,2,6-triméthyl-N-[1-phényl-2-[4-[2-(pyridine-2-yl-méthyl)-pyrrolidine-1-yl]pyrimidine-2-yl]oxy-éthyl]benzènesulfonique,
amide d'acide 4-méthoxy-N,2,6-triméthyl-N-[1-phényl-2-[4-[4-(3-pyridine-4-yl-[1,2,4]oxadiazole-5-yl)-pipéridine-1-yl]pyrimidine-2-yl]oxy-éthyl]benzènesulfonique,
amide d'acide N-[2-[4-[4-(3-fluorophényl)-4-(4-méthyl-pipérazine-1-yl)-pipéridine-1-yl]pyrimidine-2-yl]oxy-1-phényl-éthyl]-4-inéthoxy-N,2,6-triméthyl-benzènesulfonique,
amide d'acide 4-méthoxy-N,2,6-triméthyl-N-[1-phényl-2-[4-(4-pyridine-2-yloxy-pipéridine-1-yl)-pyrimidine-2-yl]-oxy-éthyl]benzènesulfonique,
amide d'acide 4-méthoxy-N,2,6-triméthyl-N-[2-[4-(4-méthyl-4-morpholine-4-yl-pipéridine-1-yl)-pyrimidine-2-yl]-oxy-1-phényl-éthyl]benzènesulfonique,
amide d'acide N-[2-[4-[2-(4-hydroxy-4-pyridine-3-yl-pipéridine-1-yl)-éthyl-méthyl-amino]pyrimidine-2-yl]oxy-1-phényl-éthyl]-4-méthoxy-N,2,6-triméthyl-benzènesulfonique,
4-[[1-(4-méthoxy-2,6-diméthylphényl)sulfonyl-2-pipéridinyl]méthoxy]-2-(4-(1-méthyl-4-pipéridinyl)-1-pipérazinyl]pyrimidine,
4-[[1-(4-méthoxy-2,6-diméthylphényl)sulfonyl-2-pipéridinyl]méthoxy]-2-[4-(2-1-pyrrolidinyléthyl)-1-pipéridinyl]pyrimidine,
4-[[1-(4-méthoxy-2,6-diméthylphényl)sulfonyl-2-pipéridiny1]méthoxy]-2-(4-méthyl-1-pipérazinyl)pyrimidine,
4-[[1-(4-méthoxy-2,6-diméthylphényl)sulfonyl-2-pipéridinyl]méthoxy]-2-[4-(2-pyrimidinyl)-1-pipérazinyl]-pyrimidine,
4-[[1-(4-méthoxy-2,6-diméthylphényl)sulfonyl-2-pipéridinyl]méthoxy]-2- (4-(4-pyridyl)-1-pipérazinyl]pyrimidine,
4-[[1-(4-méthoxy-2,6-diméthylphényl)sulfonyl-2-pipéridinyl]méthoxy]-2-[4-[(1-méthyl-4-pipéridinyl)méthyl]-1-pipérazinyl]pyrimidine,
1-(4-[[1-(4-méthoxy-2,6-diméthylphényl)sulfonyl-2-pipéridinyl]méthoxy]-2-pyrimidinyl]-4-(3-pyridyl)-4-pipéridinol,
4-[[(2S)-1-(4-méthoxy-2,6-diméthylphényl)sulfonyl-2-pyrrolidinyl]méthoxy]-2-(4-méthyl-1-pipérazinyl)-pyrimidine,
4-[[(2S)-1-(4-méthoxy-2,6-diméthylphényl)sulfonyl-2-pyrrolidinyl]méthoxy]-2-[4-(2-1-pyrrolidinyléthyl)-1-pipéridinyl]pyrimidine,
4-[[(2S)-1-(4-méthoxy-2,6-diméthylphényl)sulfonyl-2-pyrrolidinyl]méthoxy]-2-[4-(4-méthyl-1-pipérazinyl)-1-pipéridinyl]pyrimidine,
4-[[(2S)-1-(4-méthoxy-2,6-diméthylphényl)sulfonyl-2-pyrrolidinyl]méthoxy]-2-[4-(2-pyrimidinyl)-1-pipérazinyl]-pyrimidine,
4-[[(2S)-1-(4-méthoxy-2,6-diméthylphényl)sulfonyl-2-pyrrolidinyl]méthoxy]-2-[4-[[1-méthyl-4-pipéridinyl)-méthyl]-1-pipérazinyl]pyrimidine,
1-[4-[[(2S)-1-(4-méthoxy-2,6-diméthylphényl)sulfonyl-2-pyrrolidinyl]méthoxy]-2-pyrimidinyl]-4-(3-pyridyl)-4-pipéridinol,
4-[[1-(4-méthoxy-2,6-diméthylphényl)sulfonyl-3-azétidinyl]oxy-2-[4-(2-1-pyrrolidinyléthyl)-1-pipéridinyl]-pyrimidine,
4-[[1-(4-méthoxy-2,6-diméthylphényl)sulfonyl-3-azétidinyl]oxy-2-(4-(4-méthyl-1-pipérazinyl)-1-pipéridinyl]-pyrimidine,
(2S)-1-(4-méthoxy-2,6-diméthylphényl)sulfonyl-2-[[2-(4-méthyl-1-pipérazinyl)-4-pyrimidinyl]oxyméthyl]indoline,
(2S)-1-(4-méthoxy-2,6-diméthylphényl)sulfonyl-2-[[2-[4-(2-1-pyrrolidinyléthyl)-1-pipéridinyl]-4-pyrimidinyl]-oxyméthyl]indoline,
(2S)-1-(4-méthoxy-2,6-diméthylphényl)sulfonyl-2-[[2-(4-(4-méthyl-1-pipérazinyl)-1-pipéridinyl]-4-pyrimidinyl]-oxyméthyl]indoline,
(2S)-1-(4-méthoxy-2,6-diméthylphényl)sulfonyl-2-[[2-[4-(4-pyridyl)-1-pipérazinyl]-4-pyrimidinyl]oxyméthyl]-indoline,
(2S)-1-(4-méthoxy-2,6-diméthylphényl)sulfonyl-2-[[2-[4-[(1-méthyl-4-pipéridinyl)méthyl]-1-pipérazinyl]-4-pyrimidinyl]oxyméthyl]indoline,
3-benzyl-7-[4-[[1-(4-méthoxy-2,6-diméthylphényl)-sulfonyl-2-pipéridinyl]méthoxy]-2-pyrimidinyl]-3,7-diazaspiro[4.4]nonane,
4-[[1-(4-mêthoxy-2,6-diméthylphényl)sulfonyl-2-pipéridinyl]méthoxy]-2-[4-[2-(1-pipéridyl)éthyl]-1-pipéridinyl]pyrimidine,
3-benzyl-7-[4-[[(2S)-1-(4-méthoxy-2,6-diméthylphényl)-sulfonyl-2-pyrrolidinyl]méthoxy]-2-pyrimidinyl]-3,7-diazaspiro[4.4]nonane,
8-[4-[[(2S)-1-(4-méthoxy-2,6-diméthylphényl)sulfonyl-2-pyrrolidinyl]méthoxy]-2-pyrimidinyl]-4-phényl-2,4,8-triazaspiro[4.5]décane-1-one,
4-[[(2S)-1-(4-méthoxy-2,6-diméthylphényl)sulfonyl-2-pyrrolidinyl]méthoxy]-2-[4-(1-méthyl-4-pipéridinyl)-1-pipérazinyl]pyrimidine,
4-[[(2S)-1-(4-méthoxy-2,6-diméthylphényl)sulfonyl-2-pyrrolidinyl]méthoxyl-2-[4-[2-(1-pipéridyl)éthyl]-1-pipéridinyl]pyrimidine,
(2S)-1-(4-méthoxy-2,6-diméthylphényl)sulfonyl-2-[[2-[4-(1-méthyl-4-pipéridinyl)-1-pipérazinyl]-4-pyrimidinyl]-oxyméthyl]indoline,
(2S)-1-(4-méthoxy-2,6-diméthylphényl)sulfonyl-2-[[2-[4-[2-(1-pipéridyl)éthyl]-1-pipéridinyl]-4-pyrimidinyl]-oxyméthyl]indoline,
4-méthoxy-N,2,6-triméthyl-N-[2-[[2-[3-(4-pyridyl)-3,9-diazaspiro[5.5]undécane-9-yl]-4-pyrimidinyl]-oxy]-éthyl]benzènesulfonamide,
3-[(4-fluorophényl)méthyl]-8-(4-[[1-(4-méthoxy-2,6-diméthylphényl)sulfonyl-2-pipéridinyl]méthoxy]-2-pyrimidinyl]-3,8-diazaspiro[4.5]décane-4-one,
N-[[1-[4-[[1-(4-méthoxy-2,6-diméthylphényl)sulfonyl-2-pipéridinyl]méthoxy]-2-pyrimidinyl]-4-(4-méthyl-1-pipérazinyl)-4-pipéridinyl]méthyl]-4-pyridinecarboxamide,
9-[4-[[1-(4-méthoxy-2,6-diméthylphényl)sulfonyl-2-pipéridinyl]méthoxy]-2-pyrimidinyl-3-(4-pyridyl)-3,9-diazaspiro[5.5]undécane,
4-méthoxy-N,2,6-triméthyl-N-[2-[[2-[4-(3-pyridyl)-4-(2-1-pyrrolidinyléthoxy)-1-pipéridinyl]-4-pyrimidinyl]-oxy]éthyl]benzènesulfonamide,
4-méthoxy-N,2,6-triméthyl-N-[2-[[2-[2-(4-pyridyl-méthyl)-2,5-diazabicyclo[2.2.1]heptane-5-yl]-4-pyrimidinyl]-oxy]éthyl]benzènesulfonamide,
4-[[1-(4-méthoxy-2,6-diméthylphényl)sulfonyl-2-pipéridinyl]méthoxy]-2-[4-(3-pyridyl)-4-(2-1-pyrrolidinyl-éthoxy)-1-pipéridinyl]pyrimidine,
4-méthoxy-N,2,6-triméthyl-N-[1-phényl-2-[[2-[4-(4-pyridyl)-1-pipérazinyl]-4-pyrimidinyl]oxy-éthyl]benzènesulfonamide,
4-méthoxy-N,2,6-triméthyl-N-[1-phényl-2-[[2-[3-(4-pyridyl)-3,9-diazaspiro[5.5]undécane-9-yl]-4-pyrimidinyl]-oxy]-éthyl]benzènesulfonamide,
N-méthyl-N-[1-phényl-2-[[2-[4-(3-pyridyl)-4-(2-1-pyrrolidinyléthoxy)-1-pipéridinyl]-4-pyrimidinyl]-oxy]-éthyl]-2-naphthalènesulfonamide,
N-[2-[[2-[3-[(4-fluorophényl)méthyl]-4-oxo-3,8-diazaspiro[4.5]décane-8-yl]-4-pyrimidinyl]oxy]éthyl]-4-méthoxy-N,2,6-triméthylbenzènesulfonamide,
4-[[(2S)-1-(4-méthoxy-2,6-diméthylphényl)sulfonyl-2-pyrrolidinyl]méthoxy]-2-[4-(3-pyridyl)-4-(2-1-pyrrolidinyl-éthoxy)-1-pipéridinyl]pyrimidine, ou
4-[[1-(4-méthoxy-2,6-diméthylphényl)sulfonyl-3-pipéridinyl]oxy-2-[4-[2-(1-pipéridyl)éthyl]-1-pipéridinyl]pyrimidine,
1-[4-[[1-[(4-méthoxy-2,6-diméthyl-phényl)sulfonyl]-pipéridine-2-yl]méthoxy]pyrimidine-2-yl]-4-(pyridine-2-yl-méthyl)-[1,4]diazépane,
1-[4-[[(2R)-1-[(4-méthoxy-2,6-diméthyl-phényl)-sulfonyl]pyrrolidine-2-yl]méthoxy]pyrimidine-2-yl]-4-(pyridine-2-yl-méthyl)-[1,4]diazépane,
2-(4-diméthylamino-4-phényl-pipéridine-1-yl)-éthyl-[4-[[1-[(4-méthoxy-2,6-diméthylphényl)sulfonyl]pipéridine-2-yl]méthoxy]pyrimidine-2-yl]méthylamine,
2-(4-butyl-4-diméthylamino-pipéridine-1-yl)-éthyl-[4-[[1-[(4-méthoxy-2,6-diméthylphényl)sulfonyl]pipéridine-2-yl]méthoxy]pyrimidine-2-yl]méthylamine,
3-(4-diméthylamino-4-phényl-pipéridine-1-yl)-propyl-[4-[[(2S)-1-[(4-méthoxy-2,6-diméthylphényl)sulfonyl]-pyrrolidine-2-yl]méthoxy]pyrimidine-2-yl]méthylamine,
1-[4-[[(2S)-1-[(4-méthoxy-2,6-diméthyl-phényl)-sulfonyl]pyrrolidine-2-yl]méthoxy]pyrimidine-2-yl]-4-pyridine-2-yl-pipéridine-4-ol,
5-[1-[4-[[(2S)-1-[(4-méthoxy-2,6-diméthyl-phényl)-sulfonyl]pyrrolidine-2-yl]méthoxy]pyrimidine-2-yl]pipéridine-4-yl]-3-pyridine-4-yl-[1,2,4]oxadiazole,
[4-[[1-[(4-méthoxy-2,6-diméthyl-phényl)sulfonyl]-pipéridine-2-yl]méthoxy]pyrimidine-2-yl]méthyl-[2-(4-pyridine-4-yloxy-pipéridine-1-yl)-éthyl]amine,
(1S,5R)-8-[4-[[1-[(4-méthoxy-2,6-diméthyl-phényl)-sulfonyl]pipéridine-2-yl]méthoxy]pyrimidine-2-yl]-3-pyridine-4-yloxy-8-azabicyclo[3.2.1]octane,
[4-[[(2S)-1-[(4-méthoxy-2,6-diméthyl-phényl)sulfonyl]-pyrrolidine-2-yl]méthoxy]pyrimidine-2-yl]méthyl-[2-(4-pyridine-4-yloxy-pipéridine-1-yl)-éthyl]amine,
(1S,5R)-8-[4-[[(2S)-1-[(4-méthoxy-2,6-diméthyl-phényl)sulfonyl]pyrrolidine-2-yl]méthoxy]pyrimidine-2-yl]-3-pyridine-4-yloxy-8-azabicyclo[3.2.1]octane,
2-(4-butyl-4-diméthylamino-pipéridine-1-yl)-éthyl-[4-[1-[(4-méthoxy-2,6-diméthylphényl)sulfonyl]pipéridine-3-yl]oxy-pyrimidine-2-yl]méthylamine,
2-(4-diméthylamino-4-phényl-pipéridine-1-yl)-éthyl-[4-[[(2S)-1-[(4-méthoxy-2,6-diméthyl-phényl)sulfonyl]-pyrrolidine-2-yl]méthoxy]pyrimidine-2-yl]méthylamine,
[4-[[(2S)-1-[(4-méthoxy-2,6-diméthyl-phényl)sulfonyl]pyrrolidine-2-yl]méthoxy]pyrimidine-2-yl]méthyl-[2-(4-phényl-4-pyrrolidine-1-yl-pipéridine-1-yl)-éthyl]amine,
2-(4-butyl-4-diméthylamino-pipéridine-1-yl)-éthyl-[4-[[(2S)-1-[(4-méthoxy-2,6-diméthylphényl)sulfonyl]-pyrrolidine-2-yl]méthoxy]pyrimidine-2-yl]méthylamine,
2-(4-diméthylamino-4-phényl-pipéridine-1-yl)-éthyl-[4-[[(2S)-1-[(4-méthoxy-2,6-diméthylphényl)sulfonyl]-2,3-dihydro-1H-indole-2-yl]méthoxy]pyrimidine-2-yl]méthylamine,
2-(4-butyl-4-diméthylamino-pipéridine-1-yl)-éthyl-[4-[[(2S)-1-[(4-méthoxy-2,6-diméthyl-phényl)sulfonyl]-2,3-dihydro-1H-indole-2-yl]méthoxy]pyrimidine-2-yl]méthylamine,
amide d'acide 4-méthoxy-N,2,6-triméthyl-N-[2-[2-[méthyl-[2-(4-phényl-4-pyrrolidine-1-yl-pipéridine-1-yl)-éthyl]amino]pyrimidine-4-yl]oxy-éthyl]benzènesulfonique,
amide d'acide N-[2-[2-[2-(4-butyl-4-diméthylamino-pipéridine-1-yl)-éthyl-méthylamino]pyrimidine-4-yl]oxy]-éthyl]-4-méthoxy-N,2,6-triméthyl-benzènesulfonique,
3-(4-diméthylamino-4-phényl-pipéridine-1-yl)-propyl-[4-[[1-[(4-méthoxy-2,6-diméthylphényl)sulfonyl]pipéridine-2-yl]méthoxy]pyrimidine-2-yl]méthylamine,
[4-butyl-1-[4-[[1-[(4-méthoxy-2,6-diméthyl-phényl)-sulfonyl] pipéridine-2-yl] méthoxy] pyrimidine-2-yl] pipéridine-4-yl]-diméthylamine,
[4-[[1-((4-méthoxy-2,6-diméthyl-phényl)sulfonyl]-pipéridine-2-yl]méthoxy]pyrimidine-2-yl]méthyl-[2-(4-phényl-4-pyrrolidine-1-yl-pipéridine-1-yl)-éthyl]amine,
[1-[4-[1-[(4-méthoxy-2,6-diméthyl-phényl)sulfonyl]-pipéridine-3-yl]oxy-pyrimidine-2-yl]-4-phényl-pipéridine-4-yl]-diméthylamine,
2-(4-diméthylamino-4-phényl-pipéridine-1-yl)-éthyl-[4-[1-[(4-méthoxy-2,6-diméthyl-phényl)sulfonyl]pipéridine-3-yl]oxy-pyrimidine-2-yl]méthylamine,
[4-[1-[(4-méthoxy-2,6-diméthyl-phényl)sulfonyl]-pipéridine-3-yl]oxy-pyrimidine-2-yl]méthyl-[2-(4-phényl-4-pyrrolidine-1-yl-pipéridine-1-yl)-éthyl]amine,
amide d'acide N-[2-[2-[2-(4-diméthylamino-4-phényl-pipéridine-1-yl)-éthyl-méthylamino]pyrimidine-4-yl]oxy-éthyl]-4-méthoxy-N,2,6-triméthyl-benzènesulfonique,
[4-butyl-1-[4-[[(2S)-1-[(4-méthoxy-2,6-diméthyl-phényl)sulfonyl]pyrrolidine-2-yl]méthoxy]pyrimidine-2-yl]pipéridine-4-yl]-diméthylamine,
2-(4-diméthylamino-4-phényl-pipéridine-1-yl)-éthyl-[4-[1-[(4-méthoxy-2,6-diméthyl-phényl)sulfonyl]azétidine-3-yl]oxy-pyrimidine-2-yl]méthylamine,
[4-[1-[(4-méthoxy-2,6-diméthyl-phényl)sulfonyl]-azétidine-3-yl]oxy-pyrimidine-2-yl]méthyl-[2-(4-phényl-4-pyrrolidine-1-yl-pipéridine-1-yl)-éthyl]amine,
2-(4-butyl-4-diméthylamino-pipéridine-1-yl)éthyl-[4-[1-[(4-méthoxy-2,6-diméthyl-phényl)sulfonyl]azétidine-3-yl)oxy-pyrimidine-2-yl]méthylamine,
[4-[[(2S)-1-[(4-méthoxy-2,6-diméthyl-phényl)sulfonyl]-2,3-dihydro-1H-indole-2-yl]méthoxy]pyrimidine-2-yl]méthyl-[2-(4-phényl-4-pyrrolidine-1-yl-pipéridine-1-yl)éthyl]-amine,
3-(4-diméthylamino-4-phényl-pipéridine-1-yl)propyl-[4-[1-[(4-méthoxy-2,6-diméthyl-phényl)sulfonyl]pipéridine-3-yl]oxy-pyrimidine-2-yl]méthylamine,
3-(4-diméthylamino-4-phényl-pipéridine-1-yl)propyl-[4-[1-[(4-méthoxy-2,6-diméthylphényl)sulfonyl]azétidine-3-yl]oxy-pyrimidine-2-yl]méthylamine,
3-(4-diméthylamino-4-phényl-pipéridine-1-yl)propyl-(4-[[(2S)-1-[(4-méthoxy-2,6-diméthylphényl)sulfonyl]-2,3-dihydydro-1H-indole-2-yl]méthoxy]pyrimidine-2-yl]méthylamine,
1-[4-[1-[(4-méthoxy-2,6-diméthyl-phényl)sulfonyl]-azétidine-3-yl]oxy-pyrimidine-2-yl]-4-pyridine-3-yl-pipéridine-4-ol,
1-[4-[1-[(4-méthoxy-2,6-diméthyl-phényl)sulfonyl]-azétidine-3-yl]oxy-pyrimidine-2-yl]-4-(pyrimidine-2-yl-méthyl)-[1,4]diazépane,
4-[[1-[(4-méthoxy-2,6-diméthyl-phényl)sulfonyl]-pipéridine-2-yl]méthoxy]-2-[2-(pyridine-2-yl-méthyl)-pyrrolidine-1-yl]pyrimidine,
4-[[1-[(4-méthoxy-2,6-diméthyl-phényl)sulfonyl]-pipéridine-2-yl]méthoxy]-2-(4-pyridine-2-yloxy-pipéridine-1-yl)-pyrimidine,
4-[[1-[(4-méthoxy-2,6-diméthyl-phényl)sulfonyl]-pipéridine-2-yl]méthoxy]-2-(4-pyrazine-2-yloxy-pipéridine-1-yl)-pyrimidine,
4-[1-[4-[[1-[(4-méthoxy-2,6-diméthyl-phényl)sulfonyl]-pipéridine-2-yl]méthoxy]pyrimidine-2-yl]-4-méthyl-pipéridine-4-yl]morpholine,
4-[[1-[(4-méthoxy-2,6-diméthyl-phényl)sulfonyl]-pipéridine-2-yl]méthoxy]-2-[4-pyridine-3-yl-4-(3-pyrrolidine-1-yl-propyl)-pipéridine-1-yl]pyrimidine,
1-[4-[[1-[(4-méthoxy-2,6-diméthyl-phényl)sulfonyl]-pipéridine-2-yl]méthoxy]pyrimidine-2-yl]-4-pyridine-2-yl-pipéridine-4-ol,
4-[[(2R)-1-[(4-méthoxy-2,6-diméthyl-phényl)sulfonyl]-pyrrolidine-2-yl]méthoxy]-2-[2-(pyridine-2-yl-méthyl)-pyrrolidine-1-yl]pyrimidine,
[1-[4-[[(2S)-1-[(4-méthoxy-2,6-diméthyl-phényl)-sulfonyl]pyrrolidine-2-yl]méthoxy]pyrimidine-2-yl]-4-phényl-pipéridine-4-yl]-diméthylamine,
[1-[4-[[(2S)-1-[(4-méthoxy-2,6-diméthyl-phényl)-sulfonyl]pyrrolidine-2-yl]méthoxy]pyrimidine-2-yl]-4-thiophène-2-yl-pipéridine-4-yl]-diméthylamine,
amide d'acide 4-méthoxy-N,2,6-triméthyl-N-[2-[2-[2-(pyridine-2-yl-méthyl)-pyrrolidine-1-yl]pyrimidine-4-yl]-oxy]éthyl]benzènesulfonique,
4-[[(2S)-1-[(4-méthoxy-2,6-diméthyl-phényl)sulfonyl]-pyrrolidine-2-yl]méthoxy]-2-[2-(pyridine-2-yl-méthyl)-pyrrolidine-1-yl]pyrimidine,
amide d'acide 4-méthoxy-N,2,6-triméthyl-N-[2-[2-[méthyl-(2-(4-pyridine-4-yloxy-pipéridine-1-yl)éthyl]-amino]pyrimidine-4-yl)oxy-éthyl]benzènesulfonique,
amide d'acide N- [2- (2- [4-(3-fluorophényl)-4-(2-pyrrolidine-1-yl-éthoxy)-pipéridine-1-yl]pyrimidine-4-yl]oxy-éthyl]-4-méthoxy-N,2,6-triméthyl-benzènesulfonique,
2-(4-(3-fluorophényl)-4-(4-méthyl-pipérazine-1-yl)-pipéridine-1-yl-4-[[1-((4-méthoxy-2,6-diméthyl-phényl)-sulfonyl]pipéridine-2-yl]méthoxy]pyrimidine,
2-[4-(3-fluorophényl)-4-(2-pyrrolidine-1-yl-éthoxy)-pipéridine-1-yl]-4-[[(2S)-1-[(4-méthoxy-2,6-diméthyl-phényl)-sulfonyl-pyrrolidine-2-yl]méthoxy]pyrimidine,
amide d'acide 4-méthoxy-N,2,6-triméthyl-N-[1-phényl-2-[2-[(1S,5R)-3-pyridine-4-yloxy-8-azabicyclo[3.2.1]octane-8-yl]pyrimidine-4-yl]oxy-éthyl]benzènesulfonique.

17. Médicament contenant au moins un composé selon une ou plusieurs des revendications 1 à 15, éventuellement contenant des additifs appropriés et/ou des adjuvants et/ou d'autres principes actifs.

18. Utilisation d'au moins un composé selon l'une quelconque des revendications 1 à 15, pour la fabrication d'un médicament pour le traitement de la douleur, en particulier de la douleur aiguë, de la douleur viscérale, de la douleur neuropatique, et/ou de la douleur chronique, de la douleur inflammatoire, de la migraine, du diabète, de maladies des voies respiratoires, de maladies inflammatoires intestinales, de maladies neurologiques, d'inflammations de la peau, de maladies rhumatismales, du choc septique, du syndrome de reperfusion, de l'obésité et en tant qu'inhibiteur de l'angiogenèse.
